(19) Europäisches Patentamt
European Patent Office
Office européen des brevets

(11) **EP 4 378 938 A1**

(12) **EUROPEAN PATENT APPLICATION**
published in accordance with Art. 153(4) EPC


(43) Date of publication:
**05.06.2024 Bulletin 2024/23**

(21) Application number: **22848618.9**

(22) Date of filing: **28.07.2022**

(51) International Patent Classification (IPC):
***C07D 403/10*** (2006.01) ***C07D 237/32*** (2006.01)
***A61K 31/501*** (2006.01) ***A61P 35/00*** (2006.01)

(52) Cooperative Patent Classification (CPC):
**A61K 31/501; A61P 35/00; C07D 237/32; C07D 403/10**

(86) International application number:
**PCT/CN2022/108475**

(87) International publication number:
**WO 2023/006013 (02.02.2023 Gazette 2023/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.07.2021 CN 202110862671**
**03.03.2022 CN 202210208759**
**15.07.2022 CN 202210839712**



(71) Applicant: **Shanghai Qilu Pharmaceutical Research and Development Centre Ltd.**
**Shanghai 201203 (CN)**

(72) Inventors:
- **YAN, Xiaoxia**
  **Shanghai 201203 (CN)**
- **HUANG, Guanxin**
  **Shanghai 201203 (CN)**
- **JU, Wei**
  **Shanghai 201203 (CN)**
- **SUN, Daqing**
  **Shanghai 201203 (CN)**

(74) Representative: **De Clercq & Partners**
**Edgard Gevaertdreef 10a**
**9830 Sint-Martens-Latem (BE)**


# (54) NOVEL PARP7 INHIBITOR AND USE THEREOF


(57) Disclosed are a compound serving as a PARP7 inhibitor, and a use thereof in preparing a drug for treating relevant diseases. Specifically disclosed are a compound represented by formula (I) and a pharmaceutically acceptable salt thereof. The compound can be used for treating cancer.


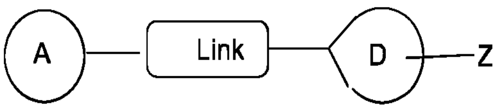

(I)

EP 4 378 938 A1

## Description

The present application claims the right of the priorities of Chinese patent application 2021108626719 filed on July 29, 2021, Chinese patent application 2022102087593 filed on March 3, 2022, and Chinese patent application 2022108397127 filed on July 15, 2022. The contents of the above Chinese patent applications are incorporated herein by reference in their entireties.

TECHNICAL FIELD

The present disclosure belongs to the field of medicinal chemistry, relates to a PARP7 inhibitor, a preparation method therefor, and a use thereof in the manufacture of a substance for treating relevant diseases, and specifically relates to a compound of formula (I) and a pharmaceutically acceptable salt thereof.

BACKGROUND

The poly(ADP-ribose) polymerase (PARP) family consists of 17 members and regulates fundamental cellular processes including gene expression, protein degradation, and multicellular stress responses (CoHen, P. Chang, Insights into the biogenesis, function, and regulation of ADP- ribosylation. Nat Chem Biol 14, 236-243 (2018)). The ability of cancer cells to survive under stress is a fundamental cancer mechanism and a new way for novel therapeutic agents.

17 members of the PARP family were identified in the human genome based on homology within the catalytic domain. However, the PARP family members are divided into three subfamilies based on differences in activity, namely polyPARPs, monoPARPs, and inactive (S.Vyas et al., Family-wide analysis of poly(ADP-ribose) polymerase activity. Nat Commun 5, 4426 (2014)). Most PARP family members catalyze the transfer of mono(ADP-ribose) units on their substrates (monoPARPs), and other members (PARP1, PARP2, TNKS, TNKS2) catalyze the transfer of poly(ADP-ribose) units on their substrates (polyPARPs), whereas PARP13 is the only PARP so far that shows no catalytic activity *in vitro* or *in vivo.* The monoPARPs modify their targets through a mono ADP-ribose unit thereby enabling the regulation of signal transduction pathways, a process similar to kinase phosphorylation. The polyPARPs modify their protein targets with large branched polymers called poly(ADP-ribose), a large and highly charged attachment that forms a protein scaffold that guides the protein to function at specific points within the cell.

It has been shown that PARP1 of the PARP family is a potent cancer target associated with DNA damage-induced cellular stress, induced by genetic mutations or cytotoxic chemotherapy. There are four clinically approved drugs, as well as several others in late-stage development (A. Ohmoto, S. Yachida, Current status of poly(ADP-ribose) polymerase inhibitors and future directions. Onco Targets Ther 10, 5195-5208 (2017)).

PARP7 gene is located on chromosome 3 (3q25), and the gene is located in a frequently amplified region in squamous cell carcinoma histology. A genome-wide association study has identified susceptibility genes for ovarian cancer, suggesting a role for PARP7 in this type of cancer (E. L. Goode et al., A genome-wide association study identifies susceptibility loci for ovarian cancer at 2q31 and 8q24. Nat Genet 42, 874-879 (2010)). PARP7 has multiple cellular functions. Under AHR signaling, PARP7 acts as a negative feedback mechanism to regulate the expression of P4501A1 and P4501B1. PARP7 has also been described to ADP-ribosylate liver X receptors, leading to regulation of its transcriptional activity (C. Bigetsboll et al., TCDD-Inducible poly-ADP-ribose (TIPARP/PARP7) mono-ADP-ribosylates and co-activates liver X receptors. Biochem J 473, 899-910 (2016)). During viral infection, PARP7 can bind to Sindbis virus to promote viral RNA degradation. Furthermore, in the context of viral infection, AHR-induced PARP7 can interact with TBK1, a major kinase during the onset of pathogen-associated molecular pattern pathways, leading to activation of type I interferon response and antiviral immunity. PARP7 is shown to ADP-ribosylate TBK1, preventing TBK1 activation and thus inhibiting type I interferon response.

Studies have shown that many cancer cells rely on PARP7 for intrinsic cell survival, and that PARP7 enables cancer cells to "hide" from the immune system. Therefore, inhibition of PARP7 can effectively inhibit the growth of cancer cells, restore interferon signaling, and effectively inhibit cancer. PARP7 inhibitors exhibit sustained tumor growth inhibition, potent antiproliferative activity, and restoration of interferon signaling in several cancer models. *In vivo* models have also demonstrated the induction of tumor-specific adaptive immune memory. This action is achieved by activating IFN-β signaling in immune cells, developing immune memory to enhance immune system signaling, and achieving immune memory for tumor suppression. Studies have shown that PARP7 used alone has good *in vivo* efficacy. There is only one PARP7 inhibitor under development by Ribon in clinical trials at this stage, and no other relevant PARP7 inhibitors have been reported. Therefore, there is a need to develop a class of compounds with PARP7 inhibitory activity.

CONTENT OF THE PRESENT INVENTION

The object of the present disclosure is to provide a compound with PARP7 inhibitory activity or a pharmaceutically

acceptable salt thereof.

The present disclosure provides a compound of formula (I) or a pharmaceutically acceptable salt thereof,

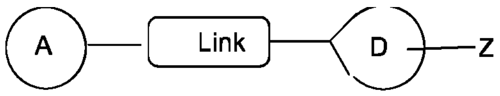
(I)

wherein

ring A is selected from the following groups:

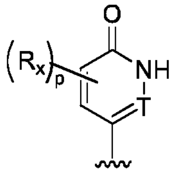 (A-1); 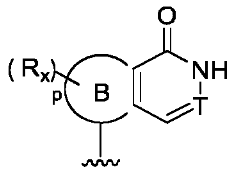 (A-2); or 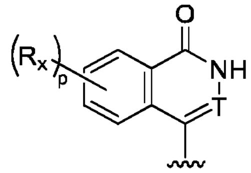 (A-3);

T is selected from CH or N;
ring B is selected from $C_{6-10}$ aryl, $C_{3-7}$ cycloalkyl, 5- to 10-membered heteroaryl, and 4- to 10-membered heterocyclyl;
ring D is 4- to 10-membered heterocyclyl, and the 4- to 10-membered heterocyclyl is optionally substituted by p substituents independently selected from $R_y$;
Z is selected from H, $Cy^2$, -NH-$Cy^2$, halogen, $C_{1-6}$ alkyl, $C_{3-7}$ cycloalkylacyl, $C_{1-6}$ alkoxy, $C_{1-6}$ haloalkyl, CN, and $NO_2$; wherein $Cy^2$ is selected from $C_{6-10}$ aryl, $C_{3-7}$ cycloalkyl, 5- to 10-membered heteroaryl, and 4- to 10-membered heterocyclyl, and each of the $Cy^2$ is optionally substituted by p substituents independently selected from $R_z$;
$R_x$ and $R_z$ are selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, CN, $NO_2$, $NH_2$, $C_{3-7}$ cycloalkyl, hydroxyl-$C_{1-6}$ alkyl-, $C_{1-6}$ alkyl-C(O)-, $C_{1-6}$ alkyl-$S(O)_2$-, and $C_{1-6}$ alkyl-NH-C(O)-;
$R_y$ is selected from hydrogen and $C_{1-6}$ alkyl; the $C_{1-6}$ alkyl is optionally substituted by OH, CN, or $OCH_3$;
Link is a group linking ring A and ring D;
p is an integer selected from 0, 1, and 2.

Specifically, the present disclosure also provides a compound of formula (II) or a pharmaceutically acceptable salt thereof:

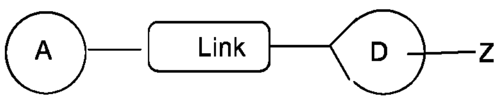
(II)

wherein

ring A is selected from the following groups:

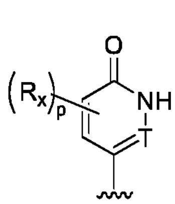 (A-1); or 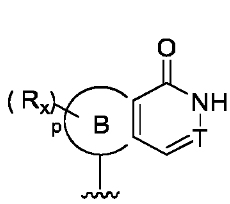 (A-2);

T is selected from CH or N;
ring B is selected from $C_{6-10}$ aryl, $C_{3-7}$ cycloalkyl, 5- to 10-membered heteroaryl, and 4- to 10-membered heterocyclyl;
ring D is 4- to 10-membered heterocyclyl, and the 4- to 10-membered heterocyclyl is optionally substituted by p substituents independently selected from $R_y$;
Z is selected from H, $Cy^2$, halogen, $C_{1-6}$ alkyl, $C_{1-6}$ alkoxy, $C_{2-6}$ alkenyl, $C_{2-6}$ alkynyl, $C_{1-6}$ haloalkyl, CN, and $NO_2$; wherein $Cy^2$ is selected from $C_{6-10}$ aryl, $C_{3-7}$ cycloalkyl, 5- to 10-membered heteroaryl, and 4- to 10-membered heterocyclyl, and each of the $Cy^2$ is optionally substituted by p substituents independently selected from $R_z$;

$R_x$, $R_y$, and $R_z$ are selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, CN, and $NO_2$;
Link is a group linking ring A and ring D;
p is an integer selected from 0, 1, and 2.

In some preferred embodiments of the present disclosure, certain groups in the compound of formula (I) or (II) or the pharmaceutically acceptable salt thereof are defined as follows, and unmentioned groups are as described in any one of the embodiments of the present disclosure (referred to as "in some embodiments of the present disclosure"), and $R_x$ is selected from hydrogen, halogen, $C_{1-6}$ alkyl, and $C_{1-6}$ haloalkyl.

In some embodiments of the present disclosure, $R_x$ is selected from H, F, Cl, Br, $CH_3$, and $CF_3$.

In some embodiments of the present disclosure, $R_x$ is selected from $NH_2$, $-CHF_2$, $C_2H_5$, CN,

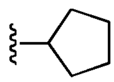, 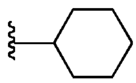, 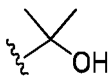, 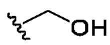, 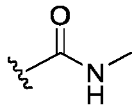, 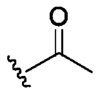, and 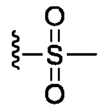.

In some embodiments of the present disclosure, $R_x$ is selected from H, F, $CH_3$, and $CF_3$.

In some embodiments of the present disclosure, $R_z$ is selected from hydrogen, halogen, $C_{1-6}$ alkyl, $C_{1-6}$ haloalkyl, CN, $NH_2$, $C_{3-7}$ cycloalkyl, hydroxyl-$C_{1-6}$ alkyl-, $C_{1-6}$ alkyl-C(O)-, $C_{1-6}$ alkyl-S(O)$_2$-, and $C_{1-6}$ alkyl-NH-C(O)-.

In some embodiments of the present disclosure, $R_z$ is selected from H, F, Cl, Br, $CH_3$, and $CF_3$.

In some embodiments of the present disclosure, $R_z$ is selected from $NH_2$, $-CHF_2$, $C_2H_5$, CN,

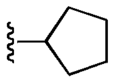, 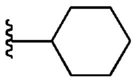, 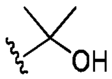, 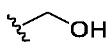, 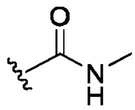, 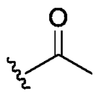, and 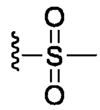.

In some embodiments of the present disclosure, $R_z$ is selected from

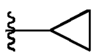.

In some embodiments of the present disclosure, $R_z$ is selected from H, F, Cl, Br, $CH_3$, $CF_3$, $NH_2$, $-CHF_2$, CN,

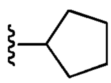, 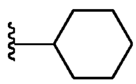, 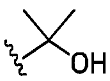, 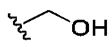, 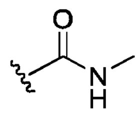, 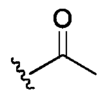,

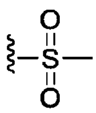, and 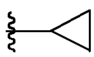.

In some embodiments of the present disclosure, $R_y$ is selected from H, F, Cl, Br, $CH_3$, and $CF_3$.

In some embodiments of the present disclosure, $R_y$ is selected from $-CH_2CH_3$, - $CH_2OH$, $-CH_2CN$, and $-CH_2OCH_3$.

In some embodiments of the present disclosure, $R_y$ is selected from H, $CH_3$, $-CH_2CH_3$, $-CH_2OH$, $-CH_2CN$, and $-CH_2OCH_3$.

In some embodiments of the present disclosure, Z is selected from $Cy^2$, $-NH-Cy^2$, and $C_{3-7}$ cycloalkylacyl; wherein $Cy^2$ is selected from $C_{6-10}$ aryl and 5- to 10-membered heteroaryl, and each of the $Cy^2$ is optionally substituted by p substituents independently selected from $R_z$.

In some embodiments of the present disclosure, Z is selected from

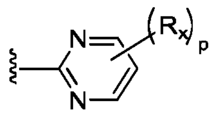

and

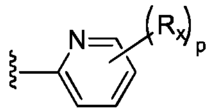

;

wherein p and $R_x$ are as defined above.

In some embodiments of the present disclosure, Z is selected from

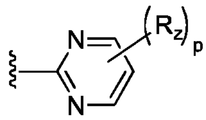

and

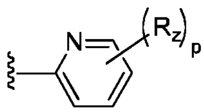

;

wherein p and $R_z$ are as defined above.

In some embodiments of the present disclosure, Z is selected from

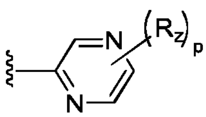

,

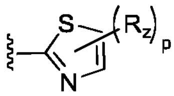

,

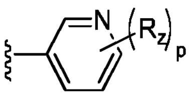

, and

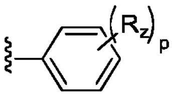

;

or Z is selected from

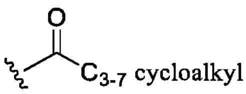

;

wherein p and $R_z$ are as defined above.

In some embodiments of the present disclosure, Z is selected from

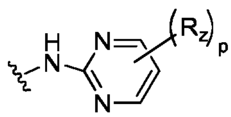

;

wherein p and $R_z$ are as defined above.

In some embodiments of the present disclosure, the

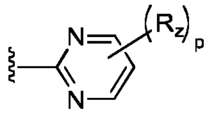

is preferably selected from

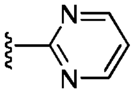, 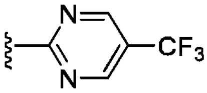, 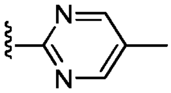, 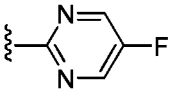, 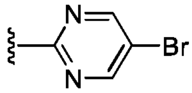,

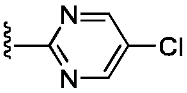, 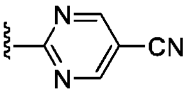, 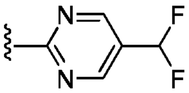, 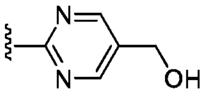, 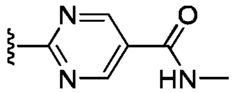,

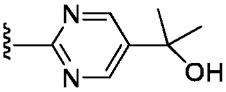, 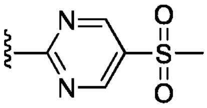, 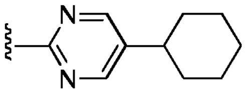, 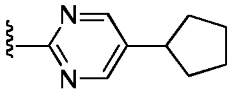,

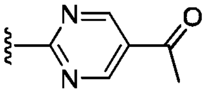, and 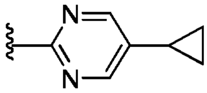.

In some embodiments of the present disclosure, the

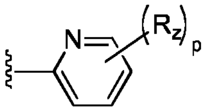

is preferably selected from

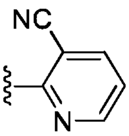, 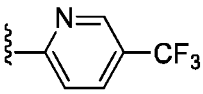, 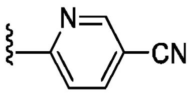, 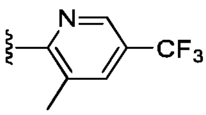,

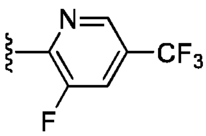, and 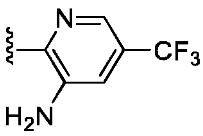.

In some embodiments of the present disclosure, Z may also be selected from

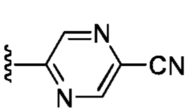, 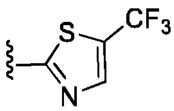, 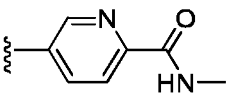, 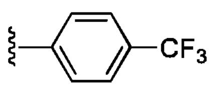, 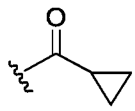,

and

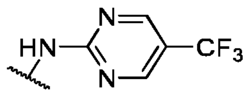.

In some embodiments of the present disclosure, ring D is selected from

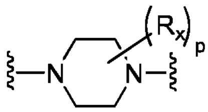;

wherein p and $R_x$ are as defined above.

In some embodiments of the present disclosure, ring D is selected from

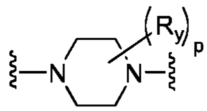;

wherein p and $R_y$ are as defined above.

In some embodiments of the present disclosure, ring D is selected from

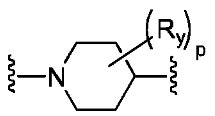, 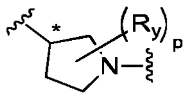, 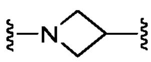, 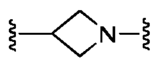, 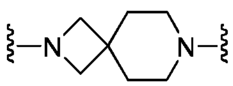,

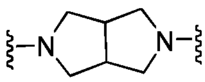, 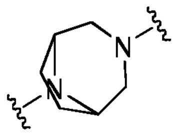, and 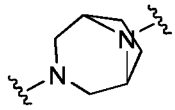;

wherein p and $R_y$ are as defined above.

In some embodiments of the present disclosure, ring D is selected from

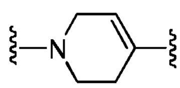.

In some embodiments of the present disclosure, the

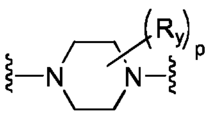

is preferably selected from

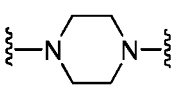, 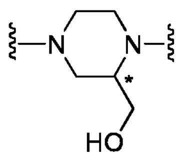, 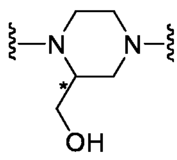, 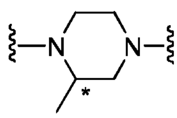, 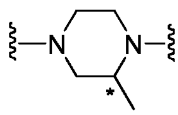,

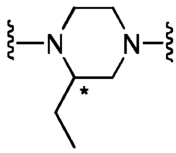, 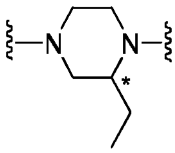, 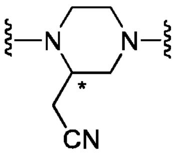, 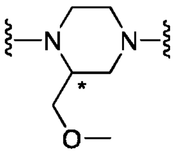, 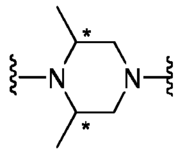, 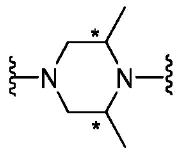,

and

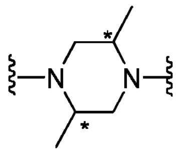;

the carbon atom marked with "*" in the structure represents a chiral carbon atom.

In some embodiments of the present disclosure, ring D may also be selected from

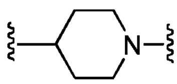, 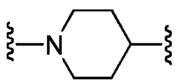, 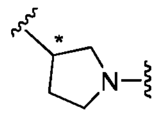, 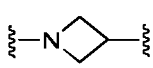, 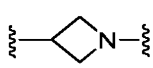, 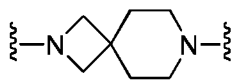,

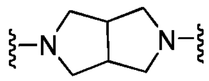, 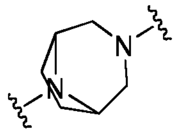, 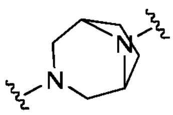, 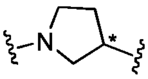, and 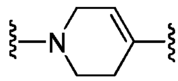;

the carbon atom marked with "*" in the structure represents a chiral carbon atom.

In some embodiments of the present disclosure, the formula (A-1) is selected from the following groups:

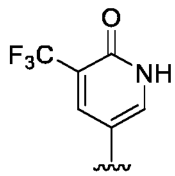 and 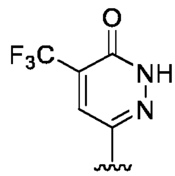.

In some embodiments of the present disclosure, the formula (A-1) is selected from the following groups:

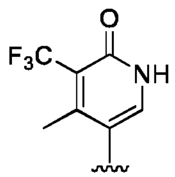, 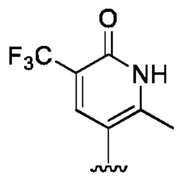, and 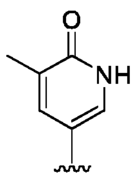.

In some embodiments of the present disclosure, the formula (A-2) is selected from the following groups:

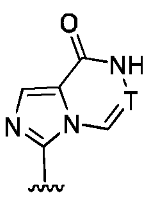, 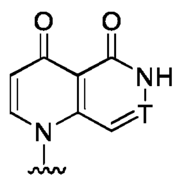, 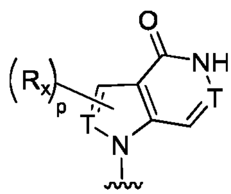, and 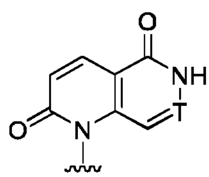;

wherein p, T, and $R_x$ are as defined above.

In some embodiments of the present disclosure, the formula (A-2) may also be selected from the following groups:

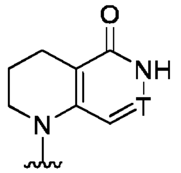 and 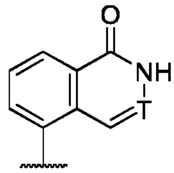 ;

wherein T is as defined above.

In some embodiments of the present disclosure, the formula (A-2) is further selected from the following groups:

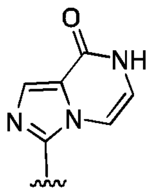 , 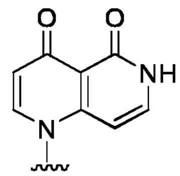 , 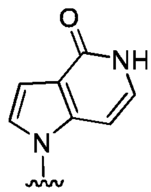 , 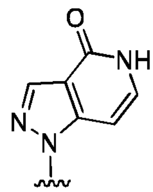 , 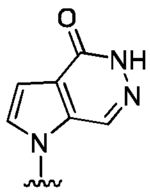 , 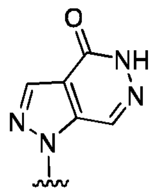 ,

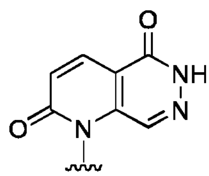 , 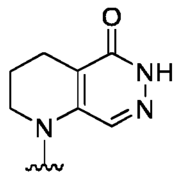 , and 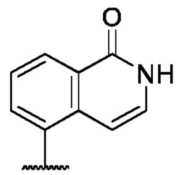 .

In some embodiments of the present disclosure, the formula (A-2) is selected from the following groups:

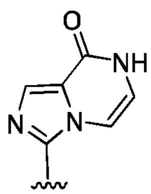 , 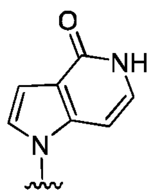 , 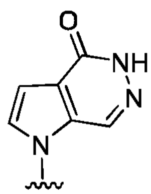 , 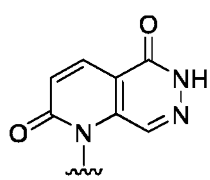 , 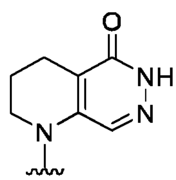 , and 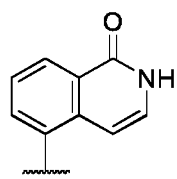 .

In some embodiments of the present disclosure, the formula (A-3) is selected from

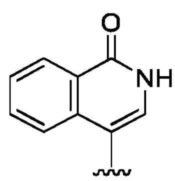 .

In some embodiments of the present disclosure, the Link group has the following group of formula (LA):

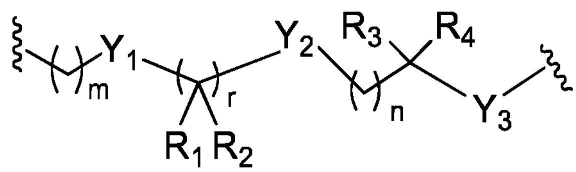

(LA)

wherein

$R_1$ and $R_2$ are each independently selected from H, $C_{1-3}$ alkyl, and hydroxy-$C_{1-3}$ alkyl, or $R_1$ and $R_2$ together with the carbon atom where they are located form a 3- to 6-membered carbocyclic ring, or $R_1$ and $R_2$ together form an oxo group;
$R_3$ and $R_4$ are each independently selected from H, $C_{1-3}$ alkyl, and hydroxy-$C_{1-3}$ alkyl, or $R_3$ and $R_4$ together form an oxo group;
$Y_1$ and $Y_2$ are selected from NH, $-N(CH_3)-$, O, $-CH_2NH-$, $-CH_2O-$, $Cy^1$, or a single bond;
$Cy^1$ is selected from $C_{6-10}$ aryl, $C_{3-7}$ cycloalkyl, 5- to 10-membered heteroaryl, and 4-to 10-membered heterocyclyl, and the groups are each optionally substituted by p substituents independently selected from $R_x$;
$Y_3$ is selected from NH, O, or a single bond;
m is an integer selected from 0, 1, and 2;
n is an integer selected from 0, 1, 2, and 3;
r is an integer selected from 0 and 1;
wherein p and $R_x$ are as defined above.

In some embodiments of the present disclosure, the Link group has the following group of formula (LA);

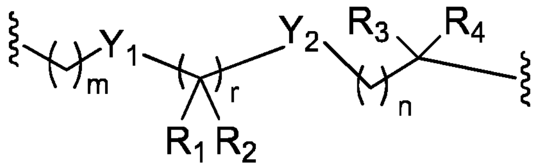

(LA)

wherein

$R_1$ and $R_2$ are each independently selected from Hand $C_{1-3}$ alkyl, or $R_1$ and $R_2$ together with the carbon atom where they are located form a 3- to 6-membered carbocyclic ring, or $R_1$ and $R_2$ together form an oxo group;
$R_3$ and $R_4$ are selected from Hand $C_{1-3}$ alkyl, or $R_3$ and $R_4$ together form an oxo group;
$Y_1$ and $Y_2$ are selected from $Y_{1a}$, $-CH_2Y_{1a}-$, $Cy^1$, or a single bond;
$Y_{1a}$ is selected from NH and O;
$Cy^1$ is selected from $C_{6-10}$ aryl, $C_{3-7}$ cycloalkyl, 5- to 10-membered heteroaryl, and 4-to 10-membered heterocyclyl, and the groups are each optionally substituted by p substituents independently selected from $R_x$;
m is an integer selected from 0, 1, and 2;
n is an integer selected from 0, 1, 2, and 3;
r is an integer selected from 0 and 1;
p and $R_x$ are as defined above.

In some embodiments of the present disclosure, the $Cy^1$ is selected from

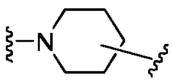, 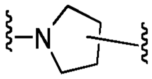, 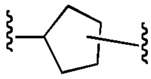, and 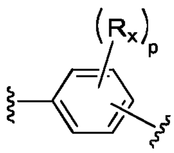;

wherein p and $R_x$ are as defined above.
In some embodiments of the present disclosure, the $Cy^1$ is selected from

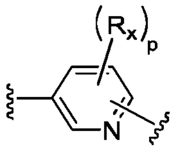 and 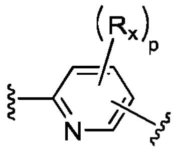;

wherein p and $R_x$ are as defined above.
In some embodiments of the present disclosure, the formula (LA) has the following structures:

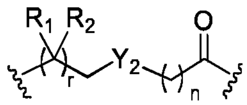 (LA-1), 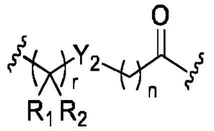 (LA-2), 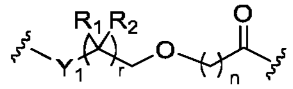 (LA-3),

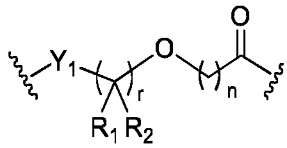 (LA-4), 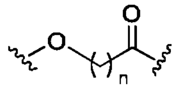 (LA-5), 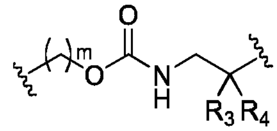 (LA-6),

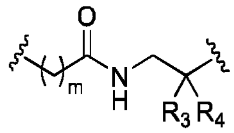 (LA-7), 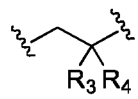 (LA-8), 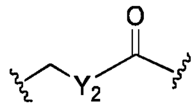 (LA-9),

or

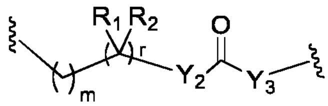 (LA-10);

wherein $R_1$, $R_2$, $R_3$, $R_4$, $Y_1$, $Y_2$, $Y_3$, m, n, and r are as defined above.

In some embodiments of the present disclosure, the formula (LA) has the following structures;

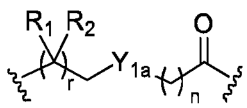 (LA-1), 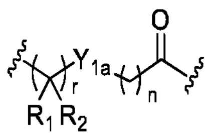 (LA-2), 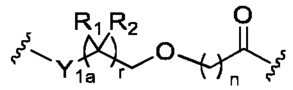 (LA-3),

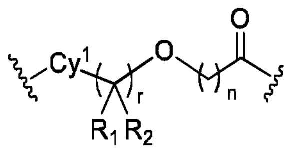 (LA-4), 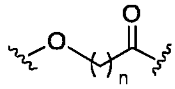 (LA-5), 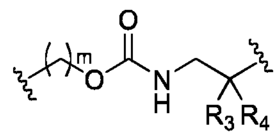 (LA-6),

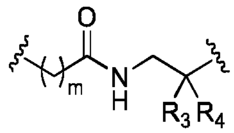 (LA-7), 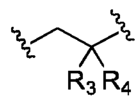 (LA-8), 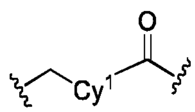 (LA-9),

or

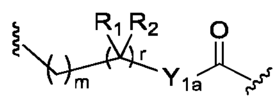 (LA-10);

wherein $R_1$, $R_2$, $R_3$, $R_4$, $Y_{1a}$, $Cy^1$, m, n, and r are as defined above.

In some embodiments of the present disclosure, the formula (LA) is further selected from the following structures:

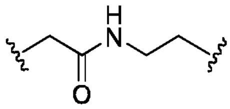, 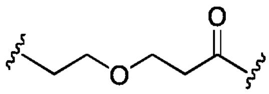, 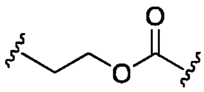, 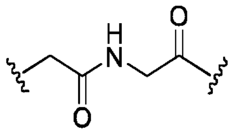,

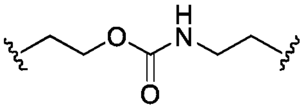, 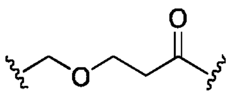, 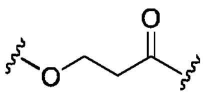, 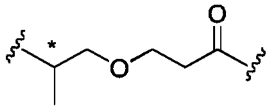,

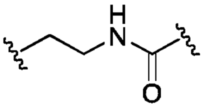, 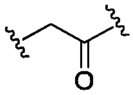, 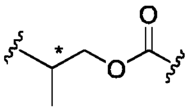, 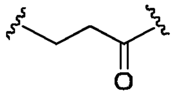, 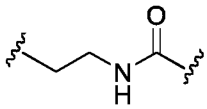,

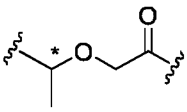, 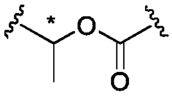, 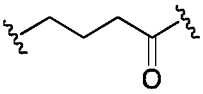, 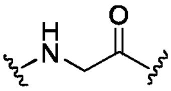, 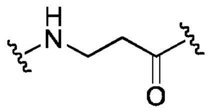,

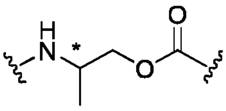, 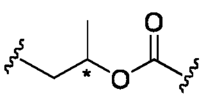, 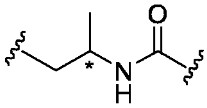, and 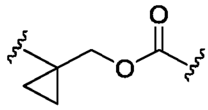;

the carbon atom marked with "*" in the structure represents a chiral carbon atom.

In some embodiments of the present disclosure, the formula (LA) is further selected from the following structures:

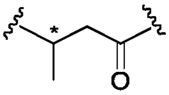, 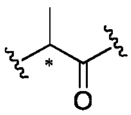, 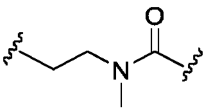, 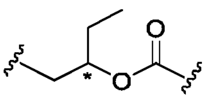,

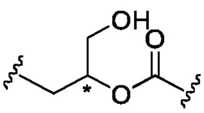, and 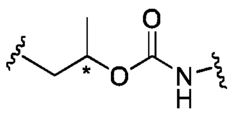;

the carbon atom marked with "*" in the structure represents a chiral carbon atom.

In some embodiments of the present disclosure, the formula (LA) may also be selected from the following structures:

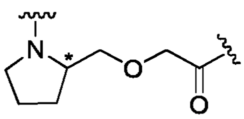, 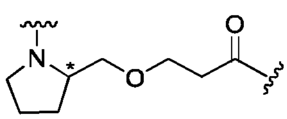, 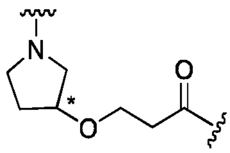, 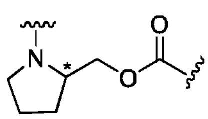,

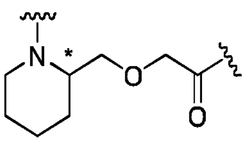, 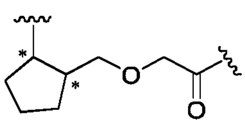, 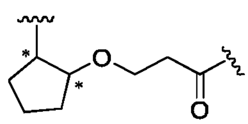, and 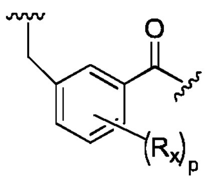;

the carbon atom marked with "*" in the structure represents a chiral carbon atom; p and $R_x$ are as defined above.

In some embodiments of the present disclosure, the formula (LA) may also be selected from the following structures:

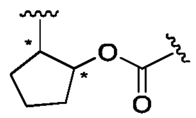,
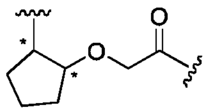,
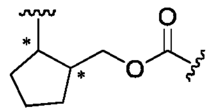,
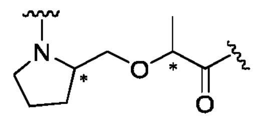,

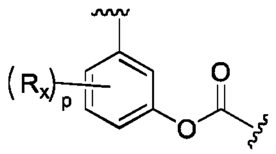,
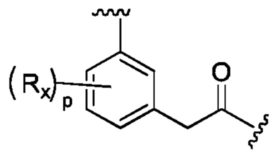,
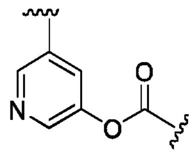,
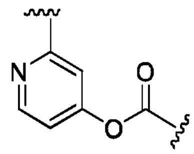,

and

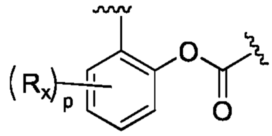;

the carbon atom marked with "*" in the structure represents a chiral carbon atom; p and $R_x$ are as defined above.

In some embodiments of the present disclosure, the compound of formula (I) or the pharmaceutically acceptable salt thereof has a structure of formula (I-1)

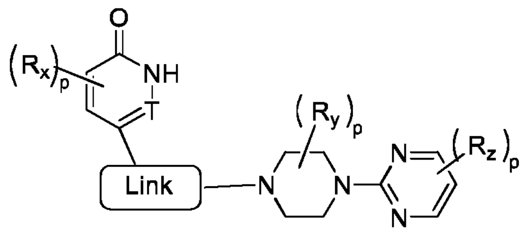

(I-1).

In some embodiments of the present disclosure, the compound of formula (I) or the pharmaceutically acceptable salt thereof has a structure of formula (I-1a)

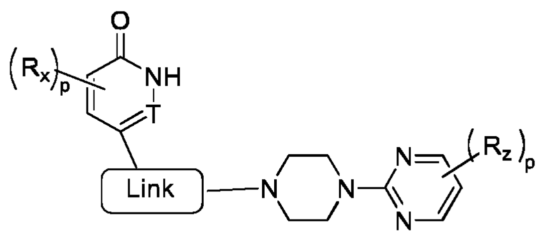

(I-1a).

In some embodiments of the present disclosure, the compound of formula (I) or the pharmaceutically acceptable salt thereof has a structure of formula (I-1b)

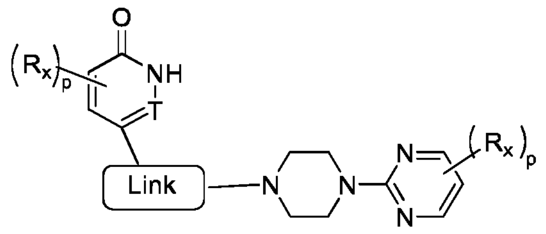

(I-1b).

In some embodiments of the present disclosure, the compound of formula (I) or the pharmaceutically acceptable salt thereof has a structure of formula (I-2)

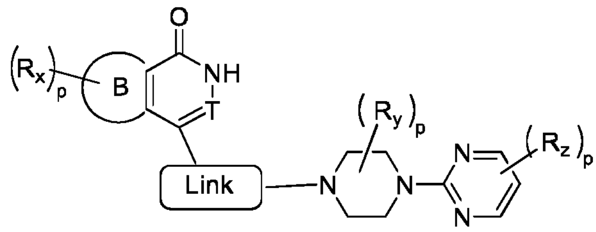

(I-2).

In some embodiments of the present disclosure, the compound of formula (I) or the pharmaceutically acceptable salt thereof has a structure of formula (I-2a)

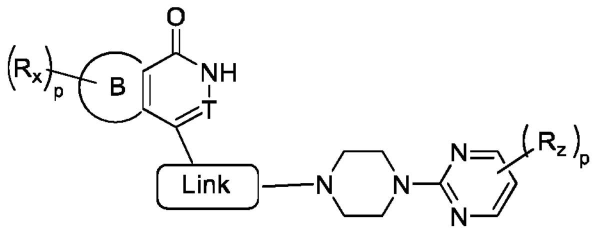

(I-2a).

In some embodiments of the present disclosure, the compound of formula (I) or the pharmaceutically acceptable salt thereof has a structure of formula (I-2b)

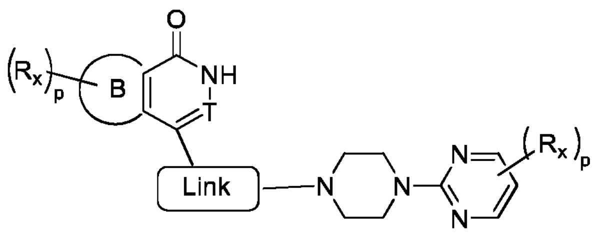

(I-2b).

In some embodiments of the present disclosure, the compound of formula (I) or the pharmaceutically acceptable salt thereof has a structure of formula (I-3)

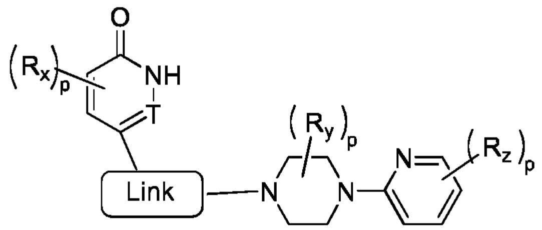

(I-3).

In some embodiments of the present disclosure, the compound of formula (I) or the pharmaceutically acceptable

salt thereof has a structure of formula (I-3a)

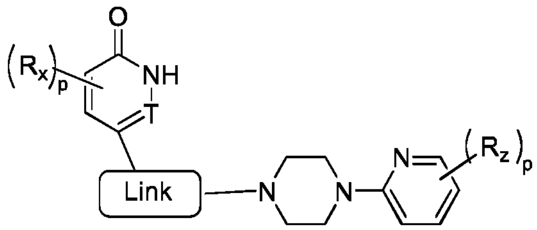

(I-3a).

In some embodiments of the present disclosure, the compound of formula (I) or the pharmaceutically acceptable salt thereof has a structure of formula (I-3b)

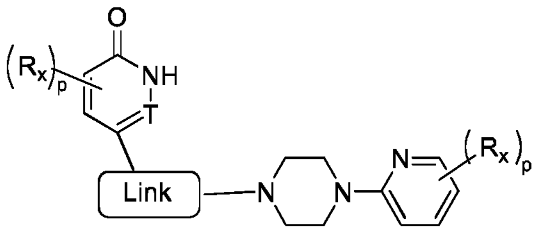

(I-3b).

In some embodiments of the present disclosure, the compound of formula (I) or the pharmaceutically acceptable salt thereof has a structure of formula (I-4)

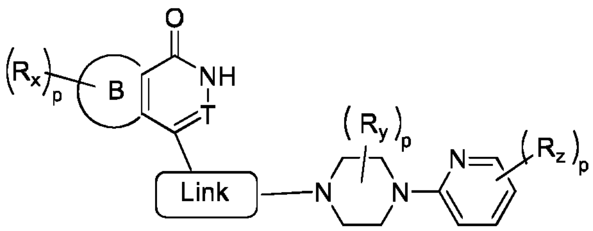

(I-4).

In some embodiments of the present disclosure, the compound of formula (I) or the pharmaceutically acceptable salt thereof has a structure of formula (I-4a)

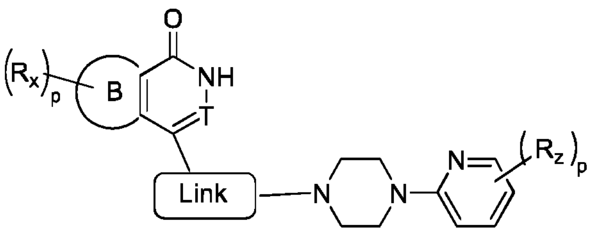

(I-4a).

In some embodiments of the present disclosure, the compound of formula (I) or the pharmaceutically acceptable salt thereof has a structure of formula (I-4b)

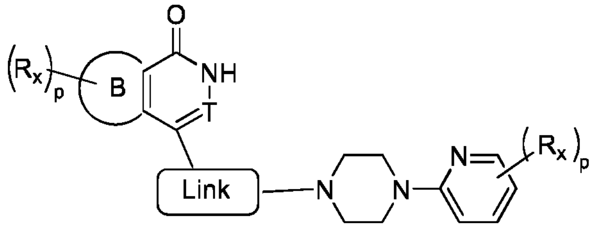

(I-4b).

In some embodiments of the present disclosure, in ring B, the $C_{6-10}$ aryl is phenyl or naphthyl, preferably phenyl.

In some embodiments of the present disclosure, in ring B, the 5- to 10-membered heteroaryl is 5-membered or 6-membered heteroaryl containing a carbon atom and 1, 2, 3, or 4 cycloheteroatoms independently selected from N, O, and S; preferably 5-membered or 6-membered heteroaryl containing a carbon atom and 1 or 2 N cycloheteroatoms.

In some embodiments of the present disclosure, in ring B, the 4- to 10-membered heterocyclyl is a 5-membered or 6-membered monocyclic, saturated or unsaturated cyclic group containing a carbon atom and 1, 2, 3, or 4 cycloheteroatoms independently selected from N, O, and S; preferably a 5-membered or 6-membered monocyclic, saturated or unsaturated cyclic group containing a carbon atom and 1 or 2 N cycloheteroatoms.

In some embodiments of the present disclosure, in ring D, the 4- to 10-membered heterocyclyl is a 4-membered monocyclic, 5-membered monocyclic, 6-membered monocyclic, 8-membered bicyclic, or 9-membered bicyclic, saturated or unsaturated cyclic group containing a carbon atom and 1, 2, 3, or 4 cycloheteroatoms independently selected from N, O, and S; preferably a 4-membered monocyclic, 5-membered monocyclic, 6-membered monocyclic, 8-membered bicyclic, or 9-membered bicyclic, saturated or unsaturated cyclic group containing a carbon atom and 1 or 2 N cycloheteroatoms.

In some embodiments of the present disclosure, in $Cy^2$, the $C_{6-10}$ aryl is phenyl or naphthyl, preferably phenyl.

In some embodiments of the present disclosure, in $Cy^2$, the 5- to 10-membered heteroaryl is 5-membered or 6-membered heteroaryl containing a carbon atom and 1, 2, 3, or 4 cycloheteroatoms independently selected from N, O, and S; preferably 5-membered or 6-membered heteroaryl containing a carbon atom and 1 or 2 cycloheteroatoms independently selected from N and S.

In some embodiments of the present disclosure, in $R_x$ and $R_z$, the halogen is independently fluorine, chlorine, bromine, or iodine, preferably fluorine, chlorine, or bromine.

In some embodiments of the present disclosure, in $R_x$ and $R_z$, in the $C_{1-6}$ alkyl, $C_{1-6}$ haloalkyl, hydroxyl-$C_{1-6}$ alkyl-, $C_{1-6}$ alkyl-C(O)-, $C_{1-6}$ alkyl-$S(O)_2$-, $C_{1-6}$ alkyl-NH-C(O)-, the $C_{1-6}$ alkyl is independently methyl, ethyl, *n*-propyl, isopropyl, *n*-butyl, isobutyl, *sec*-butyl, or *tert*-butyl, preferably methyl, ethyl, or isopropyl.

In some embodiments of the present disclosure, in $R_x$ and $R_z$, the $C_{3-7}$ cycloalkyl is cyclopropyl, cyclobutyl, cyclopentyl, or cyclohexyl.

In some embodiments of the present disclosure, in $R_y$, the $C_{1-6}$ alkyl is methyl, ethyl, *n*-propyl, isopropyl, *n*-butyl, isobutyl, *sec*-butyl, or *tert*-butyl, preferably methyl or ethyl.

In some embodiments of the present disclosure, in $Cy^1$, the $C_{6-10}$ aryl is phenyl or naphthyl, preferably phenyl.

In some embodiments of the present disclosure, in $Cy^1$, the $C_{3-7}$ cycloalkyl is cyclopropyl, cyclobutyl, cyclopentyl, or cyclohexyl, preferably cyclopentyl.

In some embodiments of the present disclosure, in $Cy^1$, the 5- to 10-membered heteroaryl is 5-membered or 6-membered heteroaryl containing a carbon atom and 1, 2, 3, or 4 cycloheteroatoms independently selected from N, O, and S; preferably 6-membered heteroaryl containing a carbon atom and 1 N cycloheteroatom.

In some embodiments of the present disclosure, in $Cy^1$, the 4- to 10-membered heterocyclyl is a 5-membered or 6-membered monocyclic, saturated or unsaturated cyclic group containing a carbon atom and 1, 2, 3, or 4 cycloheteroatoms independently selected from N, O, and S; preferably a 5-membered or 6-membered monocyclic, saturated cyclic group containing a carbon atom and 1 or 2 N cycloheteroatoms.

The present disclosure also provides a compound or a pharmaceutically acceptable salt thereof, wherein the compound is selected from

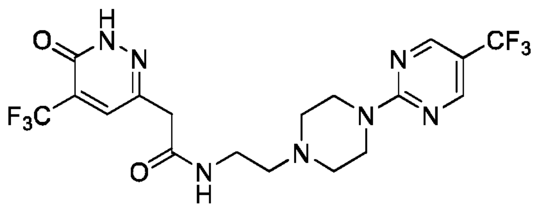,

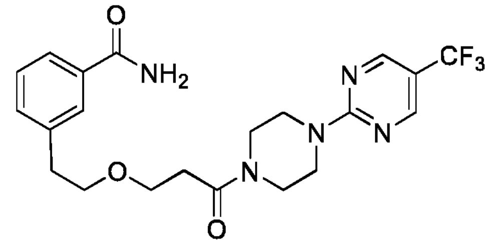,

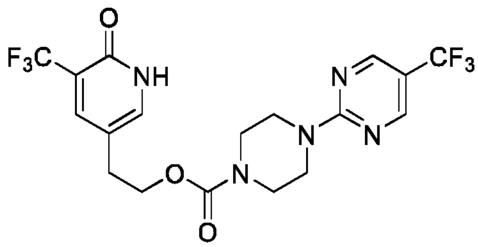,

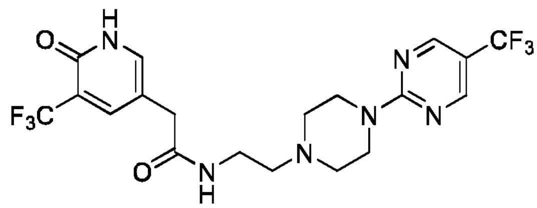,

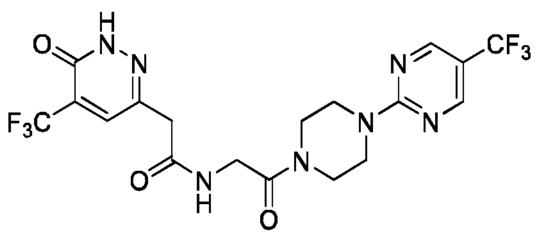,

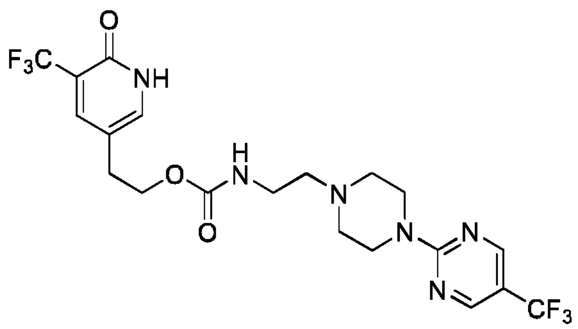,

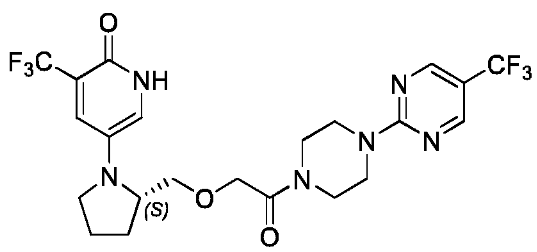,

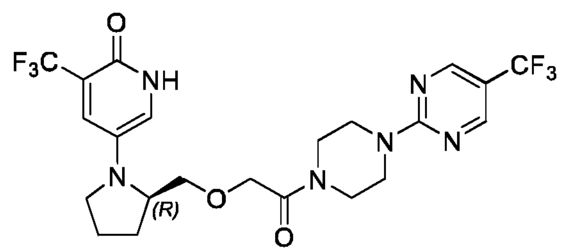,

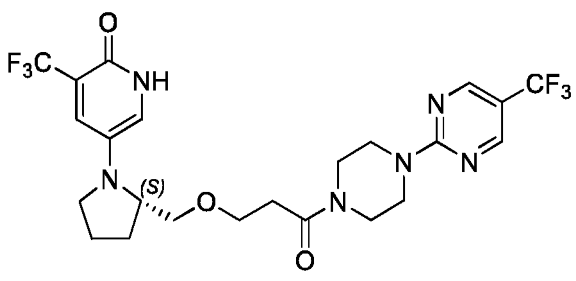,

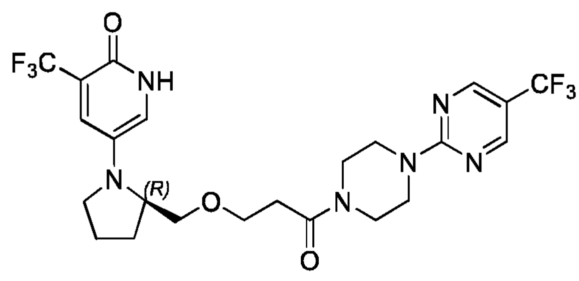,

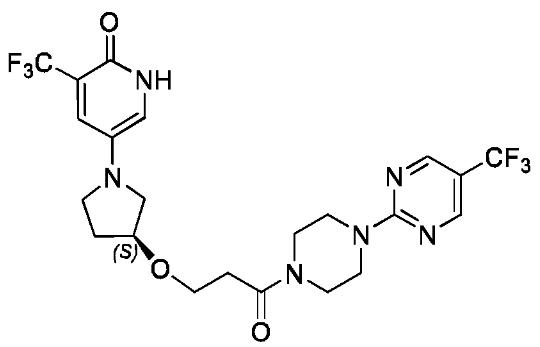,

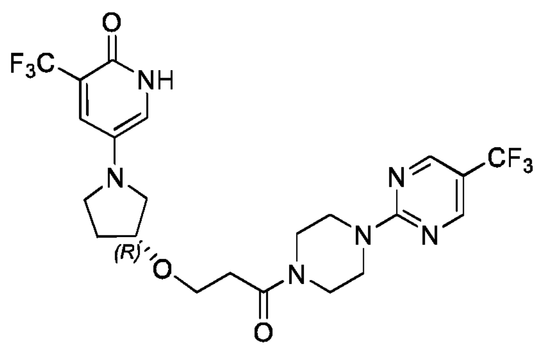,

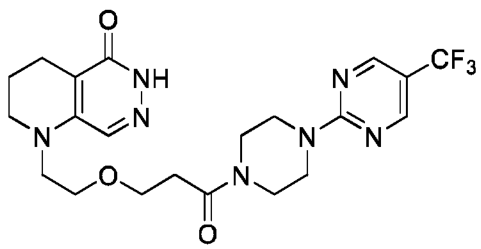,

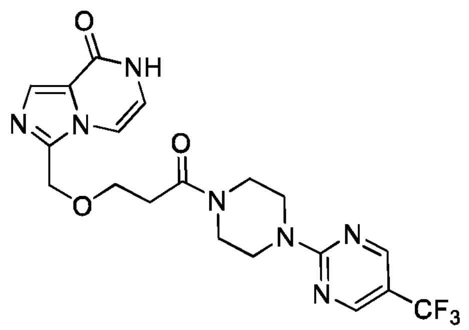,

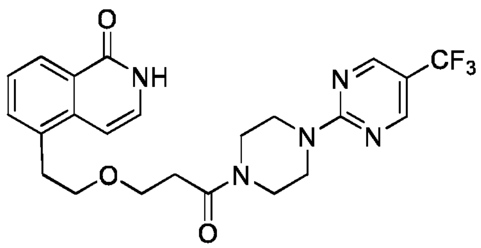,

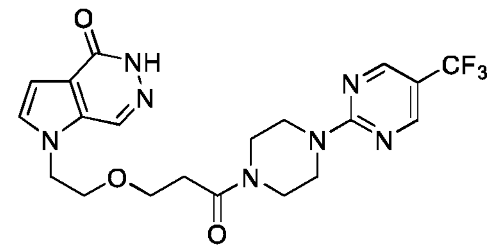,

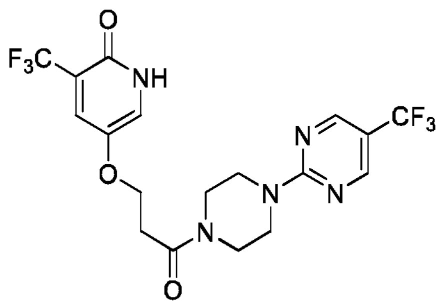,

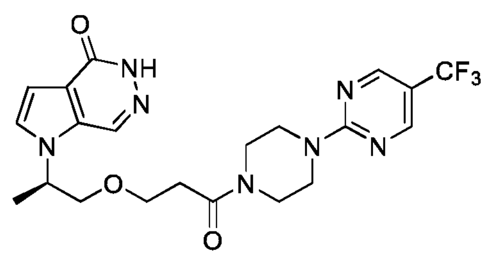,

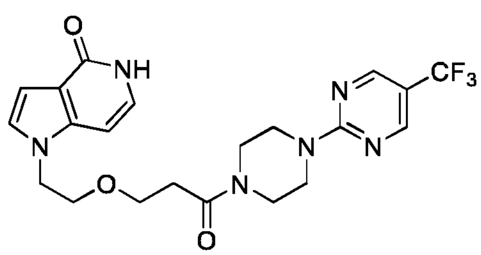,

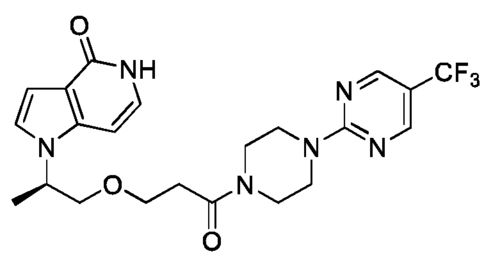,

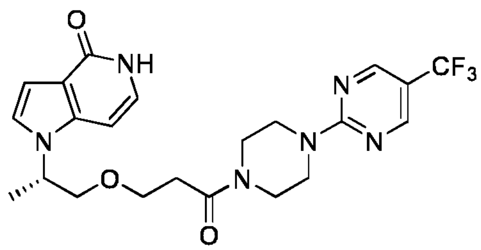

,

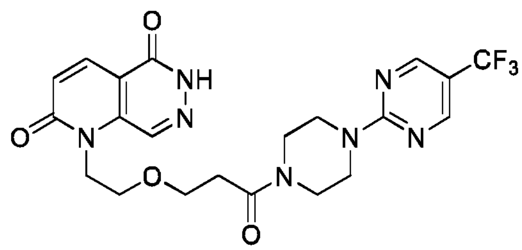

,

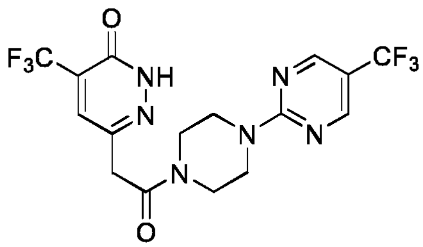

,

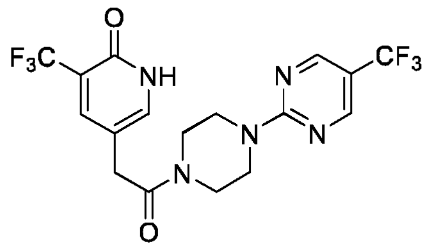

,

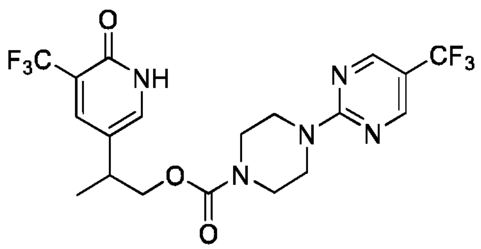

,

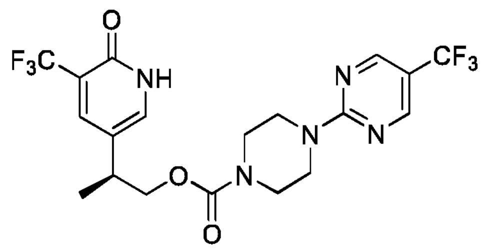

,

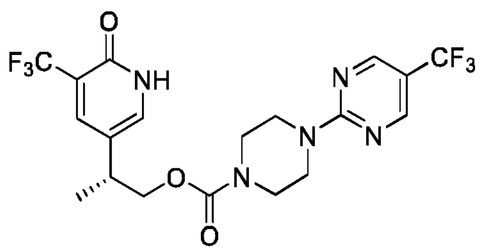

,

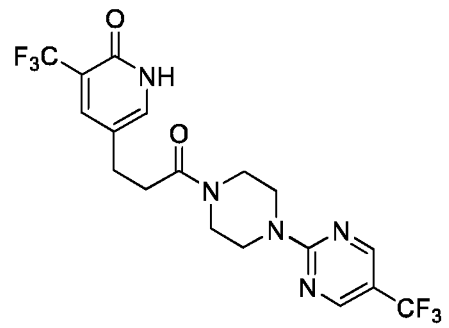

,

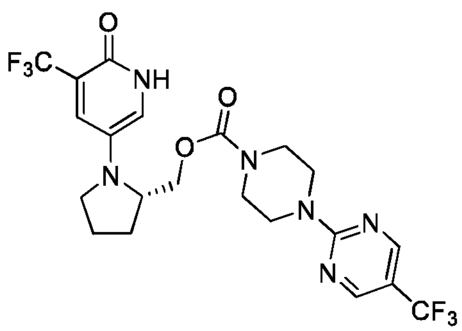

,

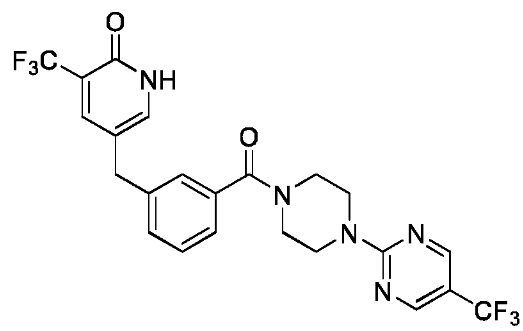

,

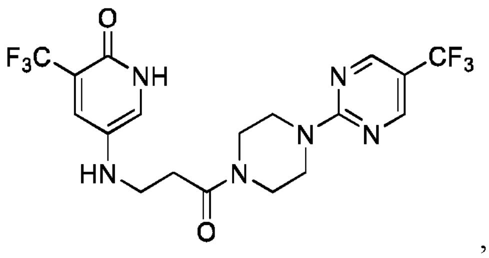

, and

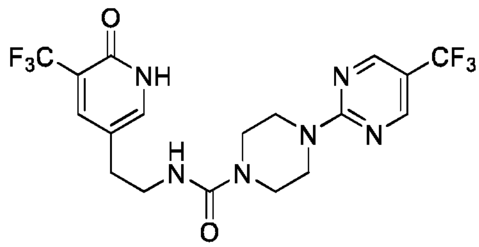

.

The present disclosure also provides the following compounds or pharmaceutically acceptable salts thereof, wherein the compounds are selected from

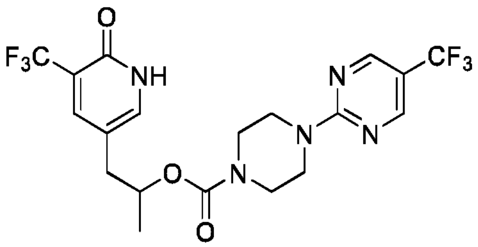

,

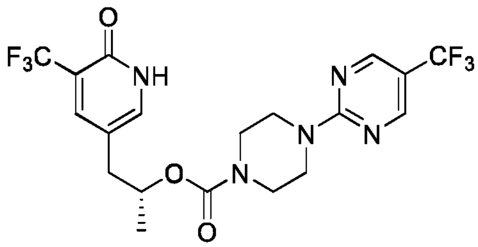

,

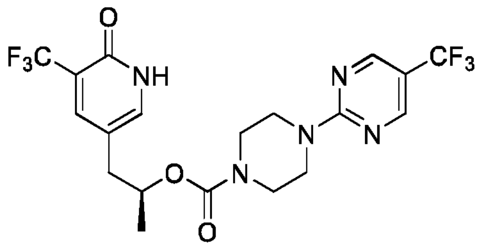

,

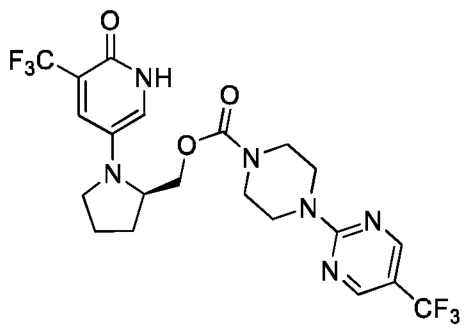

,

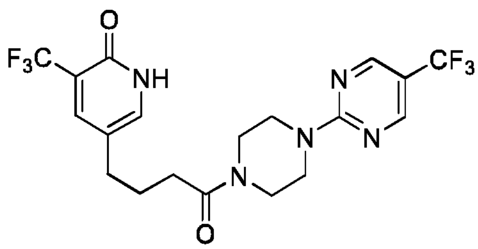

,

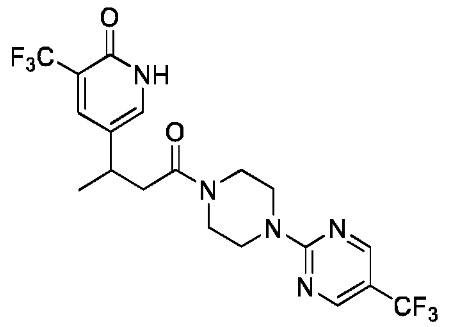

,

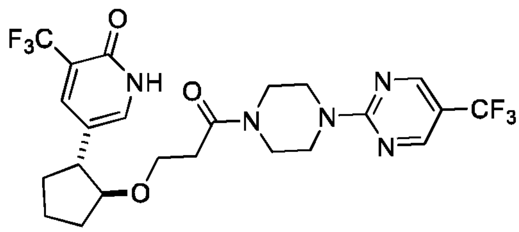

,

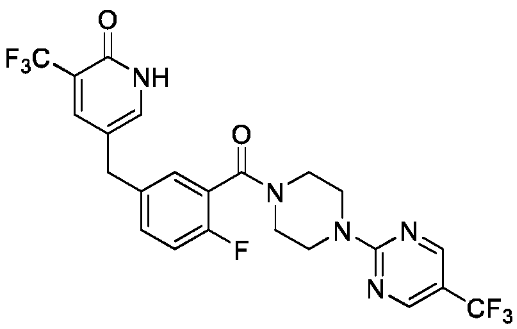

,

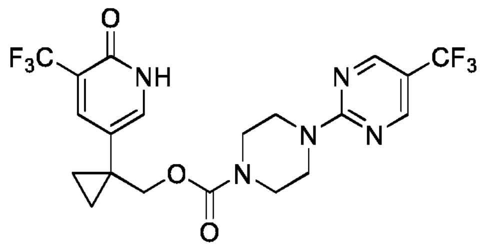
,
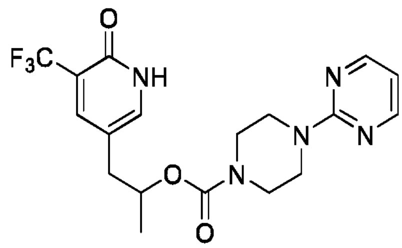
,
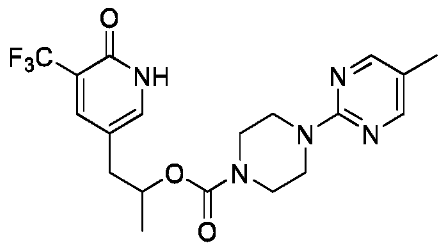
,
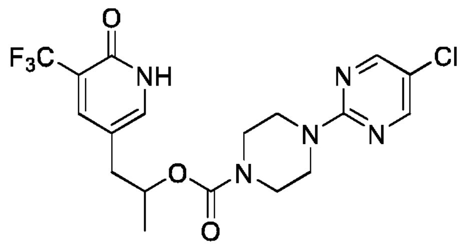
,
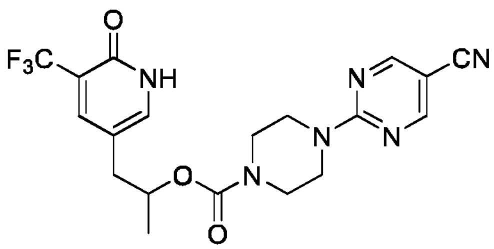
,
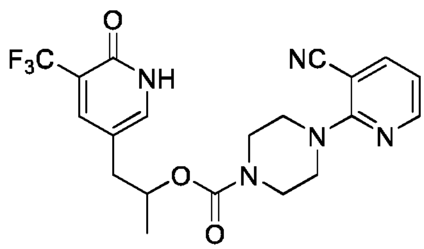
,
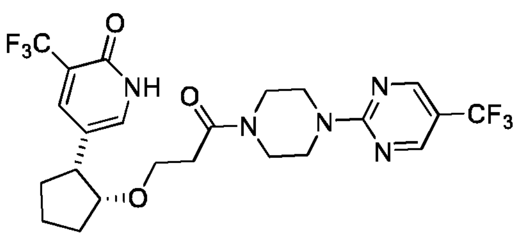
,
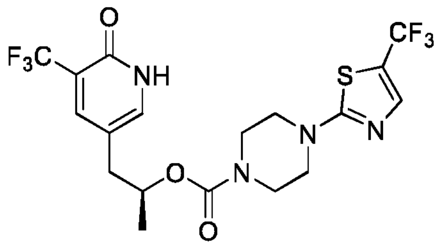
,
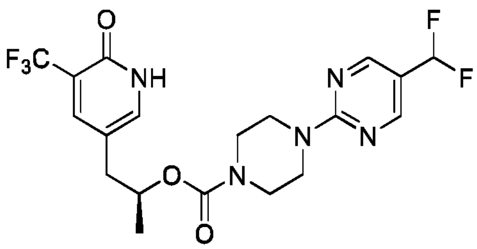
,
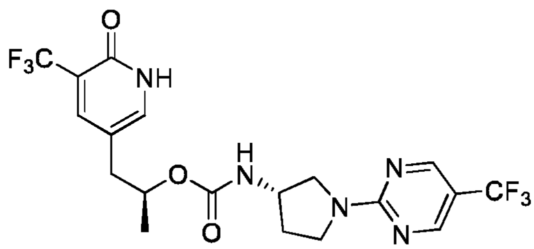
,

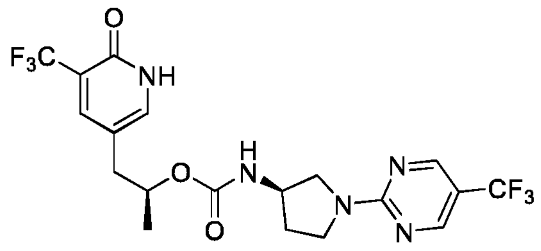

,

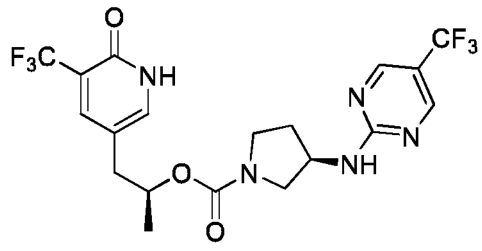

,

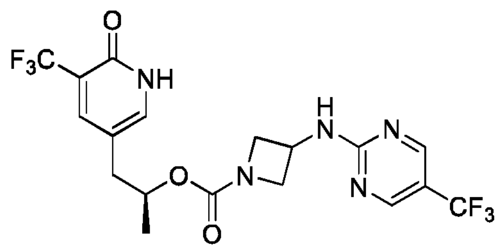

,

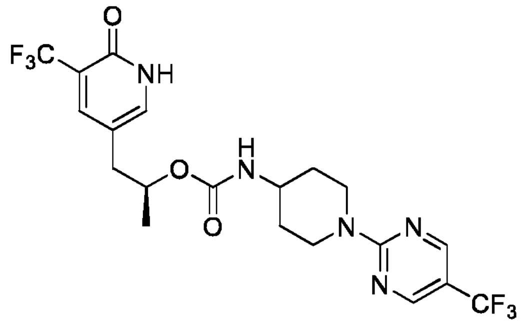

,

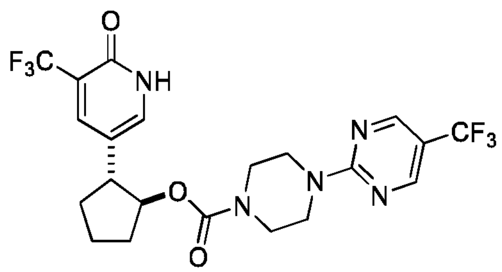

,

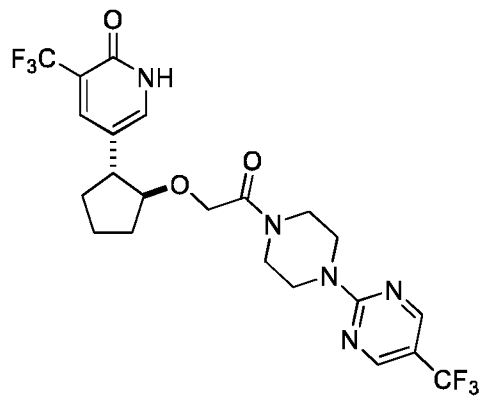

,

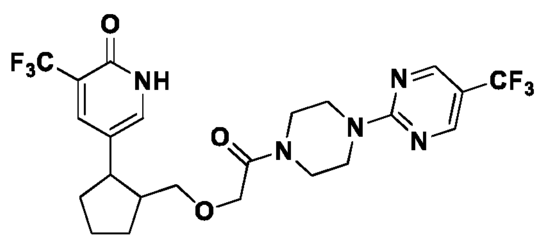

,

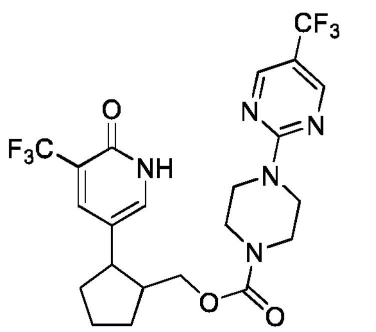

,

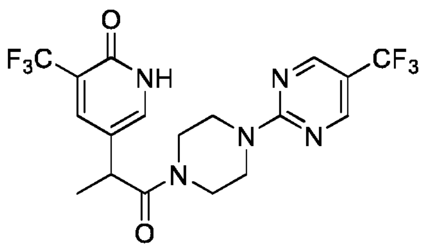
,
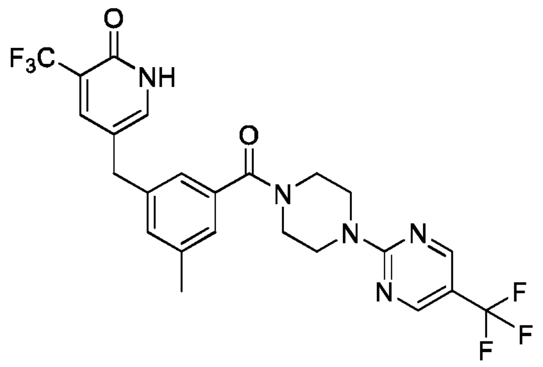
,
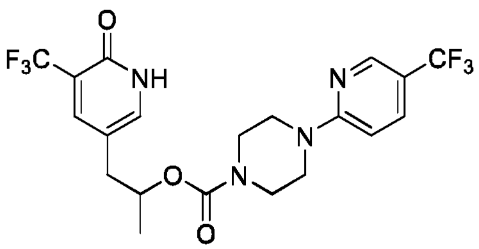
,
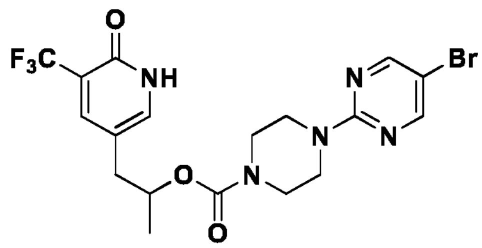
,
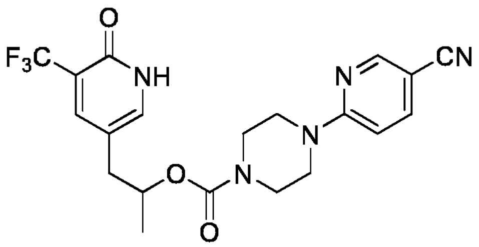
,
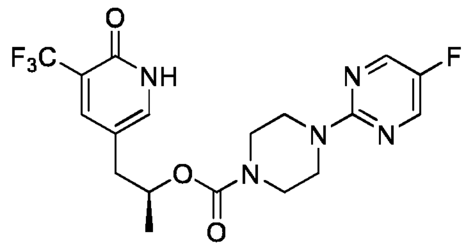
,
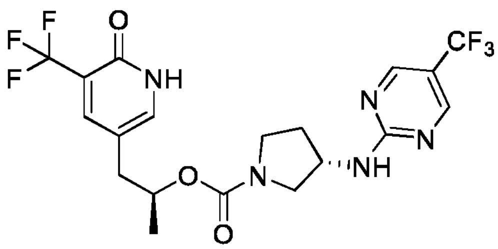
,
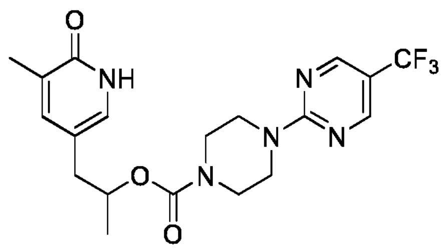
,
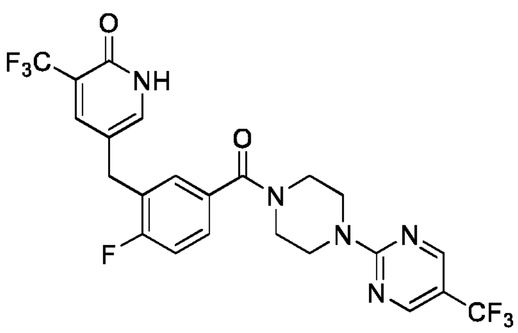
,
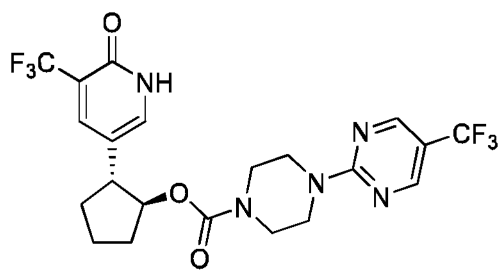
,

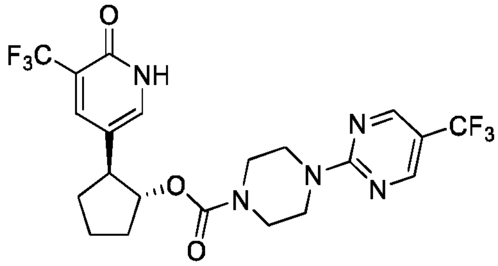
,
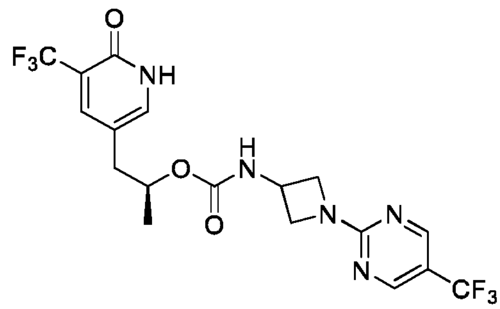
,
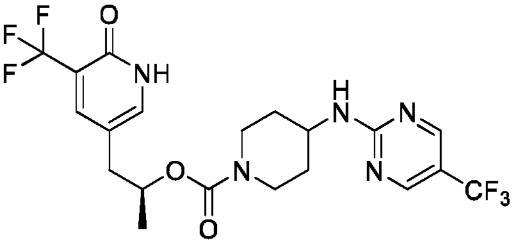
,
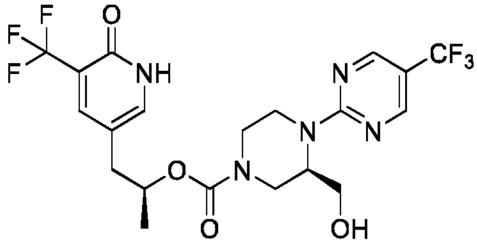
,
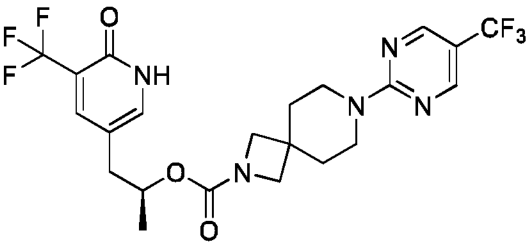
,
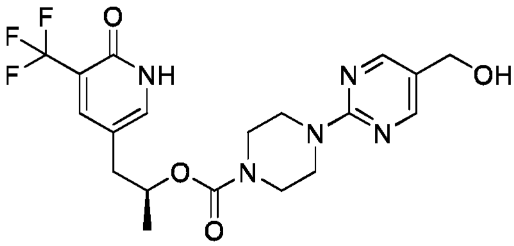
,
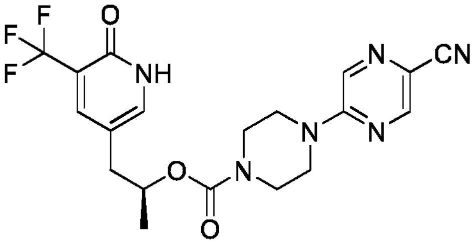
,
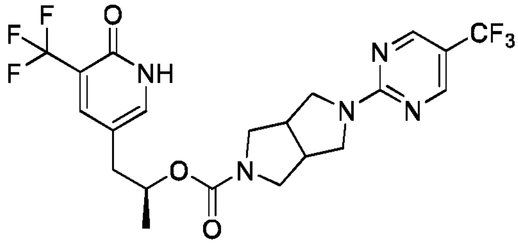
,
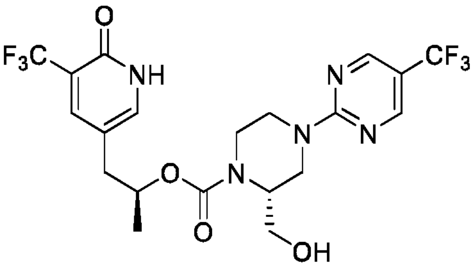
,
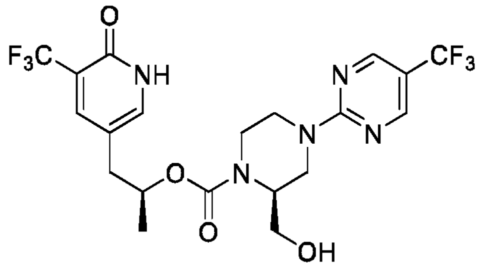
,

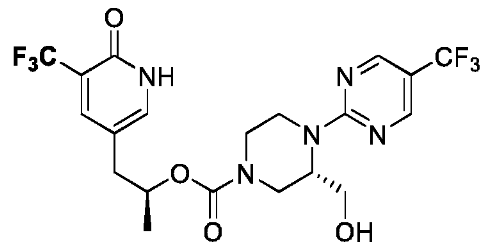
,
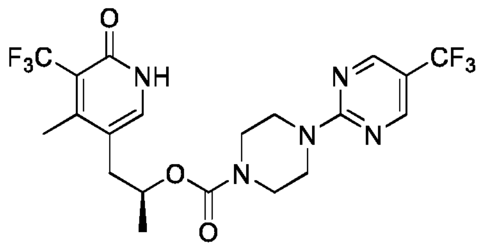
,
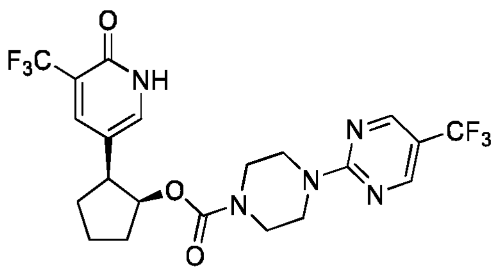
,
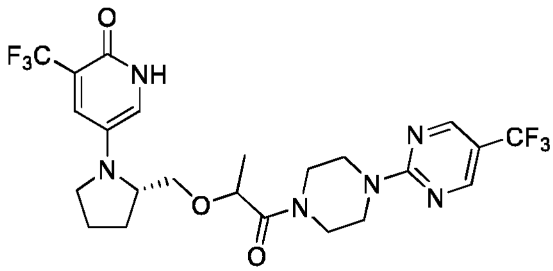
,
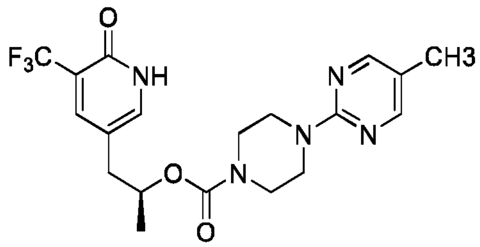
,
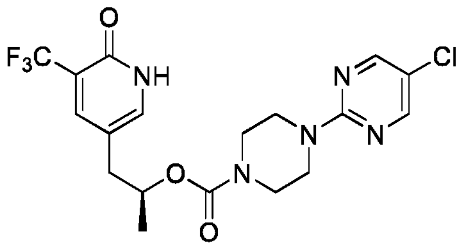
,
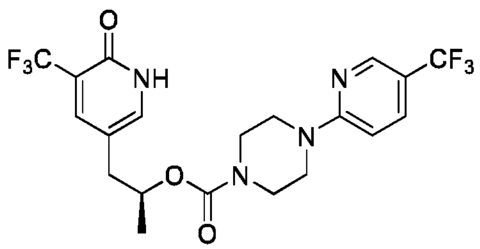
,
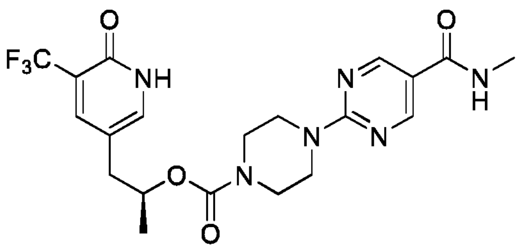
,
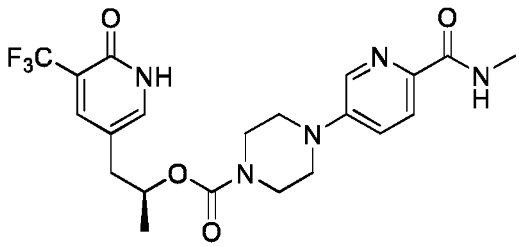
,
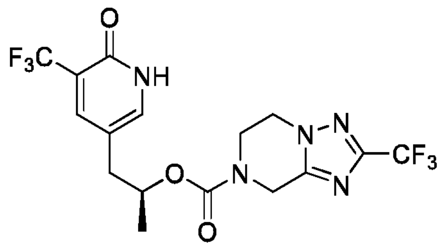
,

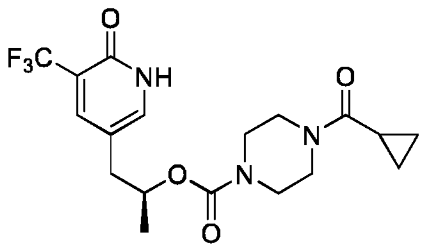

,

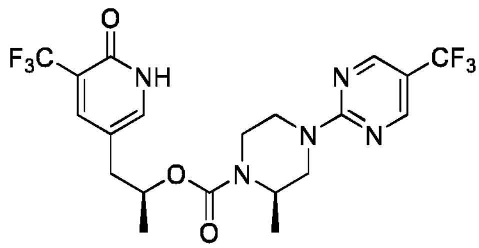

,

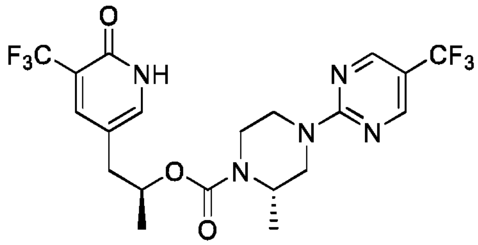

,

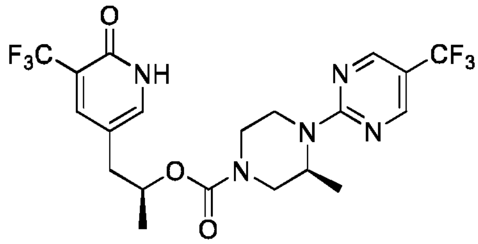

,

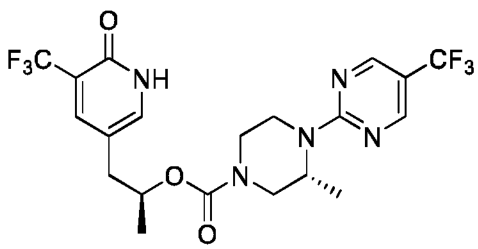

,

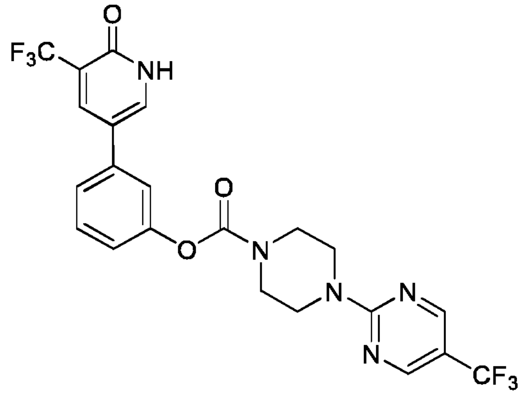

,

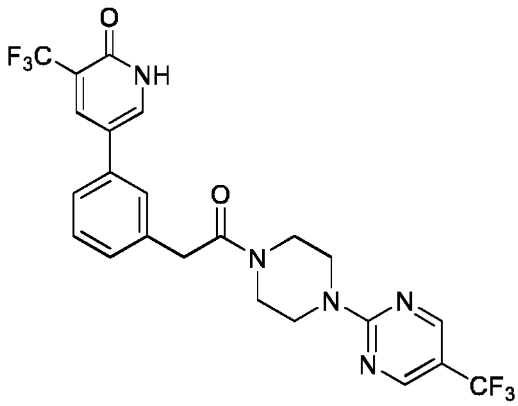

,

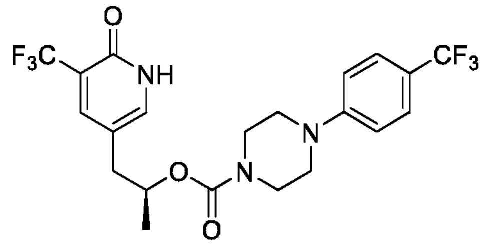

,

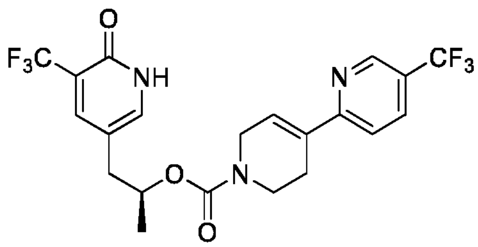

,

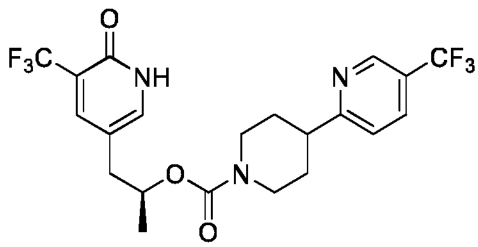

,

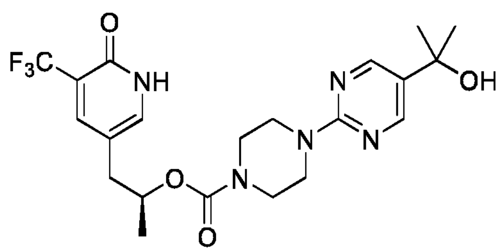

,

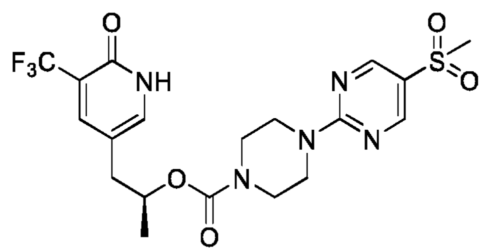

,

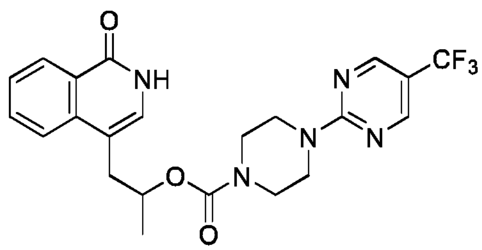

,

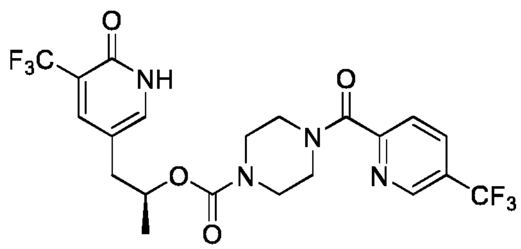

,

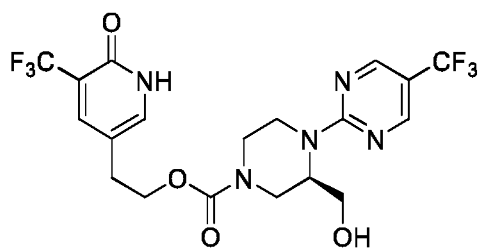

,

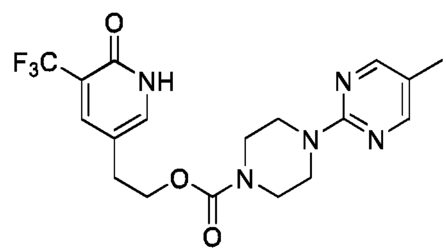

,

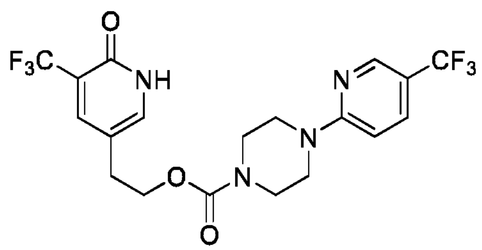

,

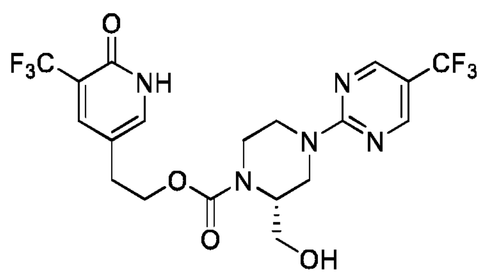

,

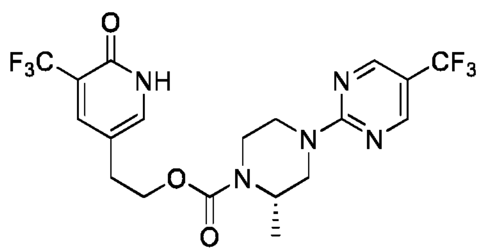

,

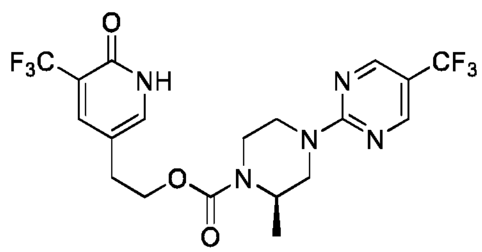

,

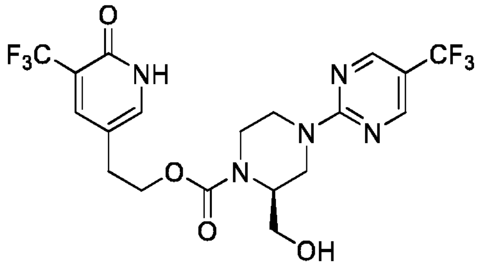
,

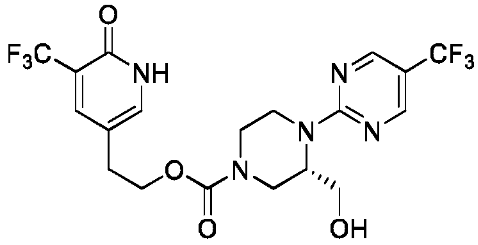
,

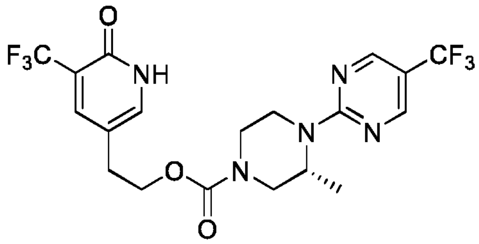
,

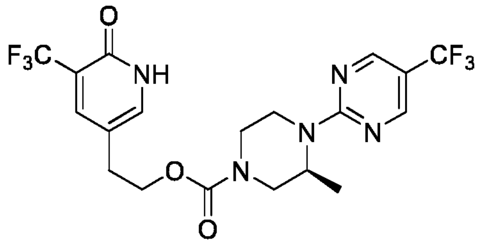
,

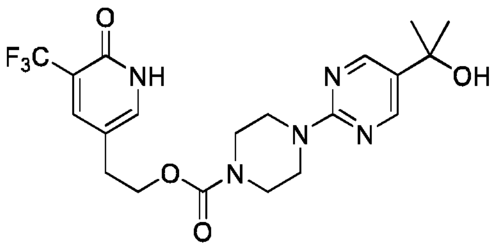
,

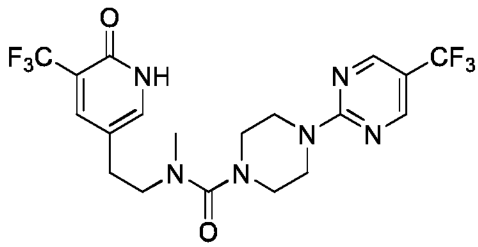
,

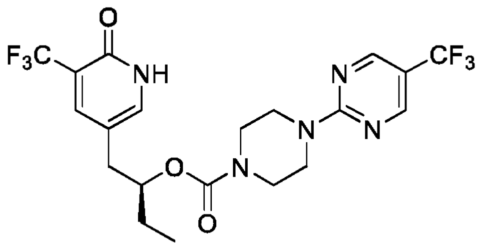
,

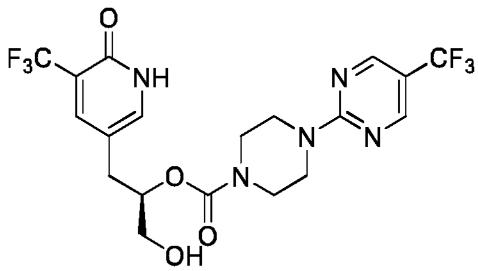
,

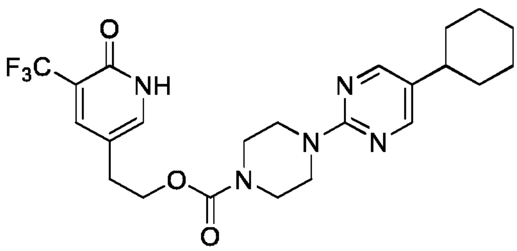
,

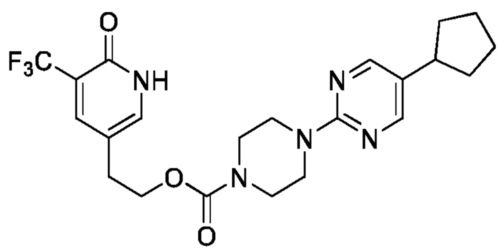
,

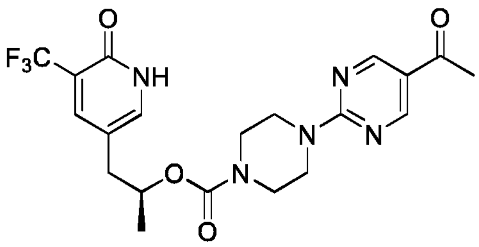,

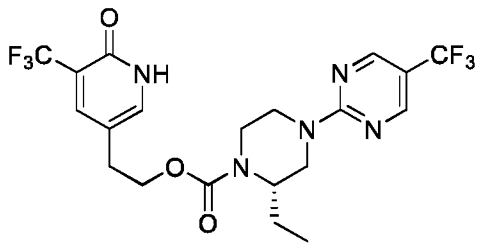,

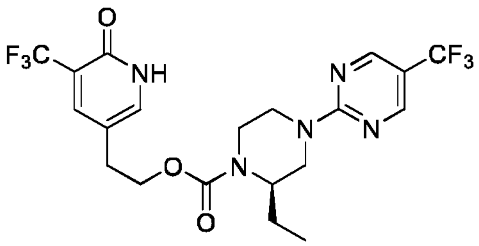,

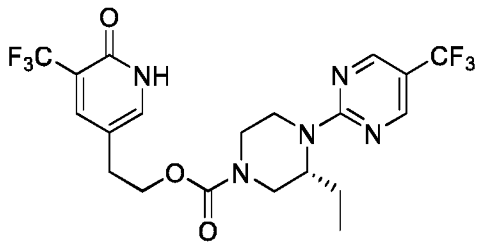,

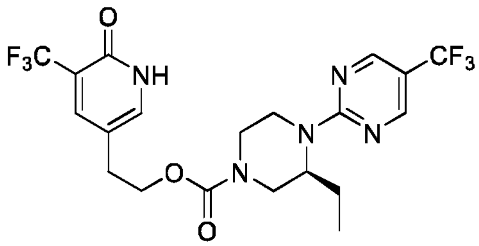,

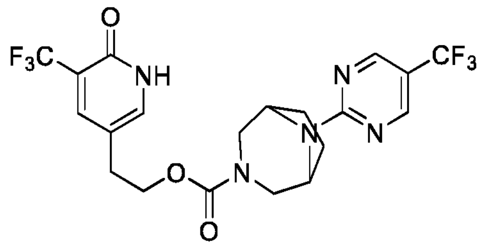,

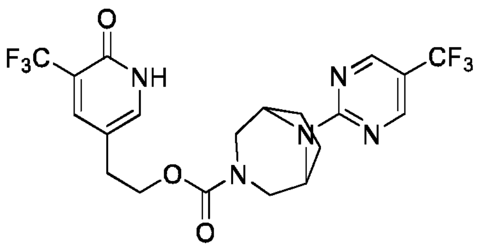,

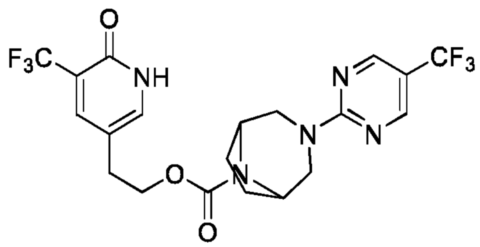,

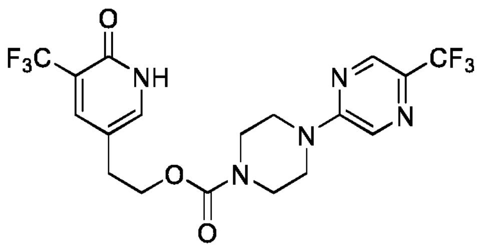,

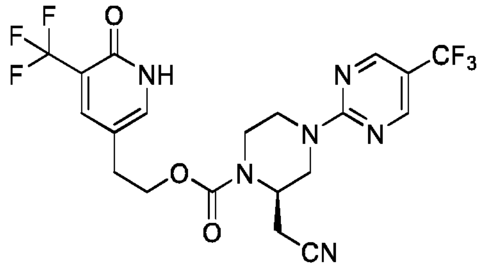,

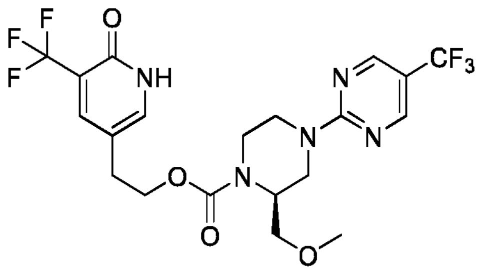 ,

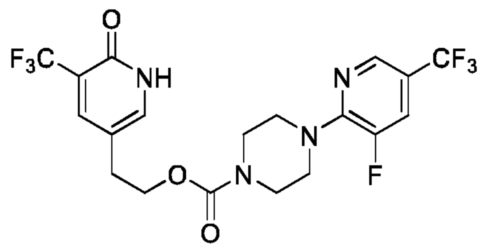 ,

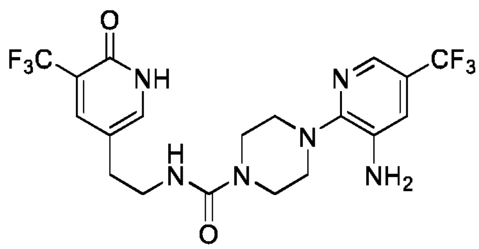 ,

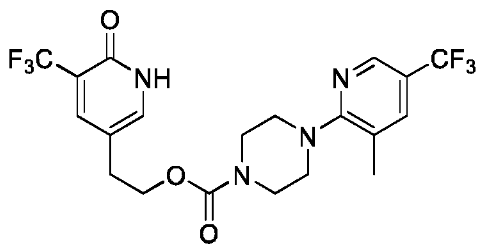 ,

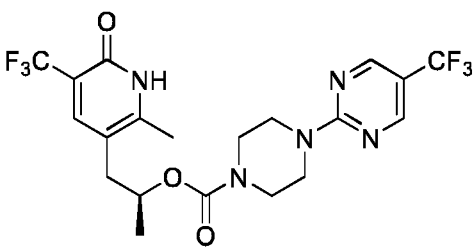 ,

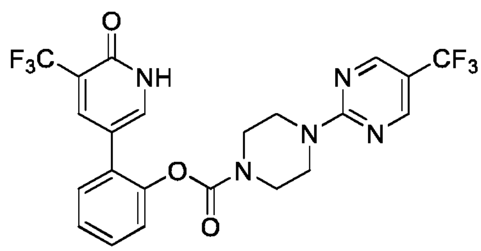 ,

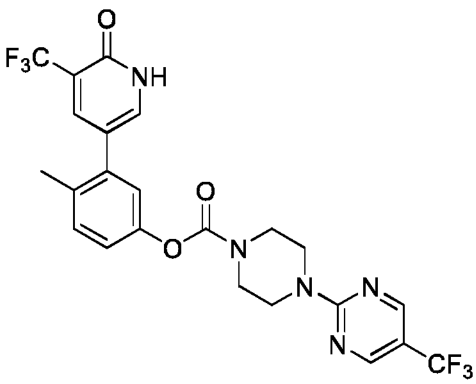 ,

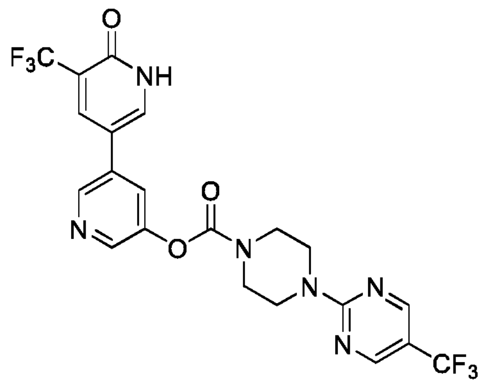 ,

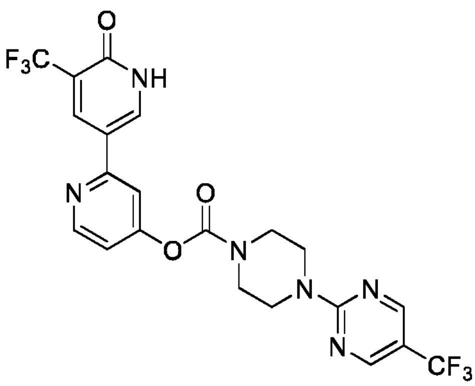 ,

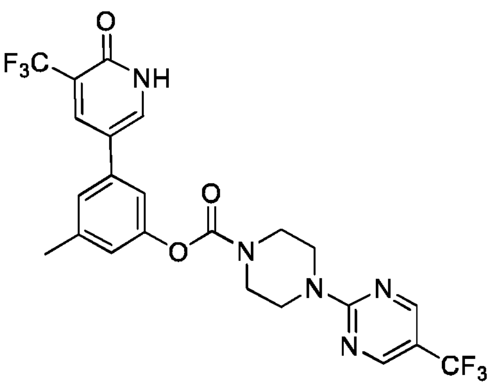 ,

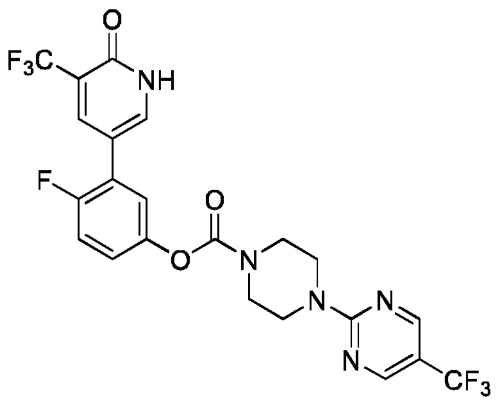 , 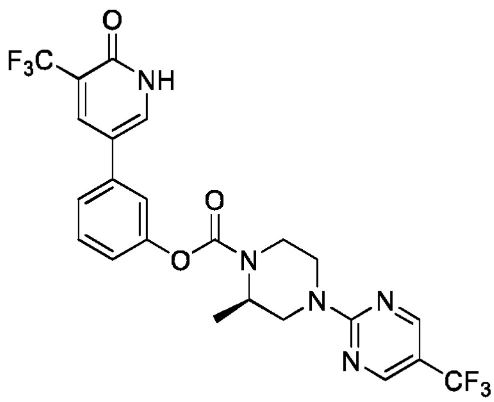 , and

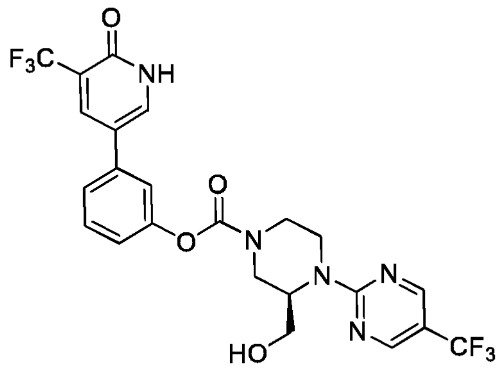 .

The present disclosure also provides the following compounds or pharmaceutically acceptable salts thereof, wherein the compounds are selected from

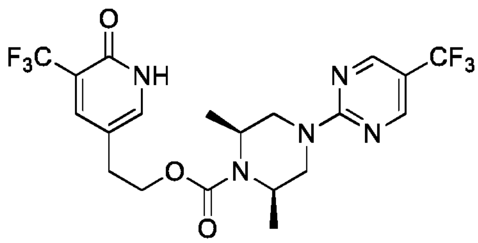 , 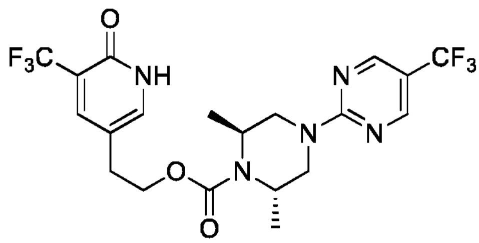 ,

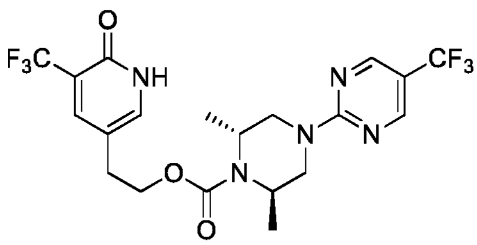 , 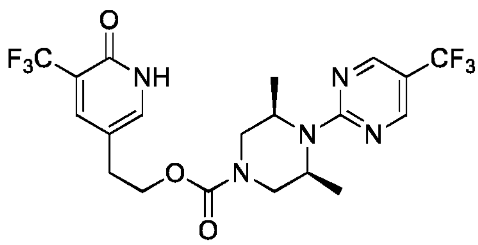 ,

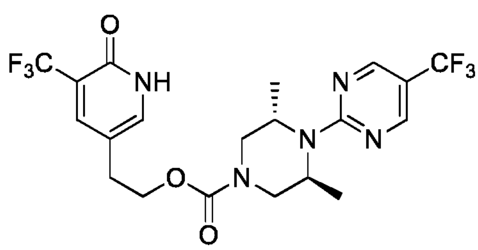 , 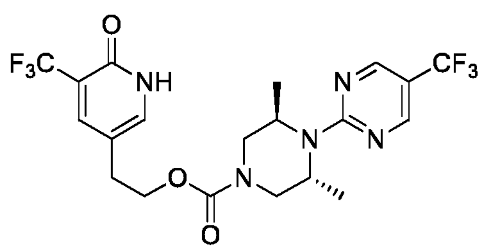 ,

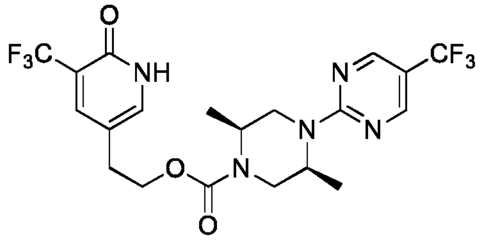,

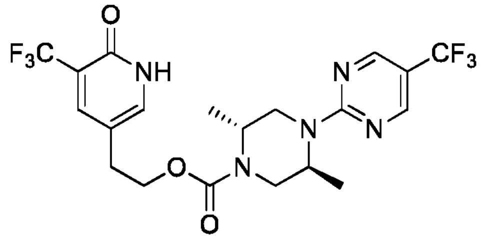,

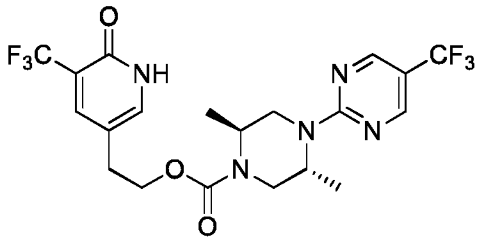

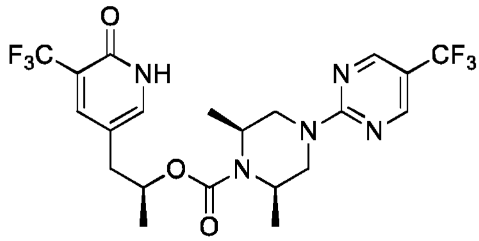

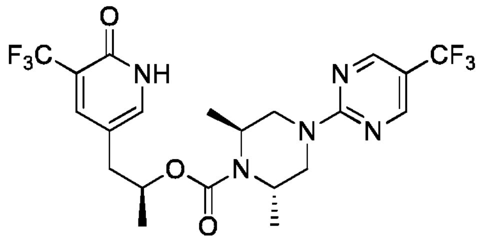

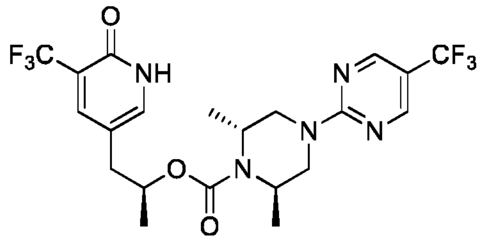

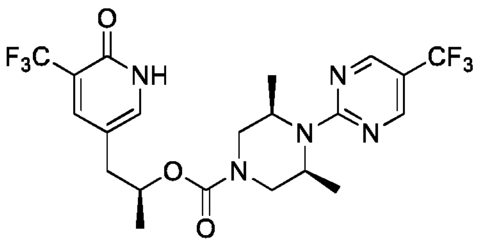,

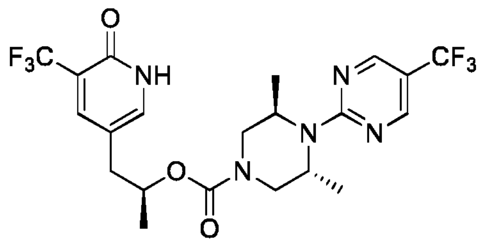,

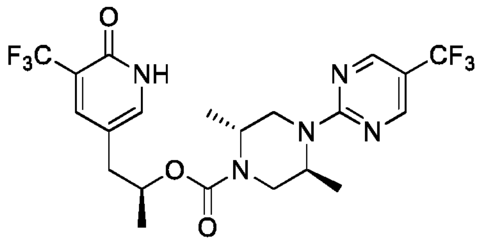,

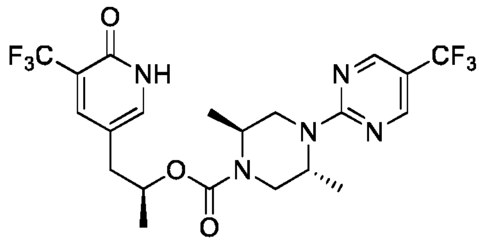,

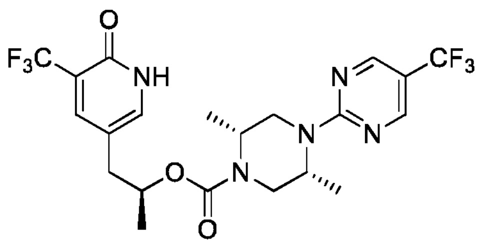,

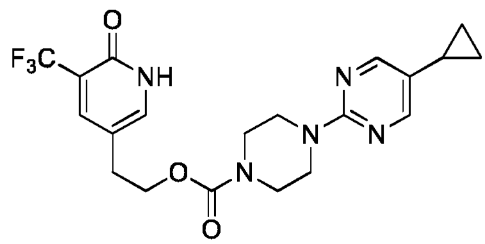,

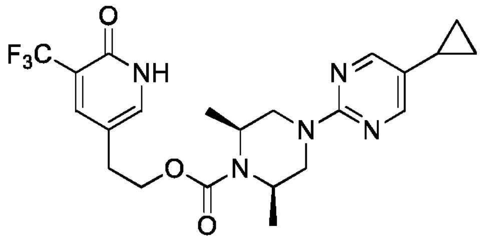

,

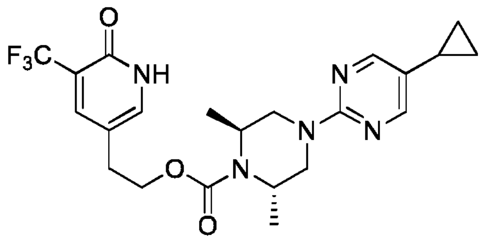

,

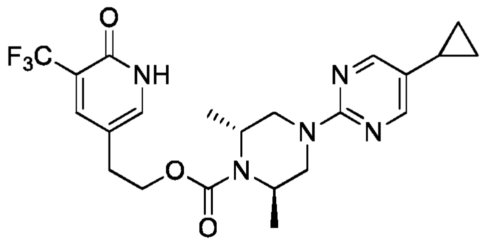

,

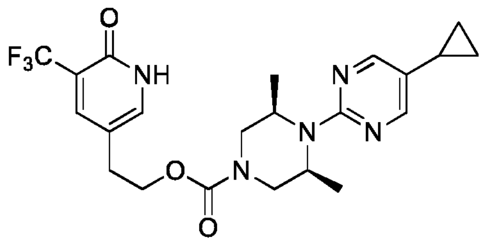

,

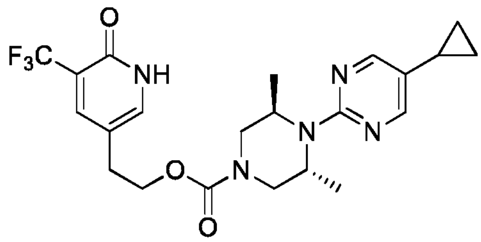

,

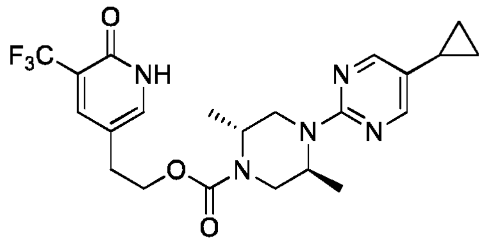

,

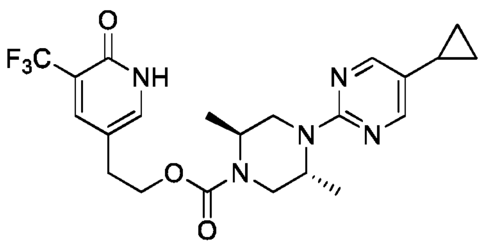

,

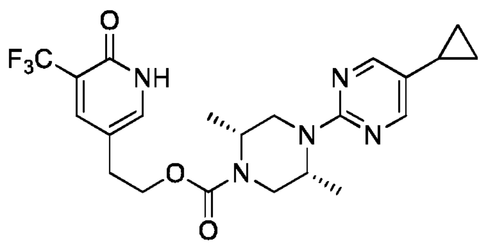

,

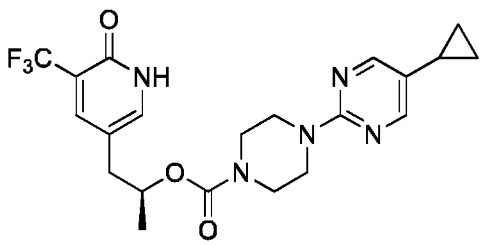

,

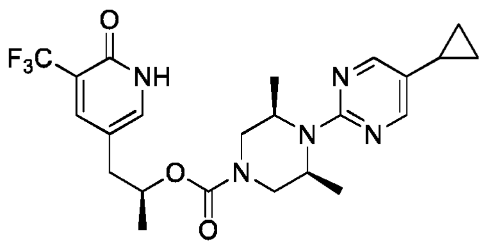

,

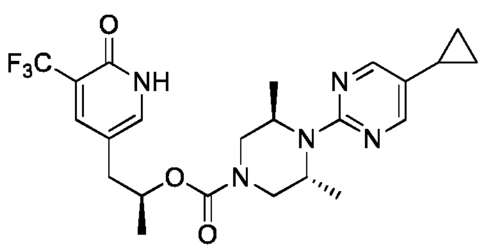

,

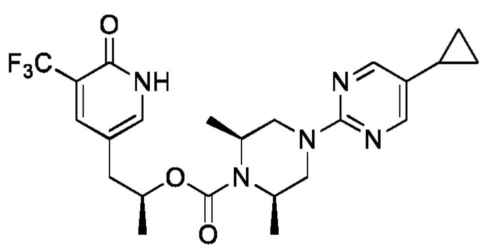

,

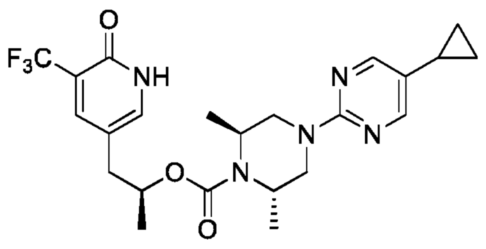

,

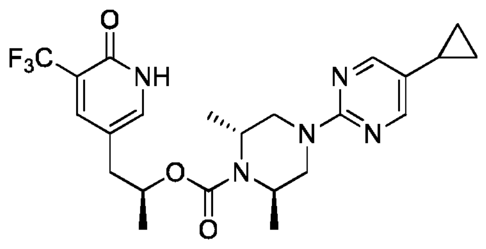

,

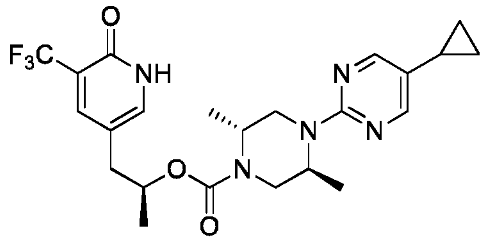

,

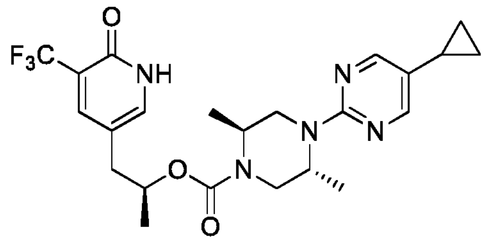

,

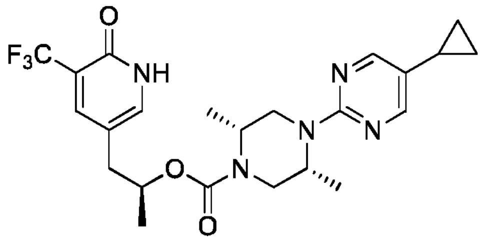

,

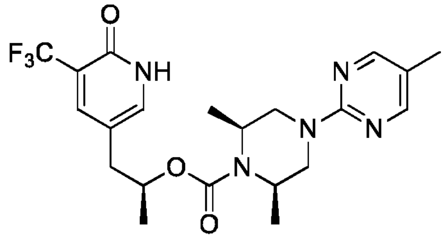

,

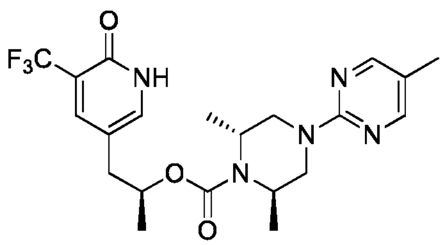

,

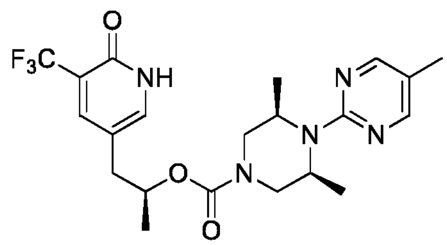

,

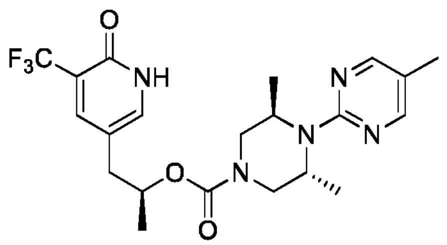

,

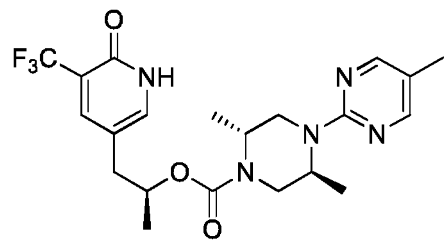

,

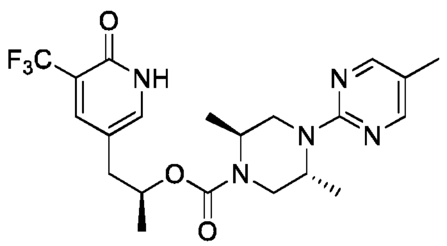

,

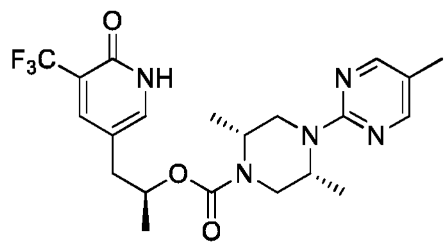

,

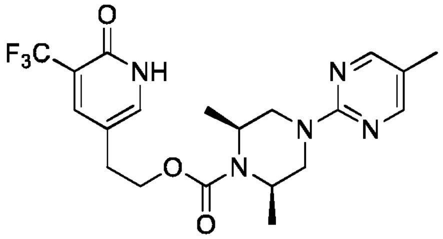

,

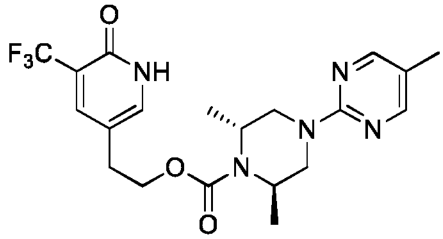

,

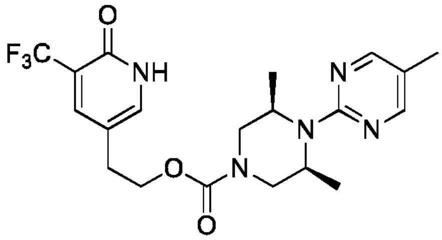

,

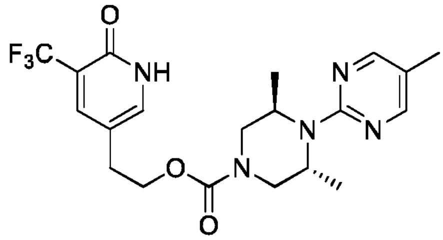

,

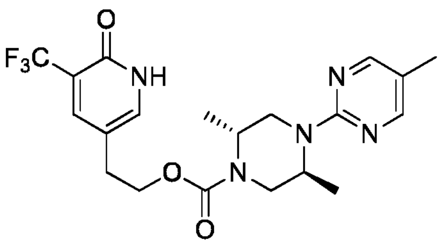

,

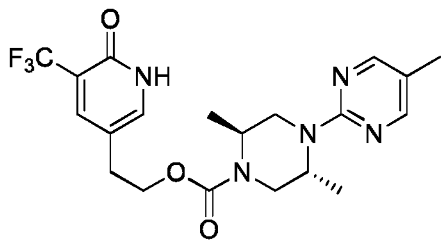

,

and

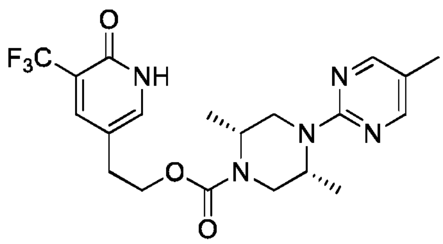

.

The present disclosure also provides a pharmaceutical composition comprising the compound or the pharmaceutically acceptable salt thereof as an active ingredient and a pharmaceutically acceptable carrier.

The present disclosure also provides a PARP7 inhibitor comprising the compound or the pharmaceutically acceptable salt thereof or the pharmaceutical composition. In addition, the present disclosure also provides a use of the PARP7 inhibitor in the manufacture of a medicament for treating cancer.

The present disclosure also provides a use of the compound or the pharmaceutically acceptable salt thereof or the pharmaceutical composition in the manufacture of a medicament for treating cancer.

The present disclosure also provides a method for inhibiting the activity of PARP7, comprising contacting the compound or the pharmaceutically acceptable salt thereof or the pharmaceutical composition with the PARP7.

The present disclosure also provides a method for treating cancer, comprising administering to a patient a therapeutically effective amount of the compound or the pharmaceutically acceptable salt thereof.

**Technical effect**

The compound of the present disclosure has a good inhibitory effect on PARP7, and thus results in excellent tumor growth inhibitory activity.

## Definition and description

Unless otherwise specified, the following terms and phrases used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting the compound with a sufficient amount of a base in a pure solution or a suitable inert solvent. When the compound of the present disclosure contains a relatively basic functional group, an acid addition salt can be obtained by contacting the compound with a sufficient amount of acid in a pure solution or a suitable inert solvent. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereoisomers, (*D*)-isomers, (*L*)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and"(±)" refers to racemic.

Unless otherwise specified, the absolute configuration of a stereogenic center is represented by a wedged solid bond (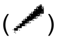) and a wedged dashed bond (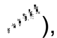), the relative configuration of a stereogenic center is represented by a straight solid bond (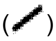) and a straight dashed bond (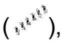), a wave line (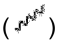) is used to represent a wedged solid bond (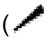) or a wedged dashed bond (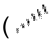), or the wave line (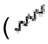) is used to represent a straight solid bond (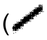) and a straight dashed bond (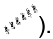).

Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as ketoenol isomerization and imine-enamine isomerization. Valence tautomer includes some recombination of bonding electrons for mutual transformation. A specific example of ketoenol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between

the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomer, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more than one atom that constitute the compound. For example, the compound can be radiolabeled with a radioactive isotope, such as tritium ($^3H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

"Optional" or "optionally" means that the subsequently described event or circumstance may, but does not necessarily, occur, and the description includes instances where the event or circumstance occurs and instances where it does not.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted by a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0 to 2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent is absent, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is the same as left-to-right reading order, for example, the linking group L contained in

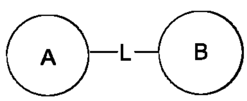

is -M-W-, then -M-W- links ring A and ring B to form

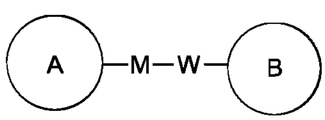

in the direction same as left-to-right reading order. A combination of the linking groups, substituents and/or variables thereof is allowed only when such combination can result in a stable compound.

Unless otherwise specified, when a group has one or more than one linkable site, any one or more than one site of the group can be linked to other groups through chemical bonds. When the linking site of the chemical bond is

not positioned, and there is H atom at the linkable site, then the number of H atom at the site will decrease correspondingly with the number of chemical bond linking thereto so as to meet the corresponding valence. The chemical bond between the site and other groups can be represented by a straight solid bond (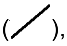), a straight dashed bond (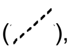), or a wavy line (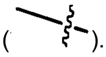). For example, the straight solid bond in $-OCH_3$ means that it is linked to other groups through the oxygen atom in the group; the straight dashed bond in

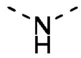

means that it is linked to other groups through the two ends of the nitrogen atom in the group; the wave lines in

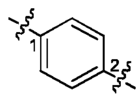

means that the phenyl group is linked to other groups through carbon atoms at position 1 and position 2;

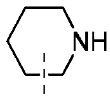

means that it can be linked to other groups through any linkable sites on the piperidinyl by one chemical bond, including at least four types of linkage, including

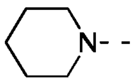 , 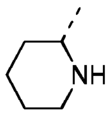 , 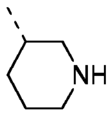 , 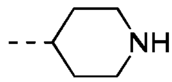 .

Even though the H atom is drawn on the -N-,

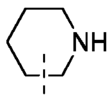

still includes the linkage of

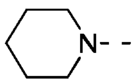 ,

merely when one chemical bond was connected, the H of this site will be reduced by one to the corresponding monovalent piperidinyl.

Unless otherwise specified, the number of atoms in a ring is usually defined as the number of ring members, for example, "5- to 7-membered ring" refers to a "ring" in which 5 to 7 atoms are arranged around.

Unless otherwise specified, the term "3- to 6-membered carbocyclic ring" refers to a saturated carbocyclic ring consisting of 3 to 6 carbon atoms. The 3- to 6-membered carbocyclic ring includes 3-membered, 4-membered, 5-membered, and 6-membered carbocyclic rings.

Unless otherwise specified, the term "$C_{1-6}$ alkyl" refers to a linear or branched saturated hydrocarbon group consisting of 1 to 6 carbon atoms. The $C_{1-6}$ alkyl includes $C_{1-5}$, $C_{1-4}$, $C_{1-3}$, $C_{1-2}$, $C_{2-6}$, $C_{2-4}$, $C_6$, $C_5$ alkyl, etc.; it can be monovalent (such as methyl), divalent (such as methylene), or multivalent (such as methine). Examples of $C_{1-6}$ alkyl include, but are not limited to, methyl (Me), ethyl (Et), propyl (including *n*-propyl and isopropyl), butyl (including *n*-butyl, isobutyl, *s*-butyl, and *t*-butyl), pentyl (including *n*-pentyl, isopentyl, and neopentyl), hexyl, etc.

Unless otherwise specified, the term "$C_{1-4}$ alkyl" refers to a linear or branched saturated hydrocarbon group consisting of 1 to 4 carbon atoms. The $C_{1-4}$ alkyl includes $C_{1-2}$, $C_{1-3}$, $C_{2-3}$ alkyl, etc.; it can be monovalent (such as methyl), divalent (such as methylene), or multivalent (such as methine). Examples of $C_{1-4}$ alkyl include, but are not limited to, methyl (Me), ethyl (Et), propyl (including *n*-propyl and isopropyl), butyl (including *n*-butyl, isobutyl, *s*-butyl, and *t*-butyl), etc.

Unless otherwise specified, the term "$C_{1-3}$ alkyl" refers to a linear or branched saturated hydrocarbon group consisting of 1 to 3 carbon atoms. The $C_{1-3}$ alkyl includes $C_{1-2}$, $C_{2-3}$ alkyl, etc.; it can be monovalent (such as methyl), divalent (such as methylene), or multivalent (such as methine). Examples of $C_{1-3}$ alkyl include, but are not limited to, methyl (Me), ethyl (Et), propyl (including *n*-propyl and isopropyl), etc.

Unless otherwise specified, the term "$C_{1-6}$ alkoxy" refers to an alkyl group containing 1 to 6 carbon atoms that are connected to the rest of the molecule through an oxygen atom. The $C_{1-6}$ alkoxy includes $C_{1-4}$, $C_{1-3}$, $C_{1-2}$, $C_{2-6}$, $C_{2-4}$, $C_6$, $C_5$, $C_4$, $C_3$ alkoxy, etc. Examples of $C_{1-6}$ alkoxy include, but are not limited to, methoxy, ethoxy, propoxy (including n-propoxy and isopropoxy), butoxy (including *n*-butoxy, isobutoxy, *s*-butoxy, and *t*-butoxy), pentyloxy (including *n*-pentyloxy, isopentyloxy, and neopentyloxy), hexyloxy, etc.

Unless otherwise specified, the term "$C_{1-3}$ alkoxy" refers to an alkyl group containing 1 to 3 carbon atoms that are connected to the rest of the molecule through an oxygen atom. The $C_{1-3}$ alkoxy includes $C_{1-2}$, $C_{2-3}$, $C_3$, $C_2$ alkoxy, etc. Examples of $C_{1-3}$ alkoxy include, but are not limited to, methoxy, ethoxy, propoxy (including *n*-propoxy and isopropoxy), etc.

Unless otherwise specified, the term "halogen element" or "halogen" by itself or as part of another substituent refers to a fluorine, chlorine, bromine, or iodine atom.

Unless otherwise specified, the term "aryl" refers to an unsaturated, usually aromatic, hydrocarbon group, which may be a single ring or multiple rings fused together. Examples of aryl include, but are not limited to, phenyl and naphthyl. $C_{5-10}$ aryl is preferred, and $C_{5-6}$ aryl and $C_{6-10}$ aryl are more preferred.

Unless otherwise specified, "$C_{3-7}$ cycloalkyl" refers to a saturated cyclic hydrocarbon group consisting of 3 to 7 carbon atoms, which is a monocyclic and bicyclic system, and the $C_{3-7}$ cycloalkyl includes $C_{3-6}$, $C_{3-5}$, $C_{3-4}$, $C_{4-6}$, $C_{4-5}$, $C_{5-6}$ cycloalkyl, etc.; it can be monovalent, divalent, or multivalent. Examples of $C_{3-7}$ cycloalkyl include, but are not limited to, cyclopropyl, cyclobutyl, cyclopentyl, cyclohexyl, cycloheptyl, etc.

Unless otherwise specified, the term "4- to 10-membered heterocyclyl" by itself or in combination with other terms refers to a saturated or unsaturated cyclic group consisting of 4 to 10 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and $S(O)_p$, p is 1 or 2). It includes monocyclic and bicyclic systems, wherein the bicyclic systems include a spiro ring, a fused ring, and a bridged ring. In addition, with regard to the "4- to 10-membered heterocyclyl", a heteroatom may occupy the connection position of the heterocyclyl with the rest of the molecule. The 4- to 10-membered heterocyclyl includes 4- to 8-membered, 4- to 7-membered, 4- to 6-membered, 4- to 5-membered, 5- to 8-membered, 5- to 7-membered, 5-to 6-membered, 6- to 8-membered, 7- to 8-membered, 4-membered, 5-membered, 6-membered, and 7-membered heterocyclyl, etc. Examples of 4- to 10-membered heterocyclyl include, but are not limited to, azetidinyl, oxetanyl, thietanyl, pyrrolidinyl, pyrazolidinyl, imidazolidinyl, tetrahydrothienyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, homopiperidinyl, dioxacycloheptyl, etc.

Unless otherwise specified, the term "5- to 10-membered heteroaryl" refers to a stable monocyclic or polycyclic aromatic hydrocarbon group containing at least one heteroatom (N, O, S, NO, SO, $S(O)_2$, or NR), including 5-membered, 6-membered, or 7-membered monocyclic or bicyclic, or 7-membered, 8-membered, 9-membered, or 10-membered bicyclic heteroaryl; preferably containing a carbon atom and 1, 2, 3, or 4 cycloheteroatoms independently selected from N, O, and S. Examples of 5- to 10-membered heteroaryl include, but are not limited to, pyrrolyl, pyrazolyl, imidazolyl, pyrazinyl, oxazolyl, benzoxazolyl, isoxazolyl, thiazolyl, furyl, thienyl, pyridyl, pyrimidinyl, benzothiazolyl, purinyl, benzimidazolyl, indolyl, isoquinolinyl, quinoxalinyl, and quinolinyl. The "5- to 10-membered heteroaryl" is preferably 5- to 6-membered heteroaryl.

The term "5- to 6-membered heteroaryl" refers to a monocyclic group consisting of 5 to 6 ring atoms with a conjugated $\pi$-electron system, wherein 1, 2, 3, or 4 ring atoms are heteroatoms independently selected from O, S, and N, and the rest are carbon atoms. Herein, nitrogen atoms are optionally quaternized, and nitrogen and sulfur heteroatoms can be optionally oxidized (i.e., NO and $S(O)_p$, p is 1 or 2). The 5- to 6-membered heteroaryl can be linked to the rest of the molecule through a heteroatom or a carbon atom. The 5- to 6-membered heteroaryl includes 5-membered and 6-membered heteroaryl. Examples of the 5-to 6-membered heteroaryl include, but are not limited to, pyrrolyl (including N-pyrrolyl, 2-pyrrolyl, 3-pyrrolyl, etc.), pyrazolyl (including 2-pyrazolyl, 3-pyrrolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1*H*-1,2,3-triazolyl, 2*H*-1,2,3-triazolyl, 1*H*-1,2,4-triazolyl, 4*H*-1,2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furyl (including 2-furyl, 3-furyl, etc.), thienyl (including 2-thienyl, 3-thienyl, etc.), pyridyl (including 2-pyridyl, 3-pyridyl, 4-pyridyl, etc.), pyrazinyl, or pyrimidinyl (including 2-pyrimidinyl, 4-pyrimidinyl, etc.).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

The structure of the compounds of the present disclosure can be confirmed by conventional methods known to those skilled in the art, and if the disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuK$\alpha$ radiation as the light source and scanning mode: $\varphi/\omega$ scan, and after collecting the relevant data, the crystal structure can be further analyzed by direct method (Shelxs97).

The solvents used in the present disclosure are commercially available.

Unless otherwise specified, the reagent ratios used in silica gel column chromatography, silica gel chromatographic column chromatography, and silica gel thin-layer chromatography plates of the present disclosure are all volume ratios.

The following abbreviations are used in the present disclosure: HATU represents 2-(7-azabenzotriazol-1-yl)-*N*,*N*,*N'*,*N'*-tetramethyluronium hexafluorophosphate; DMSO represents dimethyl sulfoxide; CDsOD represents deuterated methanol; $CDCl_3$ represents deuterated chloroform; TBSO represents tert-butyldimethylsilyloxy.

The compounds of the present disclosure are named according to the conventional naming principles in the art or by ChemDraw® software, and the commercially available compounds use the supplier catalog names.

**Summary of experimental instruments**

The structures of the compounds of the present disclosure were determined by nuclear magnetic resonance (NMR) or/and liquid chromatography-mass spectrometry (LC-MS), or ultra-performance liquid chromatography-mass spectrometry (UPLC-MS). NMR chemical shift ($\delta$) was given in units of parts per million (ppm). NMR was determined using a Bruker Neo 400M or Bruker Ascend 400 NMR instrument with deuterated dimethyl sulfoxide (DMSO-$d_6$), deuterated methanol (CDsOD), deuterated chloroform ($CDCl_3$) and deuterium oxide ($D_2O$) as solvents and tetramethylsilane (TMS) as an internal standard.

Liquid chromatography-mass spectrometry (LC-MS) was determined using an Agilent 1260-6125B single quadrupole mass spectrometer, and the column was Welch Biomate column (C18, 2.7 $\mu$m, 4.6 * 50 mm) or waters H-Class SQD2, and the column was Welch Ultimate column (XB-C18, 1.8 $\mu$m, 2.1 * 50 mm) mass spectrometer (ion source was electrospray ionization).

Ultra-performance liquid chromatography-mass spectrometry (UPLC-MS) was determined using a Waters UPLC H-class SQD mass spectrometer (ion source was electrospray ionization).

HPLC was determined using Waters e2695-2998 or Waters ARC and Agilent 1260 or Agilent Poroshell HPH high performance liquid chromatography.

Preparative HPLC was determined using Waters 2555-2489 (10 $\mu$m, ODS 250 cm $\times$ 5 cm) or GILSON Trilution LC, and the column was Welch XB-C18 column (5 $\mu$m, 21.2 * 150 mm).

Chiral HPLC was determined using waters acquity UPC2; the column was Daicel chiralpak AD-H (5 $\mu$m, 4.6 * 250 mm), Daicel chiralpak OD-H (5 $\mu$m, 4.6 * 250 mm), Daicel chiralpak IG-3 (3 $\mu$m, 4.6 * 150 mm), Chiral Technologies Europe AD-3 (3 $\mu$m, 3.0 * 150 mm), and Trefoil TM Technology Trefoil TM AMY1 (2.5 $\mu$m, 3.0 * 150 mm).

Supercritical fluid chromatography (SFC) was determined using waters SFC 80Q, and the column was Daicel Chiralcel OD/OJ/OZ (20 $\times$ 250 mm, 10 $\mu$m) or Daicel Chiralpak IC/IG/IH/AD/AS (20 $\times$ 250 mm, 10 $\mu$m).

GF254 silica gel plate from Yantai Jiangyou Silica Gel Development Co., Ltd. or Rushan Sanpont New Materials Co., Ltd. was used as a thin-layer chromatography silica gel plate. The specification used for TLC was 0.15 mm to 0.20 mm. The specification used for preparative TLC was 20 $\times$ 20 cm. Column chromatography generally used 200 to 300 mesh silica gel from Yucheng Chemical as a carrier.

DETAILED DESCRIPTION OF THE PREFERRED EMBODIMENT

The present disclosure is described in detail by the examples below, but it does not mean that there are any adverse restrictions on the present disclosure. The compounds of the present disclosure can be prepared by a variety of synthetic methods known to those skilled in the art, including the specific embodiments listed below, the embodiments

formed by their combination with other chemical synthesis methods, and equivalent alternatives known to those skilled in the art, and preferred embodiments include, but are not limited to, the examples of the present disclosure. For those skilled in the art, it is obvious to make various modifications and improvements to the specific embodiments of the present disclosure without departing from the spirit and scope of the present disclosure.

**Intermediate 1A**

**2-(4-(5-(Trifluoromethyl)pyrimidin-2-yl)piperazin-1-yl)ethan-1-amine**

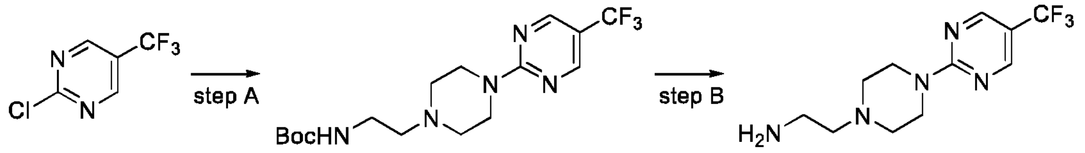


**1A-1** **1A-2** **intermediate 1A**

**Step A:** 2-Chloro-5-(trifluoromethyl)pyrimidine (1 g, 5.5 mmol) and 1-(N-Boc-aminoethyl)piperazine (1.25 g, 5.5 mmol) were dissolved in *N*-methylmorpholine (50 mL), then potassium carbonate (15 g, 0.11 mol) was added thereto, and the reaction mixture was stirred at 80°C for 3 hours. The reaction mixture was cooled to room temperature, filtered, washed with water, and dried to obtain 1.9 g of *tert*-butyl (2-(4-(5-(trifluoromethyl)pyrimidin-2-yl)piperazin-1-yl)ethyl)carbamate (**1A-2**).

MS (ESI) M/Z: 376.2 $[M+H]^+$.

$^1H$ NMR (400 MHz, $CDCl_3$) $\delta$ 8.48 (s, 2H), 5.18 (brs, 1H), 4.18 - 3.75 (m, 4H), 3.45 - 3.21 (m, 2H), 2.63 (brs, 6H), 1.46 (s, 9H).

**Step B:** *tert*-Butyl (2-(4-(5-(trifluoromethyl)pyrimidin-2-yl)piperazin-1-yl)ethyl)carbamate (170 mg, 0.45 mmol) was dissolved in dichloromethane (2 mL), then trifluoroacetic acid (2 mL) was added thereto in an ice bath, and the reaction mixture was stirred at room temperature for 2 hours. The reaction mixture was concentrated under reduced pressure, and then neutralized with saturated sodium bicarbonate solution to adjust the pH to neutrality. The aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure to obtain 120 mg of 2-(4-(5-(trifluoromethyl)pyrimidin-2-yl)piperazin-1-yl)ethan-1-amine (compound **1A**).

MS (ESI) M/Z: 276.1 $[M+H]^+$.

**Intermediate 1B**

**Ethyl 2-(6-oxo-5-(trifluoromethyl)-1,6-dihydropyridazin-3-yl)acetate**

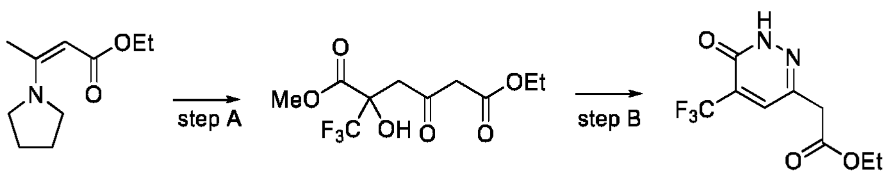


**1B-1** **1B-2** **1B**

**Step A:** Ethyl (Z)-3-(pyrrolidin-1-yl)but-2-enoate (0.5 g, 2.7 mmol) was dissolved in toluene (10 mL), then methyl trifluoropyruvate (0.4 g, 2.7 mmol) was added thereto, and the reaction mixture was stirred at 40°C for 6 hours. The reaction mixture was cooled to room temperature and concentrated under reduced pressure. The resulting residue was dissolved in dichloromethane (10 mL), then 5% dilute hydrochloric acid (5 mL) was added thereto, and the reaction mixture was stirred at room temperature overnight. The reaction mixture was concentrated under reduced pressure, and the residue was neutralized with saturated sodium bicarbonate solution to adjust the pH to neutrality. The aqueous phase was extracted with dichloromethane (50 mL × 3), and the organic phases were combined. The combined organic phases were first washed with saturated brine (30 mL × 2), then dried over anhydrous sodium sulfate, filtered, and

finally concentrated under reduced pressure to obtain 700 mg of 6-ethyl 1-methyl 2-hydroxy-4-oxo-2-(trifluoromethyl)hexanedioate (**1B-2**), which was directly used in the next reaction step without purification.

MS (ESI) M/Z: 308.9 $[M+Na]^+$.

**Step B: 1B-2** (540 mg, 1.9 mmol) was dissolved in glacial acetic acid (5 mL) at room temperature under nitrogen atmosphere, then 98% hydrazine hydrate (476 mg, 9.4 mmol) was added thereto, and the reaction mixture was heated to 130°C under microwave irradiation and reacted for 30 minutes. The reaction mixture was cooled to room temperature, then concentrated under reduced pressure, and neutralized with saturated sodium bicarbonate solution to adjust the pH to neutrality. The aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure. The resulting residue was purified by silica gel column chromatography to obtain 240 mg of ethyl 2-(6-oxo-5-(trifluoromethyl)-1,6-dihydropyridazin-3-yl)acetate (compound **1B**).

MS (ESI) M/Z: 249.0 $[M-H]^-$.

$^1H$ NMR (400 MHz, DMSO-$d_6$) δ 13.67 (s, 1H), 7.98 (s, 1H), 4.12 (q, *J* = 7.1 Hz, 2H), 3.79 (s, 2H), 1.20 (t, *J* = 7.1 Hz, 3H).

**Intermediate 1C**

**2-(Piperazin-1-yl)-5-(trifluoromethyl)pyrimidine**

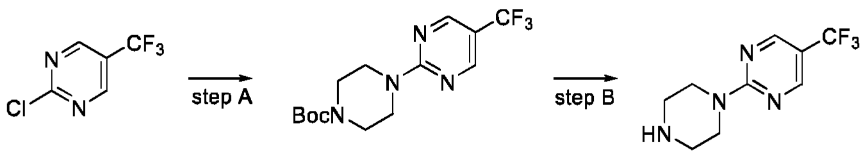


**1A-1** **1C-2** **intermediate 1C**

**Step A:** 2-Chloro-5-(trifluoromethyl)pyrimidine (10 g, 55 mmol) and *N*-Boc-piperazine (11 g, 60 mmol) were dissolved in N-methylmorpholine (50 mL), then potassium carbonate (15 g, 0.11 mol) was added thereto, and the reaction mixture was stirred at 80°C for 3 hours. The reaction mixture was cooled to room temperature, then poured into water, and stirred thoroughly. The precipitated white solid was then filtered, washed with water, and dried to obtain 15 g of *tert*-butyl 4-(5-(trifluoromethyl)pyrimidin-2-yl)piperazine-1-carboxylate (**1C-2**).

$^1H$ NMR (400 MHz, $CDCl_3$) δ 8.53 (s, 2H), 3.96 - 3.88 (m, 4H), 3.56 - 3.49 (m, 4H), 1.49 (s, 9H).

**Step B:** *tert*-Butyl 4-(5-(trifluoromethyl)pyrimidin-2-yl)piperazine-1 -carboxylate (10 g, 30 mmol) was dissolved in dichloromethane (20 mL), and trifluoroacetic acid (20 mL) was added dropwise thereto at room temperature. After the dropwise addition was completed, the reaction mixture was stirred at room temperature for 2 hours. The reaction mixture was concentrated and poured into water, and added with saturated sodium bicarbonate solution to adjust the pH to 8 to 9. The precipitated white solid was filtered, washed with water, and dried to obtain 4.0 g of 2-(piperazin-1-yl)-5-(trifluoromethyl)pyrimidine (compound 1C).

MS (ESI) M/Z: 232.6 $[M+H]^+$.

$^1H$ NMR (400 MHz, $CDCl_3$) δ 8.47 (s, 2H), 3.93 - 3.83 (m, 4H), 2.98 - 2.89 (m, 4H).

**Intermediate 1D**

**1-(5-(Trifluoromethyl)pyridin-2-yl)piperazine**

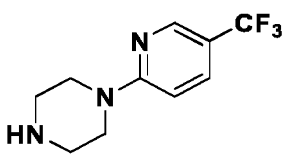

2-Chloro-5-(trifluoromethyl)pyridine was used as a raw material instead of 2-chloro-5-(trifluoromethyl)pyrimidine with the preparation method referring to that of **intermediate 1C.**

MS (ESI) M/Z: 232.2 $[M+H]^+$.

$^1H$ NMR (400 MHz, DMSO-$d_6$) δ 9.22 (brs, 2H), 8.46 (s, 1H), 7.88 (d, *J* = 8.8 Hz, 1H), 7.05 (d, *J* = 9.2 Hz, 1H), 3.85 (brs, 4H), 3.20 (brs, 4H).

**Intermediate 1E**

**Piperazin-1-yl(5-(trifluoromethyl)pyridin-2-yl)methanone**

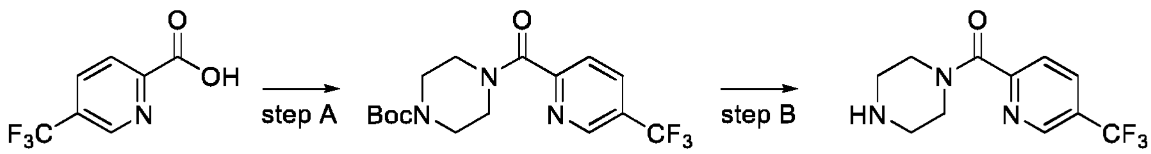


1E-1 1E-2 intermediate 1E

**Step A:** 5-(Trifluoromethyl)picolinic acid (200 mg, 1.05 mmol) was dissolved in *N,N*-dimethylformamide (5 mL) at room temperature, then 2-(7-azabenzotriazol-1-yl)-*N,N,N',N'*-tetramethyluronium hexafluorophosphate (600 mg, 1.58 mmol), *tert*-butyl piperazine-1-carboxylate (234.7 mg, 1.26 mmol), and *N,N*-diisopropylethylamine (407 mg, 3.15 mmol) were added thereto, and the reaction mixture was stirred at room temperature for 2 hours. The reaction mixture was added with water (30 mL) to quench, extracted with ethyl acetate (10 mL × 3), and the organic phases were combined. The combined organic phases were sequentially washed with water (30 mL) and saturated brine (30 mL), then dried over anhydrous sodium sulfate, filtered, and finally concentrated under reduced pressure. The resulting residue was purified by silica gel column chromatography to obtain 346 mg of *tert*-butyl 4-(5-(trifluoromethyl)picolinoyl)piperazine-1 -carboxylate.

MS (ESI) M/Z: 304.0 [M+H]$^+$.

**Step B:** *tert*-Butyl 4-(5-(trifluoromethyl)picolinoyl)piperazine-1-carboxylate (346 mg, 0.96 mmol) was dissolved in dichloromethane (6 mL) at room temperature. Trifluoroacetic acid (3 mL) was then added to the above solution. The reaction mixture was stirred at room temperature for 2 hours. The reaction mixture was concentrated under reduced pressure to obtain 250 mg of piperazin-1-yl(5-(trifluoromethyl)pyridin-2-yl)methanone.

MS (ESI) M/Z: 260.1 [M+H]$^+$.

**Intermediate 1F**

**2-(2-(Piperazin-1-yl)pyrimidin-5-yl)propan-2-ol**

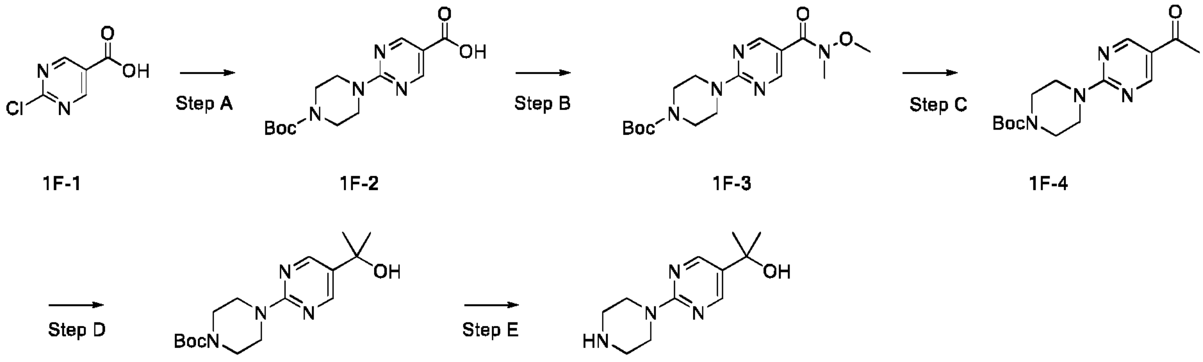


1F-1 1F-2 1F-3 1F-4

1F-5 intermediate 1F

**Step A:** 2-Chloropyrimidine-5-carboxylic acid (200 mg, 1.26 mmol) was dissolved in acetonitrile (5 mL) at room temperature. *tert*-Butyl piperazine-1-carboxylate (234.6 mg, 1.26 mmol) and *N,N*-diisopropylethylamine (244.3 mg, 1.89 mmol) were then added to the above solution. The reaction mixture was stirred at room temperature for 16 hours and concentrated under reduced pressure to obtain 388 mg of 2-(4-(*tert*-butoxycarbonyl)piperazin-1-yl)pyrimidine-5-carboxylic acid.

MS (ESI) M/Z: 253.1 [M+H-*t*-Bu]$^+$.

**Step B:** 2-(4-(*tert*-Butoxycarbonyl)piperazin-1-yl)pyrimidine-5-carboxylic acid (1.94 g, 6.3 mmol) was dissolved in *N,N*-dimethylformamide (25 mL) at room temperature. 2-(7-Azabenzotriazol-1-yl)-*N,N,N',N'*-tetramethyluronium hexafluorophosphate (2.88 g, 7.56 mmol), *N*-methyl-*N*-methoxylamine hydrochloride (738 mg, 7.56 mmol), and *N,N*-diisopropylethylamine (2.44 g, 18.9 mmol) were then added to the above solution. The reaction mixture was stirred at room temperature for 3 hours, and added with water (100 mL) to quench. The mixture was extracted with ethyl acetate (50 mL × 3), and the organic phases were combined. The combined organic phases were sequentially washed with water (100 mL × 2) and saturated brine (100 mL). The organic phases were then dried over anhydrous sodium sulfate, filtered, and finally concentrated under reduced pressure. The resulting residue was purified by silica gel column chromatography to obtain 800 mg of *tert*-butyl 4-(5-(methoxy(methyl)carbamoyl)pyrimidin-2-yl)piperazine-1-carboxylate.

MS (ESI) M/Z: 296.2 [M+H-*t*-Bu]$^+$.

**Step C:** *tert*-Butyl 4-(5-(methoxy(methyl)carbamoyl)pyrimidin-2-yl)piperazine-1-carboxylate (700 mg, 1.99 mmol) was dissolved in anhydrous tetrahydrofuran (20 mL) at room temperature. The reaction mixture was replaced with nitrogen three times, and then slowly added dropwise with methylmagnesium bromide solution (6 mL, 6 mmol) in an ice-water bath. The reaction mixture was added with saturated ammonium chloride aqueous solution (30 mL) to quench. The mixture was extracted with ethyl acetate (10 mL × 3), and the organic phases were combined. The combined organic phases were washed with saturated brine (30 mL). The organic phases were then dried over anhydrous sodium sulfate, filtered, and finally concentrated under reduced pressure. The resulting residue was purified by silica gel column chromatography to obtain 580 mg of *tert*-butyl 4-(5-acetylpyrimidin-2-yl)piperazine-1-carboxylate.

MS (ESI) M/Z: 251.2 [M+H-*t*-Bu]$^+$.

**Step D:** *tert*-Butyl 4-(5-acetylpyrimidin-2-yl)piperazine-1-carboxylate (200 mg, 0.65 mmol) was dissolved in anhydrous tetrahydrofuran (5 mL) at room temperature. The reaction mixture was replaced with nitrogen three times, and then slowly added dropwise with methylmagnesium bromide solution (1.95 mL, 1.95 mmol) in an ice-water bath. The reaction mixture was stirred at room temperature for 3 hours, and added with saturated ammonium chloride aqueous solution (30 mL) to quench. The mixture was extracted with ethyl acetate (10 mL × 3), and the organic phases were combined. The combined organic phases were washed with saturated brine (30 mL). The organic phases were then dried over anhydrous sodium sulfate, filtered, and finally concentrated under reduced pressure. The resulting residue was purified by silica gel column chromatography to obtain 100 mg of *tert*-butyl 4-(5-(2-hydroxypropan-2-yl)pyrimidin-2-yl)piperazine-1-carboxylate.

MS (ESI) M/Z: 323.2 [M+H]$^+$.

**Step E:** *tert*-Butyl 4-(5-(2-hydroxypropan-2-yl)pyrimidin-2-yl)piperazine-1-carboxylate (100 mg, 0.31 mmol) was dissolved in 1,4-dioxane (2 mL) at room temperature. A solution of hydrochloric acid in dioxane (1 mL) was then added to the above solution. The reaction mixture was stirred at room temperature for 16 hours and concentrated under reduced pressure to obtain 68.8 mg of 2-(2-(piperazin-1-yl)pyrimidin-5-yl)propan-2-ol.

MS (ESI) M/Z: 205.1 [M+H-$H_2O$]$^+$.

**Intermediate 1G**

**5-(Trifluoromethyl)-1',2',3',6'-tetrahydro-2,4'-bipyridine**

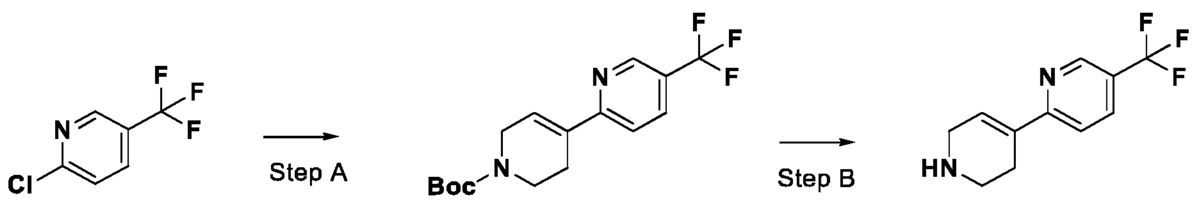


1G-1 1G-2 intermediate 1G

**Step A:** 5-Bromo-2-methoxy-3-(trifluoromethyl)pyridine (1.0 g, 5.5 mmol), *tert*-butyl 4-(4,4,5,5-tetramethyl-1,3,2-dioxaborolan-2-yl)-3,6-dihydropyridine-1(2*H*)-carboxylate(2.2g, 7.1 mmol), and sodium carbonate (1.16 g, 11.0 mmol) were dissolved in 1,4-dioxane (30 mL) and water (3 mL) at room temperature. The reaction mixture was replaced with nitrogen for 15 minutes, and then added with [1,1'-bis(diphenylphosphino)ferrocene]palladium(II) dichloride (402.0 mg, 0.55 mmol). The reaction mixture was replaced with nitrogen for 15 minutes, and then stirred in a sealed tube under microwave irradiation at 100°C for 1 hour. The reaction mixture was concentrated under reduced pressure, and the resulting residue was purified by silica gel column chromatography to obtain 1.68 g of *tert*-butyl 5-(trifluoromethyl)-3',6'-dihydro-[2,4'-bipyridine]-1'(2'*H*)-carboxylate.

MS (ESI) M/Z: 273.1 [M+H-*t*-Bu]$^+$.

**Step B:** *tert*-Butyl -5-(trifluoromethyl)-3',6'-dihydro-[2,4'-bipyridine]-1'(2'*H*) carboxylate (400 mg, 1.22 mmol) was dissolved in dichloromethane (3 mL) at room temperature, and then trifluoroacetic acid (1 mL) was added to the above solution. The reaction mixture was stirred at room temperature for 2 hours and concentrated under reduced pressure to obtain 278 mg of 5-(trifluoromethyl)-1',2',3',6'-tetrahydro-2,4'-bipyridine, which was directly used in the next reaction step.

MS (ESI) M/Z: 229.2 [M+H]$^+$.

**Intermediate 1H**

**2-(Piperidin-4-yl)-5-(trifluoromethyl)pyridine**

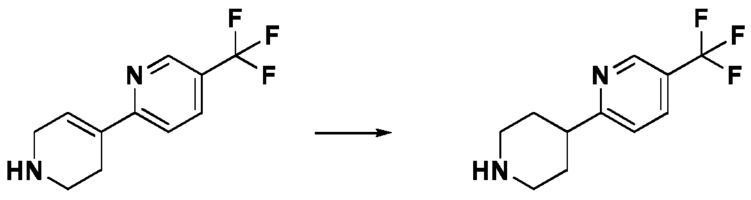

1G 1H

5-(Trifluoromethyl)-1',2',3',6'-tetrahydro-2,4'-bipyridine (200 mg, 0.87 mmol) was dissolved in methanol (4 mL). 10% palladium on carbon (wet basis) (20 mg) was then added to the above solution. After the reaction system was replaced with hydrogen three times, the reaction mixture was stirred at room temperature for 1 hour. The reaction mixture was filtered through diatomite, and the filter cake was washed with methanol (10 mL × 3) and concentrated under reduced pressure to obtain 200 mg of 2-(piperidin-4-yl)-5-(trifluoromethyl)pyridine. The crude product was directly used in the next reaction step.

MS (ESI) M/Z: 231.2 [M+H]$^+$.

**Intermediate 1I**

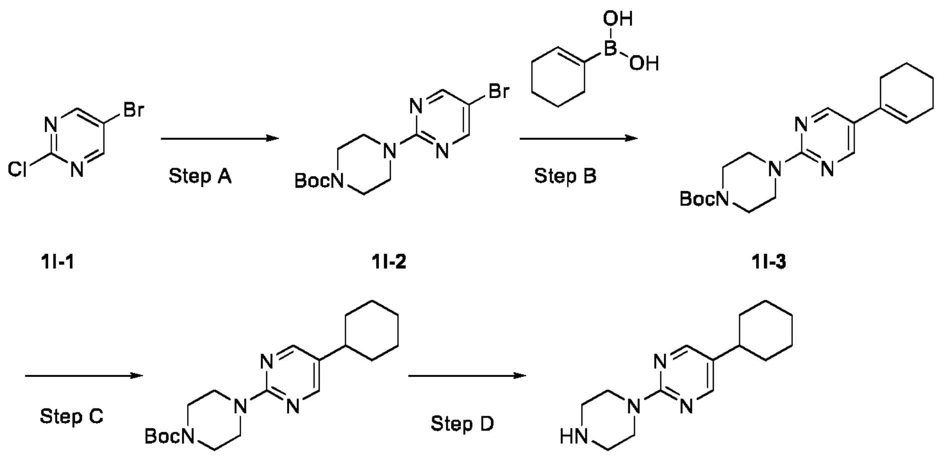


1I-1 1I-2 1I-3

1I-4 intermediate 1I

**Step A:** 5-Bromo-2-chloropyrimidine (1.0 g, 5.1 mmol) and *N*-Boc-piperazine (1.06 g, 5.61 mmol) were dissolved in *N*-methylmorpholine (5 mL), then potassium carbonate (1.4 g, 10.2 mol) was added thereto, and the reaction mixture was stirred at 80°C for 3 hours. The reaction mixture was cooled to room temperature, then poured into water, and stirred thoroughly. The precipitated white solid was then filtered, washed with water, and dried to obtain 1.7 g of *tert*-butyl

4-(5-pyrimidin-2-yl)piperazine-1-carboxylate.

**Step B:** *tert*-Butyl 4-(5-bromopyrimidin-2-yl)piperazine-1-carboxylate (300 mg, 0.87 mmol), (cyclohex-1-en-1-yl)boronic acid (142 mg, 1.14 mmol), [1,1'-bis(diphenylphosphino)ferrocene]palladium(II) dichloride (63 mg, 0.087 mmol), and cesium carbonate (566 mg, 1.74 mmol) were dissolved in 1,4-dioxane (20 mL) under nitrogen atmosphere, and the reaction mixture was heated to 100°C and stirred overnight. The reaction mixture was concentrated under reduced pressure and extracted with ethyl acetate (3 × 30 mL). The organic phases were combined, washed with saturated brine (100 mL), dried over anhydrous sodium sulfate, filtered, and concentrated. The resulting residue was purified by silica gel column chromatography to obtain 200 mg of *tert*-butyl 4-(5-(cyclohex-1-en-1-yl)pyrimidin-2-yl)piperazine-1-carboxylate.

MS (ESI) M/Z: 345.3 $[M+H]^+$.

**Step C:** *tert*-Butyl 4-(5-(cyclohex-1-en-1-yl)pyrimidin-2-yl)piperazine-1-carboxylate (100 mg, 0.28 mmol) was dissolved in ethanol (5 mL). 10% palladium on carbon (wet basis) (10 mg) was added to the above solution. After the reaction system was replaced with hydrogen three times, the reaction mixture was stirred at room temperature for 1 hour. The reaction mixture was filtered through diatomite, and the filter cake was washed with methanol (3 × 10 mL). The resulting organic phase was concentrated under reduced pressure to obtain 100 mg of tert-butyl 4-(5-cyclohexylpyrimidin-2-yl)piperazine-1-carboxylate.

MS (ESI) M/Z: 347.3 $[M+H]^+$.

**Step D:** *tert*-Butyl 4-(5-cyclohexylpyrimidin-2-yl)piperazine-1-carboxylate (100 mg, 0.29 mmol) was dissolved in dichloromethane (2 mL) at room temperature. Trifluoroacetic acid (1 mL) was then added to the above solution, and the reaction mixture was stirred at room temperature for 2 hours. The reaction mixture was concentrated under reduced pressure to obtain 71 mg of 5-cyclohexyl-2-(piperazin-1-yl)pyrimidine.

MS (ESI) M/Z: 247.1 $[M+H]^+$.

**Intermediate 1J**

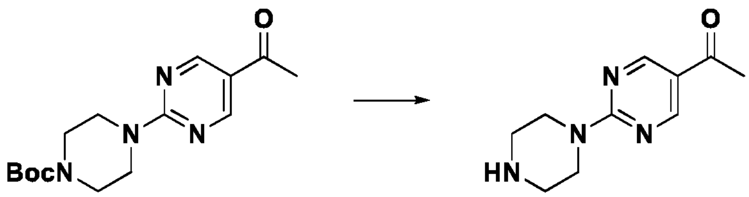

1F-4 intermediate 1J

*tert*-Butyl 4-(5-acetylpyrimidin-2-yl)piperazine-1-carboxylate (80 mg, 0.26 mmol) was dissolved in 1,4-dioxane (1 mL) at room temperature. A solution of hydrochloric acid in dioxane (1 mL) was then added to the above solution. The reaction mixture was stirred at room temperature for 3 hours. The reaction mixture was concentrated under reduced pressure to obtain 53.8 mg of 1-(2-(piperazin-1-yl)pyrimidin-5-yl)ethan-1-one (intermediate **1J**).

MS (ESI) M/Z: 207.2 $[M+H]^+$.

**Intermediate 1K**

**(*R*)-2-(4-(5-(Trifluoromethyl)pyrimidin-2-yl)piperazin-2-yl)acetonitrile**

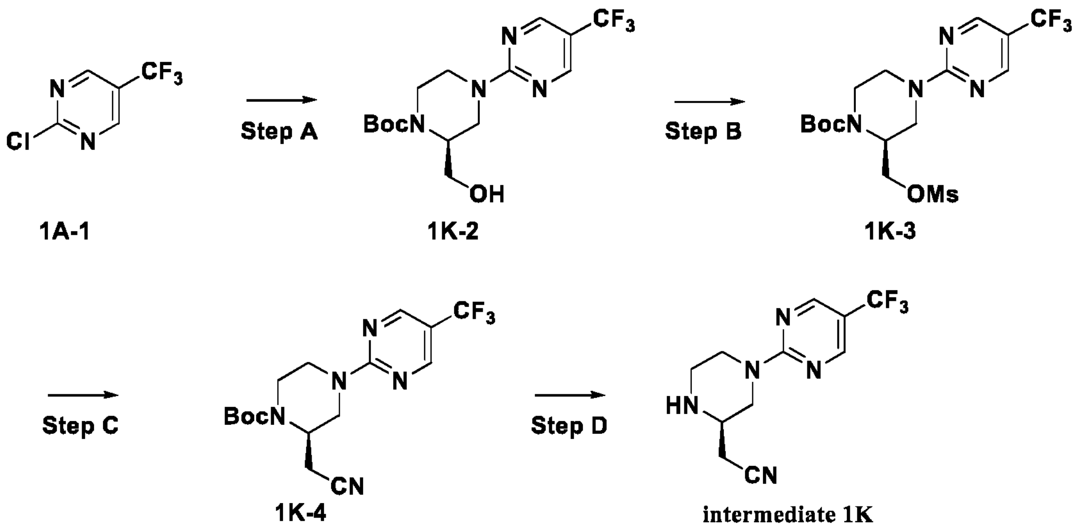


1A-1 1K-2 1K-3 1K-4 intermediate 1K

**Step A:** *tert*-Butyl (*S*)-2-(hydroxymethyl)piperazine-1-carboxylate (117 mg, 0.54 mmol) and potassium carbonate (76 mg, 0.54 mmol) were dissolved in *N*-methylpyrrolidone (2 mL). 2-Chloro-5-(trifluoromethyl)pyrimidine (100 mg, 0.54 mmol) was then added thereto. The reaction mixture was heated to 80°C and stirred for 2 hours, and added with water (20 mL) to quench. The mixture was extracted with ethyl acetate (5 mL × 3), and the organic phases were combined. The combined organic phases were sequentially washed with water (20 mL) and saturated brine (20 mL), then dried over anhydrous sodium sulfate, filtered, and finally concentrated under reduced pressure. The resulting residue was purified by silica gel column chromatography to obtain 140 mg of *tert*-butyl (S)-2-(hydroxymethyl)-4-(5-(trifluoromethyl)pyrimidin-2-yl)piperazine-1-carboxylate (**1K-2**).

$^1$H NMR (400 MHz, DMSO-*d*$_6$) δ 8.71 (s, 2H), 4.78 (t, *J* = 5.3 Hz, 1H), 4.69 (d, *J* = 13.2 Hz, 1H), 4.45 - 4.35 (m, 1H), 4.06 (brs, 1H), 3.88 - 3.78 (m, 1H), 3.37 - 3.34 (m, 2H), 3.28 (dd, *J* = 13.5, 4.3 Hz, 1H), 3.19 - 3.05 (m, 2H), 1.42 (s, 9H).

**Step B:** *tert*-Butyl (*S*)-2-(hydroxymethyl)-4-(5-(trifluoromethyl)pyrimidin-2-yl)piperazine-1-carboxylate (200 mg, 0.55 mmol) was dissolved in dichloromethane (10 mL) at room temperature. Triethylamine (167 mg, 1.65 mmol) was then added to the above solution under nitrogen atmosphere, and methanesulfonyl chloride (95 mg, 0.83 mmol) was slowly added dropwise to the above solution in an ice-water bath. The reaction mixture was stirred at room temperature for 2 hours, and added with water (30 mL) to quench. The mixture was extracted with dichloromethane (10 mL × 3), and the organic phases were combined. The combined organic phases were washed with saturated brine (30 mL). The organic phases were then dried over anhydrous sodium sulfate, filtered, and finally concentrated under reduced pressure. The resulting residue was purified by silica gel column chromatography to obtain 200 mg of *tert*-butyl (*S*)-2-((methylsulfonyl)oxy)methyl)-4-(5-(trifluoromethyl)pyrimidin-2-yl)piperazine-1-carboxylate (**1K-3**).

MS (ESI) M/Z: 385.0 [M+H-*t*-Bu]$^+$.

**Step C:** *tert*-Butyl (*S*)-2-((methylsulfonyl)oxy)methyl)-4-(5-(trifluoromethyl)pyrimidin-2-yl)piperazine-1-carboxylate (150 mg, 0.34 mmol) was dissolved in anhydrous tetrahydrofuran (80 mL) at room temperature. Trimethylsilyl cyanide (67.5 mg, 0.68 mmol) and tetrabutylammonium fluoride (0.68 mL, 0.68 mmol) were then added to the above solution. The reaction mixture was stirred at 80°C for 16 hours. The mixture was added with water (30 mL) to quench, extracted with ethyl acetate (10 mL × 3), and the organic phases were combined. The combined organic phases were washed with saturated brine (30 mL). The organic phases were then dried over anhydrous sodium sulfate, filtered, and finally concentrated under reduced pressure. The resulting residue was purified by silica gel column chromatography to obtain 72 mg of *tert*-butyl (*R*)-2-(cyanomethyl)-4-(5-(trifluoromethyl)pyrimidin-2-yl)piperazine-1-carboxylate (**1K-4**).

$^1$H NMR (400 MHz, DMSO-*d*$_6$) δ 8.74 (s, 2H), 4.61 - 4.49 (m, 3H), 3.97 - 3.87 (m, 1H), 3.35 - 3.33 (m, 1H), 3.10 - 3.03 (m, 1H), 2.92 - 2.82 (m, 1H), 2.79 - 2.71 (m, 1H), 2.03 - 1.95 (m, 1H), 1.44 (s, 9H).

**Step D:** *tert*-Butyl (*R*)-2-(cyanomethyl)-4-(5-(trifluoromethyl)pyrimidin-2-yl)piperazine-1-carboxylate (72 mg, 0.19 mmol) was dissolved in dichloromethane (3 mL) at room temperature. Trifluoroacetic acid (0.6 mL) was then added to the above solution. The reaction mixture was stirred at room temperature for 2 hours. The reaction mixture was concentrated under reduced pressure to obtain 54 mg of (*R*)-2-(4-(5-(trifluoromethyl)pyrimidin-2-yl)piperazin-2-yl)acetonitrile (intermediate **1K**).

MS (ESI) M/Z: 272.1 [M+H]$^+$.

**Intermediate 1L**

**(*S*)-2-(3-(Methoxymethyl)piperazin-1-yl)-5-(trifluoromethyl)pyrimidine**

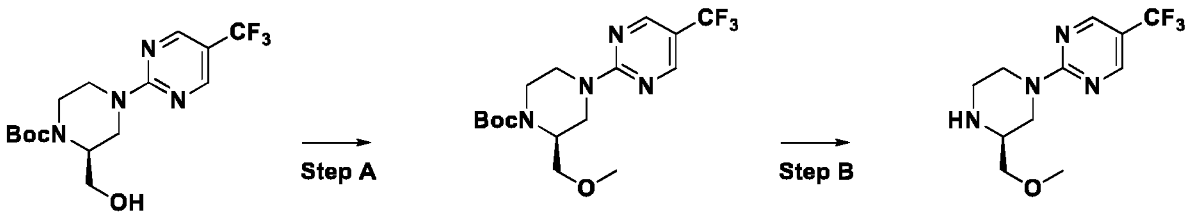


1K-2 1L-2 intermediate 1L

**Step B:** Compound *tert*-butyl (*S*)-2-(hydroxymethyl)-4-(5-(trifluoromethyl)pyrimidin-2-yl)piperazine-1-carboxylate (100 mg, 0.28 mmol) was dissolved in *N,N*-dimethylformamide (2 mL), then iodomethane (58.2 mg, 0.41 mmol) was added thereto, and sodium hydride (16.6 mg, 0.41 mmol) was added thereto under cooling in an ice bath. The reaction mixture was stirred at room temperature for 2 hours, and added with water (20 mL) to quench. The mixture was extracted with ethyl acetate (5 mL × 3), and the organic phases were combined. The combined organic phases were first washed with saturated brine (20 mL), then dried over anhydrous sodium sulfate, filtered, and finally concentrated under reduced pressure. The resulting residue was purified by silica gel column chromatography to obtain 100 mg of compound *tert*-butyl (*S*)-2-(methoxymethyl)-4-(5-(trifluoromethyl)pyrimidin-2-yl)piperazine-1-carboxylate **(1L-2).**
MS (ESI) M/Z: 321.1 $[M+H\text{-}t\text{-}Bu]^+$.
**Step:** *tert*-Butyl (*S*)-2-(methoxymethyl)-4-(5-(trifluoromethyl)pyrimidin-2-yl)piperazine-1-carboxylate (100 mg, 0.26 mmol) was dissolved in dichloromethane (2 mL) at room temperature. Trifluoroacetic acid (1 mL) was then added to the above solution. The reaction mixture was stirred at room temperature for 16 hours. The reaction mixture was concentrated under reduced pressure to obtain 73 mg of (*S*)-2-(3-(methoxymethyl)piperazin-1-yl)-5-(trifluoromethyl)pyrimidine (intermediate **1L**).
MS (ESI) M/Z: 277.2 $[M+H]^+$.

**Example 1**

**2-(6-Oxo-5-(trifluoromethyl)-1,6-dihydropyridazin-3-yl)-N (2-(4-(5-(trifluoromethyl)pyrimidin-2-yl)piperazin-1-yl)ethyl)acetamide**

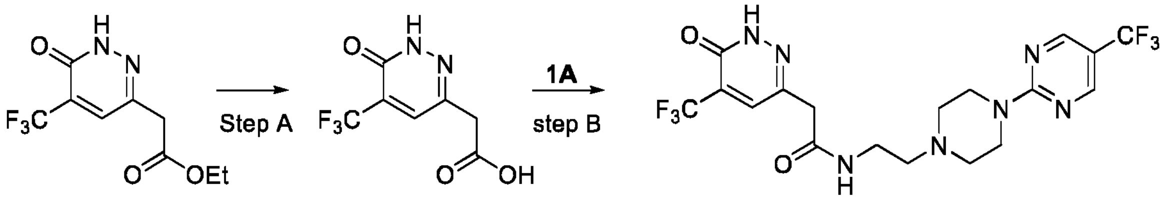


1B 1-1 compound 1

**Step A:** Ethyl 2-(6-oxo-5-(trifluoromethyl)-1,6-dihydropyridazin-3-yl)acetate (100 mg, 0.4 mmol) was dissolved in tetrahydrofuran (2 mL), then 2 *M* sodium hydroxide solution (0.2 mL) was added thereto, and the reaction mixture was stirred at room temperature for 2 hours. The reaction mixture was added with dilute hydrochloric acid to adjust the pH to 3 to 4, and extracted with ethyl acetate (20 mL × 3). The organic phase was washed with saturated brine (20 mL), dried over anhydrous sodium sulfate, filtered, and finally concentrated under reduced pressure to obtain 81 mg of 2-(6-oxo-5-(trifluoromethyl)-1,6-dihydropyridazin-3-yl)acetic acid (**1-1**), which was directly used in the next reaction step.
MS (ESI) M/Z: 222.9 $[M+H]^+$.
**Step B:** 2-(6-Oxo-5-(trifluoromethyl)-1,6-dihydropyridazin-3-yl)acetic acid (81 mg, 0.36 mmol) was dissolved in dichloromethane (5 mL) and *N,N*-dimethylformamide (5 mL) at room temperature. 2-(4-(5-(Trifluoromethyl)pyrimidin-2-yl)piperazin-1-yl)ethan-1-amine (**1A,** 100 mg, 0.36 mmol), HATU (205 mg, 0.54 mmol), and *N,N*-diisopropylethylamine

(140 mg, 1.1 mmol) were then sequentially added to the above solution in an ice-water bath. The reaction mixture was stirred at room temperature for 2 hours, added with dilute hydrochloric acid to adjust the pH to neutrality, and the aqueous phase was extracted with dichloromethane (50 mL × 3). The organic phase was washed with saturated brine (20 mL), then dried over anhydrous sodium sulfate, filtered, and finally concentrated under reduced pressure. The resulting residue was purified by silica gel column chromatography to obtain 43 mg of 2-(6-oxo-5-(trifluoromethyl)-1,6-dihydropyridazin-3-yl)-*N*-(2-(4-(5-(trifluoromethyl)pyrimidin-2-yl)piperazin-1-yl)ethyl)acetamide (compound **1**).

MS (ESI) M/Z: 478.1 $[M-H]^-$.

$^1H$ NMR (400 MHz, DMSO-$d_6$) δ 13.58 (s, 1H), 8.69 (s, 2H), 8.15 (t, *J* = 5.0 Hz, 1H), 7.88 (s, 1H), 3.81 (brs, 4H), 3.56 (s, 2H), 3.23 (q, *J* = 6.2 Hz, 2H), 2.48 - 2.35 (m, 6H).

**Example 2**

**3-(2-(3-Oxo-3-(4-(5-(trifluoromethyl)pyrimidin-2-yl)piperazin-1-yl)propoxy)ethyl)benzamide**

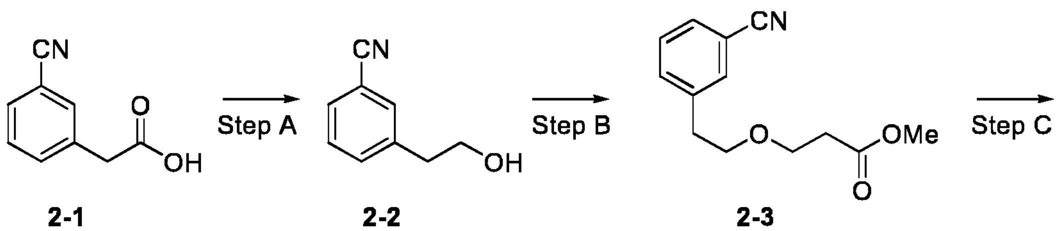


**2-1** **2-2** **2-3**

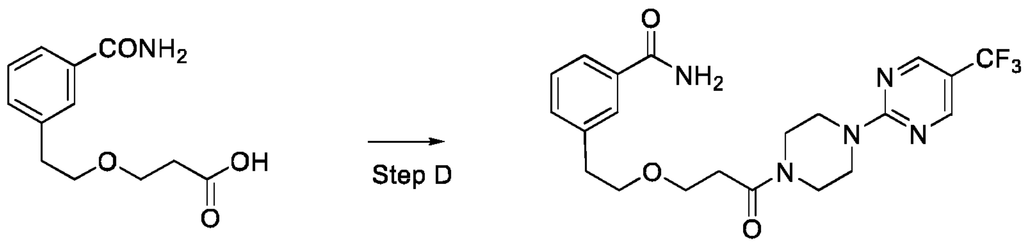


**2-4** **compound 2**

**Step A:** 3-Cyanophenylacetic acid (1.0 g, 6.2 mmol) was dissolved in anhydrous tetrahydrofuran (50 mL), then sodium borohydride (354 mg, 9.3 mmol) was added thereto in batches in an ice-water bath, and boron trifluoride diethyl etherate (1.3 g, 9.3 mmol) was added dropwise thereto. After the dropwise addition was completed, the reaction mixture was stirred at room temperature for 3 hours. The reaction mixture was cooled in an ice bath, added dropwise with dilute hydrochloric acid to quench, stirred at room temperature for another 30 minutes, and the aqueous phase was extracted with ethyl acetate (100 mL × 5). The organic phases were combined, first washed with saturated brine (30 mL × 2), then dried over anhydrous sodium sulfate, filtered, and finally concentrated under reduced pressure to obtain 620 mg of 3-(2-hydroxyethyl)benzonitrile (**2-2**).

MS (ESI) M/Z: 146.1 $[M-H]^-$.

**Step B:** 3-(2-Hydroxyethyl)benzonitrile (500 mg, 3.4 mmol) was dissolved in acetonitrile (20 mL), then methyl acrylate (440 mg, 5.1 mmol) and cesium carbonate (3.3 g, 10.2 mmol) were added thereto, and the reaction mixture was stirred at room temperature overnight. The reaction mixture was added with water to quench, and extracted with ethyl acetate (20 mL × 3). The organic phase was washed with saturated brine (20 mL), then dried over anhydrous sodium sulfate, filtered, and finally concentrated under reduced pressure to obtain 660 mg of methyl 3-(3-cyanophenethoxy)propanoate (**2-3**), which was directly used in the next reaction step.

MS (ESI) M/Z: 234.2 $[M+H]^+$.

**Step C:** Methyl 3-(3-cyanophenethoxy)propanoate (400 mg, 1.7 mmol) was dissolved in tetrahydrofuran (5 mL), then 1 *M* sodium hydroxide aqueous solution (5 mL, 5 mmol) was added thereto, and the reaction mixture was heated to 50°C and stirred overnight. The reaction mixture was cooled to room temperature, poured into water, and added with citric acid to adjust the pH to 3 to 4. The aqueous phase was extracted with ethyl acetate (50 mL × 3), and the organic phases were combined. The combined organic phases were first washed with saturated brine (20 mL × 2), then dried over anhydrous sodium sulfate, filtered, and concentrated under reduced pressure to obtain 370 mg of 3-(3-carbamoylphenethoxy)propanoic acid (**2-4**).

MS (ESI) M/Z: 239.0 $[M+H]^+$.

**Step D:** 3-(3-Carbamoylphenethoxy)propanoic acid (300 mg, 1.2 mmol) was dissolved in dichloromethane (5 mL) and *N,N*-dimethylformamide (5 mL) at room temperature. 2-(Piperazin-1-yl)-5-(trifluoromethyl)pyrimidine (290 mg, 1.2 mmol), HATU (500 mg, 1.3 mmol), and *N,N*-diisopropylethylamine (460 mg, 3.6 mmol) were then sequentially added to the above solution in an ice-water bath. The reaction mixture was stirred at room temperature for another 2 hours. The reaction mixture was added with dilute hydrochloric acid to adjust the pH to 5 to 6, and then extracted with ethyl acetate (20 mL × 3). The organic phase was washed with saturated brine (20 mL), then dried over anhydrous sodium sulfate, filtered, and finally concentrated under reduced pressure. The resulting residue was purified by preparative high performance liquid chromatography to obtain 33.2 mg of 3-(2-(3-oxo-3-(4-(5-(trifluoromethyl)pyrimidin-2-yl)piperazin-1-yl)propoxy)ethyl)benzamide (compound 2).

MS (ESI) M/Z: 452.0 $[M+H]^+$.

$^1H$ NMR (400 MHz, DMSO-$d_6$) δ 8.73 (s, 2H), 7.91 (s, 1H), 7.74 (s, 1H), 7.68 (d, *J* = 7.5 Hz, 1H), 7.40 - 7.29 (m, 3H), 3.86 - 3.75 (m, 4H), 3.69 - 3.59 (m, 4H), 3.58 - 3.51 (m, 4H), 2.84 (t, *J* = 6.9 Hz, 2H), 2.61 (t, *J* = 6.6 Hz, 2H).

**Example 3**

**2-(6-Oxo-5-(trifluoromethyl)-1,6-dihydropyridin-3-yl)ethyl 4-(5-(trifluoromethyl)pyrimidin-2-yl)piperazine-1-carboxylate**

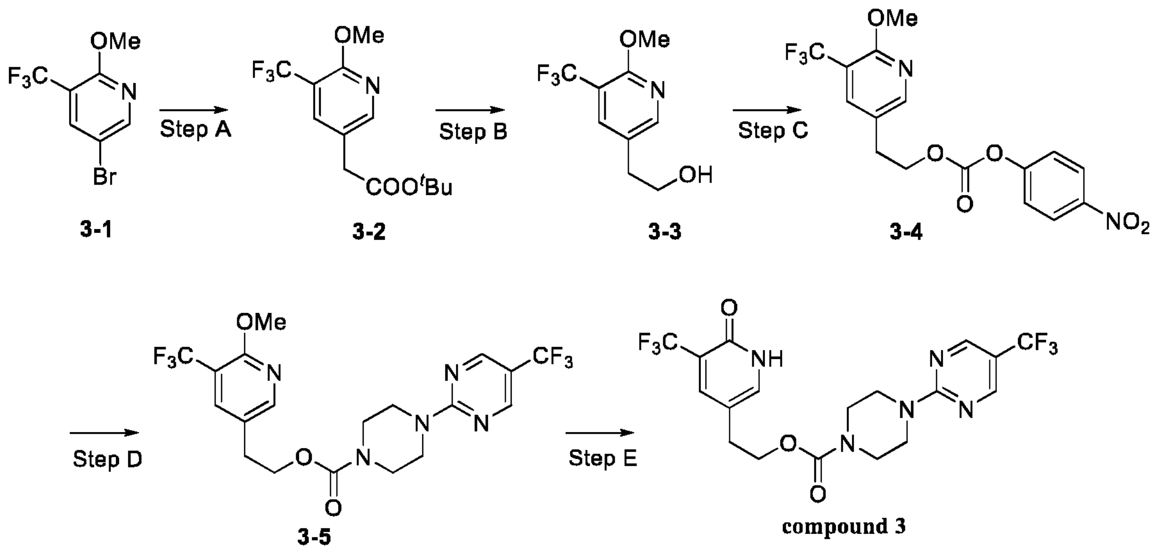

3-1 3-2 3-3 3-4 3-5 compound 3

**Step A:** Activated zinc powder (0.8 g, 12 mmol) was added to dry tetrahydrofuran (20 mL) under nitrogen atmosphere, then tert-butyl bromoacetate (1.5 g, 7.6 mmol) was added dropwise thereto under reflux, and the reaction mixture was refluxed for another 1 hour after initiation. The reaction mixture was added with 5-bromo-2-methoxy-3-(trifluoromethyl)pyridine (300 mg, 1.2 mmol), and then added with a catalytic amount of tris(dibenzylideneacetone)dipalladium ($Pd_2(dba)_3$) and 2-dicyclohexylphosphino-2',4',6'-triisopropylbiphenyl (X-Phos). The reaction mixture was then refluxed and stirred overnight. The reaction mixture was cooled to room temperature, and added with water to quench. The aqueous phase was extracted with ethyl acetate (100 mL × 3), and the organic phases were combined. The combined organic phases were first washed with saturated brine (30 mL × 2), then dried over anhydrous sodium sulfate, filtered, and concentrated under reduced pressure. The resulting residue was purified by silica gel column chromatography to obtain 350 mg of *tert*-butyl 2-(6-methoxy-5-(trifluoromethyl)pyridin-3-yl)acetate (**3-2**).

MS (ESI) M/Z: 292.1 $[M+H]^+$.

$^1H$ NMR (400 MHz, $CDCl_3$) δ 8.18 (d, *J* = 2.2 Hz, 1H), 7.82 (d, *J* = 2.4 Hz, 1H), 4.03 (s, 3H), 3.50 (s, 2H), 1.45 (s, 9H).

**Step B:** *tert*-Butyl 2-(6-methoxy-5-(trifluoromethyl)pyridin-3-yl)acetate (200 mg, 0.7 mmol) was dissolved in tetrahydrofuran (10 mL), then lithium aluminum hydride (60 mg, 1.5 mmol) was added thereto, and the reaction mixture was heated to reflux and stirred for 5 hours. The reaction mixture was cooled to room temperature, and added with saturated ammonium chloride solution to quench. The aqueous phase was extracted with ethyl acetate (50 mL × 3),

and the organic phases were combined. The combined organic phases were first washed with saturated brine (20 mL × 2), then dried over anhydrous sodium sulfate, filtered, and concentrated under reduced pressure. The resulting residue was purified by silica gel column chromatography to obtain 104 mg of 2-(6-methoxy-5-(trifluoromethyl)pyridin-3-yl)ethan-1-ol (**3-3**).

MS (ESI) M/Z: 221.9 $[M+H]^+$.

**Step C:** 2-(6-Methoxy-5-(trifluoromethyl)pyridin-3-yl)ethan-1-ol (75 mg, 0.34 mmol) was dissolved in *N,N*-dimethylformamide (2 mL), then bis(4-nitrophenyl) carbonate (206 mg, 0.68 mmol) and *N,N*-diisopropylethylamine (90 mg, 0.68 mmol) were added thereto, and the reaction mixture was heated to 80°C and stirred for 2 hours. The reaction mixture was cooled to room temperature, then added with water to quench, and extracted with ethyl acetate (20 mL × 3). The organic phase was washed with saturated brine (20 mL), then dried over anhydrous sodium sulfate, filtered, and finally concentrated under reduced pressure. The resulting residue was purified by silica gel column chromatography to obtain 130 mg of 2-(6-methoxy-5-(trifluoromethyl)pyridin-3-yl)ethyl (4-nitrophenyl) carbonate (**3-4**), which was directly used in the next reaction step.

MS (ESI) M/Z: 386.9 $[M+H]^+$.

**Step D:** 2-(6-Methoxy-5-(trifluoromethyl)pyridin-3-yl)ethyl (4-nitrophenyl) carbonate (130 mg, 0.34 mmol) was dissolved in *N,N*-dimethylformamide (2 mL) at room temperature, and 2-(piperazin-1-yl)-5-(trifluoromethyl)pyrimidine (80 mg, 0.34 mmol) and *N,N*-diisopropylethylamine (90 mg, 0.68 mmol) were added thereto. The reaction mixture was heated to 80°C and stirred for 2 hours. The reaction mixture was cooled to room temperature, then added with saturated ammonium chloride solution to quench, and the aqueous phase was extracted with dichloromethane (50 mL × 2). The organic phase was washed with saturated brine (20 mL), then dried over anhydrous sodium sulfate, filtered, and finally concentrated under reduced pressure to obtain 205 mg of 2-(6-methoxy-5-(trifluoromethyl)pyridin-3-yl)ethyl 4-(5-(trifluoromethyl)pyrimidin-2-yl)piperazine-1-carboxylate (**3-5**), which was directly used in the next reaction step.

MS (ESI) M/Z: 480.2 $[M+H]^+$.

**Step E:** 2-(6-Methoxy-5-(trifluoromethyl)pyridin-3-yl)ethyl 4-(5-(trifluoromethyl)pyrimidin-2-yl)piperazine-1-carboxylate (200 mg, 0.2 mmol) was dissolved in dichloromethane (5 mL) at room temperature. Iodotrimethylsilane (400 mg, 2 mmol) was then added to the above solution in an ice-water bath. The reaction mixture was stirred at room temperature for another 2 hours. The reaction mixture was added with ice water to quench under cooling in an ice bath, added with saturated sodium bicarbonate solution to adjust the pH to 7 to 8, and extracted with dichloromethane (20 mL × 3). The organic phase was washed with saturated brine (20 mL), then dried over anhydrous sodium sulfate, filtered, and finally concentrated under reduced pressure. The resulting residue was purified by preparative high performance liquid chromatography to obtain 5.6 mg of 2-(6-oxo-5-(trifluoromethyl)-1,6-dihydropyridin-3-yl)ethyl 4-(5-(trifluoromethyl)pyrimidin-2-yl)piperazine-1-carboxylate (compound **3**).

MS (ESI) M/Z: 466.4 $[M+H]^+$.

$^1$H NMR (400 MHz, DMSO-$d_6$) δ 12.21 (s, 1H), 8.73 (s, 2H), 7.90 (d, *J* = 2.0 Hz, 1H), 7.59 (d, *J* = 1.9 Hz, 1H), 4.16 (t, *J* = 6.3 Hz, 2H), 3.86 - 3.76 (m, 4H), 3.48 - 3.41 (m, 4H), 2.73 (t, *J* = 6.2 Hz, 2H).

**Example 4**

**2-(6-Oxo-5-(trifluoromethyl)-1,6-dihydropyridin-3-yl)-*N*-(2-(4-(5-(trifluoromethyl)pyrimidin-2-yl)piperazin-1-yl)ethyl)acetamide**

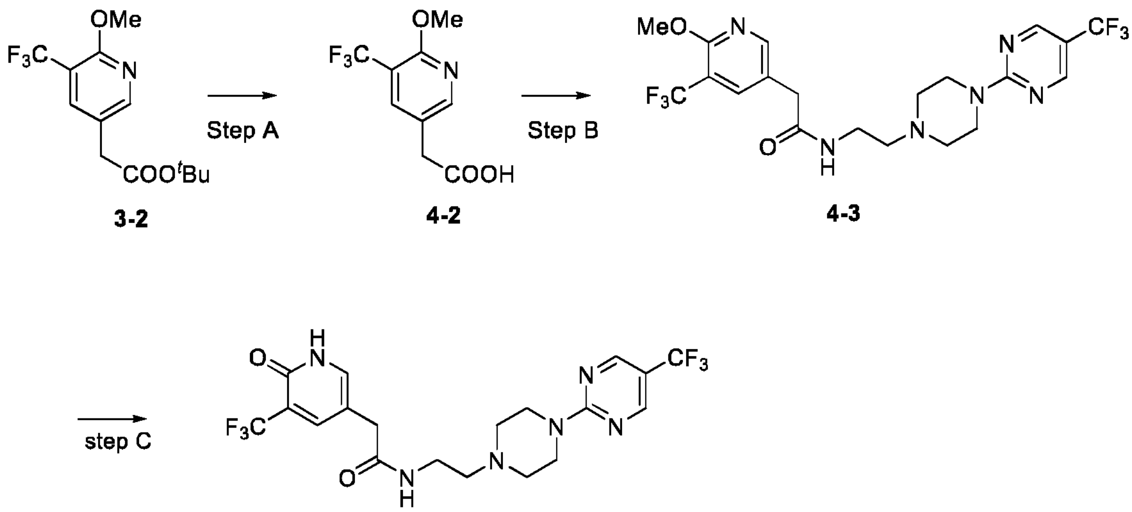


3-2 4-2 4-3

compound 4

**Step A:** *tert*-Butyl 2-(6-methoxy-5-(trifluoromethyl)pyridin-3-yl)acetate (200 mg, 0.7 mmol) was dissolved in dichloromethane (2 mL), then trifluoroacetic acid (2 mL) was added thereto, and the reaction mixture was stirred at room temperature for 1 hour. The reaction mixture was concentrated under reduced pressure to obtain 160 mg of 2-(6-methoxy-5-(trifluoromethyl)pyridin-3-yl)acetic acid (**4-2**).

MS (ESI) M/Z: 235.7 $[M+H]^+$.

**Step B:** 2-(6-Methoxy-5-(trifluoromethyl)pyridin-3-yl)acetic acid (160 mg, 0.69 mmol) was dissolved in dichloromethane (5 mL) and *N,N*-dimethylformamide (5 mL), and then 2-(4-(5-(trifluoromethyl)pyrimidin-2-yl)piperazin-1-yl)ethylamine (190 mg, 0.69 mmol), HATU (400 mg, 1.03 mmol), and *N,N*-diisopropylethylamine (270 mg, 2.07 mmol) were sequentially added to the above solution in an ice-water bath. The reaction mixture was stirred at room temperature for another 2 hours. The reaction mixture was added with water to quench, and then extracted with ethyl acetate (20 mL $\times$ 3). The organic phase was washed with saturated brine (20 mL), then dried over anhydrous sodium sulfate, filtered, and finally concentrated under reduced pressure. The resulting residue was purified by silica gel column chromatography to obtain 230 mg of 2-(6-methoxy-5-(trifluoromethyl)pyridin-3-yl)-*N*-(2-(4-(5-(trifluoromethyl)pyrimidin-2-yl)piperazin-1-yl)ethyl)acetamide (**4-3**), which was directly used in the next reaction step.

MS (ESI) M/Z: 493.1 $[M+H]^+$.

**Step C:** 2-(6-Methoxy-5-(trifluoromethyl)pyridin-3-yl)-*N*-(2-(4-(5-(trifluoromethyl)pyrimidin-2-yl)piperazin-1-yl)ethyl)acetamide (200 mg, 0.4 mmol) was dissolved in dichloromethane (5 mL) at room temperature. Iodotrimethylsilane (800 mg, 4 mmol) was then added to the above solution under cooling in an ice-water bath. The reaction mixture was stirred at room temperature for another 2 hours. The reaction mixture was carefully added with ice water to quench in an ice bath, added with saturated sodium bicarbonate solution to adjust the pH to 7 to 8, and extracted with dichloromethane (20 mL $\times$ 3). The organic phase was washed with saturated brine (20 mL), then dried over anhydrous sodium sulfate, filtered, and finally concentrated under reduced pressure. The resulting residue was purified by preparative high performance liquid chromatography to obtain 23.2 mg of 2-(6-oxo-5-(trifluoromethyl)-1,6-dihydropyridin-3-yl)-N (2-(4-(5-(trifluoromethyl)pyrimidin-2-yl)piperazin-1-yl)ethyl)acetamide (compound **4**).

MS (ESI) M/Z: 479.0 $[M+H]^+$.

$^1$H NMR (400 MHz, DMSO-$d_6$) $\delta$ 12.25 (s, 1H), 9.83 (s, 1H), 8.23 (s, 2H), 8.27 (t, *J* = 5.7 Hz, 1H), 7.84 (d, *J* = 2.1 Hz, 1H), 7.56 (s, 1H), 4.88 - 4.63 (m, 2H), 3.70 - 3.52 (m, 2H), 3.50 - 3.34 (m, 4H), 3.32 (s, 2H), 3.25 - 3.06 (m, 4H).

**Example 5**

***N*-(2-Oxo-2-(4-(5-(trifluoromethyl)pyrimidin-2-yl)piperazin-1-yl)ethyl)-2-(6-oxo-5-(trifluoromethyl)-1,6-dihydropyridazin-3-yl)acetamide**

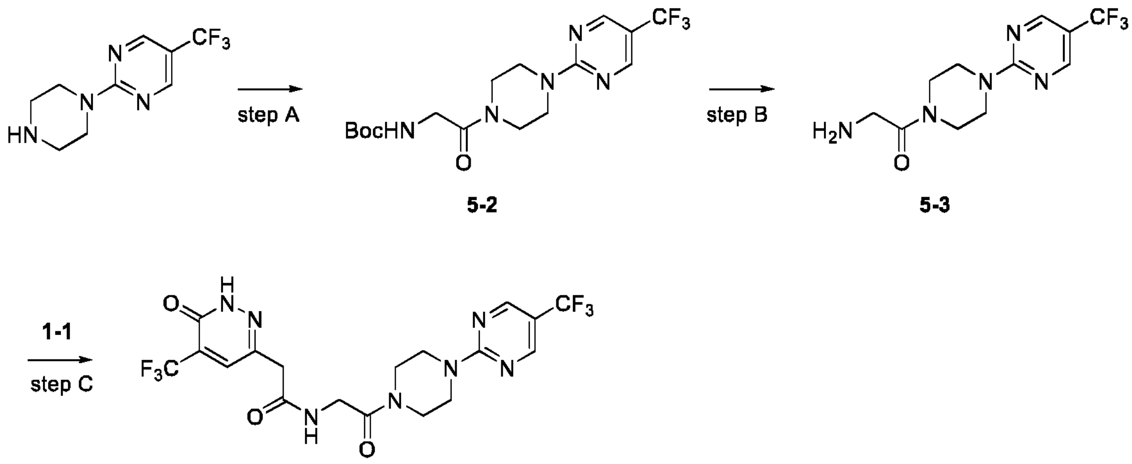


5-2

5-3

compound 5

**Step A:** *N*-Boc-glycine (160 mg, 0.86 mmol) was dissolved in dichloromethane (5 mL) and *N,N*-dimethylformamide (5 mL) at room temperature. 2-(Piperazin-1-yl)-5-(trifluoromethyl)pyrimidine (200 mg, 0.86 mmol), HATU (490 mg, 1.29 mmol), and *N,N*-diisopropylethylamine (350 mg, 2.58 mmol) were then sequentially added to the above solution in an ice-water bath. The reaction mixture was stirred at room temperature for another 2 hours. The reaction mixture was added with water to quench, then the aqueous phase was extracted with dichloromethane (50 mL × 3), and the organic phases were combined. The combined organic phases were first washed with saturated brine (30 mL × 2), then dried over anhydrous sodium sulfate, filtered, and finally concentrated under reduced pressure. The resulting residue was purified by silica gel column chromatography to obtain 237 mg of *tert*-butyl 2-oxo-2-(4-(5-(trifluoromethyl)pyrimidin-2-yl)piperazin-1-yl)ethylcarbamate (**5-2**).

MS (ESI) M/Z: 412.0 $[M+Na]^+$.

**Step B:** *tert*-Butyl 2-oxo-2-(4-(5-(trifluoromethyl)pyrimidin-2-yl)piperazin-1-yl)ethylcarbamate (237 mg, 0.60 mmol) was dissolved in dichloromethane (2 mL), then trifluoroacetic acid (2 mL) was added thereto in an ice bath, and the reaction mixture was stirred at room temperature for 2 hours. The reaction mixture was concentrated under reduced pressure, and then neutralized with saturated sodium bicarbonate solution. The aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure to obtain 200 mg of 2-amino-1-(4-(5-(trifluoromethyl)pyrimidin-2-yl)piperazin-1-yl)ethan-1-one (**5-3**).

MS (ESI) M/Z: 290.2 $[M+H]^+$.

**Step C:** 2-(6-Oxo-5-(trifluoromethyl)-1,6-dihydropyridin-3-yl)acetic acid (150 mg, 0.59 mmol) was dissolved in dichloromethane (5 mL) and *N,N*-dimethylformamide (5 mL) at room temperature. 2-Amino-1-(4-(5-(trifluoromethyl)pyrimidin-2-yl)piperazin-1-yl)ethan-1-one (100 mg, 0.36 mmol), HATU (205 mg, 0.54 mmol), and *N,N*-diisopropylethylamine (140 mg, 1.1 mmol) were then sequentially added to the above solution in an ice-water bath. The reaction mixture was stirred at room temperature for another 2 hours. The reaction mixture was added with dilute hydrochloric acid to adjust the pH to neutrality, and the aqueous phase was extracted with dichloromethane (50 mL × 3). The organic phase was washed with saturated brine (20 mL), then dried over anhydrous sodium sulfate, filtered, and finally concentrated under reduced pressure. The resulting residue was purified by silica gel column chromatography to obtain 43 mg of *N*-(2-oxo-2-(4-(5-(trifluoromethyl)pyrimidin-2-yl)piperazin-1-yl)ethyl)-2-(6-oxo-5-(trifluoromethyl)-1,6-dihydropyridazin-3-yl)acetamide (compound **5**).

MS (ESI) M/Z: 494.2 $[M+H]^+$.

$^1H$ NMR (400 MHz, DMSO-$d_6$) δ 13.60 (s, 1H), 8.74 (s, 2H), 8.41 (t, *J* = 5.3 Hz, 1H), 7.91 (s, 1H), 4.08 - 4.02 (m, 2H), 3.91 - 3.85 (m, 2H), 3.85 - 3.79 (m, 2H), 3.66 (s, 2H), 3.60 - 3.52 (m, 4H).

## Example 6

**2-(6-Oxo-5-(trifluoromethyl)-1,6-dihydropyridin-3-yl)ethyl (2-(4-(5-(trifluoromethyl)pyrimidin-2-yl)piperazin-1-yl)ethyl)carbamate**

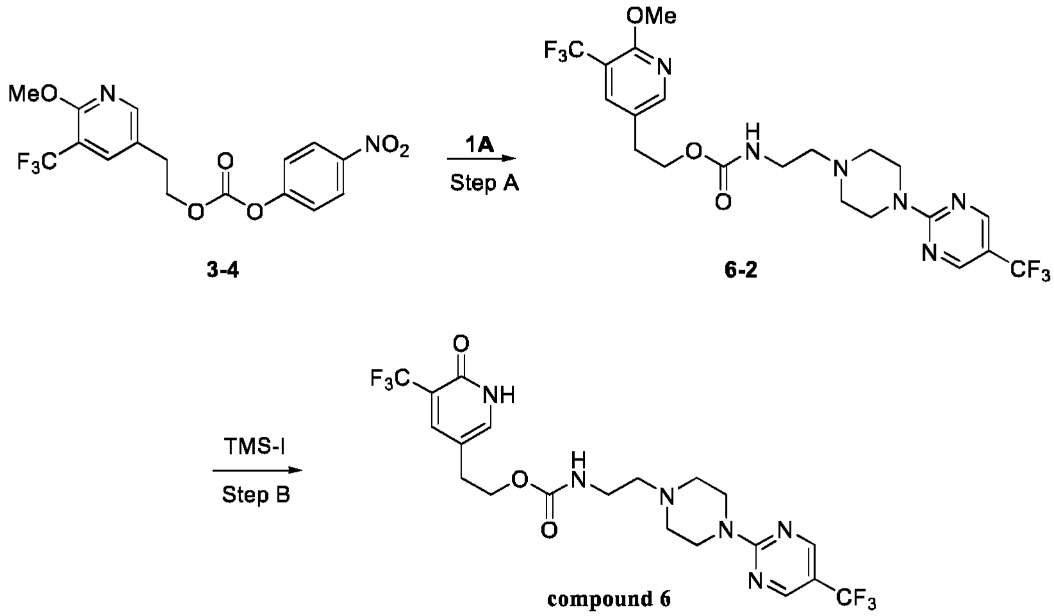


3-4

6-2

compound 6

**Step A:** 2-(6-Methoxy-5-(trifluoromethyl)pyridin-3-yl)ethyl (4-nitrophenyl) carbonate (130 mg, 0.34 mmol) was dissolved in *N,N*-dimethylformamide (2 mL) at room temperature, and 2-(4-(5-(trifluoromethyl)pyrimidin-2-yl)piperazin-1-yl)ethylamine (95 mg, 0.34 mmol) and *N,N*-diisopropylethylamine (90 mg, 0.68 mmol) were added thereto. The reaction mixture was heated to 80°C and stirred for 2 hours. The reaction mixture was cooled to room temperature, then added with saturated ammonium chloride solution to quench, and the aqueous phase was extracted with dichloromethane (50 mL $\times$ 2). The organic phase was washed with saturated brine (20 mL), then dried over anhydrous sodium sulfate, filtered, and finally concentrated under reduced pressure. The resulting residue was purified by silica gel column chromatography to obtain 60 mg of 2-(6-methoxy-5-(trifluoromethyl)pyridin-3-yl)ethyl (2-(4-(5-(trifluoromethyl)pyrimidin-2-yl)piperazin-1-yl)ethyl)carbamate (**6-2**), which was directly used in the next reaction step.

MS (ESI) M/Z: 523.0 $[M+H]^+$.

**Step B:** 2-(6-Methoxy-5-(trifluoromethyl)pyridin-3-yl)ethyl (2-(4-(5-(trifluoromethyl)pyrimidin-2-yl)piperazin-1-yl)ethyl)carbamate (60 mg, 0.11 mmol) was dissolved in dichloromethane (5 mL) at room temperature. Iodotrimethylsilane (220 mg, 1.1 mmol) was then added to the above solution in an ice-water bath. The reaction mixture was stirred at room temperature for another 2 hours. The reaction mixture was carefully added with ice water to quench in an ice bath, added with saturated sodium bicarbonate solution to adjust the pH to 7 to 8, and extracted with dichloromethane (20 mL $\times$ 3). The organic phase was washed with saturated brine (20 mL), then dried over anhydrous sodium sulfate, filtered, and finally concentrated under reduced pressure. The resulting residue was purified by preparative high performance liquid chromatography to obtain 7 mg of 2-(6-oxo-5-(trifluoromethyl)-1,6-dihydropyridin-3-yl)ethyl (2-(4-(5-(trifluoromethyl)pyrimidin-2-yl)piperazin-1-yl)ethyl)carbamate (compound **6**).

MS (ESI) M/Z: 509.0 $[M+H]^+$.

$^1$H NMR (400 MHz, DMSO-$d_6$) $\delta$ 8.69 (s, 2H), 7.87 (s, 1H), 7.54 (s, 1H), 7.08 (t, *J* = 5.2 Hz, 1H), 4.50 - 4.44 (m, 2H), 4.11 - 4.05 (m, 2H), 3.85 - 3.76 (m, 4H), 3.15 - 3.06 (m, 4H), 2.46 - 2.41 (m, 4H).

## Example 7

**(*S*)-5-(2-((2-Oxo-2-(4-(5-(trifluoromethyl)pyrimidin-2-yl)piperazin-1-yl)ethoxy)methyl)pyrrolidin-1-yl)-3-(trifluoromethyl)pyridin-2(1*H*)-one**

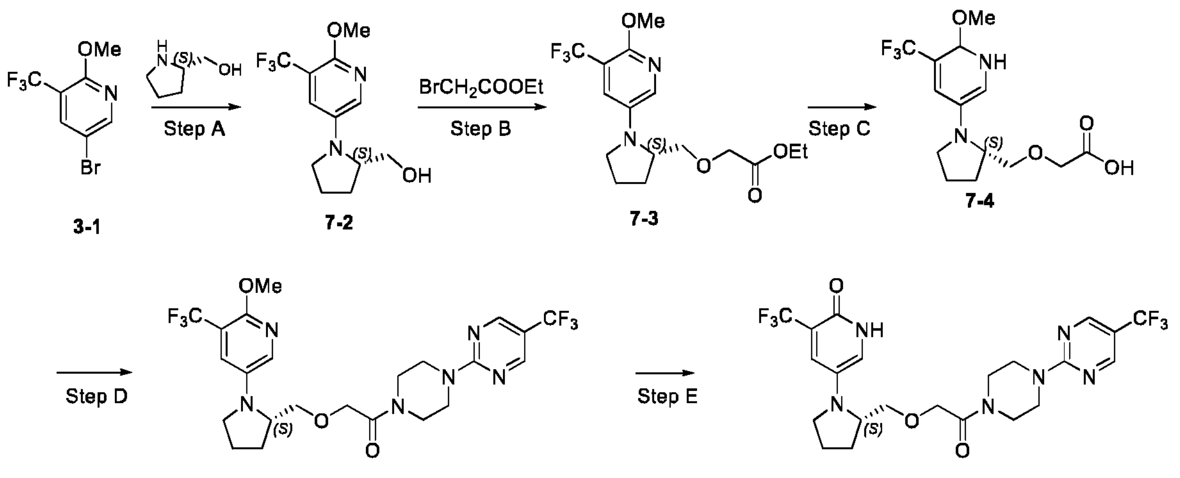


3-1 7-2 7-3 7-4 7-5 compound 7

**Step A:** 5-Bromo-2-methoxy-3-(trifluoromethyl)pyridine (500 mg, 2.0 mmol) was added to *L*-prolinol (5 mL), then cuprous chloride (100 mg, 1 mmol) and potassium hydroxide (224 mg, 4 mmol) were added thereto, and the reaction mixture was heated to 100°C under microwave irradiation for 2 hours. The reaction mixture was cooled to room temperature, and added with water to quench. The aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure. The resulting residue was purified by silica gel column chromatography to obtain 110 mg of (*S*)-(1-(6-methoxy-5-(trifluoromethyl)pyridin-3-yl)pyrrolidin-2-yl)methanol (**7-2**).

MS (ESI) M/Z: 276.8 $[M+H]^+$.

$^1H$ NMR (400 MHz, $CDCl_3$) $\delta$ 7.79 (s, 1H), 7.30 (d, *J* = 2.5 Hz, 1H), 3.96 (s, 3H), 3.83 - 3.76 (m, 1H), 3.71 - 3.60 (m, 2H), 3.57 - 3.50 (m, 1H), 3.14 - 3.05 (m, 1H), 2.16 - 1.96 (m, 4H).

**Step B:** (*S*)-(1-(6-Methoxy-5-(trifluoromethyl)pyridin-3-yl)pyrrolidin-2-yl)methanol (80 mg, 0.29 mmol) was dissolved in tetrahydrofuran (5 mL), then 60% sodium hydride (23 mg, 0.58 mmol) was added thereto under an ice bath, and the reaction mixture was stirred at room temperature for 30 minutes. Ethyl bromoacetate (90 mg, 0.54 mmol) was then added thereto, and the reaction mixture was stirred at room temperature for 4 hours. The reaction mixture was added with saturated ammonium chloride solution to quench. The aqueous phase was extracted with ethyl acetate (50 mL × 3), and the organic phases were combined. The combined organic phases were first washed with saturated brine (20 mL × 2), then dried over anhydrous sodium sulfate, filtered, and finally concentrated under reduced pressure. The resulting residue was purified by silica gel column chromatography to obtain 48 mg of ethyl (*S*)-2-((1-(6-methoxy-5-(trifluoromethyl)pyridin-3-yl)pyrrolidin-2-yl)methoxy)acetate (**7-3**).

MS (ESI) M/Z: 362.8 $[M+H]^+$.

**Step C:** Ethyl (*S*)-2-((1-(6-methoxy-5-(trifluoromethyl)pyridin-3-yl)pyrrolidin-2-yl)methoxy)acetate (48 mg, 0.13 mmol) was dissolved in tetrahydrofuran (2 mL), then 2 *M* sodium hydroxide solution (0.1 mL) was added thereto, and the reaction mixture was stirred at room temperature for 2 hours. The reaction mixture was added with dilute hydrochloric acid to adjust the pH to 2 to 3, and extracted with ethyl acetate (20 mL × 3). The organic phase was washed with saturated brine (20 mL), then dried over anhydrous sodium sulfate, filtered, and finally concentrated under reduced pressure to obtain 55 mg of (*S*)-2-((1-(6-methoxy-5-(trifluoromethyl)pyridin-3-yl)pyrrolidin-2-yl)methoxy)acetic acid (**7-4**), which was directly used in the next reaction step.

**Step D:** (*S*)-2-((1-(6-Methoxy-5-(trifluoromethyl)pyridin-3-yl)pyrrolidin-2-yl)methoxy)acetic acid (55 mg, 0.13 mmol) was dissolved in *N,N*-dimethylformamide (2 mL) at room temperature, and 2-(piperazin-1-yl)-5-(trifluoromethyl)pyrimidine (30 mg, 0.13 mmol), HATU (72 mg, 0.19 mmol), and *N,N*-diisopropylethylamine (52 mg, 0.40 mmol) were added thereto. The reaction mixture was stirred at room temperature for 2 hours. The reaction mixture was added with saturated ammonium chloride solution to quench, and the aqueous phase was extracted with dichloromethane (50 mL × 2). The organic phase was washed with saturated brine (20 mL), then dried over anhydrous sodium sulfate, filtered, and finally concentrated under reduced pressure. The resulting residue was purified by silica gel column chromatography to obtain 42 mg of (S)-2-((1-(6-methoxy-5-(trifluoromethyl)pyridin-3-yl)pyrrolidin-2-yl)methoxy)-1-(4-(5-(trifluoromethyl)pyrimidin-2-yl)piperazin-1-yl)ethan-1-one (**7-5**).

MS (ESI) M/Z: 548.9 $[M+H]^+$.

**Step E:**

(*S*)-2-((1-(6-Methoxy-5-(trifluoromethyl)pyridin-3-yl)pyrrolidin-2-yl)methoxy)-1-(4-(5-(trifluoromethyl)pyrimidin-2-yl)piperazin-1-yl)ethan-1-one (46 mg, 0.08 mmol) was dissolved in dichloromethane (5 mL) at room temperature. Iodotrimethylsilane (160 mg, 0.8 mmol) was then added to the above solution in an ice-water bath. The reaction mixture was stirred at room temperature for another 2 hours. The reaction mixture was carefully added with ice water to quench in an ice bath, added with saturated sodium bicarbonate solution to adjust the pH to 7 to 8, and extracted with dichloromethane (20 mL × 3). The organic phase was washed with saturated brine (20 mL), then dried over anhydrous sodium sulfate, filtered, and finally concentrated under reduced pressure. The resulting residue was purified by preparative silica gel plate chromatography to obtain 16.4 mg of (*S*)-5-(2-((2-oxo-2-(4-(5-(trifluoromethyl)pyrimidin-2-yl)piperazin-1-yl)ethoxy)methyl)pyrrolidin-1-yl)-3-(trifluoromethyl)pyridin-2(1*H*)-one (compound 7).

MS (ESI) M/Z: 534.8 $[M+H]^+$.

$^1H$ NMR (400 MHz, $CDCl_3$) δ 8.50 (s, 2H), 7.77 (s, 1H), 7.04 (s, 1H), 4.28 - 4.17 (m, 2H), 3.90 (brs, 4H), 3.82 - 3.75 (m, 1H), 3.73 - 3.63 (m, 2H), 3.56- 3.46 (m, 4H), 3.39 - 3.31 (m, 1H), 3.02 - 2.93 (m, 1H), 2.06 - 1.94 (m, 4H).

## Example 8

**(*R*)-5-(2-((2-Oxo-2-(4-(5-(trifluoromethyl)pyrimidin-2-yl)piperazin-1-yl)ethoxy)methyl)pyrrolidin-1-yl)-3-(trifluoromethyl)pyridin-2(1*H*)-one**

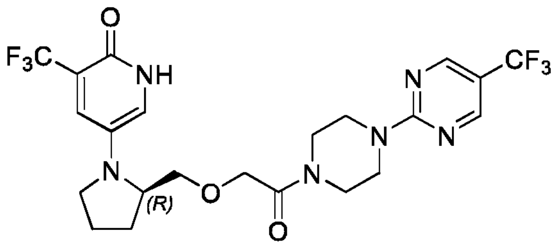

*D*-Prolinol was used as a solvent instead of *L*-prolinol with the preparation method referring to **example 7**.

MS (ESI) M/Z: 535.2 $[M+H]^+$.

$^1H$ NMR (400 MHz, $CDCl_3$) δ 8.50 (s, 2H), 7.78 (s, 1H), 7.10 (s, 1H), 4.29 - 4.17 (m, 2H), 3.91 (brs, 4H), 3.83 - 3.77 (m, 1H), 3.74 - 3.63 (m, 2H), 3.56 - 3.44 (m, 4H), 3.41 - 3.33 (m, 1H), 3.04 - 2.94 (m, 1H), 2.08 - 1.93 (m, 4H).

## Example 9

**(*S*)-5-(2-((3-Oxo-3-(4-(5-(trifluoromethyl)pyrimidin-2-yl)piperazin-1-yl)propoxy)methyl)pyrrolidin-1-yl)-3-(trifluoromethyl)pyridin-2(1*H*)-one**

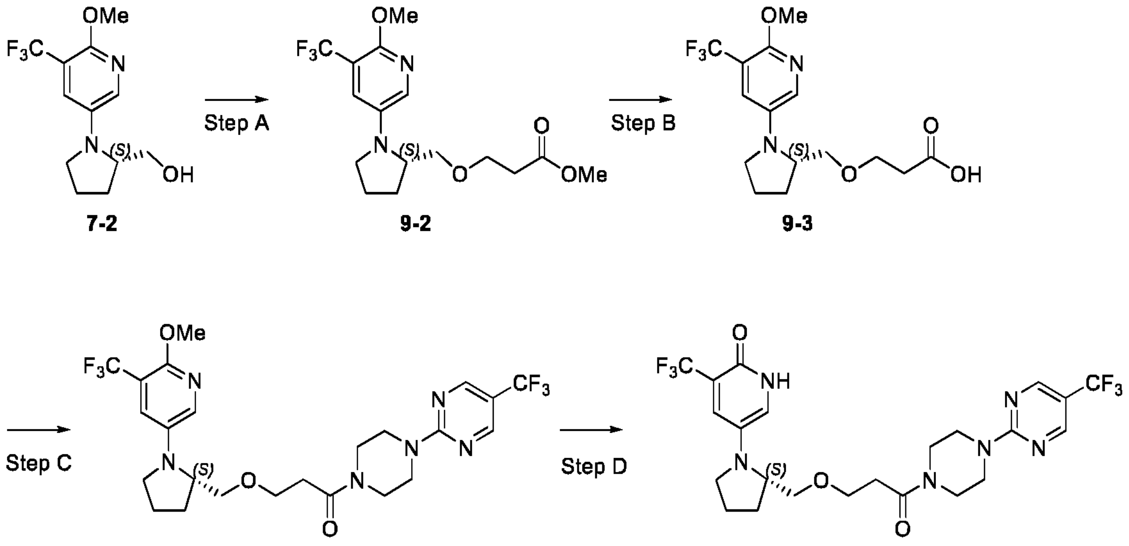


7-2 9-2 9-3

9-4 **compound 9**

**Step A:** (*S*)-(1-(6-Methoxy-5-(trifluoromethyl)pyridin-3-yl)pyrrolidin-2-yl)methanol (100 mg, 0.36 mmol) was dissolved in acetonitrile (5 mL), then methyl acrylate (46 mg, 0.53 mmol) and cesium carbonate (350 mg, 1.07 mmol) were added thereto, and the reaction mixture was heated to 50°C and stirred for 5 hours. The reaction mixture was added with saturated ammonium chloride solution to quench. The aqueous phase was extracted with ethyl acetate (50 mL × 3), and the organic phases were combined. The combined organic phases were first washed with saturated brine (20 mL × 2), then dried over anhydrous sodium sulfate, filtered, and finally concentrated under reduced pressure. The resulting residue was purified by silica gel column chromatography to obtain 50 mg of methyl (S)-3-((1-(6-methoxy-5-(trifluoromethyl)pyridin-3-yl)pyrrolidin-2-yl)methoxy)propanoate (**9-2**).

MS (ESI) M/Z: 363.9 $[M+H]^+$.

**Step B:** Methyl (*S*)-3-((1-(6-methoxy-5-(trifluoromethyl)pyridin-3-yl)pyrrolidin-2-yl)methoxy)propanoate (50 mg, 0.14 mmol) was dissolved in tetrahydrofuran (2 mL), then 2 M sodium hydroxide solution (0.1 mL) was added thereto, and the reaction mixture was stirred at room temperature for 2 hours. The reaction mixture was added with dilute hydrochloric acid to adjust the pH to 2 to 3, and extracted with ethyl acetate (20 mL × 3). The organic phase was washed with saturated brine (20 mL), then dried over anhydrous sodium sulfate, filtered, and finally concentrated under reduced pressure to obtain 50 mg of (*S*)-3-((1-(6-methoxy-5-(trifluoromethyl)pyridin-3-yl)pyrrolidin-2-yl)methoxy)propanoic acid (**9-3**), which was directly used in the next reaction step.

MS (ESI) M/Z: 347.2 $[M-H]^-$.

**Step C:** (*S*)-3-((1-(6-Methoxy-5-(trifluoromethyl)pyridin-3-yl)pyrrolidin-2-yl)methoxy)propanoic acid (50 mg, 0.14 mmol) was dissolved in *N,N*-dimethylformamide (2 mL) at room temperature, and 2-(piperazin-1-yl)-5-(trifluoromethyl)pyrimidine (35 mg, 0.14 mmol), HATU (80 mg, 0.21 mmol), and *N,N*-diisopropylethylamine (90 mg, 0.68 mmol) were added thereto. The reaction mixture was stirred at room temperature for 2 hours. The reaction mixture was added with saturated ammonium chloride solution to quench, and the aqueous phase was extracted with dichloromethane (50 mL × 2). The organic phase was washed with saturated brine (20 mL), then dried over anhydrous sodium sulfate, filtered, and finally concentrated under reduced pressure. The resulting residue was purified by silica gel column chromatography to obtain 35 mg of (*S*)-3-((1-(6-methoxy-5-(trifluoromethyl)pyridin-3-yl)pyrrolidin-2-yl)methoxy)-1-(4-(5-(trifluoromethyl)pyrimidin-2-yl)piperazin-1-yl)propan-1-one (**9-4**).

MS (ESI) M/Z: 563.3 $[M+H]^+$.

**Step D:** (*S*)-3-((1-(6-Methoxy-5-(trifluoromethyl)pyridin-3-yl)pyrrolidin-2-yl)methoxy)-1-(4-(5-(trifluoromethyl)pyrimidin-2-yl)piperazin-1-yl)propan-1-one (35 mg, 0.06 mmol) was dissolved in dichloromethane (5 mL) at room temperature. Iodotrimethylsilane (120 mg, 0.6 mmol) was then added to the above solution in an ice-water bath. The reaction mixture was stirred at room temperature for another 2 hours. The reaction mixture was carefully added with ice water to quench in an ice bath, added with saturated sodium bicarbonate solution to adjust the pH to 7 to 8, and extracted with dichloromethane (20 mL × 3). The organic phase was washed with saturated brine (20 mL), then dried over anhydrous sodium sulfate, filtered, and finally concentrated under reduced pressure. The resulting residue was purified by preparative high performance liquid chromatography to obtain 11 mg of (*S*)-5-(2-((3-oxo-3-(4-(5-(trifluoromethyl)pyrimidin-2-yl)piperazin-1-yl)propoxy)methyl)pyrrolidin-1-yl)-3-(trifluoromethyl)pyridin-2(1*H*)-one (compound **9**).

MS (ESI) M/Z: 549.0 $[M+H]^+$.

$^1H$ NMR (400 MHz, $CDCl_3$) δ 8.51 (s, 2H), 8.01 (brs, 1H), 7.21 (brs, 1H), 3.98 - 3.78 (m, 6H), 3.78 - 3.66 (m,

3H), 3.59 - 3.38 (m, 5H), 3.12 - 3.01 (m, 1H), 2.71 - 2.54 (m, 2H), 2.13 - 1.97 (m, 3H), 1.92 - 1.83 (m, 1H).

**Example 10**

**(*R*)-5-(2-((3-Oxo-3-(4-(5-(trifluoromethyl)pyrimidin-2-yl)piperazin-1-yl)propoxy)methyl)pyrrolidin-1-yl)-3-(trifluoromethyl)pyridin-2(1*H*)-one**

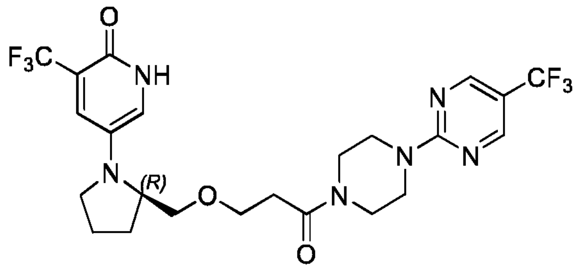

*D*-Prolinol was used as a solvent instead of *L*-prolinol with the preparation method referring to **example 9**.
MS (ESI) M/Z: 549.0 $[M+H]^+$.
$^1H$ NMR (400 MHz, $CDCl_3$) δ 8.50 (s, 2H), 7.97 (s, 1H), 7.16 (s, 1H), 3.97 - 3.86 (m, 4H), 3.86 - 3.78 (m, 2H), 3.78 - 3.67 (m, 3H), 3.58 - 3.35 (m, 5H), 3.09 - 2.98 (m, 1H), 2.71 - 2.53 (m, 2H), 2.11 - 1.96 (m, 3H), 1.94 - 1.83 (m, 1H).

**Example 11**

**(*S*)-5-(3-(3-Oxo-3-(4-(5-(trifluoromethyl)pyrimidin-2-yl)piperazin-1-yl)propoxy)pyrrolidin-1-yl)-3-(trifluoromethyl)pyridin-2(1*H*)-one**

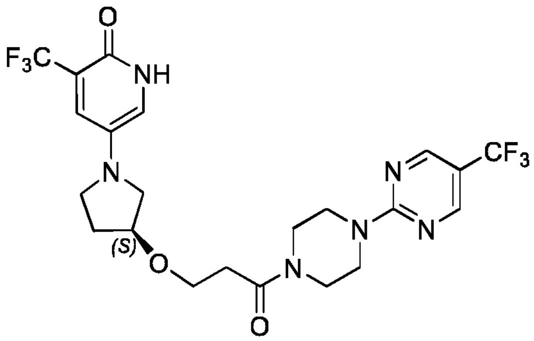

(*S*)-3-Pyrrolidinol was used as a solvent instead of L-prolinol with the preparation method referring to **example 9.**
MS (ESI) M/Z: 535.1 $[M+H]^+$.
$^1H$ NMR (400 MHz, $CDCl_3$) δ 8.49 (s, 2H), 7.52 (s, 1H), 6.93 (s, 1H), 4.24 (brs, 1H), 3.95 - 3.83 (m, 5H), 3.81 - 3.68 (m, 3H), 3.59 - 3.52 (m, 2H), 3.42 - 3.34 (m, 1H), 3.28 (q, $J$ = 7.9 Hz, 1H), 3.22 - 3.14 (m, 2H), 2.73 - 2.61 (m, 2H), 2.23 - 2.11 (m, 2H).

**Example 12**

**(*R*)-5-(3-(3-Oxo-3-(4-(5-(trifluoromethyl)pyrimidin-2-yl)piperazin-1-yl)propoxy)pyrrolidin-1-yl)-3-(trifluoromethyl)pyridin-2(1*H*)-one**

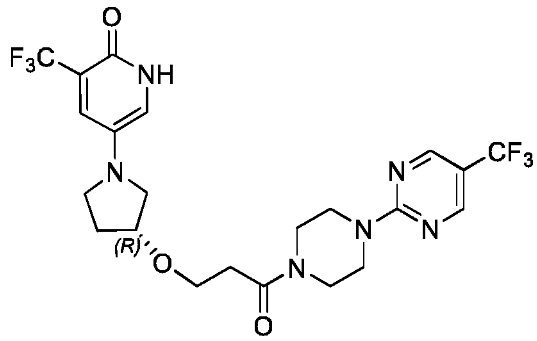

(*R*)-3-Pyrrolidinol was used as a solvent instead of *L*-prolinol with the preparation method referring to **example 9**.

MS (ESI) M/Z: 535.0 $[M+H]^+$.

$^1H$ NMR (400 MHz, DMSO-$d_6$) δ 8.71 (s, 2H), 7.48 (s, 1H), 6.96 (s, 1H), 4.17 (s, 1H), 3.88 - 3.74 (m, 4H), 3.71 - 3.63 (m, 2H), 3.34 - 3.28 (m, 2H), 3.20 - 3.06 (m, 4H), 2.62 (t, *J* = 6.8 Hz, 2H), 2.12 1.89 (m, 4H).

**Example 13**

**1-(2-(3-Oxo-3-(4-(5-(trifluoromethyl)pyrimidin-2-yl)piperazin-1-yl)propoxy)ethyl)-2,3,4,6-tetrahydropyrido[2,3-*d*]pyridazin-5(1*H*)-one**

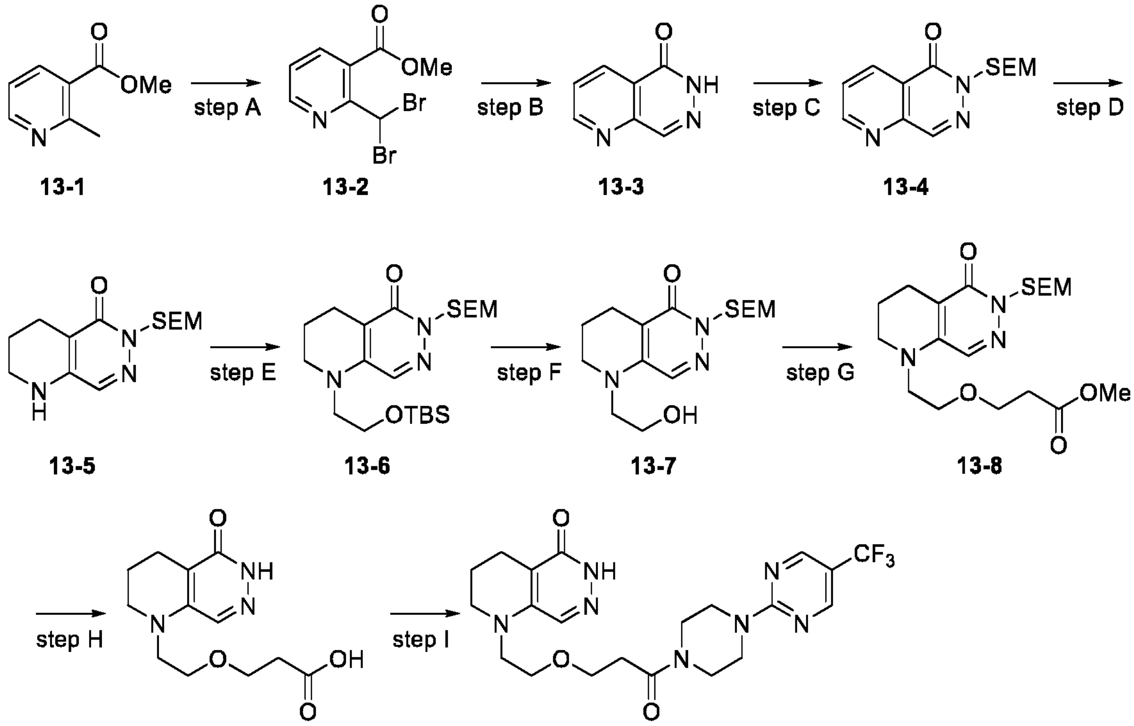


13-1 13-2 13-3 13-4 13-5 13-6 13-7 13-8 13-9 compound 13

**Step A:** Methyl 2-methylnicotinate (2.0 g, 13.2 mmol) was dissolved in carbon tetrachloride (50 mL), then N-bromosuccinimide (7.1 g, 40 mmol) and azobisisobutyronitrile (0.2 g, 1.3 mmol) were added thereto, and the reaction mixture was heated to 90°C under light irradiation and refluxed overnight. The reaction mixture was cooled to room temperature, and then diluted with dichloromethane (200 mL). The organic phase was washed with saturated brine (50 mL × 1), dried over anhydrous sodium sulfate, filtered, and concentrated under reduced pressure. The resulting residue was purified by silica gel column chromatography to obtain 3.5 g of methyl 2-(dibromomethyl)nicotinate (**13-2**).

MS (ESI) M/Z: 309.9 $[M+H]^+$.

$^1H$ NMR (400 MHz, $CDCl_3$) δ 8.90 (dd, *J* = 4.7, 1.7 Hz, 1H), 8.27 (dd, *J* = 8.0, 1.8 Hz, 1H), 8.02 (s, 1H), 7.38

(dd, *J* = 8.0, 4.7 Hz, 1H), 3.99 (s, 3H).

**Step B:** Methyl 2-(dibromomethyl)nicotinate (2.0 g, 6.5 mmol) and hydrazine hydrate (3.3 g, 65 mmol) were dissolved in 1-butanol (20 mL), and the reaction mixture was heated to 120°C and reacted for 4 hours. The reaction mixture was cooled to room temperature, and diluted with ethyl acetate (200 mL). The organic phase was first washed with saturated brine (30 mL × 2), then dried over anhydrous sodium sulfate, filtered, and finally concentrated under reduced pressure to obtain 0.9 g of pyrido[2,3-*d*]pyridazin-5(6*H*)-one (**13-3**).

MS (ESI) M/Z: 148.1 $[M+H]^+$.

**Step C:** Pyrido[2,3-*d*]pyridazin-5(6*H*)-one (0.5 g, 3.4 mmol) was dissolved in *N,N*-dimethylformamide (10 mL) under an ice bath, then 60% sodium hydride (0.2 g, 5.1 mmol) was added thereto, and the reaction mixture was stirred for 30 minutes. 2-(Trimethylsilyl)ethoxymethyl chloride (0.85 g, 5.1 mmol) was added dropwise thereto, and the reaction mixture was stirred at room temperature for 2 hours after the dropwise addition was completed. The reaction mixture was diluted with ethyl acetate (100 mL). The organic phase was washed with saturated brine (20 mL × 3), then dried over anhydrous sodium sulfate, filtered, and finally concentrated under reduced pressure. The resulting residue was purified by silica gel column chromatography to obtain 0.55 g of 6-((2-(trimethylsilyl)ethoxy)methyl)pyrido[2,3-*d*]pyridazin-5(6*H*)-one (**13-4**).

MS (ESI) M/Z: 278.1 $[M+H]^+$.

**Step D:** 6-((2-(Trimethylsilyl)ethoxy)methyl)pyrido[2,3-*d*]pyridazin-5(6*H*)-one (0.55 g, 2.0 mmol) was dissolved in methanol (25 mL) at room temperature, and a catalytic amount of palladium on carbon was added thereto. The reaction mixture was then stirred at room temperature for another 3 hours under hydrogen atmosphere. The reaction mixture was filtered, and the filtrate was concentrated under reduced pressure to obtain 390 mg of 6-((2-(trimethylsilyl)ethoxy)methyl)-2,3,4,6-tetrahydropyrido[2,3-*d*]pyridazin-5(6*H*)-one (**13-5**).

MS (ESI) M/Z: 282.0 $[M+H]^+$.

$^1H$ NMR(400 MHz, $CDCl_3$) δ 7.30 (s, 1H), 5.41 (s, 2H), 4.50 (s, 1H), 3.80 - 3.64 (m, 2H), 3.33 (t, *J* = 5.5 Hz, 2H), 2.58 (t, *J* = 6.3 Hz, 2H), 1.95 - 1.85 (m, 2H), 1.03 - 0.90 (m, 2H), 0.02 (s, 9H).

**Step E:** 6-((2-(Trimethylsilyl)ethoxy)methyl)-2,3,4,6-tetrahydropyrido[2,3-*d*]pyridazin-5(6*H*)-one (390 mg, 1.4 mmol) was dissolved in *N,N*-dimethylformamide (10 mL), then 60% sodium hydride (80 mg, 2.8 mmol) was added thereto, and the reaction mixture was stirred for 30 minutes. (2-Bromoethoxy)(*tert*-butyl)dimethylsilane (400 mg, 1.7 mmol) was added thereto, and the reaction mixture was stirred at room temperature for 2 hours. The reaction mixture was poured into water, then the aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure to obtain 0.8 g of 1-(2-(*tert*butyldimethylsilyl)ethyl)-6-((2-(trimethylsilyl)ethoxy)methyl)-2,3,4,6-tetrahydropyrido[2,3-*d*]pyridazin-5(6*H*)-one (**13-6**), which was directly used in the next reaction step.

**Step F:** 1-(2-(*tert*-Butyldimethylsilyl)ethyl)-6-((2-(trimethylsilyl)ethoxy)methyl)-2,3,4,6-tetrahydropyrido[2,3-*d*]pyridazin-5(6*H*)-one (0.4 g, 0.9 mmol) as described above was dissolved in tetrahydrofuran (10 mL), then tetrabutylammonium fluoride trihydrate (430 mg, 1.4 mmol) was added thereto, and the reaction mixture was reacted at room temperature for 1 hour. The reaction mixture was diluted with ethyl acetate (100 mL). The organic phase was washed with saturated brine (20 mL × 3), then dried over anhydrous sodium sulfate, filtered, and finally concentrated under reduced pressure to obtain 0.3 g of 1-(2-hydroxyethyl)-6-((2-(trimethylsilyl)ethoxy)methyl)-2,3,4,6-tetrahydropyrido[2,3-*d*]pyridazin-5(6*H*)-one (**13-7**).

MS (ESI) M/Z: 326.0 $[M+H]^+$.

**Step G:** 1-(2-Hydroxyethyl)-6-((2-(trimethylsilyl)ethoxy)methyl)-2,3,4,6-tetrahydropyrido[2,3-*d*]pyridazin-5(6*H*)-one (300 mg, 0.9 mmol) and methyl acrylate (120 mg, 1.4 mmol) were dissolved in acetonitrile (15 mL), then cesium carbonate (0.9 g, 2.8 mmol) was added thereto, and the reaction mixture was stirred at room temperature overnight. The reaction mixture was poured into water, then the aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure. The resulting residue was purified by silica gel column chromatography to obtain 220 mg of methyl 3-(2-(5-oxo-6-((2-(trimethylsilyl)ethoxy)methyl)-3,4,5,6-tetrahydropyrido[2,3-*d*]pyridazin-1(2*H*)-yl)ethoxy)propanoate (**13-8**).

MS (ESI) M/Z: 412.2 $[M+H]^+$.

**Step H:** Methyl 3-(2-(5-oxo-6-((2-(trimethylsilyl)ethoxy)methyl)-3,4,5,6-tetrahydropyrido[2,3-*d*]pyridazin-1(2*H*)-yl)ethoxy)propanoate (100 mg, 0.24 mmol) was dissolved in dichloromethane (2 mL), and trifluoroacetic acid (2 mL) was added dropwise thereto at room temperature. After the dropwise addition was completed, the reaction mixture was stirred and reacted at room temperature for another 2 hours. The reaction mixture was concentrated and poured into water, added with saturated sodium bicarbonate solution to adjust the pH to 8 to 9, and extracted with dichloromethane (50 mL × 3). The combined organic phases were washed with saturated brine (20 mL × 2), then dried over anhydrous sodium sulfate, filtered, and finally concentrated under reduced pressure. The resulting residue was dissolved in tetrahydrofuran (2 mL), and 2 *M* sodium hydroxide solution (0.1 mL) was added thereto, and the reaction mixture was

stirred at room temperature for 2 hours. The reaction mixture was added with dilute hydrochloric acid to adjust the pH to 3 to 4, and extracted with ethyl acetate (20 mL × 3). The organic phase was washed with saturated brine (20 mL), then dried over anhydrous sodium sulfate, filtered, and finally concentrated under reduced pressure to obtain 50 mg of 3-(2-(5-oxo-3,4,5,6-tetrahydropyrido[2,3-*d*]pyridazin-1(2*H*)-yl)ethoxy)propanoic acid (**13-9**).

MS (ESI) M/Z: 268.1 $[M+H]^+$.

**Step I:** 3-(2-(5-Oxo-3,4,5,6-tetrahydropyrido[2,3-*d*]pyridazin-1(2*H*)-yl)ethoxy)propanoic acid (50 mg, 0.18 mmol) was dissolved in dichloromethane (5 mL) at room temperature. 2-(Piperazin-1-yl)-5-(trifluoromethyl)pyrimidine (43 mg, 0.18 mmol), HATU (71 mg, 0.18 mmol), and *N,N*-diisopropylethylamine (77 mg, 0.6 mmol) were then sequentially added to the above solution in an ice-water bath. The reaction mixture was stirred at room temperature for another 1 hour. After the reaction system was diluted with dichloromethane (50 mL), the organic phase was first washed with saturated brine (10 mL × 2), then dried over anhydrous sodium sulfate, filtered, and finally concentrated under reduced pressure. The resulting residue was purified by preparative high performance liquid chromatography to obtain 13 mg of 1-(2-(3-oxo-3-(4-(5-(trifluoromethyl)pyrimidin-2-yl)piperazin-1-yl)propoxy)ethyl)-2,3,4,6-tetrahydropyrido[2,3-*d*]pyridazin-5(1*H*)-one **(compound 13)**.

MS (ESI) M/Z: 481.8 $[M+H]^+$.

$^1H$ NMR (400 MHz, DMSO-$d_6$) δ 12.12 (s, 1H), 8.73 (s, 2H), 7.74 (s, 1H), 3.91 (brs, 2H), 3.88 - 3.74 (m, 4H), 3.65 (t, *J* = 6.3 Hz, 2H), 3.54 (brs, 6H), 3.27 (brs, 2H), 2.57 (t, *J* = 6.3 Hz, 2H), 2.33 (s, 2H), 1.81 - 1.68 (m, 2H).

**Example 14**

**3-((3-Oxo-3-(4-(5-(trifluoromethyl)pyrimidin-2-yl)piperazin-1-yl)propoxy)methyl)imidazo[1,5-*a*]pyrazin-8(7*H*)-one**

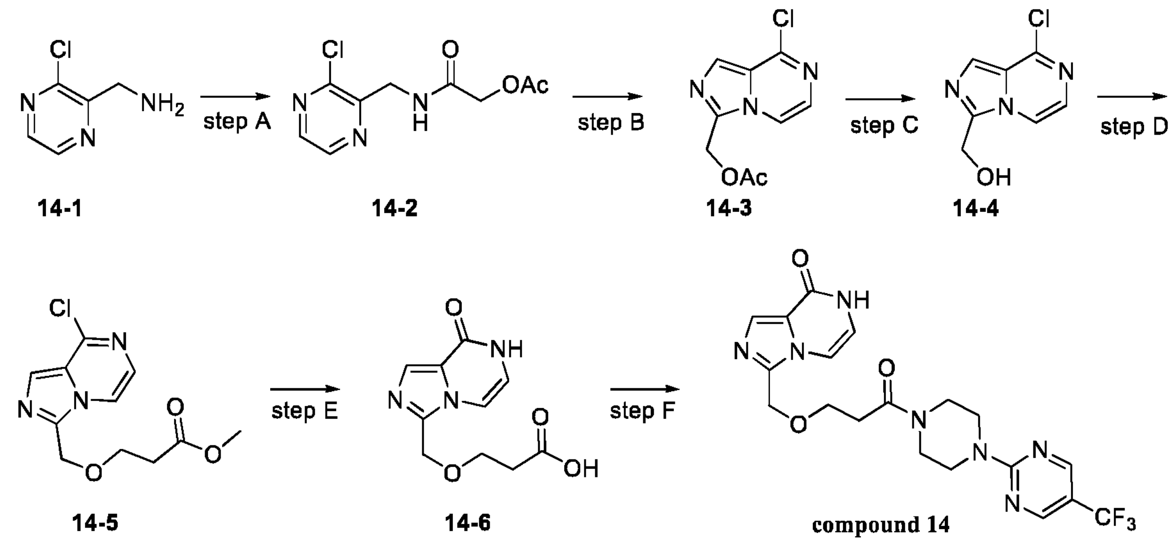


14-1 14-2 14-3 14-4 14-5 14-6 compound 14

**Step A:** 2-Aminomethyl-3-chloropyrazine (1.0 g, 5.6 mmol) was suspended in DMF (50 mL), and triethylamine (1.9 mL, 13.9 mmol) was added thereto. Acetoxyacetyl chloride (0.9 g, 6.7 mmol) was added dropwise thereto in an ice bath, and the reaction mixture was stirred at room temperature for 2 hours. The reaction mixture was diluted with dichloromethane (200 mL). The organic phase was sequentially washed with saturated potassium bisulfate solution (50 mL × 1) and saturated brine (50 mL × 1), dried over anhydrous sodium sulfate, filtered, and concentrated under reduced pressure. The resulting residue was purified by silica gel column chromatography to obtain 1.2 g of 2-((3-chloropyrazin-2-yl)methyl)amino)-2-oxoethyl acetate (**14-2**).

MS (ESI) M/Z: 265.8 $[M+Na]^+$.

**Step B:** 2-((3-Chloropyrazin-2-yl)methyl)amino)-2-oxoethyl acetate (500 mg, 2.0 mmol) was dissolved in dioxane (5 mL), then phosphorus oxychloride (0.42 mL, 4.5 mmol) was added thereto, and the reaction mixture was heated to 80°C and reacted for 2 hours. The reaction mixture was cooled to room temperature, poured into ice, added with saturated sodium bicarbonate solution to adjust the pH to 8 to 9, and extracted with ethyl acetate (50 mL × 3). The organic phase was first washed with saturated brine (30 mL × 2), then dried over anhydrous sodium sulfate, filtered, and finally concentrated under reduced pressure. The resulting residue was purified by silica gel column chromatography

to obtain 110 mg of (8-chloroimidazo[1,5-*a*]pyrazin-3-yl)methyl acetate (**14-3**).

MS (ESI) M/Z: 225.9 $[M+H]^+$.

**Step C:** (8-Chloroimidazo[1,5-*a*]pyrazin-3-yl)methyl acetate (110 mg, 0.5 mmol) was dissolved in tetrahydrofuran (2 mL) in an ice bath, then 1 *M* sodium hydroxide solution (1 mL, 1.0 mmol) was added thereto, and the reaction mixture was stirred at room temperature for 30 minutes. The reaction mixture was neutralized with 1 *N* dilute hydrochloric acid, and extracted with ethyl acetate (50 mL × 2). The organic phase was washed with saturated brine (20 mL), then dried over anhydrous sodium sulfate, filtered, and finally concentrated under reduced pressure to obtain 100 mg of (8-chloroimidazo[1,5-*a*]pyrazin-3-yl)methanol (**14-4**).

MS (ESI) M/Z: 183.5 $[M+H]^+$.

**Step D:** (8-Chloroimidazo[1,5-*a*]pyrazin-3-yl)methanol (280 mg, 1.5 mmol) and methyl acrylate (520 mg, 6.1 mmol) were dissolved in acetonitrile (5 mL) at room temperature, then cesium carbonate (1.0 g, 3.0 mmol) was added thereto, and the reaction mixture was stirred at room temperature for 5 hours. The reaction mixture was poured into water, then the aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure. The resulting residue was purified by silica gel column chromatography to obtain 120 mg of methyl 3-((8-chloroimidazo[1,5-*a*]pyrazin-3-yl)methoxy)propanoate (**14-5**).

MS (ESI) M/Z: 269.7 $[M+H]^+$.

**Step E:** Methyl 3-((8-chloroimidazo[1,5-*a*]pyrazin-3-yl)methoxy)propanoate (100 mg, 0.37 mmol) was dissolved in dioxane (2 mL) and concentrated hydrochloric acid (2 mL), and the reaction mixture was refluxed for 2 hours. The reaction mixture was concentrated under reduced pressure to obtain 100 mg of 3-((8-oxo-7,8-dihydroimidazo[1,5-*a*]pyrazin-3-yl)methoxy)propanoic acid (**14-6**), which was directly used in the next reaction step.

MS (ESI) M/Z: 236.0 $[M-H]^-$.

**Step F:** 3-((8-Oxo-7,8-dihydroimidazo[1,5-*a*]pyrazin-3-yl)methoxy)propanoic acid (80 mg, 0.33 mmol) was dissolved in dichloromethane (10 mL) and *N,N*-dimethylformamide (10 mL) at room temperature. 2-(Piperazin-1-yl)-5-(trifluoromethyl)pyrimidine (80 mg, 0.33 mmol), HATU (200 mg, 0.50 mmol), and *N,N*-diisopropylethylamine (130 mg, 1.0 mmol) were then sequentially added to the above solution in an ice-water bath. The reaction mixture was stirred at room temperature for another 1 hour. After the reaction system was diluted with dichloromethane (100 mL), the organic phase was first washed with saturated brine (20 mL × 2), then dried over anhydrous sodium sulfate, filtered, and finally concentrated under reduced pressure. The resulting residue was purified by preparative high performance liquid chromatography to obtain 9 mg of 3-((3-oxo-3-(4-(5-(trifluoromethyl)pyrimidin-2-yl)piperazin-1-yl)propoxy)methyl)imidazo[1,5-*a*]pyrazin-8(7*H*)-one **(compound 14)**.

MS (ESI) M/Z: 474.2 $[M+Na]^+$.

$^1$H NMR (400 MHz, $CDCl_3$) δ 10.56 (brs, 1H), 8.51 (s, 2H), 8.21 (brs, 1H), 7.28 (s, 1H), 6.88 (brs, 1H), 5.08 (s, 2H), 4.04 - 3.83 (m, 6H), 3.74 (brs, 2H), 3.60 (brs, 2H), 2.78 (brs, 2H).

**Example 15**

**5-(2-(3-Oxo-3-(4-(5-(trifluoromethyl)pyrimidin-2-yl)piperazin-1-yl)propoxy)ethyl)isoquinolin-1(2*H*)-one**

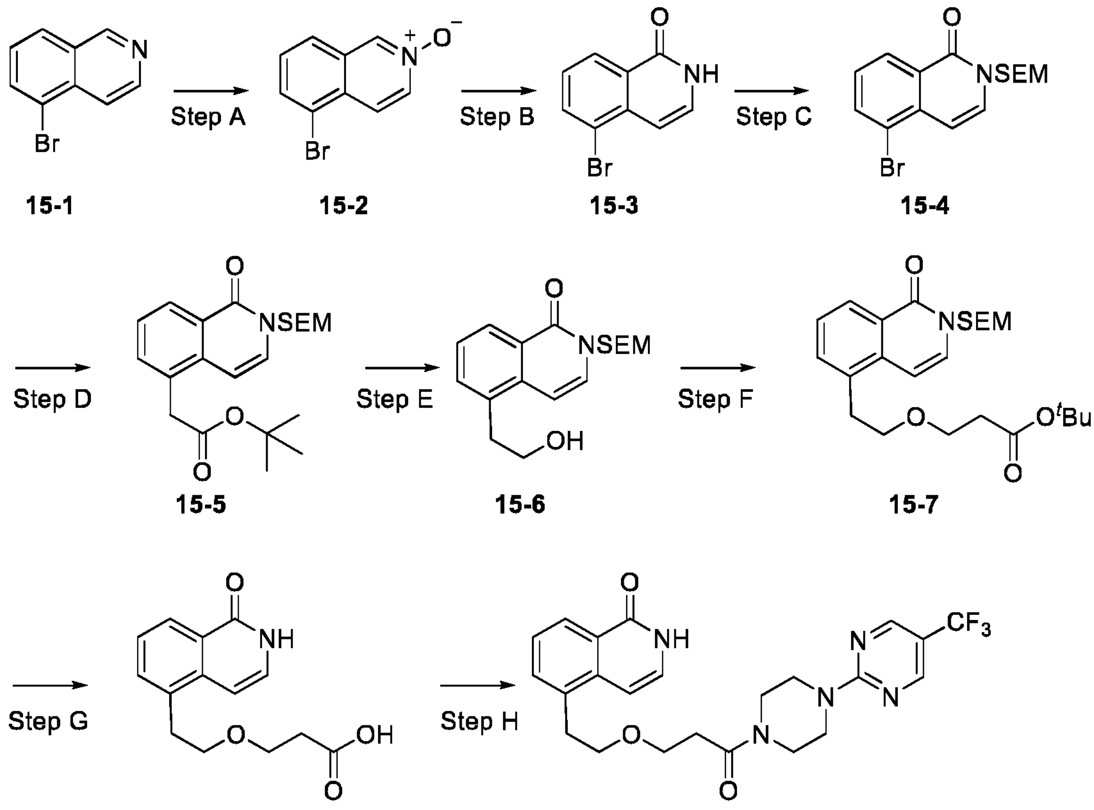


15-1 15-2 15-3 15-4

15-5 15-6 15-7

15-8 compound 15

**Step A:** 5-Bromoisoquinoline (5.0 g, 24 mmol) was dissolved in dichloromethane (50 mL), then 3-chloroperoxybenzoic acid (6.2 g, 36 mmol) was added thereto in batches in an ice-water bath, and the reaction mixture was stirred at room temperature overnight. The reaction mixture was filtered, and the filter cake was washed with dichloromethane (50 mL × 2) to obtain 5 g of 5-bromoisoquinoline 2-oxide (**15-2**).

MS (ESI) M/Z: 223.4 [M+H]$^+$.

**Step B:** 5-Bromoisoquinoline 2-oxide (5.0 g, 22.5 mmol) was suspended in water (50 mL), then methanesulfonyl chloride (3 mL) was added dropwise thereto, and the reaction mixture was stirred at room temperature overnight. The reaction mixture was neutralized with saturated sodium bicarbonate solution to adjust the pH to neutrality. The aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure. The resulting residue was purified by silica gel column chromatography to obtain 1.9 g of 5-bromoisoquinolin-1(2*H*)-one (**15-3**).

MS (ESI) M/Z: 224.0 [M+H]$^+$.

**Step C:** 5-Bromoisoquinolin-1(2*H*)-one (1.5 g, 6.7 mmol) was dissolved in *N,N*-dimethylformamide (10 mL), then 60% sodium hydride (0.4 g, 10 mmol) was added thereto, and the reaction mixture was stirred at room temperature for 30 minutes. (2-Bromoethoxy)(*tert*-butyl)dimethylsilane (1.68 g, 10 mmol) was added dropwise thereto, and the reaction mixture was stirred at room temperature for 4 hours. The reaction mixture was poured into water, then the aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure. The resulting residue was purified by silica gel column chromatography to obtain 1.5 g of 5-bromo-2-((2-(trimethylsilyl)ethoxy)methyl)isoquinolin-1(2*H*)-one (**15-4**).

MS (ESI) M/Z: 377.9 [M+Na]$^+$.

**Step D:** 5-Bromo-2-((2-(trimethylsilyl)ethoxy)methyl)isoquinolin-1(2*H*)-one was used as a raw material instead of 5-bromo-2-methoxy-3-(trifluoromethyl)pyridine with the preparation method referring to that of **3-2** to obtain *tert*-butyl 2-(1-oxo-2-((2-(trimethylsilyl)ethoxy)methyl)-1,2-dihydroisoquinolin-5-yl)acetate (**15-5**).

MS (ESI) M/Z: 412.1 [M+Na]$^+$.

$^1$H NMR (400 MHz, $CDCl_3$) δ 8.39 (d, *J* = 8.0 Hz, 1H), 7.55 (d, *J* = 6.7 Hz, 1H), 7.44 (t, *J* = 7.7 Hz, 1H), 7.23 (s, 1H), 6.65 (d, *J* = 7.7 Hz, 1H), 5.43 (s, 2H), 3.79 (s, 2H), 3.69 - 3.60 (m, 2H), 1.42 (s, 9H), 1.00 - 0.90 (m, 2H), -0.02 (s, 9H).

**Step E:** *tert-Butyl* 2-(1-oxo-2-((2-(trimethylsilyl)ethoxy)methyl)-1,2-dihydroisoquinolin-5-yl)acetate (189 mg, 0.5 mmol) was dissolved in tetrahydrofuran (10 mL), then lithium aluminum hydride (50 mg, 1.1 mmol) was added thereto,

and the reaction mixture was heated to reflux and stirred for 5 hours. The reaction mixture was cooled to room temperature, and poured into water. The aqueous phase was extracted with ethyl acetate (50 mL × 3), and the organic phases were combined. The combined organic phases were first washed with saturated brine (20 mL × 2), then dried over anhydrous sodium sulfate, filtered, and finally concentrated under reduced pressure. The resulting residue was purified by silica gel column chromatography to obtain 75 mg of 5-(2-hydroxyethyl)-2-((2-(trimethylsilyl)ethoxy)methyl)isoquinolin-1(2*H*)-one (**15-6**).

MS (ESI) M/Z: 320.2 $[M+H]^+$.

$^1H$ NMR (400 MHz, $CDCl_3$) δ 8.32 (d, *J* = 8.1 Hz, 1H), 7.53 (d, *J* = 6.9 Hz, 1H), 7.40 (t, *J* = 7.7 Hz, 1H), 7.21 (d, *J* = 7.7 Hz, 1H), 6.71 (d, *J* = 7.7 Hz, 1H), 5.40 (s, 2H), 3.92 (t, *J* = 6.7 Hz, 2H), 3.67 - 3.59 (m, 2H), 3.14 (t, *J* = 6.7 Hz, 2H), 0.98 - 0.90 (m, 2H), -0.03 (s, 9H).

**Step F:** 5-(2-Hydroxyethyl)-2-((2-(trimethylsilyl)ethoxy)methyl)isoquinolin-1(2*H*)-one (75 mg, 0.24 mmol) was dissolved in acetonitrile (2 mL), then tert-butyl acrylate (45 mg, 0.35 mmol) and cesium carbonate (250 mg, 0.8 mmol) were added thereto, and the reaction mixture was heated to 80°C and stirred overnight. The reaction mixture was cooled to room temperature, added with water to quench, and then extracted with ethyl acetate (20 mL × 3). The organic phase was washed with saturated brine (20 mL), then dried over anhydrous sodium sulfate, filtered, and finally concentrated under reduced pressure. The resulting residue was purified by silica gel column chromatography to obtain 56 mg of *tert*-butyl 3-(2-(1-oxo-2-((2-(trimethylsilyl)ethoxy)methyl)-1,2-dihydroisoquinolin-5-yl)ethoxy)propanoate (**15-7**), which was directly used in the next reaction step.

MS (ESI) M/Z: 448.3 $[M+H]^+$.

$^1H$ NMR (400 MHz, $CDCl_3$) δ 8.34 (d, *J* = 8.0 Hz, 1H), 7.52 (d, *J* = 7.1 Hz, 1H), 7.41 (t, *J* = 7.7 Hz, 1H), 7.24 - 7.20 (m, 1H), 6.70 (d, *J* = 7.7 Hz, 1H), 5.43 (s, 2H), 3.69 (t, *J* = 6.6 Hz, 4H), 3.67 - 3.59 (m, 2H), 3.14 (t, *J* = 7.2 Hz, 2H), 2.48 (t, *J* = 6.3 Hz, 2H), 1.43 (s, 9H), 1.00 - 0.91 (m, 2H), -0.02 (s, 9H).

**Step G:** *tert*-Butyl 3-(2-(1-oxo-2-((2-(trimethylsilyl)ethoxy)methyl)-1,2-dihydroisoquinolin-5-yl)ethoxy)propanoate (56 mg, 125 μmol) was dissolved in dichloromethane (2 mL) at room temperature, and trifluoroacetic acid (2 mL) was added thereto. The reaction mixture was stirred at room temperature for another 2 hours. The reaction mixture was concentrated under reduced pressure. The resulting residue was diluted with dichloromethane (50 mL), and then neutralized with saturated sodium bicarbonate solution to adjust the pH to neutrality. The aqueous phase was extracted with dichloromethane (50 mL × 2), and the organic phase was washed with saturated brine (20 mL), dried over anhydrous sodium sulfate, filtered, and finally concentrated under reduced pressure to obtain 50 mg of 3-(2-(1-oxo-1,2-dihydroisoquinolin-5-yl)ethoxy)propanoic acid (**15-8**), which was directly used in the next reaction step.

MS (ESI) M/Z: 262.0 $[M+H]^+$.

**Step H:** 3-(2-(1-Oxo-1,2-dihydroisoquinolin-5-yl)ethoxy)propanoic acid (50 mg, 0.19 mmol) was dissolved in dichloromethane (5 mL) and *N,N*-dimethylformamide (5 mL) at room temperature. 2-(Piperazin-1-yl)-5-(trifluoromethyl)pyrimidine (44 mg, 0.19 mmol), HATU (55 mg, 0.29 mmol), and *N,N*-diisopropylethylamine (77 mg, 0.6 mmol) were then sequentially added to the above solution in an ice-water bath. The reaction mixture was stirred at room temperature for another 2 hours. The reaction mixture was added with dilute hydrochloric acid to adjust the pH to 5 to 6, and then extracted with ethyl acetate (20 mL × 3). The organic phase was washed with saturated brine (20 mL), then dried over anhydrous sodium sulfate, filtered, and finally concentrated under reduced pressure. The resulting residue was purified by preparative high performance liquid chromatography to obtain 14 mg of 5-(2-(3-oxo-3-(4-(5-(trifluoromethyl)pyrimidin-2-yl)piperazin-1-yl)propoxy)ethyl)isoquinolin-1(2*H*)-one (compound **15**).

MS (ESI) M/Z: 476.4 $[M+H]^+$.

$^1H$ NMR (400 MHz, $CDCl_3$) δ 11.65 (brs, 1H), 8.51 (s, 2H), 8.33 (brs, 1H), 7.62 (d, *J* = 6.8 Hz, 1H), 7.49 (s, 1H), 7.22 (brs, 1H), 6.91 (s, 1H), 3.97 - 3.84 (m, 4H), 3.82 - 3.73 (m, 2H), 3.75 (t, *J* = 6.4 Hz, 2H), 3.70 (brs, 2H), 3.54 (brs, 2H), 3.25 - 3.12 (m 2H), 2.70 - 2.60 (m, 2H).

### Example 16

**1-(2-(3-Oxo-3-(4-(5-(trifluoromethyl)pyrimidin-2-yl)piperazin-1-yl)propoxy)ethyl)-1,5-dihydro-4*H*-pyrrolo[2,3-*d*]pyridazin-4-one**

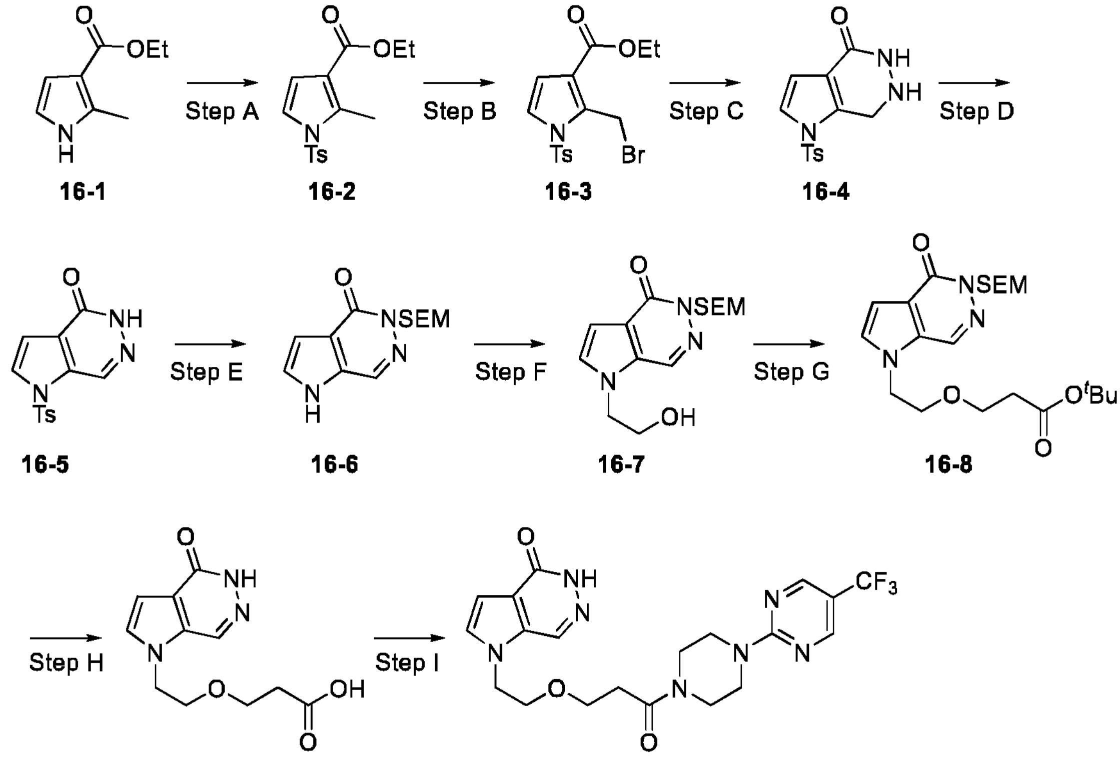


16-1 16-2 16-3 16-4

16-5 16-6 16-7 16-8

16-9 compound 16

**Step A:** Ethyl 2-methyl-1*H*-pyrrole-3-carboxylate (9.0 g, 58.5 mmol) was dissolved in toluene (200 mL), and tetrabutylammonium bromide (1.88 g, 5.85 mmol) and tosyl chloride (16.7 g, 87.8 mmol) were added thereto in an ice-water bath. 10 *M* sodium hydroxide solution (58 mL, 0.58 mol) was then added dropwise thereto, and the reaction mixture was stirred at room temperature for 4 hours. The reaction mixture was added with water to quench. The aqueous phase was extracted with ethyl acetate (200 mL × 3), and the organic phases were combined. The combined organic phases were first washed with saturated brine (30 mL × 2), then dried over anhydrous sodium sulfate, filtered, and finally concentrated under reduced pressure. The resulting residue was purified by silica gel column chromatography to obtain 17 g of ethyl 2-methyl-1-tosyl-1*H*-pyrrole-3-carboxylate (**16-2**).

**Step B:** Ethyl 2-methyl-1-tosyl-1*H*-pyrrole-3-carboxylate (17.0 g, 55.4 mmol) was suspended in glacial acetic acid (200 mL), then liquid bromine (9.75 g, 60.9 mmol) was added dropwise thereto, and the reaction mixture was stirred at 120°C overnight. The reaction mixture was cooled to room temperature, concentrated under reduced pressure to remove most of glacial acetic acid, and the resulting residue was neutralized with saturated sodium bicarbonate solution to adjust the pH to neutrality. The aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure. The resulting residue was purified by silica gel column chromatography to obtain 8 g of ethyl 2-(bromomethyl)-1-tosyl-1*H*-pyrrole-3-carboxylate (**16-3**).

MS (ESI) M/Z: 386.0 $[M+H]^+$.

**Step C:** Ethyl 2-(bromomethyl)-1-tosyl-1*H*-pyrrole-3-carboxylate (8.0 g, 20.7 mmol) was dissolved in ethanol (100 mL) and water (40 mL), and 98% hydrazine hydrate (12.0 mL, 0.31 mol) was added thereto. The reaction mixture was stirred at room temperature for 30 minutes, and then stirred at 50°C overnight. The reaction mixture was poured into water, then the aqueous phase was extracted with ethyl acetate (100 mL × 3), and the organic phases were combined. The combined organic phases were first washed with saturated brine (30 mL × 2), then dried over anhydrous sodium sulfate, filtered, and finally concentrated under reduced pressure. The resulting residue was purified by silica gel column chromatography to obtain 1.75 g of 1-tosyl-1,5,6,7-tetrahydro-1*H*-pyrrolo[2,3-*d*]pyridazin-4-one (**16-4**).

MS (ESI) M/Z: 292.1 $[M+H]^+$.

**Step D:** 1-Tosyl-1,5,6,7-tetrahydro-1*H*-pyrrolo[2,3-*d*]pyridazin-4-one (1.7 g, 5.8 mmol) was dissolved in dichloromethane (100 mL), then IBX (3.4 g, 12.0 mmol) was added thereto, and the reaction mixture was stirred at room temperature overnight. The reaction mixture was filtered to remove IBX. The filtrate was first washed with saturated brine (30 mL × 2), then dried over anhydrous sodium sulfate, filtered, and finally concentrated under reduced pressure to obtain 1.5 g of 1-tosyl-1,5-dihydro-4*H*-pyrrolo[2,3-*d*]pyridazin-4-one (**16-5**).

MS (ESI) M/Z: 290.0 $[M+H]^+$.

$^1H$ NMR (400 MHz, $CDCl_3$) δ 10.65 (s, 1H), 8.64 (s, 1H), 7.87 - 7.79 (m, 2H), 7.56 (d, *J* = 3.4 Hz, 1H), 7.35 (d, *J* = 8.2 Hz, 2H), 6.97 (d, *J* = 3.4 Hz, 1H), 2.43 (s, 3H).

**Step E:** 1-Tosyl-1,5-dihydro-4*H*-pyrrolo[2,3-*d*]pyridazin-4-one (500 mg, 1.73 mmol) was dissolved in anhydrous *N,N*-dimethylformamide (10 mL), then 60% sodium hydride (80 mg, 1.9 mmol) was added thereto in an ice bath, and the reaction mixture was stirred at room temperature for 30 minutes. 2-(Trimethylsilyl)ethoxymethyl chloride (0.32 mg, 1.9 mmol) was then added dropwise thereto, and the reaction mixture was stirred at room temperature for 3 hours after the dropwise addition was completed. The reaction mixture was poured into water, then the aqueous phase was added with citric acid to adjust the pH to 6 to 7, extracted with ethyl acetate (50 mL × 3), and the organic phases were combined. The combined organic phases were first washed with saturated brine (20 mL × 2), then dried over anhydrous sodium sulfate, filtered, and finally concentrated under reduced pressure. The resulting residue was dissolved in tetrahydrofuran (2 mL) and water (2 mL), then sodium hydroxide (100 mg, 2.5 mmol) was added thereto, and the reaction mixture was stirred at room temperature for 2 hours. The reaction mixture was poured into water, then the aqueous phase was acidified with citric acid to adjust the pH to 5 to 6, extracted with ethyl acetate (50 mL × 3), and the organic phases were combined. The combined organic phases were first washed with saturated brine (20 mL × 2), then dried over anhydrous sodium sulfate, filtered, and finally concentrated under reduced pressure to obtain 145 mg of 5-((2-(trimethylsilyl)ethoxy)methyl)-1,5-dihydro-4*H*-pyrrolo[2,3-*d*]pyridazin-4-one (**16-6**).

MS (ESI) M/Z: 288.2 $[M+Na]^+$.

$^1H$ NMR (400 MHz, $CDCl_3$) δ 10.73 (s, 1H), 8.22 (s, 1H), 7.24 (s, 1H), 6.85 (s, 1H), 5.64 (s, 2H), 3.77 - 3.69 (m, 2H), 1.01 - 0.92 (m, 2H), -0.04 (s, 9H).

**Step F:** 5-((2-(Trimethylsilyl)ethoxy)methyl)-1,5-dihydro-4*H*-pyrrolo[2,3-d]pyridazin-4-one (100 mg, 0.38 mmol) was dissolved in anhydrous *N,N*-dimethylformamide (10 mL), then 60% sodium hydride (30 mg, 0.76 mmol) was added thereto in an ice bath, and the reaction mixture was stirred at room temperature for 30 minutes. (2-Bromoethoxy)(*tert*-butyl)dimethylsilane (110 mg, 0.45 mmol) was then added dropwise thereto, and the reaction mixture was stirred at 80°C for 3 hours after the dropwise addition was completed. The reaction mixture was poured into water and stirred at room temperature for 30 minutes. The aqueous phase was then neutralized with citric acid to adjust the pH to 6 to 7, extracted with ethyl acetate (50 mL × 3), and the organic phases were combined. The combined organic phases were first washed with saturated brine (20 mL × 2), then dried over anhydrous sodium sulfate, filtered, and finally concentrated under reduced pressure. The resulting residue was purified by silica gel column chromatography to obtain 100 mg of 1-(2-hydroxyethyl)-5-((2-(trimethylsilyl)ethoxy)methyl)-1,5-dihydro-4*H*-pyrrolo[2,3-d]pyridazin-4-one (**16-7**).

MS (ESI) M/Z: 310.3 $[M+H]^+$.

$^1H$ NMR (400 MHz, $CDCl_3$) δ 8.13 (s, 1H), 7.09 - 7.04 (m, 1H), 6.64 - 6.58 (m, 1H), 5.51 - 5.48 (m, 2H), 4.24 (t, *J* = 4.9 Hz, 2H), 3.98 (t, *J* = 4.9 Hz, 2H), 3.74 - 3.59 (m, 2H), 0.98 - 0.87 (m, 2H), -0.04 (s, 9H).

**Step G:** 1-(2-Hydroxyethyl)-5-((2-(trimethylsilyl)ethoxy)methyl)-1,5-dihydro-4*H*-pyrrolo[2,3-*d*]pyridazin-4-one (100 mg, 0.32 mmol) was dissolved in acetonitrile (2 mL), then *tert*-butyl acrylate (62 mg, 0.49 mmol) and cesium carbonate (320 mg, 1.0 mmol) were added thereto, and the reaction mixture was heated to 80°C and stirred overnight. The reaction mixture was added with water to quench, and then extracted with ethyl acetate (20 mL × 3). The organic phase was washed with saturated brine (20 mL), then dried over anhydrous sodium sulfate, filtered, and finally concentrated under reduced pressure. The resulting residue was purified by silica gel column chromatography to obtain 45 mg of *tert*-butyl 3-(2-(4-oxo-5-((2-(trimethylsilyl)ethoxy)methyl)-4,5-dihydro-1*H*-pyrrolo[2,3-*d*]pyridazin-1-yl)ethoxy)propanoate (**16-8**), which was directly used in the next reaction step.

MS (ESI) M/Z: 438.1 $[M+H]^+$.

**Step H:** *tert*-Butyl 3-(2-(4-oxo-5-((2-(trimethylsilyl)ethoxy)methyl)-4,5-dihydro-1*H*-pyrrolo[2,3-*d*]pyridazin-1-yl)ethoxy)propanoate (45 mg, 103 μmol) was dissolved in dichloromethane (2 mL) at room temperature, and trifluoroacetic acid (2 mL) was added thereto. The reaction mixture was stirred at room temperature for another 2 hours. The reaction mixture was concentrated under reduced pressure. The resulting residue was diluted with dichloromethane (50 mL), and then neutralized with saturated sodium bicarbonate solution to adjust the pH to neutrality. The aqueous phase was extracted with dichloromethane (50 mL × 2), and the organic phase was washed with saturated brine (20 mL), dried over anhydrous sodium sulfate, filtered, and finally concentrated under reduced pressure to obtain 50 mg of 3-(2-(4-oxo-4,5-dihydro-1*H*-pyrrolo[2,3-*d*]pyridazin-1-yl)ethoxy)propanoic acid (**16-9**), which was directly used in the next reaction step.

MS (ESI) M/Z: 252.1 $[M+H]^+$.

**Step I:** 3-(2-(4-Oxo-4,5-dihydro-1*H*-pyrrolo[2,3-*d*]pyridazin-1-yl)ethoxy)propanoic acid (50 mg, 0.18 mmol) was dissolved in dichloromethane (5 mL) and *N,N*-dimethylformamide (5 mL) at room temperature. 2-(Piperazin-1-yl)-5-(trifluoromethyl)pyrimidine (44 mg, 0.18 mmol), HATU (55 mg, 0.26 mmol), and *N,N*-diisopropylethylamine (77 mg, 0.6 mmol) were then sequentially added to the above solution in an ice-water bath. The reaction mixture was stirred at room temperature for another 2 hours. The reaction mixture was added with dilute hydrochloric acid to adjust the pH to 5 to 6, and then extracted with ethyl acetate (20 mL × 3). The organic phase was washed with saturated brine (20 mL), then dried over anhydrous sodium sulfate, filtered, and finally concentrated under reduced pressure. The resulting residue

was purified by preparative high performance liquid chromatography to obtain 5 mg of 1-(2-(3-oxo-3-(4-(5-(trifluoromethyl)pyrimidin-2-yl)piperazin-1-yl)propoxy)ethyl)-1,5-dihydro-4*H*-pyrrolo[2,3-*d*]pyridazin-4-one (**compound 16**).

MS (ESI) M/Z: 466.1 $[M+H]^+$.

$^1H$ NMR (400 MHz, $CDCl_3$) $\delta$ 10.15 (s, 1H), 8.51 (s, 2H), 8.13 (s, 1H), 7.12 (s, 1H), 6.87 (s, 1H), 4.34 - 4.25 (m, 2H), 3.89 (brs, 4H), 3.85 - 3.73 (m, 4H), 3.69 (brs, 2H), 3.49 (brs, 2H), 2.58 - 2.50 (m, 2H).

### Example 17

**5-(3-Oxo-3-(4-(5-(trifluoromethyl)pyrimidin-2-yl)piperazin-1-yl)propoxy)-3-(trifluoromethyl)pyridin-2(1*H*)-one**

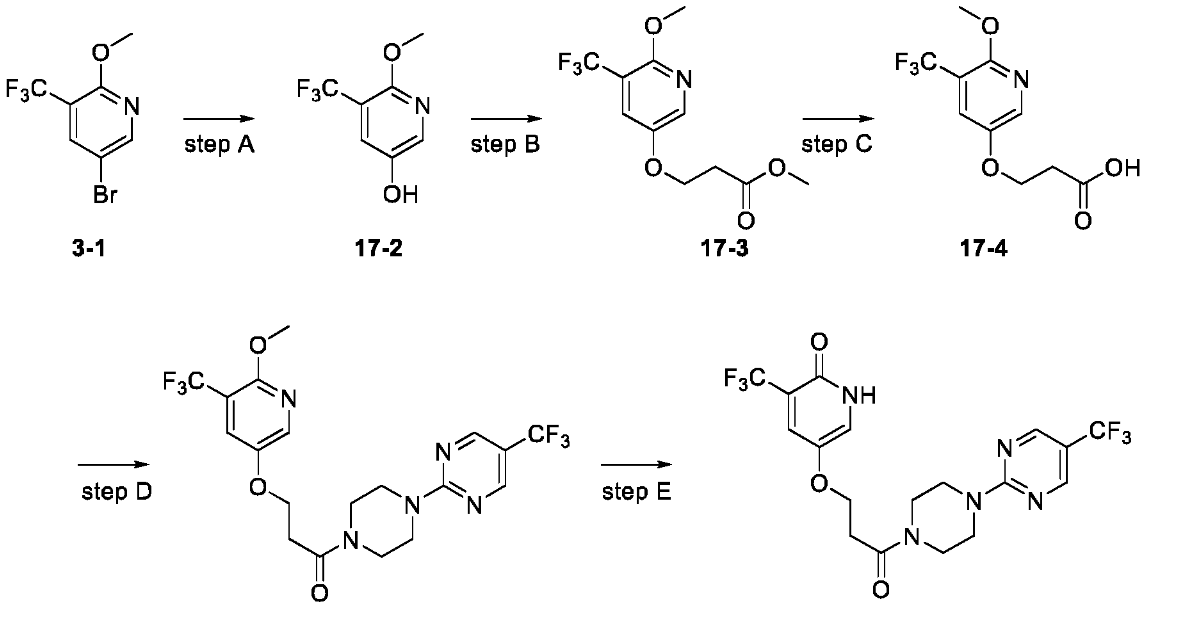


3-1 17-2 17-3 17-4

17-5 compound 17

**Step A:** 5-Bromo-2-methoxy-3-(trifluoromethyl)pyridine (2.56 g, 10 mmol) was dissolved in dry tetrahydrofuran (50 mL) at room temperature under nitrogen atmosphere, and the reaction mixture was cooled to -78°C. *n*-Butyllithium (2.0 mol/L, 10 mL, 20 mmol) was added dropwise to the system to ensure that the internal temperature was controlled -70°C or less. After the addition was completed, the system was stirred at -78°C for 1 hour, then slowly added with trimethyl borate (1.56 g, 15 mmol), warmed to room temperature and reacted for another 1 hour. The reaction mixture was added with saturated ammonium chloride aqueous solution (50 mL) to quench, then extracted with ethyl acetate (100 mL × 3), and the organic phase was concentrated. The resulting residue was dissolved in dichloromethane (100 mL) and cooled to 0°C, and hydrogen peroxide (30%, 20 mL) was slowly added to the system. After the addition was completed, the reaction mixture was warmed to room temperature and reacted for another 3 hours. The reaction mixture was added with saturated sodium thiosulfate (20 mL) to quench, and the organic phases were combined. The combined organic phases were first washed with saturated brine (100 mL × 3), then dried over anhydrous sodium sulfate, filtered, and finally concentrated under reduced pressure. The resulting residue was purified by silica gel column chromatography to obtain 920 mg of 6-methoxy-5-(trifluoromethyl)pyridin-3-ol (**17-2**).

MS (ESI) M/Z: 194.2 $[M+H]^+$.

**Step B:** 6-Methoxy-5-(trifluoromethyl)pyridin-3-ol (200 mg, 1.04 mmol) was dissolved in acetonitrile (10 mL), then methyl acrylate (891.8 mg, 10.4 mmol) and cesium carbonate (677.7 mg, 2.08 mmol) were sequentially added thereto, and the reaction mixture was reacted at room temperature for 4 hours. The reaction mixture was added with ethyl acetate (100 mL), and then washed with water (50 mL × 3). The organic phase was dried over anhydrous sodium sulfate, filtered, and finally concentrated under reduced pressure. The resulting residue was purified by silica gel column chromatography to obtain 85 mg of methyl 3-((6-methoxy-5-(trifluoromethyl)pyridin-3-yl)oxy)propanoate (**17-3**).

MS (ESI) M/Z: 280.1 $[M+H]^+$.

**Step C:** Methyl 3-((6-methoxy-5-(trifluoromethyl)pyridin-3-yl)oxy)propanoate (85.0 mg, 0.30 mmol) was dissolved in methanol (5 mL) and water (2 mL), then lithium hydroxide (14.4 mg, 0.6 mmol) was added thereto, and the reaction mixture was reacted at room temperature for 2 hours. The reaction mixture was added with water (50 mL), and

extracted with ethyl acetate (10 mL × 3). The aqueous phase was added with trifluoroacetic acid to adjust the pH to 3 to 4, and concentrated under reduced pressure to obtain a crude product of trifluoroacetate salt of 3-((6-methoxy-5-(trifluoromethyl)pyridin-3-yl)oxy)propanoic acid (**17-4**), which was directly used in the next reaction step.

MS (ESI) M/Z: 266.3 $[M+H]^+$.

**Step D:** The crude product of trifluoroacetate salt of 3-((6-methoxy-5-(trifluoromethyl)pyridin-3-yl)oxy)propanoic acid was dissolved in *N,N* dimethylformamide (3 mL), and then 2-(piperazin-1-yl)-5-(trifluoromethyl)pyrimidine (92.7 mg, 0.3 mmol), HATU (114.1 mg, 0.3 mmol), and triethylamine (242.9 mg, 2.4 mmol) were sequentially added thereto. After the addition was completed, the reaction system was reacted at room temperature for 1 hour. The reaction mixture was added with ethyl acetate (100 mL), and then washed with water (50 mL × 3). The organic phase was concentrated, and the resulting residue was purified by silica gel column chromatography to obtain 43 mg of 3-((6-methoxy-5-(trifluoromethyl)pyridin-3-yl)oxy)-1-(4-(5-(trifluoromethyl)pyrimidin-2-yl)piperazin-1-yl)propan-1-one (**17-5**).

MS (ESI) M/Z: 480.4 $[M+H]^+$.

**Step E:** 3-((6-Methoxy-5-(trifluoromethyl)pyridin-3-yl)oxy)-1-(4-(5-(trifluoromethyl)pyrimidin-2-yl)piperazin-1-yl)propan-1-one (43.2 mg, 0.09 mmol) was dissolved in dichloromethane (5 mL) and cooled to 0°C, and boron tribromide (1 mol/L, 0.9 mL, 0.9 mol) was slowly added to the reaction mixture. After the addition was completed, the reaction mixture was warmed to room temperature and reacted for 5 hours. The reaction mixture was concentrated under reduced pressure, and the resulting residue was purified by preparative high performance liquid chromatography to obtain 14 mg of 5-(3-oxo-3-(4-(5-(trifluoromethyl)pyrimidin-2-yl)piperazin-1-yl)propoxy)-3-(trifluoromethyl)pyridin-2(1*H*)-one (compound **17**).

MS (ESI) M/Z: 466.4 $[M+H]^+$.

$^1H$ NMR (400 MHz, $CDCl_3$) δ 12.03 (brs, 1H), 8.74 (s, 2H), 7.75 (d, *J* = 3.2 Hz, 1H), 7.47 (d, *J* = 3.2 Hz, 1H), 4.15 (t, *J* = 6.1 Hz, 2H), 3.90 - 3.81 (m, 4H), 3.62 - 3.59 (m, 4H), 2.82 (t, *J* = 6.2 Hz, 2H).

### Example 18

**(*R*)-1-(1-(3-Oxo-3-(4-(5-(trifluoromethyl)pyrimidin-2-yl)piperazin-1-yl)propoxy-2-yl)-1,5-dihydro-4*H*-pyrrolo[2,3-*d*]pyridazin-4-one**

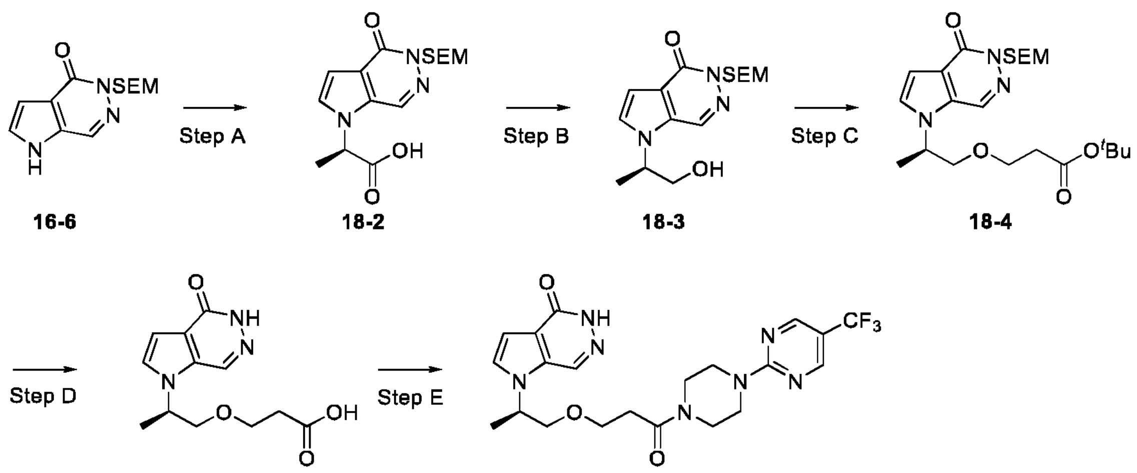


16-6, 18-2, 18-3, 18-4, 18-5, compound 18

**Step A:** Methyl *L*-lactate (5 g, 48 mmol) was dissolved in dichloromethane (100 mL), then triethylamine (14.6 g, 145 mmol) was added thereto in an ice-water bath. Tosyl chloride (11 g, 58 mmol) was added thereto in batches, and the reaction mixture was stirred at room temperature for 2 hours. The reaction mixture was poured into water, and the phases were separated. The organic phase was sequentially washed with citric acid aqueous solution (50 mL) and saturated brine (50 mL), then dried over anhydrous sodium sulfate, filtered, and finally concentrated under reduced pressure. The resulting residue was purified by silica gel column chromatography to obtain methyl (*S*)-2-(*p*-tolyloxy)propanoate (10 g, colorless transparent liquid) for later use.

5-((2-(Trimethylsilyl)ethoxy)methyl)-1,5-dihydro-4*H*-pyrrolo[2,3-*d*]pyridazin-4-one (150 mg, 0.57 mmol) was dissolved in anhydrous *N,N*-dimethylformamide (10 mL), then 60% sodium hydride (50 mg, 1.2 mmol) was added thereto in an ice bath, and the reaction mixture was stirred at room temperature for 30 minutes. Methyl (*S*)-2-(*p*-tolyloxy)propanoate (110 mg, 0.45 mmol) was then added dropwise thereto, and the reaction mixture was stirred at 80°C for 3 hours

after the dropwise addition was completed. The reaction mixture was cooled to room temperature, poured into water (10 mL), and stirred at room temperature for 30 minutes. The aqueous phase was then neutralized with citric acid to adjust the pH to 6 to 7, extracted with ethyl acetate (50 mL × 3), and the organic phases were combined. The combined organic phases were first washed with saturated brine (20 mL × 2), then dried over anhydrous sodium sulfate, filtered, and finally concentrated under reduced pressure to obtain 250 mg of (*R*)-2-(4-oxo-5-((2-(trimethylsilyl)ethoxy)methyl)-4,5-dihydro-1*H*-pyrrolo[2,3-*d*]pyridazin-1-yl)propanoic acid (**18-2**), which was directly used in the next reaction step.

MS (ESI) M/Z: 338.4 $[M+H]^+$.

**Step B:** (*R*)-2-(4-Oxo-5-((2-(trimethylsilyl)ethoxy)methyl)-4,5-dihydro-1*H*-pyrrolo[3,2-*d*]pyridazin-1-yl)propanoic acid (250 mg, 0.57 mmol) was dissolved in anhydrous tetrahydrofuran (3 mL), then sodium borohydride (45 mg, 1.2 mmol) was added thereto in an ice-water bath, and the reaction mixture was stirred for 10 minutes. Boron trifluoride diethyl etherate (85 mg, 0.6 mmol) was added dropwise thereto, and the reaction mixture was heated to 50°C and stirred for 2 hours after the dropwise addition was completed. The reaction mixture was cooled to room temperature, added with water to quench, and then extracted with ethyl acetate (20 mL × 3). The organic phase was washed with saturated brine (20 mL), then dried over anhydrous sodium sulfate, filtered, and finally concentrated under reduced pressure. The resulting residue was purified by silica gel column chromatography to obtain 135 mg of (*R*)-1-(1-hydroxypropan-2-yl)-5-((2-(trimethylsilyl)ethoxy)methyl)-1,5-dihydro-4*H*-pyrrolo[2,3-*d*]pyridazin-4-one (**18-3**), which was directly used in the next reaction step.

MS (ESI) M/Z: 324.3 $[M+H]^+$.

**Step C:** (*R*)-1-(1-Hydroxypropan-2-yl)-5-((2-(trimethylsilyl)ethoxy)methyl)-1,5-dihydro-4*H*-pyrrolo[2,3-*d*]pyridazin-4-one (135 mg, 0.42 mmol) was dissolved in acetonitrile (3 mL), then *tert*-butyl acrylate (80 mg, 0.63 mmol) and cesium carbonate (410 mg, 1.2 mmol) were added thereto, and the reaction mixture was heated to 90°C under microwave irradiation and stirred for 30 minutes. The reaction mixture was cooled to room temperature, added with water to quench, and then extracted with ethyl acetate (20 mL × 3). The organic phase was washed with saturated brine (20 mL), then dried over anhydrous sodium sulfate, filtered, and finally concentrated under reduced pressure. The resulting residue was purified by silica gel column chromatography to obtain 50 mg of *tert*-butyl (*R*)-3-(2-(4-oxo-5-((2-(trimethylsilyl)ethoxy)methyl)-4,5-dihydro-1*H*-pyrrolo[2,3-*d*]pyridazin-1-yl)propoxy) (**18-4**), which was directly used in the next reaction step.

MS (ESI) M/Z: 452.2 $[M+H]^+$.

**Step D:** *tert*-Butyl (*R*)-3-(2-(4-oxo-5-((2-(trimethylsilyl)ethoxy)methyl)-4,5-dihydro-1*H*-pyrrolo[2,3-*d*]pyridazin-1-yl)propoxy) (50 mg, 111 μmol) was dissolved in dichloromethane (2 mL) at room temperature, and trifluoroacetic acid (2 mL) was added thereto. The reaction mixture was stirred at room temperature for another 3 hours. The reaction mixture was concentrated under reduced pressure. The resulting residue was diluted with dichloromethane (50 mL), and then neutralized with saturated sodium bicarbonate solution to adjust the pH to neutrality. The aqueous phase was extracted with dichloromethane (50 mL × 2), and the organic phase was washed with saturated brine (20 mL), dried over anhydrous sodium sulfate, filtered, and finally concentrated under reduced pressure to obtain 50 mg of (*R*)-3-(2-(4-oxo-4,5-dihydro-1*H*-pyrrolo[2,3-*d*]pyridazin-1-yl)propoxy)propanoic acid (**18-5**), which was directly used in the next reaction step.

MS (ESI) M/Z: 265.8 $[M+H]^+$.

**Step E:** (R)-3-(2-(4-Oxo-4,5-dihydro-1*H*-pyrrolo[2,3-*d*]pyridazin-1-yl)propoxy)propanoic acid (50 mg, 0.18 mmol) was dissolved in dichloromethane (5 mL) and *N,N*-dimethylformamide (5 mL) at room temperature. 2-(Piperazin-1-yl)-5-(trifluoromethyl)pyrimidine (44 mg, 0.18 mmol), HATU (55 mg, 0.26 mmol), and *N,N*-diisopropylethylamine (77 mg, 0.6 mmol) were then sequentially added to the above solution in an ice-water bath. The reaction mixture was stirred at room temperature for another 2 hours, added with dilute hydrochloric acid to adjust the pH to 5 to 6, and then extracted with ethyl acetate (20 mL × 3). The organic phase was washed with saturated brine (20 mL), then dried over anhydrous sodium sulfate, filtered, and finally concentrated under reduced pressure. The resulting residue was purified by preparative high performance liquid chromatography to obtain 6 mg of (*R*)-1-(1-(3-oxo-3-(4-(5-(trifluoromethyl)pyrimidin-2-yl)piperazin-1-yl)propoxy-2-yl)-1,5-dihydro-4*H*-pyrrolo[2,3-*d*]pyridazin-4-one (compound **18**).

MS (ESI) M/Z: 480.0 $[M+H]^+$.

$^1H$ NMR (400 MHz, $CDCl_3$) δ 10.10 (brs, 1H), 8.51 (s, 2H), 8.15 (brs, 1H), 7.20 (d, *J* = 3.0 Hz, 1H), 6.90 (d, *J* = 3.0 Hz, 1H), 4.70 - 4.58 (m, 1H), 3.86 (q, *J* = 5.1 Hz, 4H), 3.83 - 3.75 (m, 2H), 3.70 - 3.60 (m, 4H), 3.47 - 3.41 (m, 2H), 2.55 - 2.41 (m, 2H), 1.58 (d, *J* = 7.0 Hz, 3H).

**Example 19**

**1-(2-(3-Oxo-3-(4-(5-(trifluoromethyl)pyrimidin-2-yl)piperazin-1-yl)propoxy)ethyl)-1,5-dihydro-4*H*-pyrrolo[3,2-*c*]pyridin-4-one**

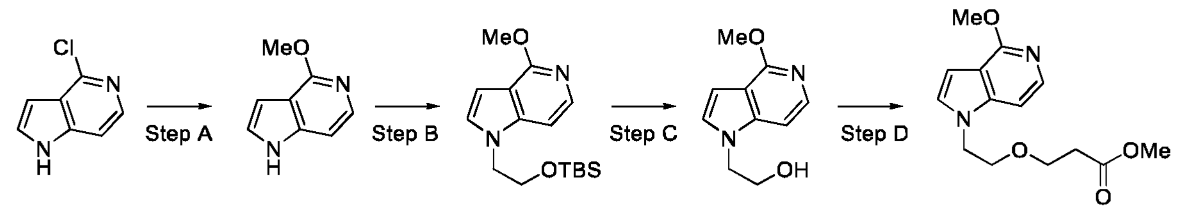


19-1 19-2 19-3 19-4 19-5

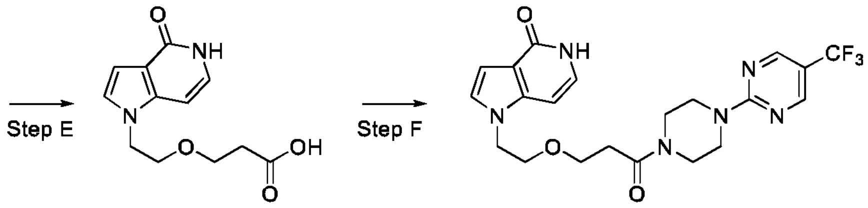


19-6 compound 19

**Step A:** 4-Chloro-5-azaindole (1.6 g, 10.5 mmol) was dissolved in methanol (5 mL) and dioxane (5 mL), then sodium methoxide (2.8 g, 52.5 mmol) was added thereto, and the reaction mixture was heated to 140°C under microwave irradiation and stirred for 40 minutes. The reaction mixture was cooled to room temperature, and added with water to quench. The aqueous phase was extracted with ethyl acetate (50 mL × 3), and the organic phases were combined. The combined organic phases were first washed with saturated brine (30 mL × 1), then dried over anhydrous sodium sulfate, filtered, and finally concentrated under reduced pressure. The resulting residue was purified by silica gel column chromatography to obtain 940 mg of 4-methoxy-1*H*-pyrrolo[3,2-*c*]pyridine (**19-2**).

MS (ESI) M/Z: 149.1 $[M+H]^+$.

**Step B:** 4-Methoxy-1*H*-pyrrolo[3,2-*c*]pyridine (200 mg, 1.35 mmol) was dissolved in anhydrous *N,N*-dimethylformamide (10 mL), then 60% sodium hydride (100 mg, 2.70 mmol) was added thereto in an ice bath, and the reaction mixture was stirred at room temperature for 30 minutes. (2-Bromoethoxy)(*tert*-butyl)dimethylsilane (390 mg, 1.6 mmol) was then added dropwise thereto, and the reaction mixture was stirred at room temperature overnight after the dropwise addition was completed. The reaction mixture was poured into water (10 mL) and stirred at room temperature for 30 minutes. The aqueous phase was then neutralized with citric acid to adjust the pH to 6 to 7, extracted with ethyl acetate (50 mL × 3), and the organic phases were combined. The combined organic phases were first washed with saturated brine (20 mL × 2), then dried over anhydrous sodium sulfate, filtered, and finally concentrated under reduced pressure. The resulting residue was purified by silica gel column chromatography to obtain 180 mg of 1-(2-(*tert*-butyldimethylsilyloxy)ethyl)-4-methoxy-1*H*-pyrrolo[3,2-c]pyridine (**19-3**), which was directly used in the next reaction step.

MS (ESI) M/Z: 307.4 $[M+H]^+$.

**Step C:** 1-(2-(*tert*-Butyldimethylsilyloxy)ethyl)-4-methoxy-1*H*-pyrrolo[3,2-c]pyridine (180 mg, 0.59 mmol) was dissolved in anhydrous tetrahydrofuran (5 mL), then tetrabutylammonium fluoride trihydrate (280 mg, 0.88 mmol) was added thereto in an ice-water bath, and the reaction mixture was stirred at room temperature for 2 hours. The reaction mixture was added with water to quench, and then extracted with ethyl acetate (50 mL × 3). The organic phase was washed with saturated brine (20 mL), then dried over anhydrous sodium sulfate, filtered, and finally concentrated under reduced pressure to obtain 180 mg of 2-(4-methoxy-1*H*-pyrrolo[3,2-*c*]pyridin-1-yl)ethan-1-ol (**19-4**).

MS (ESI) M/Z: 192.9 $[M+H]^+$.

**Step D:** 2-(4-Methoxy-1*H*-pyrrolo[3,2-*c*]pyridin-1-yl)ethan-1-ol (100 mg, 0.52 mmol) was dissolved in acetonitrile (3 mL), then methyl acrylate (67 mg, 0.78 mmol) and cesium carbonate (500 mg, 1.56 mmol) were added thereto, and the reaction mixture was stirred at room temperature overnight. The reaction mixture was added with water to quench, and then extracted with ethyl acetate (20 mL × 3). The organic phase was washed with saturated brine (20 mL), then dried over anhydrous sodium sulfate, filtered, and finally concentrated under reduced pressure. The resulting residue was purified by silica gel column chromatography to obtain 95 mg of methyl 3-(2-(4-methoxy-1*H*-pyrrolo[3,2-*c*]pyridin-1-yl)ethoxy)propanoate (**19-5**), which was directly used in the next reaction step.

MS (ESI) M/Z: 279.1 $[M+H]^+$.

**Step E:** Methyl 3-(2-(4-methoxy-1*H*-pyrrolo[3,2-*c*]pyridin-1-yl)ethoxy)propanoate (50 mg, 0.18 μmol) was dissolved in glacial acetic acid (2 mL) at room temperature, and 48% hydrobromic acid aqueous solution (3 mL) was added thereto. The reaction mixture was heated to 95°C and stirred overnight. The reaction mixture was cooled to room temperature and concentrated under reduced pressure. The resulting residue was purified by preparative high performance liquid chromatography to obtain 30 mg of 3-(2-(4-oxo-4,5-dihydro-1*H*-pyrrolo[3,2-*c*]pyridin-1-yl)ethoxy)propanoic acid (**19-6**), which was directly used in the next reaction step.

MS (ESI) M/Z: 251.1 $[M+H]^+$.

**Step F:** 3-(2-(4-Oxo-4,5-dihydro-1*H*-pyrrolo[3,2-*c*]pyridin-1-yl)ethoxy)propanoic acid (30 mg, 0.12 mmol) was dissolved in dichloromethane (3 mL) and *N,N*-dimethylformamide (3 mL) at room temperature. 2-(Piperazin-1-yl)-5-(trifluoromethyl)pyrimidine (34 mg, 0.14 mmol), HATU (55 mg, 0.14 mmol), and *N,N*-diisopropylethylamine (50 mg, 0.36 mmol) were then sequentially added to the above solution in an ice-water bath. The reaction mixture was stirred at room temperature for another 2 hours, added with dilute hydrochloric acid to adjust the pH to 5 to 6, and then extracted with ethyl acetate (20 mL × 3). The organic phase was washed with saturated brine (20 mL), then dried over anhydrous sodium sulfate, filtered, and finally concentrated under reduced pressure. The resulting residue was purified by preparative high performance liquid chromatography to obtain 9 mg of 1-(2-(3-oxo-3-(4-(5-(trifluoromethyl)pyrimidin-2-yl)piperazin-1-yl)propoxy)ethyl)-1,5-dihydro-4*H*-pyrrolo[3,2-c]pyridin-4-one (compound **19**).

MS (ESI) M/Z: 465.4 $[M+H]^+$.

$^1H$ NMR (400 MHz, $CDCl_3$) δ 11.30 (s, 1H), 8.48 (s, 2H), 7.18 - 7.12 (m, 1H), 6.97 (d, *J* = 3.1 Hz, 1H), 6.82 (d, *J* = 3.1 Hz, 1H), 6.47 (d, *J* = 7.2 Hz, 1H), 4.19 (t, *J* = 5.3 Hz, 2H), 3.89 - 3.83 (m, 4H), 3.80 - 3.72 (m, 4H), 3.71 - 3.64 (m, 2H), 3.51 - 3.43 (m, 2H), 2.52 (t, *J*= 6.4 Hz, 2H).

**Example 20**

**(*R*)-1-(1-(3-Oxo-3-(4-(5-(trifluoromethyl)pyrimidin-2-yl)piperazin-1-yl)propoxy-2-yl)-1,5-dihydro-4*H*-pyrrolo[3,2-*c*]pyridin-4-one**

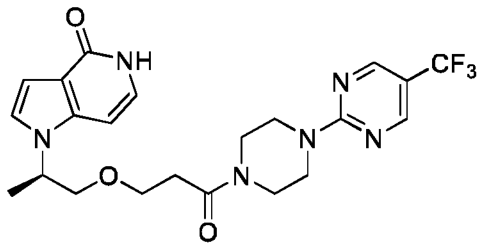

4-Methoxy-1*H*-pyrrolo[3,2-*c*]pyridine (**19-2**) was used as a raw material instead of 5-((2-(trimethylsilyl)ethoxy)methyl)-1,5-dihydro-4*H*-pyrrolo[2,3-*d*]pyridazin-4-one (**16-6**) with the preparation method referring to example **16** and example **19**.

MS (ESI) M/Z: 479.4 $[M+H]^+$.

$^1H$ NMR (400 MHz, DMSO-$d_6$) δ 10.73 (d, *J* = 5.3 Hz, 1H), 8.73 (s, 2H), 7.21 (d, *J* = 3.2 Hz, 1H), 7.00 - 6.91 (m, 1H), 6.54 (d, *J* = 7.2 Hz, 1H), 6.49 (d, *J* = 3.1 Hz, 1H), 4.68 - 4.58 (m, 1H), 3.84 - 3.72 (m, 4H), 3.72 - 3.55 (m, 4H), 3.55 - 3.42 (m, 4H), 1.37 (d, *J* = 6.8 Hz, 3H).

**Example 21**

**(*S*)-1-(1-(3-Oxo-3-(4-(5-(trifluoromethyl)pyrimidin-2-yl)piperazin-1-yl)propoxy-2-yl)-1,5-dihydro-4*H*-pyrrolo[3,2-*c*]pyridin-4-one**

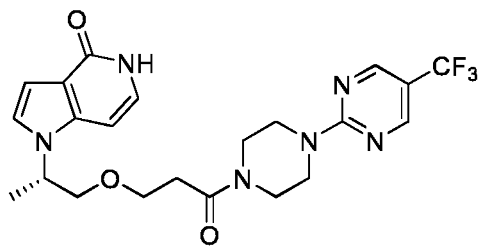

4-Methoxy-1*H*-pyrrolo[3,2-*c*]pyridine (**19-2**) was used as a raw material instead of 5-((2-(trimethylsilyl)ethoxy)methyl)-1,5-dihydro-4*H*-pyrrolo[2,3-*d*]pyridazin-4-one (**16-6**) with the preparation method referring to example **16** and example **19**.

MS (ESI) M/Z: 479.3 $[M+H]^+$.

$^1H$ NMR (400 MHz, DMSO-$d_6$) δ 10.73 (d, $J$ = 5.3 Hz, 1H), 8.73 (s, 2H), 7.21 (d, $J$ = 3.2 Hz, 1H), 7.01 - 6.93 (m, 1H), 6.54 (d, $J$ = 7.2 Hz, 1H), 6.49 (d, $J$ = 3.1 Hz, 1H), 4.69 - 4.58 (m, 1H), 3.85 - 3.73 (m, 4H), 3.71 - 3.54 (m, 4H), 3.54 - 3.42 (m, 4H), 2.76 - 2.69 (m, 1H), 1.37 (d, $J$= 6.9 Hz, 3H).

**Example 22**

**1-(2-(3-Oxo-3-(4-(5-(trifluoromethyl)pyrimidin-2-yl)piperazin-1-yl)propoxy)ethyl)pyrido[2,3-*d*]pyridazine-2,5(1*H*,6*H*)-dione**

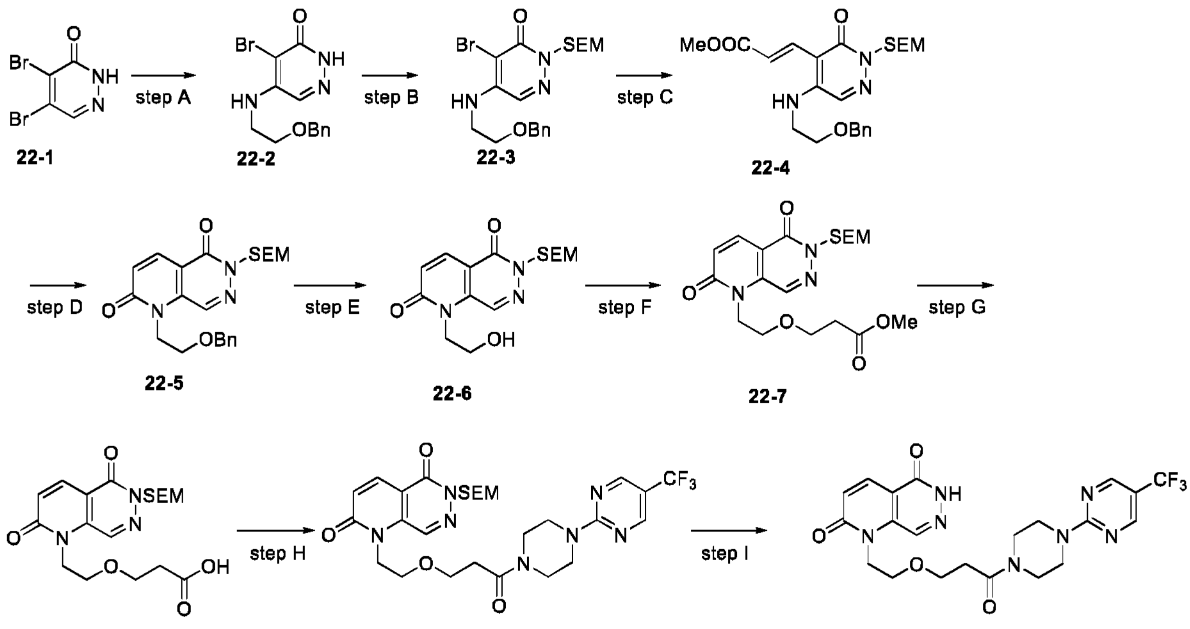


22-1 22-2 22-3 22-4 22-5 22-6 22-7 22-8 22-9 compound 22

**Step A:** 4,5-Dibromopyridazin-3-one (2.0 g, 7.9 mmol) was dissolved in *N,N*-dimethylformamide (15 mL), then *N,N*-diisopropylethylamine (2.0 g, 15.8 mmol) and 2-(benzyloxy)-1-ethanamine (1.8 g, 11.8 mmol) were added thereto, and the reaction mixture was heated to 125°C under microwave irradiation and stirred for 40 minutes. The reaction mixture was cooled to room temperature, and added with water to quench. The aqueous phase was extracted with ethyl acetate (50 mL × 3), and the organic phases were combined. The combined organic phases were first washed with saturated brine (30 mL × 1), then dried over anhydrous sodium sulfate, filtered, and finally concentrated under reduced pressure. The resulting residue was purified by silica gel column chromatography to obtain 1.2 g of 5-((2-(benzyloxy)ethyl)amino)-4-bromopyridazin-3(2*H*)-one (**22-2**).

MS (ESI) M/Z: 325.7 $[M+H]^+$.

$^1H$ NMR (400 MHz, DMSO-$d_6$) δ 12.50 (s, 1H), 7.79 (s, 1H), 7.40 - 7.20 (m, 5H), 6.42 (s, 1H), 4.49 (s, 2H), 3.61 - 3.51 (m, 4H).

**Step B:** 5-((2-(Benzyloxy)ethyl)amino)-4-bromopyridazin-3(2*H*)-one (1.0 g, 3.1 mmol) was dissolved in anhydrous tetrahydrofuran (10 mL), then 60% sodium hydride (250 mg, 6.2 mmol) was added thereto in an ice bath, and the reaction mixture was stirred at room temperature for 30 minutes. (2-Bromoethoxy)(*tert*-butyl)dimethylsilane (1.0 g, 6.2 mmol) was then added dropwise thereto, and the reaction mixture was stirred at room temperature for 3 hours after the dropwise addition was completed. The reaction mixture was poured into water (10 mL) and stirred at room temperature for 30 minutes. The aqueous phase was then neutralized with citric acid to adjust the pH to 6 to 7, extracted with ethyl acetate (50 mL × 3), and the organic phases were combined. The combined organic phases were first washed with saturated brine (20 mL × 2), then dried over anhydrous sodium sulfate, filtered, and finally concentrated under reduced pressure. The resulting residue was purified by silica gel column chromatography to obtain 320 mg of 5-((2-(benzyloxy)ethyl)amino)-4-bromo-2-((2-(trimethylsilyl)ethoxy)methyl)pyridazin-3(2*H*)-one (**22-3**).

MS (ESI) M/Z: 455.9 $[M+H]^+$.

**Step C:** 5-((2-(Benzyloxy)ethyl)amino)-4-bromo-2-((2-(trimethylsilyl)ethoxy)methyl)pyridazin-3(2*H*)-one (200 mg, 0.44 mmol) was dissolved in anhydrous *N,N*-dimethylformamide (10 mL), and methyl acrylate (76 mg, 0.88 mmol),

triethylamine (89 mg, 0.88 mmol), and Xphos-Pd (G2) (catalytic amount) were added thereto. The reaction mixture was replaced with nitrogen to remove oxygen, heated to 120°C under microwave irradiation and stirred for 40 minutes. The reaction mixture was cooled to room temperature, added with water to quench, and then extracted with ethyl acetate (50 mL × 3). The organic phase was washed with saturated brine (20 mL), then dried over anhydrous sodium sulfate, filtered, and finally concentrated under reduced pressure. The resulting residue was purified by silica gel column chromatography to obtain 170 mg of methyl (*E*)-3-(5-((2-(benzyloxy)ethyl)amino)-3-oxo-2-((2-(trimethylsilyl)ethoxy)methyl)-2,3-dihydropyridazin-4-yl)acrylate (**22-4**).

MS (ESI) M/Z: 460.3 $[M+H]^+$.

**Step D:** Methyl (*E*)-3-(5-((2-(benzyloxy)ethyl)amino)-3-oxo-2-((2-(trimethylsilyl)ethoxy)methyl)-2,3-dihydropyridazin-4-yl)acrylate (170 mg, 0.37 mmol) was dissolved in methanol (5 mL), then sodium methoxide (40 mg, 0.75 mmol) was added thereto, and the reaction mixture was heated to 70°C and stirred for 2 hours. The reaction mixture was added with water to quench, and extracted with ethyl acetate (50 mL × 3). The organic phase was washed with saturated brine (20 mL), then dried over anhydrous sodium sulfate, filtered, and finally concentrated under reduced pressure to obtain 120 mg of 1-(2-(benzyloxy)ethyl)-6-((2-(trimethylsilyl)ethoxy)methyl)pyrido[2,3-*d*]pyridazine-2,5(1*H*,6*H*)-dione (**22-5**).

MS (ESI) M/Z: 428.3 $[M+H]^+$.

**Step E:** 1-(2-(Benzyloxy)ethyl)-6-((2-(trimethylsilyl)ethoxy)methyl)pyrido[2,3-*d*]pyridazine-2,5(1*H*,6*H*)-dione (340 mg, 0.79 mmol) was dissolved in methanol (10 mL), and then palladium on carbon (catalytic amount) was added thereto. The reaction mixture was replaced with hydrogen, and then stirred at room temperature for 4 to 5 hours. The reaction mixture was filtered and concentrated under reduced pressure to obtain 230 mg of 1-(2-hydroxyethyl)-6-((2-(trimethylsilyl)ethoxy)methyl)pyrido[2,3-*d*]pyridazine-2,5(1*H*,6*H*-dione (**22-6**).

MS (ESI) M/Z: 338.1 $[M+H]^+$.

**Step F:** 1-(2-Hydroxyethyl)-6-((2-(trimethylsilyl)ethoxy)methyl)pyrido[2,3-*d*]pyridazine-2,5(1*H*,6*H*)-dione (180 mg, 0.54 mmol) was dissolved in *N,N*-dimethylformamide (5 mL), then methyl acrylate (230 mg, 2.67 mmol) and cesium carbonate (525 mg, 1.60 mmol) were added thereto, and the reaction mixture was stirred at room temperature overnight. The reaction mixture was added with water to quench, and then extracted with ethyl acetate (50 mL × 3). The organic phase was washed with saturated brine (20 mL), then dried over anhydrous sodium sulfate, filtered, and finally concentrated under reduced pressure. The resulting residue was purified by preparative high performance liquid chromatography to obtain 56 mg of methyl 3-(2-(2,5-dioxo-6-((2-(trimethylsilyl)ethoxy)methyl)-5,6-dihydropyrido[2,3-*d*]pyridazin-1(2*H*)-yl)ethoxy)propanoate (**22-7**).

MS (ESI) M/Z: 446.3 $[M+Na]^+$.

**Step G:** Methyl 3-(2-(2,5-dioxo-6-((2-(trimethylsilyl)ethoxy)methyl)-5,6-dihydropyrido[2,3-*d*]pyridazin-1(2*H*)-yl)ethoxy)propanoate (56 mg, 0.13 mmol) was dissolved in methanol (2 mL) and water (2 mL), then sodium hydroxide (10 mg, 0.26 mmol) was added thereto, and the reaction mixture was stirred at room temperature for 2 hours. The reaction mixture was added with water to quench, acidified with dilute hydrochloric acid to adjust the pH to 2 to 3, and then extracted with ethyl acetate (50 mL × 3). The organic phase was washed with saturated brine (20 mL), then dried over anhydrous sodium sulfate, filtered, and finally concentrated under reduced pressure to obtain 38 mg of 3-(2-(2,5-dioxo-6-((2-(trimethylsilyl)ethoxy)methyl)-5,6-dihydropyrido[2,3-*d*]pyridazin-1(2*H*)-yl)ethoxy)propanoic acid (**22-8**). The crude product was directly used in the next reaction step.

**Step H:** 3-(2-(2,5-Dioxo-6-((2-(trimethylsilyl)ethoxy)methyl)-5,6-dihydropyrido[3,2-*d*]pyridazin-1(2*H*)-yl)ethoxy)propanoic acid (36 mg, 88 μmol) was dissolved in dichloromethane (3 mL) and *N,N*-dimethylformamide (3 mL). 2-(Piperazin-1-yl)-5-(trifluoromethyl)pyrimidine (23 mg, 97 μmol), HATU (100 mg, 97 μmol), and *N,N*-diisopropylethylamine (50 mg, 0.36 mmol) were then sequentially added to the above solution in an ice-water bath. The reaction mixture was stirred at room temperature for another 2 hours. The reaction mixture was added with dilute hydrochloric acid to adjust the pH to 5 to 6, and extracted with ethyl acetate (20 mL × 3). The organic phase was washed with saturated brine (20 mL), then dried over anhydrous sodium sulfate, filtered, and finally concentrated under reduced pressure to obtain 80 mg of 1-(2-(3-oxo-3-(4-(5-(trifluoromethyl)pyrimidin-2-yl)piperazin-1-yl)propoxy)ethyl)-6-((2-(trimethylsilyl)ethoxy)methyl)pyrido[2,3-*d*]pyridazine-2,5(1*H*,6*H*)-dione (**22-9**). The crude product was directly used in the next reaction step.

MS (ESI) M/Z: 646.2 $[M+Na]^+$.

**Step I:** 1-(2-(3-Oxo-3-(4-(5-(trifluoromethyl)pyrimidin-2-yl)piperazin-1-yl)propoxy)ethyl)-6-((2-(trimethylsilyl)ethoxy)methyl)pyrido[3,2-*d*]pyridazine-2,5(1*H*,6*H*)-dione (80 mg, 88 μmol) was dissolved in dichloromethane (1 mL) at room temperature, and then trifluoroacetic acid (1 mL) was added thereto. The reaction mixture was stirred at room temperature for another 2 hours. The reaction mixture was concentrated under reduced pressure, and the residue was purified by preparative high performance liquid chromatography to obtain 5.5 mg of 1-(2-(3-oxo-3-(4-(5-(trifluoromethyl)pyrimidin-2-yl)piperazin-1-yl)propoxy)ethyl)pyrido[2,3-*d*]pyridazine-2,5(1*H*,6*H*)-dione (compound **22**).

MS (ESI) M/Z: 494.1 $[M+H]^+$.

$^1$H NMR (400 MHz, DMSO-$d_6$) δ 13.06 (s, 1H), 8.73 (s, 2H), 8.39 (s, 1H), 8.01 (d, *J* = 9.5 Hz, 1H), 6.78 (d, *J* = 9.5 Hz, 1H), 4.41 (t, *J* = 4.9 Hz, 2H), 3.83 - 3.73 (m, 4H), 3.67 (t, *J* = 5.0 Hz, 2H), 3.62 (t, *J* = 6.2 Hz, 2H), 3.51 - 3.43

(m, 4H), 2.49 - 2.45 (m, 2H).

**Example 23**

**6-(2-Oxo-2-(4-(5-(trifluoromethyl)pyrimidin-2-yl)piperazin-1-yl)ethyl)-4-(trifluoromethyl)pyridazin-3(2*H*)-one**

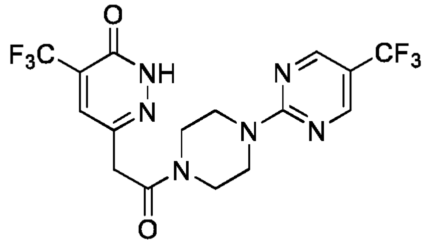

Intermediate **1C** was used instead of **1A** with the preparation method referring to example **1**.
MS (ESI) M/Z: 437.3 $[M+H]^+$.
$^1H$ NMR (400 MHz, DMSO-$d_6$) δ 13.54 (s, 1H), 8.75 (s, 2H), 7.79 (s, 1H), 3.95 - 3.89 (m, 2H), 3.88 (s, 2H), 3.87 - 3.81 (m, 2H), 3.66 - 3.61 (m, 2H), 3.61 - 3.55 (m, 2H).

**Example 24**

**5-(2-Oxo-2-(4-(5-(trifluoromethyl)pyrimidin-2-yl)piperazin-1-yl)ethyl)-3-(trifluoromethyl)pyridin-2(1*H*)-one**

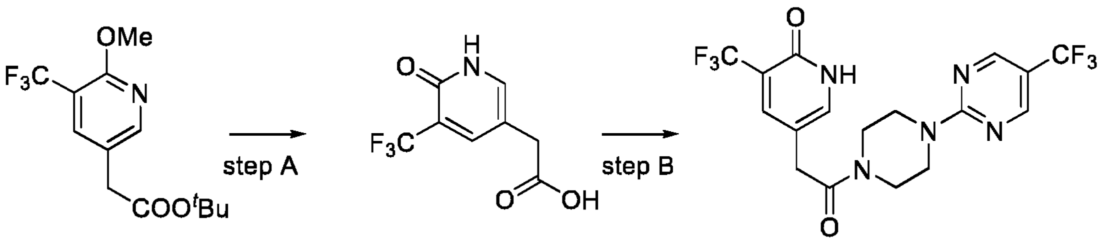


**3-2** **24-2** **example 24**

**Step A:** tert-Butyl 2-(6-methoxy-5-(trifluoromethyl)pyridin-3-yl)acetate (200 mg, 0.45 mmol) was dissolved in glacial acetic acid (2 mL) and water (2 mL), then 48% hydrobromic acid (1 mL) was added thereto, and the reaction mixture was heated to 100°C and stirred for 2 hours. The reaction mixture was cooled to room temperature and concentrated under reduced pressure. The residue was neutralized with saturated sodium bicarbonate solution to adjust the pH to 4 to 5. The aqueous phase was extracted with ethyl acetate (30 mL × 3), and the organic phases were combined. The combined organic phases were first washed with saturated brine (20 mL × 1),then dried over anhydrous sodium sulfate, filtered, and finally concentrated under reduced pressure to obtain 125 mg of 2-(6-oxo-5-(trifluoromethyl)-1,6-dihydropyridin-3-yl)acetic acid (**24-2**).
MS (ESI) M/Z: 221.8 $[M+H]^+$.
**Step B:** 2-(6-Oxo-5-(trifluoromethyl)-1,6-dihydropyridin-3-yl)acetic acid (**24-2**) and 2-(piperazin-1-yl)-5-(trifluoromethyl)pyrimidine (**1C**) were used as raw materials with the preparation method referring to example **1**.
MS (ESI) M/Z: 436.2 $[M+H]^+$.
$^1H$ NMR (400 MHz, $CDCl_3$) δ 8.55 - 8.50 (m, 2H), 7.84 (d, *J* = 2.0 Hz, 1H), 7.51 (d, *J* = 2.2 Hz, 1H), 4.03 - 3.96 (m, 2H), 3.96 - 3.90 (m, 2H), 3.78 - 3.69 (m, 2H), 3.65 - 3.59 (m, 2H), 3.55 (s, 2H).

**Example 25**

**2-(6-Oxo-5-(trifluoromethyl)-1,6-dihydropyridin-3-yl)propyl 4-(5-(trifluoromethyl)pyrimidin-2-yl)piperazine-1-carboxylate**

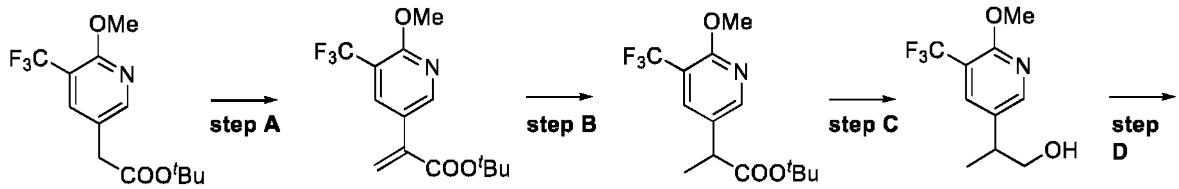


3-2 25-2 25-3 25-4

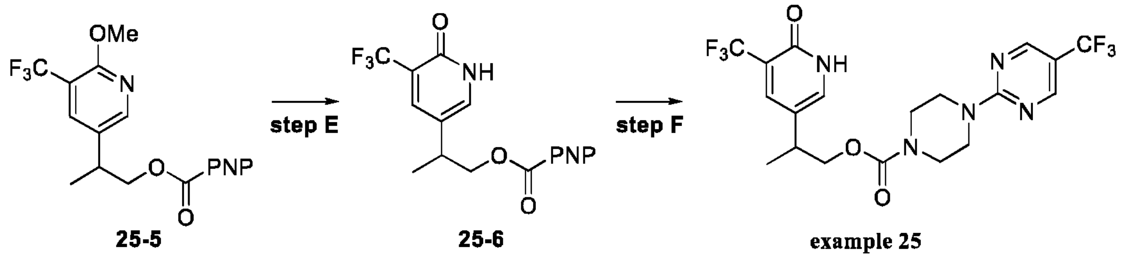


25-5 25-6 example 25

**Step A:** *tert*-Butyl 2-(6-methoxy-5-(trifluoromethyl)pyridin-3-yl)acetate (1.0 g, 3.43 mmol) was dissolved in toluene (30 mL), and then paraformaldehyde (463 mg, 5.15 mmol), potassium carbonate (948.1 mg, 6.86 mmol), and tetra-n-butylammonium hydrogen sulfate (116.4 mg, 0.34 mmol) were sequentially added thereto. After the addition was completed, the reaction system was stirred at 80°C for 0.5 hours until the raw material disappeared. The reaction mixture was cooled to room temperature, filtered, and the filter cake was washed with ethyl acetate (50 mL × 3). The organic phase was concentrated under reduced pressure, and the resulting residue was purified by silica gel column chromatography to obtain 801 mg of tert-butyl 2-(6-methoxy-5-(trifluoromethyl)pyridin-3-yl)acrylate (**25-2**).

MS (ESI) M/Z: 304.1 $[M+H]^+$.

$^1H$ NMR (400 MHz, $CDCl_3$) δ 8.33 (d, *J* = 2.0 Hz, 1H), 7.96 (d, *J* = 1.9 Hz, 1H), 6.36 (d, *J* = 0.9 Hz, 1H), 5.85 (d, *J* = 0.9 Hz, 1H), 4.04 (s, 3H), 1.53 (s, 9H).

**Step B:** tert-Butyl 2-(6-methoxy-5-(trifluoromethyl)pyridin-3-yl)acrylate (801 mg, 2.64 mmol) was dissolved in methanol (15 mL), and then Pd/C (80 mg) was added thereto. The reaction mixture was replaced with hydrogen three times, and reacted at room temperature for 0.5 hours until the raw material disappeared. The reaction mixture was filtered, and washed with ethyl acetate (10 mL × 3). The organic phase was concentrated under reduced pressure, and the resulting residue was purified by silica gel column chromatography to obtain 403.1 mg of *tert*-butyl 2-(6-methoxy-5-(trifluoromethyl)pyridin-3-yl)propanoate (**25-3**).

MS (ESI) M/Z: 306.2 $[M+H]^+$.

**Step C:** *tert*-Butyl 2-(6-methoxy-5-(trifluoromethyl)pyridin-3-yl)propanoate (403.1 mg, 1.32 mmol) and tetrahydrofuran (10 mL) were added to a dry, 100 mL three-necked flask replaced with nitrogen, then the reaction mixture was cooled to 0°C, and a solution of lithium aluminum hydride in tetrahydrofuran (1.2 mL, 2.5 mol/L) was slowly added dropwise to the system. After the addition was completed, the reaction mixture was warmed to room temperature and reacted for 1 hour. The system was added with ethyl acetate (50 mL), then cooled to 0°C, and slowly added dropwise with water (10 mL). The aqueous phase was extracted with ethyl acetate (10 mL × 3), and the organic phase was concentrated under reduced pressure. The resulting residue was purified by silica gel column chromatography to obtain 161.6 mg of 2-(6-methoxy-5-(trifluoromethyl)pyridin-3-yl)propan-1-ol (**25-4**).

MS (ESI) M/Z: 236.2 $[M+H]^+$.

**Step D:** 2-(6-Methoxy-5-(trifluoromethyl)pyridin-3-yl)propan-1-ol (161.6 mg, 0.69 mmol) was dissolved in DMF (10 mL), then bis(4-nitrophenyl) carbonate (312.6 mg, 1.03 mmol) and triethylamine (138.6 mg, 1.37 mmol) were sequentially added thereto, and the system was reacted at room temperature for 1 hour until the raw material disappeared. The system was added with ethyl acetate (150 mL), and then extracted with water (10 mL × 5). The organic phase was concentrated under reduced pressure, and the resulting residue was purified by silica gel column chromatography to obtain 326.9 mg of 2-(6-methoxy-5-(trifluoromethyl)pyridin-3-yl)propyl (4-nitrophenyl) carbonate (**25-5**).

MS (ESI) M/Z: 401.0 $[M+H]^+$.

**Step E:** 2-(6-Methoxy-5-(trifluoromethyl)pyridin-3-yl)propyl (4-nitrophenyl) carbonate (326.9 mg, 0.69 mmol) was dissolved in 1,4-dioxane (5 mL), and then a solution of boron tribromide in dichloromethane (3.45 mL, 1 mol/L) was slowly added dropwise thereto at room temperature. After the addition was completed, the reaction mixture was reacted at room temperature for 3 hours until the raw material disappeared. The reaction system was added with saturated sodium bicarbonate aqueous solution (10 mL) to quench, and then extracted with ethyl acetate (50 mL × 3). The organic phase was concentrated under reduced pressure, and the resulting residue was purified by silica gel column chromatography to obtain 187.2 mg of 2-(6-hydroxy-5-(trifluoromethyl)pyridin-3-yl)propyl (4-nitrophenyl) carbonate (**25-6**).

MS (ESI) M/Z: 387.0 $[M+H]^+$.

**Step F:** 2-(6-Hydroxy-5-(trifluoromethyl)pyridin-3-yl)propyl (4-nitrophenyl) carbonate (187.2 mg, 0.485 mmol) was dissolved in DMF (5 mL), then 2-(piperazin-1-yl)-5-(trifluoromethyl)pyrimidine (123.8 mg, 0.534 mmol) was added thereto, and the reaction mixture was reacted at room temperature for 0.5 hours until the raw material disappeared. The reaction mixture was added with ethyl acetate (100 mL), and then washed with water (10 mL × 5). The organic phase was concentrated, and the residue was separated by high performance liquid chromatography to obtain 182.1 mg of 2-(6-oxo-5-(trifluoromethyl)-1,6-dihydropyridin-3-yl)propyl 4-(5-(trifluoromethyl)pyrimidin-2-yl)piperazine-1-carboxylate (compound **25**).

MS (ESI) M/Z: 480.2 $[M+H]^+$.

$^1H$ NMR (400 MHz, DMSO-$d_6$) δ 12.25 (s, 1H), 8.78 - 8.68 (m, 2H), 7.91 (d, *J* = 2.2 Hz, 1H), 7.57 (d, *J* = 2.1 Hz, 1H), 4.11 - 4.00 (m, 2H), 3.80 (brs, 4H), 3.49 - 3.39 (m, 4H), 3.04 - 2.93 (m, 1H), 1.19 (d, *J* = 7.1 Hz, 3H).

## Example 26 and Example 27

**(*S*)-2-(6-Oxo-5-(trifluoromethyl)-1,6-dihydropyridin-3-yl)propyl 4-(5-(trifluoromethyl)pyrimidin-2-yl)piperazine-1-carboxylate**

**(*R*)-2-(6-Oxo-5-(trifluoromethyl)-1,6-dihydropyridin-3-yl)propyl 4-(5-(trifluoromethyl)pyrimidin-2-yl)piperazine-1-carboxylate**

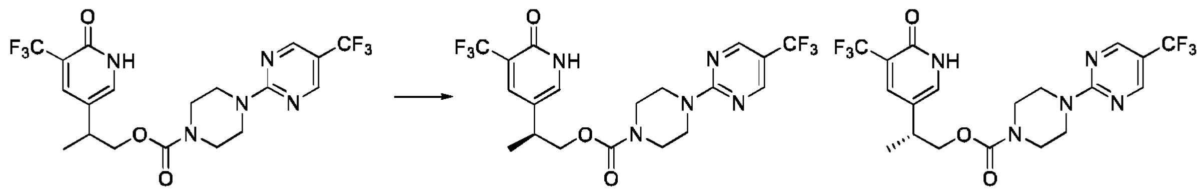

compound 25 compound 26 or compound 27 compound 27 or compound 26

### Compound 26:

Compound **25** was subjected to chiral resolution, and the resolution conditions were as follows:
Chiral column: Daicel IG-3 (25 * 250 mm, 10 μm), temperature: 30°C, mobile phase: $CO_2$/MeOH (0.2% methanol ammonia) = 65/35, flow rate: 100 g/min, pressure: 100 bar, detection wavelength: 214 nm, cycle time: 10 min. Retention time: $t_R$ = 0.987 min, ee value: 100%. Absolute configuration unknown, enantiomer of compound **27**.

MS (ESI) M/Z: 478.1 $[M-H]^-$.

$^1H$ NMR (400 MHz, DMSO-$d_6$) δ 12.24 (s, 1H), 8.73 (s, 2H), 7.91 (d, *J* = 2.0 Hz, 1H), 7.58 (s, 1H), 4.13 - 3.99 (m, 2H), 3.80 (s, 4H), 3.47 - 3.40 (m, 4H), 3.04 - 2.93 (m, 1H), 1.19 (d, *J* = 7.1 Hz, 3H).

### Compound 27:

Compound **25** was subjected to chiral resolution, and the resolution conditions were as follows:
Chiral column: Daicel IG-3 (25 * 250 mm, 10 μm), temperature: 30°C, mobile phase: $CO_2$/MeOH (0.2% methanol ammonia) = 65/35, flow rate: 100 g/min, pressure: 100 bar, detection wavelength: 214 nm, cycle time: 10 min. Retention time: $t_R$ = 1.718 min, ee value: 100%. Absolute configuration unknown, enantiomer of compound **26**.

MS (ESI) M/Z: 478.2 $[M-H]^-$.

$^1H$ NMR (400 MHz, DMSO-$d_6$) δ 12.24 (s, 1H), 8.73 (s, 2H), 7.88 (s, 1H), 7.59 (s, 1H), 4.14 - 4.02 (m, 2H), 3.80 (s, 4H), 3.45 - 3.40 (m, 4H), 3.04 - 2.93 (m, 1H), 1.19 (d, *J* = 7.1 Hz, 3H).

**Example 28**

**5-(3-Oxo-3-(4-(5-(trifluoromethyl)pyrimidin-2-yl)piperazin-1-yl)propyl)-3-(trifluoromethyl)pyridin-2(1*H*)-one**

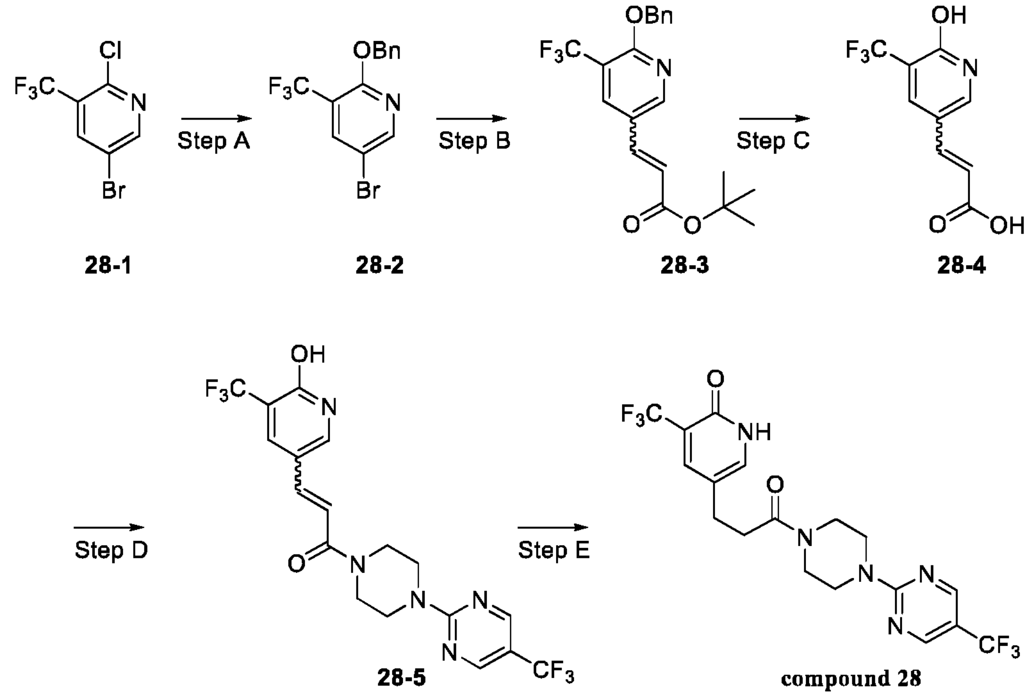


**28-1** **28-2** **28-3** **28-4** **28-5** **compound 28**

**Step A:** Sodium hydride (15 g, 57.6 mmol) was added to a three-necked flask, and the system was replaced with nitrogen, and then *N,N*-dimethylformamide (100 mL) was added thereto in an ice bath. Benzyl alcohol (7.8 g, 72.1 mmol) was slowly added to the above mixture in an ice bath, and the mixture was stirred in an ice bath for 30 minutes. A solution of 5-bromo-2-chloro-3-(trifluoromethyl)pyridine (15 g, 57.6 mmol) in *N,N*-dimethylformamide (20 mL) was added thereto, and the reaction mixture was stirred at room temperature overnight. The reaction mixture was added with water (200 mL) to quench. The mixture was extracted with ethyl acetate (80 mL × 2), and the organic phases were combined. The combined organic phases were first washed with saturated brine (60 mL × 1), then dried over anhydrous sodium sulfate, filtered, and finally concentrated under reduced pressure. The resulting residue was purified by silica gel column chromatography to obtain compound 2-(benzyloxy)-5-bromo-3-(trifluoromethyl)pyridine (12.0 g, white solid, yield: 62.6%).

$^1$H NMR (400 MHz, DMSO-$d_6$) δ 8.62 (d, *J* = 2.0 Hz, 1H), 8.36 (d, *J* = 2.1 Hz, 1H), 7.46 - 7.30 (m, 5H), 5.50 (s, 2H).

**Step B:** Compound 2-(benzyloxy)-5-bromo-3-(trifluoromethyl)pyridine (200 mg, 0.60 mmol), *tert*-butyl acrylate (92 mg, 0.72 mmol), palladium acetate (13 mg, 0.06 mmol), and *N,N*-diisopropylethylamine (154 mg, 1.2 mmol) were dissolved in *N,N*-dimethylformamide (2 mL) in a sealed tank under nitrogen atmosphere. The reaction mixture was heated to 100°C and stirred overnight. The reaction mixture was cooled to room temperature, then concentrated under reduced pressure, and extracted with ethyl acetate (3 × 10 mL). The organic phases were combined, first washed with saturated brine (10 mL), dried over anhydrous sodium sulfate, filtered, and concentrated. The resulting residue was purified by silica gel column chromatography to obtain 142 mg of *tert*-butyl 3-(6-(benzyloxy)-5-(trifluoromethyl)pyridin-3-yl)acrylate (**28-3**).

MS (ESI) M/Z: 380.0 [M+H]$^+$.

**Step C:** Compound *tert*-butyl 3-(6-(benzyloxy)-5-(trifluoromethyl)pyridin-3-yl)acrylate (142 mg, 0.37 mmol) was dissolved in dichloromethane (2 mL) at room temperature. Trifluoroacetic acid (1 mL) was then added to the above solution. The reaction mixture was stirred at room temperature for 2 hours. The reaction mixture was concentrated under reduced pressure to obtain 86 mg of compound 3-(6-hydroxy-5-(trifluoromethyl)pyridin-3-yl)acrylic acid (**28-4**).

MS (ESI) M/Z: 234.0 [M+H]$^+$.

**Step D:** Compound 3-(6-hydroxy-5-(trifluoromethyl)pyridin-3-yl)acrylic acid (86 mg, 0.37 mmol) was dissolved in *N,N*-dimethylformamide (1 mL) at room temperature. 2-(Piperazin-1-yl)-5-(trifluoromethyl)pyrimidine (127 mg, 0.55 mmol), HATU (210 mg, 0.55 mmol), and *N,N*-diisopropylethylamine (143 mg, 1.11 mmol) were then added to the above

solution. The reaction mixture was stirred at room temperature for 2 hours. The reaction mixture was added with water (20 mL) to quench. The mixture was extracted with ethyl acetate (20 mL × 3), and the organic phases were combined. The combined organic phases were first washed with water (10 mL × 3) and saturated brine (10 mL), then dried over anhydrous sodium sulfate, filtered, and finally concentrated under reduced pressure. The resulting residue was purified by silica gel column chromatography to obtain 119 mg of 3-(6-hydroxy-5-(trifluoromethyl)pyridin-3-yl)-1-(4-(5-(trifluoromethyl)pyrimidin-2-yl)piperazin-1-yl)prop-2-en-1-one (**28-5**).

MS (ESI) M/Z: 448.0 $[M+H]^+$.

$^1$H NMR (400 MHz, DMSO-$d_6$) δ 12.64 (s, 1H), 8.75 (s, 2H), 8.50 (d, *J* = 1.8 Hz, 1H), 8.14 (d, *J* = 2.1 Hz, 1H), 7.45 (d, *J* = 15.3 Hz, 1H), 7.19 (d, *J* = 15.3 Hz, 1H), 3.94 - 3.81 (m, 6H), 3.73 - 3.62 (m, 2H).

**Step E:** Compound 3-(6-hydroxy-5-(trifluoromethyl)pyridin-3-yl)-1-(4-(5-(trifluoromethyl)pyrimidin-2-yl)piperazin-1-yl)prop-2-en-1-one (100 mg, 0.22 mmol) was dissolved in methanol (3 mL). 10% palladium on carbon (wet basis, 20 mg) was then added to the above solution. After the reaction system was replaced with hydrogen three times, the reaction mixture was stirred at room temperature for 1 hour. The reaction mixture was filtered through diatomite, and the filter cake was washed with methanol (10 mL × 3). The resulting residue was purified by preparative high performance liquid chromatography to obtain 40 mg of 5-(3-oxo-3-(4-(5-(trifluoromethyl)pyrimidin-2-yl)piperazin-1-yl)propyl)-3-(trifluoromethyl)pyridin-2(1*H*)-one (compound **28**).

MS (ESI) M/Z: 450.0 $[M+H]^+$.

$^1$H NMR (400 MHz, DMSO-$d_6$) δ 12.16 (s, 1H), 8.73 (d, *J* = 0.6 Hz, 2H), 7.92 (d, *J* = 2.0 Hz, 1H), 7.56 (d, *J* = 2.0 Hz, 1H), 3.91 - 3.70 (m, 4H), 3.63 - 3.48 (m, 4H), 2.64 (s, 4H).

**Example 29**

**(*S*)-(1-(6-Oxo-5-(trifluoromethyl)-1,6-dihydropyridin-3-yl)pyrrolidin-2-yl)methyl 4-(5-(trifluoromethyl)pyrimidin-2-yl)piperazine-1-carboxylate**

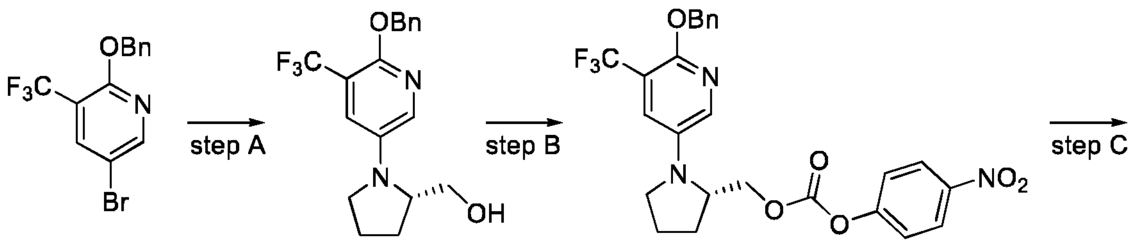


**28-2** **29-2** **29-3**

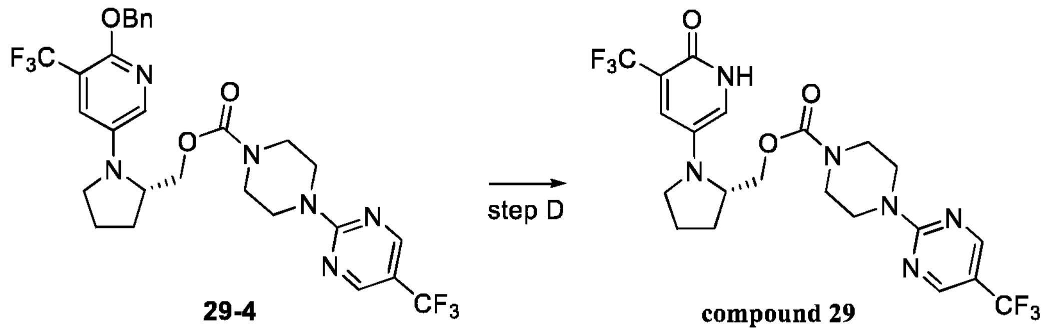


**29-4** **compound 29**

**Step A:** Cuprous iodide (28 mg, 0.15 mmol), *L*-proline (20 mg, 0.18 mmol), 2-(benzyloxy)-5-bromo-3-(trifluoromethyl)pyridine (1.0 g, 3 mmol), (*S*)-pyrrolidin-2-ylmethanol (364 mg, 1.8 mmol), and dimethyl sulfoxide (10 mL) were added to a three-necked flask. The reaction mixture was replaced with nitrogen, then heated to 90°C, and stirred for 2 hours. The reaction mixture was cooled to room temperature, and then added with water (200 mL) to quench. The mixture was extracted with ethyl acetate (80 mL × 2), and the organic phases were combined. The combined organic phases were first washed with saturated brine (60 mL × 1), then dried over anhydrous sodium sulfate, filtered, and finally concentrated under reduced pressure. The resulting residue was purified by silica gel column chromatography to obtain 200 mg of (*S*)-(1-(6-(benzyloxy)-5-(trifluoromethyl)pyridin-3-yl)pyrrolidin-2-yl)methanol (**29-2**).

MS (ESI) M/Z: 353.0 $[M+H]^+$.

**Step B:** (*S*)-(1-(6-(Benzyloxy)-5-(trifluoromethyl)pyridin-3-yl)pyrrolidin-2-yl)methanol (150 mg, 0.43 mmol), bis(4-nitrophenyl) carbonate (259 mg, 0.85 mmol), and diisopropylethylamine (109 mg, 0.85 mmol) were dissolved in *N,N*-dimethylacetamide solution (5 mL). The reaction mixture was stirred at room temperature for 3 hours. The reaction mixture was added with water (200 mL) to quench, and extracted with ethyl acetate (3 × 10 mL). The organic phases were combined, first washed with saturated brine (10 mL), dried over anhydrous sodium sulfate, filtered, and concentrated. The resulting residue was purified by silica gel column chromatography to obtain (*S*)-(1-(6-(benzyloxy)-5-(trifluoromethyl)pyridin-3-yl)pyrrolidin-2-yl)methyl (4-nitrophenyl) carbonate (**29-3**).

MS (ESI) M/Z: 518.0 $[M+H]^+$.

**Step C:** (*S*)-(1-(6-(Benzyloxy)-5-(trifluoromethyl)pyridin-3-yl)pyrrolidin-2-yl)methyl (4-nitrophenyl) carbonate (230 mg, 0.44 mmol) was dissolved in DMF (4 mL) at room temperature. 2-(Piperazin-1-yl)-5-(trifluoromethyl)pyrimidine (103 mg, 0.44 mmol) and *N,N*-diisopropylethylamine (114 mg, 0.88 mmol) were then added to the above solution. The reaction mixture was stirred at room temperature for 3 hours. The reaction mixture was added with water (20 mL) to quench. The mixture was extracted with ethyl acetate (20 mL × 3), and the organic phases were combined. The combined organic phases were first washed with water (10 mL × 3) and saturated brine (10 mL), then dried over anhydrous sodium sulfate, filtered, and finally concentrated under reduced pressure. The resulting residue was purified by silica gel column chromatography to obtain 240 mg of (*S*)-(1-(6-(benzyloxy)-5-(trifluoromethyl)pyridin-3-yl)pyrrolidin-2-yl)methyl 4-(5-(trifluoromethyl)pyrimidin-2-yl)piperazine-1-carboxylate (**29-4**).

**Step D:** (*S*)-(1-(6-(Benzyloxy)-5-(trifluoromethyl)pyridin-3-yl)pyrrolidin-2-yl)methyl 4-(5-(trifluoromethyl)pyrimidin-2-yl)piperazine-1-carboxylate (100 mg, 0.16 mmol) was dissolved in dichloromethane (5 mL). Trifluoroacetic acid (0.5 mL) was then added to the above solution under cooling in an ice bath. The reaction mixture was stirred at room temperature for 1 hour. The reaction mixture was neutralized with sodium bicarbonate to adjust the pH to 8 to 9, extracted with ethyl acetate, and the organic phases were combined. The resulting residue was purified by preparative high performance liquid chromatography to obtain 51 mg of (*S*)-(1-(6-oxo-5-(trifluoromethyl)-1,6-dihydropyridin-3-yl)pyrrolidin-2-yl)methyl 4-(5-(trifluoromethyl)pyrimidin-2-yl)piperazine-1-carboxylate (compound **29**).

MS (ESI) M/Z: 521.0 $[M+H]^+$.

$^1H$ NMR (400 MHz, DMSO-$d_6$) δ 11.82 (s, 1H), 8.73 (s, 2H), 7.71 (d, *J* = 2.4 Hz, 1H), 7.11 (s, 1H), 4.16 - 4.03 (m, 1H), 3.93 - 3.71 (m, 6H), 3.45 (brs, 4H), 3.40 - 3.34 (m, 1H), 3.02 - 2.82 (m, 1H), 2.05 - 1.88 (m, 3H), 1.88 - 1.74 (m, 1H).

## Example 30

### 3-(Trifluoromethyl)-5-(3-(4-(5-(trifluoromethyl)pyrimidin-2-yl)piperazine-1-carbonyl)benzyl)pyridin-2(1*H*)-one

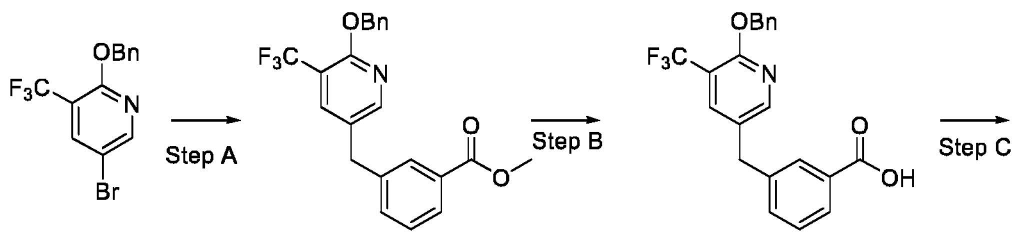


**28-2** **30-2** **30-3**

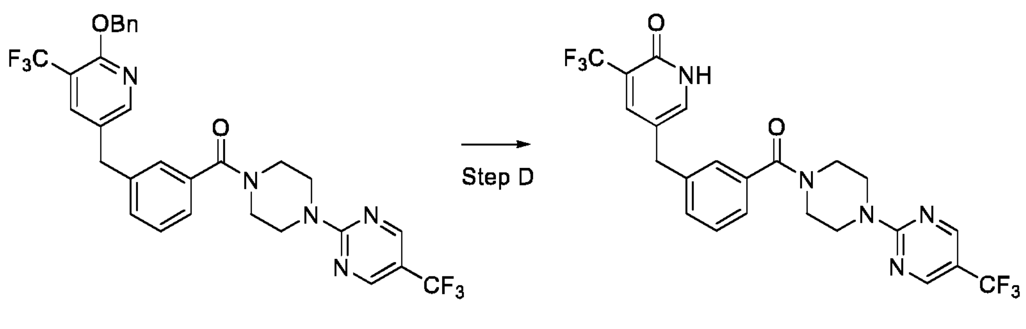


**30-4** **compound 30**

**Step A:** Activated zinc powder (2.0 g, 30.7 mmol) was added to a three-necked flask, and the system was

replaced with nitrogen, and then tetrahydrofuran (10 mL) was added thereto. Chlorotrimethylsilane (0.05 mL) was slowly added to the above solution, which was stirred at room temperature for 45 minutes. Methyl 3-(bromomethyl)benzoate (4.4 g, 19.23 mmol) was added thereto, and the reaction mixture was stirred at 75°C for 1 hour and then cooled to room temperature for later use.

Tris(dibenzylideneacetone)dipalladium (137 mg, 0.15 mmol), 2-dicyclohexylphosphino-2',4',6'-triisopropylbiphenyl (86 mg, 0.18 mmol), and 2-(benzyloxy)-5-bromo-3-(trifluoromethyl)pyridine (1.0 g, 3 mmol) were added to tetrahydrofuran (10.5 mL) in another three-necked flask. The reaction mixture was replaced with nitrogen, then added dropwise with the above zinc reagent, heated to 70°C, and stirred for 16 hours. The reaction mixture was cooled to room temperature, and added with water (200 mL) to quench. The mixture was extracted with ethyl acetate (80 mL × 2), and the organic phases were combined. The combined organic phases were first washed with saturated brine (60 mL × 1), then dried over anhydrous sodium sulfate, filtered, and finally concentrated under reduced pressure. The resulting residue was purified by silica gel column chromatography to obtain 1.1 g of methyl 3-((6-(benzyloxy)-5-(trifluoromethyl)pyridin-3-yl)methyl)benzoate (**30-2**).

MS (ESI) M/Z: 402.0 $[M+H]^+$.

**Step B:** Methyl 3-((6-(benzyloxy)-5-(trifluoromethyl)pyridin-3-yl)methyl)benzoate (900 mg, 2.2 mmol) was dissolved in a solution of lithium hydroxide (917 mg, 22 mmol) in water/tetrahydrofuran (V/V = 1/1, 30 mL). The reaction mixture was heated to 50°C and stirred overnight. The reaction mixture was cooled to room temperature, then the pH was adjusted to 5 to 6, and the reaction mixture was extracted with ethyl acetate (3 × 10 mL). The organic phases were combined, first washed with saturated brine (10 mL), dried over anhydrous sodium sulfate, filtered, and concentrated. The resulting residue was purified by silica gel column chromatography to obtain 480 mg of 3-((6-(benzyloxy)-5-(trifluoromethyl)pyridin-3-yl)methyl)benzoic acid (**30-3**).

MS (ESI) M/Z: 388.0 $[M+H]^+$.

**Step C:** 3-((6-(Benzyloxy)-5-(trifluoromethyl)pyridin-3-yl)methyl)benzoic acid (380 mg, 0.98 mmol) was dissolved in DMF (8 mL) at room temperature. 2-(Piperazin-1-yl)-5-(trifluoromethyl)pyrimidine (341 mg, 1.47 mmol), HATU (560 mg, 1.47 mmol), and *N,N*-diisopropylethylamine (380 mg, 2.94 mmol) were then added to the above solution. The reaction mixture was stirred at room temperature for 16 hours. The reaction mixture was added with water (20 mL) to quench. The mixture was extracted with ethyl acetate (20 mL × 3), and the organic phases were combined. The combined organic phases were first washed with water (10 mL × 3) and saturated brine (10 mL), then dried over anhydrous sodium sulfate, filtered, and finally concentrated under reduced pressure. The resulting residue was purified by silica gel column chromatography to obtain 480 mg of (3-((6-(benzyloxy)-5-(trifluoromethyl)pyridin-3-yl)methyl)phenyl)(4-(5-(trifluoromethyl)pyrimidin-2-yl)piperazin-1-yl)methanone (**30-4**).

MS (ESI) M/Z: 602.4 $[M+H]^+$.

**Step D:** (3-((6-(Benzyloxy)-5-(trifluoromethyl)pyridin-3-yl)methyl)phenyl)(4-(5-(trifluoromethyl)pyrimidin-2-yl)piperazin-1-yl)methanone (200 mg, 0.33 mmol) was dissolved in methanol (8 mL). 10% wet palladium on carbon (176 mg) was then added to the above solution. After the reaction system was replaced with hydrogen three times, the reaction mixture was stirred at room temperature for 1 hour. The reaction mixture was filtered through diatomite, and the filter cake was washed with methanol (10 mL × 3). The resulting residue was purified by preparative high performance liquid chromatography to obtain 147 mg of 3-(trifluoromethyl)-5-(3-(4-(5-(trifluoromethyl)pyrimidin-2-yl)piperazine-1-carbonyl)benzyl)pyridin-2(1*H*)-one (compound **30**).

MS (ESI) M/Z: 512.0 $[M+H]^+$.

$^1H$ NMR (400 MHz, DMSO-$d_6$) δ 12.23 (s, 1H), 8.74 (s, 2H), 7.84 (d, *J* = 2.1 Hz, 1H), 7.64 (d, *J* = 2.0 Hz, 1H), 7.44 - 7.34 (m, 3H), 7.32 - 7.27 (m, 1H), 4.04 - 3.83 (m, 4H), 3.81 (s, 2H), 3.72 (brs, 2H), 3.43 (brs, 2H).

### Example 31

**5-((3-Oxo-3-(4-(5-(trifluoromethyl)pyrimidin-2-yl)piperazin-1-yl)propyl)amino)-3-(trifluoromethyl)pyridin-2(1*H*)-one**

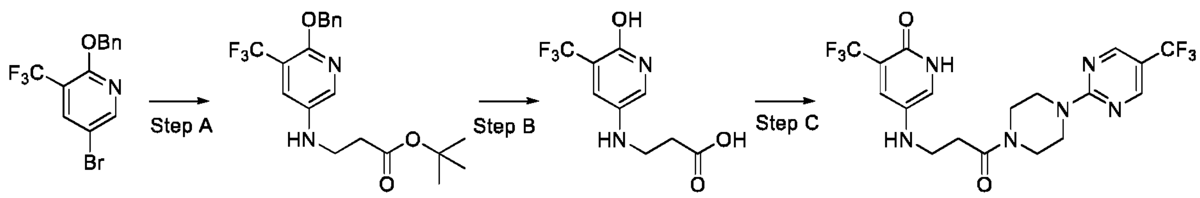


**28-2** **31-2** **31-3** **compound 31**

**Step A:** 2-(Benzyloxy)-5-bromo-3-(trifluoromethyl)pyridine (200 mg, 0.60 mmol), tert-butyl 3-aminopropanoate (130 mg, 0.9 mmol), tris(dibenzylideneacetone)dipalladium (55 mg, 0.06 mmol), 2-dicyclohexylphosphino-2',4',6'-triisopropylbiphenyl (57 mg, 0.12 mmol), and cesium carbonate (390 mg, 1.2 mmol) were dissolved in 1,4-dioxane (5 mL) under nitrogen atmosphere. The reaction mixture was heated to 110°C and stirred overnight. The reaction mixture was cooled to room temperature and concentrated under reduced pressure. The resulting residue was purified by silica gel column chromatography to obtain 117 mg of *tert*-butyl 3-((6-(benzyloxy)-5-(trifluoromethyl)pyridin-3-yl)amino)propanoate (**31-2**).

MS (ESI) M/Z: 397.2 $[M+H]^+$.

**Step B:** *tert-Butyl* 3-((6-(benzyloxy)-5-(trifluoromethyl)pyridin-3-yl)amino)propanoate (142 mg, 0.37 mmol) was dissolved in dichloromethane (2 mL) at room temperature. Trifluoroacetic acid (1 mL) was then added to the above solution. The reaction mixture was stirred at room temperature for 2 hours. The reaction mixture was concentrated under reduced pressure to obtain 72 mg of 3-((6-hydroxy-5-(trifluoromethyl)pyridin-3-yl)amino)propanoic acid (**31-3**).

MS (ESI) M/Z: 251.0 $[M+H]^+$.

**Step C:** 3-((6-Hydroxy-5-(trifluoromethyl)pyridin-3-yl)amino)propanoic acid (72 mg, 0.29 mmol) was dissolved in DMF (1 mL) at room temperature. 2-(Piperazin-1-yl)-5-(trifluoromethyl)pyrimidine (100 mg, 0.43 mmol), HATU (165 mg, 0.43 mmol), and *N,N*-diisopropylethylamine (112 mg, 0.87 mmol) were then added to the above solution. The reaction mixture was stirred at room temperature for 2 hours, and added with water (20 mL) to quench. The mixture was extracted with ethyl acetate (20 mL × 3), and the organic phases were combined. The combined organic phases were first washed with water (10 mL × 3) and saturated brine (10 mL), then dried over anhydrous sodium sulfate, filtered, and finally concentrated under reduced pressure. The resulting residue was purified by silica gel column chromatography to obtain 20.6 mg of 5-((3-oxo-3-(4-(5-(trifluoromethyl)pyrimidin-2-yl)piperazin-1-yl)propyl)amino)-3-(trifluoromethyl)pyridin-2(1*H*)-one (compound **31**).

MS (ESI) M/Z: 465.0 $[M+H]^+$.

$^1H$ NMR (400 MHz, DMSO-$d_6$) δ 11.68 (brs, 1H), 8.73 (d, *J* = 0.5 Hz, 2H), 7.60 (d, *J* = 2.3 Hz, 1H), 6.90 (s, 1H), 5.11 - 4.90 (m, 1H), 3.95 - 3.72 (m, 4H), 3.62 - 3.50 (m, 4H), 3.20 - 3.07 (m, 2H), 2.64 - 2.55 (m, 2H).

**Example 32**

***N*-(2-(6-Oxo-5-(trifluoromethyl)-1,6-dihydropyridin-3-yl)ethyl)-4-(5-(trifluoromethyl)pyrimidin-2-yl)piperazine-1-carboxamide**

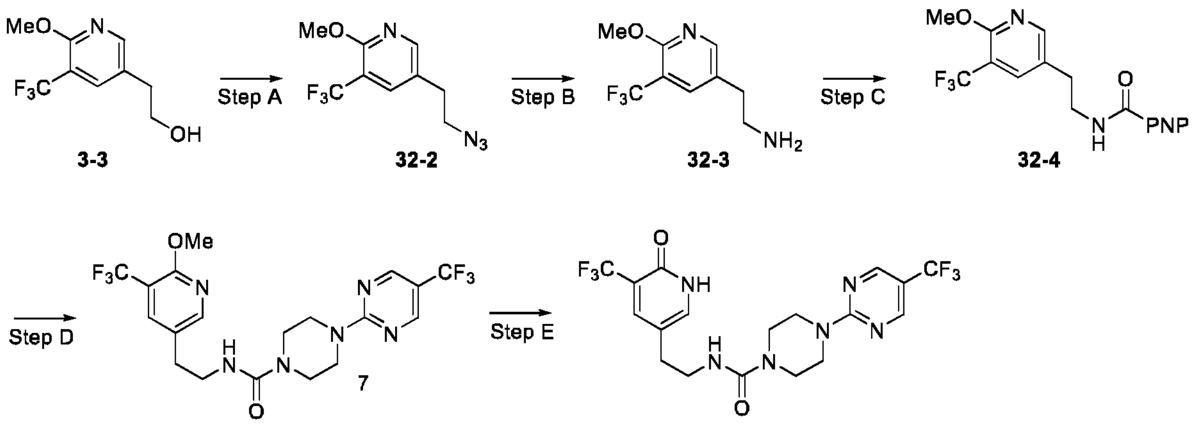

3-3 32-2 32-3 32-4
32-5 7 compound 32

**Step A:** 2-(6-Methoxy-5-(trifluoromethyl)pyridin-3-yl)ethan-1-ol (640 mg, 2.9 mmol), diphenylphosphoryl azide (1.6 g, 5.8 mmol), and 1,8-diazabicyclo[5.4.0]undec-7-ene (1.32 g, 8.7 mmol) were dissolved in toluene (8 mL) at room temperature. The reaction mixture was replaced with nitrogen, and then heated to 110°C and stirred for 16 hours. The reaction mixture was cooled to room temperature and concentrated under reduced pressure. The mixture was extracted with ethyl acetate (20 mL × 3), and the organic phases were combined. The combined organic phases were sequentially washed with water (50 mL) and saturated brine (50 mL), then dried over anhydrous sodium sulfate, filtered, and finally concentrated under reduced pressure. The resulting residue was purified by silica gel column chromatography to obtain 550 mg of 5-(2-azidoethyl)-2-methoxy-3-(trifluoromethyl)pyridine (**32-2**).

MS (ESI) M/Z: 247.1 $[M+H]^+$.

**Step B:** 5-(2-Azidoethyl)-2-methoxy-3-(trifluoromethyl)pyridine (100 mg, 0.41 mmol) was dissolved in tetrahydrofuran (3 mL) at room temperature. Triphenylphosphine (214.8 mg, 0.82 mmol) was then added thereto. The reaction mixture was stirred at room temperature for 16 hours. The reaction mixture was concentrated under reduced pressure. The mixture was extracted with ethyl acetate (10 mL × 3). The organic phases were combined, sequentially washed with water (30 mL) and saturated brine (30 mL), dried over anhydrous sodium sulfate, filtered, and concentrated. The resulting residue was purified by silica gel column chromatography to obtain 45 mg of 2-(6-methoxy-5-(trifluoromethyl)pyridin-3-yl)ethan-1-amine (**32-3**).

MS (ESI) M/Z: 221.1 $[M+H]^+$.

**Step C:** 2-(6-Methoxy-5-(trifluoromethyl)pyridin-3-yl)ethan-1-amine (110 mg, 0.5 mmol) was dissolved in *N*,*N*-dimethylformamide (3 mL) at room temperature. Bis(4-nitrophenyl) carbonate (304.2 mg, 1.0 mmol) and *N,N*-diisopropylethylamine (129.2 mg, 1.0 mmol) were then added thereto. The reaction mixture was stirred at room temperature for 16 hours. The mixture was added with water (30 mL) to quench, and extracted with ethyl acetate (10 mL × 3). The organic phases were combined, first washed with saturated brine (30 mL), dried over anhydrous sodium sulfate, filtered, and concentrated. The resulting residue was purified by silica gel column chromatography to obtain 120 mg of 4-nitrophenyl (2-(6-methoxy-5-(trifluoromethyl)pyridin-3-yl)ethyl)carbamate (**32-4**).

MS (ESI) M/Z: 386.0 $[M+H]^+$.

**Step D:** 4-Nitrophenyl (2-(6-methoxy-5-(trifluoromethyl)pyridin-3-yl)ethyl)carbamate (120 mg, 0.31 mmol) was dissolved in *N*,*N*-dimethylformamide (5 mL) at room temperature. 2-(Piperazin-1-yl)-5-(trifluoromethyl)pyrimidine (143.8 mg, 0.62 mmol) and *N,N*-diisopropylethylamine (120.2 mg, 0.93 mmol) were then added thereto. The reaction mixture was stirred at room temperature for 16 hours. The reaction mixture was added with water (30 mL) to quench. The mixture was extracted with ethyl acetate (10 mL × 3). The organic phases were combined, first washed with saturated brine (30 mL), dried over anhydrous sodium sulfate, filtered, and concentrated. The resulting residue was purified by silica gel column chromatography to obtain 140 mg of *N*-(2-(6-methoxy-5-(trifluoromethyl)pyridin-3-yl)ethyl)-4-(5-(trifluoromethyl)pyrimidin-2-yl)piperazine-1-carboxamide (**32-5**).

MS (ESI) M/Z: 479.0 $[M+H]^+$.

**Step E:** *N*-(2-(6-Methoxy-5-(trifluoromethyl)pyridin-3-yl)ethyl)-4-(5-(trifluoromethyl)pyrimidin-2-yl)piperazine-1-carboxamide (90 mg, 0.19 mmol) was dissolved in methanol (5 mL). Iodotrimethylsilane (152.0 mg, 0.76 mmol) was then added to the above solution. The reaction mixture was stirred at room temperature for 16 hours. The reaction mixture was added with water (10 mL) to quench. The mixture was extracted with ethyl acetate (10 mL × 3). The organic phases were combined, sequentially washed with saturated sodium sulfate aqueous solution (30 mL × 2) and saturated brine (30 mL), dried over anhydrous sodium sulfate, filtered, and concentrated. The resulting residue was purified by preparative high performance liquid chromatography to obtain 57 mg of *N*-(2-(6-oxo-5-(trifluoromethyl)-1,6-dihydropyridin-3-yl)ethyl)-4-(5-(trifluoromethyl)pyrimidin-2-yl)piperazine-1-carboxamide (compound **32).**

MS (ESI) M/Z: 465.0 $[M+H]^+$.

$^1H$ NMR (400 MHz, DMSO-$d_6$) δ 12.04 (s, 1H), 8.72 (s, 2H), 7.79 (d, *J* = 1.6 Hz, 1H), 7.46 (d, *J* = 1.6 Hz, 1H), 6.64 (t, *J* = 5.2 Hz, 1H), 3.84 - 3.71 (m, 4H), 3.39 - 3.36 (m, 4H), 3.21 (q, *J* = 6.4 Hz, 2H), 2.57 - 2.52 (m, 2H).

**Example 33**

**1-(6-Oxo-5-(trifluoromethyl)-1,6-dihydropyridin-3-yl)propan-2-yl 4-(5-(trifluoromethyl)pyrimidin-2-yl)piperazine-1-carboxylate**

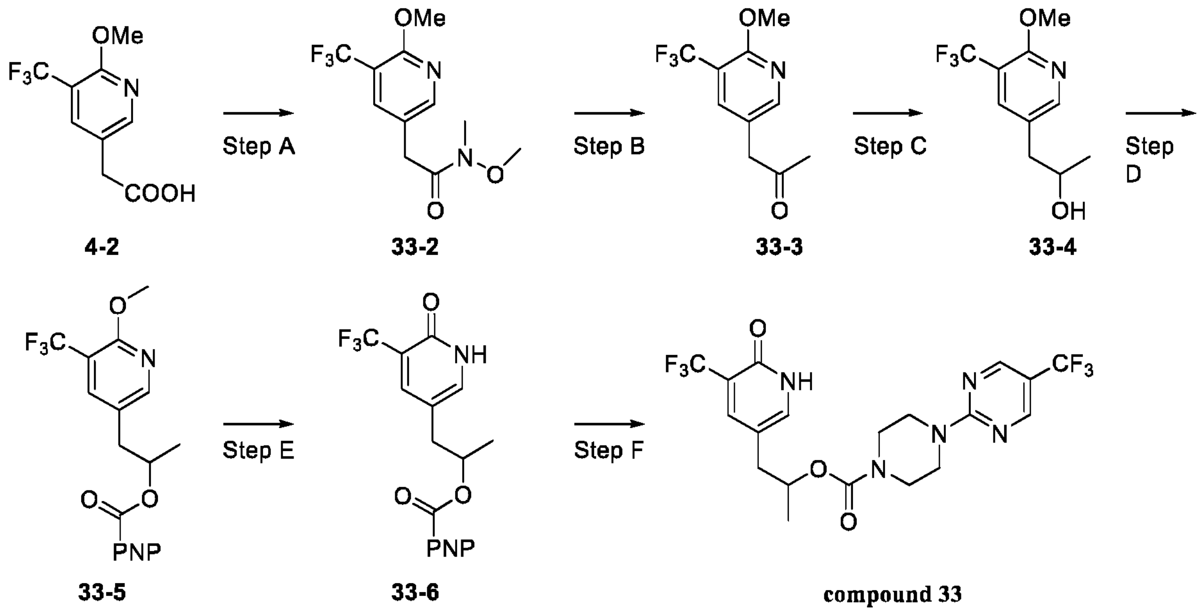


4-2 33-2 33-3 33-4

33-5 33-6 compound 33

**Step A:** 2-(6-Methoxy-5-(trifluoromethyl)pyridin-3-yl)acetic acid (1.1 g, 4.68 mmol), dimethylhydroxylamine hydrochloride (428 mg, 7.02 mmol), 1-(3-dimethylaminopropyl)-3-ethylcarbodiimide hydrochloride (983 mg, 5.14 mmol), and 1-hydroxybenzotriazole (695 mg, 5.14 mmol) were dissolved in *N*,*N*-dimethylformamide (15 mL). *N*-Methylmorpholine (1.41 g, 14.0 mmol) was then added to the above solution. The reaction mixture was stirred at room temperature for 5 hours. The mixture was added with water (50 mL), extracted with ethyl acetate (50 mL × 3), and the organic phases were combined. The combined organic phases were first washed with saturated brine (40 mL × 1), then dried over anhydrous sodium sulfate, filtered, and finally concentrated under reduced pressure. The residue was purified by silica gel column chromatography to obtain 1.3 g of N-methoxy-2-(6-methoxy-5-(trifluoromethyl)pyridin-3-yl)-*N*-methylacetamide (33-2).

MS (ESI) M/Z: 279.0 $[M+H]^+$.

**Step B:** *N*-Methoxy-2-(6-methoxy-5-(trifluoromethyl)pyridin-3-yl)-*N*-methylacetamide (1.1 g, 3.95 mmol) was dissolved in tetrahydrofuran (10 mL). Methyl Grignard reagent (11.8 mL, 11.8 mmol) was then added to the above solution under cooling in an ice bath. The reaction mixture was stirred in an ice bath for 2 hours. The mixture was added with saturated ammonium chloride (50 mL) to quench, extracted with ethyl acetate (50 mL × 3), and the organic phases were combined. The combined organic phases were first washed with saturated brine (40 mL × 1), then dried over anhydrous sodium sulfate, filtered, and finally concentrated under reduced pressure. The resulting residue was purified by silica gel column chromatography to obtain 900 mg of 1-(6-methoxy-5-(trifluoromethyl)pyridin-3-yl)propan-2-one **(33-3).**

MS (ESI) M/Z: 234.0 $[M+H]^+$.

**Step C:** 1-(6-Methoxy-5-(trifluoromethyl)pyridin-3-yl)propan-2-one (800 mg, 3.4 mmol) was dissolved in methanol (10 mL). Sodium borohydride (194 mg, 5.1 mmol) was then added to the above solution under cooling in an ice bath. The reaction mixture was stirred at room temperature for 3 hours. The reaction mixture was added with saturated sodium bicarbonate (50 mL) to quench. The mixture was extracted with ethyl acetate (50 mL × 3), and the organic phases were combined. The combined organic phases were first washed with saturated brine (40 mL × 1), then dried over anhydrous sodium sulfate, filtered, and finally concentrated under reduced pressure. The resulting residue was purified by silica gel column chromatography to obtain 800 mg of 1-(6-methoxy-5-(trifluoromethyl)pyridin-3-yl)propan-2-ol **(33-4).**

MS (ESI) M/Z: 236.0 $[M+H]^+$.

**Step D:** 1-(6-Methoxy-5-(trifluoromethyl)pyridin-3-yl)propan-2-ol (760 mg, 3.23 mmol) was dissolved in *N*,*N*-dimethylformamide (10 mL) at room temperature. Bis(4-nitrophenyl) carbonate (1.96 g, 6.46 mmol) and *N*,*N*-diisopropylethylamine (834 mg, 6.46 mmol) were then added thereto. The reaction mixture was stirred at room temperature for 6 hours. The reaction mixture was added with water (30 mL) to quench, and extracted with ethyl acetate (10 mL × 3). The organic phases were combined, first washed with saturated brine (30 mL), dried over anhydrous sodium sulfate, filtered, and concentrated. The resulting residue was purified by silica gel column chromatography to obtain 1 g of 1-(6-methoxy-5-(trifluoromethyl)pyridin-3-yl)propan-2-yl (4-nitrophenyl) carbonate **(33-5).**

MS (ESI) M/Z: 401.0 $[M+H]^+$.

**Step E:** 1-(6-Methoxy-5-(trifluoromethyl)pyridin-3-yl)propan-2-yl (4-nitrophenyl) carbonate (1 g, 2.5 mmol) was

dissolved in dichloromethane (10 mL). Iodotrimethylsilane (1 mL) was then added to the above solution. The reaction mixture was stirred at room temperature for 3 hours. The mixture was added with water (30 mL) to quench, and extracted with ethyl acetate (10 mL × 3). The organic phases were combined, first washed with saturated brine (30 mL), dried over anhydrous sodium sulfate, filtered, and concentrated. The resulting residue was purified by silica gel column chromatography to obtain 900 mg of 1-(6-hydroxy-5-(trifluoromethyl)pyridin-3-yl)propan-2-yl (4-nitrophenyl) carbonate **(33-6).**

MS (ESI) M/Z: 387.0 $[M+H]^+$.

**Step F:** 1-(6-Hydroxy-5-(trifluoromethyl)pyridin-3-yl)propan-2-yl (4-nitrophenyl) carbonate (600 mg, 1.55 mmol) was dissolved in N,N-dimethylformamide (15 mL) at room temperature. 2-(Piperazin-1-yl)-5-(trifluoromethyl)pyrimidine (360 mg, 1.5 mmol) and *N,N*-diisopropylethylamine (400 mg, 3.05 mmol) were then added thereto. The reaction mixture was stirred at room temperature for 16 hours. The mixture was added with water (30 mL) to quench, and extracted with ethyl acetate (10 mL × 3). The organic phases were combined, first washed with saturated brine (30 mL), dried over anhydrous sodium sulfate, filtered, and concentrated. The resulting residue was purified by preparative high performance liquid chromatography to obtain 600 mg of 1-(6-oxo-5-(trifluoromethyl)-1,6-dihydropyridin-3-yl)propan-2-yl 4-(5-(trifluoromethyl)pyrimidin-2-yl)piperazine-1-carboxylate (compound **33).**

MS (ESI) M/Z: 480.0 $[M+H]^+$.

$^1$H NMR (400 MHz, DMSO-$d_6$) δ 12.21 (s, 1H), 8.73 (s, 2H), 7.85 (s, 1H), 7.56 (d, *J* = 2.1 Hz, 1H), 4.96 - 4.73 (m, 1H), 3.89 (brs, 2H), 3.69 (brs, 2H), 3.44 (brs, 4H), 2.74 - 2.67 (m, 1H), 2.65 - 2.56 (m, 1H), 1.19 (d, *J* = 6.2 Hz, 3H).

**Example 34 and Example 35**

**(*R*)-1-(6-Oxo-5-(trifluoromethyl)-1,6-dihydropyridin-3-yl)propan-2-yl 4-(5-(trifluoromethyl)pyrimidin-2-yl)piperazine-1-carboxylate**

**(*S*)-1-(6-Oxo-5-(trifluoromethyl)-1,6-dihydropyridin-3-yl)propan-2-yl 4-(5-(trifluoromethyl)pyrimidin-2-yl)piperazine-1-carboxylate**

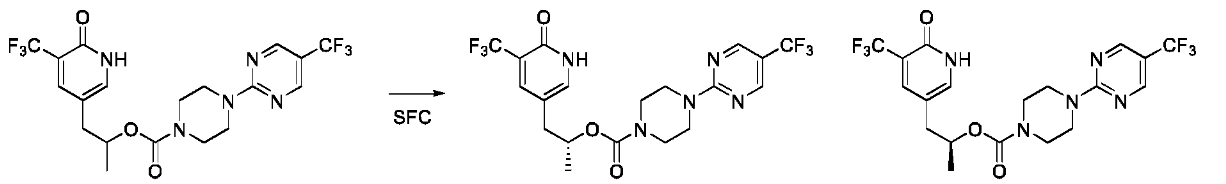


compound 33 　 compound 34 or compound 35 　 compound 35 or compound 34

Compound **33** was separated by a preparative chiral chromatography column (separation column: OD-H (250 mm * 30 mm, 5 μm); mobile phase: $CO_2$: (a solution of 0.1% ammonia water in ethanol) = 60:40, flow rate: 50 mL/min, pressure: 100 bar, detection wavelength: 220 nM) to obtain the following compounds.

Compound **34:**

Analytical conditions: OD-H (0.46 cm I.D. * 5cm L); $CO_2$: (a solution of 0.05% diethylamine in ethanol) = 70:30, flow rate: 2.5 mL/min, detection wavelength: 254 nM; retention time: 1.132 min; ee: 100%; absolute configuration unknown, enantiomer of compound **35.**

MS (ESI) M/Z: 480.0 $[M+H]^+$.

$^1$H NMR (400 MHz, DMSO-$d_6$) δ 12.21 (s, 1H), 8.73 (s, 2H), 7.85 (s, 1H), 7.56 (d, *J* = 2.0 Hz, 1H), 4.92 - 4.73 (m, 1H), 3.89 (brs, 2H), 3.69 (brs, 2H), 3.44 (s, 4H), 2.76 - 2.68 (m, 1H), 2.65 - 2.57 (m, 1H), 1.19 (d, *J* = 6.2 Hz, 3H).

Compound 35:

Analytical conditions: OD-H (0.46 cm I.D. * 5 cm L); $CO_2$: (a solution of 0.05% diethylamine in ethanol) = 70:30, flow rate: 2.5 mL/min, detection wavelength: 254 nM; retention time: 1.709 min; ee: 100%; absolute configuration unknown, enantiomer of compound **34.**

MS (ESI) M/Z: 480.0 $[M+H]^+$.

$^1$H NMR(400 MHz, DMSO-$d_6$) δ 12.22 (s, 1H), 8.71 (s, 2H), 7.85 (d, *J* = 2.0 Hz, 1H), 7.56 (d, *J* = 2.0 Hz, 1H), 4.90 - 4.74 (m, 1H), 3.89 (brs, 2H), 3.69 (brs, 2H), 3.44 (s, 4H), 2.76 - 2.68 (m, 1H), 2.65 - 2.56 (m, 1H), 1.19 (d, *J* = 6.2

Hz, 3H).

**Example 36**

**(*R*)-(1-(6-Oxo-5-(trifluoromethyl)-1,6-dihydropyridin-3-yl)pyrrolidin-2-yl)methyl 4-(5-(trifluoromethyl)pyrimidin-2-yl)piperazine-1-carboxylate**

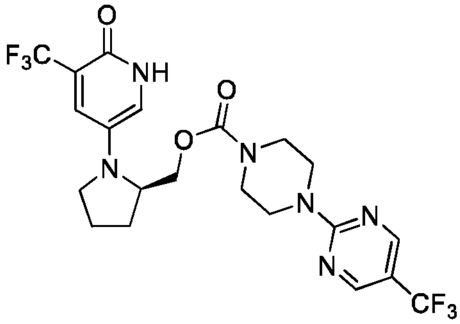

(*R*)-Pyrrolidin-2-ylmethanol was used as a raw material instead of (*S*)-pyrrolidin-2-ylmethanol with the preparation method referring to example **29.**
MS (ESI) M/Z: 521.0 $[M+H]^+$.
$^1H$ NMR (400 MHz, DMSO-$d_6$) δ 11.82 (brs, 1H), 8.73 (s, 2H), 7.71 (d, *J* = 2.5 Hz, 1H), 7.11 (s, 1H), 4.12 - 4.02 (m, 1H), 3.90 - 3.74 (m, 6H), 3.45 (brs, 4H), 3.40 - 3.33 (m, 1H), 2.95 - 2.86 (m, 1H), 2.01 - 1.88 (m, 3H), 1.87 - 1.77 (m, 1H).

**Example 37 and Example 38**

**5-(4-Oxo-4-(4-(5-(trifluoromethyl)pyrimidin-2-yl)piperazin-1-yl)butyl)-3-(trifluoromethyl)pyridin-2(1*H*)-one (Example 37)**

**5-(4-Oxo-4-(4-(5-(trifluoromethyl)pyrimidin-2-yl)piperazin-1-yl)butan-2-yl)-3-(trifluoromethyl)pyridin-2(1*H*)-one (Example 38)**

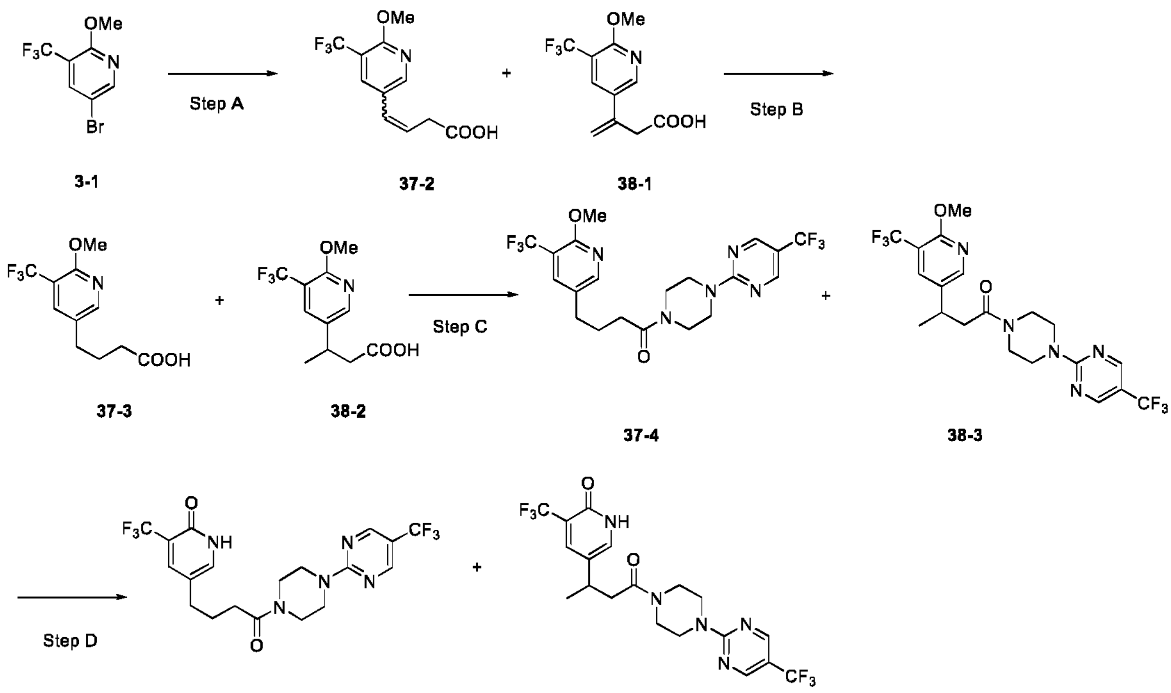


compound 37 compound 38

**Step A:** 5-Bromo-2-methoxy-3-(trifluoromethyl)pyridine (2.56 g, 10 mmol), methyl acrylate (1.722 g, 20 mmol), palladium acetate (112.3 mg, 0.5 mmol), triphenylphosphine (157.4 mg, 0.6 mmol), and triethylamine (2.024 g, 20 mmol) were dissolved in 1,4-dioxane (50 mL), and the reaction mixture was heated to 110°C in a sealed tube and reacted overnight. The reaction system was added with 1 A hydrochloric acid to adjust the pH to 5 to 6, and then extracted with ethyl acetate (50 mL × 3). The organic phase was concentrated under reduced pressure, and the resulting residue was purified by preparative high performance liquid chromatography to obtain 905 mg of a mixture of 4-(6-methoxy-5-(trifluoromethyl)pyridin-3-yl)but-3-enoic acid **(37-2)** and 3-(6-methoxy-5-(trifluoromethyl)pyridin-3-yl)but-3-enoic acid **(38-1).**

MS (ESI) M/Z: 262.2 $[M+H]^+$.

**Step B:** The mixture of 4-(6-methoxy-5-(trifluoromethyl)pyridin-3-yl)but-3-enoic acid and 3-(6-methoxy-5-(trifluoromethyl)pyridin-3-yl)but-3-enoic acid (905 mg, 3.46 mmol) was dissolved in methanol (15 mL), and then Pd/C (90 mg) was added thereto. The reaction mixture was replaced with $H_2$ three times, and reacted at room temperature for 2 hours. The reaction mixture was filtered, and the filter cake was washed with ethyl acetate (10 mL × 3). The organic phase was concentrated under reduced pressure, and the resulting residue was purified by silica gel column chromatography to obtain 220 mg of a mixture of 4-(6-methoxy-5-(trifluoromethyl)pyridin-3-yl)butanoic acid **(37-3)** and 3-(6-methoxy-5-(trifluoromethyl)pyridin-3-yl)butanoic acid **(38-2).**

MS (ESI) M/Z: 264.0 $[M+H]^+$.

**Step C:** The mixture of 4-(6-methoxy-5-(trifluoromethyl)pyridin-3-yl)butanoic acid and 3-(6-methoxy-5-(trifluoromethyl)pyridin-3-yl)butanoic acid (220 mg, 0.83 mmol) was dissolved in *N*,*N*-dimethylformamide (3 mL), then 2-(piperazin-1-yl)-5-(trifluoromethyl)pyrimidine (194.0 mg, 0.84 mmol), HATU (319.4 mg, 0.84 mmol), and triethylamine (429.7 mg, 4.2 mmol) were added to the above solution, and the reaction mixture was reacted at room temperature for 0.5 hours. The reaction system was added with ethyl acetate (100 mL), and then washed with water (10 mL × 5). The organic phase was concentrated, and the resulting residue was purified by silica gel column chromatography to obtain 200 mg of 4-(6-methoxy-5-(trifluoromethyl)pyridin-3-yl)-1-(4-(5-(trifluoromethyl)pyrimidin-2-yl)piperazin-1-yl)butanone **(37-4)** and 100 mg of 3-(6-methoxy-5-(trifluoromethyl)pyridin-3-yl)-1-(4-(5-(trifluoromethyl)pyrimidin-2-yl)piperazin-1-yl)butanone **(38-3),** respectively.

MS (ESI) M/Z: 478.2 $[M+H]^+$.

**Compound 37-4:**

$^1H$ NMR (400 MHz, $CDCl_3$) δ 8.51 (s, 2H), 8.14 (s, 1H), 7.71 (s, 1H), 4.02 (s, 3H), 3.92 (brs, 4H), 3.72 (brs, 2H), 3.52 (brs, 2H), 2.73 - 2.65 (m, 2H), 2.40 (t, *J* = 7.2 Hz, 2H), 2.05 - 1.94 (m, 2H).

**Compound 38-3:**

$^1$H NMR (400 MHz, $CDCl_3$) δ 8.51 (s, 2H), 8.24 (d, *J* = 2.1 Hz, 1H), 7.74 (d, *J* = 2.2 Hz, 1H), 4.01 (s, 3H), 3.97 - 3.87 (m, 2H), 3.87 - 3.76 (m, 2H), 3.70 - 3.64 (m, 2H), 3.57 - 3.41 (m, 3H), 2.63 (d, *J* = 7.0 Hz, 2H), 1.36 (d, *J* = 7.0 Hz, 3H).

**Step D:** 4-(6-Methoxy-5-(trifluoromethyl)pyridin-3-yl)-1-(4-(5-(trifluoromethyl)pyrimidin-2-yl)piperazin-1-yl)butanone (100 mg, 0.21 mmol) and 3-(6-methoxy-5-(trifluoromethyl)pyridin-3-yl)-1-(4-(5-(trifluoromethyl)pyrimidin-2-yl)piperazin-1-yl)butanone (100 mg, 0.21 mmol) were dissolved in 1,4-dioxane (5 mL), respectively, and then boron tribromide (3.45 mL, 1 mol/L) was slowly added dropwise thereto at room temperature, respectively. After the addition was completed, the reaction mixture was reacted at room temperature for 3 hours. The reaction system was added with saturated sodium bicarbonate aqueous solution (10 mL) respectively to quench, and then extracted with ethyl acetate (50 mL × 3), respectively. The organic phase was concentrated under reduced pressure, and the resulting residue was purified by preparative high performance liquid chromatography and lyophilized under reduced pressure to obtain 17.8 mg of 5-(4-oxo-4-(4-(5-(trifluoromethyl)pyrimidin-2-yl)piperazin-l-yl)butyl)-3-(trifluoromethyl)pyridin-2(1*H*)-one (compound **37)** and 36.1 mg of 5-(4-oxo-4-(4-(5-(trifluoromethyl)pyrimidin-2-yl)piperazin-1-yl)butan-2-yl)-3-(trifluoromethyl)pyridin-2(1*H*)-one (compound **38),** respectively.

Compound **37:**

MS (ESI) M/Z: 464.0 [M+H]$^+$.

$^1$H NMR (400 MHz, DMSO-$d_6$) δ 12.16 (s, 1H), 8.74 (s, 2H), 7.85 (d, *J* = 2.1 Hz, 1H), 7.51 (s, 1H), 3.89 - 3.84 (m, 2H), 3.84 - 3.77 (m, 2H), 3.60 - 3.51 (m, 4H), 2.42 (t, *J* = 7.5 Hz, 2H), 2.36 (t, *J* = 7.3 Hz, 2H), 1.80 - 1.69 (m, 2H).

Compound **38:**

MS (ESI) M/Z: 464.0 [M+H]$^+$.

$^1$H NMR (400 MHz, DMSO-$d_6$) δ 8.73 (s, 2H), 7.93 (d, *J* = 2.1 Hz, 1H), 7.55 (d, *J* = 2.3 Hz, 1H), 3.94 - 3.68 (m, 4H), 3.63 - 3.46 (m, 4H), 3.08 (q, *J* = 7.0 Hz, 1H), 2.64 (d, *J* = 7.2 Hz, 2H), 1.17 (d, *J* = 6.9 Hz, 3H).

**Example 39**

**5-((1*R*,2*S*)-2-(3-Oxo-3-(4-(5-(trifluoromethyl)pyrimidin-2-yl)piperazin-1-yl)propoxy)cyclopentyl)-3-(trifluoromethyl)pyridin-2(1*H*)-one**

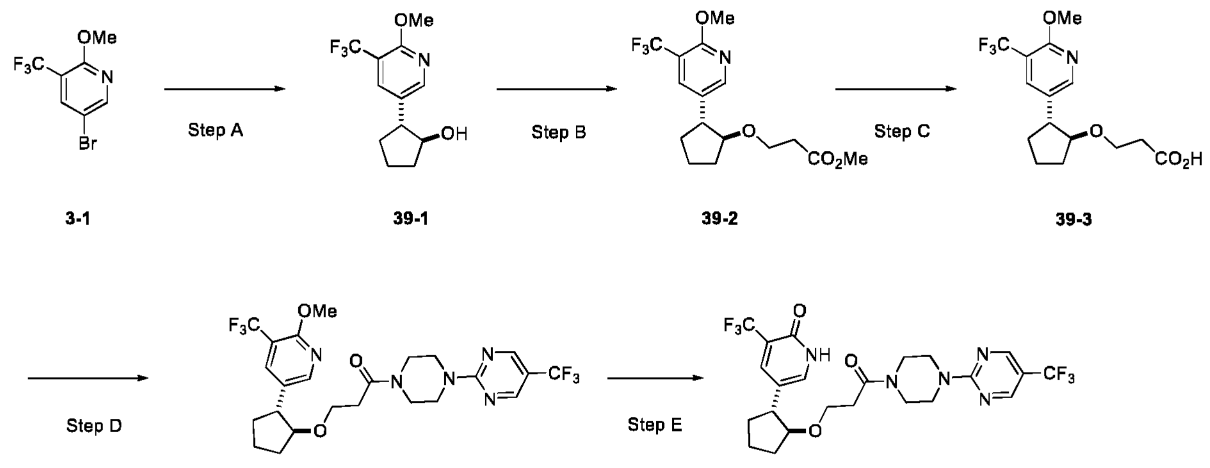


3-1 39-1 39-2 39-3 39-4 compound 39

**Step A:** 5-Bromo-2-methoxy-3-(trifluoromethyl)pyridine (3.85 g, 15 mmol) and tetrahydrofuran (50 mL) were added to a dry, 100 mL three-necked flask, then the system was cooled to -78°C, and *n*-butyllithium (9.4 mL, 15 mmol) was slowly added dropwise thereto. After the addition was completed, the reaction mixture was stirred for 1 hour. Boron trifluoride diethyl etherate (11.25 mL, 11.2 mmol) and cyclopentene oxide (736.5 mg, 7.5 mmol) were then added thereto, and the reaction mixture was reacted at -78°C for another 1 hour. The reaction system was added with water (10 mL) to quench, and then extracted with ethyl acetate (20 mL × 3). The organic phase was concentrated under reduced

pressure, and the resulting residue was purified by silica gel column chromatography to obtain 1.03 g of (1S,2R)-2-(6-methoxy-5-(trifluoromethyl)pyridin-3-yl)cyclopentan-1-ol **(39-1).**

MS (ESI) M/Z: 262.2 [M+H]+.

$^1H$ NMR (400 MHz, $CDCl_3$) δ 8.20 (s, 1H), 7.75 (d, *J* = 1.8 Hz, 1H), 4.10 (q, *J* = 7.3 Hz, 1H), 4.02 - 3.99 (m, 3H), 2.92 - 2.83 (m, 1H), 2.22 - 2.06 (m, 2H), 1.96 - 1.75 (m, 3H), 1.75 - 1.62 (m, 2H).

**Step B:** (1S,2R)-2-(6-Methoxy-5-(trifluoromethyl)pyridin-3-yl)cyclopentan-1-ol (261 mg, 1 mmol) and methyl acrylate (860 mg, 10 mmol) were dissolved in dry tetrahydrofuran (10 mL), then sodium hydride (60%, 80 mg, 2 mmol) was added thereto, and the reaction mixture was reacted at room temperature for 4 hours. The reaction system was added with water (10 mL) to quench, and then extracted with ethyl acetate (15 mL × 3). The organic phase was concentrated under reduced pressure, and the resulting residue was purified by silica gel column chromatography to obtain 138.2 mg of methyl 3-((1*S*,2*R*)-2-(6-methoxy-5-(trifluoromethyl)pyridin-3-yl)cyclopentyl)oxypropanoate **(39-2).**

MS (ESI) M/Z: 348.2 [M+H]+.

**Step C:** Methyl 3-((1*S*,2*R*)-2-(6-methoxy-5-(trifluoromethyl)pyridin-3-yl)cyclopentyl)oxypropanoate (130 mg, 0.375 mmol) was dissolved in methanol (2 mL) and water (1 mL), then lithium hydroxide (18 mg, 0.75 mmol) was added thereto, and the reaction mixture was reacted at room temperature for 2 hours. The reaction mixture was added with water (15 mL), and extracted with ethyl acetate (15 mL × 3). The aqueous phase was added with 1 *N* hydrochloric acid to adjust the pH to 5 to 6, and then extracted with ethyl acetate (15 mL × 3). The organic phase was concentrated under reduced pressure, and the resulting residue 3-(((1*S*,2*R*)-2-(6-methoxy-5-(trifluoromethyl)pyridin-3-yl)cyclopentyl)oxy)propanoic acid **(39-3)** was directly used in the next reaction step.

MS (ESI) M/Z: 334.4 [M+H]+.

**Step D:** 3-(((1*S*,2*R*)-2-(6-Methoxy-5-(trifluoromethyl)pyridin-3-yl)cyclopentyl)oxy)propanoic acid was dissolved in *N*,*N*-dimethylformamide (3 mL) at room temperature, then 2-(piperazin-1-yl)-5-(trifluoromethyl)pyrimidine (96 mg, 0.413 mmol), HATU (157 mg, 0.413 mmol), and triethylamine (191.8 mg, 1.875 mmol) were added to the above solution, and the reaction mixture was reacted at room temperature for 0.5 hours. The reaction system was added with ethyl acetate (100 mL), and then washed with water (10 mL × 5). The organic phase was concentrated, and the resulting residue was purified by silica gel column chromatography to obtain 159.8 mg of 3-(((1*S*,2*R*)-2-(6-methoxy-5-(trifluoromethyl)pyridin-3-yl)cyclopentyl)oxy)-1-(4-(5-(trifluoromethyl)pyrimidin-2-yl)piperazin-1-yl)propan-1-one **(39-4).**

MS (ESI) M/Z: 548.2 [M+H]+.

**Step E:** 3-(((1*S*,2*R*)-2-(6-Methoxy-5-(trifluoromethyl)pyridin-3-yl)cyclopentyl)oxy)-1-(4-(5-(trifluoromethyl)pyrimidin-2-yl)piperazin-1-yl)propan-1-one (159.8 mg, 0.292 mmol) was dissolved in dry dichloromethane, and then iodotrimethylsilane (116.8 mg, 0.59 mmol) was added dropwise thereto. The reaction mixture was reacted at room temperature for 2 hours, added with saturated sodium thiosulfate (1 mL) to quench, and then extracted with ethyl acetate (15 mL × 3). The organic phase was concentrated, and the resulting residue was purified by preparative high performance liquid chromatography to obtain 116.2 mg of 5-((1*R*,2*S*)-2-(3-oxo-3-(4-(5-(trifluoromethyl)pyrimidin-2-yl)piperazin-1- yl)propoxy)cyclopentyl)-3-(trifluoromethyl)pyridin-2(1H)-one (compound **39).**

MS (ESI) M/Z: 534.2 [M+H]+.

$^1H$ NMR (400 MHz, DMSO-$d_6$) δ 12.20 (s, 1H), 8.72 (s, 2H), 7.85 (d, *J* = 2.0 Hz, 1H), 7.51 (s, 1H), 3.86 - 3.74 (m, 5H), 3.68 - 3.58 (m, 1H), 3.59 - 3.50 (m, 5H), 2.82 - 2.70 (m, 1H), 2.58 - 2.52 (m, 2H), 2.03 - 1.89 (m, 2H), 1.72 - 1.62 (m, 2H), 1.61 - 1.42 (m, 2H).

**Example 40**

**5-(4-Fluoro-3-(4-(5-(trifluoromethyl)pyrimidin-2-yl)piperazine-1-carbonyl)benzyl)-3-(trifluoromethyl)pyridin-2(1H)-one**

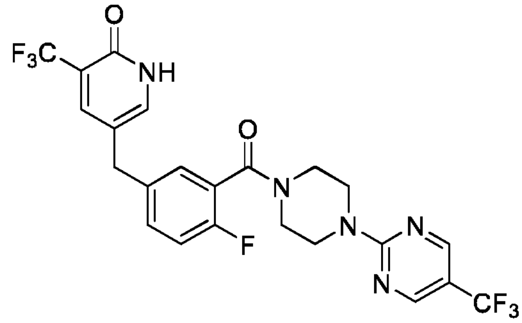

2-(Benzyloxy)-5-bromo-3-(trifluoromethyl)pyridine and methyl 5-(bromomethyl)-2-fluorobenzoate were used as raw materials with the preparation method referring to example 30.

MS (ESI) M/Z: 530.0 $[M+H]^+$.

$^1H$ NMR (400 MHz, DMSO-$d_6$) δ 12.25 (s, 1H), 8.74 (s, 2H), 7.85 (d, *J* = 2. 1Hz, 1H), 7.64 (s, 1H), 7.44 - 7.38 (m, 1H), 7.37 (dd, *J* = 6.5, 2.1Hz, 1H), 7.26 (t, *J* = 9.0Hz, 1H), 3.98 - 3.89 (m, 2H), 3.86 - 3.80 (m, 2H), 3.78 (s, 2H), 3.77 - 3.71 (m, 2H), 3.38 - 3.33 (m, 2H).

**Example 41**

**(1-(6-Oxo-5-(trifluoromethyl)-1,6-dihydropyridin-3-yl)cyclopropyl)methyl 4-(5-(trifluoromethyl)pyrimidin-2-yl)piperazine-1-carboxylate**

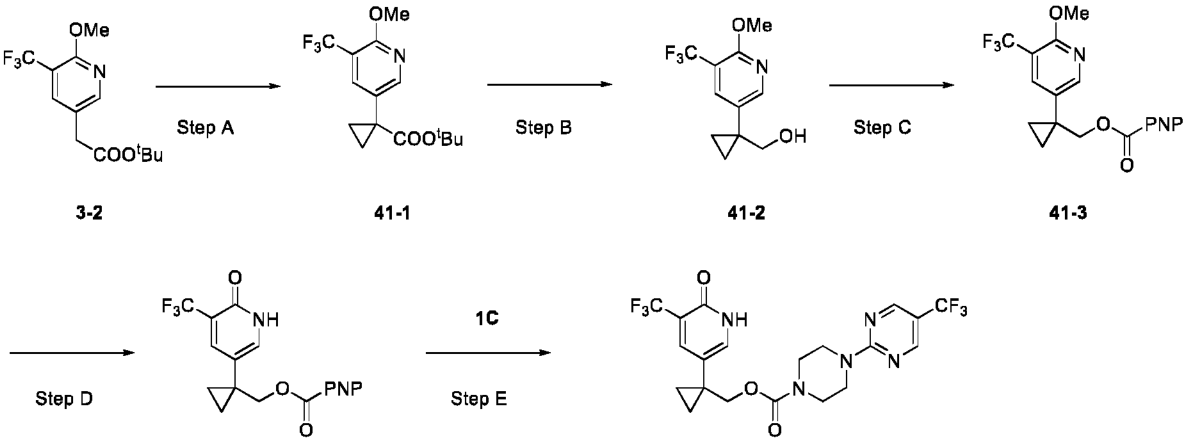


3-2 41-1 41-2 41-3 41-4

compound 41

**Step A:** *tert*-Butyl 2-(6-methoxy-5-(trifluoromethyl)pyridin-3-yl)acetate (500 mg, 1.71 mmol) was dissolved in *N*,*N*-dimethylformamide (7 mL). Sodium hydride (200.5 mg, 5.1 mmol) was added to the above solution in an ice bath. After the addition was completed, the reaction mixture was stirred for another 15 minutes, then added with 1,2-dibromoethane (317.9 mg, 1.7 mmol), returned to room temperature and stirred for 1.5 hours. Water and ethyl acetate (20 mL) were added thereto. The organic phases were separated and combined, washed with saturated brine (40 mL × 1), dried over anhydrous sodium sulfate, filtered, and finally concentrated under reduced pressure. The resulting residue was purified by silica gel column chromatography to obtain 220 mg of *tert*-butyl 1-(6-methoxy-5-(trifluoromethyl)pyridin-3-yl)cyclopropane-1-carboxylate **(41-1).**

MS (ESI) M/Z: 318.3 $[M+H]^+$.

**Step B:** *tert*-Butyl 1-(6-methoxy-5-(trifluoromethyl)pyridin-3-yl)cyclopropane-1-carboxylate (220 mg, 0.75 mmol) was dissolved in tetrahydrofuran solution (5 mL), and a solution of lithium aluminum hydride in tetrahydrofuran (2.5 mol/L, 0.45 mL, 1.1 mmol) was added thereto in an ice bath. After the addition was completed, the reaction mixture was stirred in an ice bath for another 1.5 hours. Sodium sulfate decahydrate was slowly added thereto in an ice bath until no bubbles were produced in the reaction mixture. The reaction mixture was filtered through diatomite, and the filtrate was extracted with ethyl acetate (50 mL × 3). The organic phases were combined, washed with saturated brine (40 mL × 1), dried over anhydrous sodium sulfate, filtered, and finally concentrated under reduced pressure. The resulting residue was purified by silica gel column chromatography to obtain 100 mg of (1-(6-methoxy-5-(trifluoromethyl)pyridin-3-yl)cyclopropyl)methanol **(41-2).**

MS (ESI) M/Z: 248.1 $[M+H]^+$.

**Step C:** (1-(6-Methoxy-5-(trifluoromethyl)pyridin-3-yl)cyclopropyl)methanol (100 mg, 0.4 mmol) was dissolved in *N*,*N*-dimethylformamide (3 mL) at room temperature, then bis(4-nitrophenyl) carbonate (184 mg, 0.6 mmol) and *N*,*N*-diisopropylethylamine (129 mg, 1.0 mmol) were added thereto, and the reaction mixture was stirred at room temperature for 6 hours. The reaction mixture was added with water (30 mL) to quench, and extracted with ethyl acetate (10 mL × 3). The organic phases were combined, washed with saturated brine (30 mL), dried over anhydrous sodium sulfate, filtered, and concentrated. The resulting residue was purified by silica gel column chromatography to obtain 100 mg of (1-(6-methoxy-5-(trifluoromethyl)pyridin-3-yl)cyclopropyl)methyl 2-(4-nitrophenoxy)acetate **(41-3).**

MS (ESI) M/Z: 413.2 $[M+H]^+$.

**Step D:** (1-(6-Methoxy-5-(trifluoromethyl)pyridin-3-yl)cyclopropyl)methyl 2-(4-nitrophenoxy)acetate (100 mg, 0.23 mmol) was dissolved in dichloromethane (4 mL), then iodotrimethylsilane (0.1 mL) was added thereto, and the reaction mixture was stirred at room temperature for 3 hours after the addition was completed. The reaction mixture was added with water (30 mL) to quench. The mixture was extracted with ethyl acetate (10 mL × 3). The organic phases were combined, then washed with saturated brine (30 mL), dried over anhydrous sodium sulfate, filtered, and concentrated. The resulting residue was purified by silica gel column chromatography to obtain 30 mg of (1-(6-methoxy-5-(trifluoromethyl)pyridin-3-yl)cyclopropyl)methyl 2-(4-nitrophenoxy)acetate **(41-4).**

MS (ESI) M/Z: 399.3 $[M+H]^+$.

**Step E:** (1-(6-Methoxy-5-(trifluoromethyl)pyridin-3-yl)cyclopropyl)methyl 2-(4-nitrophenoxy)acetate (30 mg, 0.07 mmol) was dissolved in *N,N*-dimethylformamide (3 mL), then 2-(piperazin-1-yl)-5-(trifluoromethyl)pyrimidine (25 mg, 0.11 mmol) and *N,N*-diisopropylethylamine (27 mg, 0.21 mmol) were added thereto, and the reaction mixture was stirred at room temperature for 16 hours. The reaction mixture was added with water (20 mL) to quench, and extracted with ethyl acetate (50 mL × 3). The organic phases were combined, first washed with saturated brine (30 mL × 1), dried over anhydrous sodium sulfate, filtered, and finally concentrated under reduced pressure. The resulting residue was purified by preparative high performance liquid chromatography to obtain 1.5 mg of (1-(6-oxo-5-(trifluoromethyl)-1,6-dihydropyridin-3-yl)cyclopropyl)methyl 4-(5-(trifluoromethyl)pyrimidin-2-yl)piperazine-1-carboxylate.

MS (ESI) M/Z: 492.0 $[M+H]^+$.

$^1$H NMR (400 MHz, DMSO-$d_6$) δ 12.16 (s, 1H), 8.74 (s, 2H), 7.87 (d, *J* = 2.6 Hz, 1H), 7.58 (s, 1H), 4.02 (s, 2H), 3.86 - 3.79 (m, 4H), 3.45 (s, 4H), 0.88 - 0.81 (m, 4H).

**Example 42**

**1-(6-Oxo-5-(trifluoromethyl)-1,6-dihydropyridin-3-yl)propan-2-yl 4-(pyrimidin-2-yl)piperazine-1-carboxylate**

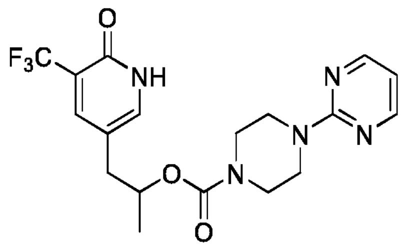

2-(Methoxy)-5-bromo-3-(trifluoromethyl)pyridine and 2-chloropyrimidine were used as raw materials with the preparation method referring to **intermediate 1C** and **example 33.**

MS (ESI) M/Z: 412.2 $[M+H]^+$.

$^1$H NMR (400 MHz, DMSO-*$d_6$*) δ 12.23 (s, 1H), 8.38 (d, *J* = 4.7 Hz, 2H), 7.85 (d, *J*= 2.5 Hz, 1H), 7.56 (d, *J* = 2.5 Hz, 1H), 6.66 (t, *J* = 4.8 Hz, 1H), 4.88 - 4.79 (m, 1H), 3.80 (s, 2H), 3.56 (s, 2H), 3.40 (s, 4H), 2.74 (d, *J* = 4.4 Hz, 0.4H), 2.70 (d, *J* = 4.3 Hz, 0.6H), 2.63 (d, *J* = 8.0 Hz, 0.6H), 2.59 (d, *J* = 7.9 Hz, 0.3H),1.19 (d, *J* = 6.2 Hz, 3H).

**Example 43**

**1-(6-Oxo-5-(trifluoromethyl)-1,6-dihydropyridin-3-yl)propan-2-yl 4-(5-methylpyrimidin-2-yl)piperazine-1-carboxylate**

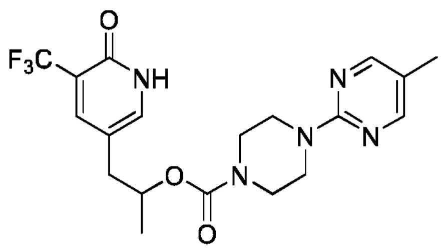

2-(Methoxy)-5-bromo-3-(trifluoromethyl)pyridine and 2-chloro-5-methylpyrimidine were used as raw materials with the preparation method referring to **intermediate 1C** and **example 33.**

MS (ESI) M/Z: 426.2 $[M+H]^+$.

$^1H$ NMR (400 MHz, DMSO-$d_6$) δ 12.23 (s, 1H), 8.25 (s, 2H), 7.85 (d, *J* = 2.5 Hz, 1H), 7.56 (s, 1H), 4.87 - 4.78 (m, 1H), 3.75 (s, 2H), 3.60 - 3.38 (m, 6H), 2.74 (d, *J* = 4.4 Hz, 0.3H), 2.70 (d, *J* = 4.3 Hz, 0.7H), 2.62 (d, *J* = 8.0 Hz, 0.7H), 2.59 (d, *J* = 8.0 Hz, 0.3H), 2.09 (s, 3H), 1.19 (d, *J* = 6.2 Hz, 3H).

**Example 44**

**1-(6-Oxo-5-(trifluoromethyl)-1,6-dihydropyridin-3-yl)propan-2-yl 4-(5-chloropyrimidin-2-yl)piperazine-1-carboxylate**

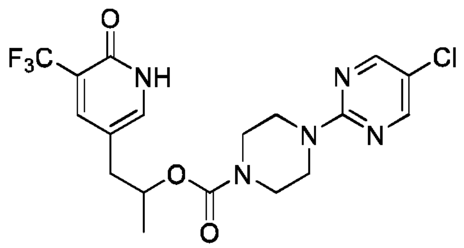

2-(Methoxy)-5-bromo-3-(trifluoromethyl)pyridine and 2,5-dichloropyrimidine were used as raw materials with the preparation method referring to **intermediate 1C** and **example** 33.

MS (ESI) M/Z: 446.0 $[M+H]^+$.

$^1H$ NMR (400 MHz, DMSO-$d_6$) δ 12.22 (s, 1H), 8.45 (s, 2H), 7.85 (d, *J* = 2.5 Hz, 1H), 7.56 (d, *J*= 2.5 Hz, 1H), 4.86 - 4.77 (m, 1H), 3.78 (s, 2H), 3.55 (s, 6H), 2.74 (d, *J*= 4.4 Hz, 0.3H), 2.70 (d, *J* = 4.3 Hz, 0.6H), 2.62 (d, *J* = 8.0 Hz, 0.6H), 2.59 (d, *J* = 8.0 Hz, 0.3H),1.19 (d, *J* = 6.2 Hz, 3H).

**Example 45**

**1-(6-Oxo-5-(trifluoromethyl)-1,6-dihydropyridin-3-yl)propan-2-yl 4-(5-cyanopyrimidin-2-yl)piperazine-1-carboxylate**

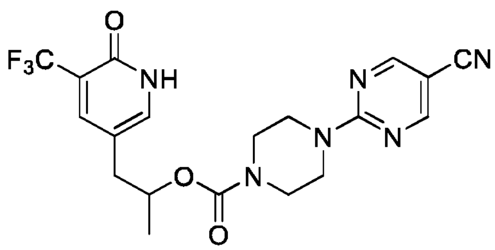

2-(Methoxy)-5-bromo-3-(trifluoromethyl)pyridine and 2-chloro-5-cyanopyrimidine were used as raw materials with the preparation method referring to **intermediate 1C** and **example 33.**

MS (ESI) M/Z: 437.2 $[M+H]^+$.

$^1H$ NMR (400 MHz, DMSO-$d_6$) δ 12.21 (s, 1H), 8.79 (s, 2H), 7.86 (d, *J* = 2.5 Hz, 1H), 7.56 (d, *J* = 2.6 Hz, 1H), 4.88 - 4.77 (m, 1H), 3.90 (s, 2H), 3.70 (s, 2H), 3.44 (s, 4H), 2.74 (d, *J* = 4.4 Hz, 0.3H), 2.70 (d, *J* = 4.3 Hz, 0.7H), 2.63 (d, *J* = 8.0 Hz, 0.7H), 2.59 (d, *J* = 7.9 Hz, 0.3H), 1.19 (d, *J* = 6.2 Hz, 3H).

**Example 46**

**1-(6-Oxo-5-(trifluoromethyl)-1,6-dihydropyridin-3-yl)propan-2-yl 4-(3-cyanopyridin-2-yl)piperazine-1-carboxylate**

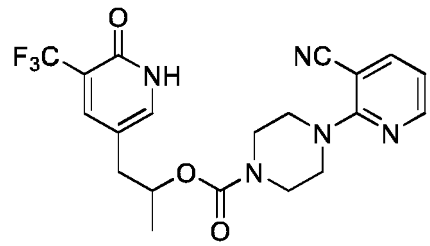

2-(Methoxy)-5-bromo-3-(trifluoromethyl)pyridine and 2-chloro-3-cyanopyridine were used as raw materials with the preparation method referring to **intermediate 1C** and **example 33.**
MS (ESI) M/Z: 436.2 $[M+H]^+$.
$^1H$ NMR (400 MHz, DMSO-$d_6$) δ 12.23 (s, 1H), 8.43 (dd, *J* = 4.8, 1.9 Hz, 1H), 8.11 (dd, *J* = 7.7, 1.9 Hz, 1H), 7.86 (d, *J* = 2.4 Hz, 1H), 7.57 (s, 1H), 6.97 (dd, *J* = 7.7, 4.8 Hz, 1H), 4.87 - 4.78 (m, 1H), 3.67 - 3.45 (m, 8H), 2.73 (d, *J* = 4.4 Hz, 0.3H), 2.70 (d, *J* = 4.3 Hz, 0.7H), 2.63 (d, *J* = 7.9 Hz, 0.6H), 2.59 (d, *J* = 8.1 Hz, 0.3H), 1.19 (d, *J* = 6.2 Hz, 3H).

**Example 47**

**5-((1R,2R)-2-(3-Oxo-3-(4-(5-(trifluoromethyl)pyrimidin-2-yl)piperazin-1-yl)propoxy)cyclopentyl)-3-(trifluoromethyl)pyridin-2(1*H*)-one**

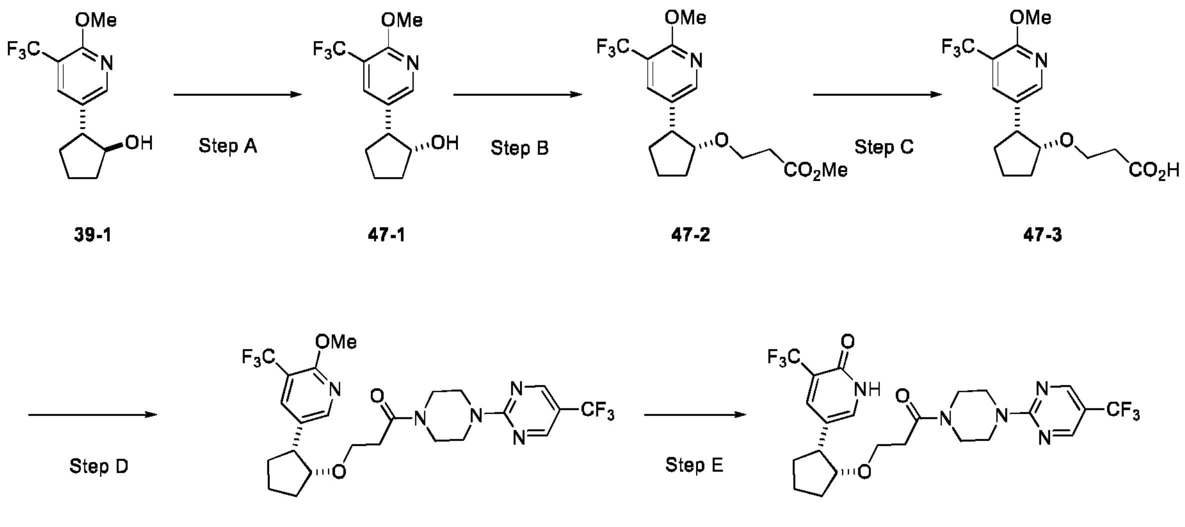


39-1 47-1 47-2 47-3

47-4 compound 47

**Step A:** (1*S*,2*R*)-2-(6-Methoxy-5-(trifluoromethyl)pyridin-3-yl)cyclopentan-1-ol (600 mg, 2.3 mmol) was dissolved in tetrahydrofuran (20 mL) at room temperature under nitrogen atmosphere, and then triphenylphosphine (1.27 g, 4.83 mmol), p-nitrobenzoic acid (807.1 mg, 4.83 mmol), and diethyl azodicarboxylate (801.3 mg, 4.6 mmol) were sequentially added to the above reaction mixture. After the addition was completed, the reaction mixture was heated to 60°C and reacted for 2 hours. The system was added with water (10 mL) to quench, and extracted with ethyl acetate (15 mL × 3). The organic phase was concentrated under reduced pressure, and the resulting residue was purified by silica gel column chromatography to obtain 975.4 mg of intermediate (1*R*,2*R*)-2-(6-methoxy-5-(trifluoromethyl)pyridin-3-yl)cyclopentyl (4-nitrophenyl) carbonate.
The above obtained (1*R*,2*R*)-2-(6-methoxy-5-(trifluoromethyl)pyridin-3-yl)cyclopentyl (4-nitrophenyl) carbonate

(975.4 mg, 2.29 mmol) was dissolved in methanol (20 mL), then sodium hydroxide (146.4 mg, 3.66 mmol) was added thereto, and the reaction mixture was reacted at room temperature for 2 hours. The system was added with 1 *N* hydrochloric acid to adjust the pH to 6 to 7, and extracted with ethyl acetate (25 mL × 3). The organic phase was concentrated under reduced pressure, and the resulting residue was purified by silica gel column chromatography to obtain 458.4 mg of (1R,2R)-2-(6-methoxy-5-(trifluoromethyl)pyridin-3-yl)cyclopentan-1-ol **(47-1).**

MS (ESI) M/Z: 262.2 $[M+H]^+$.

$^1H$ NMR (400 MHz, $CDCl_3$) δ 8.24 (d, *J* = 2.3 Hz, 1H), 7.84 (d, *J* = 2.3 Hz, 1H), 4.32 - 4.26 (m, 1H), 4.03 (s, 3H), 3.01 - 2.93 (m, 1H), 2.08 - 1.97 (m, 4H), 1.87 - 1.81 (m, 1H), 1.77 - 1.71 (m, 1H).

**Steps B to E:** (1*R*,2*R*)-2-(6-Methoxy-5-(trifluoromethyl)pyridin-3-yl)cyclopentan-1-ol was used instead of (1*S*,2*R)*-2-(6-methoxy-5-(trifluoromethyl)pyridin-3-yl)cyclopentan-1-ol with the preparation method referring to **example 39.**

MS (ESI) M/Z: 534.2 $[M+H]^+$.

$^1H$ NMR (400 MHz, DMSO-$d_6$) δ 12.12 (s, 1H), 8.73 (d, *J* = 0.9 Hz, 2H), 7.84 (d, *J* = 2.5 Hz, 1H), 7.45 (d, *J* = 2.5 Hz, 1H), 3.88 - 3.73 (m, 5H), 3.67 - 3.58 (m, 1H), 3.57 - 3.44 (m, 4H), 3.37 - 3.34 (m, 1H), 2.91 - 2.78 (m, 1H), 2.48 - 2.37 (m, 2H), 1.91 - 1.72 (m, 4H), 1.69 - 1.53 (m, 2H).

**Example 48**

**(*S*)-1-(6-Oxo-5-(trifluoromethyl)-1,6-dihydropyridin-3-yl)propan-2-yl 4-(5-(trifluoromethyl)pyrimidin-2-yl)piperazine-1-carboxylate**

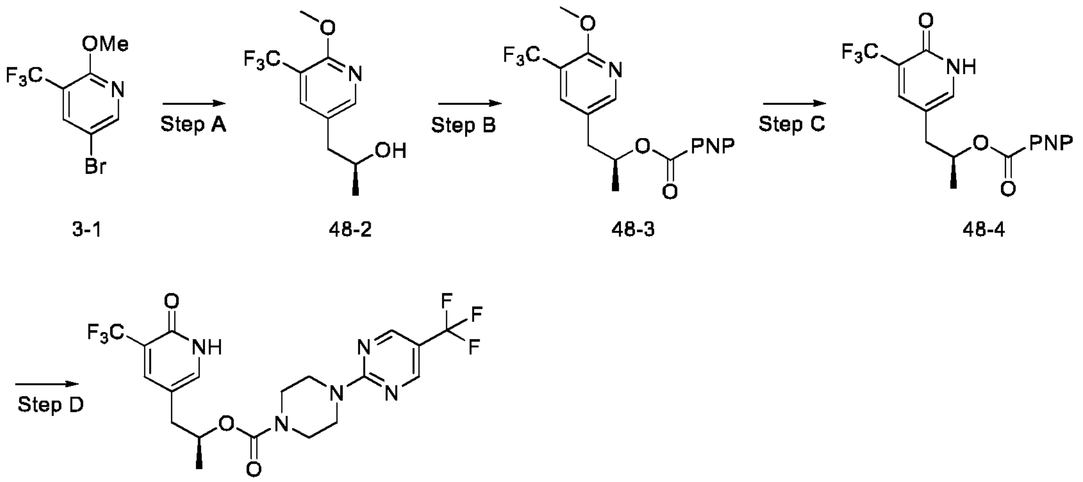

3-1 48-2 48-3 48-4

**example 48**

**Step A:** 5-Bromo-2-methoxy-3-(trifluoromethyl)pyridine (6 g, 23 mmol) was dissolved in anhydrous tetrahydrofuran (200 mL). The reaction mixture was cooled in a dry ice/acetone bath, and added dropwise with a solution of *n*-butyllithium in tetrahydrofuran (2.5 *M,* 9.7 mg, 23 mmol). After the dropwise addition was completed, the reaction mixture was stirred at this temperature for another 1 hour. The reaction mixture was then sequentially added with (S)-2-methyloxirane (700 mg, 11.7 mmol) and boron trifluoride diethyl etherate solution (5.3 g, 17.5 mmol), and stirred in a dry ice/acetone bath for another three hours. The reaction mixture was added with saturated sodium bicarbonate aqueous solution (80 mL) to quench. The reaction mixture was extracted with ethyl acetate (150 mL). The organic phases were separated and combined, washed with 2 *M* sodium hydroxide aqueous solution (50 mL × 1) and saturated brine (80 mL × 1), then dried over anhydrous sodium sulfate, filtered, and finally concentrated under reduced pressure. The resulting residue was purified by silica gel column chromatography to obtain 2.1 g of (*S*)-1-(6-methoxy-5-(trifluoromethyl)pyridin-3-yl)propan-2-ol **(48-2).**

MS (ESI) M/Z: 236.2 $[M+H]^+$.

**Step B:** (*S*)-1-(6-Methoxy-5-(trifluoromethyl)pyridin-3-yl)propan-2-ol was used as a raw material instead of 2-(6-methoxy-5-(trifluoromethyl)pyridin-3-yl)ethan-1-ol with the preparation method referring to **step C** of **example 3.**

MS (ESI) M/Z: 401.0 $[M+H]^+$.

**Step C:** (*S*)-1-(6-Methoxy-5-(trifluoromethyl)pyridin-3-yl)propan-2-yl (4-nitrophenyl) carbonate (2 g, 0.5 mmol) was dissolved in dichloromethane (4 mL). Iodotrimethylsilane (0.2 mL) was then added to the above solution. The

reaction mixture was stirred at room temperature for 3 hours. The mixture was added with water (30 mL) to quench, and extracted with ethyl acetate (10 mL × 3). The organic phases were combined, first washed with saturated brine (30 mL), dried over anhydrous sodium sulfate, filtered, and concentrated. The resulting residue was purified by silica gel column chromatography to obtain 900 mg of (*S*)-3-(6-oxo)phenyl-1-trifluoro-2-trifluoro-1-trifluoro-6-oxo-1-trifluoro-1-pyridyl carbonate **(48-4).**

MS (ESI) M/Z: 387.2 $[M+H]^+$.

**Step D:** (*S*)-3-(6-Oxo)phenyl-1-trifluoro-2-trifluoro-1-trifluoro-6-oxo-1-trifluoro-1-pyridyl carbonate (200 mg, 0.51 mmol) was dissolved in *N*,*N*-dimethylformamide (5 mL) at room temperature. 2-(Piperazin-1-yl)-5-(trifluoromethyl)pyrimidine (180 mg, 0.77 mmol) and *N*,*N*-diisopropylethylamine (0.28 mL, 1.54 mmol) were then added thereto. The reaction mixture was stirred at room temperature for 16 hours. The reaction mixture was added with water (30 mL) to quench. The mixture was extracted with ethyl acetate (10 mL × 3). The organic phases were combined, first washed with saturated brine (30 mL), dried over anhydrous sodium sulfate, filtered, and concentrated. The resulting residue was purified by preparative high performance liquid chromatography to obtain 111 mg of (*S*)-1-(6-oxo-5-(trifluoromethyl)-1,6-dihydropyridin-3-yl)propan-2-yl 4-(5-(trifluoromethyl)pyrimidin-2-yl)piperazine-1-carboxylate.

MS (ESI) M/Z: 480.0 $[M+H]^+$.

$^1$H NMR (400 MHz, DMSO-$d_6$) δ 12.21 (s, 1H), 8.73 (s, 2H), 7.85 (s, 1H), 7.56 (s, 1H), 4.96 - 4.73 (m, 1H), 3.89 (brs, 2H), 3.69 (brs, 2H), 3.44 (s, 4H), 2.74 - 2.67 (m, 1H), 2.65 - 2.56 (m, 1H), 1.19 (d, *J* = 6.2 Hz, 3H).

**Example 49**

**(*S*)-1-(6-Oxo-5-(trifluoromethyl)-1,6-dihydropyridin-3-yl)propan-2-yl 4-(5-(trifluoromethyl)thiazol-2-yl)piperazine-1-carboxylate**

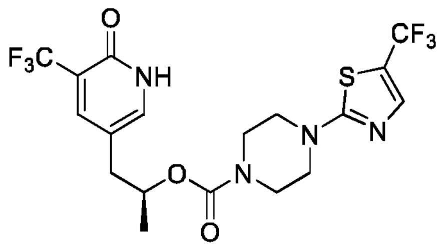

*tert*-Butyl piperazine-1-carboxylate and 2-chloro-5-(trifluoromethyl)thiazole were used as raw materials with the preparation method referring to **intermediate 1C** and **example** 48.

MS (ESI) M/Z: 485.0 $[M+H]^+$.

$^1$H NMR (400 MHz, DMSO-$d_6$) δ 12.21 (s, 1H), 7.85 (d, *J* = 2.4 Hz, 1H), 7.77 - 7.75 (m, 1H), 7.55 (s, 1H), 4.87 - 4.79 (m, 1H), 3.49 - 3.33 (m, 8H), 2.75 - 2.67 (m, 1H), 2.64 - 2.58 (m, 1H), 1.19 (d, *J* = 6.4 Hz, 3H).

**Example 50**

**(*S*)-1-(6-Oxo-5-(trifluoromethyl)-1,6-dihydropyridin-3-yl)propan-2-yl 4-(5-(difluoromethyl)pyrimidin-2-yl)piperazine-1-carboxylate**

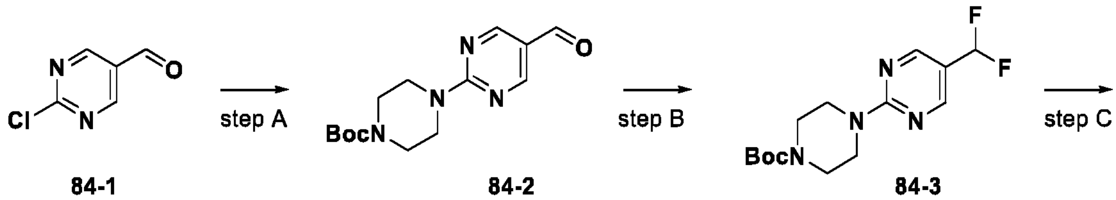


84-1 84-2 84-3

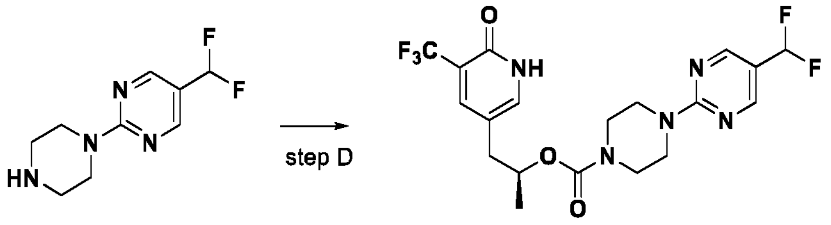


84-4 compound 84

**Step A:** 2-Chloropyrimidine-5-carbaldehyde (300.0 mg, 2.08 mmol) was dissolved in N-methylpyrrolidone (6 mL) at room temperature. *tert*-Butyl piperazine-1-carboxylate (387 mg, 2.08 mmol) and potassium carbonate (345 mg, 2.5 mmol) were sequentially added thereto. The reaction mixture was stirred at 80°C for 3 hours. The reaction mixture was cooled to room temperature, and added with water (25 mL) to quench. The mixture was extracted with ethyl acetate (15 mL × 3), and the organic phases were combined. The combined organic phases were first washed with saturated brine (15 mL), then dried over anhydrous sodium sulfate, filtered, and finally concentrated under reduced pressure. The resulting residue was purified by silica gel column chromatography to obtain 136 mg of *tert*-butyl 4-(5-formylpyrimidin-2-yl)piperazine-1-carboxylate **(84-2).**

MS (ESI) M/Z: 237.0 $[M+H\text{-}t\text{-}Bu]^+$.

**Step B:** Compound *tert*-butyl 4-(5-formylpyrimidin-2-yl)piperazine-1-carboxylate (136 mg, 0.68 mmol) was dissolved in dichloromethane (5 mL) at room temperature. The reaction mixture was then cooled to 0°C, and added with diethylaminosulfur trifluoride (0.85 mL, 3.41 mmol). The reaction mixture was stirred at room temperature for 16 hours. The reaction mixture was added with water (15 mL) to quench. The mixture was extracted with dichloromethane (5 mL × 3), and the organic phases were combined. The combined organic phases were first washed with saturated brine (10 mL), then dried over anhydrous sodium sulfate, filtered, and finally concentrated under reduced pressure. The resulting residue was purified by silica gel column chromatography to obtain 122 mg of *tert*-butyl 4-(5-(difluoromethyl)pyrimidin-2-yl)piperazine-1-carboxylate **(84-3).**

MS (ESI) M/Z: 264.4 $[M+H\text{-}t\text{-}Bu]^+$.

**Steps C to D:** *tert*-Butyl 4-(5-(difluoromethyl)pyrimidin-2-yl)piperazine-1-carboxylate and 2-(methoxy)-5-bromo-3-(trifluoromethyl)pyridine were used as raw materials with the preparation method referring to **intermediate 1C** and **example 48.**

MS (ESI) M/Z: 462.0 $[M+H]^+$.

$^1H$ NMR (400 MHz, DMSO-$d_6$) δ 12.21 (s, 1H), 8.58 (s, 2H), 7.85 (d, *J* = 1.8 Hz, 1H), 7.56 (s, 1H), 6.98 (t, *J* = 55.5 Hz, 1H), 4.89 - 4.78 (m, 1H), 3.86 (brs, 2H), 3.63 (brs, 2H), 3.43 (brs, 4H), 2.72 (dd, *J* = 13.9, 4.5 Hz, 1H), 2.61 (dd, *J* = 14.1, 7.9 Hz, 1H), 1.20 (d, *J* = 6.2 Hz, 3H).

## Example 51

### (*S*)-1-(6-Oxo-5-(trifluoromethyl)-1,6-dihydropyridin-3-yl)propan-2-yl ((*S*)-1-(5-(trifluoromethyl)pyrimidin-2-yl)pyrrolidin-3-yl)carbamate

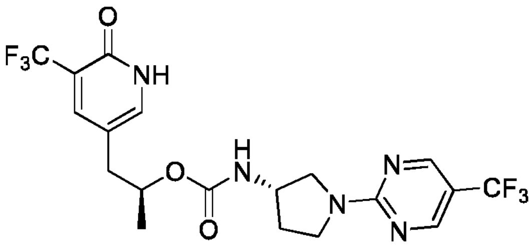

*tert*-Butyl (*S*)-pyrrolidin-3-ylcarbamate and 2-chloro-5-(trifluoromethyl)pyrimidine were used as raw materials with the preparation method referring to **intermediate 1C** and **example 48.**

MS (ESI) M/Z: 480.0 $[M+H]^+$.

$^1$H NMR (400 MHz, DMSO-$d_6$) δ 12.23 (s, 1H), 8.68 (d, *J* = 3.2 Hz, 2H), 7.81 (d, *J* = 0.4 Hz, 1H), 7.66 - 7.34 (m, 2H), 4.96 - 4.67 (m, 1H), 4.37 - 3.97 (m, 1H), 3.82 - 3.51 (m, 3H), 3.50 - 3.37 (m, 1H), 2.73 - 2.63 (m, 1H), 2.60 - 2.53 (m, 1H), 2.18 - 2.06 (m, 1H), 1.90 - 1.77 (m, 1H), 1.15 (d, *J* = 6.2 Hz, 3H).

**Example 52**

**(*S*)-1-(6-Oxo-5-(trifluoromethyl)-1,6-dihydropyridin-3-yl)propan-2-yl ((*R*)-1-(5-(trifluoromethyl)pyrimidin-2-yl)pyrrolidin-3-yl)carbamate**

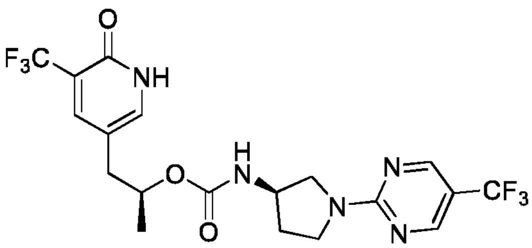

*tert*-Butyl (*R*)-pyrrolidin-3-ylcarbamate and 2-chloro-5-(trifluoromethyl)pyrimidine were used as raw materials with the preparation method referring to **intermediate 1C** and **example 48.**

MS (ESI) M/Z: 480.2 $[M+H]^+$.

$^1$H NMR (400 MHz, DMSO-$d_6$) δ 12.10 (s, 1H), 8.68 (d, *J* = 4.1 Hz, 2H), 7.78 (s, 1H), 7.56 - 7.47 (m, 2H), 4.84 - 4.74 (m, 1H), 4.15 - 4.08 (m, 1H), 3.77 - 3.66 (m, 1H), 3.66 - 3.60 (m, 1H), 3.59 - 3.54 (m, 1H), 3.41 - 3.37 (m, 1H), 2.68 - 2.57 (m, 2H), 2.19 - 2.07 (m, 1H), 1.95 - 1.80 (m, 1H), 1.14 (d, *J* = 5.9 Hz, 3H).

**Example 53**

**(*S*)-1-(6-Oxo-5-(trifluoromethyl)-1,6-dihydropyridin-3-yl)propan-2-yl (*R*)-3-((5-(trifluoromethyl)pyrimidin-2-yl)amino)pyrrolidine-1-carboxylate**

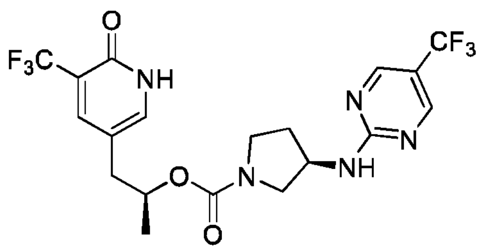

*tert*-Butyl (*R*)-3-aminopyrrolidine-1-carboxylate and 2-chloro-5-(trifluoromethyl)pyrimidine were used as raw materials with the preparation method referring to **intermediate 1C** and **example 48.**

MS (ESI) M/Z: 480.0 $[M+H]^+$.

$^1H$ NMR (400 MHz, DMSO-$d_6$) δ 12.17 (s, 1H), 8.65 (s, 2H), 8.36 (d, *J* = 6.4 Hz, 1H), 7.83 (s, 0.5H), 7.76 (s, 0.5H), 7.53 (d, *J* = 6.2 Hz, 1H), 4.80 - 4.73 (m, 1H), 4.45 - 4.35 (m, 1H), 3.68 - 3.61 (m, 0.6H), 3.54 - 3.37 (m, 2.4H), 3.24 - 3.18 (m, 0.5H), 3.11 - 3.06 (m, 0.5H), 2.73 - 2.64 (m, 1H), 2.62 - 2.54 (m, 1H), 2.16 - 2.07 (m, 1H), 1.96 - 1.87 (m, 1H), 1.17 - 1.13 (m, 3H).

**Example 54**

**(*S*)-1-(6-Oxo-5-(trifluoromethyl)-1,6-dihydropyridin-3-yl)propan-2-yl 3-((5-(trifluoromethyl)pyrimidin-2-yl)amino)azetidine-1-carboxylate**

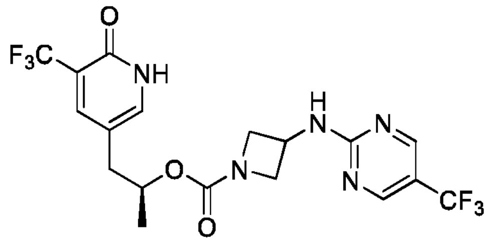

*tert*-Butyl (*R*)-3-aminoazetidine-1-carboxylate and 2-chloro-5-(trifluoromethyl)pyrimidine were used as raw materials with the preparation method referring to **intermediate 1C** and **example 48.**
MS (ESI) M/Z: 466.0 [M+H]+.
$^1H$ NMR (400 MHz, DMSO-$d_6$) δ 12.14 (s, 1H), 8.67 (s, 3H), 7.79 (s, 1H), 7.53 (d, *J* = 1.8 Hz, 1H), 4.73 (brs, 1H), 4.66 - 4.56 (m, 1H), 4.14 (brs, 2H), 3.89 - 3.84 (m, 1H), 3.81 - 3.76 (m, 1H), 2.70 - 2.63 (m, 1H), 2.60 - 2.52 (m, 1H), 1.14 (d, *J* = 6.2 Hz, 3H).

**Example 55**

**(*S*)-1-(6-Oxo-5-(trifluoromethyl)-1,6-dihydropyridin-3-yl)propan-2-yl (1-(5-(trifluoromethyl)pyrimidin-2-yl)piperidin-4-yl)carbamate**

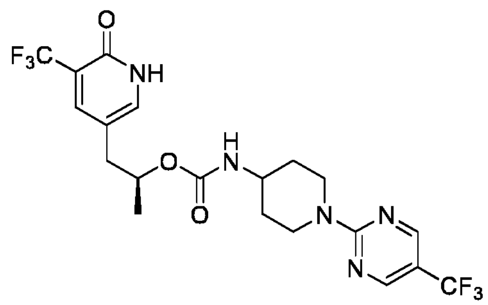

*tert*-Butyl piperidin-4-ylcarbamate and 2-chloro-5-(trifluoromethyl)pyrimidine were used as raw materials with the preparation method referring to **intermediate 1C** and **example 48.**
MS (ESI) M/Z: 494.0 [M+H]+.
$^1H$ NMR (400 MHz, DMSO-$d_6$) δ 12.22 (s, 1H), 8.68 (s, 2H), 7.80 (s, 1H), 7.49 (s, 1H), 7.17 (d, *J* = 7.9 Hz, 1H), 4.84 - 4.78 (m, 1H), 4.56 - 4.49 (m, 2H), 3.60 - 3.54 (m, 1H), 3.22 - 3.14 (m, 2H), 2.70 - 2.63 (m, 1H), 2.57 - 2.54 (m, 1H), 1.81 - 1.71 (m, 2H), 1.29 - 1.23 (m, 2H), 1.15 (d, *J* = 6.1 Hz, 3H).

### Example 56

**(1*S*,2*R*)-2-(6-Oxo-5-(trifluoromethyl)-1,6-dihydropyridin-3-yl)cyclopentyl 4-(5-(trifluoromethyl)pyrimidin-2-yl)piperazine-1-carboxylate**

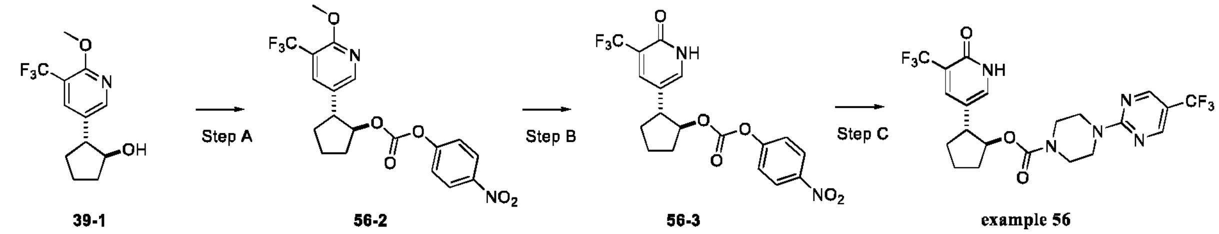

39-1 56-2 56-3 example 56

**Steps A to C:** (1*S*,2*R*)-2-(6-Methoxy-5-(trifluoromethyl)pyridin-3-yl)cyclopentan-1-ol was used as a raw material instead of 1-(6-methoxy-5-(trifluoromethyl)pyridin-3-yl)propan-2-ol with the preparation method referring to **example 33.**

MS (ESI) M/Z: 506.0 [M+H]$^+$.

$^1$H NMR (400 MHz, DMSO-$d_6$) $\delta$ 12.22 (s, 1H), 8.73 (s, 2H), 7.92 (d, *J* = 2.5 Hz, 1H), 7.56 (d, *J* = 2.5 Hz, 1H), 4.86 - 4.81 (m, 1H), 3.81 (brs, 4H), 3.46 (brs, 4H), 3.01 - 2.94 (m, 1H), 2.16 - 2.07 (m, 1H), 2.02 - 1.95 (m, 1H), 1.78 - 1.71 (m, 2H), 1.69 - 1.58 (m, 2H).

### Example 57

**5-((1*R*,2*S*)-2-(2-Oxo-2-(4-(5-(trifluoromethyl)pyrimidin-2-yl)piperazin-1-yl)ethoxy)cyclopentyl)-3-(trifluoromethyl)pyridin-2(1*H*)-one**

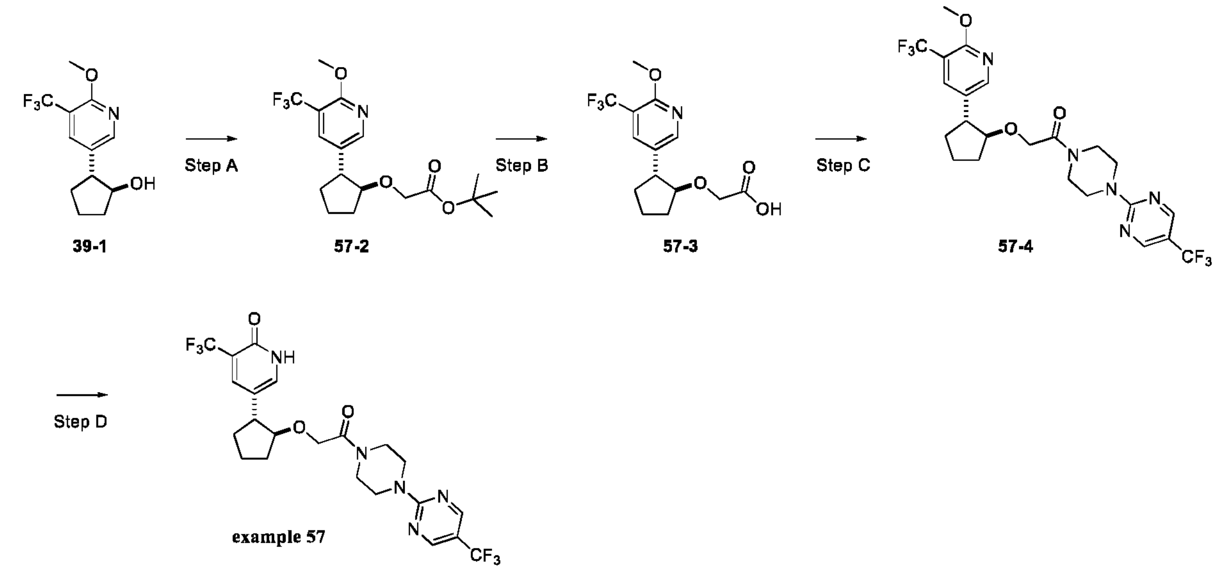

39-1 57-2 57-3 57-4 example 57

**Steps A to D:** (1*S*,2*R*)-2-(6-Methoxy-5-(trifluoromethyl)pyridin-3-yl)cyclopentan-1-ol was used as a raw material instead of (*S*)-(1-(6-methoxy-5-(trifluoromethyl)pyridin-3-yl)pyrrolidin-2-yl)methanol with the preparation method referring to **steps B to E** of **example 7.**

MS (ESI) M/Z: 520.0 [M+H]$^+$.

$^1$H NMR (400 MHz, DMSO-$d_6$) $\delta$ 12.20 (s, 1H), 8.72 (s, 2H), 7.91 (d, *J* = 2.5 Hz, 1H), 7.61 (d, *J* = 2.5 Hz, 1H), 4.22 - 4.19 (m, 1H), 4.12 - 4.08 (m, 1H), 3.90 (q, *J* = 6.9 Hz, 1H), 3.79 - 3.72 (m, 4H), 3.52 - 3.37 (m, 4H), 2.88 - 2.81 (m, 1H), 2.05 - 1.93 (m, 2H), 1.73 - 1.65 (m, 2H), 1.63 - 1.46 (m, 2H).

**Example 58**

**5-(2-((2-Oxo-2-(4-(5-(trifluoromethyl)pyrimidin-2-yl)piperazin-1-yl)ethoxy)methyl)cyclopentyl)-3-(trifluoromethyl)pyridin-2(1H)-one**

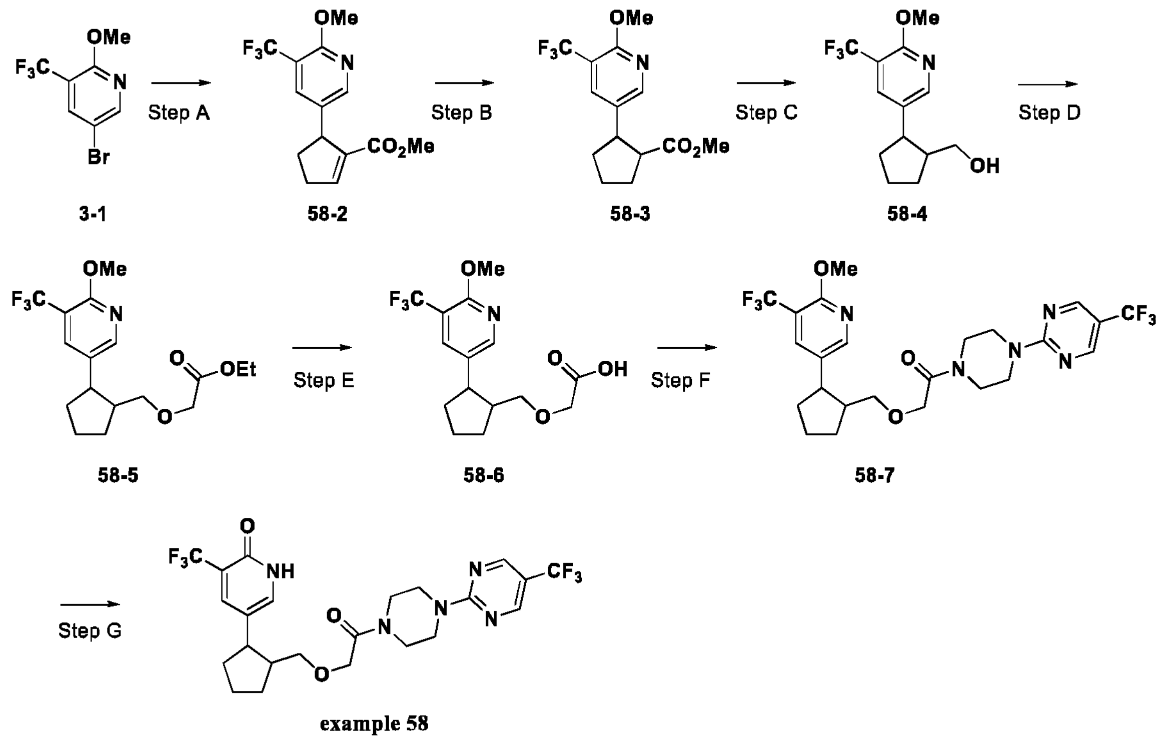


**Step A:** 5-Bromo-2-methoxy-3-(trifluoromethyl)pyridine (1.0 g, 3.9 mmol), methyl cyclopentene-1-carboxylate (1.0 g, 7.8 mmol), palladium acetate (88.0 mg, 0.4 mmol), cesium fluoride (1.2 g, 7.8 mmol), and N,N-dimethylformamide (30 mL) were added to a dry, 100 mL single-necked flask. The reaction mixture was replaced with $N_2$, and then heated and reacted in an oil bath at 130°C overnight. The reaction mixture was cooled to room temperature, filtered through diatomite, and the filter residue was washed with ethyl acetate (30 mL). The organic phase was washed with water (50 mL × 3) and saturated brine (30 mL). The organic phase was dried over anhydrous sodium sulfate and concentrated under reduced pressure, and the resulting residue was purified by silica gel column chromatography to obtain 362 mg of methyl 5-(6-methoxy-5-(trifluoromethyl)pyridin-3-yl)cyclopent-1-ene-1-carboxylate **(58-2).**

MS (ESI) M/Z: 302.2 $[M+H]^+$.

$^1H$ NMR (400 MHz, $CDCl_3$) δ 8.14 (d, *J* = 2.1 Hz, 1H), 7.61 (d, *J* = 2.2 Hz, 1H), 7.05 - 7.00 (m, 1H), 4.17 - 4.11 (m, 1H), 4.01 (s, 3H), 3.63 (s, 3H), 2.80 - 2.47 (m, 3H), 1.95 - 1.82 (m, 1H).

**Step B:** Methyl 5-(6-methoxy-5-(trifluoromethyl)pyridin-3-yl)cyclopent-1-ene-1-carboxylate (330.0 mg, 1.1 mmol) and palladium on carbon (33 mg) were dissolved in methanol (10 mL). The reaction mixture was then replaced with hydrogen three times, and reacted at room temperature for 4 hours. The reaction mixture was filtered through diatomite and concentrated under reduced pressure, and the crude product was directly used in the next reaction step without purification **(58-3).**

MS (ESI) M/Z: 304.2 $[M+H]^+$.

**Step C:** The above crude product was dissolved in anhydrous tetrahydrofuran (10 mL) under cooling in an ice bath, then a solution of lithium aluminum hydride in tetrahydrofuran (2.5 *M,* 0.13 mL, 0.3 mmol) was slowly added to the system, and the reaction mixture was stirred and reacted under cooling in an ice bath for another 0.5 hours. The reaction mixture was added with water (15 mL) to quench, and then added with 1 *N* dilute hydrochloric acid to adjust the system until it was clarified. The reaction mixture was extracted with ethyl acetate (15 mL × 3), and the organic phases were combined and washed once with saturated brine (15 mL). The organic phase was dried over anhydrous sodium sulfate and concentrated under reduced pressure, and the resulting residue was purified by silica gel column chromatography to obtain 237 mg of (2-(6-methoxy-5-(trifluoromethyl)pyridin-3-yl)cyclopentyl)methanol **(58-4).**

MS (ESI) M/Z: 276.2 $[M+H]^+$.

**Steps D to G:** (2-(6-Methoxy-5-(trifluoromethyl)pyridin-3-yl)cyclopentyl)methanol was used as a raw material instead of (*S*)-(1-(6-methoxy-5-(trifluoromethyl)pyridin-3-yl)pyrrolidin-2-yl)methanol with the preparation method referring to **steps B to E** of **example** 7 (compound **58).**

MS (ESI) M/Z: 534.4 $[M+H]^+$.

$^1H$ NMR (400 MHz, $CDCl_3$) $\delta$ 8.51 (s, 2H), 7.81 (s, 1H), 7.45 (s, 1H), 4.29 - 3.78 (m, 7H), 3.69 (brs, 2H), 3.49 (brs, 3H), 3.30 (brs, 1H), 3.13 (brs, 2H), 2.47 (brs, 1H), 2.04 (brs, 2H), 1.89 (brs, 2H).

**Example 59**

**(2-(6-Oxo-5-(trifluoromethyl)-1,6-dihydropyridin-3-yl)cyclopentyl)methyl 4-(5-(trifluoromethyl)pyrimidin-2-yl)piperazine-1-carboxylate**

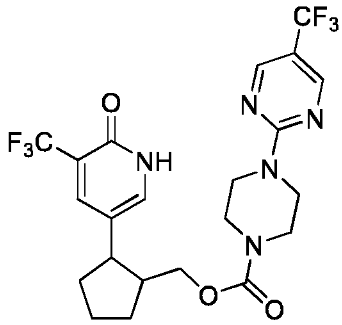

(2-(6-Methoxy-5-(trifluoromethyl)pyridin-3-yl)cyclopentyl)methanol was used as a raw material instead of (*S*)-(1-(6-methoxy-5-(trifluoromethyl)pyridin-3-yl)pyrrolidin-2-yl)methanol with the preparation method referring to **steps C to E** of **example 3.**

MS (ESI) M/Z: 520.2 $[M+H]^+$.

$^1H$ NMR (400 MHz, $CDCl_3$) $\delta$ 8.60 (s, 2H), 7.80 (s, 1H), 7.58 (s, 1H), 4.02 - 3.89 (m, 4H), 3.86 - 3.77 (m, 1H), 3.14 (q, *J* = 7.9 Hz, 1H), 2.61 - 2.51 (m, 1H), 2.32 - 2.20 (m, 2H), 2.15 - 2.03 (m, 2H), 2.02 - 1.87 (m, 3H), 1.86 - 1.69 (m, 3H), 1.56 - 1.43 (m, 1H).

**Example 60**

**5-(1-Oxo-1-(4-(5-(trifluoromethyl)pyrimidin-2-yl)piperazin-1-yl)propan-2-yl)-3-(trifluoromethyl)pyridin-2(1*H*)-one**

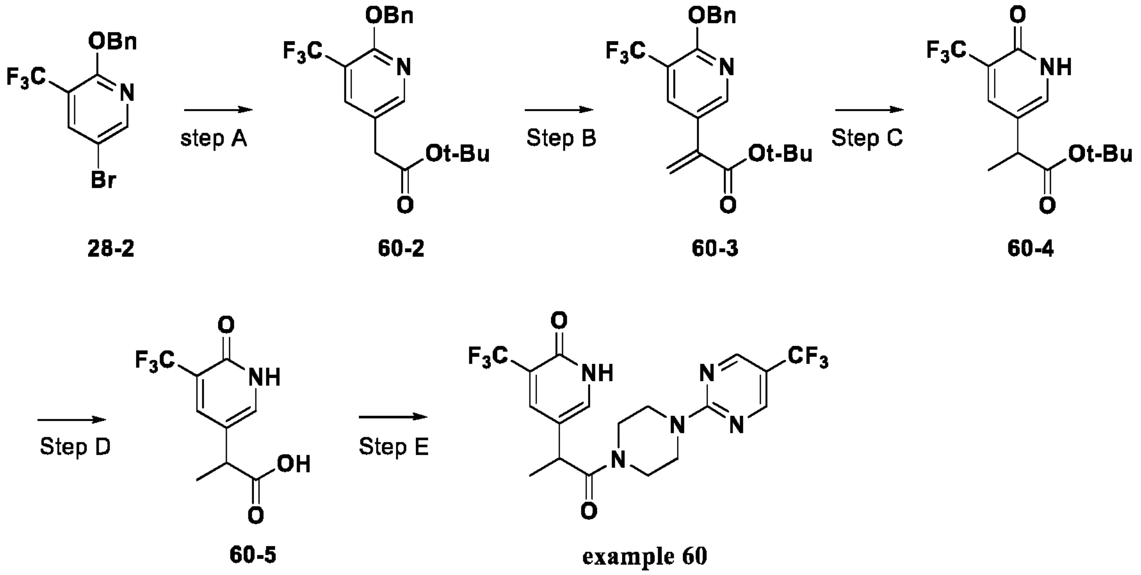


28-2 60-2 60-3 60-4 60-5 example 60

**Step A:** 2-(Benzyloxy)-5-bromo-3-(trifluoromethyl)pyridine was used as a raw material instead of 5-bromo-2-methoxy-3-(trifluoromethyl)pyridine with the preparation method referring to **step A** of **example 3.**

MS (ESI) M/Z: 368.0 $[M+H]^+$.

**Step B:** *tert*-Butyl 2-(6-(benzyloxy)-5-(trifluoromethyl)pyridin-3-yl)acetate (1.0 g, 2.72 mmol), paraformaldehyde (163 mg, 4.05 mmol), tetra-*n*-butylammonium hydrogen sulfate (46 mg, 0.13 mmol), and potassium carbonate (752 mg, 5.44 mmol) were dissolved in toluene solution (12 mL). The reaction mixture was heated to 80°C and stirred overnight. The reaction mixture was added with water (80 mL) to quench. The mixture was extracted with ethyl acetate (80 mL × 2), and the organic phases were combined. The combined organic phases were first washed with saturated brine (60 mL × 1), then dried over anhydrous sodium sulfate, filtered, and finally concentrated under reduced pressure. The resulting residue was purified by silica gel column chromatography to obtain 400 mg of *tert*-butyl 2-(6-(benzyloxy)-5-(trifluoromethyl)pyridin-3-yl)acrylate **(60-3).**

MS (ESI) M/Z: 380.0 $[M+H]^+$.

**Step C:** *tert*-Butyl 2-(6-(benzyloxy)-5-(trifluoromethyl)pyridin-3-yl)acrylate (320 mg, 0.84 mmol) was dissolved in methanol (8 mL). 10% palladium on carbon (wet basis) (447 mg) was then added to the above solution. After the reaction system was replaced with hydrogen three times, the reaction mixture was stirred at room temperature for 2 hours. The reaction mixture was filtered through diatomite, and the filter cake was washed with methanol (10 mL × 3) and concentrated. The resulting residue was purified by silica gel column chromatography to obtain 90 mg of *tert*-butyl 2-(6-oxo-5-(trifluoromethyl)-1,6-dihydropyridin-3-yl)propanoate **(60-4).**

MS (ESI) M/Z: 292.0 $[M+H]^+$.

**Steps D to E:** *tert*-Butyl 2-(6-oxo-5-(trifluoromethyl)-1,6-dihydropyridin-3-yl)propanoate was used instead of ethyl 2-(6-oxo-5-(trifluoromethyl)-1,6-dihydropyridazin-3-yl)acetate with the preparation method referring to **example 1.**

MS (ESI) M/Z: 450.0 $[M+H]^+$.

$^1H$ NMR (400 MHz, DMSO-$d_6$) δ 12.19 (s, 1H), 8.73 (s, 2H), 7.90 (d, *J* = 2.2 Hz, 1H), 7.62 (d, *J* = 2.2 Hz, 1H), 4.13 (q, *J* = 6.9 Hz, 1H), 3.91 - 3.68 (m, 5H), 3.68 - 3.48 (m, 3H), 1.28 (d, *J* = 6.9 Hz, 3H).

## Example 61

**5-(3-Methyl-5-(4-(5-(trifluoromethyl)pyrimidin-2-yl)piperazine-1-carbonyl)benzyl)-3-(trifluoromethyl)pyridin-2(1*H*)-one**

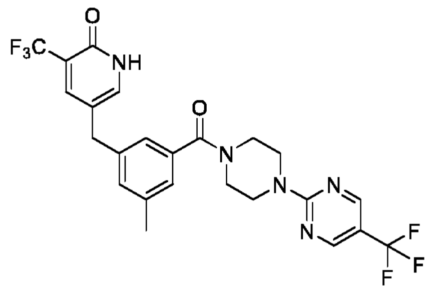

Methyl 3-(bromomethyl)-5-methylbenzoate was used instead of methyl 3-(bromomethyl)benzoate with the preparation method referring to **example** 30.
MS (ESI) M/Z: 526.2 $[M+H]^+$.
$^1H$ NMR (400 MHz, DMSO-$d_6$) δ 12.22 (s, 1H), 8.74 (s, 2H), 7.82 (d, *J* = 2.0 Hz, 1H), 7.62 (s, 1H), 7.19 (s, 1H), 7.13 (s, 1H), 7.10 (s, 1H), 4.01 -3.79 (m, 4H), 3.76 (s, 2H), 3.73 - 3.62 (m, 2H), 3.47 - 3.38 (m, 2H), 2.32 (s, 3H).

**Example 62**

**1-(6-Oxo-5-(trifluoromethyl)-1,6-dihydropyridin-3-yl)propan-2-yl 4-(5-(trifluoromethyl)pyridin-2-yl)piperazine-1-carboxylate**

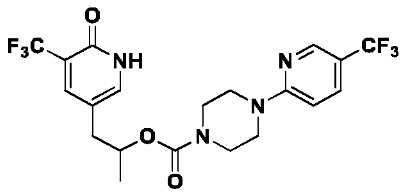

2-(Methoxy)-5-bromo-3-(trifluoromethyl)pyridine and 2-chloro-5-trifluoromethylpyridine were used as raw materials with the preparation method referring to **example 33.**
MS (ESI) M/Z: 479.0 $[M+H]^+$.
$^1H$ NMR (400 MHz, DMSO-$d_6$) δ 12.18 (s, 1H), 8.43 (s, 1H), 8.04 - 7.68 (m, 2H), 7.56 (s, 1H), 6.96 (d, *J* = 9.2 Hz, 1H), 5.20 - 4.60 (m, 1H), 3.82 - 3.61 (m, 2H), 3.57 - 3.41 (m, 6H), 2.77 - 2.68 (m, 1H), 2.65 - 2.56 (m, 1H), 1.19 (d, *J* = 6.4 Hz, 3H).

**Example 63**

**1-(6-Oxo-5-(trifluoromethyl)-1,6-dihydropyridin-3-yl)propan-2-yl 4-(5-bromopyrimidin-2-yl)piperazine-1-carboxylate**

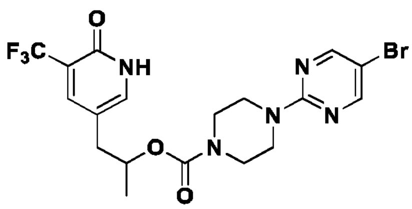

2-(Methoxy)-5-bromo-3-(trifluoromethyl)pyridine and 2-chloro-5-bromopyrimidine were used as raw materials with the preparation method referring to **example** 33.
MS (ESI) M/Z: 492.0 $[M+H]^+$.
$^1H$ NMR(400 MHz, DMSO-$d_6$) δ 12.21 (s, 1H), 8.49 (d, *J* = 0.4 Hz, 2H), 7.85 (s, 1H), 7.55 (s, 1H), 4.99 - 4.71

(m, 1H), 3.77 (brs, 2H), 3.56 (brs, 2H), 3.41 (brs, 4H), 2.76 - 2.67 (m, 1H), 2.64 - 2.56 (m, 1H), 1.19 (d, *J* = 6.2 Hz, 3H).

**Example 64**

**1-(6-Oxo-5-(trifluoromethyl)-1,6-dihydropyridin-3-yl)propan-2-yl 4-(5-cyanopyridin-2-yl)piperazine-1-carboxylate**

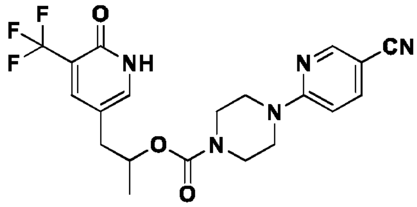

2-(Methoxy)-5-bromo-3-(trifluoromethyl)pyridine and 2-chloro-5-cyanopyridine were used as raw materials with the preparation method referring to **intermediate 1C** and **example 33.**
MS (ESI) M/Z: 436.3 $[M+H]^+$.
$^1H$ NMR (400 MHz, DMSO-$d_6$) δ 12.21 (s, 1H), 8.51 (d, *J* = 2.2 Hz, 1H), 7.89 (dd, *J* = 9.1, 2.3 Hz, 1H), 7.84 (d, *J* = 2.0 Hz, 1H), 7.55 (d, *J* = 1.5 Hz, 1H), 6.93 (d, *J* = 9.1 Hz, 1H), 4.92 - 4.74 (m, 1H), 3.70 (brs, 2H), 3.56 (brs, 2H), 3.43 (brs, 4H), 2.76 - 2.68 (m, 1H), 2.64 - 2.55 (m, 1H), 1.19 (d, *J* = 6.2 Hz, 3H).

**Example 65**

**(*S*)-1-(6-Oxo-5-(trifluoromethyl)-1,6-dihydropyridin-3-yl)propan-2-yl 4-(5-fluoropyrimidin-2-yl)piperazine-1-carboxylate**

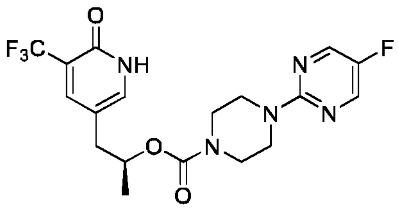

2-(Methoxy)-5-bromo-3-(trifluoromethyl)pyridine and 2-chloro-5-fluoropyrimidine were used as raw materials with the preparation method referring to **intermediate 1C** and **example 48.**
MS (ESI) M/Z: 430.0 $[M+H]^+$.
$^1H$ NMR (400 MHz, DMSO-$d_6$) δ 12.21 (s, 1H), 8.48 (s, 2H), 7.85 (d, *J* = 1.7 Hz, 1H), 7.56 (s, 1H), 4.86 - 4.79 (m, 1H), 3.74 (brs, 2H), 3.53 (brs, 2H), 3.41 (brs, 4H), 2.75 - 2.69 (m, 1H), 2.64 - 2.57 (m, 1H), 1.19 (d, *J* = 6.2 Hz, 3H).

**Example 66**

**(*S*)-1-(6-Oxo-5-(trifluoromethyl)-1,6-dihydropyridin-3-yl)propan-2-yl (*S*)-3-((5-(trifluoromethyl)pyrimidin-2-yl)amino)pyrrolidine-1-carboxylate**

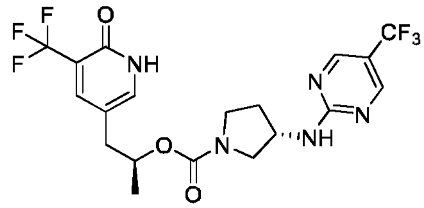

*tert*-Butyl (*S*)-3-aminopyrrolidine-1-carboxylate and 2-chloro-5-(trifluoromethyl)pyrimidine were used as raw materials with the preparation method referring to **intermediate 1C** and **example 48.**

MS (ESI) M/Z: 480.0 [M+H]$^+$.

$^1$H NMR (400 MHz, DMSO-$d_6$) δ 12.14 (s, 1H), 8.66 (d, *J*= 7.2 Hz, 2H), 8.36 (t, *J* = 6.5 Hz, 1H), 7.83 (d, *J* = 13.1 Hz, 1H), 7.54 (d, *J* = 5.5 Hz, 1H), 4.83 - 4.71 (m, 1H), 4.47 - 4.32 (m, 1H), 3.64 - 3.40 (m, 2H), 3.28 - 3.13 (m, 2H), 2.73 - 2.56 (m, 2H), 2.22 - 2.02 (m, 1H), 1.94 - 1.82 (m, 1H), 1.14 (t, *J* = 6.3 Hz, 3H).

**Example 67**

**1-(5-Methyl-6-oxo-1,6-dihydropyridin-3-yl)propan-2-yl 4-(5-(trifluoromethyl)pyrimidin-2-yl)piperazine-1-carboxylate**

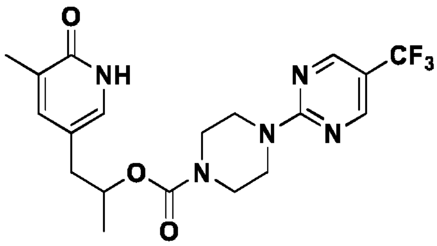

5-Bromo-2-methoxy-3-methylpyridine was used as a raw material instead of 2-(methoxy)-5-bromo-3-(trifluoromethyl)pyridine with the preparation method referring to **example 33.**

MS (ESI) M/Z: 426.2 [M+H]$^+$.

$^1$H NMR (400 MHz, DMSO-$d_6$) δ 11.33 (s, 1H), 8.73 (d, *J* = 0.6 Hz, 2H), 7.22 (d, 1H), 7.04 (d, *J* = 1.8 Hz, 1H), 4.82 - 4.76 (m, 1H), 3.89 (brs, 2H), 3.72 (brs, 2H), 3.45 (brs, 4H), 2.58 - 2.52 (m, 2H), 1.94 (s, 3H), 1.18 (d, *J* = 6.2 Hz, 3H).

**Example 68**

**5-(2-Fluoro-5-(4-(5-(trifluoromethyl)pyrimidin-2-yl)piperazine-1-carbonyl)benzyl)-3-(trifluoromethyl)pyridin-2(1*H*)-one**

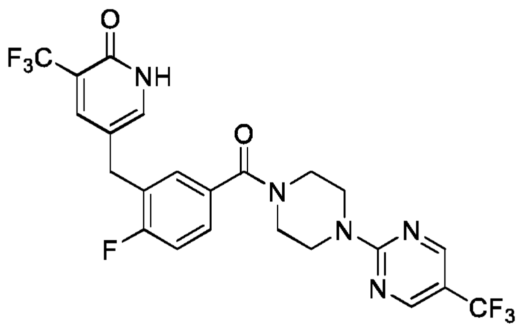

2-(Methoxy)-5-bromo-3-(trifluoromethyl)pyridine and methyl (bromomethyl)-4-fluorobenzoate were used as raw

materials with the preparation method referring to **example** 30.

MS (ESI) M/Z: 530.0 $[M+H]^+$.

$^1$H NMR (400 MHz, DMSO-$d_6$) δ 12.24 (s, 1H), 8.74 (s, 2H), 7.84 (s, 1H), 7.60 (s, 1H), 7.45 (d, *J* = 7.3 Hz, 1H), 7.44 - 7.35 (m, 1H), 7.32 - 7.24 (m, 1H), 3.89 (brs, 3H), 3.84 (s, 3H), 3.68 (brs, 2H), 3.45 (brs, 2H).

**Example 69 and Example 70**

**(1*S*,2*R*)-2-(6-Oxo-5-(trifluoromethyl)-1,6-dihydropyridin-3-yl)cyclopentyl 4-(5-(trifluoromethyl)pyrimidin-2-yl)piperazine-1-carboxylate**

**(1*R*,2*S*)-2-(6-Oxo-5-(trifluoromethyl)-1,6-dihydropyridin-3-yl)cyclopentyl 4-(5-(trifluoromethyl)pyrimidin-2-yl)piperazine-1-carboxylate**

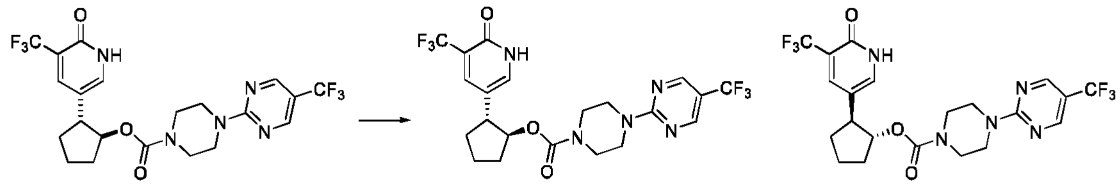

compound 56 compound 69 or compound 70 compound 70 or compound 69

Compound **56** was separated by a chiral chromatography column Daicel OD (25 * 250 mm, 10 μm); temperature: 30°C; mobile phase: COz/0.2% $NH_3$ in MeOH = 75/25; flow rate: 100 g/min; pressure: 100 bar; detection wavelength: 214 nm.

Compound 69:

Analytical conditions: Daicel OD-3 (4.6 * 100 mm 3 μm); temperature: 40°C; mobile phase: $CO_2$/0.2% $NH_3$ in MeOH = 80/20; flow rate: 3 mL/min; pressure: 2000 psi; retention time: 1.433 min; ee: 100%; absolute configuration unknown, enantiomer of compound **70.**

MS (ESI) M/Z: 506.0 $[M+H]^+$.

$^1$H NMR (400 MHz, DMSO-$d_6$) δ 12.20 (s, 1H), 8.73 (d, *J* = 0.9 Hz, 2H), 7.92 (d, *J* = 2.6 Hz, 1H), 7.56 (d, *J* = 2.5 Hz, 1H), 4.86 - 4.81 (m, 1H), 3.81 (brs, 4H), 3.46 (brs, 4H), 3.01 - 2.94 (m, 1H), 2.16 - 2.07 (m, 1H), 2.01 - 1.95 (m, 1H), 1.82 - 1.55 (m, 4H).

Compound **70:**

Analytical conditions: Daicel OD-3 (4.6 * 100 mm 3 μm); temperature: 40°C; mobile phase: $CO_2$/0.2% $NH_3$ in MeOH = 80/20; flow rate: 3 mL/min; pressure: 2000 psi; retention time: 3.255 min; ee: 100%; absolute configuration unknown, enantiomer of compound **69.**

MS (ESI) M/Z: 506.0 $[M+H]^+$.

$^1$H NMR (400 MHz, DMSO-$d_6$) δ 12.21 (s, 1H), 8.73 (s, 2H), 7.92 (d, *J* = 2.5 Hz, 1H), 7.56 (d, *J* = 2.6 Hz, 1H), 4.84 (q, *J* = 7.3 Hz, 1H), 3.81 (brs, 4H), 3.46 (brs, 4H), 2.97 (q, *J* = 7.8 Hz, 1H), 2.18 - 2.04 (m, 1H), 2.03 - 1.92 (m, 1H), 1.80 - 1.55 (m, 4H).

**Example 71**

**(*S*)-1-(6-Oxo-5-(trifluoromethyl)-1,6-dihydropyridin-3-yl)propan-2-yl (1-(5-(trifluoromethyl)pyrimidin-2-yl)azetidin-3-yl)carbamate**

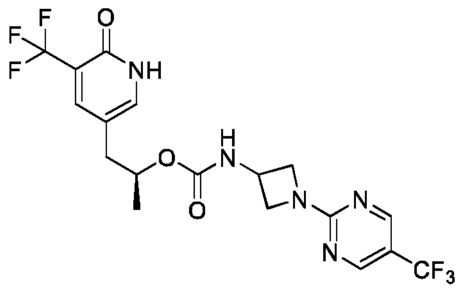

*tert*-Butyl azetin-3-ylcarbamate and 2-chloro-5-(trifluoromethyl)pyrimidine were used as raw materials with the preparation method referring to **intermediate 1C** and **example 48.**

MS (ESI) M/Z: 466.0 $[M+H]^+$.

$^1$H NMR (400 MHz, DMSO-$d_6$) δ 12.24 (s, 1H), 8.68 (s, 2H), 7.89 (d, *J* = 7.3 Hz, 1H), 7.80 (s, 1H), 7.51 (s, 1H), 4.89 - 4.72 (m, 1H), 4.50 - 4.38 (m, 1H), 4.37 - 4.25 (m, 2H), 4.00 - 3.81 (m, 2H), 2.72 - 2.62 (m, 1H), 2.61 - 2.54 (m, 1H), 1.15 (d, *J* = 6.3 Hz, 3H).

**Example 72**

**(*S*)-1-(6-Oxo-5-(trifluoromethyl)-1,6-dihydropyridin-3-yl)propan-2-yl 4-((5-(trifluoromethyl)pyrimidin-2-yl)amino)piperidine-1-carboxylate**

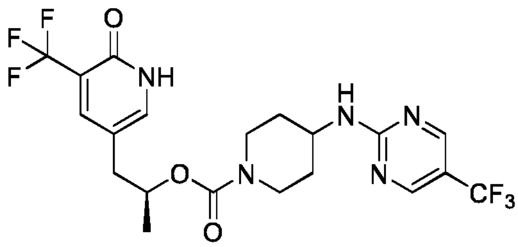

*tert*-Butyl piperidin-4-ylcarbamate and 2-chloro-5-(trifluoromethyl)pyrimidine were used as raw materials with the preparation method referring to **intermediate 1C** and **example 48.**

MS (ESI) M/Z: 494.0 $[M+H]^+$.

$^1$H NMR (400 MHz, DMSO-$d_6$) δ 12.21 (s, 1H), 8.63 (d, *J* = 11.4 Hz, 2H), 8.04 (s, 1H), 7.83 (s, 1H), 7.54 (s, 1H), 4.82 - 4.71 (m, 1H), 4.03 - 3.80 (m, 3H), 2.89 (brs, 2H), 2.74 - 2.65 (m, 1H), 2.64 - 2.56 (m, 1H), 1.88 - 1.75 (m, 2H), 1.45 - 1.27 (m, 2H), 1.17 (d, *J* = 6.2 Hz, 3H).

**Example 73**

**(*S*)-1-(6-Oxo-5-(trifluoromethyl)-1,6-dihydropyridin-3-yl)propan-2-yl (R)-3-(hydroxymethyl)-4-(5-(trifluoromethyl)pyrimidin-2-yl)piperazine-1-carboxylate**

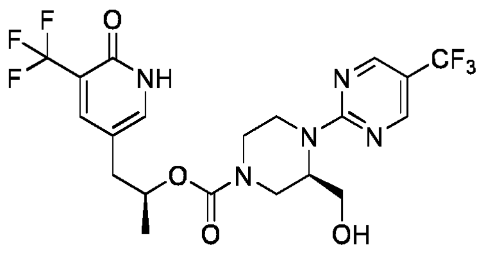

*tert*-Butyl (*R*)-3-(hydroxymethyl)piperazine-1-carboxylate and 2-chloro-5-(trifluoromethyl)pyrimidine were used

as raw materials with the preparation method referring to **intermediate 1C** and **example 48.**

MS (ESI) M/Z: 510.0 [M+H]+.

$^1$H NMR (400 MHz, DMSO-$d_6$) δ 12.16 (s, 1H), 8.72 (s, 2H), 7.86 (s, 1H), 7.56 (s, 1H), 4.92 - 4.71 (m, 3H), 4.50 (d, *J* = 13.4 Hz, 1H), 4.06 (brs, 1H), 3.90 (d, *J* = 12.5 Hz, 1H), 3.56 - 3.39 (m, 2H), 3.16 (brs, 2H), 3.00 - 2.84 (m, 1H), 2.75 - 2.57 (m, 2H), 1.17 (s, 3H).

**Example 74**

**(*S*)-1-(6-Oxo-5-(trifluoromethyl)-1,6-dihydropyridin-3-yl)propan-2-yl 7-(5-(trifluoromethyl)pyrimidin-2-yl)-2,7-diazaspiro[3.5]nonane-2-carboxylate**

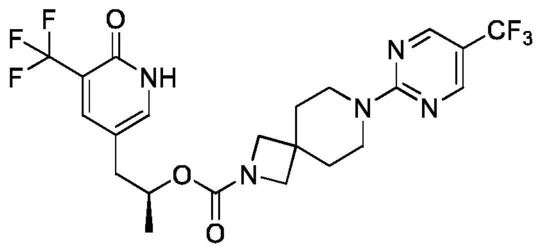

*tert*-Butyl 2,7-diazaspiro[3.5]nonane-2-carboxylate and 2-chloro-5-(trifluoromethyl)pyrimidine were used as raw materials with the preparation method referring to **intermediate 1C** and **example 48.**

MS (ESI) M/Z: 520.0 [M+H]+.

$^1$H NMR (400 MHz, DMSO-$d_6$) δ 8.68 (s, 2H), 7.81 (s, 1H), 7.54 (s, 1H), 4.82 - 4.69 (m, 1H), 3.94 - 3.65 (m, 5H), 3.60 (d, *J* = 7.4 Hz, 3H), 2.74 - 2.63 (m, 1H), 2.60 - 2.51 (m, 1H), 1.69 (brs, 4H), 1.15 (d, *J* = 6.2 Hz, 3H).

**Example 75**

**(*S*)-1-(6-Oxo-5-(trifluoromethyl)-1,6-dihydropyridin-3-yl)propan-2-yl 4-(5-(hydroxymethyl)pyrimidin-2-yl)piperazine-1-carboxylate**

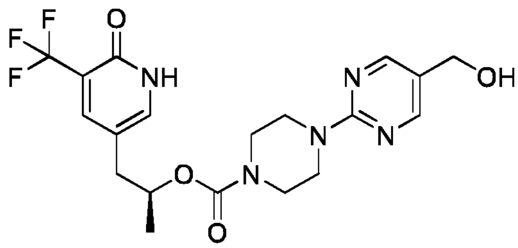

*tert*-Butyl piperazine-1-carboxylate and (2-chloropyrimidin-5-yl)methanol were used as raw materials with the preparation method referring to **intermediate 1C** and **example 48.**

MS (ESI) M/Z: 442.0 [M+H]+.

$^1$H NMR (400 MHz, DMSO-$d_6$) δ 12.22 (s, 1H), 8.33 (s, 2H), 7.85 (d, *J* = 2.0 Hz, 1H), 7.59 - 7.49 (m, 1H), 5.09 (t, *J* = 5.5 Hz, 1H), 4.91 - 4.77 (m, 1H), 4.33 (d, *J* = 5.5 Hz, 2H), 3.80 (brs, 2H), 3.56 (brs, 2H), 3.39 (brs, 4H), 2.76 - 2.68 (m, 1H), 2.65 - 2.55 (m, 1H), 1.19 (d, *J* = 6.2 Hz, 3H).

**Example 76**

**(*S*)-1-(6-Oxo-5-(trifluoromethyl)-1,6-dihydropyridin-3-yl)propan-2-yl 4-(5-cyanopyrazin-2-yl)piperazine-1-carboxylate**

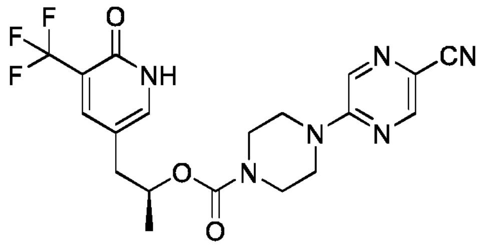

*tert*-Butyl piperazine-1-carboxylate and 5-chloropyrazine-2-carbonitrile were used as raw materials with the preparation method referring to **intermediate 1C** and **example 48.**
MS (ESI) M/Z: 437.0 $[M+H]^+$.
$^1H$ NMR (400 MHz, DMSO-$d_6$) δ 12.17 (s, 1H), 8.59 (d, *J* = 1.2 Hz, 1H), 8.43 (d, *J* = 1.2 Hz, 1H), 7.85 (d, *J* = 1.7 Hz, 1H), 7.56 (d, *J* = 0.5 Hz, 1H), 4.94 - 4.66 (m, 1H), 3.81 (brs, 4H), 3.47 (brs, 4H), 2.84 - 2.67 (m, 1H), 2.67 - 2.56 (m, 1H), 1.19 (d, *J* = 6.2 Hz, 3H).

**Example 77**

**(*S*)-1-(6-Oxo-5-(trifluoromethyl)-1,6-dihydropyridin-3-yl)propan-2-yl 5-(5-(trifluoromethyl)pyrimidin-2-yl)hexahydropyrrolo[3,4-*c*]pyrrole-2(1*H*)-carboxylate**

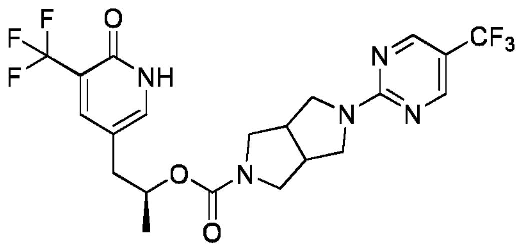

*tert*-Butyl hexahydropyrrolo[3,4-c]pyrrole-2(1*H*)-carboxylate and (2-chloropyrimidin-5-yl)methanol were used as raw materials with the preparation method referring to **intermediate 1C** and **example 48.**
MS (ESI) M/Z: 506.2 $[M+H]^+$.
$^1H$ NMR (400 MHz, DMSO-$d_6$) δ 12.21 (s, 1H), 8.69 (s, 2H), 7.80 (d, *J* = 17.3 Hz, 1H), 7.53 (s, 1H), 4.81 - 4.70 (m, 1H), 3.86 - 3.73 (m, 2H), 3.64 - 3.47 (m, 2H), 3.46 - 3.34 (m, 2H), 3.25 - 3.08 (m, 2H), 3.00 (brs, 2H), 2.72 - 2.64 (m, 1H), 2.62 - 2.52 (m, 1H), 1.14 (d, *J*= 6.2 Hz, 3H).

**Example 78**

**(*S*)-1-(6-Oxo-5-(trifluoromethyl)-1,6-dihydropyridin-3-yl)propan-2-yl (*R*)-2-(hydroxymethyl)-4-(5-(trifluoromethyl)pyrimidin-2-yl)piperazine-1-carboxylate**

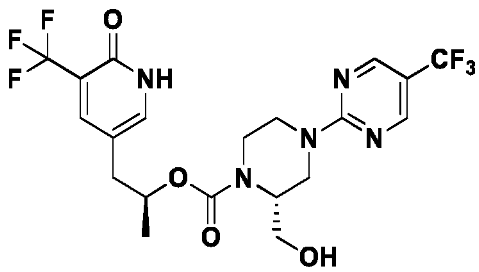

*tert*-Butyl (*R*)-2-(hydroxymethyl)piperazine-1-carboxylate and 2-chloro-5-(trifluoromethyl)pyrimidine were used as raw materials with the preparation method referring to **intermediate 1C** and **example 48.**

MS (ESI) M/Z: 510.0 $[M+H]^+$.

$^1H$ NMR (400 MHz, DMSO-$d_6$) δ 12.13 (s, 1H), 8.73 (s, 2H), 7.83 (s, 1H), 7.54 (s, 1H), 4.91 - 4.81 (m, 1H), 4.78 (t, *J* = 5.2 Hz, 1H), 4.75 - 4.65 (m, 1H), 4.42 (brs, 1H), 4.05 (brs, 1H), 3.91 - 3.79 (m, 1H), 3.24 - 2.96 (m, 5H), 2.76 - 2.65 (m, 1H), 2.64 - 2.56 (m, 1H), 1.18 (d, *J* = 6.2 Hz, 3H).

**Example 79**

**(*S*)-1-(6-Oxo-5-(trifluoromethyl)-1,6-dihydropyridin-3-yl)propan-2-yl (S)-2-(hydroxymethyl)-4-(5-(trifluoromethyl)pyrimidin-2-yl)piperazine-1-carboxylate**

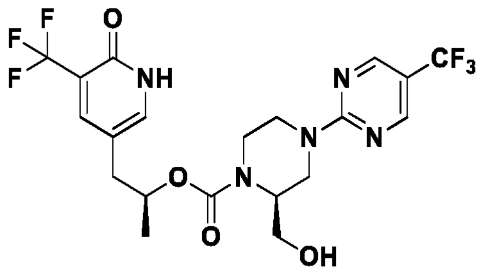

*tert*-Butyl (*S*)-2-(hydroxymethyl)piperazine-1-carboxylate and 2-chloro-5-(trifluoromethyl)pyrimidine were used as raw materials with the preparation method referring to **intermediate 1C** and **example 48.**

MS (ESI) M/Z: 510.0 $[M+H]^+$.

$^1H$ NMR (400 MHz, DMSO-$d_6$) δ 12.15 (s, 1H), 8.72 (s, 2H), 7.87 (s, 1H), 7.57 (s, 1H), 4.91 - 4.80 (m, 1H), 4.80 - 4.72 (m, 1H), 4.49 - 4.35 (m, 1H), 4.14 - 4.02 (m, 1H), 4.08 (brs, 1H), 3.86 (d, *J* = 12.2 Hz, 1H), 3.40 - 3.33 (m, 2H), 3.22 (d, *J* = 11.6 Hz, 1H), 3.09 (brs, 2H), 2.76 - 2.66 (m, 1H), 2.65 - 2.56 (m, 1H), 1.18 (d, *J* = 6.2 Hz, 3H).

**Example 80**

**(*S*)-1-(6-Oxo-5-(trifluoromethyl)-1,6-dihydropyridin-3-yl)propan-2-yl (*S*)-3-(hydroxymethyl)-4-(5-(trifluoromethyl)pyrimidin-2-yl)piperazine-1-carboxylate**

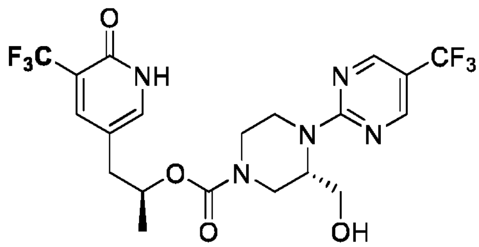

*tert*-Butyl (S)-3-(hydroxymethyl)piperazine-1-carboxylate and 2-chloro-5-(trifluoromethyl)pyrimidine were used as raw materials with the preparation method referring to **intermediate 1C** and **example 48.**

MS (ESI) M/Z: 510.0 $[M+H]^+$.

$^1H$ NMR (400 MHz, DMSO-$d_6$) δ 12.23 (s, 1H), 8.73 (s, 2H), 7.85 (s, 1H), 7.56 (s, 1H), 4.89 - 4.68 (m, 3H), 4.50 (d, *J* = 12.8 Hz, 1H), 4.10 (d, *J* = 13.6 Hz, 1H), 3.89 (d, *J* = 11.2 Hz, 1H), 3.59 - 3.36 (m, 2H), 3.23 - 2.83 (m, 3H), 2.77 - 2.67 (m, 1H), 2.66 - 2.56 (m, 1H), 1.18 (d, J = 6 Hz, 3H).

**Example 81**

**(*S*)-1-(4-Methyl-6-oxo-5-(trifluoromethyl)-1,6-dihydropyridin-3-yl)propan-2-yl 4-(5-(trifluoromethyl)pyrimidin-2-yl)piperazine-1-carboxylate**

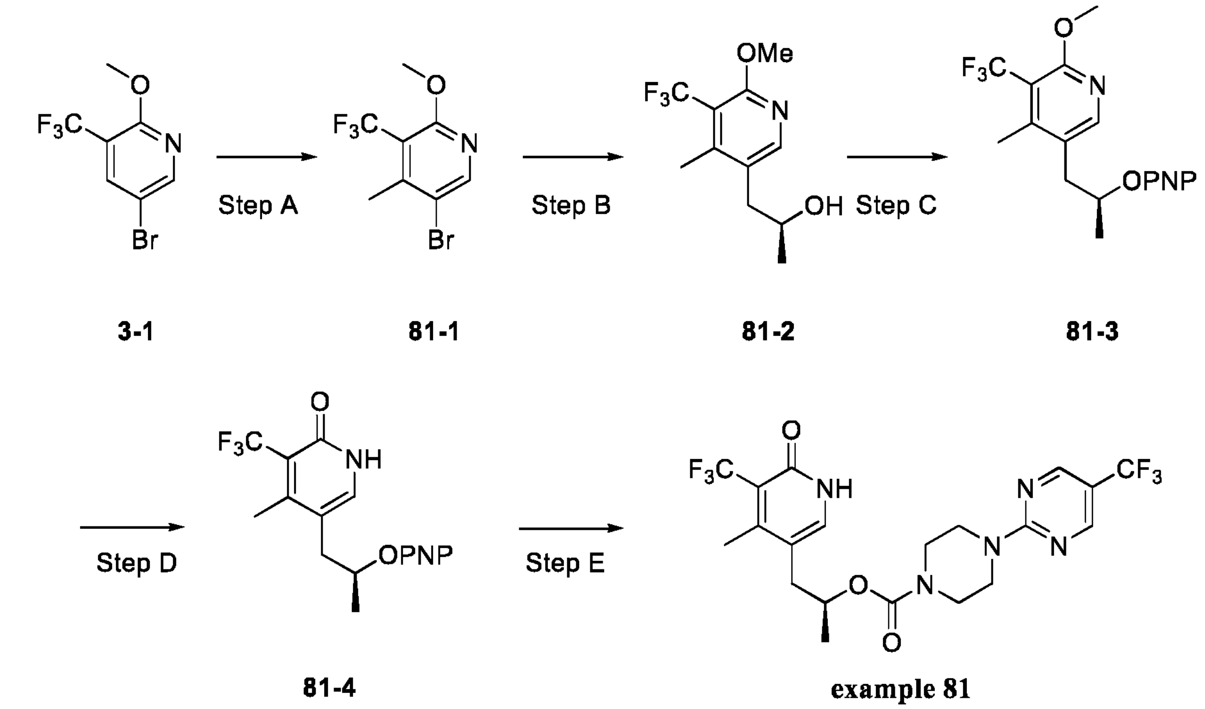


3-1 81-1 81-2 81-3

81-4 example 81

**Step A:** 5-Bromo-2-methoxy-3-(trifluoromethyl)pyridine (1.28 g, 5 mmol) was dissolved in anhydrous tetrahydrofuran (15 mL). The reaction mixture was cooled to -78°C, and slowly added dropwise with a solution of lithium diisopropylamide in tetrahydrofuran (2.0 mol/L, 2.75 mL, 5.5 mmol). After the addition was completed, the reaction mixture was stirred at -78°C for another 0.5 hours. The reaction mixture was then slowly added with iodomethane (781.0 mg, 5.5 mmol), and reacted at -78°C for 1 hour. The reaction mixture was added with saturated ammonium chloride aqueous solution (5 mL) to quench, and extracted with ethyl acetate (50 mL × 3). The organic phase was concentrated under reduced pressure, and the resulting residue was purified by silica gel column chromatography to obtain 1.12 g of 5-bromo-2-methoxy-4-methyl-3-(trifluoromethyl)pyridine **(81-1).**

MS (ESI) M/Z: 270.0 $[M+H]^+$.

$^1H$ NMR (400 MHz, $CDCl_3$) δ 8.36 (s, 1H), 3.98 (s, 3H), 2.56 (q, *J* = 2.6 Hz, 3H).

**Steps B to E:** 5-Bromo-2-methoxy-4-methyl-3-(trifluoromethyl)pyridine was used instead of 5-bromo-2-methoxy-3-(trifluoromethyl)pyridine with the preparation method referring to **example 48.**

MS (ESI) M/Z: 494.2 $[M+H]^+$.

$^1H$ NMR (400 MHz, DMSO-$d_6$) δ 12.01 (s, 1H), 8.73 (d, *J* = 0.9 Hz, 2H), 7.46 (s, 1H), 4.87 - 4.77 (m, 1H), 3.90 (brs, 2H), 3.70 (brs, 2H), 3.54 - 3.35 (m, 4H), 2.77 - 2.63 (m, 2H), 2.36 - 2.27 (m, 3H), 1.23 (d, *J* = 6.3 Hz, 3H).

**Example 82**

**(1*S*,2*S*)-2-(6-Oxo-5-(trifluoromethyl)-1,6-dihydropyridin-3-yl)cyclopentyl 4-(5-(trifluoromethyl)pyrimidin-2-yl)piperazine-1-carboxylate**

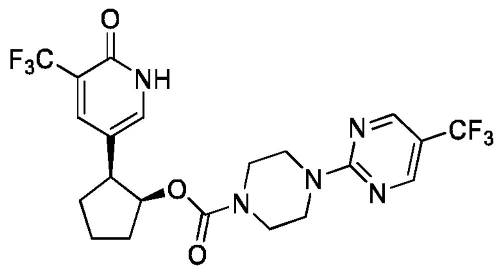

(1*S*,2*S*)-2-(6-Methoxy-5-(trifluoromethyl)pyridin-3-yl)cyclopentan-1-ol was used as a raw material instead of

1-(6-methoxy-5-(trifluoromethyl)pyridin-3-yl)propan-2-ol with the preparation method referring to **example 39.**

MS (ESI) M/Z: 506.0 $[M+H]^+$.

$^1H$ NMR (400 MHz, DMSO-$d_6$) δ 12.23 (s, 1H), 8.73 (s, 2H), 7.85 (d, *J* = 2.5 Hz, 1H), 7.53 (d, *J* = 2.6 Hz, 1H), 5.12 - 5.06 (m, 1H), 3.89 (brs, 2H), 3.58 (brs, 2H), 3.44 - 3.32 (m, 4H), 3.12 - 3.01 (m, 1H), 2.10 - 1.97 (m, 1H), 1.96 - 1.81 (m, 3H), 1.80 - 1.70 (m, 1H), 1.69 - 1.58 (m, 1H).

**Example 83**

**5-((2*S*)-2-((1-Oxo-1-(4-(5-(trifluoromethyl)pyrimidin-2-yl)piperazin-1-yl)propan-2-yl)oxy)methyl)pyrrolidin-1-yl)-3-(trifluoromethyl)pyridin-2(1*H*)-one**

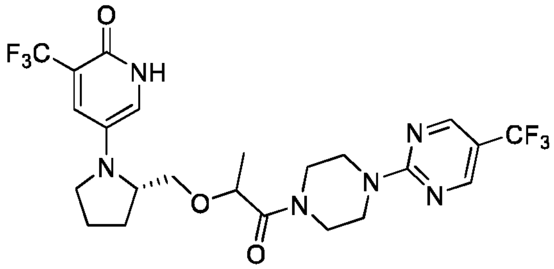

5-Bromo-2-methoxy-3-(trifluoromethyl)pyridine and methyl 2-bromopropanoate were used as raw materials with the preparation method referring to **example 7.**

MS (ESI) M/Z: 549.2 $[M+H]^+$.

$^1H$ NMR (400 MHz, DMSO-$d_6$) δ 11.86 (s, 1H), 8.77 - 8.68 (m, 2H), 7.70 (d, *J* = 31.6 Hz, 1H), 6.99 (s, 1H), 4.37 (q, *J* = 6.5 Hz, 1H), 3.95 - 3.68 (m, 5H), 3.66 - 3.46 (m, 5H), 3.38 - 3.23 (m, 2H), 1.97 - 1.73 (m, 3H), 1.25 - 1.20 (m, 3H).

**Example 84**

**(*S*)-1-(6-Oxo-5-(trifluoromethyl)-1,6-dihydropyridin-3-yl)propan-2-yl 4-(5-methylpyrimidin-2-yl)piperazine-1-carboxylate**

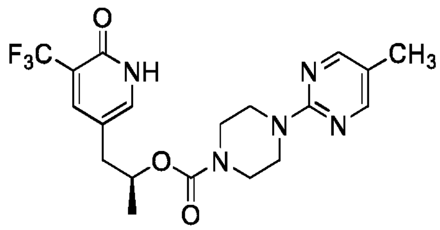

5-Bromo-2-methoxy-3-(trifluoromethyl)pyridine was used as a raw material, and 2-chloro-5-methylpyrimidine was used as a raw material instead of 2-chloro-5-(trifluoromethyl)pyrimidine with the preparation method referring to **intermediate 1C** and **example 48.**

MS (ESI) M/Z: 426.2 $[M+H]^+$.

$^1H$ NMR (400 MHz, DMSO-$d_6$) δ 12.18 (s, 1H), 8.25 (d, *J*= 0.8 Hz, 2H), 7.84 (d, *J* = 2.5 Hz, 1H), 7.55 (d, *J* = 2.5 Hz, 1H), 4.87 - 4.77 (m, 1H), 3.88 - 3.34 (m, 8H), 2.76 - 2.66 (m, 1H), 2.65 - 2.55 (m, 1H), 2.09 (s, 3H), 1.19 (d, *J* = 6.2 Hz, 3H).

**Example 85**

**(*S*)-1-(6-Oxo-5-(trifluoromethyl)-1,6-dihydropyridin-3-yl)propan-2-yl 4-(5-chloropyrimidin-2-yl)piperazine-1-carboxylate**

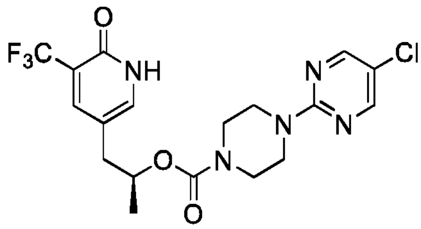

5-Bromo-2-methoxy-3-(trifluoromethyl)pyridine and 2,5-dichloropyrimidine were used as raw materials with the preparation method referring to **intermediate 1C** and **example 48.**

MS (ESI) M/Z: 446.0 $[M+H]^+$.

$^1H$ NMR(400 MHz, DMSO-$d_6$) δ11.92 (s, 1H), 8.45 (s, 2H), 7.84 (s, 1H), 7.55 (s, 1H), 4.85 - 4.77 (m, 1H), 3.88 - 3.38 (m, 8H), 2.74 - 2.66 (m, 1H), 2.64 - 2.56 (m, 1H), 1.19 (d, *J* = 6.2 Hz, 3H).

**Example 86**

**(*S*)-1-(6-Oxo-5-(trifluoromethyl)-1,6-dihydropyridin-3-yl)propan-2-yl 4-(5-(trifluoromethyl)pyridin-2-yl)piperazine-1-carboxylate**

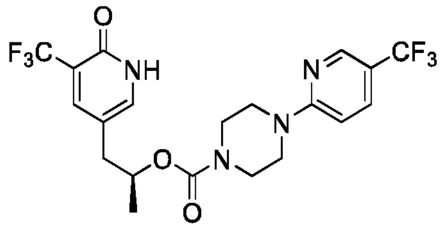

5-Bromo-2-methoxy-3-(trifluoromethyl)pyridine and intermediate **1D** were used as raw materials with the preparation method referring to **example 48.**

MS (ESI) M/Z: 479.0 $[M+H]^+$.

$^1H$ NMR (400 MHz, DMSO-$d_6$) δ 12.22 (s, 1H), 8.43 (d, *J* = 2.4 Hz, 1H), 7.87 - 7.80 (m, 2H), 7.55 (d, *J* = 2.4 Hz, 1H), 6.96 (d, *J* = 9.1 Hz, 1H), 4.88 - 4.78 (m, 1H), 3.76 - 3.39 (m, 8H), 2.75 - 2.68 (m, 1H), 2.64 - 2.58 (m, 1H), 1.19 (d, *J* = 6.2 Hz, 3H).

**Example 87**

**(*S*)-1-(6-Oxo-5-(trifluoromethyl)-1,6-dihydropyridin-3-yl)propan-2-yl 4-(5-(methylcarbamoyl)pyrimidin-2-yl)piperazine-1-carboxylate**

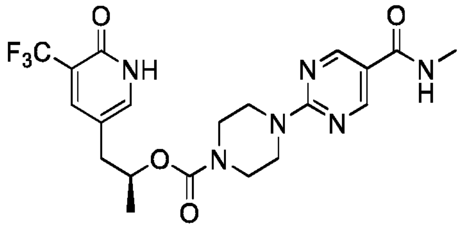

5-Bromo-2-methoxy-3-(trifluoromethyl)pyridine and *N*-methyl-2-(piperazin-1-yl)pyrimidine-5-carboxamide were used as raw materials with the preparation method referring to **example 48.**
MS (ESI) M/Z: 469.2 [M+H]$^+$.
$^1$H NMR (400 MHz, DMSO-$d_6$) δ 12.21 (s, 1H), 8.77 (s, 2H), 8.36 - 8.30 (m, 1H), 7.85 (d, *J* = 2.5 Hz, 1H), 7.56 (d, *J* = 2.4 Hz, 1H), 4.89 - 4.79 (m, 1H), 3.88 (brs, 2H), 3.66 (brs, 2H), 3.42 (brs, 4H), 2.78 - 2.67 (m, 4H), 2.65 - 2.57 (m, 1H), 1.19 (d, *J* = 6.3 Hz, 3H).

**Example 88**

**(*S*)-1-(6-Oxo-5-(trifluoromethyl)-1,6-dihydropyridin-3-yl)propan-2-yl 4-(6-(methylcarbamoyl)pyridin-3-yl)piperazine-1-carboxylate**

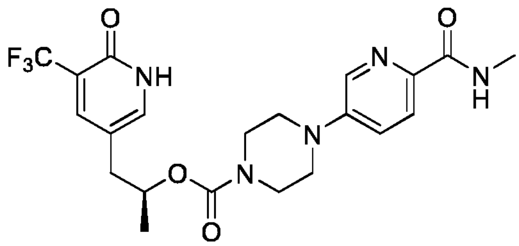

5-Bromo-2-methoxy-3-(trifluoromethyl)pyridine and *N*-methyl-5-(piperazin-1-yl)picolinamide were used as raw materials with the preparation method referring to **example 48.**
MS (ESI) M/Z: 468.2 [M+H]$^+$.
$^1$H NMR (400 MHz, DMSO-$d_6$) δ 12.23 (s, 1H), 8.46 - 8.39 (m, 1H), 8.27 (d, *J* = 2.9 Hz, 1H), 7.89 - 7.81 (m, 2H), 7.55 (s, 1H), 7.41 (dd, *J* = 8.8, 2.9 Hz, 1H), 4.89 - 4.78 (m, 1H), 3.62 - 3.14 (m, 8H), 2.78 (d, *J* = 4.8 Hz, 3H), 2.76 - 2.66 (m, 1H), 2.65 - 2.57 (m, 1H), 1.20 (d, *J* = 6.2 Hz, 3H).

**Example 89**

**(*S*)-1-(6-Oxo-5-(trifluoromethyl)-1,6-dihydropyridin-3-yl)propan-2-yl 2-(trifluoromethyl)-5,6-dihydro-[1,2,4]triazolo[1,5-*a*]pyrazine-7(8*H*)-carboxylate**

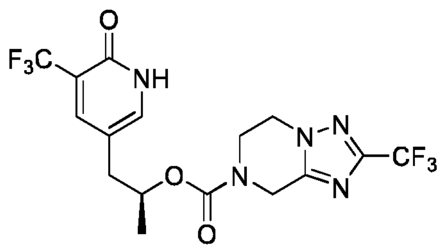

5-Bromo-2-methoxy-3-(trifluoromethyl)pyridine and 2-(trifluoromethyl)-5,6-dihydro-8*H*-λ$^2$-[1,2,4]triazolo[5,1-*c*]pyrazine were used as raw materials with the preparation method referring to **example 48.**
MS (ESI) M/Z: 440.0 [M+H]$^+$.

$^1H$ NMR (400 MHz, DMSO-$d_6$) δ 12.16 (s, 1H), 7.87 (brs, 1H), 7.59 (d, *J* = 2.5 Hz, 1H), 4.89 - 4.67 (m, 3H), 4.23 (brs, 2H), 4.01 - 3.84 (m, 2H), 2.75 - 2.61 (m, 2H), 1.21 (d, *J*= 6.2 Hz, 3H).

**Example 90**

**(*S*)-1-(6-Oxo-5-(trifluoromethyl)-1,6-dihydropyridin-3-yl)propan-2-yl 4-(cyclopropanecarbonyl)piperazine-1-carboxylate**

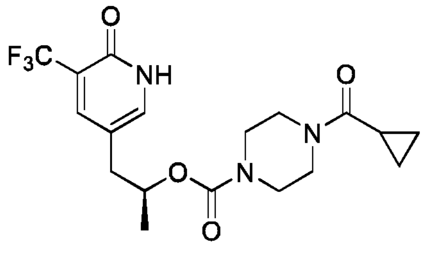

5-Bromo-2-methoxy-3-(trifluoromethyl)pyridine and 1-(cyclopropanecarbonyl)piperazine were used as raw materials with the preparation method referring to **example 48.**

MS (ESI) M/Z: 402.0 [M+H]$^+$.

$^1H$ NMR (400 MHz, DMSO-$d_6$) δ 12.19 (s, 1H), 7.84 (d, *J* = 2.5 Hz, 1H), 7.54 (d, *J* = 2.6 Hz, 1H), 4.86 - 4.76 (m, 1H), 3.81 - 3.32 (m, 8H), 2.74 - 2.66 (m, 1H), 2.64 - 2.56 (m, 1H), 2.00 - 1.90 (m, 1H), 1.18 (d, *J* = 6.2 Hz, 3H), 0.77 - 0.68 (m, 4H).

**Example 91**

**(*S*)-1-(6-Oxo-5-(trifluoromethyl)-1,6-dihydropyridin-3-yl)propan-2-yl (R)-2-methyl-4-(5-(trifluoromethyl)pyrimidin-2-yl)piperazine-1-carboxylate**

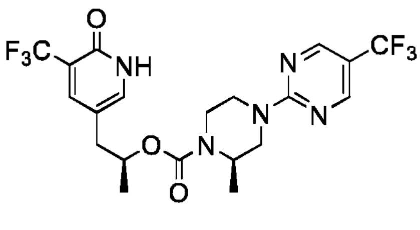

5-Bromo-2-methoxy-3-(trifluoromethyl)pyridine and tert-butyl (R)-2-methylpiperazine-1-carboxylate were used as raw materials with the preparation method referring to **intermediate 1C** and **example 48.**

MS (ESI) M/Z: 494.2 [M+H]$^+$.

$^1H$ NMR (400 MHz, DMSO-$d_6$) δ 12.21 (s, 1H), 8.72 (s, 2H), 7.85 (d, *J* = 2.5 Hz, 1H), 7.57 (s, 1H), 4.87 - 4.75 (m, 1H), 4.59 - 4.41 (m, 2H), 4.26 (brs, 1H), 3.83 (d, *J* = 12.4 Hz, 1H), 3.22 (d, *J* = 13.8 Hz, 1H), 3.15 - 2.96 (m, 2H), 2.75 - 2.65 (m, 1H), 2.65 - 2.57 (m, 1H), 1.20 (d, *J* = 6.2 Hz, 3H), 0.97 (d, *J* = 6.7 Hz, 3H).

**Example 92**

**(*S*)-1-(6-Oxo-5-(trifluoromethyl)-1,6-dihydropyridin-3-yl)propan-2-yl (*S*)-2-methyl-4-(5-(trifluoromethyl)pyrimidin-2-yl)piperazine-1-carboxylate**

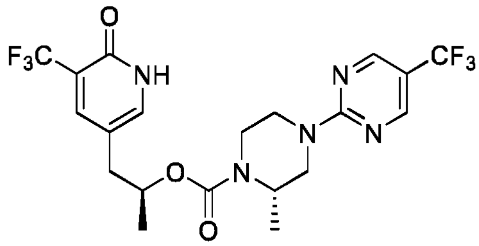

5-Bromo-2-methoxy-3-(trifluoromethyl)pyridine and *tert*-butyl (*S*)-2-methylpiperazine-1-carboxylate were used as raw materials with the preparation method referring to **intermediate 1C** and **example 48.**
MS (ESI) M/Z: 494.0 $[M+H]^+$.
$^1H$ NMR (400 MHz, DMSO-$d_6$) δ 12.18 (s, 1H), 8.72 (s, 2H), 7.83 (d, *J*= 2.5 Hz, 1H), 7.58 - 7.51 (m, 1H), 4.94 - 4.82 (m, 1H), 4.57 - 4.44 (m, 2H), 4.27 - 4.18 (m, 1H), 3.88 - 3.78 (m, 1H), 3.24 (dd, *J* = 13.4, 4.0 Hz, 1H), 3.13 - 3.01 (m, 2H), 2.76 - 2.68 (m, 1H), 2.63 - 2.54 (m, 1H), 1.19 (d, *J* = 6.3 Hz, 3H), 0.96 (brs, 3H).

**Example 93**

**(*S*)-1-(6-Oxo-5-(trifluoromethyl)-1,6-dihydropyridin-3-yl)propan-2-yl (*S*)-3-methyl-4-(5-(trifluoromethyl)pyrimidin-2-yl)piperazine-1-carboxylate**

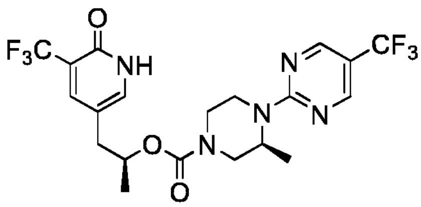

5-Bromo-2-methoxy-3-(trifluoromethyl)pyridine and tert-butyl (*S*)-3-methylpiperazine-1-carboxylate were used as raw materials with the preparation method referring to **intermediate 1C** and **example 48.**
MS (ESI) M/Z: 494.0 $[M+H]^+$.
$^1H$ NMR (400 MHz, DMSO-$d_6$) δ 11.81 (brs, 1H), 8.74 (s, 2H), 7.86 (s, 1H), 7.57 (s, 1H), 4.85 (brs, 2H), 4.51 - 4.39 (m, 1H), 3.96 (d, *J* = 13.0 Hz, 1H), 3.79 (d, *J* = 13.3 Hz, 1H), 3.26 - 3.05 (m, 2H), 2.93 (brs, 1H), 2.75 - 2.58 (m, 2H), 1.20 (d, *J* = 6.1 Hz, 3H), 1.13 - 0.95 (m, 3H).

**Example 94**

**(*S*)-1-(6-Oxo-5-(trifluoromethyl)-1,6-dihydropyridin-3-yl)propan-2-yl (R)-3-methyl-4-(5-(trifluoromethyl)pyrimidin-2-yl)piperazine-1-carboxylate**

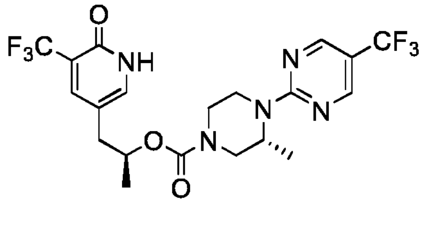

5-Bromo-2-methoxy-3-(trifluoromethyl)pyridine and *tert*-butyl (*R*)-3-methylpiperazine-1-carboxylate were used as raw materials with the preparation method referring to **intermediate 1C** and **example 48.**
MS (ESI) M/Z: 494.0 $[M+H]^+$.
$^1H$ NMR (400 MHz, DMSO-$d_6$) δ 12.22 (s, 1H), 8.74 (s, 2H), 7.87 (s, 1H), 7.58 (s, 1H), 4.98 - 4.79 (m, 2H), 4.46 (d, *J* = 12.5 Hz, 1H), 4.01 - 3.92 (m, 1H), 3.82 (d, *J* = 13.2 Hz, 1H), 3.20 - 2.88 (m, 3H), 2.77 - 2.68 (m, 1H), 2.65

- 2.57 (m, 1H), 1.20 (d, *J* = 6.2 Hz, 3H), 1.13 - 0.90 (m, 3H).

**Example 95**

**3-(6-Oxo-5-(trifluoromethyl)-1,6-dihydropyridin-3-yl)phenyl** 4-(5-**(trifluoromethyl)pyrimidin-2-yl)piperazine-1-carboxylate**

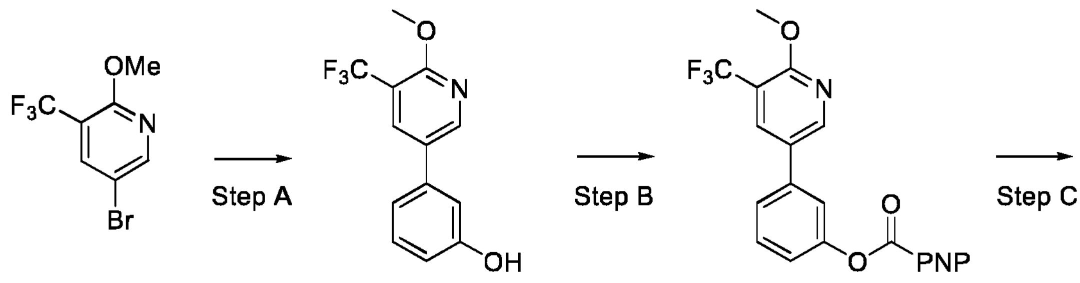


**3-1** **95-2** **95-3**

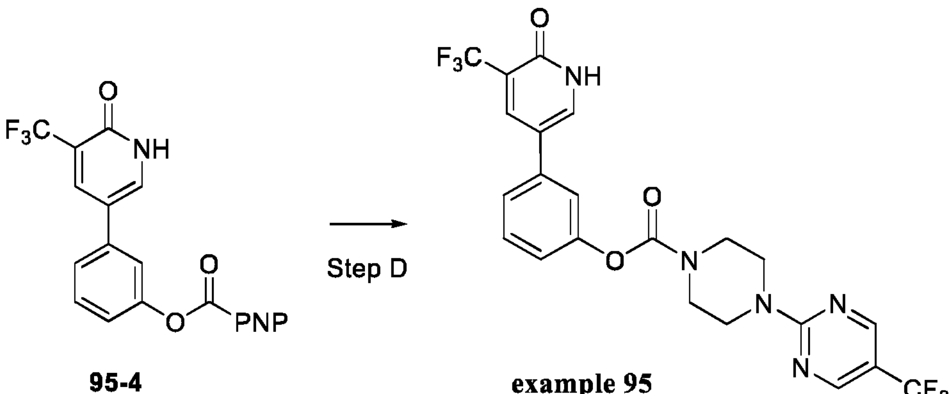


**95-4** **example 95**

**Step A:** 5-Bromo-2-methoxy-3-(trifluoromethyl)pyridine (100 mg, 0.4 mmol), (3-hydroxyphenyl)boronic acid (69 mg, 0.5 mmol), and sodium carbonate (85 mg, 0.8 mmol) were dissolved in 1,4-dioxane (2 mL) and water (0.2 mL) at room temperature. The reaction mixture was replaced with nitrogen for 15 minutes, then added with [1,1'-bis(diphenylphosphino)ferrocene]palladium(II) dichloride (29.0 mg, 0.045 mmol), and reacted in a sealed tube under microwave irradiation at 120°C for 1 hour. The reaction mixture was concentrated under reduced pressure, and the resulting residue was purified by silica gel column chromatography to obtain 135 mg of 3-(6-methoxy-5-(trifluoromethyl)pyridin-3-yl)phenol **(95-2).**

MS (ESI) M/Z: 270.1 [M+H]$^+$.

**Step B:** 3-(6-Methoxy-5-(trifluoromethyl)pyridin-3-yl)phenol (135 mg, 0.5 mmol) was dissolved in *N*,*N*-dimethylformamide (3 mL) at room temperature, and then bis(4-nitrophenyl) carbonate (305 mg, 1.0 mmol) and *N*,*N*-diisopropylethylamine (129 mg, 1.0 mmol) were added thereto. The reaction mixture was stirred at room temperature for 2 hours, added with water (30 mL) to quench, and extracted with ethyl acetate (10 mL × 3). The organic phases were combined, first washed with water (10 mL × 3) and saturated brine (30 mL), dried over anhydrous sodium sulfate, filtered, and concentrated. The resulting residue was purified by silica gel column chromatography to obtain 93 mg of 3-(6-methoxy-5-(trifluoromethyl)pyridin-3-yl)phenyl (4-nitrophenyl) carbonate **(95-3).**

MS (ESI) M/Z: 435.3 [M+H]$^+$.

**Step C:** 3-(6-Methoxy-5-(trifluoromethyl)pyridin-3-yl)phenyl (4-nitrophenyl) carbonate (90 mg, 0.2 mmol) was dissolved in dichloromethane (1 mL), and then iodotrimethylsilane (0.06 mL) was added to the above solution. The reaction mixture was stirred at room temperature for 3 hours, and added with ice water (10 mL) to quench. The mixture was extracted with ethyl acetate (30 mL × 3). The organic phases were combined, first washed with saturated sodium thiosulfate solution (20 mL), then washed with saturated brine (20 mL), dried over anhydrous sodium sulfate, filtered, and concentrated. The resulting residue was purified by silica gel column chromatography to obtain 50 mg of 4-nitrophenyl (3-(6-oxo-5-(trifluoromethyl)-1,6-dihydropyridin-3-yl)phenyl) carbonate **(95-4).**

MS (ESI) M/Z: 421.3 $[M+H]^+$.

**Step D:** 4-Nitrophenyl (3-(6-oxo-5-(trifluoromethyl)-1,6-dihydropyridin-3-yl)phenyl) carbonate and intermediate **1C** were used as raw materials with the preparation method referring to example **48.**

MS (ESI) M/Z: 514.0 $[M+H]^+$.

$^1$H NMR (400 MHz, DMSO-$d_6$) δ 12.64 (s, 1H), 8.76 (d, *J* = 0.9 Hz, 2H), 8.25 - 8.19 (m, 1H), 8.08 (d, *J* = 2.7 Hz, 1H), 7.54 - 7.48 (m, 2H), 7.47 - 7.40 (m, 1H), 7.15 - 7.09 (m, 1H), 3.97 (brs, 4H), 3.73 (brs, 2H), 3.57 (brs, 2H).

**Example 96**

**5-(3-(2-Oxo-2-(4-(5-(trifluoromethyl)pyrimidin-2-yl)piperazin-1-yl)ethyl)phenyl)-3-(trifluoromethyl)pyridin-2(1*H*)-one**

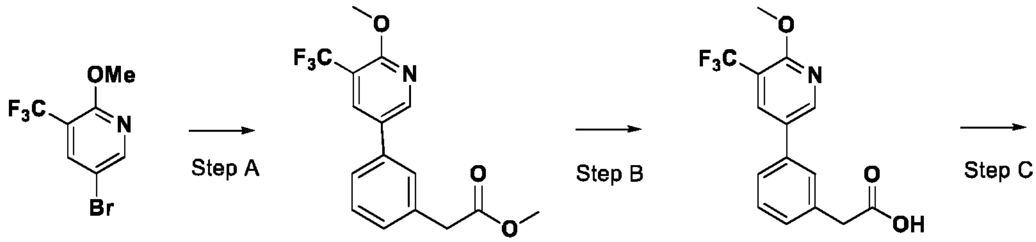


**3-1** **96-2** **96-3**

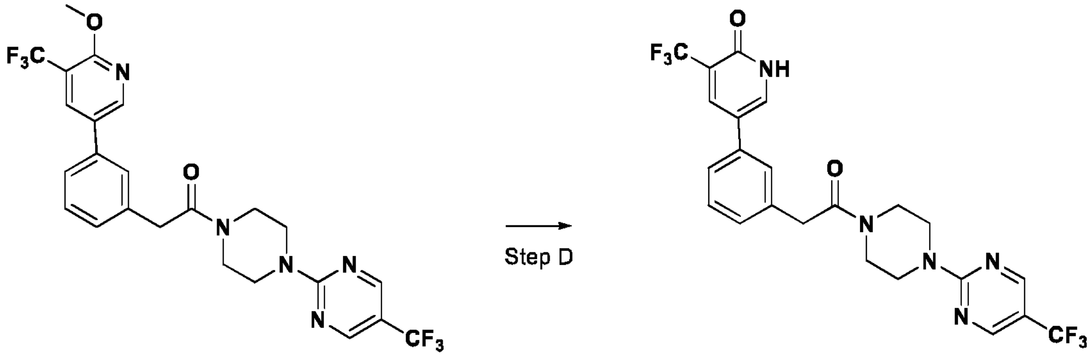


**96-4** **example 96**

**Step A:** (3-(2-Methoxy-2-oxoethyl)phenyl)boronic acid was used as a raw material with the preparation method referring to example **95-2.**

MS (ESI) M/Z: 326.0 $[M+H]^+$.

**Step B:** Methyl 2-(3-(6-methoxy-5-(trifluoromethyl)pyridin-3-yl)phenyl)acetate (125 mg, 0.38 mmol) was dissolved in tetrahydrofuran (3 mL) and water (1 mL) at room temperature. Lithium hydroxide monohydrate (46 mg, 1.92 mmol) was then added thereto. The reaction mixture was stirred at room temperature for 18 hours. The reaction mixture was added with water (5 mL) to quench, added with 1 mol of dilute hydrochloric acid to adjust the pH to 5, and extracted with ethyl acetate (20 mL × 3). The organic phases were combined, first washed with saturated brine (20 mL), dried over anhydrous sodium sulfate, filtered, and concentrated. 118 mg of 2-(3-(6-methoxy-5-(trifluoromethyl)pyridin-3-yl)phenyl)acetic acid **(96-3)** was obtained.

MS (ESI) M/Z: 312.0 $[M+H]^+$.

**Step C:** 2-(3-(6-Methoxy-5-(trifluoromethyl)pyridin-3-yl)phenyl)acetic acid (60 mg, 0.2 mmol) was dissolved in *N*,*N*-dimethylformamide (1 mL). 2-(7-Azabenzotriazol-1-yl)-*N*,*N*,*N'*,*N'*-tetramethyluronium hexafluorophosphate (110 mg, 0.3 mmol) was then added to the above solution. 2-(Piperazin-1-yl)-5-(trifluoromethyl)pyrimidine (67 mg, 0.3 mmol) and *N*,*N*-diisopropylethylamine (73 mg, 0.57 mmol) were then added thereto. The reaction mixture was stirred at room temperature for 2 hours. The mixture was added with water (20 mL) to quench, and extracted with ethyl acetate (30 mL × 3). The organic phases were combined, first washed with water (20 mL × 3), then washed with saturated brine (20 mL), dried over anhydrous sodium sulfate, filtered, and concentrated. The resulting residue was purified by silica gel column chromatography to obtain 72 mg of 2-(3-(6-methoxy-5-(trifluoromethyl)pyridin-3-yl)phenyl)-1-(4-(5-(trifluoromethyl)pyrimidin-2-yl)piperazin-1-yl)ethan-1-one **(96-4).**

MS (ESI) M/Z: 526.0 $[M+H]^+$.

**Step D:** 2-(3-(6-Methoxy-5-(trifluoromethyl)pyridin-3-yl)phenyl)-1-(4-(5-(trifluoromethyl)pyrimidin-2-yl)piperazin-1-yl)ethan-1-one was used as a raw material with the preparation method referring to example **95-4** to obtain 52.3 mg of 5-(3-(2-oxo-2-(4-(5-(trifluoromethyl)pyrimidin-2-yl)piperazin-1-yl)ethyl)phenyl)-3-(trifluoromethyl)pyridin-2(1*H*)-one **(compound 96).**

MS (ESI) M/Z: 512.1 $[M+H]^+$.

$^1H$ NMR (400 MHz, DMSO-$d_6$) δ 12.63 (s, 1H), 8.73 (d, *J* = 1.0 Hz, 2H), 8.18 (d, *J* = 2.7 Hz, 1H), 8.01 (s, 1H), 7.53 - 7.47 (m, 2H), 7.40 - 7.33 (m, 1H), 7.20 (d, *J* = 7.5 Hz, 1H), 3.82 (s, 6H), 3.68 - 3.54 (m, 4H).

**Example 97**

**(*S*)-1-(6-Oxo-5-(trifluoromethyl)-1,6-dihydropyridin-3-yl)propan-2-yl 4-(4-(trifluoromethyl)phenyl)piperazine-1-carboxylate**

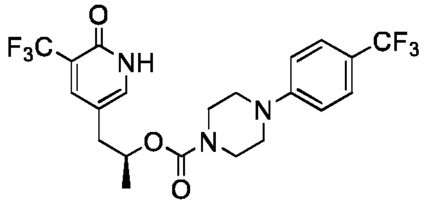

5-Bromo-2-methoxy-3-(trifluoromethyl)pyridine and 1-(4-(trifluoromethyl)phenyl)piperazine were used as raw materials with the preparation method referring to **example 48.**

MS (ESI) M/Z: 476.1 $[M-H]^-$.

$^1H$ NMR (400 MHz, DMSO-$d_6$) δ 12.05 (s, 1H), 7.84 (d, J= 2.5 Hz, 1H), 7.59 - 7.49 (m, 3H), 7.07 (d, *J* = 8.7 Hz, 2H), 4.90 - 4.78 (m, 1H), 3.48 (brs, 4H), 3.30 - 3.07 (m, 4H), 2.77 - 2.67 (m, 1H), 2.66 - 2.56 (m, 1H), 1.20 (d, *J* = 6.2 Hz, 3H).

**Example 98**

**(*S*)-1-(6-oxo-5-(trifluoromethyl)-1,6-dihydropyridin-3-yl)propan-2-yl 5-(trifluoromethyl)-3',6'-dihydro-[2,4'-bipyridine]-1'(2'*H*)-carboxylate**

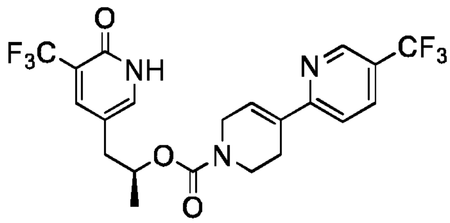

5-Bromo-2-methoxy-3-(trifluoromethyl)pyridine and intermediate **1G** were used as raw materials with the preparation method referring to **example 48**.

MS (ESI) M/Z: 476.0 $[M+H]^+$.

$^1H$ NMR (400 MHz, DMSO-$d_6$) δ 11.67 (s, 1H), 8.92 (d, *J* = 2.4 Hz, 1H), 8.22 - 8.14 (m, 1H), 7.85 (s, 1H), 7.77 (d, *J* = 8.4 Hz, 1H), 7.56 (s, 1H), 6.87 (s, 1H), 4.90 - 4.76 (m, 1H), 4.08 (brs, 2H), 3.68 - 3.46 (m, 2H), 2.77 - 2.53 (m, 4H), 1.19 (d, *J* = 6.2 Hz, 3H).

**Example 99**

**(*S*)-1-(6-oxo-5-(trifluoromethyl)-1,6-dihydropyridin-3-yl)propan-2-yl 4-(5-(trifluoromethyl)pyridin-2-yl)piperidine-1-carboxylate**

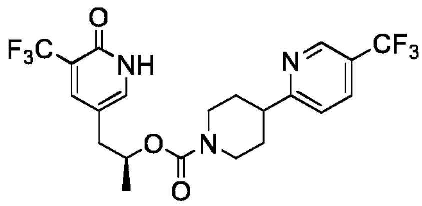

5-Bromo-2-methoxy-3-(trifluoromethyl)pyridine and intermediate **1H** were used as raw materials with the preparation method referring to **example 48.**
MS (ESI) M/Z: 478.0 $[M+H]^+$.
$^1H$ NMR (400 MHz, DMSO-$d_6$) δ 8.88 (brs, 1H), 8.13 (dd, *J* = 8.3, 2.5 Hz, 1H), 7.83 (d, *J* = 2.5 Hz, 1H), 7.58 - 7.47 (m, 2H), 4.87 - 4.76 (m, 1H), 4.12 - 3.98 (m, 2H), 3.05 - 2.77 (m, 3H), 2.74 - 2.65 (m, 1H), 2.65 - 2.55 (m, 1H), 1.87 - 1.77 (m, 2H), 1.65 - 1.41 (m, 2H), 1.18 (d, *J* = 6.2 Hz, 3H).

**Example 100**

**(*S*)-1-(6-oxo-5-(trifluoromethyl)-1,6-dihydropyridin-3-yl)propan-2-yl 4-(5-(2-hydroxypropan-2-yl)pyrimidin-2-yl)piperazine-1-carboxylate**

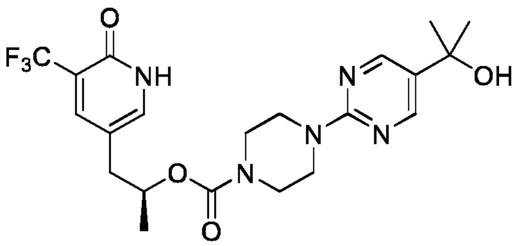

5-Bromo-2-methoxy-3-(trifluoromethyl)pyridine and intermediate **1F** were used as raw materials with the preparation method referring to **example 48**.
MS (ESI) M/Z: 468.2 $[M-H]^-$.
$^1H$ NMR (400 MHz, DMSO-$d_6$) δ 12.17 (s, 1H), 8.45 (s, 2H), 7.85 (d, *J* = 2.5 Hz, 1H), 7.56 (d, *J* = 2.5 Hz, 1H), 5.09 (s, 1H), 4.88 - 4.77 (m, 1H), 3.77 (brs, 2H), 3.55 (brs, 2H), 3.39 (brs, 4H), 2.75 - 2.66 (m, 1H), 2.64 - 2.54 (m, 1H), 1.41 (s, 6H), 1.19 (d, *J* = 6.2 Hz, 3H).

**Example 101**

**(*S*)-1-(6-oxo-5-(trifluoromethyl)-1,6-dihydropyridin-3-yl)propan-2-yl 4-(5-(methylsulfonyl)pyrimidin-2-yl)piperazine-1-carboxylate**

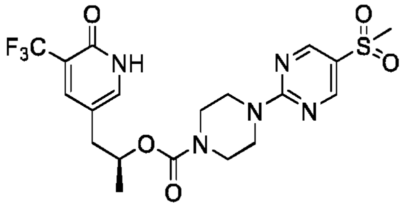

5-Bromo-2-methoxy-3-(trifluoromethyl)pyridine and 2-chloro-5-(methylsulfonyl)pyrimidine were used as raw materials with the preparation method referring to **intermediate 1C** and **example 48**.
MS (ESI) M/Z: 490.0 $[M+H]^+$.
$^1H$ NMR (400 MHz, DMSO-$d_6$) δ 12.17 (s, 1H), 8.75 (s, 2H), 7.86 (d, *J* = 2.5 Hz, 1H), 7.56 (d, *J* = 2.5 Hz, 1H), 4.91 - 4.74 (m, 1H), 4.00 - 3.64 (m, 4H), 3.55 - 3.37 (m, 4H), 3.31 (s, 3H), 2.75 - 2.66 (m, 1H), 2.63 - 2.56 (m, 1H), 1.20 (d, *J* = 6.2 Hz, 3H).

**Example 102**

**1-(1-oxo-1,2-dihydroisoquinolin-4-yl)propan-2-yl 4-(5-(trifluoromethyl)pyrimidin-2-yl)piperazine-1-carboxylate**

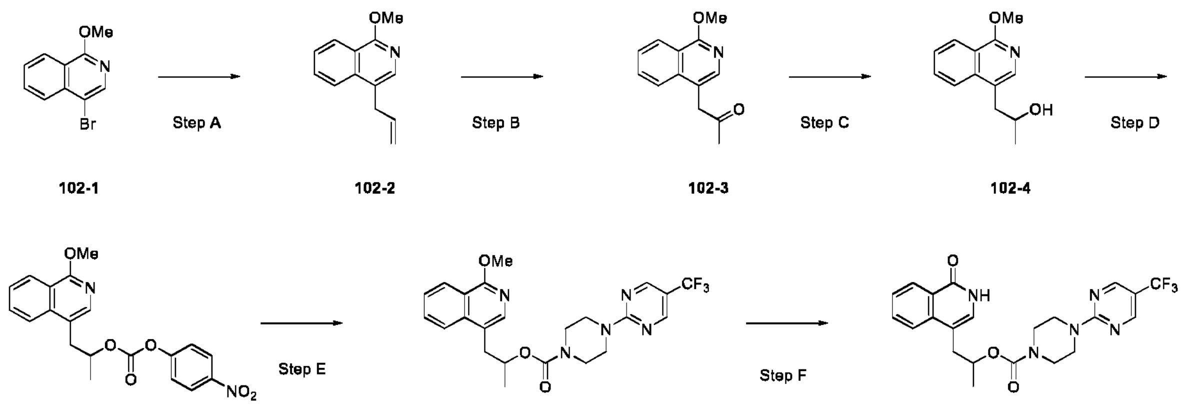


102-1 102-2 102-3 102-4

102-5 102-6 compound 102

**Step A:** 4-Bromo-1-methoxyisoquinoline (500.0 mg, 2.1 mmol), allylboronic acid pinacol ester (105.0 mg, 6.3 mmol), tetrakis(triphenylphosphine)palladium (245.0 mg, 0.21 mmol), potassium carbonate (435.0 mg, 3.2 mmol), and 1,4-dioxane (5.0 mL) were sequentially added to a reaction flask, and the reaction mixture was heated and reacted at 110°C for 4 hours under nitrogen atmosphere. The reaction mixture was cooled to room temperature, and then filtered through diatomite. The organic phase was concentrated under reduced pressure, and the resulting residue was purified by silica gel column chromatography to obtain 368.0 mg of 4-allyl-1-methoxyisoquinoline (**102-2**).
MS (ESI) M/Z: 200.2 $[M+H]^+$.
**Step B:** 4-Allyl-1-methoxyisoquinoline (368.0 mg, 2.0 mmol), palladium chloride (35.5 mg, 0.2 mmol), and manganese dioxide (695.2 mg, 8.0 mmol) were dissolved in a mixed solvent of acetonitrile/water (v/v = 7/1, 12 mL), and the reaction mixture was reacted at 60°C overnight. The reaction mixture was cooled to room temperature, filtered through diatomite, and the organic phase was concentrated under reduced pressure to obtain a crude product of 1-(1-methoxyisoquinolin-4-yl)propan-2-one (**102-3**), which was directly used in the next reaction step.
MS (ESI) M/Z: 216.2 $[M+H]^+$.
**Step C:** The crude product of 1-(1-methoxyisoquinolin-4-yl)propan-2-one was dissolved in methanol (12.0 mL) at room temperature, then sodium borohydride (226.8 mg, 6.0 mmol) was added thereto, and the reaction mixture was stirred at room temperature for 15 minutes. The reaction mixture was concentrated under reduced pressure, and the resulting residue was purified by silica gel column chromatography to obtain 310.0 mg of 1-(1-methoxyisoquinolin-4-yl)propan-2-ol (**102-4**).
$^1H$ NMR (400 MHz, $CDCl_3$) δ 8.30 (dd, *J* = 8.4, 1.4 Hz, 1H), 7.93 - 7.86 (m, 2H), 7.74 - 7.68 (m, 1H), 7.59 - 7.53 (m, 1H), 4.15 - 4.10 (m, 4H), 3.09 (dd, *J* = 14.1, 4.9 Hz, 1H), 2.97 (dd, *J* = 14.1, 8.0 Hz, 1H), 1.32 (d, *J* = 6.1 Hz, 3H).
**Steps D to F**: 1-(1-Methoxyisoquinolin-4-yl)propan-2-ol was used instead of 1-(6-methoxy-5-(trifluoromethyl)pyridin-3-yl)propan-2-ol with the preparation method referring to **example 33.**

MS (ESI) M/Z: 462.2 [M+H]$^+$.

$^1$H NMR (400 MHz, DMSO-$d_6$) δ 11.21 (s, 1H), 8.73 (s, 2H), 8.22 (dd, *J* = 8.0, 1.4 Hz, 1H), 7.91 (d, *J* = 8.1 Hz, 1H), 7.80 - 7.72 (m, 1H), 7.54 - 7.47 (m, 1H), 7.07 (s, 1H), 4.98 - 4.87 (m, 1H), 3.94 - 3.57 (m, 4H), 3.42 (brs, 4H), 3.01 - 2.84 (m, 2H), 1.26 (d, *J* = 6.3 Hz, 3H).

**Example 103**

**(*S*)-1-(6-oxo-5-(trifluoromethyl)-1,6-dihydropyridin-3-yl)propan-2-yl 4-(5-(trifluoromethyl)picolinoyl)piperazine-1-carboxylate**

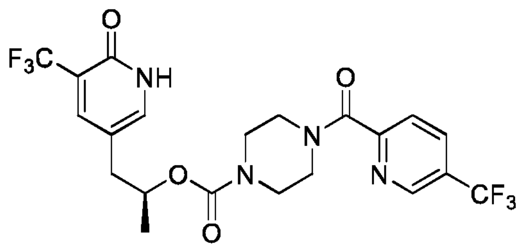

5-Bromo-2-methoxy-3-(trifluoromethyl)pyridine and intermediate **1E** were used as raw materials with the preparation method referring to **intermediate 1C** and **example 48**.

MS (ESI) M/Z: 507.0 [M+H]$^+$.

$^1$H NMR (400 MHz, DMSO-$d_6$) δ 12.19 (s, 1H), 9.04 - 8.97 (m, 1H), 8.38 (dd, *J* = 8.3, 2.3 Hz, 1H), 7.88 - 7.79 (m, 2H), 7.54 (s, 1H), 4.86 - 4.75 (m, 1H), 3.83 - 3.33 (m, 8H), 2.75 - 2.66 (m, 1H), 2.64 - 2.54 (m, 1H), 1.18 (d, *J* = 6.2 Hz, 3H).

**Example 104**

**2-(6-Oxo-5-(trifluoromethyl)-1,6-dihydropyridin-3-yl)ethyl (*R*)-3-(hydroxymethyl)-4-(5-(trifluoromethyl)pyrimidin-2-yl)piperazine-1-carboxylate**

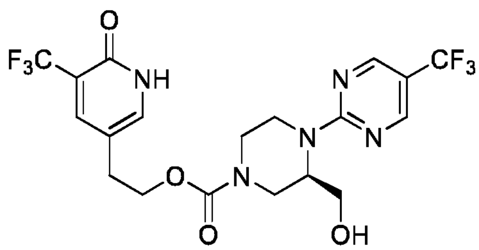

5-Bromo-2-methoxy-3-(trifluoromethyl)pyridine and tert-butyl (R)-3-(hydroxymethyl)piperazine-1-carboxylate were used as raw materials with the preparation method referring to **intermediate 1C** and **example 3**.

MS (ESI) M/Z: 496.0 [M+H]$^+$.

$^1$H NMR (400 MHz, DMSO-$d_6$) δ 12.21 (s, 1H), 8.72 (s, 2H), 7.90 (d, *J* = 2.5 Hz, 1H), 7.59 (s, 1H), 4.85 (t, *J* = 5.2 Hz, 1H), 4.75 (brs, 1H), 4.50 (d, *J* = 13.7 Hz, 1H), 4.15 (t, *J* = 6.3 Hz, 2H), 4.07 (d, *J* = 13.4 Hz, 1H), 3.88 (brs, 1H), 3.53 - 3.39 (m, 2H), 3.23 - 2.90 (m, 3H), 2.72 (t, *J* = 6.4 Hz, 2H).

**Example 105**

**2-(6-Oxo-5-(trifluoromethyl)-1,6-dihydropyridin-3-yl) 4-(5-methylpyrimidin-2-yl)piperazine-1-carboxylate**

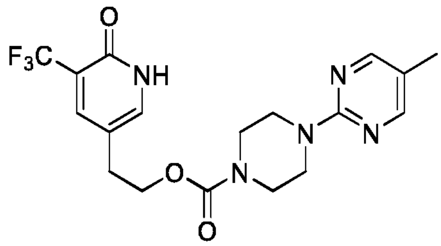

5-Bromo-2-methoxy-3-(trifluoromethyl)pyridine and 2-chloro-5-methylpyrimidine were used as raw materials with the preparation method referring to **intermediate 1C** and **example 3**.

MS (ESI) M/Z: 412.0 [M+H]$^+$.

$^1$H NMR (400 MHz, DMSO-$d_6$) δ 11.92 (brs, 1H), 8.25 (s, 2H), 7.89 (d, *J* = 2.5 Hz, 1H), 7.59 (d, *J* = 2.5 Hz, 1H), 4.15 (t, *J* = 6.3 Hz, 2H), 3.69 - 3.60 (m, 4H), 3.42 - 3.36 (m, 4H), 2.72 (t, *J* = 6.3 Hz, 2H), 2.09 (s, 3H).

**Example 106**

**2-(6-Oxo-5-(trifluoromethyl)-1,6-dihydropyridin-3-yl)ethyl 4-(5-(trifluoromethyl)pyridin-2-yl)piperazine-1-carboxylate**

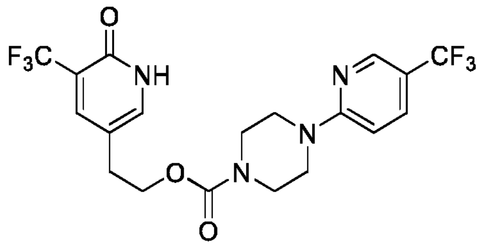

2-(Methoxy)-5-bromo-3-(trifluoromethyl)pyridine and intermediate **1D** were used as raw materials with the preparation method referring to **example 3.**

MS (ESI) M/Z: 465.0 [M+H]$^+$.

$^1$H NMR (400 MHz, DMSO-$d_6$) δ 12.16 (brs, 1H), 8.43 (d, *J* = 2.5 Hz, 1H), 7.90 (d, *J* = 2.5 Hz, 1H), 7.83 (dd, *J* = 9.1, 2.6 Hz, 1H), 7.59 (d, *J* = 2.5 Hz, 1H), 6.96 (d, *J* = 9.1 Hz, 1H), 4.16 (t, *J* = 6.3 Hz, 2H), 3.67 - 3.60 (m, 4H), 3.49 - 3.41 (m, 4H), 2.73 (t, *J* = 6.3 Hz, 2H).

**Example 107**

**2-(6-Oxo-5-(trifluoromethyl)-1,6-dihydropyridin-3-yl)ethyl (R)-2-(hydroxymethyl)-4-(5-(trifluoromethyl)pyrimidin-2-yl)piperazine-1-carboxylate**

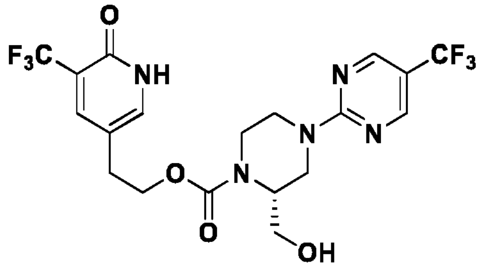

2-(Methoxy)-5-bromo-3-(trifluoromethyl)pyridine, *tert*-Butyl (R)-2-(hydroxymethyl)piperazine-1-carboxylate, and 2-chloro-5-(trifluoromethyl)pyrimidine were used as raw materials with the preparation method referring to **intermediate 1C** and **example 3**.

MS (ESI) M/Z: 496.0 [M+H]+.

$^1$H NMR (400 MHz, DMSO-$d_6$) δ 12.15 (s, 1H), 8.72 (s, 2H), 7.90 (d, *J* = 2.0 Hz, 1H), 7.59 (s, 1H), 4.77 (t, *J* = 5.3 Hz, 1H), 4.67 (d, *J* = 13.5 Hz, 1H), 4.43 (d, *J* = 5.6 Hz, 1H), 4.16 (t, *J* = 6.3 Hz, 2H), 4.08 (brs, 1H), 3.86 (d, *J* = 9.9 Hz, 1H), 3.30 - 3.21 (m, 3H), 3.12 (d, *J* = 9.3 Hz, 2H), 2.72 (t, *J* = 6.3 Hz, 2H).

**Example 108**

**2-(6-Oxo-5-(trifluoromethyl)-1,6-dihydropyridin-3-yl)ethyl (S)-2-methyl-4-(5-(trifluoromethyl)pyrimidin-2-yl)piperazine-1-carboxylate**

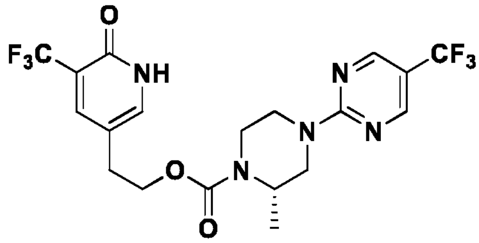

5-Bromo-2-methoxy-3-(trifluoromethyl)pyridine and *tert*-butyl (*S*)-2-methylpiperazine-1-carboxylate were used as raw materials with the preparation method referring to **intermediate 1C** and **example 3.**

MS (ESI) M/Z: 480.0 [M+H]+.

$^1$H NMR (400 MHz, DMSO-$d_6$) δ 10.96 (brs, 1H), 8.72 (s, 2H), 7.89 (d, *J* = 2.0 Hz, 1H), 7.59 (d, *J* = 2.0 Hz, 1H), 4.56 - 4.44 (m, 2H), 4.25 (brs, 1H), 4.16 (t, *J* = 6.2 Hz, 2H), 3.84 (d, *J* = 10.1 Hz, 1H), 3.26 (d, *J* = 3.9 Hz, 1H), 3.17 - 3.03 (m, 2H), 2.73 (t, *J* = 6.2 Hz, 2H), 0.99 (d, *J* = 6.7 Hz, 3H).

**Example 109**

**2-(6-Oxo-5-(trifluoromethyl)-1,6-dihydropyridin-3-yl)ethyl (R)-2-methyl-4-(5-(trifluoromethyl)pyrimidin-2-yl)piperazine-1-carboxylate**

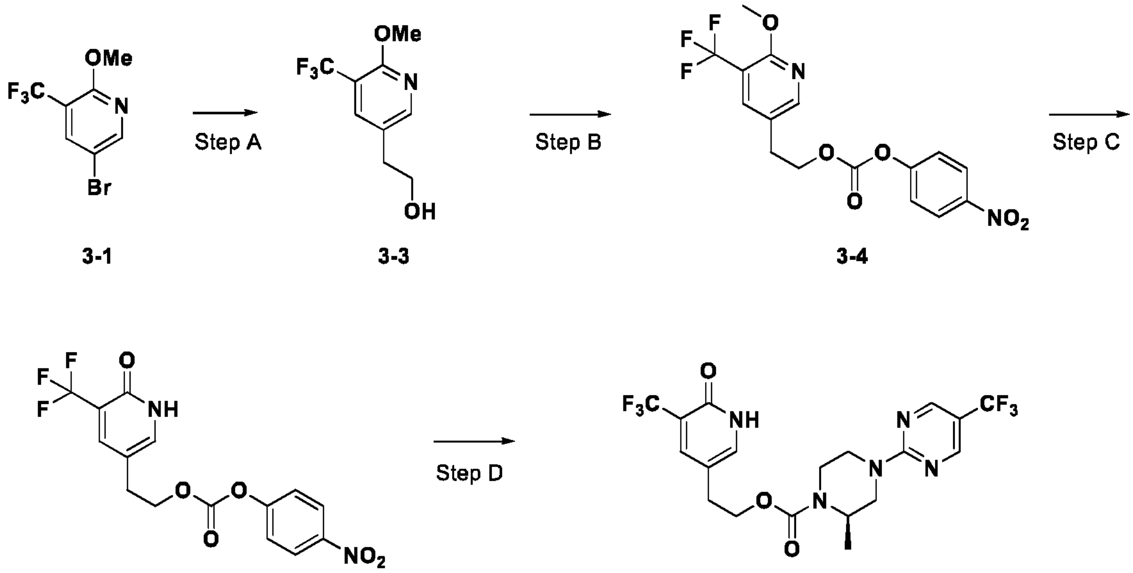


3-1 3-3 3-4

109-2 example 109

**Step A:** 5-Bromo-2-methoxy-3-(trifluoromethyl)pyridine (30 g, 117 mmol) was dissolved in anhydrous tetrahydrofuran (500 mL). A 1.6 *M* solution of *n*-butyllithium in tetrahydrofuran (73 mL, 117.3 mmol) was then added thereto under cooling in a dry ice/acetone bath. The reaction mixture was cooled in a dry ice/acetone bath, stirred and reacted for 1 hour, and then sequentially added with ethylene oxide solution (3 mol/L tetrahydrofuran solution, 26 mL, 78.1 mmol) and 47% boron trifluoride diethyl etherate solution (30 g, 101.5 mmol). The reaction mixture was stirred and reacted for another 3 hours under cooling in a dry ice/acetone bath. The reaction mixture was added with water (200 mL) to quench, and extracted with ethyl acetate (200 mL). The organic phases were separated and combined, washed with saturated brine (200 mL × 1), then dried over anhydrous sodium sulfate, filtered, and finally concentrated under reduced pressure. The resulting residue was purified by silica gel column chromatography to obtain 12.5 g of 2-(6-methoxy-5-(trifluoromethyl)pyridin-3-yl)ethan-1-ol (**3-3**).

MS (ESI) M/Z: 222.0 [M+H]$^+$.

**Step C:** 2-(6-Methoxy-5-(trifluoromethyl)pyridin-3-yl)ethyl (4-nitrophenyl) carbonate (6 g, 15.5 mmol) was dissolved in acetonitrile (60 mL). 55% hydroiodic acid aqueous solution (6 mL) was then added to the above solution. The reaction mixture was stirred at room temperature overnight, added with sodium bicarbonate aqueous solution (50 mL) to quench under cooling in an ice-water bath, and the pH was adjusted to 8. The mixture was added with saturated sodium thiosulfate solution (50 mL), filtered, and the resulting solid was washed with water and ethanol, respectively, to obtain 5 g of a product of 4-nitrophenyl (2-(6-oxo-5-(trifluoromethyl)-1,6-dihydropyridin-3-yl)ethyl) carbonate (**109-4**).

MS (ESI) M/Z: 373.1 [M+H]$^+$.

**Step D:** 4-Nitrophenyl (2-(6-oxo-5-(trifluoromethyl)-1,6-dihydropyridin-3-yl)ethyl) carbonate and *tert*-butyl (*R*)-2-methylpiperazine-1-carboxylate were used as raw materials with the preparation method referring to **intermediate 1C** and **example 48**.

MS (ESI) M/Z: 480.0 [M+H]$^+$.

$^1$H NMR (400 MHz, DMSO-$d_6$) δ 12.21 (s, 1H), 8.71 (s, 2H), 7.89 (s, 1H), 7.58 (s, 1H), 4.57 - 4.40 (m, 2H), 4.25 (brs, 1H), 4.16 (t, *J* = 6.2 Hz, 2H), 3.91 - 3.75 (m, 1H), 3.27 (dd, *J* = 13.4, 3.9 Hz, 1H), 3.18 - 3.02 (m, 2H), 2.73 (t, *J* = 6.2 Hz, 2H), 0.99 (d, *J* = 6.7 Hz, 3H).

## Example 110

**2-(6-Oxo-5-(trifluoromethyl)-1,6-dihydropyridin-3-yl)ethyl (*S*)-2-(hydroxymethyl)-4-(5-(trifluoromethyl)pyrimidin-2-yl)piperazine-1-carboxylate**

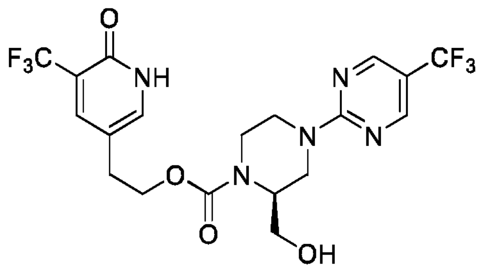

5-Bromo-2-methoxy-3-(trifluoromethyl)pyridine and tert-butyl (*S*)-2-(hydroxymethyl)piperazine-1-carboxylate were used as raw materials with the preparation method referring to **intermediate 1C** and **example 109**.

MS (ESI) M/Z: 496.0 [M+H]$^+$.

$^1$H NMR (400 MHz, DMSO-$d_6$) δ 12.11 (s, 1H), 8.72 (d, *J* = 1.0 Hz, 2H), 7.90 (d, *J* = 2.6 Hz, 1H), 7.59 (d, *J* = 2.5 Hz, 1H), 4.78 (brs, 1H), 4.67 (d, *J* = 13.6 Hz, 1H), 4.43 (brs, 1H), 4.16 (t, *J* = 6.3 Hz, 2H), 4.07 (brs, 1H), 3.86 (d, *J* = 10.6 Hz, 1H), 3.39 - 3.33 (m, 2H), 3.26 (dd, *J* = 13.6, 4.3 Hz, 1H), 3.18 - 3.06 (m, 2H), 2.72 (t, *J* = 6.3 Hz, 2H).

## Example 111

**2-(6-Oxo-5-(trifluoromethyl)-1,6-dihydropyridin-3-yl)ethyl (*S*)-3-(hydroxymethyl)-4-(5-(trifluoromethyl)pyrimidin-2-yl)piperazine-1-carboxylate**

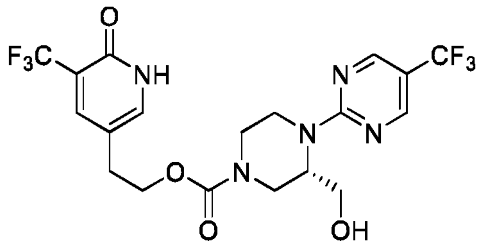

5-Bromo-2-methoxy-3-(trifluoromethyl)pyridine and *tert*-butyl (*S*)-3-(hydroxymethyl)piperazine-1-carboxylate were used as raw materials with the preparation method referring to **intermediate 1C** and **example 109**.
MS (ESI) M/Z: 496.0 $[M+H]^+$.
$^1H$ NMR (400 MHz, DMSO-$d_6$) δ 12.15 (s, 1H), 8.72 (d, J= 0.5 Hz, 2H), 7.90 (d, *J* = 2.5 Hz, 1H), 7.59 (d, *J* = 2.5 Hz, 1H), 4.85 (s, 1H), 4.76 (s, 1H), 4.50 (d, *J* = 13.5 Hz, 1H), 4.15 (t, *J* = 6.4 Hz, 2H), 4.08 (d, *J* = 13.4 Hz, 1H), 3.88 (brs, 1H), 3.54 - 3.39 (m, 2H), 3.24 - 3.14 (m, 1H), 3.10 (d, *J* = 13.0 Hz, 1H), 2.99 (brs, 1H), 2.72 (t, *J* = 6.4 Hz, 2H).

**Example 112**

**2-(6-Oxo-5-(trifluoromethyl)-1,6-dihydropyridin-3-yl)ethyl (*R*)-3-methyl-4-(5-(trifluoromethyl)pyrimidin-2-yl)piperazine-1-carboxylate**

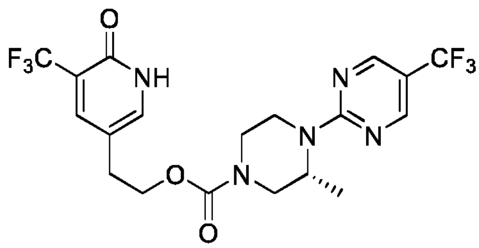

5-Bromo-2-methoxy-3-(trifluoromethyl)pyridine and tert-butyl (R)-3-methylpiperazine-1-carboxylate were used as raw materials with the preparation method referring to **intermediate 1C** and **example 109**.
MS (ESI) M/Z: 480.2 $[M+H]^+$.
$^1H$ NMR (400 MHz, DMSO-$d_6$) δ 12.11 (brs, 1H), 8.74 (s, 2H), 7.90 (d, *J* = 2.6 Hz, 1H), 7.60 (d, *J* = 2.6 Hz, 1H), 4.87 (s, 1H), 4.47 (d, *J* = 13.8 Hz, 1H), 4.16 (s, 2H), 3.95 (s, 1H), 3.80 (s, 1H), 3.25 - 3.07 (m, 2H), 2.97 (brs, 1H), 2.73 (t, *J* = 6.2 Hz, 2H), 1.06 (d, *J* = 6.4 Hz, 3H).

**Example 113**

**2-(6-Oxo-5-(trifluoromethyl)-1,6-dihydropyridin-3-yl)ethyl (*S*)-3-methyl-4-(5-(trifluoromethyl)pyrimidin-2-yl)piperazine-1-carboxylate**

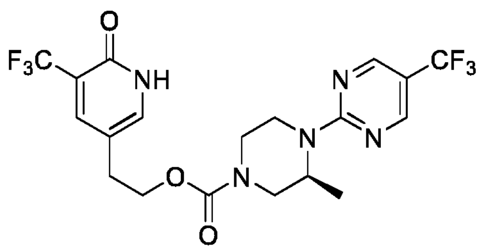

5-Bromo-2-methoxy-3-(trifluoromethyl)pyridine and *tert*-butyl (*S*)-3-methylpiperazine-1-carboxylate were used as raw materials with the preparation method referring to **intermediate 1C** and **example 109**.
MS (ESI) M/Z: 480.2 $[M+H]^+$.

$^1H$ NMR (400 MHz, DMSO-$d_6$) δ 12.21 (s, 1H), 8.74 (s, 2H), 7.91 (s, 1H), 7.60 (s, 1H), 4.87 (s, 1H), 4.47 (d, *J* = 13.7 Hz, 1H), 4.17 (brs, 2H), 3.95 (s, 1H), 3.79 (s, 1H), 3.24 - 3.11 (m, 2H), 2.97 (brs, 1H), 2.73 (t, *J* = 6.3 Hz, 2H), 1.06 (d, *J* = 6.7 Hz, 3H).

**Example 114**

**2-(6-Oxo-5-(trifluoromethyl)-1,6-dihydropyridin-3-yl)ethyl 4-(5-(2-hydroxypropan-2-yl)pyrimidin-2-yl)piperazine-1-carboxylate**

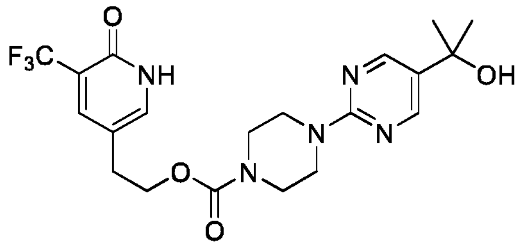

5-Bromo-2-methoxy-3-(trifluoromethyl)pyridine and 2-chloropyrimidine-5-carboxylic acid were used as raw materials with the preparation method referring to **intermediate 1F** and **example 48**.
MS (ESI) M/Z: 454.0 [M-H]$^-$.
$^1H$ NMR (400 MHz, DMSO-$d_6$) δ 8.45 (s, 2H), 7.89 (d, *J* = 2.6 Hz, 1H), 7.59 (d, *J* = 2.6 Hz, 1H), 4.15 (t, *J* = 6.3 Hz, 2H), 3.71 - 3.64 (m, 4H), 3.42 - 3.37 (m, 4H), 2.72 (t, *J* = 6.2 Hz, 2H), 1.41 (s, 6H).

**Example 115**

***N*-Methyl-*N*-(2-(6-oxo-5-(trifluoromethyl)-1,6-dihydropyridin-3-yl)ethyl)-4-(5-(trifluoromethyl)pyrimidin-2-yl)piperazine-1-carboxamide**

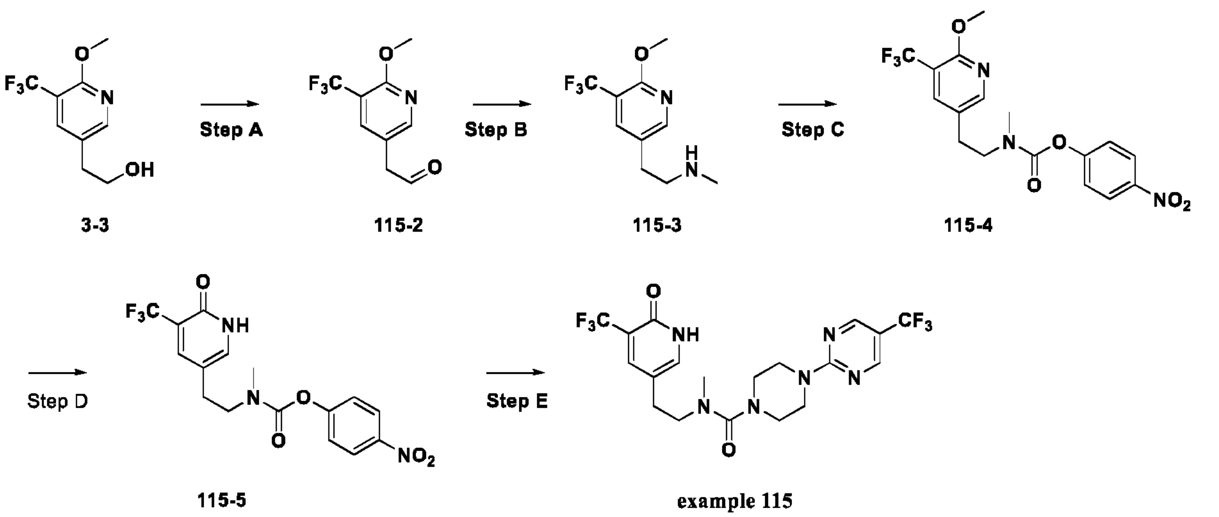

3-3
115-2
115-3
115-4
115-5
example 115

**Step A:** Compound 2-(6-methoxy-5-(trifluoromethyl)pyridin-3-yl)ethan-1-ol (780 mg, 3.53 mmol) was dissolved in dichloromethane (40 mL) at 0°C. Dess-Martin periodinane (1.79 g, 4.23 mmol) was then added thereto. The reaction mixture was stirred at room temperature for 3 hours. The reaction mixture was filtered, and the filter cake was washed with dichloromethane (10 mL × 3). The filtrate was washed with saturated brine (20 mL), then dried over anhydrous sodium sulfate, filtered, and finally concentrated under reduced pressure. The resulting residue was purified by silica gel column chromatography to obtain 620 mg of 2-(6-methoxy-5-(trifluoromethyl)pyridin-3-yl)acetaldehyde (**115-2**).
MS (ESI) M/Z: 220.2 [M+H]$^+$.

**Step B:** Compound 2-(6-methoxy-5-(trifluoromethyl)pyridin-3-yl)acetaldehyde (620 mg, 2.83 mmol) was dissolved in anhydrous methanol (20 mL) at 0°C. Glacial acetic acid (0.2 mL), a solution of methylamine in tetrahydrofuran (2.1 mL, 4.25 mmol), and sodium triacetoxyborohydride (1.2 g, 5.66 mmol) were then added thereto. The reaction mixture was stirred at room temperature for 3 hours. The reaction mixture was added with water (50 mL) to quench, and extracted with ethyl acetate (20 mL × 3). The organic phases were combined, first washed with saturated brine (30 mL), then dried over anhydrous sodium sulfate, filtered, and finally concentrated under reduced pressure. The resulting residue was purified by silica gel column chromatography to obtain 320 mg of 2-(6-methoxy-5-(trifluoromethyl)pyridin-3-yl)-*N*-methylethan-1-amine (**115-3**).

MS (ESI) M/Z: 235.2 $[M+H]^+$.

**Step C:** 2-(6-Methoxy-5-(trifluoromethyl)pyridin-3-yl)-*N*-methylethan-1-amine (320 mg, 1.37 mmol) was dissolved in *N*,*N*-dimethylformamide (5 mL) at room temperature. Bis(4-nitrophenyl) carbonate (833 mg, 2.74 mmol) and *N*,*N*-diisopropylethylamine (354 mg, 2.74 mmol) were then added thereto. The reaction mixture was stirred at room temperature for 2 hours, and added with water (30 mL) to quench. The mixture was extracted with ethyl acetate (5 mL × 3), and the organic phases were combined. The combined organic phases were first washed with saturated brine (30 mL), dried over anhydrous sodium sulfate, filtered, and concentrated. The resulting residue was purified by silica gel column chromatography to obtain 67 mg of 4-nitrophenyl (2-(6-methoxy-5-(trifluoromethyl)pyridin-3-yl)ethyl)(methyl)carbamate (**115-4**).

MS (ESI) M/Z: 400.2 $[M+H]^+$.

**Step D:** 4-Nitrophenyl (2-(6-methoxy-5-(trifluoromethyl)pyridin-3-yl)ethyl)(methyl)carbamate (67 mg, 0.17 mmol) was dissolved in acetonitrile (0.7 mL). Hydroiodic acid (0.14 mL) was then added to the above solution. The reaction mixture was stirred at room temperature overnight. The reaction mixture was added with sodium bicarbonate aqueous solution (20 mL) to quench in an ice bath, and the pH was adjusted to 8. The mixture was washed three times with dichloromethane (5 mL), and added with saturated sodium thiosulfate solution (20 mL). The organic phase was washed with saturated brine (20 mL), dried over anhydrous sodium sulfate, filtered, and concentrated. The resulting residue was purified by silica gel column chromatography to obtain 20 mg of 4-nitrophenyl methyl(2-(6-oxo-5-(trifluoromethyl)-1,6-dihydropyridin-3-yl)ethyl)carbamate (**115-5**).

MS (ESI) M/Z: 386.1 $[M+H]^+$.

**Step E:** 4-Nitrophenyl methyl(2-(6-oxo-5-(trifluoromethyl)-1,6-dihydropyridin-3-yl)ethyl)carbamate and 2-(piperazin-1-yl)-5-(trifluoromethyl)pyrimidine were used as raw materials with the preparation method referring to **example 48**.

MS (ESI) M/Z: 479.0 $[M+H]^+$.

$^1H$ NMR (400 MHz, DMSO-$d_6$) δ 12.16 (s, 1H), 8.71 (d, *J* = 0.6 Hz, 2H), 7.87 (d, *J* = 2.0 Hz, 1H), 7.55 (d, *J* = 1.9 Hz, 1H), 3.82 - 3.75 (m, 4H), 3.38 - 3.33 (m, 2H), 3.12 - 3.07 (m, 4H), 2.81 (s, 3H), 2.62 (t, *J* = 6.7 Hz, 2H).

## Example 116

**(*S*)-1-(6-oxo-5-(trifluoromethyl)-1,6-dihydropyridin-3-yl)butan-2-yl 4-(5-(trifluoromethyl)pyrimidin-2-yl)piperazine-1-carboxylate**

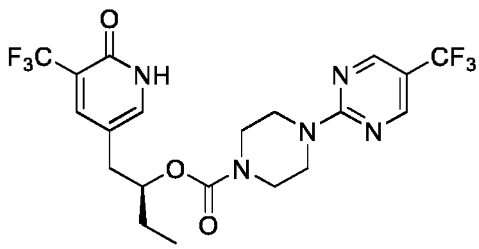

5-Bromo-2-methoxy-3-(trifluoromethyl)pyridine and (*S*)-(-)-1,2-epoxybutane were used as raw materials with the preparation method referring to **intermediate 1C** and **example 48**.

MS (ESI) M/Z: 494.2 $[M+H]^+$.

$^1H$ NMR (400 MHz, DMSO-$d_6$) δ 12.20 (s, 1H), 8.74 (s, 2H), 7.83 (d, *J* = 2.5 Hz, 1H), 7.54 (d, *J* = 2.5 Hz, 1H), 4.80 - 4.70 (m, 1H), 3.90 (brs, 2H), 3.68 (brs, 2H), 3.44 (brs, 4H), 2.74 (dd, *J* = 14.3, 4.0 Hz, 1H), 2.59 (dd, *J* = 14.3, 8.3 Hz, 1H), 1.64 - 1.46 (m, 2H), 0.88 (t, *J* = 7.4 Hz, 3H).

**Example 117**

**(*R*)-1-Hydroxy-3-(6-oxo-5-(trifluoromethyl)-1,6-dihydropyridin-3-yl)propan-2-yl 4-(5-(trifluoromethyl)pyrimidin-2-yl)piperazine-1-carboxylate**

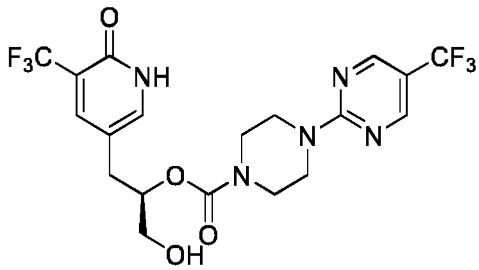

5-Bromo-2-methoxy-3-(trifluoromethyl)pyridine and (*R*)-*tert*-butyldimethyl(oxiran-2-ylmethoxy)silane were used as raw materials with the preparation method referring to **intermediate 1C** and **example 109**.

MS (ESI) M/Z: 496.20 $[M+H]^+$.

$^1$H NMR (400 MHz, DMSO-$d_6$) $\delta$ 12.13 (brs, 1H), 8.74 (s, 2H), 7.83 (d, *J* = 2.4 Hz, 1H), 7.53 (d, *J* = 2.3 Hz, 1H), 4.90 (t, *J* = 5.7 Hz, 1H), 4.80 - 4.71 (m, 1H), 3.88 (brs, 2H), 3.69 (brs, 2H), 3.51 - 3.37 (m, 6H), 2.75 (dd, *J* = 14.2, 4.5 Hz, 1H), 2.64 (dd, *J* = 14.3, 8.1 Hz, 1H).

**Example 118**

**2-(6-Oxo-5-(trifluoromethyl)-1,6-dihydropyridin-3-yl)ethyl 4-(5-cyclohexylpyrimidin-2-yl)piperazine-1-carboxylate**

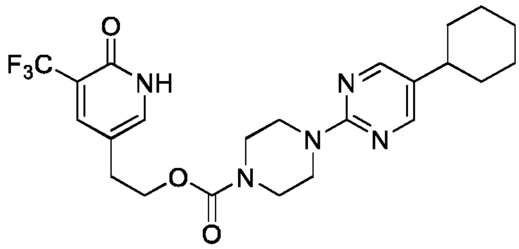

5-Bromo-2-methoxy-3-(trifluoromethyl)pyridine and intermediate **1I** were used as raw materials with the preparation method referring to **example 109**.

MS (ESI) M/Z: 480.2 $[M+H]^+$.

$^1$H NMR (400 MHz, DMSO-$d_6$) $\delta$ 12.19 (brs, 1H), 8.27 (s, 2H), 7.90 (d, *J* = 1.9 Hz, 1H), 7.58 (d, *J* = 1.6 Hz, 1H), 4.15 (t, *J* = 6.3 Hz, 2H), 3.73 - 3.58 (m, 4H), 3.49 - 3.36 (m, 4H), 2.72 (t, *J* = 6.3 Hz, 2H), 2.41 - 2.30 (m, 1H), 1.80 - 1.64 (m, 5H), 1.44 - 1.21 (m, 5H).

**Example 119**

**2-(6-Oxo-5-(trifluoromethyl)-1,6-dihydropyridin-3-yl)ethyl 4-(5-cyclopentylpyrimidin-2-yl)piperazine-1-carboxylate**

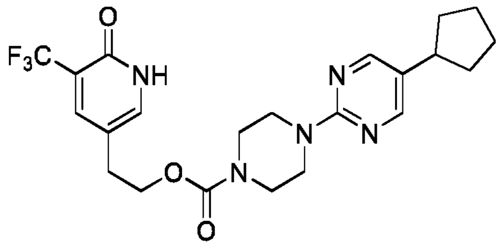

5-Bromo-2-methoxy-3-(trifluoromethyl)pyridine and cyclopenten-1-ylboronic acid were used as raw materials with the preparation method referring to **intermediate 1I** and **example 109**.

MS (ESI) M/Z: 466.2 $[M+H]^+$.

$^1H$ NMR (400 MHz, DMSO-$d_6$) δ 12.17 (s, 1H), 8.30 (s, 2H), 7.90 (d, *J* = 2.6 Hz, 1H), 7.59 (d, *J* = 2.6 Hz, 1H), 4.15 (t, *J* = 6.3 Hz, 2H), 3.73 - 3.62 (m, 4H), 3.45 - 3.36 (m, 4H), 2.88 - 2.77 (m, 1H), 2.72 (t, *J* = 6.3 Hz, 2H), 2.03 - 1.91 (m, 2H), 1.80 - 1.69 (m, 2H), 1.67 - 1.58 (m, 2H), 1.54 - 1.42 (m, 2H).

**Example 120**

**(*S*)-1-(6-oxo-5-(trifluoromethyl)-1,6-dihydropyridin-3-yl)propan-2-yl 4-(5-acetylpyrimidin-2-yl)piperazine-1-carboxylate**

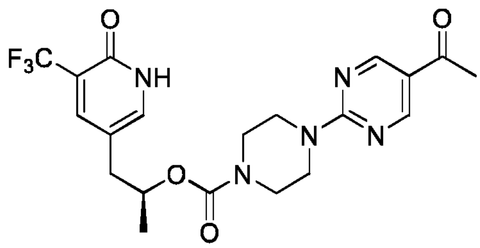

5-Bromo-2-methoxy-3-(trifluoromethyl)pyridine and intermediate **1J** were used as raw materials with the preparation method referring to **example 48**.

MS (ESI) M/Z: 454.0 $[M+H]^+$.

$^1H$ NMR (400 MHz, DMSO-$d_6$) δ 12.19 (s, 1H), 8.88 (s, 2H), 7.86 (d, *J* = 2.5 Hz, 1H), 7.56 (d, *J* = 2.6 Hz, 1H), 4.89 - 4.79 (m, 1H), 3.94 (brs, 2H), 3.73 (brs, 2H), 3.44 (brs, 4H), 2.72 (dd, *J* = 14.2, 4.3 Hz, 1H), 2.61 (dd, *J* = 14.2, 7.9 Hz, 1H), 2.46 (s, 3H), 1.20 (d, *J* = 6.3 Hz, 3H).

**Example 121**

**2-(6-Oxo-5-(trifluoromethyl)-1,6-dihydropyridin-3-yl)ethyl (*S*)-2-ethyl-4-(5-(trifluoromethyl)pyrimidin-2-yl)piperazine-1-carboxylate**

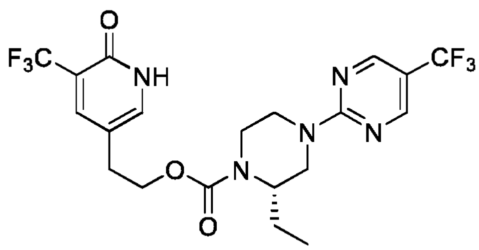

5-Bromo-2-methoxy-3-(trifluoromethyl)pyridine and *tert*-butyl (*S*)-2-ethylpiperazine-1-carboxylate were used as raw materials with the preparation method referring to **intermediate 1C** and **example 109**.

MS (ESI) M/Z: 494.2 $[M+H]^+$.

$^1H$ NMR (400 MHz, DMSO-$d_6$) δ 12.22 (s, 1H), 8.71 (s, 2H), 7.90 (s, 1H), 7.59 (s, 1H), 4.67 - 4.52 (m, 2H), 4.18 (t, *J* = 6.3 Hz, 2H), 4.03 (brs, 1H), 3.89 (d, *J* = 10.1 Hz, 1H), 3.17 (dd, *J* = 13.5, 3.9 Hz, 1H), 3.07 - 2.94 (m, 2H), 2.73 (t, *J* = 6.2 Hz, 2H), 1.45 - 1.34 (m, 2H), 0.73 (t, *J* = 7.5 Hz, 3H).

**Example 122**

**2-(6-Oxo-5-(trifluoromethyl)-1,6-dihydropyridin-3-yl)ethyl (*R*)-2-ethyl-4-(5-(trifluoromethyl)pyrimidin-2-yl)piperazine-1-carboxylate**

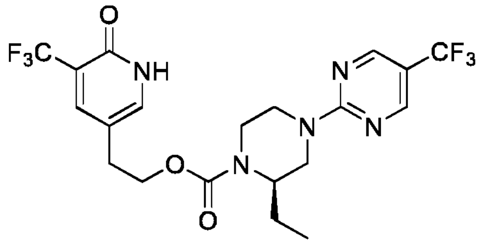

5-Bromo-2-methoxy-3-(trifluoromethyl)pyridine and *tert*-butyl (*R*)-2-ethylpiperazine-1-carboxylate were used as raw materials with the preparation method referring to **intermediate 1C** and **example 109**.

MS (ESI) M/Z: 494.2 $[M+H]^+$.

$^1H$ NMR (400 MHz, DMSO-$d_6$) δ 12.20 (s, 1H), 8.71 (s, 2H), 7.90 (d, *J* = 2.5 Hz, 1H), 7.59 (d, *J* = 2.6 Hz, 1H), 4.66 - 4.52 (m, 2H), 4.18 (t, *J* = 6.2 Hz, 2H), 4.03 (brs, 1H), 3.89 (d, *J* = 10.0 Hz, 1H), 3.17 (dd, *J* = 13.5, 3.9 Hz, 1H), 3.09 - 2.90 (m, 2H), 2.73 (t, *J* = 6.2 Hz, 2H), 1.45 - 1.33 (m, 2H), 0.73 (t, *J* = 7.4 Hz, 3H).

**Example 123**

**2-(6-Oxo-5-(trifluoromethyl)-1,6-dihydropyridin-3-yl)ethyl (*R*)-3-ethyl-4-(5-(trifluoromethyl)pyrimidin-2-yl)piperazine-1-carboxylate**

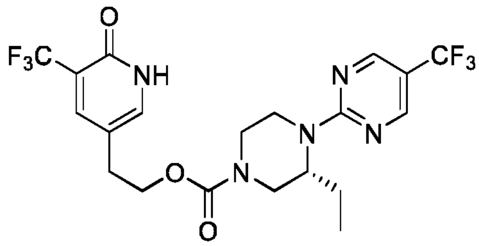

5-Bromo-2-methoxy-3-(trifluoromethyl)pyridine and tert-butyl (R)-3-ethylpiperazine-1-carboxylate were used as raw materials with the preparation method referring to **intermediate 1C** and **example 109**.

MS (ESI) M/Z: 494.0 $[M+H]^+$.

$^1H$ NMR (400 MHz, DMSO-$d_6$) δ 12.22 (s, 1H), 8.72 (s, 2H), 7.91 (d, *J* = 2.6 Hz, 1H), 7.59 (brs, 1H), 4.73 (brs, 1H), 4.62 - 4.48 (m, 1H), 4.32 - 4.06 (m, 2H), 4.03 - 3.80 (m, 2H), 3.16 - 2.78 (m, 3H), 2.73 (t, *J* = 6.4 Hz, 2H), 1.57 - 1.41 (m, 2H), 0.75 (brs, 3H).

**Example 124**

**2-(6-Oxo-5-(trifluoromethyl)-1,6-dihydropyridin-3-yl)ethyl (*S*)-3-ethyl-4-(5-(trifluoromethyl)pyrimidin-2-yl)piperazine-1-carboxylate**

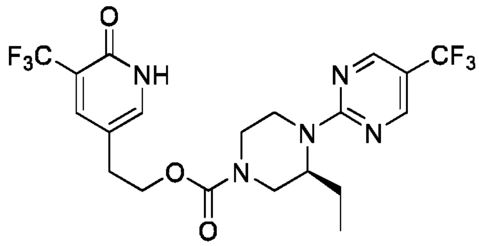

5-Bromo-2-methoxy-3-(trifluoromethyl)pyridine and tert-butyl (*S*)-3-ethylpiperazine-1-carboxylate were used as raw materials with the preparation method referring to **intermediate 1C** and **example 109**.
MS (ESI) M/Z: 494.2 $[M+H]^+$.
$^1H$ NMR (400 MHz, DMSO-$d_6$) $\delta$ 12.22 (s, 1H), 8.72 (s, 2H), 7.91 (d, *J* = 2.5 Hz, 1H), 7.59 (s, 1H), 4.73 (brs, 1H), 4.62 - 4.48 (m, 1H), 4.32 - 4.06 (m, 2H), 4.02 - 3.81 (m, 2H), 3.16 - 2.80 (m, 3H), 2.73 (t, *J* = 6.4 Hz, 2H), 1.54 - 1.43 (m, 2H), 0.75 (brs, 3H).

**Example 125**

**2-(6-Oxo-5-(trifluoromethyl)-1,6-dihydropyridin-3-yl)ethyl 8-(5-(trifluoromethyl)pyrimidin-2-yl)-3,8-diazabicyclo[3.2.1]octane-3-carboxylate**

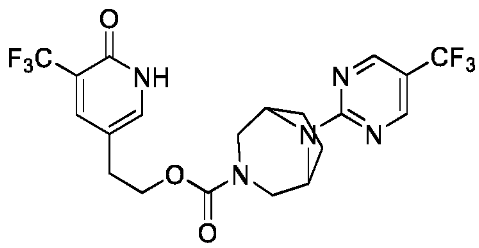

5-Bromo-2-methoxy-3-(trifluoromethyl)pyridine and tert-butyl 3,8-diazabicyclo[3.2.1]octane-3-carboxylate were used as raw materials with the preparation method referring to **intermediate 1C** and **example 109**.
MS (ESI) M/Z: 492.0 $[M+H]^+$.
$^1H$ NMR (400 MHz, DMSO-$d_6$) $\delta$ 8.73 (d, *J* = 0.7 Hz, 2H), 7.87 (d, *J* = 2.1 Hz, 1H), 7.59 (d, *J* = 2.2 Hz, 1H), 6.49 (brs, 1H), 4.76 (d, *J* = 15.5 Hz, 2H), 4.24 - 4.07 (m, 2H), 3.82 - 3.70 (m, 2H), 3.08 (d, *J* = 12.2 Hz, 1H), 2.98 (d, *J* = 12.7 Hz, 1H), 2.71 (t, *J* = 6.3 Hz, 2H), 1.96 - 1.86 (m, 2H), 1.70 - 1.58 (m, 2H).

**Example 126**

**2-(6-Oxo-5-(trifluoromethyl)-1,6-dihydropyridin-3-yl)ethyl 3-(5-(trifluoromethyl)pyrimidin-2-yl)-3,8-diazabicyclo[3.2.1]octane-8-carboxylate**

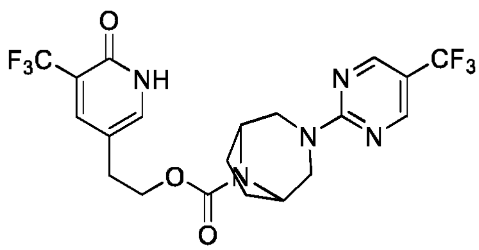

5-Bromo-2-methoxy-3-(trifluoromethyl)pyridine and *tert*-butyl 3,8-diazabicyclo[3.2.1]octane-8-carboxylate were used as raw materials with the preparation method referring to **intermediate 1C** and **example 109.**
MS (ESI) M/Z: 492.2 $[M+H]^+$.

$^1H$ NMR (400 MHz, DMSO-$d_6$) δ 12.18 (s, 1H), 8.72 (s, 2H), 7.90 (s, 1H), 7.59 (s, 1H), 4.45 (d, *J* = 12.5 Hz, 2H), 4.29 (brs, 2H), 4.18 (t, *J* = 6.2 Hz, 2H), 3.04 (d, *J* = 12.9 Hz, 2H), 2.74 (t, *J* = 6.2 Hz, 2H), 1.87 - 1.75 (m, 2H), 1.54 (q, *J* = 7.1 Hz, 2H).

**Example 127**

**2-(6-Oxo-5-(trifluoromethyl)-1,6-dihydropyridin-3-yl)ethyl 4-(5-(trifluoromethyl)pyrazin-2-yl)piperazine-1-carboxylate**

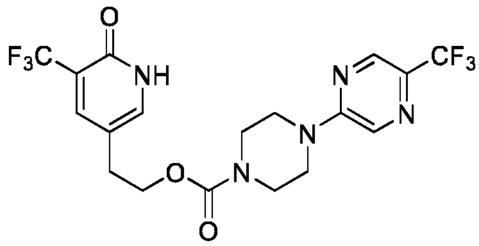

5-Bromo-2-methoxy-3-(trifluoromethyl)pyridine and 2-chloro-5-(trifluoromethyl)pyrazine were used as raw materials with the preparation method referring to **intermediate 1C** and **example 48**.
MS (ESI) M/Z: 466.0 [M+H]$^+$.
$^1H$ NMR (400 MHz, $CD_3OD$) δ 8.40 (s, 1H), 8.29 (d, *J* = 1.4 Hz, 1H), 7.97 (d, *J* = 2.6 Hz, 1H), 7.56 (d, *J* = 2.6 Hz, 1H), 4.28 (t, *J* = 6.3 Hz, 2H), 3.78 - 3.73 (m, 4H), 3.61 - 3.56 (m, 4H), 2.83 (t, *J* = 6.3 Hz, 2H).

**Example 128**

**2-(6-Oxo-5-(trifluoromethyl)-1,6-dihydropyridin-3-yl)ethyl (*R*)-2-(cyanomethyl)-4-(5-(trifluoromethyl)pyrimidin-2-yl)piperazine-1-carboxylate**

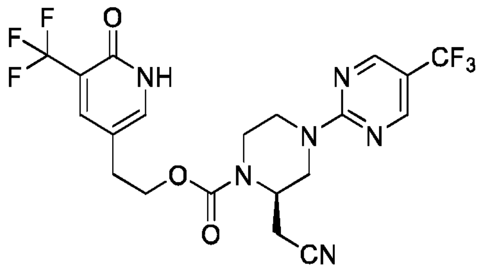

5-Bromo-2-methoxy-3-(trifluoromethyl)pyridine and intermediate **1K** were used as raw materials with the preparation method referring to **example 48**.
MS (ESI) M/Z: 505.2 [M+H]$^+$.
$^1H$ NMR (400 MHz, $CD_3OD$) δ 8.60 (d, *J* = 0.8 Hz, 2H), 8.01 - 7.96 (m, 1H), 7.58 (d, *J* = 2.5 Hz, 1H), 4.83 - 4.72 (m, 2H), 4.68 - 4.62 (m, 1H), 4.38 - 4.25 (m, 2H), 4.04 (brs, 1H), 3.39 - 3.32 (m, 1H), 3.26 - 3.16 (m, 1H), 3.14 - 3.05 (m, 1H), 2.88 - 2.83 (m, 2H), 2.82 - 2.69 (m, 2H).

**Example 129**

**2-(6-Oxo-5-(trifluoromethyl)-1,6-dihydropyridin-3-yl)ethyl 2-(methoxymethyl)-4-(5-(trifluoromethyl)pyrimidin-2-yl)piperazine-1-carboxylate**

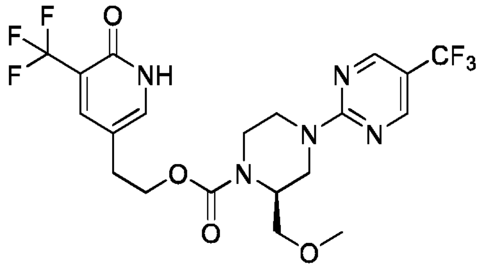

5-Bromo-2-methoxy-3-(trifluoromethyl)pyridine and intermediate **1L** were used as raw materials with the preparation method referring to **example 48**.

MS (ESI) M/Z: 510.2 [M+H]+.

$^1$H NMR (400 MHz, DMSO-$d_6$) δ 8.72 (s, 2H), 7.91 (d, *J* = 2.6 Hz, 1H), 7.58 (d, *J* = 2.5 Hz, 1H), 4.65 (d, *J* = 13.6 Hz, 1H), 4.50 (d, *J* = 7.3 Hz, 1H), 4.26 - 4.13 (m, 3H), 3.87 (d, *J* = 10.0 Hz, 1H), 3.29 - 3.21 (m, 3H), 3.14 (brs, 3H), 3.09 (d, *J* = 9.5 Hz, 2H), 2.74 (t, *J* = 6.1 Hz, 2H).

**Example 130**

**2-(6-Oxo-5-(trifluoromethyl)-1,6-dihydropyridin-3-yl)ethyl 4-(3-fluoro-5-(trifluoromethyl)pyridin-2-yl)piperazine-1-carboxylate**

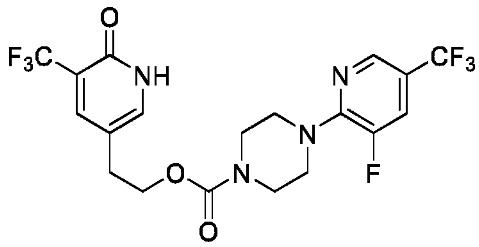

5-Bromo-2-methoxy-3-(trifluoromethyl)pyridine and 2-chloro-3-fluoro-5-(trifluoromethyl)pyridine were used as raw materials with the preparation method referring to **intermediate 1C** and **example 48**.

MS (ESI) M/Z: 483.2 [M+H]+.

$^1$H NMR (400 MHz, DMSO-$d_6$) δ 7.48 (d, *J* = 2.2 Hz, 1H), 7.18 (d, *J* = 2.7 Hz, 1H), 6.87 (dd, *J* = 13.4, 2.2 Hz, 1H), 6.77 (d, *J* = 2.7 Hz, 1H), 3.51 - 3.44 (m, 2H), 2.88 - 2.82 (m, 4H), 2.80 - 2.75 (m, 4H), 2.07 - 2.02 (m, 2H).

**Example 131**

**4-(3-Amino-5-(trifluoromethyl)pyridin-2-yl)-*N*-(2-(6-oxo-5-(trifluoromethyl)-1,6-dihydropyridin-3-yl)ethyl)piperazine-1-carboxamide**

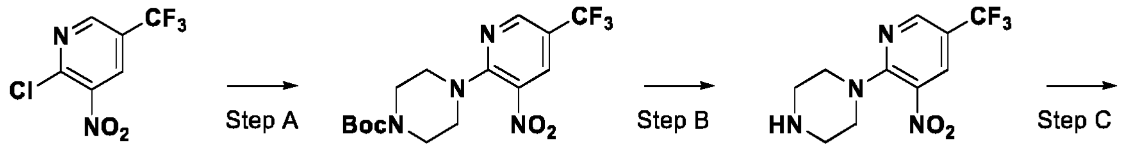


131-1 131-2 131-3

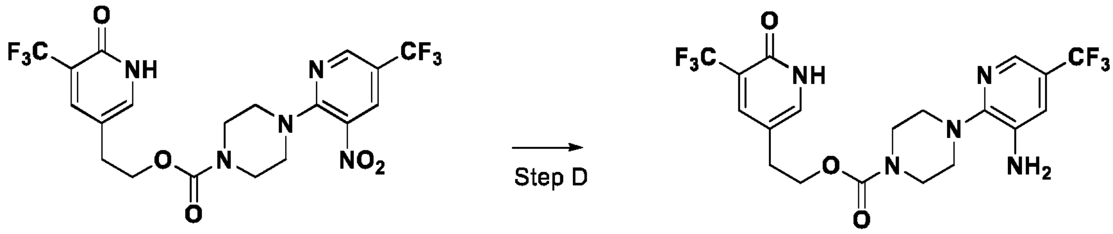


131-4 example 131

**Steps A to C:** 5-Bromo-2-methoxy-3-(trifluoromethyl)pyridine and 2-chloro-3-nitro-5-(trifluoromethyl)pyridine were used as raw materials with the preparation method referring to **intermediate 1C** and **example 48**.

MS (ESI) M/Z: 510.1 $[M+H]^+$.

$^1H$ NMR (400 MHz, DMSO-$d_6$) δ 8.76 (d, *J* = 1.7 Hz, 1H), 8.57 (d, *J* = 1.9 Hz, 1H), 7.90 (d, *J* = 2.1 Hz, 1H), 7.59 (d, *J* = 1.9 Hz, 1H), 4.16 (t, *J* = 6.3 Hz, 2H), 3.50 (brs, 8H), 2.73 (d, *J* = 4.9 Hz, 2H).

**Step D:** 2-(6-Oxo-5-(trifluoromethyl)-1,6-dihydropyridin-3-yl)ethyl 4-(3-nitro-5-(trifluoromethyl)pyridin-2-yl)piperazine-1-carboxylate was dissolved in anhydrous methanol (5 mL) at room temperature, and a palladium on carbon catalyst (20%) was added thereto. The reaction mixture was stirred at room temperature for 5 hours, filtered, and the filter cake was washed with methanol (5 mL × 3). The resulting filtrate was dried over anhydrous sodium sulfate, filtered, and concentrated. The resulting residue was purified by preparative high performance liquid chromatography to obtain 25 mg of 4-(3-amino-5-(trifluoromethyl)pyridin-2-yl)-*N*-(2-(6-oxo-5-(trifluoromethyl)-1,6-dihydropyridin-3-yl)ethyl)piperazine-1-carboxamide (**compound 131**).

MS (ESI) M/Z: 480.2 $[M+H]^+$.

$^1H$ NMR (400 MHz, $CD_3OD$) δ 7.97 (d, *J* = 2.5 Hz, 1H), 7.85 (dd, *J* = 2.2, 1.0 Hz, 1H), 7.56 (d, *J* = 2.5 Hz, 1H), 7.22 (d, *J* = 2.2 Hz, 1H), 4.27 (t, *J* = 6.3 Hz, 2H), 3.62 (t, *J* = 5.1 Hz, 4H), 3.11 (t, *J* = 4.8 Hz, 4H), 2.83 (t, *J* = 6.3 Hz, 2H).

**Example 132**

**2-(6-Oxo-5-(trifluoromethyl)-1,6-dihydropyridin-3-yl)ethyl 4-(3-methyl-5-(trifluoromethyl)pyridin-2-yl)piperazine-1-carboxylate**

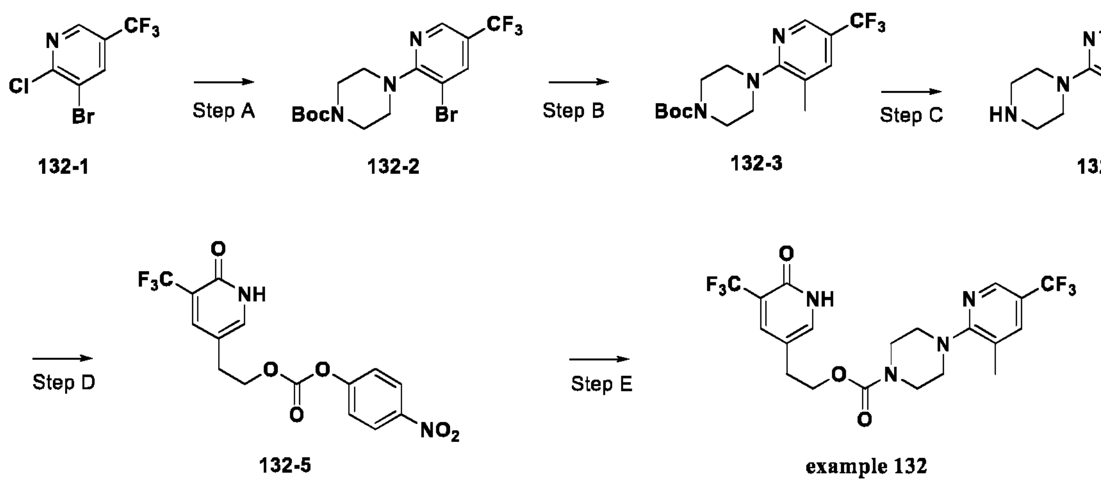


132-1 132-2 132-3 132-4

132-5 example 132

**Step A:** *tert*-Butyl piperazine-1-carboxylate and 3-bromo-2-chloro-5-(trifluoromethyl)pyridine were used as raw materials with the preparation method referring to **1C-2.**

$^1$H NMR (400 MHz, DMSO-$d_6$) δ 8.71 - 8.57 (m, 1H), 8.35 (d, *J*= 1.6 Hz, 1H), 3.51 - 3.46 (m, 4H), 3.40 - 3.36 (m, 4H), 1.42 (s, 9H).

**Step B:** *tert*-Butyl 4-(3-bromo-5-(trifluoromethyl)pyridin-2-yl)piperazine-1-carboxylate (100 mg, 0.24 mmol), trimethylboroxine (0.21 mL, 0.73 mmol), [1,1'-bis(diphenylphosphino)ferrocene]palladium(II) dichloride (17.6 mg, 0.024 mmol), and sodium carbonate (51 mg, 0.48 mmol) were dissolved in 1,4-dioxane (1 mL) and water (0.1 mL) at room temperature. The reaction mixture was replaced with nitrogen, and then heated to 100°C in a sealed tube and stirred for 16 hours. The reaction mixture was concentrated under reduced pressure, and the resulting residue was purified by silica gel column chromatography to obtain 80 mg of tert-butyl 4-(3-methyl-5-(trifluoromethyl)pyridin-2-yl)piperazine-1-carboxylate (**132-3**).

$^1$H NMR (400 MHz, DMSO-$d_6$) δ 8.43 (s, 1H), 7.85 (s, 1H), 3.49 - 3.44 (m, 4H), 3.21 - 3.15 (m, 4H), 2.31(s, 3H), 1.42 (s, 9H).

**Step C:** *tert*-Butyl 4-(3-methyl-5-(trifluoromethyl)pyridin-2-yl)piperazine-1-carboxylate (80 mg, 0.23 mmol) was dissolved in 1,4-dioxane (1 mL) at room temperature. A solution of hydrochloric acid in dioxane (1 mL) was then added to the above solution. The reaction mixture was stirred at room temperature for 2 hours. The reaction mixture was concentrated under reduced pressure to obtain 57 mg of 1-(3-methyl-5-(trifluoromethyl)pyridin-2-yl)piperazine (**132-4**).

MS (ESI) M/Z: 246.1 [M+H]$^+$.

**Steps D to E:** 5-Bromo-2-methoxy-3-(trifluoromethyl)pyridine and 1-(3-methyl-5-(trifluoromethyl)pyridin-2-yl)piperazine were used as raw materials with the preparation method referring to **example 48**.

MS (ESI) M/Z: 479.0 [M+H]$^+$.

$^1$H NMR (400 MHz, $CD_3OD$) δ 8.39 - 8.31 (m, 1H), 7.97 (d, *J* = 2.5 Hz, 1H), 7.75 (d, *J* = 2.4 Hz, 1H), 7.56 (d, *J* = 2.5 Hz, 1H), 4.27 (t, *J* = 6.3 Hz, 2H), 3.60 (t, *J* = 5.1 Hz, 4H), 3.22 (t, *J* = 5.2 Hz, 4H), 2.83 (t, *J* = 6.3 Hz, 2H), 2.36 (s, 3H).

**Example 133**

**(*S*)-1-(2-Methyl-6-oxo-5-(trifluoromethyl)-1,6-dihydropyridin-3-yl)propan-2-yl 4-(5-(trifluoromethyl)pyrimidin-2-yl)piperazine-1carboxylate**

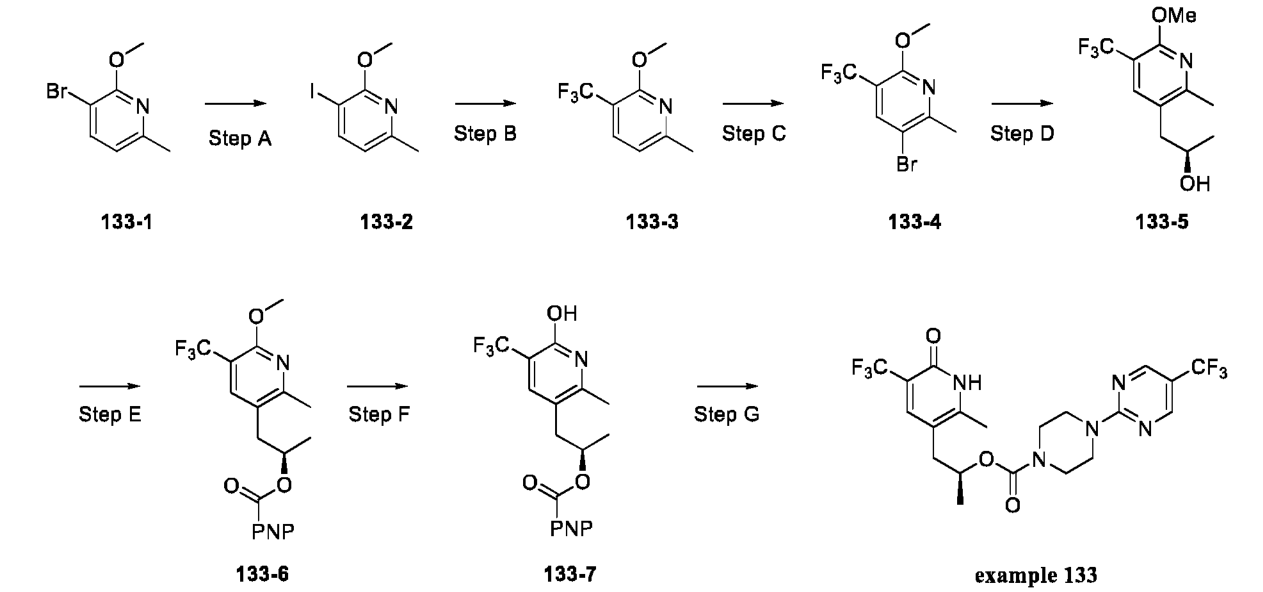


133-1 133-2 133-3 133-4 133-5

133-6 133-7 example 133

**Step A:** 3-Bromo-2-methoxy-6-methylpyridine (3.03 g, 15 mmol) was dissolved in tetrahydrofuran (30 mL). The reaction mixture was cooled to -78°C, and slowly added dropwise with n-butyllithium (14 mL, 1.6 N, 22.4 mmol). After the addition was completed, the reaction mixture was stirred at -78°C for 15 minutes. Iodine (4.57 g, 18 mmol) dissolved in tetrahydrofuran (15 mL) was then slowly added dropwise to the above reaction, and the reaction mixture was reacted for another 1 hour. The reaction system was added with saturated sodium thiosulfate aqueous solution (10 mL), stirred at room temperature for 10 minutes, and extracted with ethyl acetate (50 mL × 3). The organic phases were combined and concentrated, and the residue was purified by silica gel column chromatography to obtain 1.24 g of 3-iodo-2-methoxy-6-methylpyridine (**133-2**).

MS (ESI) M/Z: 249.9 $[M+H]^+$.

**Step B:** 3-Iodo-2-methoxy-6-methylpyridine (2.7 g, 10.84 mmol) was dissolved in DMF (40 mL) under anhydrous nitrogen atmosphere. The reaction system was then added with cupric bromide (240 mg, 1.08 mmol), and slowly added dropwise with methyl 2,2-difluoro-2-(fluorosulfonyl)acetate (5.21 g, 27.1 mmol). After the addition was completed, the reaction mixture was heated to 110°C and reacted for 2 hours. The reaction mixture was cooled to room temperature, added with ethyl acetate (300 mL), and then washed with water (100 mL × 5). The organic phase was concentrated, and the residue was purified by silica gel column chromatography to obtain 1.4 g of 2-methoxy-6-methyl-3-(trifluoromethyl)pyridine (**133-3**).

MS (ESI) M/Z: 192.1 $[M+H]^+$.

**Step C:** 2-Methoxy-6-methyl-3-(trifluoromethyl)pyridine (500 mg, 2.62 mmol) was dissolved in DMF (5 mL). The reaction system was then added with dibromohydantoin (786.6 mg, 2.75 mmol), and reacted at room temperature for 3 hours. The reaction mixture was added with ethyl acetate (100 mL), and then washed with water (100 mL × 5). The organic phase was concentrated, and the residue was purified by silica gel column chromatography to obtain 445 mg of 3-bromo-6-methoxy-2-methyl-5-(trifluoromethyl)pyridine (**133-4**).

**Step D:** 3-Bromo-6-methoxy-2-methyl-5-(trifluoromethyl)pyridine (400 mg, 1.48 mmol) was dissolved in tetrahydrofuran (10 mL). The reaction system was cooled to -78°C, and slowly added dropwise with n-butyllithium (1.6 M in THF, 0.13 mL). After the addition was completed, the reaction mixture was stirred at -78°C for 1 hour, then added with (*S*)-propylene oxide (86 mg, 1.48 mmol) and boron trifluoride diethyl etherate (252.1 mg, 1.78 mmol), and reacted at -78°C for another 1 hour. The reaction system was added with water (10 mL) to quench, and then extracted with ethyl acetate (20 mL × 3). The organic phase was concentrated under reduced pressure, and the resulting residue was purified by silica gel column chromatography to obtain 85 mg of (2*S*)-1(6-methoxy-2-methyl-5-(trifluoromethyl)pyridin-3-yl)propan-2-ol (**133-5**).

MS (ESI) M/Z: 250.0 $[M+H]^+$.

**Steps E to G:** (2*S*)-1(6-Methoxy-2-methyl-5-(trifluoromethyl)pyridin-3-yl)propan-2-ol was used as a raw material with the preparation method referring to **example 48**.

MS (ESI) M/Z: 494.2 $[M+H]^+$.

$^1$H NMR (400 MHz, DMSO-$d_6$) δ 12.18 (s, 1H), 8.73 (s, 2H), 7.73 (s, 1H), 4.86 - 4.78 (m, 1H), 3.86 (brs, 2H), 3.71 (brs, 2H), 3.42 (brs, 4H), 2.69 - 2.66 (m, 2H), 2.27 (s, 3H), 1.22 (d, *J* = 6.2 Hz, 3H).

**Example 134**

**2-(6-Oxo-5-(trifluoromethyl)-1,6-dihydropyridin-3-yl)phenyl 4-(5-(trifluoromethyl)pyrimidin-2-yl)piperazine-1-carboxylate**

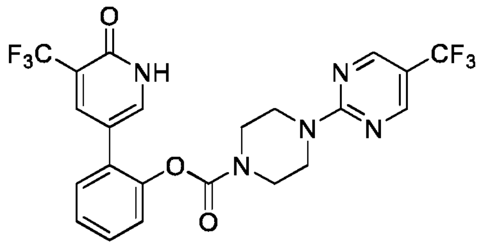

(6-Methoxy-5-(trifluoromethyl)pyridin-3-yl)boronic acid and 2-bromophenol were used as raw materials with the preparation method referring to **example 95**.

MS (ESI) M/Z: 514.2 $[M+H]^+$.

$^1H$ NMR (400 MHz, DMSO-$d_6$) δ 12.63 (s, 1H), 8.76 (s, 2H), 7.98 (d, *J* = 2.2 Hz, 1H), 7.83 (d, *J* = 2.2 Hz, 1H),7.51 (dd, *J* = 7.6, 1.7 Hz, 1H), 7.42 (td, *J* = 7.7, 1.7 Hz, 1H), 7.33 (td, *J* = 7.5, 1.2 Hz, 1H), 7.26 (dd, *J* = 8.0, 1.1 Hz, 1H), 3.85 (brs, 4H), 3.64 (brs, 2H), 3.48 (brs, 2H).

**Example 135**

**4-Methyl-3-(6-oxo-5-(trifluoromethyl)-1,6-dihydropyridin-3-yl)phenyl 4-(5-(trifluoromethyl)pyrimidin-2-yl)piperazine-1-carboxylate**

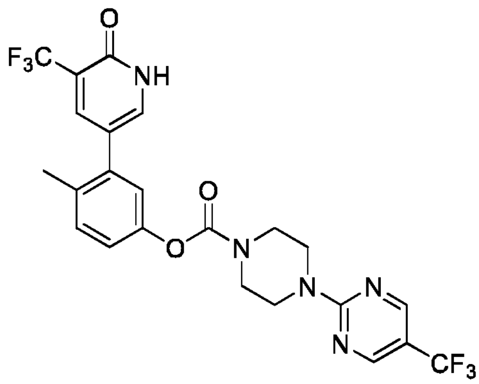

(6-Methoxy-5-(trifluoromethyl)pyridin-3-yl)boronic acid and 3-bromo-4-methylphenol were used as raw materials with the preparation method referring to **example 95**.

MS (ESI) M/Z: 528.1 $[M+H]^+$.

$^1H$ NMR (400 MHz, DMSO-$d_6$) δ 12.55 (s, 1H), 8.75 (s, 2H), 7.92 (d, *J* = 2.1 Hz, 1H), 7.74 (d, *J* = 2.3 Hz, 1H), 7.30 (d, *J* = 8.2 Hz, 1H), 7.11-7.07 (m, 2H), 3.95 (brs, 4H), 3.70 (brs, 2H), 3.55 (brs, 2H), 2.25 (s, 3H).

**Example 136**

**6'-Oxo-5'-(trifluoromethyl)-1',6'-dihydro-[3,3'-bipyridin]-5-yl 4-(5-(trifluoromethyl)pyrimidin-2-yl)piperazine-1-carboxylate**

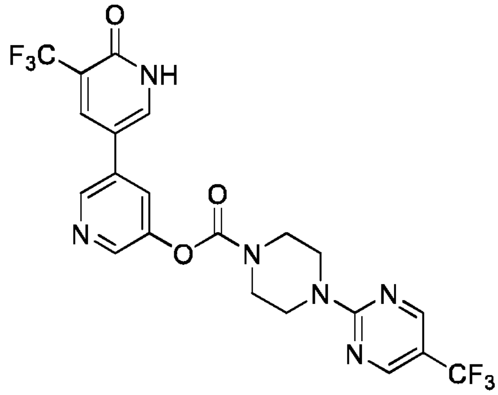

(6-Methoxy-5-(trifluoromethyl)pyridin-3-yl)boronic acid and 5-bromopyridin-3-ol were used as raw materials with the preparation method referring to **example 95**.

MS (ESI) M/Z: 515.0 $[M+H]^+$.

$^1H$ NMR (400 MHz, DMSO-$d_6$) δ 12.80 (brs, 1H), 8.79 - 8.74 (m, 3H), 8.40 (d, *J* = 2.4 Hz, 1H), 8.29 (d, *J* = 2.7 Hz, 1H), 8.24 (d, *J* = 2.8 Hz, 1H), 8.01 (t, *J* = 2.3 Hz, 1H), 4.00 (brs, 4H), 3.77 (brs, 2H), 3.60 (brs, 2H).

**Example 137**

**6'-Oxo-5'-(trifluoromethyl)-1',6'-dihydro-[2,3'-bipyridin]-4-yl 4-(5-(trifluoromethyl)pyrimidin-2-yl)piperazine-1-carboxylate**

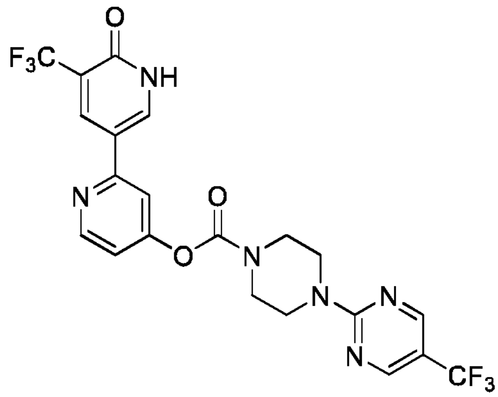

(6-Methoxy-5-(trifluoromethyl)pyridin-3-yl)boronic acid and 2-bromopyridin-4-ol were used as raw materials with the preparation method referring to **example 95.**

MS (ESI) M/Z: 515.0 $[M+H]^+$.

$^1H$ NMR (400 MHz, DMSO-$d_6$) δ 8.75 (s, 2H), 8.60 (d, *J* = 2.6 Hz, 1H), 8.57 (d, *J* = 5.5 Hz, 1H), 8.48 (d, *J* = 2.6 Hz, 1H), 7.85 (d, *J* = 2.1 Hz, 1H), 7.19 (dd, *J* = 5.5, 2.1 Hz, 1H), 3.97 (brs, 4H), 3.74 (brs, 2H), 3.59 (brs, 2H).

**Example 138**

**3-Methyl-5-(6-oxo-5-(trifluoromethyl)-1,6-dihydropyridin-3-yl)phenyl 4-(5-(trifluoromethyl)pyrimidin-2-yl)piperazine-1-carboxylate**

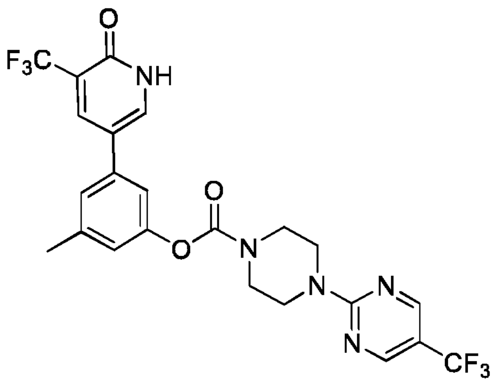

(6-Methoxy-5-(trifluoromethyl)pyridin-3-yl)boronic acid and 3-bromo-5-methylphenol were used as raw materials with the preparation method referring to **example 95**.

MS (ESI) M/Z: 528.1 $[M+H]^+$.

$^1H$ NMR (400 MHz, DMSO-$d_6$) δ 12.64 (brs,1H), 8.77 (s, 2H), 8.21 (s, 1H), 8.08 (s, 1H), 7.37 (s, 1H), 7.29 (s, 1H), 6.96 (s, 1H), 3.97 (brs, 4H), 3.73 (brs, 2H), 3.58 (brs, 2H), 2.37 (s, 3H).

## Example 139

**4-Fluoro-3-(6-oxo-5-(trifluoromethyl)-1,6-dihydropyridin-3-yl)phenyl 4-(5-(trifluoromethyl)pyrimidin-2-yl)piperazine-1-carboxylate**

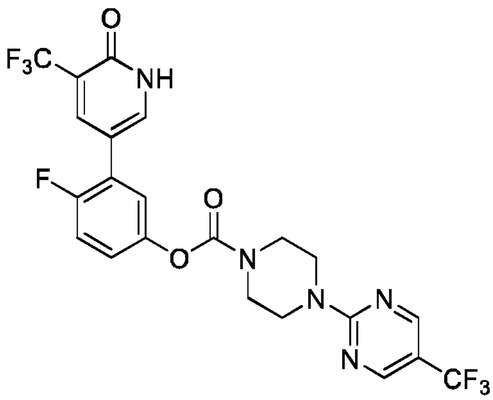

(6-Methoxy-5-(trifluoromethyl)pyridin-3-yl)boronic acid and 3-bromo-4-fluorophenol were used as raw materials with the preparation method referring to **example 95**.

MS (ESI) M/Z: 532.0 $[M+H]^+$.

$^1H$ NMR (400 MHz, DMSO-$d_6$) δ 12.70 (s, 1H), 8.76 (s, 2H), 8.11 (d, *J* = 2.4 Hz, 1H), 7.96 (s, 1H), 7.47 (dd, *J* = 6.8, 2.9 Hz, 1H), 7.36 - 7.31 (m, 1H), 7.22 - 7.18 (m, 1H), 3.96 (brs, 4H), 3.72 (brs, 2H), 3.57 (brs, 2H).

## Example 140

**3-(6-Oxo-5-(trifluoromethyl)-1,6-dihydropyridin-3-yl)phenyl (*R*)-2-methyl-4-(5-(trifluoromethyl)pyrimidin-2-yl)piperazine-1-carboxylate**

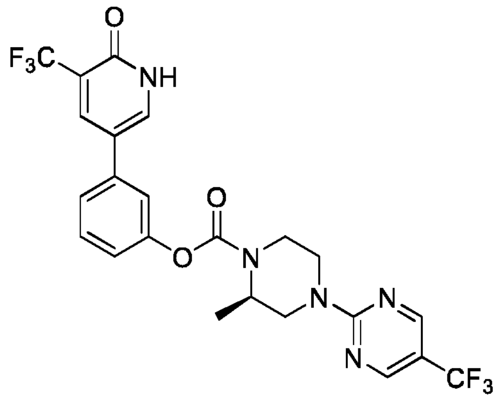

(6-Methoxy-5-(trifluoromethyl)pyridin-3-yl)boronic acid and *tert*-butyl (*R*)-2-methylpiperazine-1-carboxylate were used as raw materials with the preparation method referring to **example 95**.

MS (ESI) M/Z: 528.4 $[M+H]^+$.

$^1H$ NMR (400 MHz, DMSO-$d_6$) δ 12.64 (s, 1H), 8.75 (s, 2H), 8.23 (d, *J* = 2.2 Hz, 1H), 8.09 (d, *J* = 2.3 Hz, 1H), 7.54 - 7.48 (m, 2H), 7.44 (t, *J* = 7.9 Hz, 1H), 7.14 - 7.10 (m, 1H), 4.66 - 4.54 (m, 2H), 4.48 (brs, 1H), 4.02 (brs, 1H), 3.44 (d, *J* = 10.9 Hz, 1H), 3.30 - 3.20 (m, 2H), 1.19 (s, 3H).

**Example 141**

**3-(6-Oxo-5-(trifluoromethyl)-1,6-dihydropyridin-3-yl)phenyl (*R*)-3-(hydroxymethyl)-4-(5-(trifluoromethyl)pyrimidin-2-yl)piperazine-1-carboxylate**

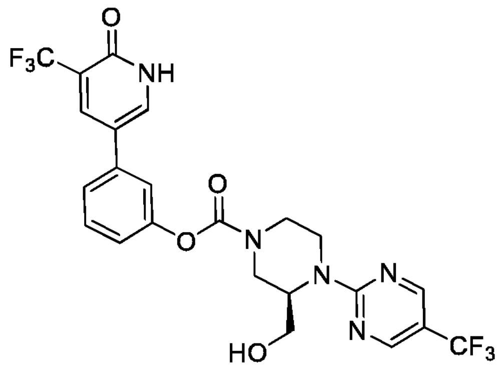

(6-Methoxy-5-(trifluoromethyl)pyridin-3-yl)boronic acid and *tert*-butyl (R)-3-(hydroxymethyl)piperazine-1-carboxylate were used as raw materials with the preparation method referring to **example 95**.

MS (ESI) M/Z: 543.5 $[M+H]^+$.

$^1H$ NMR (400 MHz, $CDCl_3$) δ 13.03 (s, 1H), 8.56 (s, 2H), 8.12 - 8.10 (m, 1H), 7.83 - 7.71 (m, 1H), 7.49 - 7.44 (m, 1H), 7.27 - 7.26 (m, 1H), 7.21 (s, 1H), 7.17 - 7.15 (m, 1H), 5.05 (brs, 1H), 4.77 (d, *J* = 13.6 Hz, 1H), 4.48 - 4.39 (m, 1H), 4.33 - 4.22(m, 1H), 3.96 - 3.83 (m, 2H), 3.49 - 3.21 (m, 3H).

**Example 142**

**3-(6-Oxo-5-(trifluoromethyl)-1,6-dihydropyridin-3-yl)phenyl (*S*)-3-(hydroxymethyl)-4-(5-(trifluoromethyl)pyrimidin-2-yl)piperazine-1-carboxylate**

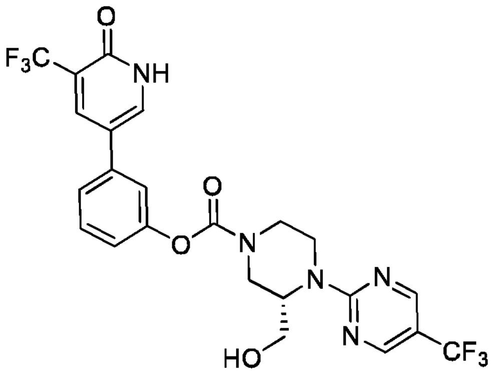

(6-Methoxy-5-(trifluoromethyl)pyridin-3-yl)boronic acid and *tert*-butyl (*S*)-3-(hydroxymethyl)piperazine-1-carboxylate were used as raw materials with the preparation method referring to **example 95**.

MS (ESI) M/Z: 543.5 $[M+H]^+$.

$^1H$ NMR (400 MHz, DMSO-$d_6$) δ 12.66 (s, 1H), 8.76 (d, *J* = 0.9 Hz, 2H), 8.23 (d, *J* = 2.7 Hz, 1H), 8.08 (s, 1H), 7.59 - 7.38 (m, 3H), 7.17 - 7.09 (m, 1H), 5.08 - 4.90 (m, 1H), 4.90 - 4.80 (m, 1H), 4.68 - 4.53 (m, 1H), 4.47 - 3.93 (m, 2H), 3.78 - 3.52 (m, 2H), 3.48 - 3.36 (m, 1H), 3.21 - 3.03 (m, 1H).

**Example 143**

**3-(6-Oxo-5-(trifluoromethyl)-1,6-dihydropyridin-3-yl)phenyl (*R*)-3-(hydroxymethyl)-4-(5-(trifluoromethyl)pyrimidin-2-yl)piperazine-1-carboxylate**

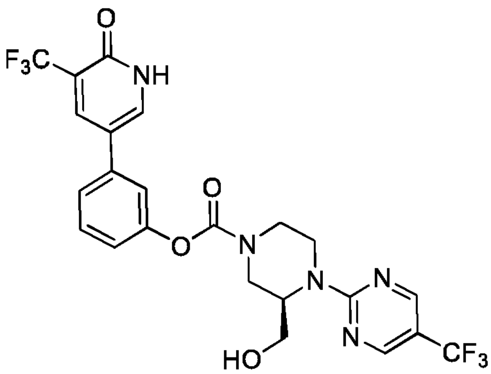

(6-Methoxy-5-(trifluoromethyl)pyridin-3-yl)boronic acid and *tert*-butyl (*R*)-3-(hydroxymethyl)piperazine-1-carboxylate were used as raw materials with the preparation method referring to **example 95**.

MS (ESI) M/Z: 543.5 $[M+H]^+$.

$^1H$ NMR (400 MHz, $CDCl_3$) δ 13.03 (s, 1H), 8.56 (s, 2H), 8.17 - 8.07 (m, 1H), 7.83 (s, 0.5H), 7.71 (s, 0.5H), 7.50 - 7.42 (m, 1H), 7.27 - 7.26 (m, 1H), 7.21 (s, 1H), 7.19 - 7.12 (m, 1H), 5.05 (brs, 1H), 4.77 (d, J = 13.6 Hz, 1H), 4.53 - 4.37 (m, 1H), 4.37 - 4.20 (m, 1H), 4.04 - 3.78 (m, 2H), 3.54 - 3.19 (m, 3H).

**Example 144**

**2-(6-Oxo-5-(trifluoromethyl)-1,6-dihydropyridin-3-yl)ethyl 4-(5-cyanopyridin-2-yl)piperazine-1-carboxylate**

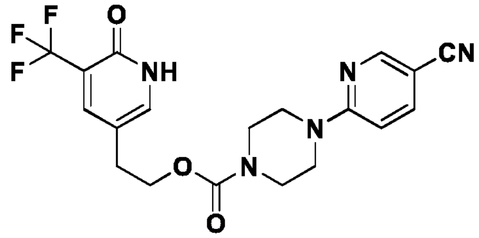

(6-Methoxy-5-(trifluoromethyl)pyridin-3-yl)boronic acid and 6-chloronicotinonitrile were used as raw materials with the preparation method referring to **example 48**.

MS (ESI) M/Z: 422.0 [M+H]$^+$.

$^1$H NMR (400 MHz, DMSO-$d_6$) δ 12.20 (brs, 1H), 8.50 (d, *J* = 2.3 Hz, 1H), 7.88 (dd, *J* = 9.1, 2.3 Hz, 2H), 7.59 (d, *J* = 1.6 Hz, 1H), 6.94 (d, *J* = 9.1 Hz, 1H), 4.16 (t, *J* = 6.3 Hz, 2H), 3.73 - 3.59 (m, 4H), 3.48 - 3.40 (m, 4H), 2.72 (t, *J* = 6.2 Hz, 2H).

**Example 145**

**2-(6-Oxo-5-(trifluoromethyl)-1,6-dihydropyridin-3-yl)ethyl (2*S*,6*S*)-2,6-dimethyl-4-(5-(trifluoromethyl)pyrimidin-2-yl)piperazine-1-carboxylate**

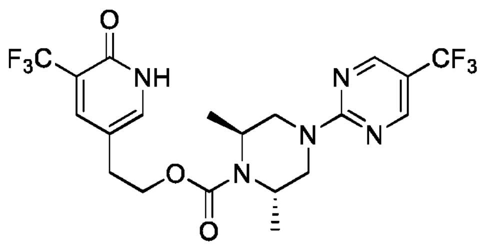

5-Bromo-2-methoxy-3-(trifluoromethyl)pyridine and *tert*-butyl (2*S*,6*S*)-2,6-dimethylpiperazine-1-carboxylate were used as raw materials with the preparation method referring to **intermediate 1C** and **example 109**.

MS (ESI) M/Z: 493.9 [M+H]$^+$.

$^1$H NMR (400 MHz, DMSO-*d*6) δ 12.22 (s, 1H), 8.76 (d, *J* = 0.8 Hz, 2H), 7.90 (d, *J* = 2.5 Hz, 1H), 7.59 (s, 1H), 4.32 - 3.99 (m, 6H), 3.66 (dd, *J* = 13.9, 4.0 Hz, 2H), 2.74 (t, *J* = 6.2 Hz, 2H), 1.13 (d, *J* = 6.6 Hz, 6H).

**Example 146**

**2-(6-Oxo-5-(trifluoromethyl)-1,6-dihydropyridin-3-yl)ethyl (*2S,5R*)-2,5-dimethyl-4-(5-(trifluoromethyl)pyrimidin-2-yl)piperazine-1-carboxylate**

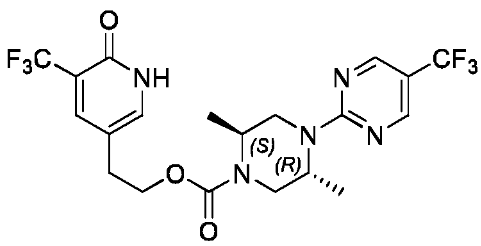

5-Bromo-2-methoxy-3-(trifluoromethyl)pyridine and *tert*-butyl (2*S*,5*R*)-2,5-dimethylpiperazine-1-carboxylate were used as raw materials with the preparation method referring to **intermediate 1C** and **example 109**.

MS (ESI) M/Z: 494.0 [M+H]$^+$.

$^1$H NMR (400 MHz, DMSO-*d*6) δ 12.22 (s, 1H), 8.73 (d, *J* = 10.0 Hz, 2H), 7.90 (d, *J* = 5.0 Hz, 1H), 7.59 (s, 1H),

4.91 (d, $J$ = 24.0 Hz, 1H), 4.44 - 4.10 (m, 4H), 3.68 (dd, $J$= 29.3, 13.7 Hz, 1H), 3.37 - 3.33 (m, 1H), 3.29 - 3.18 (m, 1H), 2.78 - 2.69 (m, 2H), 1.15 - 0.95 (m, 6H).

**Example 147**

**2-(6-Oxo-5-(trifluoromethyl)-1,6-dihydropyridin-3-yl)ethyl (2*R*,5*S*)-2,5-dimethyl-4-(5-(trifluoromethyl)pyrimidin-2-yl)piperazine-1-carboxylate**

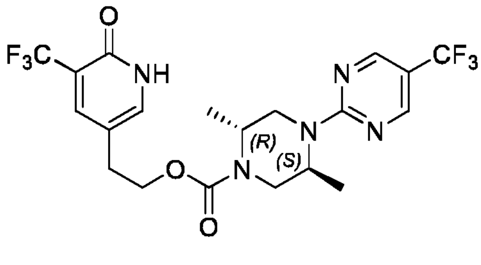

5-Bromo-2-methoxy-3-(trifluoromethyl)pyridine and *tert*-butyl (2*R*,5*S*)-2,5-dimethylpiperazine-1-carboxylate were used as raw materials with the preparation method referring to **intermediate 1C** and **example 109**.

MS (ESI) M/Z: 494.2 $[M+H]^+$.

$^1H$ NMR (400 MHz, DMSO-$d_6$) δ 12.18 (s, 1H), 8.73 (d, $J$ = 9.2 Hz, 2H), 7.90 (d, $J$ = 2.8 Hz, 1H), 7.60 (d, $J$ = 3.2 Hz, 1H), 4.98 - 4.85 (m, 1H), 4.42 - 4.13 (m, 4H), 3.73 - 3.63 (m, 1H), 3.38 - 3.34 (m, 1H), 3.28 - 3.18 (m, 1H), 2.79 - 2.69 (m, 2H), 1.12 - 1.00 (m, 6H).

**Example 148**

**2-(6-Oxo-5-(trifluoromethyl)-1,6-dihydropyridin-3-yl)ethyl (2*R*,6*R*)-2,6-dimethyl-4-(5-(trifluoromethyl)pyrimidin-2-yl)piperazine-1-carboxylate**

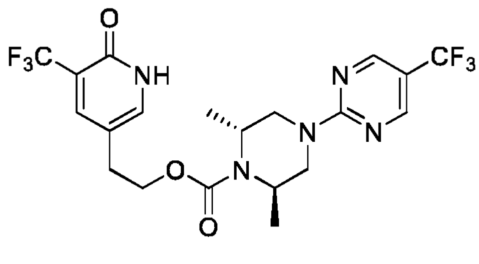

5-Bromo-2-methoxy-3-(trifluoromethyl)pyridine and tert-butyl (2*R*,6*R*)-2,6-dimethylpiperazine-1-carboxylate were used as raw materials with the preparation method referring to **intermediate 1C** and **example 109**.

MS (ESI) M/Z: 494.7 $[M+H]^+$.

$^1H$ NMR (400 MHz, DMSO-$d_6$) δ 12.23 (s, 1H), 8.75 (s, 2H), 7.90 (s, 1H), 7.59 (s, 1H), 4.25 - 4.09 (m, 4H), 4.09 (d, $J$ = 13.9 Hz, 2H), 3.66 (dd, $J$ = 13.8, 3.8 Hz, 2H), 2.74 (t, $J$ = 6.1 Hz, 2H), 1.13 (d, $J$ = 6.6 Hz, 6H).

**Example 149**

2-(6-Oxo-5-(trifluoromethyl)-1,6-dihydropyridin-3-yl)ethyl (3R,5R)-3,5-dimethyl-4-(5-(trifluoromethyl)pyrimidin-2-yl)piperazine-1 -carboxylate

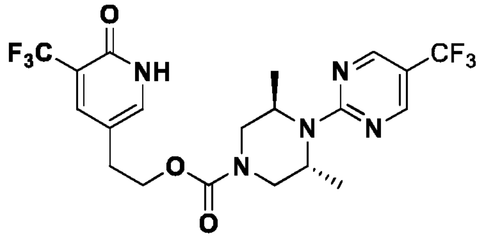

5-Bromo-2-methoxy-3-(trifluoromethyl)pyridine and *tert*-butyl (*3R,5R*)-3,5-dimethylpiperazine-1-carboxylate were used as raw materials with the preparation method referring to **intermediate 1C** and **example 109**.

MS (ESI) M/Z: 493.8 $[M+H]^+$.

$^1$H NMR (400 MHz, DMSO-$d_6$) δ 12.23 (s, 1H), 8.76 (s, 2H), 7.91 (d, *J* = 1.9 Hz, 1H), 7.60 (d, *J* = 1.7 Hz, 1H), 4.68 - 4.45 (m, 2H), 4.31 - 4.09 (m, 2H), 3.71 (dd, *J* = 13.4, 1.6 Hz, 1H), 3.63 (d, *J* = 2.7 Hz, 2H), 3.56 (dd, *J* = 13.5, 3.7 Hz, 1H), 2.75 (t, *J* = 6.2 Hz, 2H), 1.22 (t, *J* = 6.9 Hz, 6H).

**Example 150**

**2-(6-Oxo-5-(trifluoromethyl)-1,6-dihydropyridin-3-yl)ethyl (2*S*,6*R*)-2,6-dimethyl-4-(5-(trifluoromethyl)pyrimidin-2-yl)piperazine-1-carboxylate**

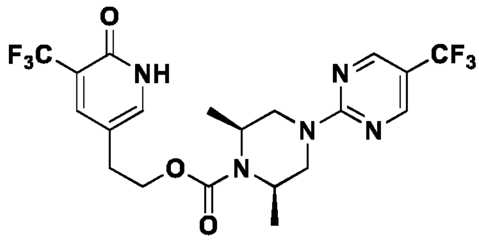

5-Bromo-2-methoxy-3-(trifluoromethyl)pyridine and tert-butyl (2S,6R)-2,6-dimethylpiperazine-1-carboxylate were used as raw materials with the preparation method referring to **intermediate 1C** and **example 109**.

MS (ESI) M/Z: 493.8 $[M+H]^+$.

$^1$H NMR (400 MHz, DMSO-$d_6$) δ 12.25 (s, 1H), 8.72 (d, *J* = 0.6 Hz, 2H), 7.88 (d, *J* = 1.9 Hz, 1H), 7.60 (s, 1H), 4.60 (d, *J* = 13.2 Hz, 2H), 4.24 - 4.11 (m, 4H), 3.21 - 3.15 (m, 2H), 2.74 (t, *J* = 6.1 Hz, 2H), 1.05 (d, *J* = 6.9 Hz, 6H).

**Example 151**

**2-(6-Oxo-5-(trifluoromethyl)-1,6-dihydropyridin-3-yl)ethyl (2*R*,6*S*)-4-(5-cyclopropylpyrimidin-2-yl)-2,6-dimethylpiperazine-1-carboxylate**

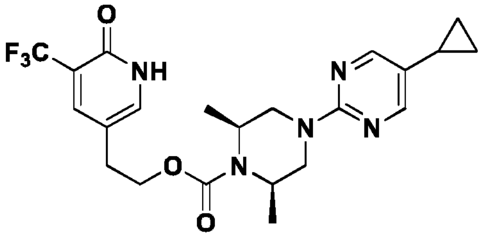

5-Bromo-2-methoxy-3-(trifluoromethyl)pyridine, 2-chloro-5-cyclopropylpyrimidine, and *tert-butyl* (2*S*,6*R*)-2,6-dimethylpiperazine-1-carboxylate were used as raw materials with the preparation method referring to **intermediate 1C** and **example 109**.

MS (ESI) M/Z: 466.0 $[M+H]^+$.

$^1$H NMR (400 MHz, DMSO-$d_6$) δ 12.21 (brs, 1H), 8.17 (s, 2H), 7.87 (d, *J* = 1.8 Hz, 1H), 7.59 (d, *J* = 1.9 Hz, 1H), 4.49 (d, *J* = 13.0 Hz, 2H), 4.26 - 4.02 (m, 4H), 2.99 (dd, *J* = 13.0, 4.4 Hz, 2H), 2.73 (t, *J* = 6.1 Hz, 2H), 1.81 - 1.70 (m, 1H), 1.05 (d, *J* = 6.8 Hz, 6H), 0.89 - 0.82 (m, 2H), 0.66 - 0.60 (m, 2H).

**Example 152**

**2-(6-Oxo-5-(trifluoromethyl)-1,6-dihydropyridin-3-yl)ethyl 4-(5-cyclopropylpyrimidin-2-yl)piperazine-1-carboxylate**

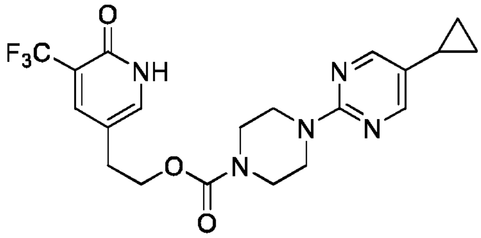

5-Bromo-2-methoxy-3-(trifluoromethyl)pyridine and 2-chloro-5-cyclopropylpyrimidine were used as raw materials with the preparation method referring to **intermediate 1C** and **example 109**.
MS (ESI) M/Z: 438.0 [M+H]$^+$.
$^1$H NMR (400 MHz, DMSO-$d_6$) δ 12.21 (s, 1H), 8.19 (s, 2H), 7.90 (d, *J* = 1.8 Hz, 1H), 7.71 - 7.40 (m, 1H), 4.15 (t, *J* = 6.2 Hz, 2H), 3.71 - 3.56 (m, 4H), 3.45 - 3.35 (m, 4H), 2.72 (t, *J* = 6.2 Hz, 2H), 1.82 - 1.72 (m, 1H), 0.91 - 0.81 (m, 2H), 0.68 - 0.55 (m, 2H).

**Example 153**

2-(6-Oxo-5-(trifluoromethyl)-1,6-dihydropyridin-3-yl)ethyl (3*R*,5*S*)-3,5-dimethyl-4-(5-(trifluoromethyl)pyrimidin-2-yl)piperazine-1-carboxylate

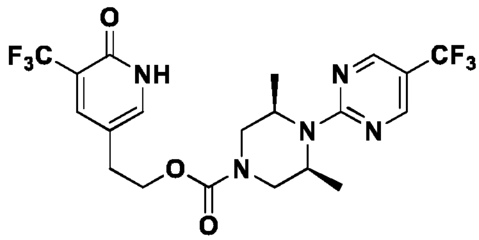

5-Bromo-2-methoxy-3-(trifluoromethyl)pyridine and *tert*-butyl (3*R*,5*S*)-3,5-dimethylpiperazine-1-carboxylate were used as raw materials with the preparation method referring to **intermediate 1C** and **example 109**.
MS (ESI) M/Z: 494.4 [M+H]$^+$.
$^1$H NMR (400 MHz, DMSO-$d_6$) δ 12.22 (s, 1H), 8.74 (s, 2H), 7.87 (s, 1H), 7.58 (s, 1H), 5.00 - 4.77 (m, 2H), 4.46 (d, *J* = 11.6 Hz, 1H), 3.96 (d, *J* = 10.3 Hz, 1H), 3.82 (d, *J* = 12.1 Hz, 1H), 3.04 (s, 3H), 2.79 - 2.68 (m, 1H), 2.61 (dd, *J* = 14.0, 8.4 Hz, 1H), 1.20 (d, *J* = 6.0 Hz, 3H), 1.13 - 0.89 (m, 3H).

**Example 154**

**2-(6-Oxo-5-(trifluoromethyl)-1,6-dihydropyridin-3-yl)ethyl (2*R*,5*R*)-2,5-dimethyl-4-(5-(trifluoromethyl)pyrimidin-2-yl)piperazine-1-carboxylate**

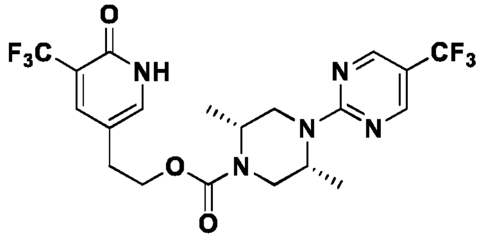

5-Bromo-2-methoxy-3-(trifluoromethyl)pyridine and *tert*-butyl (2*R*,5*R*)-2,5-dimethylpiperazine-1-carboxylate were used as raw materials with the preparation method referring to **intermediate 1C** and **example 109**.

MS (ESI) M/Z: 494.0 $[M+H]^+$.

$^1$H NMR (400 MHz, DMSO-$d_6$) δ 12.21 (s, 1H), 8.68 (s, 2H), 7.85 (s, 1H), 7.57 (s, 1H), 4.69 - 4.54 (m, 1H), 4.34 - 4.20 (m, 1H), 4.16 - 4.02 (m, 2H), 3.96 (d, *J* = 7.0 Hz, 1H), 3.89 - 3.80 (m, 1H), 3.18 - 3.03 (m, 2H), 2.69 (t, *J* = 6.1 Hz, 2H), 1.13 (d, *J* = 6.1 Hz, 3H), 1.06 (d, *J* = 6.1 Hz, 3H).

**Example 155**

**(*S*)-1-(6-oxo-5-(trifluoromethyl)-1,6-dihydropyridin-3-yl)propan-2-yl (2*R*,5*S*)-2,5-dimethyl-4-(5-(trifluoromethyl)pyrimidin-2-yl)piperazine-1-carboxylate**

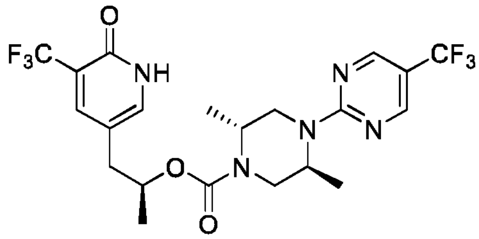

5-Bromo-2-methoxy-3-(trifluoromethyl)pyridine and tert-butyl (2R,5S)-2,5-dimethylpiperazine-1-carboxylate were used as raw materials with the preparation method referring to **intermediate 1C** and **example 48**.

MS (ESI) M/Z: 508.6 $[M+H]^+$.

$^1$H NMR (400 MHz, DMSO-$d_6$) δ 12.25 (s, 1H), 8.72 (d, *J* = 9.6 Hz, 2H), 7.86 (d, *J* = 4.4 Hz, 1H), 7.58 (s, 1H), 4.98 - 4.76 (m, 2H), 4.41 - 4.25 (m, 2H), 3.68 (t, *J* = 13.2 Hz, 1H), 3.38 - 3.34 (m, 0.5H), 3.31 - 3.25 (m, 1H), 3.21 - 3.14 (m, 0.5H), 2.78 - 2.58 (m, 2H), 1.21 (dd, *J* = 14.0, 6.4 Hz, 3H), 1.12 (d, *J* = 6.8 Hz, 1.5H), 1.02 (dd, *J* = 14.0, 6.4 Hz, 3H), 0.96 (d, *J* = 6.4 Hz, 1.5H).

**Example 156**

**(*S*)-1-(6-oxo-5-(trifluoromethyl)-1,6-dihydropyridin-3-yl)propan-2-yl (2*R*,6*S*)-2,6-dimethyl-4-(5-(trifluoromethyl)pyrimidin-2-yl)piperazine-1-carboxylate**

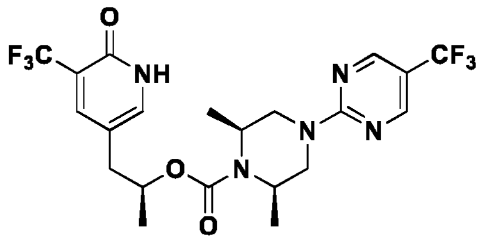

5-Bromo-2-methoxy-3-(trifluoromethyl)pyridine and *tert*-butyl (2*R*,6*S*)-2,6-dimethylpiperazine-1-carboxylate were used as raw materials with the preparation method referring to **intermediate 1C** and **example 48**.

MS (ESI) M/Z: 508.4 $[M+H]^+$.

$^1H$ NMR (400 MHz, DMSO-$d_6$) δ 12.22 (s, 1H), 8.72 (s, 2H), 7.84 (s, 1H), 7.56 (s, 1H), 4.95 - 4.79 (m, 1H), 4.60 (d, *J* = 13.5 Hz, 2H), 4.23 - 4.15 (m, 2H), 3.19 - 3.12 (m, 2H), 2.80 - 2.71 (m, 1H), 2.66 - 2.57 (m, 1H), 1.19 (d, *J* = 6.2 Hz, 3H), 1.03 (t, *J* = 6.8 Hz, 6H).

**Example 157**

**(*S*)-1-(6-oxo-5-(trifluoromethyl)-1,6-dihydropyridin-3-yl)propan-2-yl (3*R*,5*R*)-3,5-dimethyl-4-(5-(trifluoromethyl)pyrimidin-2-yl)piperazine-1-carboxylate**

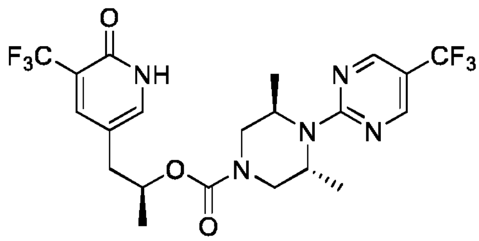

5-Bromo-2-methoxy-3-(trifluoromethyl)pyridine and *tert*-butyl (3*R*,5R)-3,5-dimethylpiperazine-1-carboxylate were used as raw materials with the preparation method referring to **intermediate 1C** and **example 48**.

MS (ESI) M/Z: 508.0 $[M+H]^+$.

$^1H$ NMR (400 MHz, DMSO-$d_6$) δ 12.19 (s, 1H), 8.76 (s, 2H), 7.84 (s, 1H), 7.56 (s, 1H), 4.96 - 4.80 (m, 1H), 4.64 - 4.48 (m, 2H), 3.72 - 3.65 (m, 2H), 3.59 - 3.51 (m, 2H), 2.78 - 2.71 (m, 1H), 2.65 - 2.57 (m, 1H), 1.26 (d, *J* = 6.8 Hz, 3H), 1.21 (d, *J* = 6.0 Hz, 3H), 1.13 (d, *J* = 6.4 Hz, 3H).

**Example 158**

**(*S*)-1-(6-Oxo-5-(trifluoromethyl)-1,6-dihydropyridin-3-yl)propan-2-yl (2*R*,6*R*)-2,6-dimethyl-4-(5-(trifluoromethyl)pyrimidin-2-yl)piperazine-1-carboxylate**

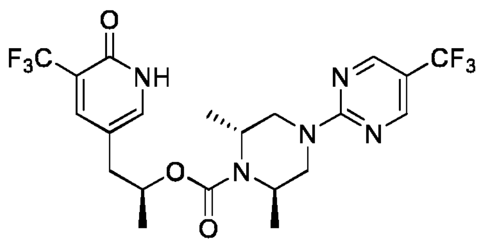

5-Bromo-2-methoxy-3-(trifluoromethyl)pyridine and *tert*-butyl (2*R*,6*R*)-2,6-dimethylpiperazine-1-carboxylate were used as raw materials with the preparation method referring to **intermediate 1C** and **example 48**.

MS (ESI) M/Z: 508.4 $[M+H]^+$.

$^1H$ NMR (400 MHz, DMSO-$d_6$) δ 8.76 (s, 2H), 7.86 (s, 1H), 7.57 (s, 1H), 4.91 - 4.81 (m, 1H), 4.18 - 4.04 (m, 4H), 3.66 (d, *J* = 13.3 Hz, 2H), 2.79 - 2.70 (m, 2H), 1.21 (dd, *J* = 5.9, 1.7 Hz, 3H), 1.13 (d, *J* = 6.0 Hz, 6H).

**Example 159**

**(*S*)-1-(6-oxo-5-(trifluoromethyl)-1,6-dihydropyridin-3-yl)propan-2-yl (2*S*,6*S*)-2,6-dimethyl-4-(5-(trifluoromethyl)pyrimidin-2-yl)piperazine-1-carboxylate**

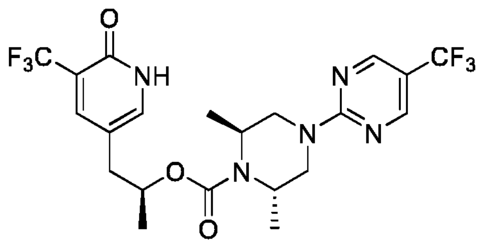

5-Bromo-2-methoxy-3-(trifluoromethyl)pyridine and *tert-butyl* (2*S*,6*S*)-2,6-dimethylpiperazine-1-carboxylate were used as raw materials with the preparation method referring to **intermediate 1C** and **example 48**.

MS (ESI) M/Z: 508.0 $[M+H]^+$.

$^1$H NMR (400 MHz, DMSO-$d_6$) δ 12.21 (s, 1H), 8.77 (d, *J* = 0.9 Hz, 2H), 7.85 (d, *J* = 2.4 Hz, 1H), 7.57 (s, 1H), 5.05 - 4.89 (m, 1H), 4.24 - 4.03 (m, 4H), 3.65 (dd, *J* = 13.7, 4.0 Hz, 2H), 2.77 (dd, *J* = 14.1, 4.2 Hz, 1H), 2.59 (dd, *J* = 14.1, 7.9 Hz, 1H), 1.21 (d, *J* = 6.3 Hz, 3H), 1.11 (d, *J* = 6.6 Hz, 6H).

**Example 160**

**(*S*)-1-(6-oxo-5-(trifluoromethyl)-1,6-dihydropyridin-3-yl)propan-2-yl (2*S*,5*R*)-2,5-dimethyl-4-(5-(trifluoromethyl)pyrimidin-2-yl)piperazine-1-carboxylate**

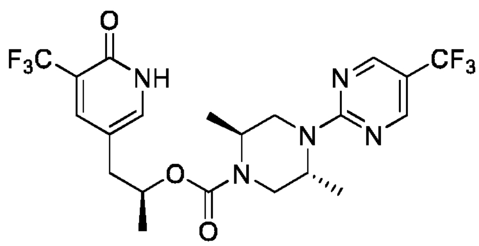

5-Bromo-2-methoxy-3-(trifluoromethyl)pyridine and *tert*-butyl (2*S*,5*R*)-2,5-dimethylpiperazine-1-carboxylate were used as raw materials with the preparation method referring to **intermediate 1C** and **example 48**.

MS (ESI) M/Z: 508.7 $[M+H]^+$.

$^1$H NMR (400 MHz, DMSO-$d_6$) δ 12.07 (s, 1H), 8.75 (s, 1H), 8.73 (s, 1H), 7.89 (s, 0.5 H), 7.82 (s, 0.5H), 7.60 (s, 0.5H), 7.54 (s, 0.5H), 5.03 - 4.81 (m, 4H), 4.46 - 4.23 (m, 2H), 3.84 - 3.60 (m, 1H), 3.30 - 3.17 (m, 2H), 2.79 - 2.55 (m, 2H), 1.19 (d, *J* = 5.9 Hz, 3H), 1.11 (d, *J* = 6.6 Hz, 1.5H), 1.05 (d, *J* = 6.6 Hz, 1.5H), 0.97 (t, *J* = 7.6 Hz, 3H).

**Example 161**

**(*S*)-1-(6-oxo-5-(trifluoromethyl)-1,6-dihydropyridin-3-yl)propan-2-yl (2*R*,5*R*)-2,5-dimethyl-4-(5-(trifluoromethyl)pyrimidin-2-yl)piperazine-1-carboxylate**

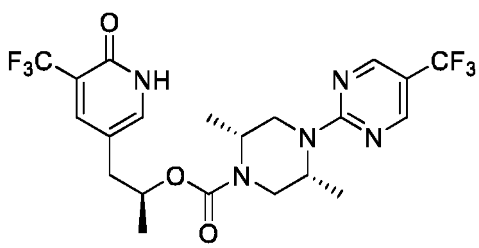

5-Bromo-2-methoxy-3-(trifluoromethyl)pyridine and tert-butyl (2R,5R)-2,5-dimethylpiperazine-1-carboxylate were used as raw materials with the preparation method referring to **intermediate 1C** and **example 48**.

MS (ESI) M/Z: 508.2 $[M+H]^+$.

$^1H$ NMR (400 MHz, $CD_3OD$) δ 8.54 (s, 2H), 7.88 (s, 1H), 7.51 (s, 1H), 4.90 - 4.86 (m, 1H), 4.75 (dd, *J* = 14.2, 6.6 Hz, 1H), 4.32 (brs, 1H), 4.09 - 3.94 (m, 2H), 3.09 - 2.96 (m, 2H), 2.80 - 2.61 (m, 2H), 1.26 - 1.20 (m, 3H), 1.17 (d, *J* = 6.2 Hz, 3H), 1.13 (d, *J* = 6.2 Hz, 3H).

**Example 162**

**(*S*)-1-(6-oxo-5-(trifluoromethyl)-1,6-dihydropyridin-3-yl)propan-2-yl (3*S*,5*R*)-3,5-dimethyl-4-(5-(trifluoromethyl)pyrimidin-2-yl)piperazine-1-carboxylate**

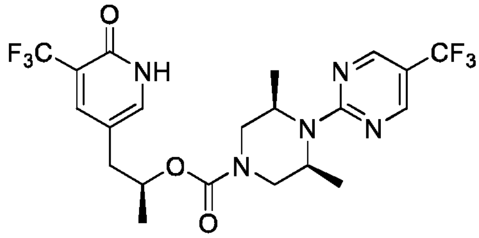

5-Bromo-2-methoxy-3-(trifluoromethyl)pyridine and tert-butyl (3S,5R)-3,5-dimethylpiperazine-1-carboxylate were used as raw materials with the preparation method referring to **intermediate 1C** and **example 48**.

MS (ESI) M/Z: 508.2 [M+H]$^+$.

$^1H$ NMR (400 MHz, DMSO-$d_6$) δ 12.20 (s, 1H), 8.76 (s, 2H), 7.86 (d, *J* = 1.8 Hz, 1H), 7.61 - 7.53 (m, 1H), 5.07 - 4.82 (m, 1H), 4.81 - 4.68 (m, 2H), 3.92 (d, *J* = 13.3 Hz, 2H), 3.18 - 2.95 (m, 2H), 2.79 - 2.58 (m, 2H), 1.21 (d, *J* = 6.3 Hz, 3H), 1.18 - 0.94 (m, 6H).

**Example 163**

**2-(6-Oxo-5-(trifluoromethyl)-1,6-dihydropyridin-3-yl)ethyl (2*R*,6*S*)-2,6-dimethyl-4-(5-methylpyrimidin-2-yl)piperazine-1-carboxylate**

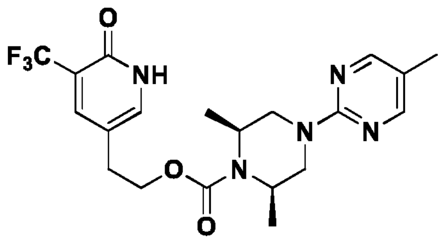

5-Bromo-2-methoxy-3-(trifluoromethyl)pyridine, *tert-butyl* (2*R*,6*S*)-2,6-dimethylpiperazine-1-carboxylate, and 2-chloro-5-methylpyrimidine were used as raw materials with the preparation method referring to **intermediate 1C and example 109**.

MS (ESI) M/Z: 440.1 [M+H]$^+$.

$^1H$ NMR (400 MHz, DMSO-$d_6$) δ 12.23 (s, 1H), 8.22 (s, 2H), 7.89 (d, *J* = 2.1 Hz, 1H), 7.59 (s, 1H), 4.50 (d, *J* = 13.1 Hz, 2H), 4.21 - 4.06 (m, 4H), 2.99 (dd, *J* = 13.0, 4.4 Hz, 2H), 2.74 (t, *J* = 6.2 Hz, 2H), 2.08 (s, 3H), 1.05 (d, *J* = 6.8 Hz, 6H).

**Example 164**

**2-(6-Oxo-5-(trifluoromethyl)-1,6-dihydropyridin-3-yl)ethyl (2*S*,6*S*)-2,6-dimethyl-4-(5-methylpyrimidin-2-yl)piperazine-1-carboxylate**

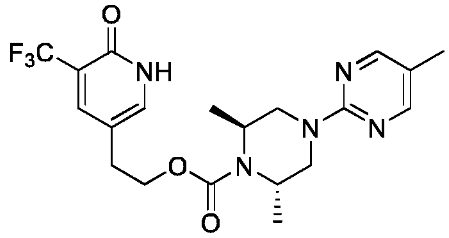

5-Bromo-2-methoxy-3-(trifluoromethyl)pyridine, tert-butyl (2S,6S)-2,6-dimethylpiperazine-1-carboxylate, and 2-chloro-5-methylpyrimidine were used as raw materials with the preparation method referring to **intermediate 1C** and **example 109**.

MS (ESI) M/Z: 440.0 $[M+H]^+$.

$^1H$ NMR (400 MHz, DMSO-$d_6$) δ 11.96 (brs, 1H), 8.22 (s, 2H), 7.87 (d, *J* = 2.4 Hz, 1H), 7.57 (d, *J* = 2.5 Hz, 1H), 4.20 - 4.13 (m, 2H), 4.11 - 4.03 (m, 3H), 4.00 - 3.96 (m, 2H), 3.55 - 3.51 (m, 1H), 2.72 (t, *J* = 6.2 Hz, 2H), 2.08 (s, 3H), 1.08 (d, *J* = 6.6 Hz, 6H).

**Example 165**

**2-(6-Oxo-5-(trifluoromethyl)-1,6-dihydropyridin-3-yl)ethyl (3*S*,5*R*)-3,5-dimethyl-4-(5-methylpyrimidin-2-yl)piperazine-1-carboxylate**

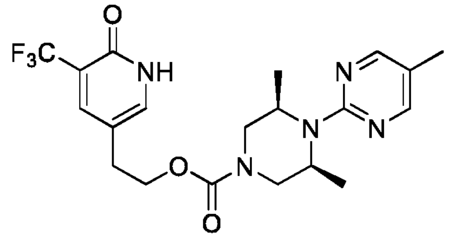

5-Bromo-2-methoxy-3-(trifluoromethyl)pyridine, *tert-butyl* (*3S,5R*)-3,5-dimethylpiperazine-1-carboxylate, and 2-chloro-5-methylpyrimidine were used as raw materials with the preparation method referring to **intermediate 1C** and **example 109**.

MS (ESI) M/Z: 440.3 $[M+H]^+$.

$^1H$ NMR (400 MHz, DMSO-$d_6$) δ 12.21 (s, 1H), 8.25 (s, 2H), 7.91 (s, 1H), 7.60 (s, 1H), 4.64 (brs, 2H), 4.34 - 4.07 (m, 2H), 3.97 - 3.73 (m, 2H), 3.16 - 2.92 (m, 2H), 2.75 (t, *J* = 6.3 Hz, 2H), 2.08 (s, 3H), 1.02 (brs, 6H).

**Example 166**

**2-(6-Oxo-5-(trifluoromethyl)-1,6-dihydropyridin-3-yl)ethyl (2*S*,5*R*)-2,5-dimethyl-4-(5-methylpyrimidin-2-yl)piperazine-1-carboxylate**

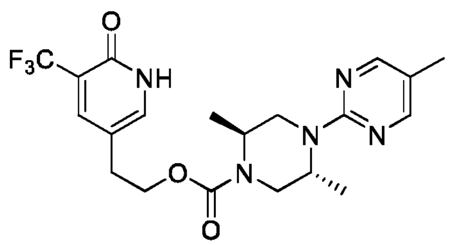

5-Bromo-2-methoxy-3-(trifluoromethyl)pyridine, *tert-butyl* (2*S*,5*R*)-2,5-dimethylpiperazine-1-carboxylate, and 2-chloro-5-methylpyrimidine were used as raw materials with the preparation method referring to **intermediate 1C** and

**example 109**.

MS (ESI) M/Z: 440.3 [M+H]$^+$.

$^1$H NMR (400 MHz, DMSO-$d_6$) δ 12.22 (s, 1H), 8.23 (s, 2H), 7.94 - 7.86 (m, 1H), 7.60 - 7.58 (m, 1H), 4.80 - 4.74 (m, 1H), 4.32 - 4.11 (m, 4H), 3.69 - 3.59 (m, 1H), 3.28 - 3.15 (m, 2H), 2.76 - 2.71 (m, 2H), 2.08 (s, 3H), 1.04 - 1.96 (m, 6H).

**Example 167**

**2-(6-Oxo-5-(trifluoromethyl)-1,6-dihydropyridin-3-yl)ethyl (2*R*,5*R*)-2,5-dimethyl-4-(5-methylpyrimidin-2-yl)piperazine-1-carboxylate**

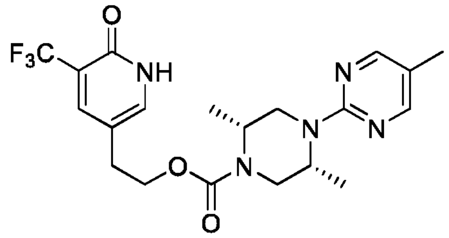

5-Bromo-2-methoxy-3-(trifluoromethyl)pyridine, *tert-butyl* (2*R*,5*R*)-2,5-dimethylpiperazine-1-carboxylate, and 2-chloro-5-methylpyrimidine were used as raw materials with the preparation method referring to **intermediate 1C** and **example 109**.

MS (ESI) M/Z: 440.8 [M+H]$^+$.

$^1$H NMR (400 MHz, $CD_3OD$) δ 8.15 (s, 2H), 7.91 (d, *J* = 1.6 Hz, 1H), 7.51 (d, *J* = 2.4 Hz, 1H), 4.61 (dd, *J* = 14.4, 6.4 Hz, 1H), 4.38 - 4.29 (m, 1H), 4.23 - 4.12 (m, 2H), 4.05 - 3.91 (m, 2H), 3.05 - 2.95 (m, 2H), 2.77 (t, *J* = 6.0 Hz, 2H), 2.11 (s, 3H), 1.14 (d, *J* = 6.0 Hz, 3H), 1.11 (d, *J* = 6.4 Hz, 3H).

**Example 168**

**2-(6-Oxo-5-(trifluoromethyl)-1,6-dihydropyridin-3-yl)ethyl (2*R*,5*S*)-2,5-dimethyl-4-(5-methylpyrimidin-2-yl)piperazine-1-carboxylate**

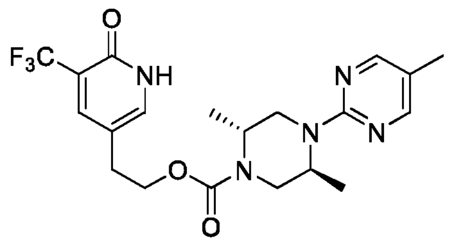

5-Bromo-2-methoxy-3-(trifluoromethyl)pyridine, *tert-butyl* (2R,5S)-2,5-dimethylpiperazine-1-carboxylate, and 2-chloro-5-methylpyrimidine were used as raw materials with the preparation method referring to **intermediate 1C** and **example 109**.

MS (ESI) M/Z: 440.3 [M+H]$^+$.

$^1$H NMR (400 MHz, DMSO-$d_6$) δ 12.23 (s, 1H), 8.23 (s, 2H), 7.90 (d, *J* = 4.0 Hz, 1H), 7.59 (s, 1H), 4.85 - 4.70 (m, 1H), 4.33 - 4.08 (m, 4H), 3.72 - 3.56 (m, 1H), 3.30 - 3.14 (m, 2H), 2.82 - 2.64 (m, 2H), 2.08 (s, 3H), 1.09 - 0.94 (m, 6H).

**Example 169**

**(*S*)-1-(6-oxo-5-(trifluoromethyl)-1,6-dihydropyridin-3-yl)propan-2-yl (2*R*,5*R*)-2,5-dimethyl-4-(5-methylpyrimidin-2-yl)piperazine-1-carboxylate**

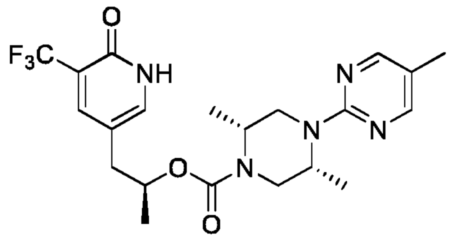

5-Bromo-2-methoxy-3-(trifluoromethyl)pyridine, *tert-butyl* (2R,5R)-2,5-dimethylpiperazine-1-carboxylate, and 2-chloro-5-methylpyrimidine were used as raw materials with the preparation method referring to **intermediate 1C** and **example 48**.

MS (ESI) M/Z: 454.2 $[M+H]^+$.

$^1H$ NMR (400 MHz, $CD_3OD$) δ 8.15 (s, 2H), 7.88 (d, *J* = 2.0 Hz, 1H), 7.50 (d, *J* = 2.0 Hz, 1H), 4.88 - 4.86 (m, 1H), 4.58 (dd, *J* = 14.4, 6.8 Hz, 1H), 4.38 - 4.25 (m, 1H), 4.04 - 3.95 (m, 2H), 3.02 - 2.90 (m, 2H), 2.77 - 2.64 (m, 2H), 2.10 (s, 3H), 1.20 (d, *J* = 5.6 Hz, 3H), 1.14 (d, *J* = 6.0 Hz, 3H), 1.11 (d, *J* = 6.4 Hz, 3H).

**Example 170**

**(*S*)-1-(6-oxo-5-(trifluoromethyl)-1,6-dihydropyridin-3-yl)propan-2-yl (2*S*,5*R*)-2,5-dimethyl-4-(5-methylpyrimidin-2-yl)piperazine-1-carboxylate**

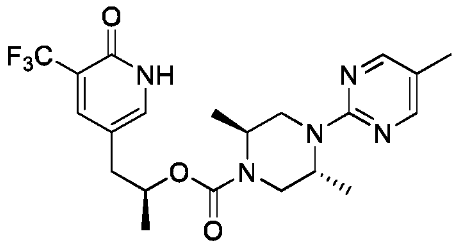

5-Bromo-2-methoxy-3-(trifluoromethyl)pyridine, *tert-butyl* (2S,5R)-2,5-dimethylpiperazine-1-carboxylate, and 2-chloro-5-methylpyrimidine were used as raw materials with the preparation method referring to **intermediate 1C** and **example 48**.

MS (ESI) M/Z: 454.4 $[M+H]^+$.

$^1H$ NMR (400 MHz, DMSO-$d_6$) δ 2.20 (s, 1H), 8.23 (d, *J* = 0.8 Hz, 2H), 7.84 (d, *J* = 25.4 Hz, 1H), 7.55 (d, *J* = 21.6 Hz, 1H), 4.98 - 4.83 (m, 1H), 4.82 - 4.71 (m, 1H), 4.29 - 4.15 (m, 2H), 3.72 - 3.59 (m, 1H), 3.24 - 3.05 (m, 2H), 2.79 - 2.65 (m, 1H), 2.64 - 2.54 (m, 1H), 2.08 (s, 3H), 1.25 - 1.13 (m, 3H), 1.09 - 0.99 (m, 3H), 0.95 (d, *J* = 6.7 Hz, 1.5H), 0.90 (d, *J* = 6.7 Hz, 1.5H).

**Example 171**

**(*S*)-1-(6-oxo-5-(trifluoromethyl)-1,6-dihydropyridin-3-yl)propan-2-yl (2*S*,6*S*)-2,6-dimethyl-4-(5-methylpyrimidin-2-yl)piperazine-1-carboxylate**

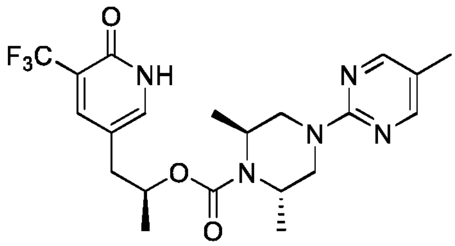

5-Bromo-2-methoxy-3-(trifluoromethyl)pyridine, *tert*-butyl (2*S*,6*S*)-2,6-dimethylpiperazine-1-carboxylate, and 2-chloro-5-methylpyrimidine were used as raw materials with the preparation method referring to **intermediate 1C** and **example 48**.

MS (ESI) M/Z: 454.0 $[M+H]^+$.

$^1H$ NMR (400 MHz, DMSO-$d_6$) δ 8.26 (d, *J* = 0.8 Hz, 2H), 7.79 (d, *J* = 2.5 Hz, 1H), 7.57 (d, *J* = 2.5 Hz, 1H), 4.99 - 4.89 (m, 1H), 4.14 - 4.05 (m, 2H), 4.01 (dd, *J* = 13.6, 1.9 Hz, 2H), 3.54 (dd, *J* = 13.6, 4.0 Hz, 2H), 2.75 (dd, *J* = 14.0, 4.3 Hz, 1H), 2.58 (dd, *J* = 14.2, 7.8 Hz, 1H), 2.10 (s, 3H), 1.20 (d, *J* = 6.3 Hz, 3H), 1.09 (d, *J* = 6.6 Hz, 6H).

**Example 172**

**(*S*)-1-(6-oxo-5-(trifluoromethyl)-1,6-dihydropyridin-3-yl)propan-2-yl (2*R*,5*S*)-2,5-dimethyl-4-(5-methylpyrimidin-2-yl)piperazine-1-carboxylate**

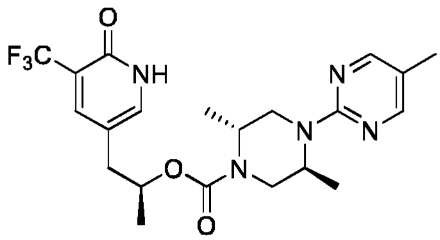

5-Bromo-2-methoxy-3-(trifluoromethyl)pyridine, *tert-butyl* (2S,5R)-2,5-dimethylpiperazine-1-carboxylate, and 2-chloro-5-methylpyrimidine were used as raw materials with the preparation method referring to **intermediate 1C** and **example 48**.

MS (ESI) M/Z: 454.0 $[M+H]^+$.

$^1H$ NMR (400 MHz, DMSO-$d_6$) δ 12.25 (s, 1H), 8.22 (s, 2H), 7.86 (s, 1H), 7.57 (s, 1H), 4.91 - 4.67 (m, 2H), 4.30 - 4.16 (m, 2H), 3.64 (t, *J*= 13.6 Hz, 1H), 3.30 - 3.08 (m, 2H), 2.77 - 2.60 (m, 2H), 2.08 (s, 3H), 1.27 - 1.14 (m, 3H), 1.06 - 1.00 (m, 3H), 0.94 (dd, *J*= 16.4, 6.4 Hz, 3H).

**Example 173**

**(*S*)-1-(6-oxo-5-(trifluoromethyl)-1,6-dihydropyridin-3-yl)propan-2-yl (2*R*,5*S*)-4-(5-cyclopropylpyrimidin-2-yl)-2,5-dimethylpiperazine-1-carboxylate**

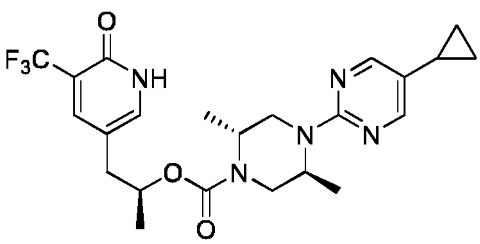

5-Bromo-2-methoxy-3-(trifluoromethyl)pyridine, *tert-butyl* (2R,5S)-2,5-dimethylpiperazine-1-carboxylate, and

2-chloro-5-cyclopropylpyrimidine were used as raw materials with the preparation method referring to **intermediate 1C** and **example 48**.

MS (ESI) M/Z: 480.0 $[M+H]^+$.

$^1$H NMR (400 MHz, DMSO-$d_6$) δ 12.25 (s, 1H), 8.17 (s, 2H), 7.86 (s, 1H), 7.57 (s, 1H), 4.90 - 4.67 (m, 2H), 4.29 - 4.16 (m, 2H), 3.70 - 3.57 (m, 1H), 3.26 - 3.17 (m, 1H), 3.16 - 3.10 (m, 1H), 2.78 - 2.61 (m, 2H), 1.79 - 1.71 (m, 1H), 1.23 - 1.18 (m, 3H), 1.03 (dd, *J* = 6.3, 3.6 Hz, 3H), 0.93 (dd, *J* = 17.2, 6.6 Hz, 3H), 0.88 - 0.83 (m, 2H), 0.65 - 0.59 (m, 2H).

**Example 174**

**(*S*)-1-(6-oxo-5-(trifluoromethyl)-1,6-dihydropyridin-3-yl)propan-2-yl (2*R*,6*S*)-4-(5-cyclopropylpyrimidin-2-yl)-2,6-dimethylpiperazine-1-carboxylate**

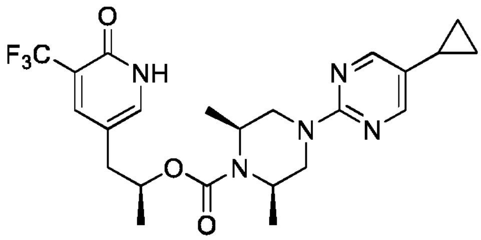

5-Bromo-2-methoxy-3-(trifluoromethyl)pyridine, *tert-butyl* (2R,6S)-2,6-dimethylpiperazine-1-carboxylate, and 2-chloro-5-cyclopropylpyrimidine were used as raw materials with the preparation method referring to **intermediate 1C** and **example 48**.

MS (ESI) M/Z: 479.9 $[M+H]^+$.

$^1$H NMR (400 MHz, DMSO-$d_6$) δ 12.21 (s, 1H), 8.17 (s, 2H), 7.83 (s, 1H), 7.55 (s, 1H), 4.93 - 4.80 (m, 1H), 4.49 (d, *J* = 13.4 Hz, 2H), 4.16 - 4.07 (m, 2H), 3.00 - 2.92 (m, 2H), 2.80 - 2.71 (m, 2H), 1.80 - 1.71 (m, 1H), 1.21 - 1.11 (m, 3H), 1.08 - 0.95 (m, 6H), 0.90 - 0.82 (m, 2H), 0.66 - 0.59 (m, 2H).

**Example 175**

**(*S*)-1-(6-Oxo-5-(trifluoromethyl)-1,6-dihydropyridin-3-yl)propan-2-yl (2*R*,6*R*)-4-(5-cyclopropylpyrimidin-2-yl)-2,6-dimethylpiperazine-1-carboxylate**

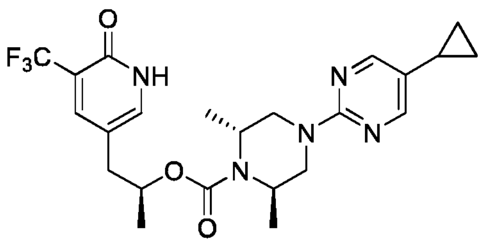

5-Bromo-2-methoxy-3-(trifluoromethyl)pyridine, *tert-butyl* (2*R*,6*R*)-2,6-dimethylpiperazine-1-carboxylate, and 2-chloro-5-cyclopropylpyrimidine were used as raw materials with the preparation method referring to **intermediate 1C** and **example** 48.

MS (ESI) M/Z: 480.4 $[M+H]^+$.

$^1$H NMR (400 MHz, DMSO-$d_6$) δ 12.24 (s, 1H), 8.19 (s, 2H), 7.85 (d, *J* = 2.1 Hz, 1H), 7.56 (d, *J* = 1.7 Hz, 1H), 4.94 - 4.79 (m, 1H), 4.14 - 4.04 (m, 2H), 3.99 (dd, *J* = 13.5, 1.6 Hz, 2H), 3.54 (dd, *J* = 13.6, 3.9 Hz, 2H), 2.74 (dd, *J* = 14.2, 4.5 Hz, 1H), 2.65 (dd, *J* = 14.3, 7.7 Hz, 1H), 1.81 - 1.73 (m, 1H), 1.20 (d, *J* = 6.2 Hz, 3H), 1.10 (d, *J* = 6.6 Hz, 6H), 0.89 - 0.84 (m, 2H), 0.66 - 0.60 (m, 2H).

**Example 176**

**(*S*)-1-(6-oxo-5-(trifluoromethyl)-1,6-dihydropyridin-3-yl)propan-2-yl 4-(5-cyclopropylpyrimidin-2-yl)piperazine-1-carboxylate**

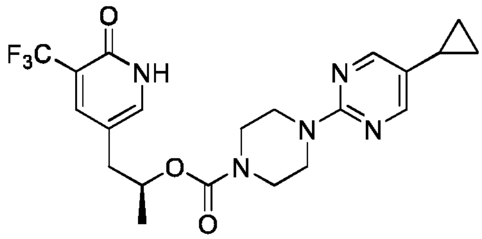

5-Bromo-2-methoxy-3-(trifluoromethyl)pyridine, tert-butyl piperazine-1-carboxylate, and 2-chloro-5-cyclopropylpyrimidine were used as raw materials with the preparation method referring to **intermediate 1C** and **example 48**.
MS (ESI) M/Z: 452.2 $[M+H]^+$.
$^1H$ NMR (400 MHz, DMSO-$d_6$) δ 12.21 (s, 1H), 8.19 (s, 2H), 7.84 (d, *J* = 2.1 Hz, 1H), 7.64 - 7.46 (m, 1H), 4.91 - 4.72 (m, 1H), 3.83 - 3.46 (m, 4H), 3.44 - 3.34 (m, 4H), 2.74 - 2.57 (m, 2H), 1.83 - 1.71 (m, 1H), 1.19 (d, *J* = 6.2 Hz, 3H), 0.90 - 0.83 (m, 2H), 0.66 - 0.60 (m, 2H).

**Example 177**

**(*S*)-1-(6-oxo-5-(trifluoromethyl)-1,6-dihydropyridin-3-yl)propan-2-yl (2*S*,6*S*)-4-(5-cyclopropylpyrimidin-2-yl)-2,6-dimethylpiperazine-1-carboxylate**

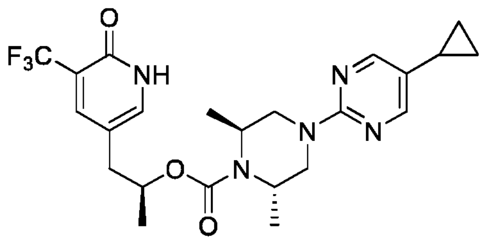

5-Bromo-2-methoxy-3-(trifluoromethyl)pyridine, *tert*-butyl (2*S*,6*S*)-2,6-dimethylpiperazine-1-carboxylate, and 2-chloro-5-cyclopropylpyrimidine were used as raw materials with the preparation method referring to **intermediate 1C** and **example 48**.
MS (ESI) M/Z: 480.8 $[M+H]^+$.
$^1H$ NMR (400 MHz, DMSO-$d_6$) δ 12.12 (s, 1H), 8.20 (s, 2H), 7.83 (d, *J* = 2.4 Hz, 1H), 7.55 (s, 1H), 5.03 - 4.88 (m, 1H), 4.13 - 4.03 (m, 2H), 4.00 (d, *J* = 13.6, 2H), 3.54 (dd, *J* = 13.6, 4.0 Hz, 2H), 2.76 (dd, *J* = 14.1, 4.3 Hz, 1H), 2.58 (dd, *J* = 14.1, 7.9 Hz, 1H), 1.88 - 1.69 (m, 1H), 1.20 (d, *J* = 6.3 Hz, 3H), 1.08 (d, *J* = 6.6 Hz, 6H), 0.92 - 0.83 (m, 2H), 0.68 - 0.60 (m, 2H).

**Example 178**

**(*S*)-1-(6-oxo-5-(trifluoromethyl)-1,6-dihydropyridin-3-yl)propan-2-yl (2*S*,5*R*)-4-(5-cyclopropylpyrimidin-2-yl)-2,5-dimethylpiperazine-1-carboxylate**

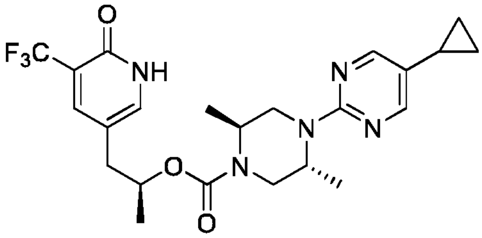

5-Bromo-2-methoxy-3-(trifluoromethyl)pyridine, *tert-butyl* (2S,5R)-2,5-dimethylpiperazine-1-carboxylate, and 2-chloro-5-cyclopropylpyrimidine were used as raw materials with the preparation method referring to **intermediate 1C** and **example 48**.

MS (ESI) M/Z: 480.2 $[M+H]^+$.

$^1H$ NMR (400 MHz, DMSO-$d_6$) δ 12.21 (s, 1H), 8.18 (s, 2H), 7.87 (s, 0.5H), 7.81 (s, 0.5H), 7.58 (s, 0.5H), 7.53 (s, 0.5H), 5.00 - 4.83 (m, 1H), 4.82 - 4.69 (m, 1H), 4.32 - 4.15 (m, 2H), 3.73 - 3.58 (m, 1H), 3.23 - 3.09 (m, 2H), 2.81 - 2.54 (m, 2H), 1.82 - 1.68 (m, 1H), 1.19 (t, *J*= 5.2 Hz, 3H), 1.07 - 0.99 (m, 3H), 0.98 - 0.84 (m, 5H), 0.69 - 0.57 (m, 2H).

**Example** 179

**(*S*)-1-(6-oxo-5-(trifluoromethyl)-1,6-dihydropyridin-3-yl)propan-2-yl (2*R*,5*R*)-4-(5-cyclopropylpyrimidin-2-yl)-2,5-dimethylpiperazine-1-carboxylate**

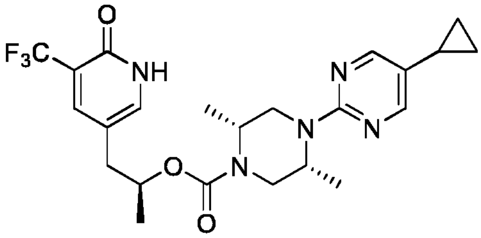

5-Bromo-2-methoxy-3-(trifluoromethyl)pyridine, *tert-butyl* (2*R*,5*R*)-2,5-dimethylpiperazine-1-carboxylate, and 2-chloro-5-cyclopropylpyrimidine were used as raw materials with the preparation method referring to **intermediate 1C** and **example 48**.

MS (ESI) M/Z: 480.2 $[M+H]^+$.

$^1H$ NMR (400 MHz, $CD_3OD$) δ 8.10 (s, 2H), 7.87 (d, *J* = 2.0 Hz, 1H), 7.50 (d, *J* = 2.4 Hz, 1H), 4.88 - 4.81 (m, 1H), 4.64 - 4.54 (m, 1H), 4.37 - 4.25 (m, 1H), 4.06 - 3.94 (m, 2H), 3.03 - 2.91 (m, 2H), 2.77 - 2.63 (m, 2H), 1.80 - 1.69 (m, 1H), 1.25 - 1.16 (m, 3H), 1.16 - 1.07 (m, 6H), 0.95 - 0.86 (m, 2H), 0.65 - 0.57 (m, 2H).

**Example 180**

**2-(6-Oxo-5-(trifluoromethyl)-1,6-dihydropyridin-3-yl)ethyl (2*R*,5*R*)-4-(5-cyclopropylpyrimidin-2-yl)-2,5-dimethylpiperazine-1-carboxylate**

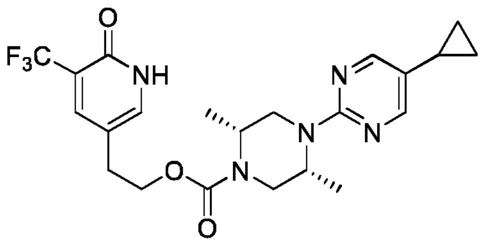

5-Bromo-2-methoxy-3-(trifluoromethyl)pyridine, *tert-butyl* (2R,5R)-2,5-dimethylpiperazine-1-carboxylate, and

2-chloro-5-cyclopropylpyrimidine were used as raw materials with the preparation method referring to **intermediate 1C** and **example 109**.

MS (ESI) M/Z: 466.8 $[M+H]^+$.

$^1H$ NMR (400 MHz, $CD_3OD$) δ 8.10 (s, 2H), 7.91 (d, *J* = 2.0 Hz, 1H), 7.52 (d, *J* = 2.4 Hz, 1H), 4.61 (dd, *J* = 14.4, 6.4 Hz, 1H), 4.38 - 4.28 (m, 1H), 4.23 - 4.13 (m, 2H), 4.05 - 3.90 (m, 2H), 3.04 - 2.92 (m, 2H), 2.77 (t, *J* = 6.4 Hz, 2H), 1.78- 1.69 (m, 1H), 1.14 (d, *J* = 6.0 Hz, 3H), 1.11 (d, *J* = 6.0 Hz, 3H), 0.93 - 0.86 (m, 2H), 0.63 - 0.56 (m, 2H).

**Example 181**

**2-(6-Oxo-5-(trifluoromethyl)-1,6-dihydropyridin-3-yl)ethyl *(3S,SR)-4-(5*-cyclopropylpyrimidin-2-yl)-3,5-dimethyl-piperazine-1-carboxylate**

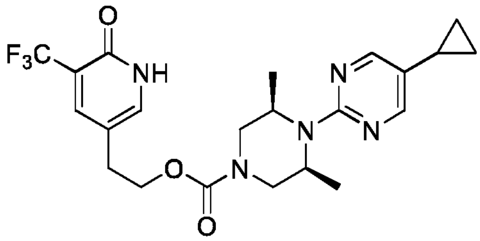

5-Bromo-2-methoxy-3-(trifluoromethyl)pyridine, *tert-butyl* (3*S*,5*R*)-3,5-dimethylpiperazine-1-carboxylate, and 2-chloro-5-cyclopropylpyrimidine were used as raw materials with the preparation method referring to **intermediate 1C** and **example** 109.

MS (ESI) M/Z: 466.6 $[M+H]^+$.

$^1H$ NMR (400 MHz, DMSO-$d_6$) δ 12.29 (brs, 1H), 8.20 (s, 2H), 7.90 (d, *J* = 2.5 Hz, 1H), 7.60 (d, *J* = 2.5 Hz, 1H), 4.64 (brs, 2H), 4.26 - 4.16 (m 2H), 3.94 - 3.76 (m, 2H), 3.06 - 3.01 (m, 2H), 2.76 - 2.73 (m, 2H), 1.85 - 1.70 (m, 1H), 1.01 (s, 6H), 0.88 - 0.85 (m, 2H), 0.64 - 0.62 (m, 2H).

**Example 182**

**2-(6-Oxo-5-(trifluoromethyl)-1,6-dihydropyridin-3-yl)ethyl (2*S*,6*S*)-4-(5-cyclopropylpyrimidin-2-yl)-2,6-dimethyl-piperazine-1-carboxylate**

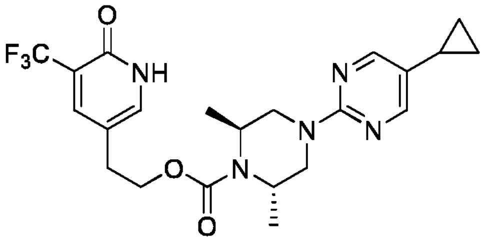

5-Bromo-2-methoxy-3-(trifluoromethyl)pyridine, *tert*-butyl (2*S*,6*S*)-2,6-dimethylpiperazine-1-carboxylate, and 2-chloro-5-cyclopropylpyrimidine were used as raw materials with the preparation method referring to **intermediate 1C** and **example 109**.

MS (ESI) M/Z: 466.0 $[M+H]^+$.

$^1H$ NMR (400 MHz, DMSO-$d_6$) δ 12.22 (s, 1H), 8.20 (s, 2H), 7.90 (d, *J* = 2.4 Hz, 1H), 7.60 (s, 1H), 4.24 - 4.14 (m, 2H), 4.13 - 4.05 (m, 2H), 4.04 - 3.97 (m, 2H), 3.56 (dd, *J* = 13.6, 4.0 Hz, 2H), 2.75 (t, *J* = 6.2 Hz, 2H), 1.88 - 1.71 (m, 1H), 1.11 (d, *J* = 6.6 Hz, 6H), 0.95 - 0.81 (m, 2H), 0.70 - 0.57 (m, 2H).

**Example 183**

**2-(6-Oxo-5-(trifluoromethyl)-1,6-dihydropyridin-3-yl)ethyl (2*S*,5*R*)-4-(5-cyclopropylpyrimidin-2-yl)-2,5-dimethylpiperazine-1-carboxylate**

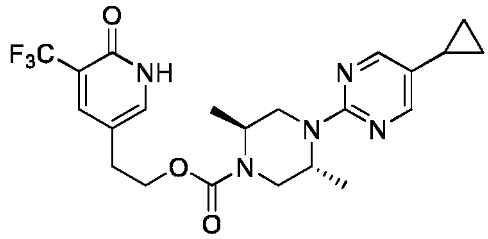

5-Bromo-2-methoxy-3-(trifluoromethyl)pyridine, *tert-butyl* (2*S*,5*R*)-2,5-dimethylpiperazine-1-carboxylate, and 2-chloro-5-cyclopropylpyrimidine were used as raw materials with the preparation method referring to **intermediate 1C** and **example 109**.

MS (ESI) M/Z: 466.0 $[M+H]^+$.

$^1H$ NMR (400 MHz, DMSO-$d_6$) δ 12.24 (s, 1H), 8.19 (s, 2H), 7.90 (d, *J* = 5.6 Hz, 1H), 7.58 (s, 1H), 4.87 - 4.67 (m, 1H), 4.31 - 4.08 (m, 4H), 3.71 - 3.55 (m, 1H), 3.29 - 3.11 (m, 2H), 2.78 - 2.69 (m, 2H), 1.84 - 1.67 (m, 1H), 1.09 - 0.94 (m, 6H), 0.92 - 0.82 (m, 2H), 0.70 - 0.55 (m, 2H).

**Example 184**

**2-(6-Oxo-5-(trifluoromethyl)-1,6-dihydropyridin-3-yl)ethyl (2*R*,5*S*)-4-(5-cyclopropylpyrimidin-2-yl)-2,5-dimethylpiperazine-1-carboxylate**

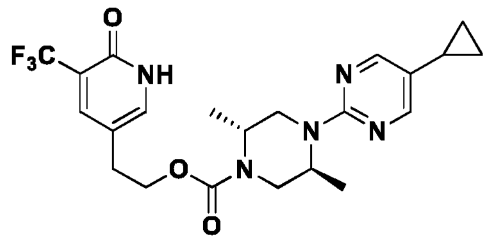

5-Bromo-2-methoxy-3-(trifluoromethyl)pyridine, *tert-butyl* (2R,5S)-2,5-dimethylpiperazine-1-carboxylate, and 2-chloro-5-cyclopropylpyrimidine were used as raw materials with the preparation method referring to **intermediate 1C** and **example 109**.

MS (ESI) M/Z: 466.0 $[M+H]^+$.

$^1H$ NMR (400 MHz, DMSO-$d_6$) δ 12.22 (s, 1H), 8.17 (s, 2H), 7.89 (d, *J* = 5.6 Hz, 1H), 7.58 (s, 1H), 4.85 - 4.65 (m, 1H), 4.36 - 4.05 (m, 4H), 3.73 - 3.56 (m, 1H), 3.25 - 3.13 (m, 2H), 2.78 - 2.69 (m, 2H), 1.80 - 1.69 (m, 1H), 1.09 - 0.90 (m, 6H), 0.90 - 0.78 (m, 2H), 0.66 - 0.58 (m, 2H).

**Example 185**

**2-(6-Oxo-5-(trifluoromethyl)-1,6-dihydropyridin-3-yl)ethyl (2*R*,6*R*)-4-(5-cyclopropylpyrimidin-2-yl)-2,6-dimethylpiperazine-1-carboxylate**

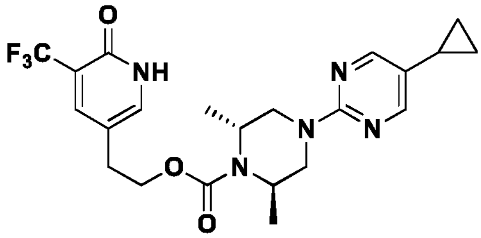

5-Bromo-2-methoxy-3-(trifluoromethyl)pyridine, *tert-butyl* (2*R*,6*R*)-2,6-dimethylpiperazine-1-carboxylate, and 2-chloro-5-cyclopropylpyrimidine were used as raw materials with the preparation method referring to **intermediate 1C** and **example 109**.

MS (ESI) M/Z: 466.0 $[M+H]^+$.

$^1H$ NMR (400 MHz, DMSO-$d_6$) δ 12.22 (s, 1H), 8.19 (s, 2H), 7.89 (d, *J* = 2.1 Hz, 1H), 7.59 (s, 1H), 4.21 - 4.13 (m, 2H), 4.11 - 4.06 (m, 2H), 3.99 (dd, *J* = 13.6, 1.7 Hz, 2H), 3.54 (dd, *J* = 13.5, 3.9 Hz, 2H), 2.74 (t, *J* = 6.2 Hz, 2H), 1.80 - 1.71 (m, 1H), 1.10 (d, *J* = 6.6 Hz, 6H), 0.89 - 0.84 (m, 2H), 0.66 - 0.60 (m, 2H).

**Example 186**

**2-(6-Oxo-5-(trifluoromethyl)-1,6-dihydropyridin-3-yl)ethyl (2*S*,6*R*)-4-(5-cyclopropylpyrimidin-2-yl)-2,6-dimethylpiperazine-1-carboxylate**

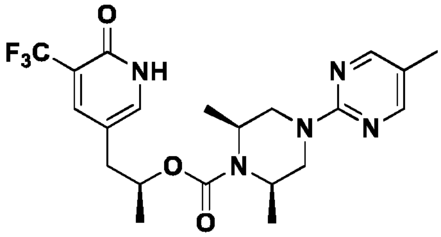

5-Bromo-2-methoxy-3-(trifluoromethyl)pyridine, *tert-butyl* (2S,6R)-2,6-dimethylpiperazine-1-carboxylate, and 2-chloro-5-methylpyrimidine were used as raw materials with the preparation method referring to intermediate 1C and example 109.

MS (ESI) M/Z: 454.0 $[M+H]^+$.

$^1H$ NMR (400 MHz, DMSO-$d_6$) δ 12.23 (s, 1H), 8.22 (s, 2H), 7.83 (d, *J* = 2.1 Hz, 1H), 7.56 (s, 1H), 4.92 - 4.86 (m, 1H), 4.50 (d, *J* = 13.4 Hz, 2H), 4.15 - 4.10 (m, 2H), 3.02 - 2.91 (m, 2H), 2.74 (dd, *J* = 14.2, 4.3 Hz, 1H), 2.61 (dd, *J* = 14.2, 7.9 Hz, 1H), 2.08 (s, 3H), 1.19 (d, *J* = 6.2 Hz, 3H), 1.03 (t, *J* = 6.7 Hz, 6H).

**Biological test evaluation:**

**I. *In vitro* enzymology experiment of PARP1**

In this experiment, the inhibitory effect of the test compound on PARP1 enzyme activity was detected by gradient dilution of the test compound as well as a duplicate well assay.

1. Preparation of 384-well histone-coated plate

25 μL of histone solution was added to each well, and the plate was incubated overnight at 4°C.

2. Preparation of PBST buffer, blocking buffer, and assay buffer

3. The 384-well histone-coated plate was washed three times with PBST buffer. The plate was blocked by 50 μL of blocking buffer for 1 hour at room temperature. The plate was then washed three times with PBST buffer.

4. Compound preparation:

1000x compound was prepared in a 96-well source plate. 1 μL of the compound was transferred from the source plate to a 96-well intermediate plate. The plate was shaken and centrifuged at 1000 rpm for 1 minute.
5 μL of DMSO solution was transferred to each well.

5. Enzymatic reaction:

A mixture of PARP1 & DNA was incubated at 25°C for 10 minutes.
10 μL of PARP1 & DNA (minimum control required) was added, and incubated with the compound at room temperature for 10 minutes. 10 μL of DNA was then added to the negative control well.
10 μL of 2.5× NAD+ was added to each well, and the plate was incubated at 25°C for 60 minutes. The plate was then washed three times with PBST buffer.

6. Detection:

20 μL of anti-poly/mono-ADP ribose rabbit mAb (Cell Signaling Technology, Cat. No.: 7074P2) was added.
The plate was incubated at room temperature for 1.5 hours and washed three times with PBST buffer.
20 μL of IgG HRP-linked antibody (anti-rabbit IgG, HRP-linked antibody) diluted at a ratio of 1:2000 was added.
The plate was incubated at room temperature for 1 hour and then washed three times with PBST buffer.
7. 25 μL of a mixture of Femto-ECL Substrate A and Femto-ECL Substrate B (1: 1) was added.
8. Chemiluminescent detection was performed immediately on an Envision instrument.

9. Data processing

Using equation (1) to fit the data in Excel to obtain inhibition values

$$\text{Equation (1): inh \%} = (\text{Max - Signal}) / (\text{Max - Min}) * 100$$

Using equation (2) to fit the data in XL-Fit to obtain $IC_{50}$ values

$$\text{Equation (2): } Y = \text{Bottom} + (\text{Top} - \text{Bottom}) / (1 + (IC_{50} / X) * \text{HillSlope})$$

Y is the percentage of inhibition, and X is the compound concentration.

**II. *In vitro* enzymology experiment of PARP2, 3, 5A, 5B, 7, 12, and 15**

In this experiment, the inhibitory effect of the test compound on PARP2, 3, 5A, 5B, 7, 12, and 15 enzyme activities was detected by gradient dilution of the test compound as well as a duplicate well assay.

1. Preparation of 384-well histone-coated plate: 25 μL of histone solution was added to each well, and the plate was incubated overnight at 4°C.
2. Preparation of PBST buffer, blocking buffer, and assay buffer
3. The 384-well histone-coated plate was washed three times with PBST buffer. The plate was blocked by 50 μL of blocking buffer for 1 hour at room temperature. The plate was then washed three times with PBST buffer.
4. Compound preparation:

    2000x compound was prepared in a 96-well source plate. 50 nL of the compound was transferred from the source plate to a 96-well intermediate plate, and 39.95 μL of assay buffer was added to each well. The plate was shaken well and centrifuged at 1000 rpm for 1 minute.

    5 μL of compound DMSO solution was transferred to each well.

5. Enzymatic reaction:

An enzyme mixture was incubated at 25°C for 10 minutes.

10 μL of the enzyme mixture was added, and incubated with the compound at room temperature for 10 minutes.
10 μL of assay buffer was added to the negative control well of the assay plate.
10 μL of 2.5x Biotin-NAD+ was added to each well, and the plate was incubated at 25°C for 60 minutes.
The plate was then washed three times with PBST buffer.

6. Detection:

25 μL of Stre-HRP was added.
The plate was incubated at room temperature for 1 hour and washed three times with PBS buffer.
25 μL of QuantaRed Enhancer mix was added. The plate was incubated for 10 minutes.
2.5 μL of QuantaRed Stop Solution was added to terminate the peroxidase reaction, and the plate was shaken for 10 to 30 seconds.
7. Paradigm was used to read the plate immediately to detect the readings at Ex550/Em620.

8. Data processing

Using equation (1) to fit the data in Excel to obtain inhibition values

$$\text{Equation (1): inh \%} = (\text{Max} - \text{Signal}) / (\text{Max} - \text{Min}) * 100$$

Using equation (2) to fit the data in XL-Fit to obtain $IC_{50}$ values

$$\text{Equation (2): } Y = \text{Bottom} + (\text{Top} - \text{Bottom}) / (1 + (IC_{50} / X) * \text{HillSlope})$$

Y is the percentage of inhibition, and X is the compound concentration.
The results are shown in Table 1. Conclusion: The representative compounds of the present disclosure can effectively inhibit PARP7 enzyme activity, and some of the compounds have good selectivity for PARP1, 2, 3, 5A, 5B, 12, and 15.

Table 1: PARP7 enzymatic inhibition results

| **Example** | PARP7 $IC_{50}$ (nM) |
|---|---|
| 3 | 1.8 |
| 5 | 5.83 |
| 7 | 2.79 |
| 9 | 7.6 |
| 10 | 6.7 |
| 13 | 5.98 |
| 17 | 2.79 |
| 22 | 3.6 |
| 25 | 0.8 |
| 26 | 0.6 |
| 27 | 1.1 |
| 28 | 0.9 |
| 29 | 5.6 |
| 30 | 0.9 |
| 31 | 1.19 |
| 32 | 0.44 |

(continued)

| **Example** | PARP7 $IC_{50}$ (nM) |
|---|---|
| 33 | 0.9 |
| 35 | 0.53 |
| 36 | 0.38 |
| 37 | 0.9 |
| 38 | 1.1 |
| 39 | 1.5 |
| 40 | 0.7 |
| 41 | 1.1 |
| 42 | 1.46 |
| 43 | 0.9 |
| 44 | 0.8 |
| 45 | 2.26 |
| 46 | 1.94 |
| 47 | 7.22 |
| 49 | 0.76 |
| 50 | 0.52 |
| 51 | 1.34 |
| 52 | 1.18 |
| 53 | 2.3 |
| 54 | 6.23 |
| 55 | 3.42 |
| 56 | 2.27 |
| 57 | 0.74 |
| 58 | 3.18 |
| 59 | 1.06 |
| 60 | 7.02 |
| 61 | 0.72 |
| 62 | 2.09 |
| 63 | 3.12 |
| 64 | 1.99 |
| 65 | 0.65 |
| 66 | 1.82 |
| 67 | 0.76 |
| 68 | 1.11 |
| 70 | 0.91 |
| 72 | 4.98 |
| 73 | 0.75 |
| 75 | 0.83 |

(continued)

| **Example** | PARP7 $IC_{50}$ (nM) |
|---|---|
| 76 | 0.67 |
| 77 | 2.15 |
| 78 | 1.4 |
| 79 | 0.68 |
| 80 | 1.5 |
| 81 | 0.75 |
| 82 | 1.44 |
| 83 | 1.38 |
| 84 | 0.52 |
| 85 | 0.56 |
| 86 | 0.6 |
| 87 | 0.5 |
| 88 | 1.98 |
| 90 | 1.08 |
| 91 | 0.78 |
| 92 | 0.19 |
| 93 | 0.78 |
| 94 | 0.68 |
| 95 | 0.72 |
| 96 | 1.03 |
| 97 | 0.86 |
| 98 | 0.83 |
| 99 | 0.56 |
| 100 | 0.61 |
| 101 | 0.67 |
| 102 | 0.95 |
| 104 | 0.53 |
| 105 | 0.92 |
| 106 | 0.46 |
| 107 | 0.72 |
| 108 | 0.63 |
| 109 | 0.38 |
| 110 | 0.65 |
| 111 | 0.61 |
| 112 | 0.62 |
| 113 | 0.65 |
| 114 | 0.53 |
| 115 | 1.0 |

(continued)

| **Example** | PARP7 $IC_{50}$ (nM) |
|---|---|
| 116 | 0.81 |
| 117 | 0.62 |
| 118 | 0.93 |
| 119 | 0.24 |
| 120 | 0.66 |
| 121 | 0.54 |
| 122 | 0.69 |
| 123 | 0.87 |
| 124 | 0.48 |
| 125 | 0.71 |
| 126 | 1.0 |
| 127 | 0.49 |
| 128 | 0.81 |
| 129 | 0.39 |
| 130 | 0.77 |
| 131 | 1.3 |
| 132 | 0.78 |
| 133 | 0.76 |
| 134 | 1.92 |
| 135 | 2.78 |
| 136 | 3.53 |
| 137 | 2.4 |
| 138 | 1.8 |
| 139 | 0.90 |
| 140 | 2.86 |
| 141 | 0.85 |
| 142 | 0.74 |
| 143 | 0.61 |
| 144 | 1.5 |
| 145 | 2.3 |
| 146 | 0.64 |
| 147 | 0.74 |
| 148 | 0.77 |
| 149 | 0.64 |
| 150 | 0.71 |
| 151 | 1.1 |
| 152 | 0.76 |
| 153 | 0.65 |

(continued)

| Example | PARP7 $IC_{50}$ (nM) |
|---|---|
| 154 | 1.1 |
| 155 | 0.59 |
| 156 | 0.98 |
| 157 | 0.90 |
| 158 | 5.0 |
| 159 | 0.90 |
| 160 | 1.3 |
| 161 | 0.91 |
| 162 | 1.2 |
| 163 | 0.75 |
| 164 | 5.6 |
| 165 | 1.0 |
| 166 | 0.65 |
| 167 | 1.5 |
| 168 | 0.90 |
| 169 | 0.70 |
| 170 | 0.87 |
| 171 | 1.4 |
| 172 | 0.84 |
| 173 | 0.74 |
| 174 | 1.3 |
| 175 | 0.76 |
| 176 | 1.7 |
| 177 | 0.78 |
| 178 | 1.4 |
| 179 | 1.0 |
| 180 | 1.0 |
| 181 | 1.3 |
| 182 | 5.5 |
| 183 | 1.0 |
| 184 | 1.2 |

Conclusion: The representative compounds of the present disclosure can effectively inhibit PARP7 enzyme activity.

**III. *In vivo* pharmacokinetic assay in mice and rats**

1. Experimental purpose:

The pharmacokinetic behavior of the compound of the present disclosure in plasma after a single intravenous

injection and oral administration was studied using male C57BL/6 mice or SD male rats as experimental animals.

2. Experimental scheme

2.1 Experimental animals

Healthy adult C57BL/6 mice or SD rats (3 mice or rats per group), male, supplied by Shanghai Jihui Laboratory Animal Care Co., Ltd. and Vital River Laboratory Animal Technology Co., Ltd.

2.2 Administration

Both the intravenous injection and oral administration groups consisted of 3 mice or rats. The intravenous injection was at a dose of 1 mg/kg and in a volume of 5 mL/kg; the oral administration was at a dose of 5 mg/kg and in a volume of 10 mL/kg. The vehicle for administration was 5% DMSO/5% Kolliphor HS 15/90% Saline.

2.3 Experimental equipment

The centrifuge was purchased from Eppendorf, and the pipette was purchased from Eppendorf.

2.4 Sample collection

After the animals were administered, 0.02 mL of blood was collected from the vein at 0.0833 (IV), 0.25, 0.5, 1, 2, 4, 8, and 24 hours, respectively, placed in an EDTA-K2 test tube, centrifuged at 4600 rpm for 5 minutes at 4°C to separate the plasma, and stored at - 80°C.

2.5 Sample processing

1) 10 μL of plasma sample was precipitated by adding 200 μL of acetonitrile, vortexed to mix, and centrifuged for 15 minutes.
2) The treated supernatant was diluted with water, and the concentration of the test compound was analyzed by LC/MS/MS.

2.6 Bioanalysis

Liquid phase conditions: Shimadzu LC-30AD
Mass spectrometry conditions: AB Sciex API 5500
Chromatographic column: Phenomenex Kinetex 2.6 μm C18
Mobile phases: A: 5 mM ammonium acetate aqueous solution (containing 0.05% formic acid); B: acetonitrile (containing 0.1% formic acid); flow rate: 0.5 mL/min

Elution gradient:

| Time (min) | A (%) | B (%) |
|---|---|---|
| 0.00 | 85.0 | 10.0 |
| 0.50 | 85.0 | 10.0 |
| 2.00 | 5.00 | 95.0 |
| 2.20 | 5.00 | 95.0 |
| 2.21 | 85.0 | 10.0 |

(continued)

| Time (min) | A (%) | B (%) |
|---|---|---|
| 2.50 | 85.0 | 10.0 |

3. Experimental results and analysis

Pharmacokinetic parameters were calculated using WinNonlin 8.0, and the pharmacokinetic parameters for intravenous injection and oral administration in mice or rats are shown in Table 2 and Table 3 below:

Table 2: Pharmacokinetic parameters for intravenous injection of representative compounds of the present disclosure in mice or rats

| Example | Animal | Dose (mg/kg) | CL (L/hr/kg) | $V_{ss}$ (L/kg) | $T_{1/2}$ (hr) | AUC (hr*ng/ mL ) |
|---|---|---|---|---|---|---|
| RBN-2397 | C57BL/6 mice | 1 | 3.4 | 0.6 | 0.2 | 288 |
| | SD rats | 1 | 5.1 | 1.2 | 0.2 | 193 |
| Example **3** | C57BL/6 mice | 1 | 0.09 | 0.6 | 6.1 | 10408 |
| | SD rats | 1 | 0.2 | 0.9 | 4.7 | 5745 |
| Example **35** | C57BL/6 mice | 1 | 0.4 | 1.5 | 4.2 | 2573 |
| | SD rats | 1 | 1.7 | 3.3 | 3.5 | 633 |
| Example **93** | SD rats | 1 | 1.0 | 1.9 | 1.9 | 978 |
| Example **109** | C57BL/6 mice | 1 | 0.1 | 0.6 | 6.3 | 8096 |
| | SD rats | 1 | 0.9 | 2.1 | 2.3 | 1056 |

Table 3: Pharmacokinetic parameters for oral administration of representative compounds of the present disclosure in mice or rats

| Example number | Animal | Dose (mg/kg) | $C_{max}$ (ng/mL) | AUC (hr*ng/mL) | F (%) |
|---|---|---|---|---|---|
| RBN-2397 | C57BL/6 mice | 5 | 612 | 203 | 14 |
| | SD rats | 5 | 319 | 264 | 27 |
| Example **3** | C57BL/6 mice | 5 | 9313 | 42155 | 80 |
| | SD rats | 5 | 2350 | 13798 | 47 |
| Example **35** | C57BL/6 mice | 5 | 3750 | 6759 | 52 |
| | SD rats | 5 | 434 | 1059 | 35 |
| Example **93** | SD rats | 5 | 809 | 3833 | 77 |
| Example **109** | C57BL/6 mice | 5 | 8493 | 29551 | 72 |
| | SD rats | 5 | 1313 | 5384 | 99 |

Conclusion: At the same dose, the representative compounds of the present disclosure show significantly higher plasma exposure in mice and rats after intravenous injection and oral administration than the control compound RBN-2397.

The control compound RBN-2397 has a structure as follows:

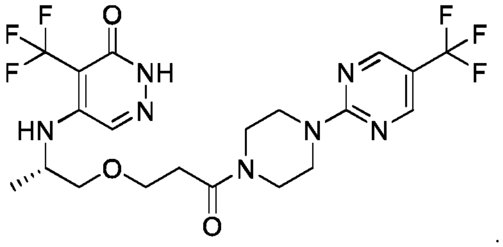

## Claims

**1.** A compound of formula (I) or a pharmaceutically acceptable salt thereof,

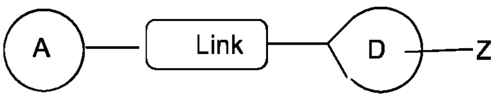
(I)

wherein

ring A is selected from the following groups:

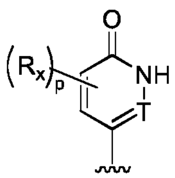 (A-1); 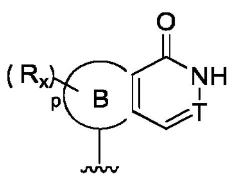 (A-2); or 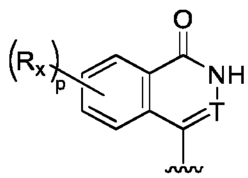 (A-3);

T is selected from CH or N;
ring B is selected from $C_{6-10}$ aryl, $C_{3-7}$ cycloalkyl, 5- to 10-membered heteroaryl, and 4-to 10-membered heterocyclyl;
ring D is 4- to 10-membered heterocyclyl, and the 4- to 10-membered heterocyclyl is optionally substituted by p substituents independently selected from $R_y$;
Z is selected from H, $Cy^2$, -NH-$Cy^2$, halogen, $C_{1-6}$ alkyl, $C_{3-7}$ cycloalkylacyl, $C_{1-6}$ alkoxy, $C_{1-6}$ haloalkyl, CN, and $NO_2$; wherein $Cy^2$ is selected from $C_{6-10}$ aryl, $C_{3-7}$ cycloalkyl, 5- to 10-membered heteroaryl, and 4- to 10-membered heterocyclyl, and each of the $Cy^2$ is optionally substituted by p substituents independently selected from $R_z$;
$R_x$ and $R_z$ are selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, CN, $NO_2$, $NH_2$, $C_{3-7}$ cycloalkyl, hydroxyl-$C_{1-6}$ alkyl-, $C_{1-6}$ alkyl-C(O)-, $C_{1-6}$ alkyl-$S(O)_2$-, and $C_{1-6}$ alkyl-NH-C(O)-;
$R_y$ is selected from hydrogen and $C_{1-6}$ alkyl; the $C_{1-6}$ alkyl is optionally substituted by OH, CN, and $OCH_3$;
Link is a group linking ring A and ring D;
p is an integer selected from 0, 1, and 2.

**2.** A compound of formula (I) or a pharmaceutically acceptable salt thereof,

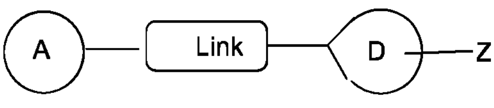
(I)

wherein

ring A is selected from the following groups:

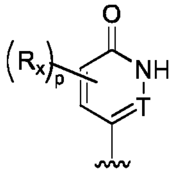 (A-1); or 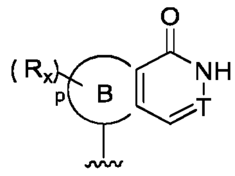 (A-2);

T is selected from CH or N;

ring B is selected from $C_{6-10}$ aryl, $C_{3-7}$ cycloalkyl, 5- to 10-membered heteroaryl, and 4-to 10-membered heterocyclyl;

ring D is 4- to 10-membered heterocyclyl, and the 4- to 10-membered heterocyclyl is optionally substituted by p substituents independently selected from $R_x$;

Z is selected from H, $Cy^2$, halogen, $C_{1-6}$ alkyl, $C_{1-6}$ alkoxy, $C_{2-6}$ alkenyl, $C_{2-6}$ alkynyl, $C_{1-6}$ haloalkyl, CN, and $NO_2$; wherein $Cy^2$ is selected from $C_{6-10}$ aryl, $C_{3-7}$ cycloalkyl, 5- to 10-membered heteroaryl, and 4- to 10-membered heterocyclyl, and each of the $Cy^2$ is optionally substituted by p substituents independently selected from $R_x$;

$R_x$ is selected from hydrogen, halogen, $C_{1-6}$ alkyl, $C_{1-6}$ alkoxy, $C_{2-6}$ alkenyl, $C_{2-6}$ alkynyl, $C_{1-6}$ haloalkyl, CN, and $NO_2$;

Link is a group linking ring A and ring D;

p is an integer selected from 0, 1, and 2.

3. The compound of formula (I) or the pharmaceutically acceptable salt thereof according to claim 1 or 2, wherein $R_x$ is selected from hydrogen, halogen, $C_{1-6}$ alkyl, and $C_{1-6}$ haloalkyl.

4. The compound of formula (I) or the pharmaceutically acceptable salt thereof according to claim 3, wherein $R_x$ is selected from H, F, $CH_3$, and $CF_3$.

5. The compound of formula (I) or the pharmaceutically acceptable salt thereof according to claim 1, wherein $R_x$ is selected from H, F, Cl, Br, $CH_3$, $CF_3$, $NH_2$, $-CHF_2$, $C_2H_5$, CN,

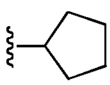, 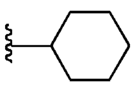, 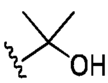, 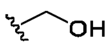, 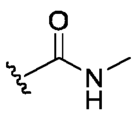, 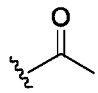, and 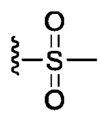.

6. The compound of formula (I) or the pharmaceutically acceptable salt thereof according to claim 1, wherein $R_z$ is selected from hydrogen, halogen, $C_{1-6}$ alkyl, $C_{1-6}$ haloalkyl, CN, $NH_2$, $C_{3-7}$ cycloalkyl, hydroxyl-$C_{1-6}$ alkyl-, $C_{1-6}$ alkyl-C(O)-, $C_{1-6}$ alkyl-$S(O)_2$-, and $C_{1-6}$ alkyl-NH-C(O)-.

7. The compound of formula (I) or the pharmaceutically acceptable salt thereof according to claim 6, wherein $R_z$ is selected from H, F, Cl, Br, $CH_3$, $CF_3$, $NH_2$, $-CHF_2$, $C_2H_5$, CN,

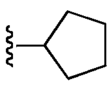, 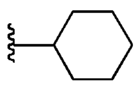, 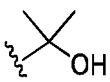, 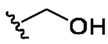, 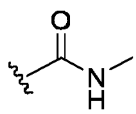, 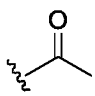, 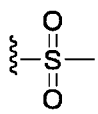, and 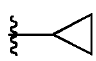.

8. The compound of formula (I) or the pharmaceutically acceptable salt thereof according to claim 1, wherein $R_y$ is selected from H, F, Cl, Br, $CH_3$, $CF_3$, $-CH_2CH_3$, $-CH_2OH$, $-CH_2CN$, and $-CH_2OCH_3$.

9. The compound of formula (I) or the pharmaceutically acceptable salt thereof according to claim 8, wherein $R_y$ is selected from H, $CH_3$, $-CH_2CH_3$, $-CH_2OH$, $-CH_2CN$, and $-CH_2OCH_3$.

10. The compound of formula (I) or the pharmaceutically acceptable salt thereof according to claim 1, wherein Z is selected from $Cy^2$, -NH-$Cy^2$, and $C_{3-7}$ cycloalkylacyl; wherein $Cy^2$ is selected from $C_{6-10}$ aryl and 5- to 10-membered heteroaryl, and each of the $Cy^2$ is optionally substituted by p substituents independently selected from $R_z$;

p is as defined in claim 1;

$R_z$ is as defined in claim 1, 6, or 7.

**11.** The compound of formula (I) or the pharmaceutically acceptable salt thereof according to claim 1, wherein

Z is selected from

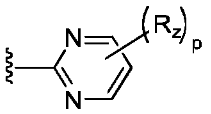, 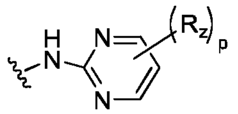, 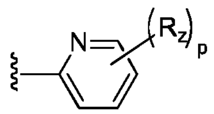,

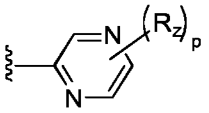, 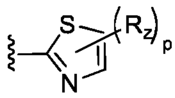, 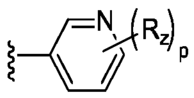, and 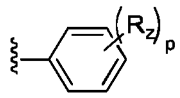;

or Z is selected from

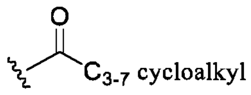,

p is as defined in claim 1;
$R_z$ is as defined in claim 1, 6, or 7.

**12.** The compound of formula (I) or the pharmaceutically acceptable salt thereof according to claim 11, wherein

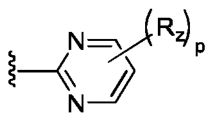

is selected from

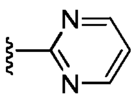, 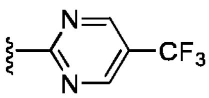,

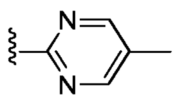, 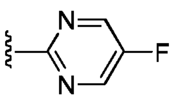, 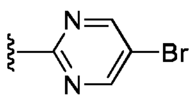, 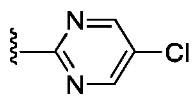, 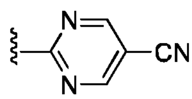,

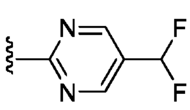, 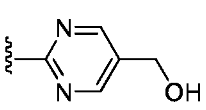, 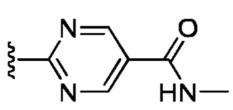, 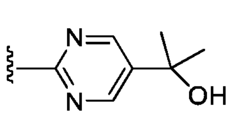, 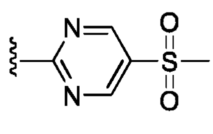,

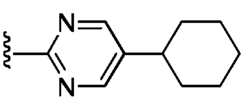, 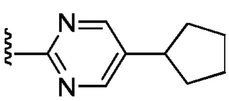, 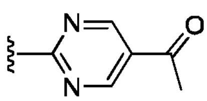, and 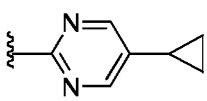.

**13.** The compound of formula (I) or the pharmaceutically acceptable salt thereof according to claim 11, wherein

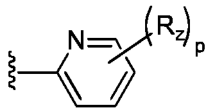

is selected from

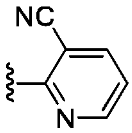, 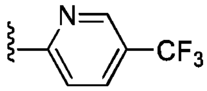,

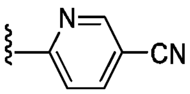, 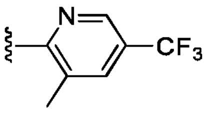, 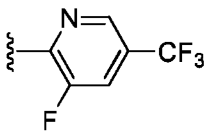, and 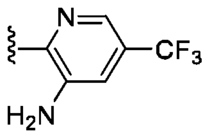.

**14.** The compound of formula (I) or the pharmaceutically acceptable salt thereof according to claim 11, wherein Z is further selected from

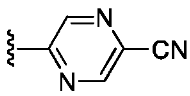, 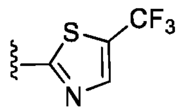,

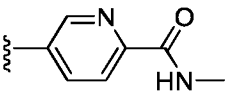, 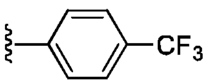, 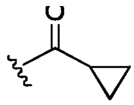, and 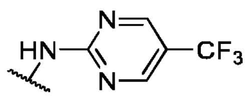.

**15.** The compound of formula (I) or the pharmaceutically acceptable salt thereof according to claim 1, wherein ring D is selected from

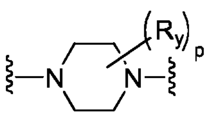, 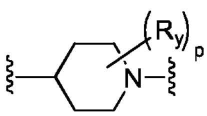,

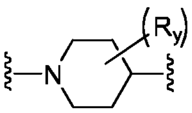, 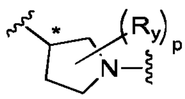, 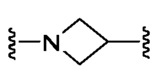, 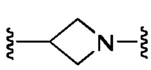, 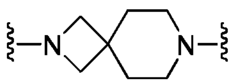,

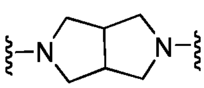, 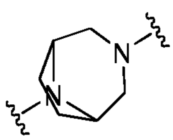, 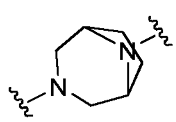, and 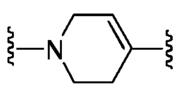;

p is as defined in claim 1;
$R_y$ is as defined in claim 1, 8, or 9.

**16.** The compound of formula (I) or the pharmaceutically acceptable salt thereof according to claim 15, wherein

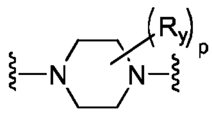

is selected from

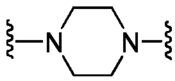, 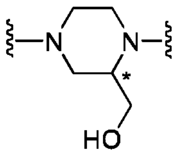,

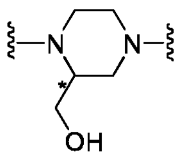, 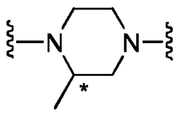, 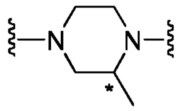, 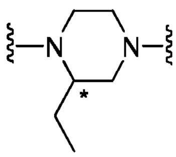, 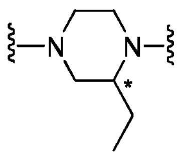, 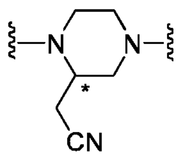,

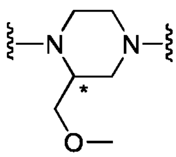, 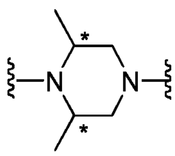, 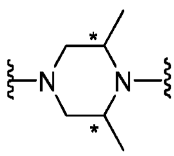, and 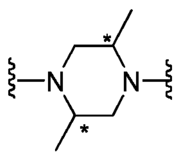;

the carbon atom marked with "*" in the structure represents a chiral carbon atom.

**17.** The compound of formula (I) or the pharmaceutically acceptable salt thereof according to claim 15, wherein ring D is further selected from

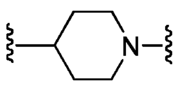, 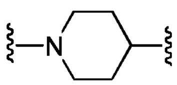,

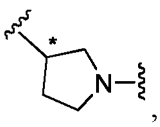, 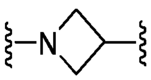, 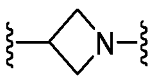, 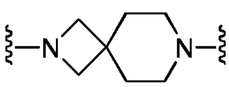, 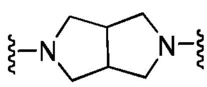, 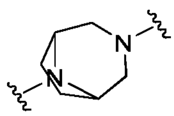,

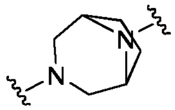, 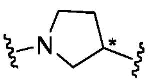, and 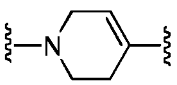;

the carbon atom marked with "*" in the structure represents a chiral carbon atom.

**18.** The compound of formula (I) or the pharmaceutically acceptable salt thereof according to any one of claims 1 to 17, wherein formula (A-1) is selected from the following groups:

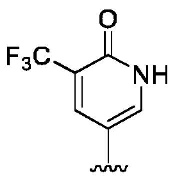, 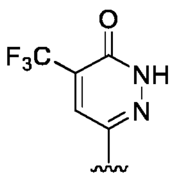, 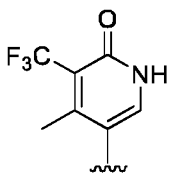, 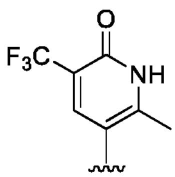, and 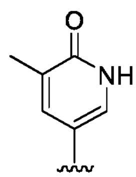.

**19.** The compound of formula (I) or the pharmaceutically acceptable salt thereof according to any one of claims 1 to 17, wherein formula (A-2) is selected from the following groups:

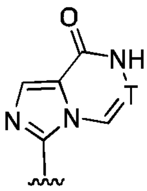 , 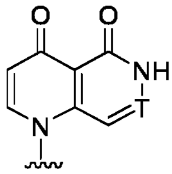 , 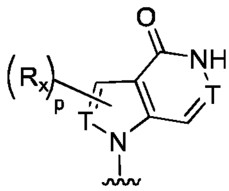 , 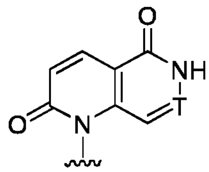 , 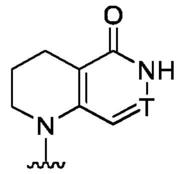 ,

and

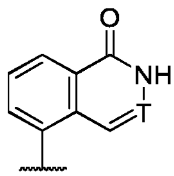 ;

p and T are as defined in claim 1 or 2;
$R_x$ is as defined in any one of claims 1 to 5.

**20.** The compound of formula (I) or the pharmaceutically acceptable salt thereof according to claim 19, wherein formula (A-2) is selected from the following groups:

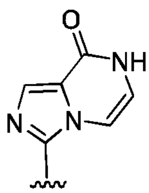 , 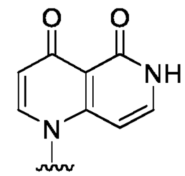 , 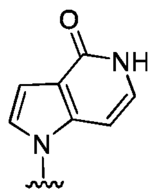 , 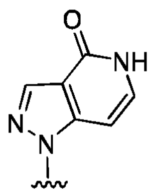 , 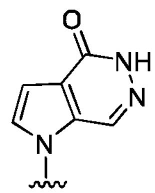 , 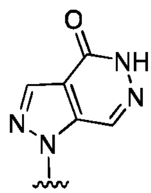 ,

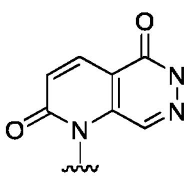 , 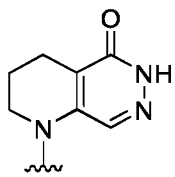 , and 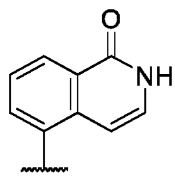 .

**21.** The compound of formula (I) or the pharmaceutically acceptable salt thereof according to any one of claims 1 to 17, wherein formula (A-3) is selected from

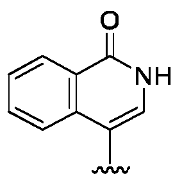 .

**22.** The compound of formula (I) or the pharmaceutically acceptable salt thereof according to any one of claims 1 to 21, wherein the Link has the following group of formula (LA):

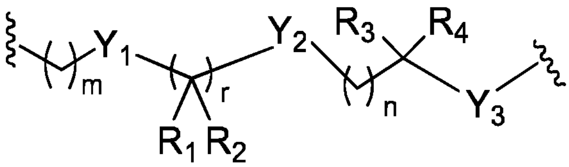

(LA)

wherein

$R_1$ and $R_2$ are each independently selected from H, $C_{1-3}$ alkyl, and hydroxy-$C_{1-3}$ alkyl, or $R_1$ and $R_2$ together with the carbon atom where they are located form a 3- to 6-membered carbocyclic ring, or $R_1$ and $R_2$ together form an oxo group;
$R_3$ and $R_4$ are each independently selected from H, $C_{1-3}$ alkyl, and hydroxy-$C_{1-3}$ alkyl, or $R_3$ and $R_4$ together form an oxo group;
$Y_1$ and $Y_2$ are selected from NH, -N($CH_3$)-, O, -$CH_2$NH-, -$CH_2$O-, $Cy^1$, or a single bond;
$Cy^1$ is selected from $C_{6-10}$ aryl, $C_{3-7}$ cycloalkyl, 5- to 10-membered heteroaryl, and 4- to 10-membered heterocyclyl, and groups are each optionally substituted by p substituents independently selected from $R_x$;
$Y_3$ is selected from NH, O, or a single bond;
m is an integer selected from 0, 1, and 2;
n is an integer selected from 0, 1, 2, and 3;
r is an integer selected from 0 and 1;
p is as defined in claim 1 or 2;
$R_x$ is as defined in any one of claims 1 to 5.

**23.** The compound of formula (I) or the pharmaceutically acceptable salt thereof according to any one of claims 1 to 21, wherein the Link has the following group of formula (LA);

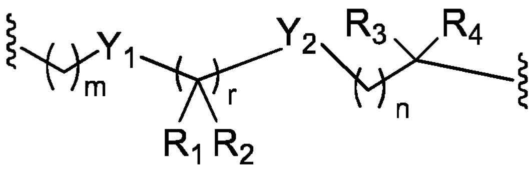

(LA)

wherein

$R_1$ and $R_2$ are each independently selected from H and $C_{1-3}$ alkyl, or $R_1$ and $R_2$ together with the carbon atom where they are located form a 3- to 6-membered carbocyclic ring, or $R_1$ and $R_2$ together form an oxo group;
$R_3$ and $R_4$ are selected from Hand $C_{1-3}$ alkyl, or $R_3$ and $R_4$ together form an oxo group;
$Y_1$ and $Y_2$ are selected from $Y_{1a}$, -$CH_2Y_{1a}$-, $Cy^1$, or a single bond;
$Y_{1a}$ is selected from NH and O;
$Cy^1$ is selected from $C_{6-10}$ aryl, $C_{3-7}$ cycloalkyl, 5- to 10-membered heteroaryl, and 4- to 10-membered heterocyclyl, and groups are each optionally substituted by p substituents independently selected from $R_x$;
m is an integer selected from 0, 1, and 2;
n is an integer selected from 0, 1, 2, and 3;
r is an integer selected from 0 and 1;
p is as defined in claim 1 or 2;
$R_x$ is as defined in any one of claims 1 to 5.

**24.** The compound of formula (I) or the pharmaceutically acceptable salt thereof according to claim 22 or 23, wherein $Cy^1$ is selected from

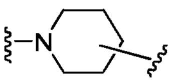, 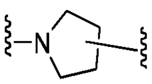, 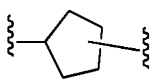,

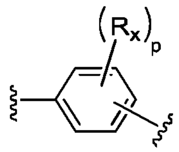, 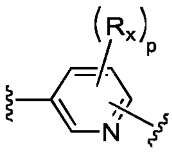, and 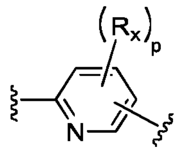;

p is as defined in claim 1 or 2;
$R_x$ is as defined in any one of claims 1 to 5.

**25.** The compound of formula (I) or the pharmaceutically acceptable salt thereof according to claim 22 or 23, wherein formula (LA) has the following structures:

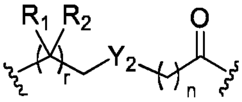 (LA-1), 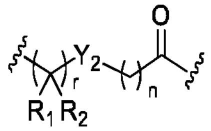 (LA-2), 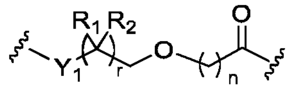 (LA-3),

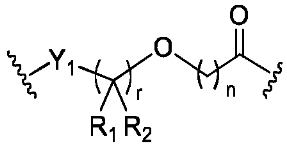 (LA-4), 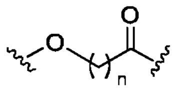 (LA-5), 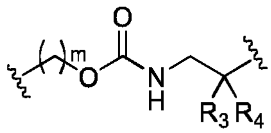 (LA-6),

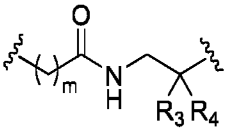 (LA-7), 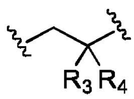 (LA-8), 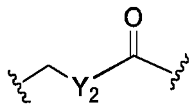 (LA-9),

or

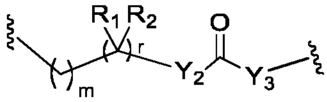 (LA-10);

wherein $R_1$, $R_2$, $R_3$, $R_4$, $Y_1$, $Y_2$, $Y_3$, m, n, and r are as defined in claim 22 or 23.

**26.** The compound of formula (I) or the pharmaceutically acceptable salt thereof according to claim 23, wherein formula (LA) has the following structures;

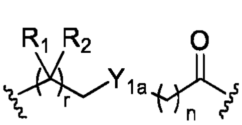 (LA-1), 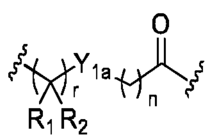 (LA-2), 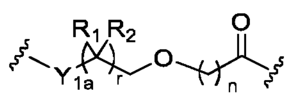 (LA-3),

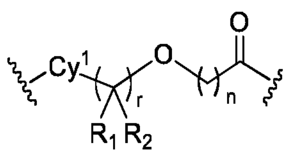 (LA-4), 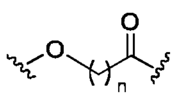 (LA-5), 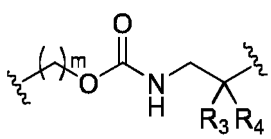 (LA-6),

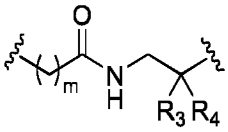 (LA-7), 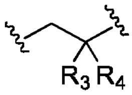 (LA-8), 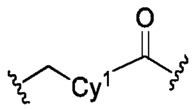 (LA-9),

or

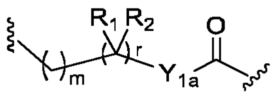 (LA-10);

wherein $R_1$, $R_2$, $R_3$, $R_4$, m, n, and r are as defined in claim 23;
$Y_{1a}$ is as defined in claim 23;
$Cy^1$ is as defined in claim 23 or 24.

**27.** The compound of formula (I) or the pharmaceutically acceptable salt thereof according to claim 25, wherein formula (LA) has the following structures:

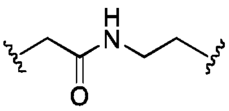, 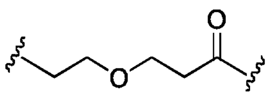, 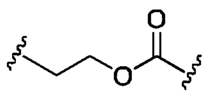, 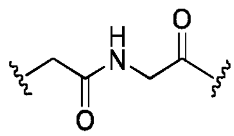,

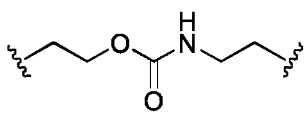, 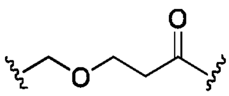, 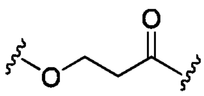, 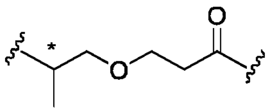,

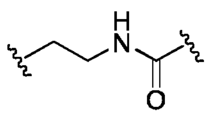, 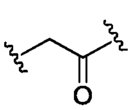, 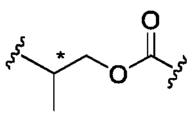, 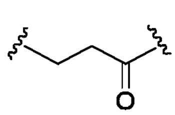, 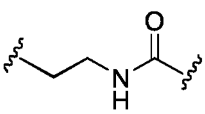,

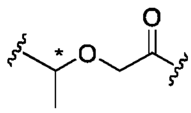, 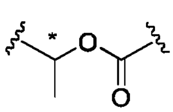, 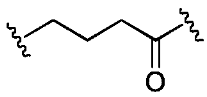, 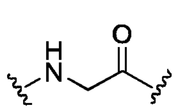, 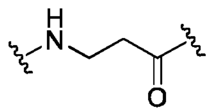,

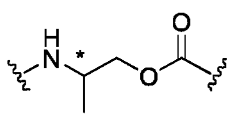, 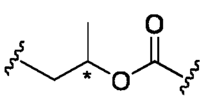, 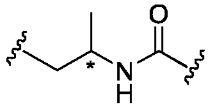, 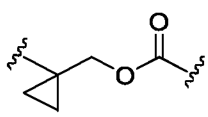, 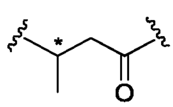,

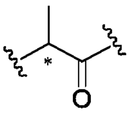, 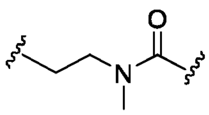, 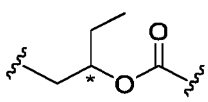, 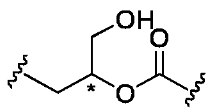, and 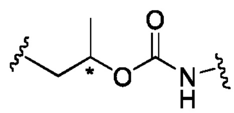;

the carbon atom marked with "*" in the structure represents a chiral carbon atom.

**28.** The compound of formula (I) or the pharmaceutically acceptable salt thereof according to claim 25, wherein formula (LA) has the following structures:

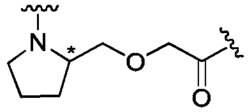 , 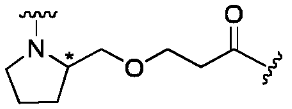 , 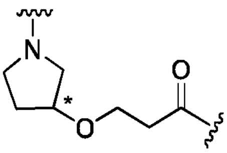 , 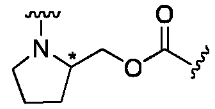 ,

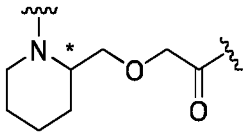 , 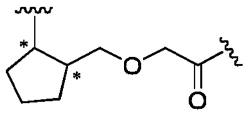 , 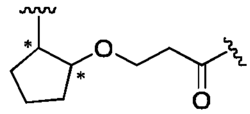 , 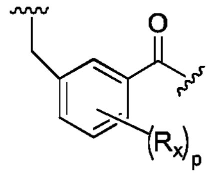 ,

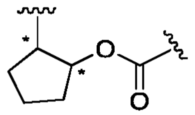 , 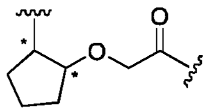 , 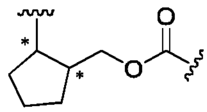 , 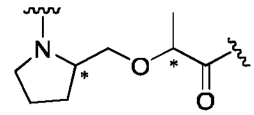 ,

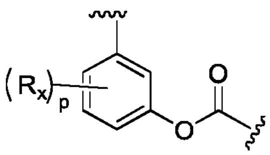 , 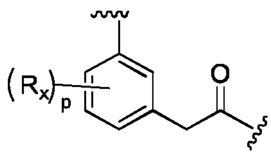 , 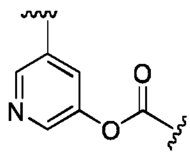 , 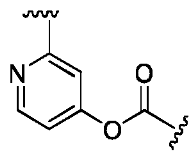 ,

and

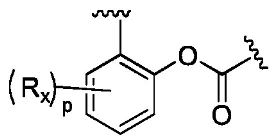 ;

the carbon atom marked with "*" in the structure represents a chiral carbon atom; p is as defined in claim 1 or 2; $R_x$ is as defined in any one of claims 1 to 5.

**29.** The compound of formula (I) or the pharmaceutically acceptable salt thereof according to any one of claims 1 and 3 to 28, wherein the compound of formula (I) or the pharmaceutically acceptable salt thereof has a structure of formula (I-1), (I-2), (I-3), or (I-4):

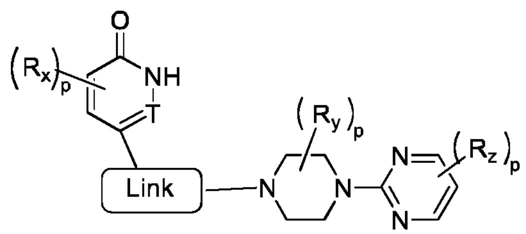

(I-1)

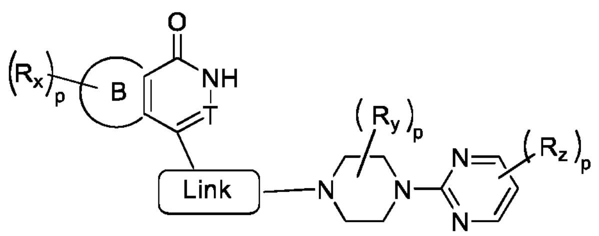

(I-2)

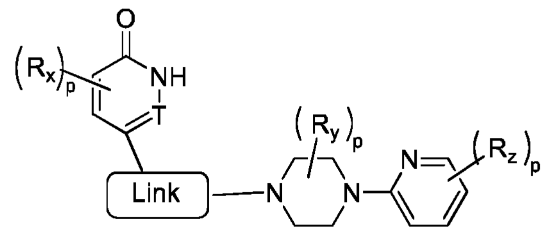

(I-3)

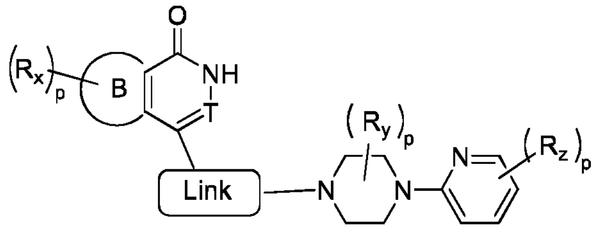

(I-4).

**30.** The compound of formula (I) or the pharmaceutically acceptable salt thereof according to any one of claims 1 and 3 to 28, wherein the compound of formula (I) or the pharmaceutically acceptable salt thereof satisfies one or more than one of the following conditions:

(1) in ring B, the $C_{6-10}$ aryl is phenyl or naphthyl, preferably phenyl;
(2) in ring B, the 5- to 10-membered heteroaryl is 5-membered or 6-membered heteroaryl containing a carbon atom and 1, 2, 3, or 4 cycloheteroatoms independently selected from N, O, and S; preferably 5-membered or 6-membered heteroaryl containing a carbon atom and 1 or 2 N cycloheteroatoms;
(3) in ring B, the 4- to 10-membered heterocyclyl is a 5-membered or 6-membered monocyclic, saturated or unsaturated cyclic group containing a carbon atom and 1, 2, 3, or 4 cycloheteroatoms independently selected from N, O, and S; preferably a 5-membered or 6-membered monocyclic, saturated or unsaturated cyclic group containing a carbon atom and 1 or 2 N cycloheteroatoms;
(4) in ring D, the 4- to 10-membered heterocyclyl is a 4-membered monocyclic, 5-membered monocyclic, 6-membered monocyclic, 8-membered bicyclic, or 9-membered bicyclic, saturated or unsaturated cyclic group containing a carbon atom and 1, 2, 3, or 4 cycloheteroatoms independently selected from N, O, and S; preferably a 4-membered monocyclic, 5-membered monocyclic, 6-membered monocyclic, 8-membered bicyclic, or 9-membered bicyclic, saturated or unsaturated cyclic group containing a carbon atom and 1 or 2 N cycloheteroatoms;
(5) in $Cy^2$, the $C_{6-10}$ aryl is phenyl or naphthyl, preferably phenyl;
(6) in $Cy^2$, the 5- to 10-membered heteroaryl is 5-membered or 6-membered heteroaryl containing a carbon atom and 1, 2, 3, or 4 cycloheteroatoms independently selected from N, O, and S; preferably 5-membered or 6-membered heteroaryl containing a carbon atom and 1 or 2 cycloheteroatoms independently selected from N and S;
(7) in $R_x$ and $R_z$, the halogen is independently fluorine, chlorine, bromine, or iodine, preferably fluorine, chlorine, or bromine;
(8) in $R_x$ and $R_z$, in the $C_{1-6}$ alkyl, $C_{1-6}$ haloalkyl, hydroxyl-$C_{1-6}$ alkyl-, $C_{1-6}$ alkyl-C(O)-, $C_{1-6}$ alkyl-$S(O)_2$-, and $C_{1-6}$ alkyl-NH-C(O)-, the $C_{1-6}$ alkyl is independently methyl, ethyl, *n*-propyl, isopropyl, *n*-butyl, isobutyl, *sec*-butyl, or *tert*-butyl, preferably methyl, ethyl, or isopropyl;
(9) in $R_x$ and $R_z$, the $C_{3-7}$ cycloalkyl is cyclopropyl, cyclobutyl, cyclopentyl, or cyclohexyl;
(10) in $R_y$, the $C_{1-6}$ alkyl is methyl, ethyl, *n*-propyl, isopropyl, *n*-butyl, isobutyl, *sec*-butyl, or *tert*-butyl, preferably methyl or ethyl.

**31.** The compound of formula (I) or the pharmaceutically acceptable salt thereof according to claim 22 or 23, wherein the compound of formula (I) or the pharmaceutically acceptable salt thereof satisfies one or more than one of the following conditions:

(1) in $Cy^1$, the $C_{6-10}$ aryl is phenyl or naphthyl, preferably phenyl;
(2) in $Cy^1$, the $C_{3-7}$ cycloalkyl is cyclopropyl, cyclobutyl, cyclopentyl, or cyclohexyl, preferably cyclopentyl;
(3) in $Cy^1$, the 5- to 10-membered heteroaryl is 5-membered or 6-membered heteroaryl containing a carbon atom and 1, 2, 3, or 4 cycloheteroatoms independently selected from N, O, and S; preferably 6-membered

heteroaryl containing a carbon atom and 1 N cycloheteroatom;

(4) in $Cy^1$, the 4- to 10-membered heterocyclyl is a 5-membered or 6-membered monocyclic, saturated or unsaturated cyclic group containing a carbon atom and 1, 2, 3, or 4 cycloheteroatoms independently selected from N, O, and S; preferably a 5-membered or 6-membered monocyclic, saturated cyclic group containing a carbon atom and 1 or 2 N cycloheteroatoms.

**32.** A compound or a pharmaceutically acceptable salt thereof, wherein the compound is selected from

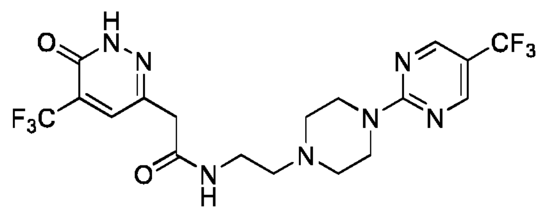
,
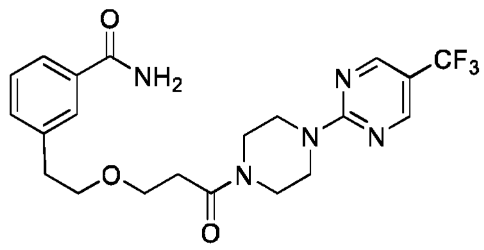
,
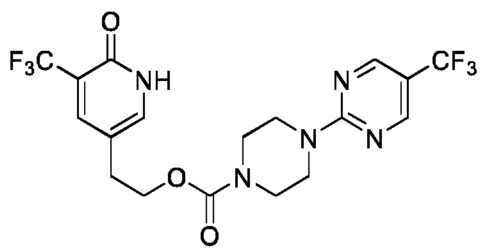
,
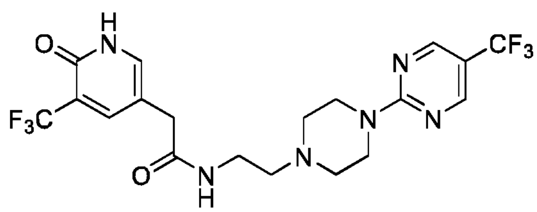
,
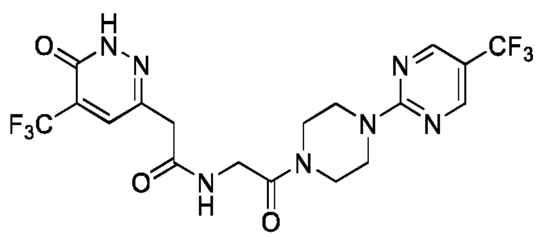
,
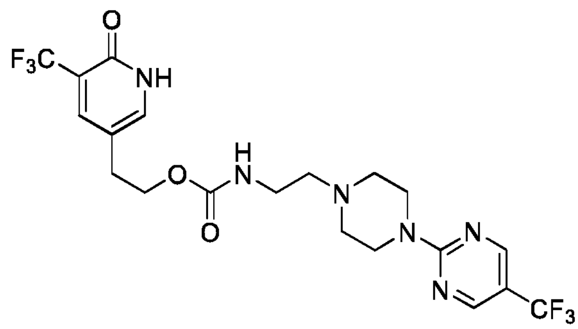
,
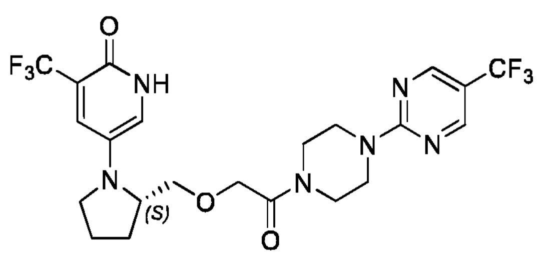
,
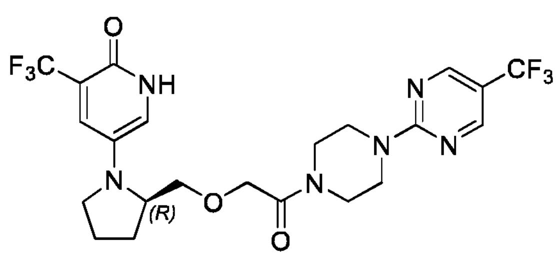
,

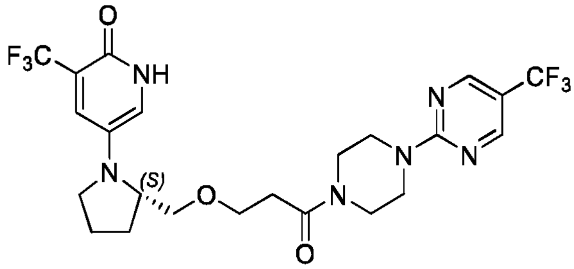

,

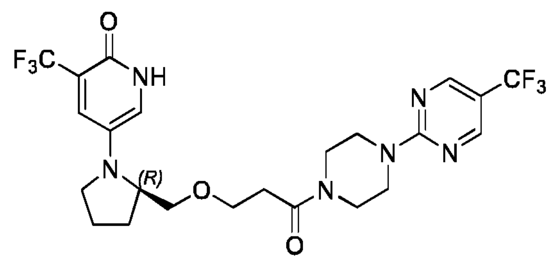

,

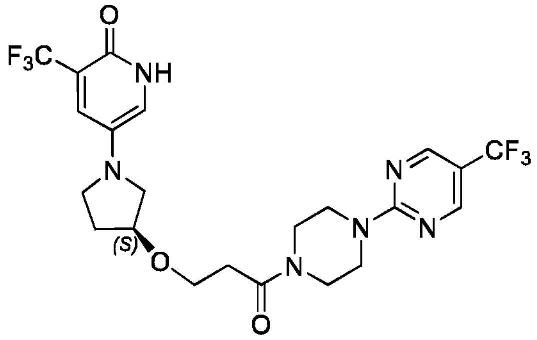

,

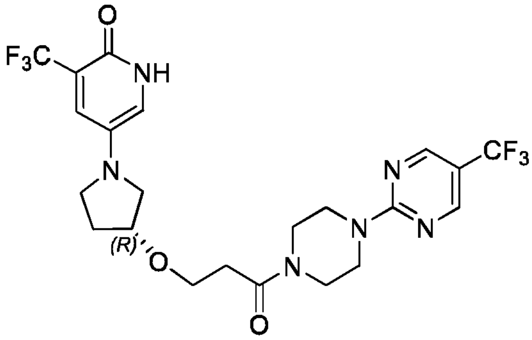

,

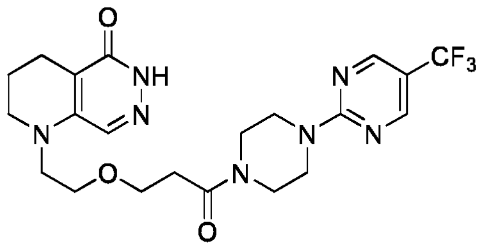

,

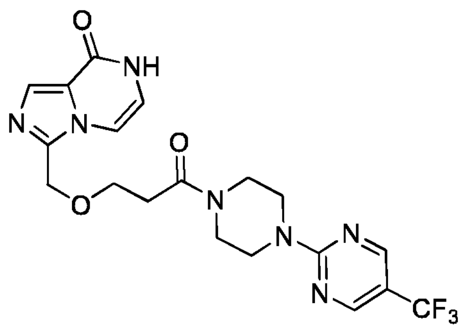

,

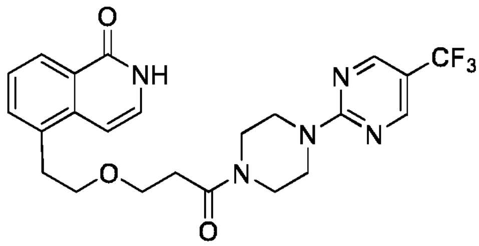

,

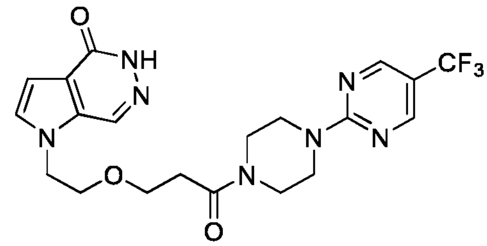

,

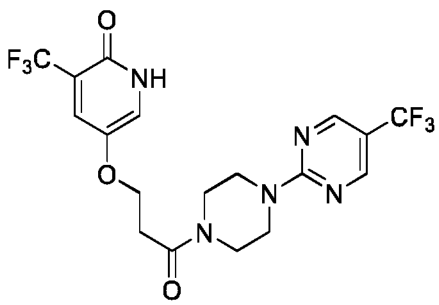

,

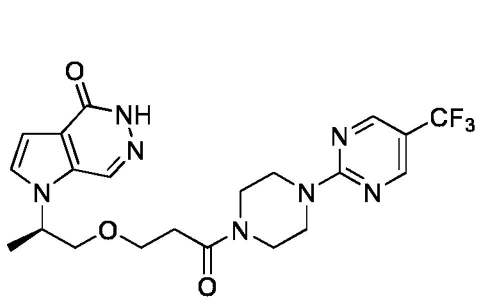

,

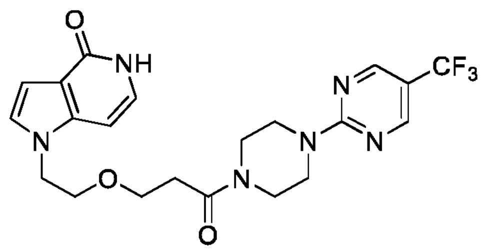

,

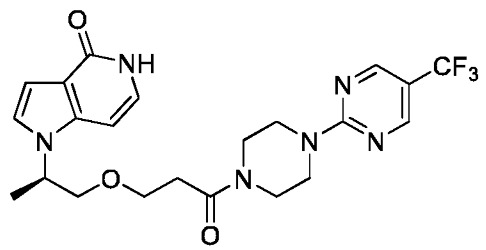

,

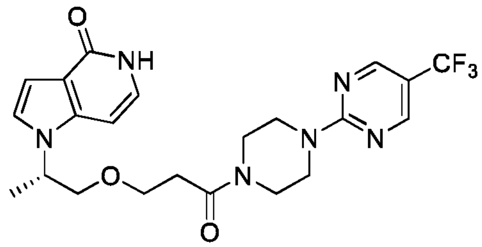

,

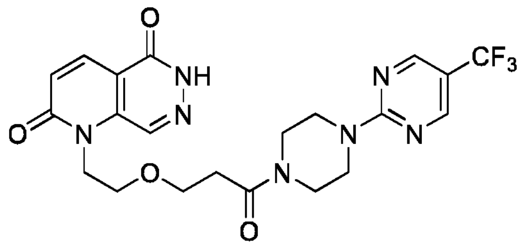

,

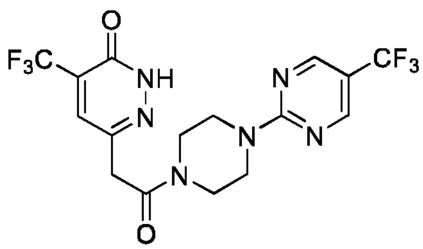

,

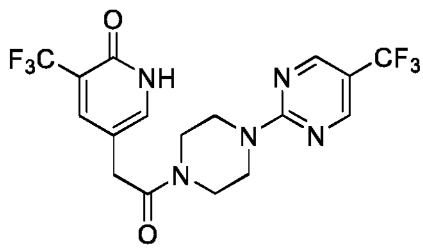

,

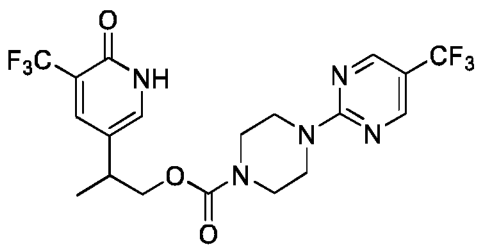

,

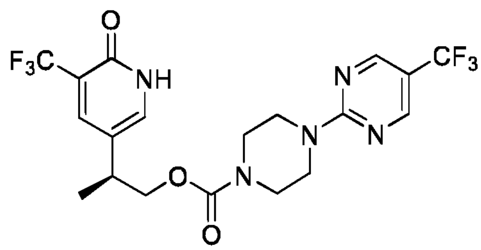

,

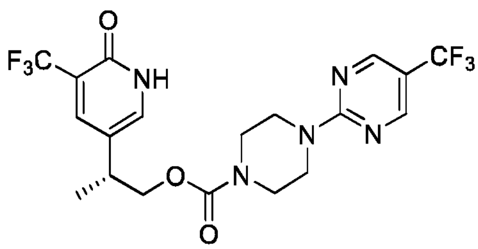

,

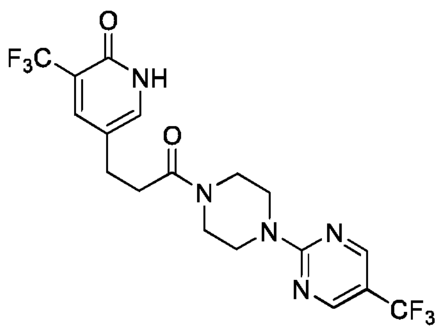

,

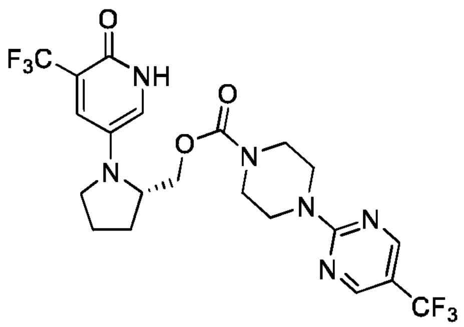
,
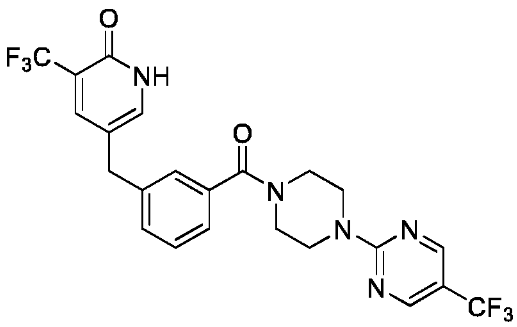
,
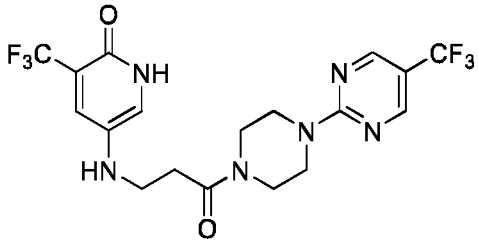
,
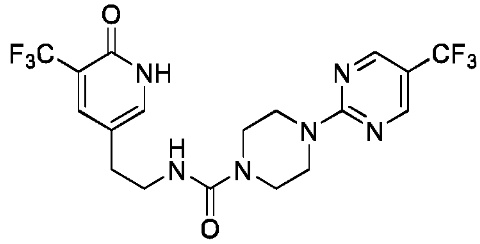
,
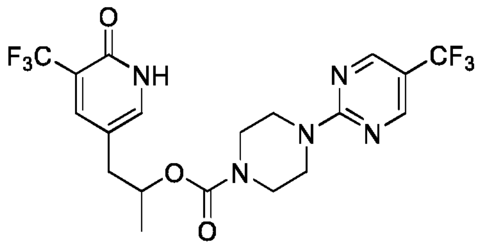
,
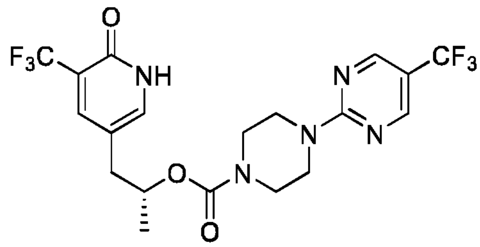
,
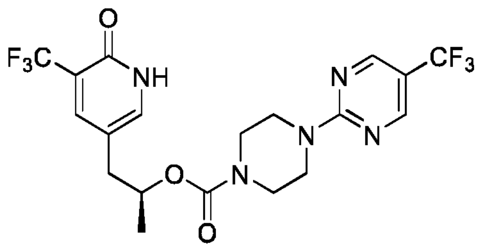
,
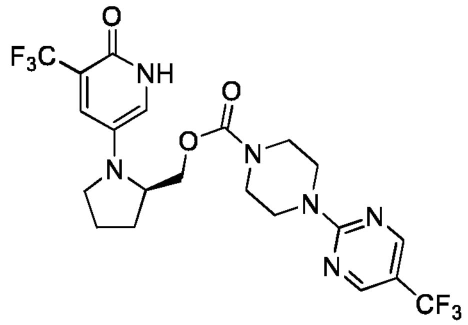
,
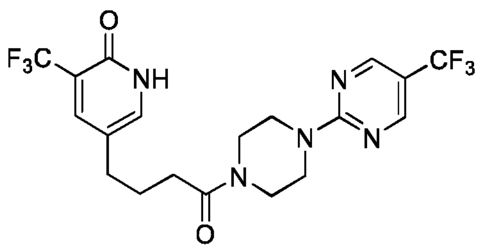
,
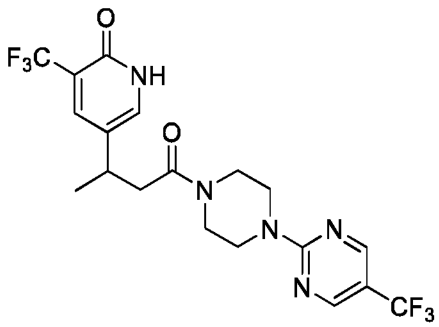
,

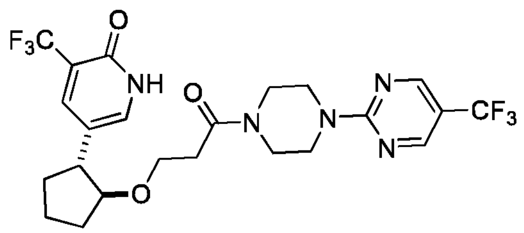

,

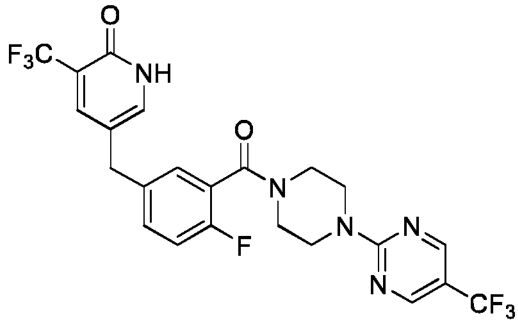

,

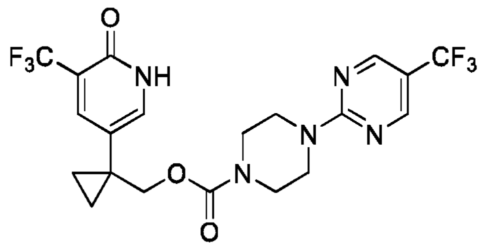

,

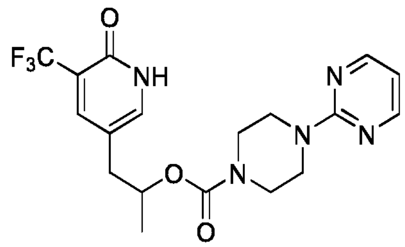

,

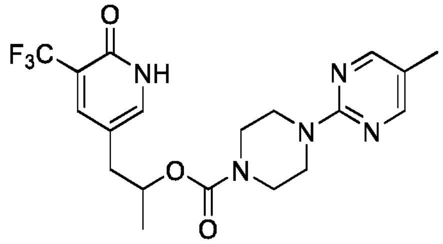

,

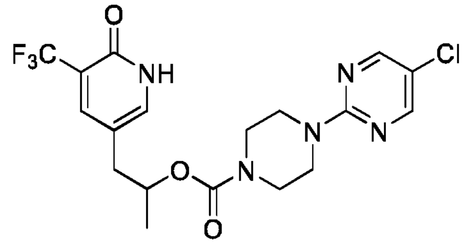

,

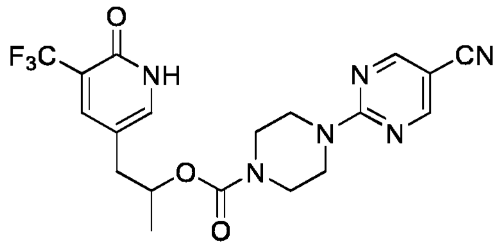

,

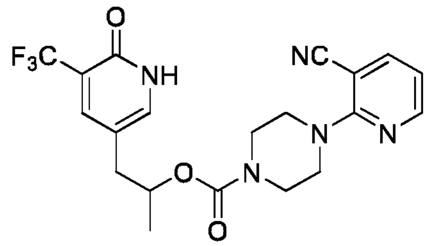

,

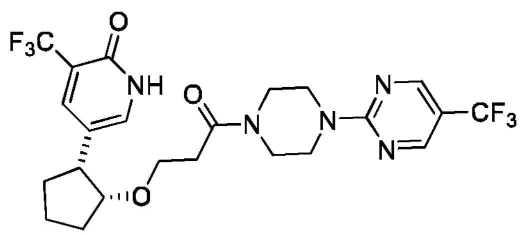

,

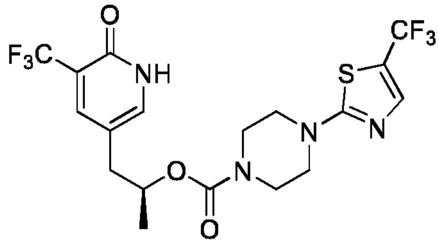

,

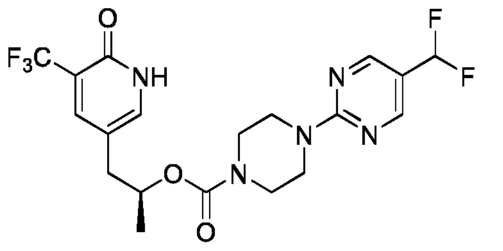
,
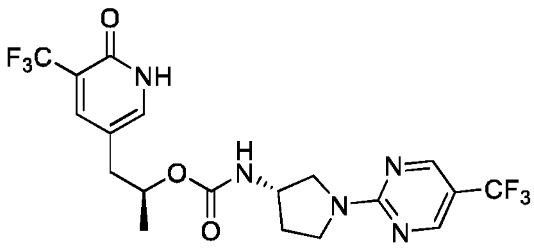
,
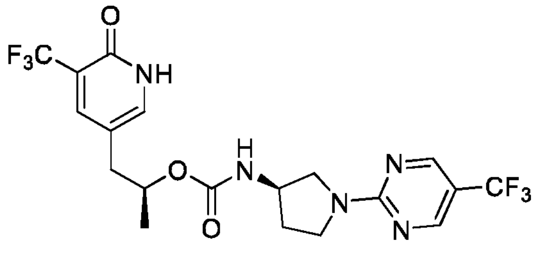
,
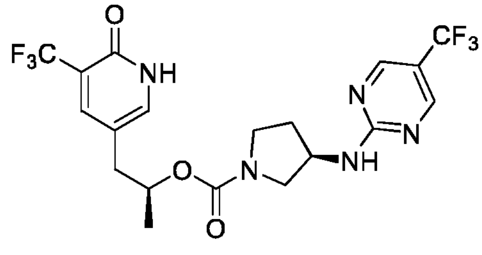
,
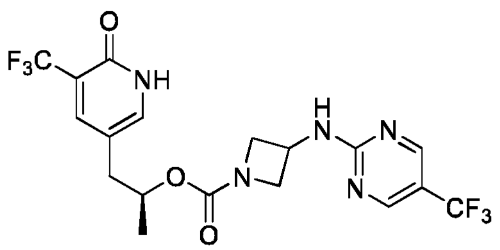
,
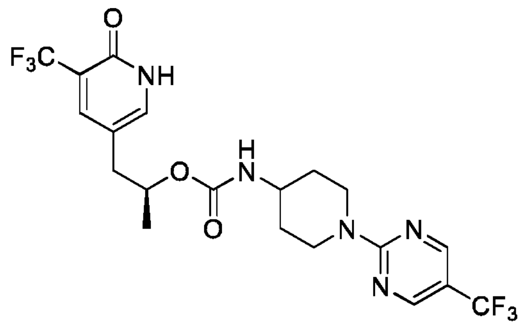
,
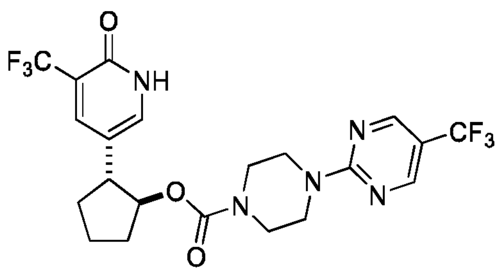
,
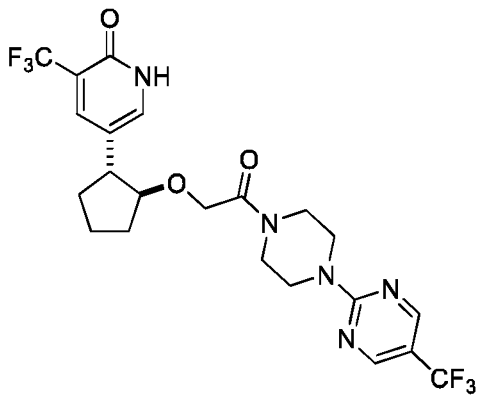
,
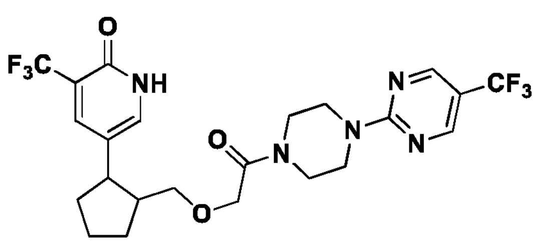
,
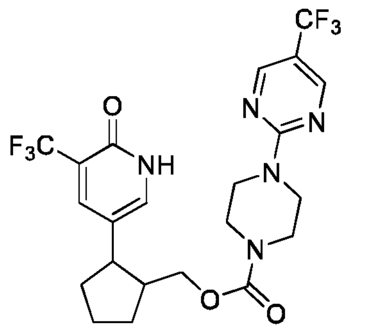
,

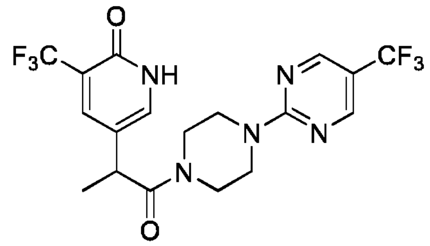
,
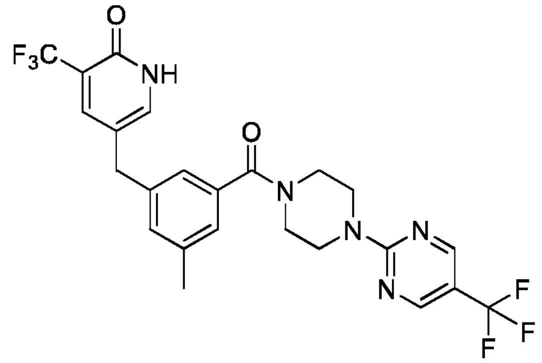
,

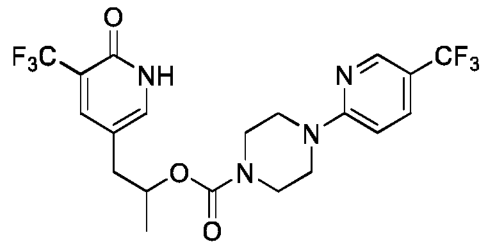
,
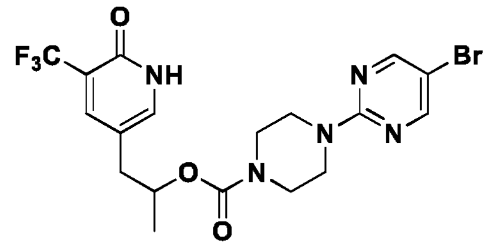
,

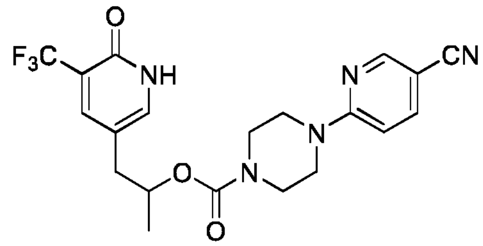
,
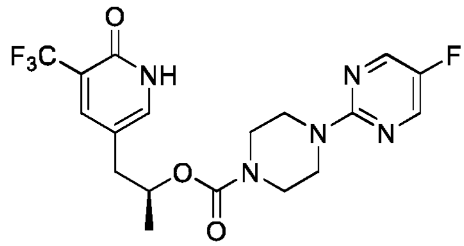
,

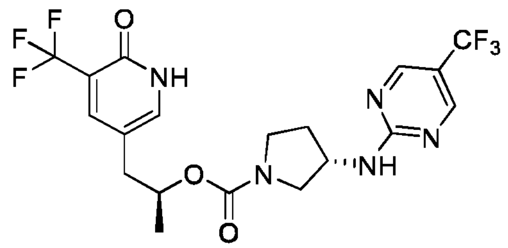
,
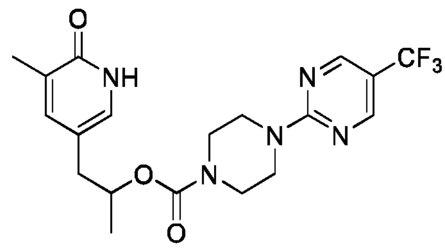
,

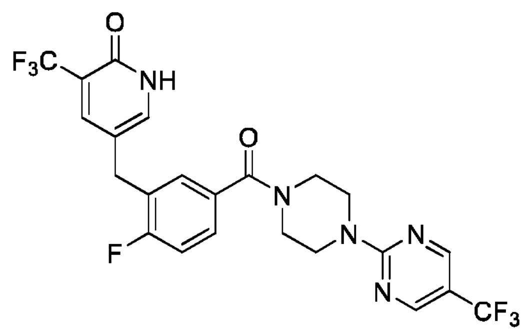
,
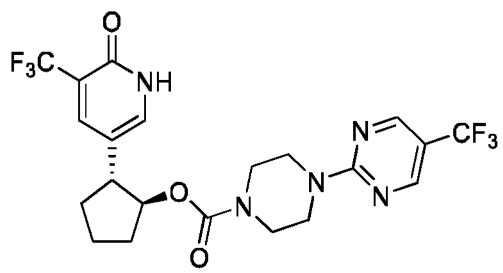
,

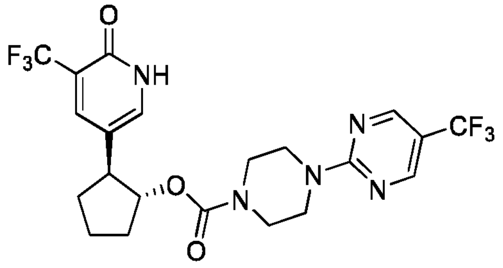
,
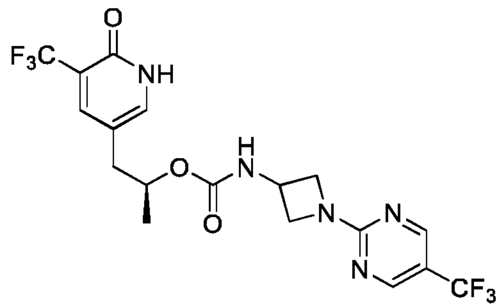
,
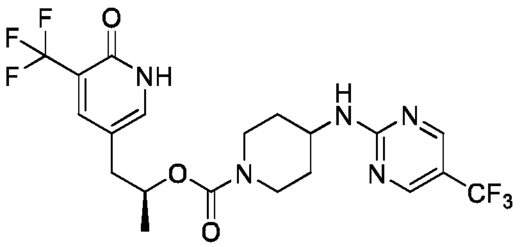
,
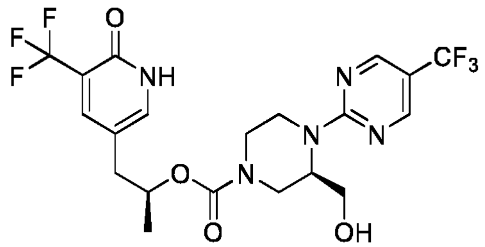
,
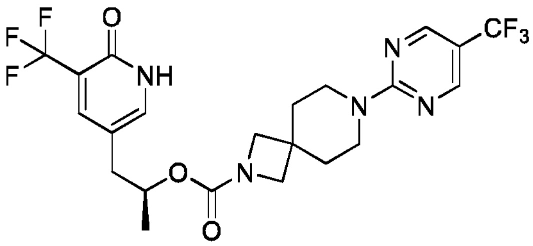
,
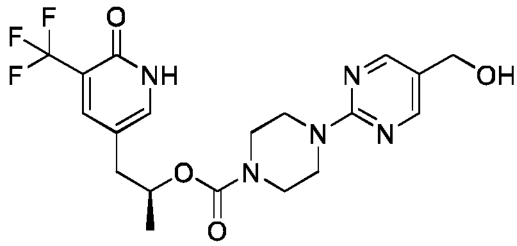
,
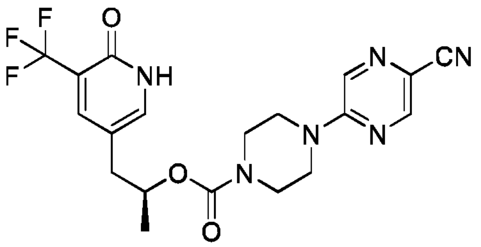
,
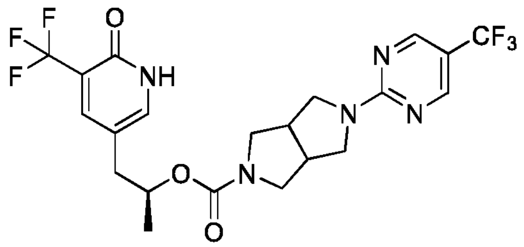
,
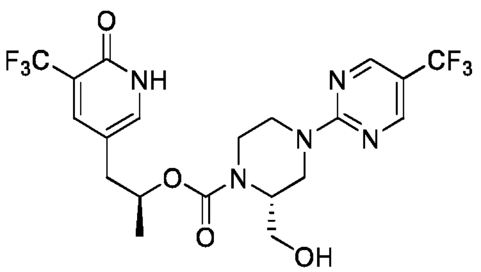
,
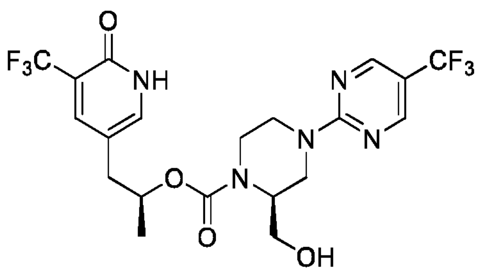
,

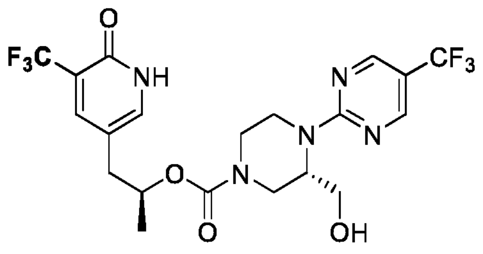

,

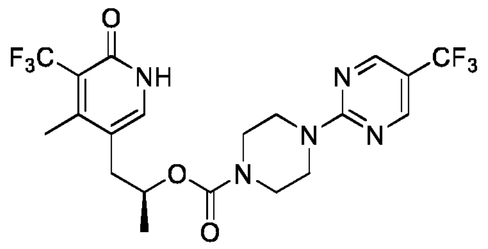

,

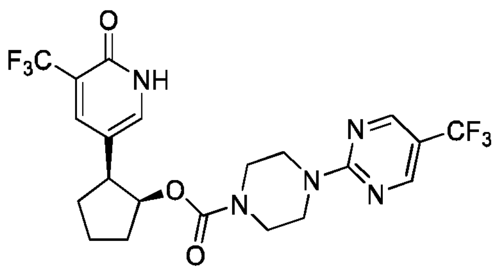

,

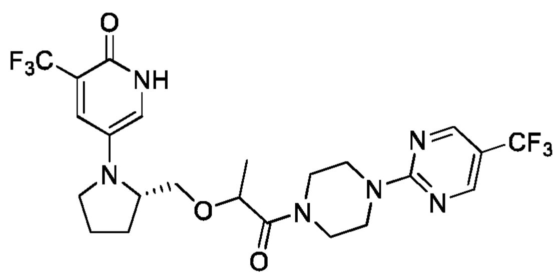

,

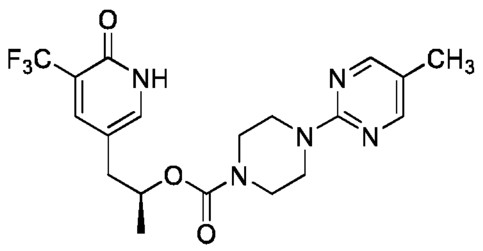

,

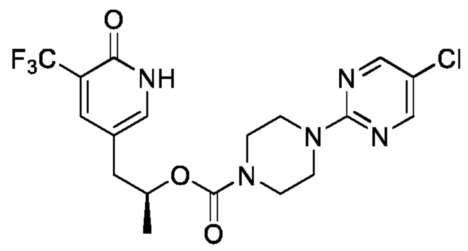

,

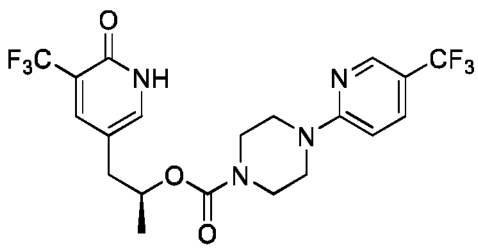

,

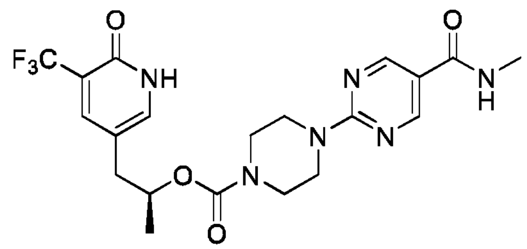

,

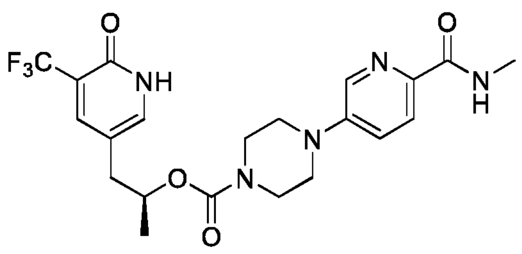

,

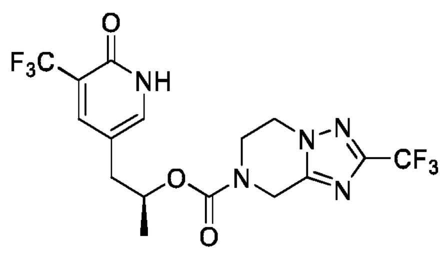

,

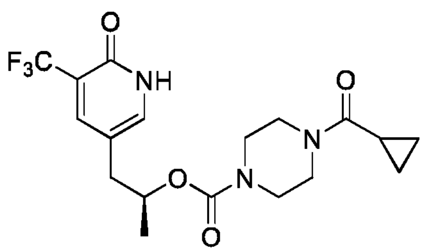

,

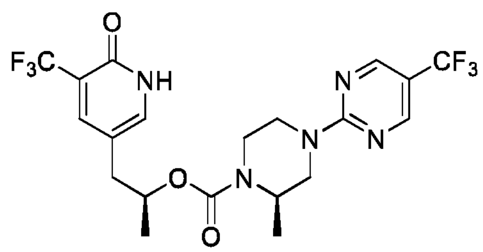

,

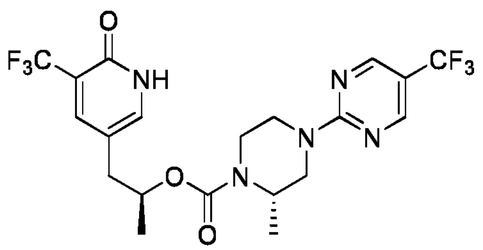

,

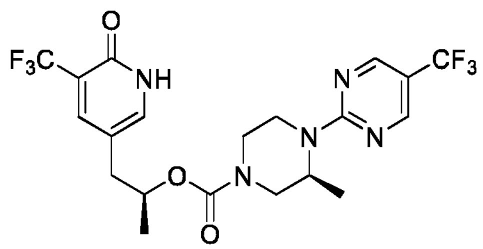

,

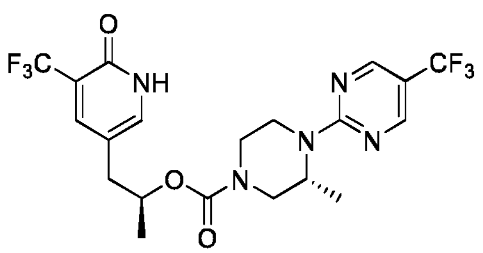

,

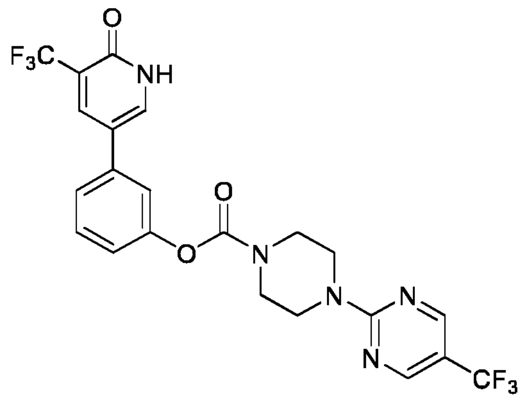

,

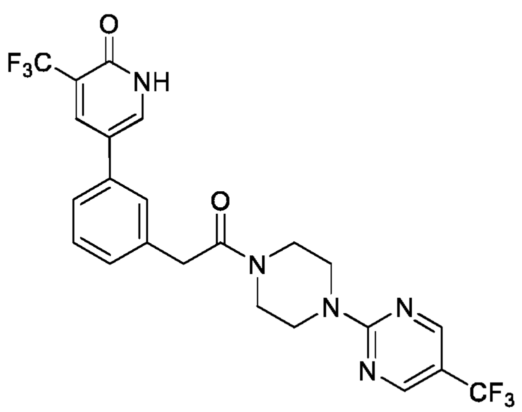

,

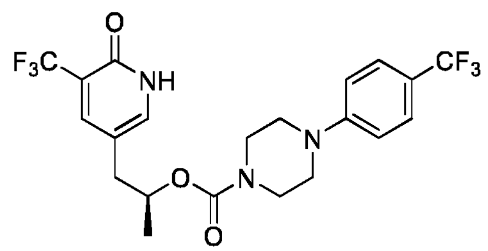

,

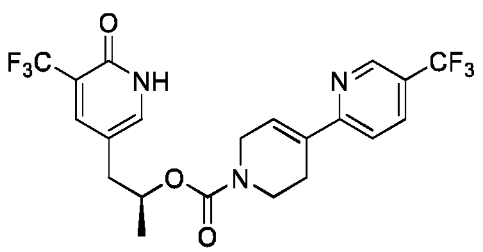

,

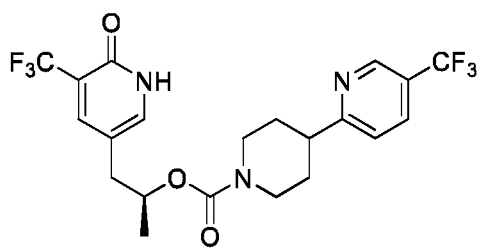

,

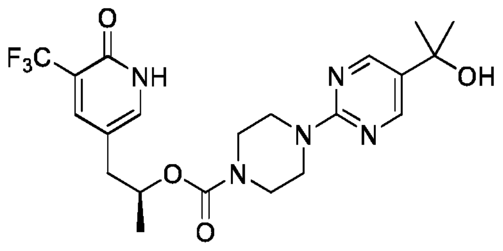
,

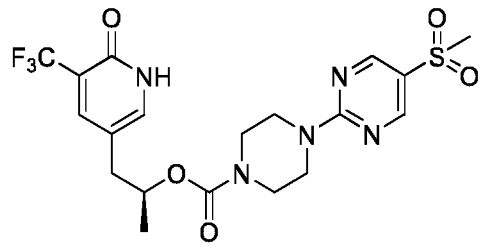
,

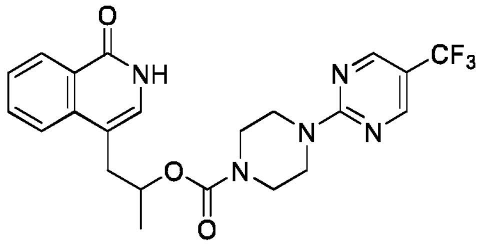
,

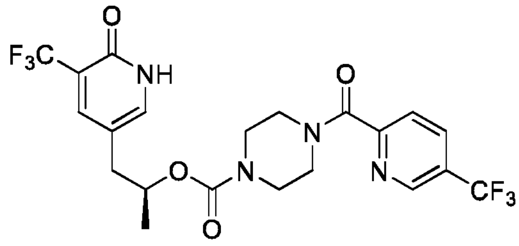
,

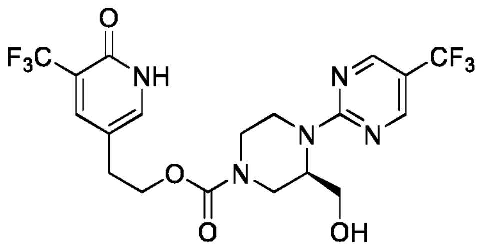
,

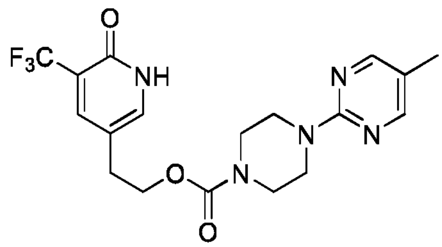
,

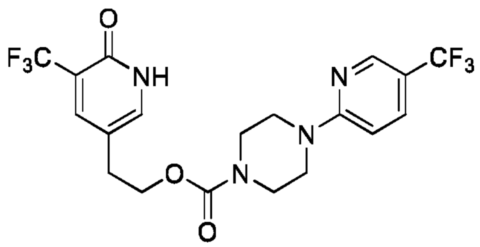
,

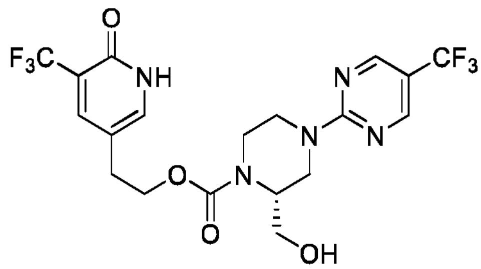
,

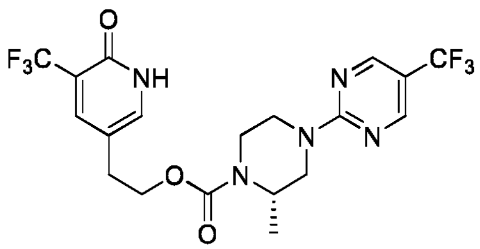
,

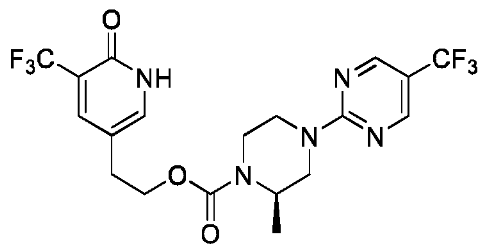
,

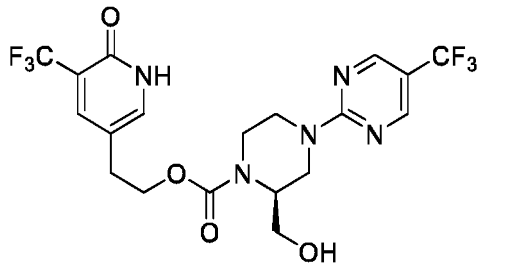
,
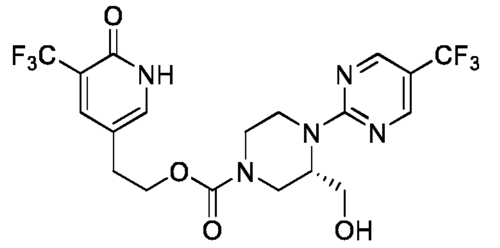
,
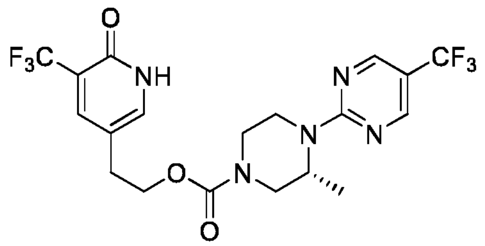
,
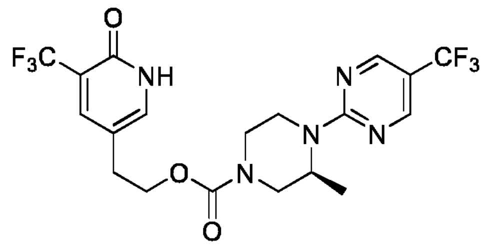
,
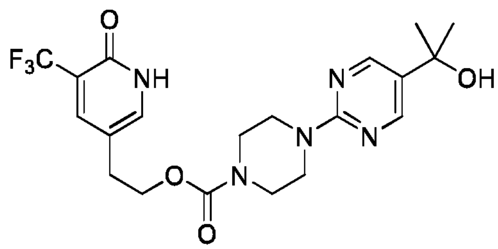
,
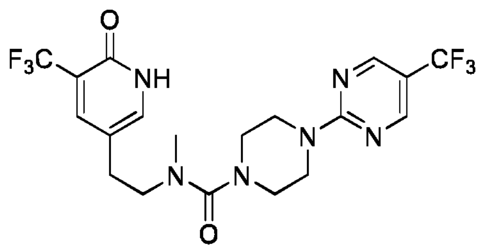
,
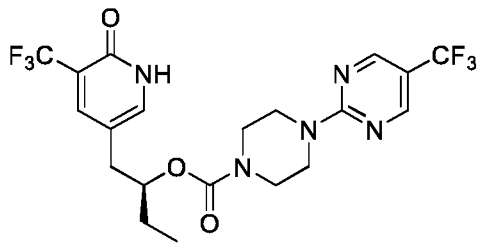
,
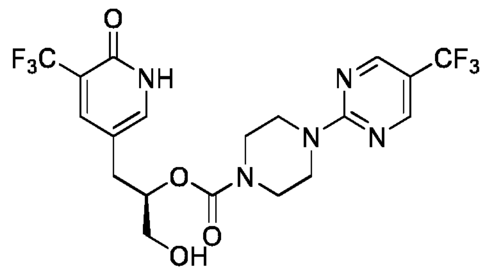
,
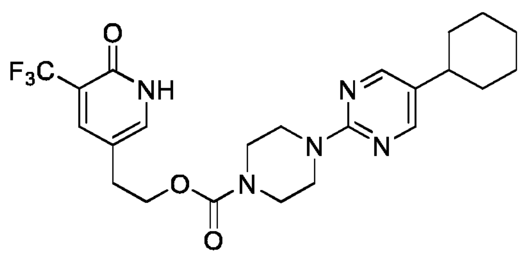
,
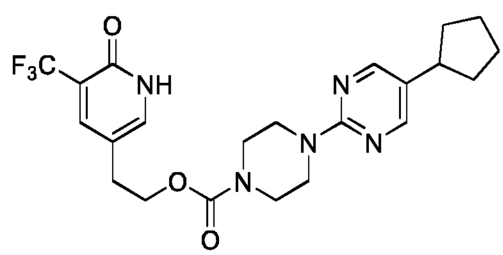
,

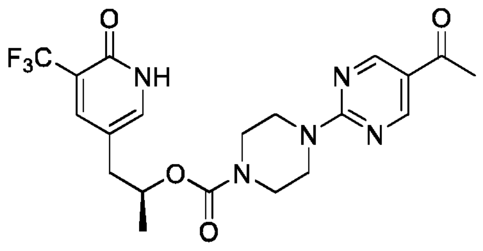
,

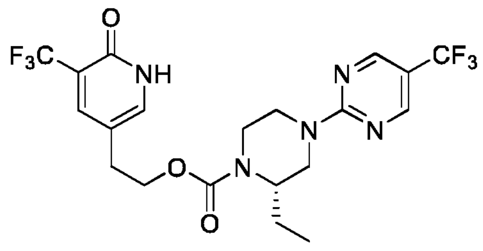
,

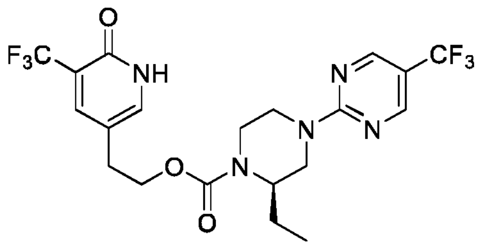
,

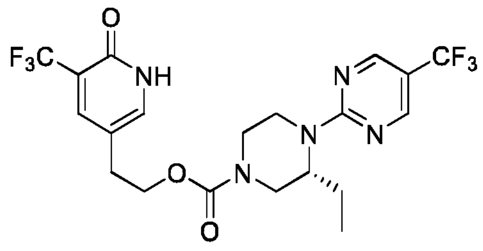
,

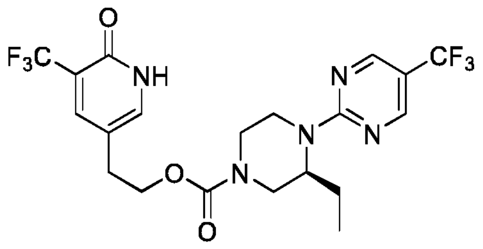
,

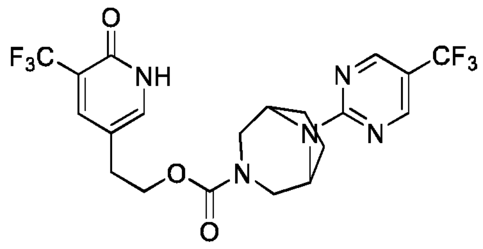
,

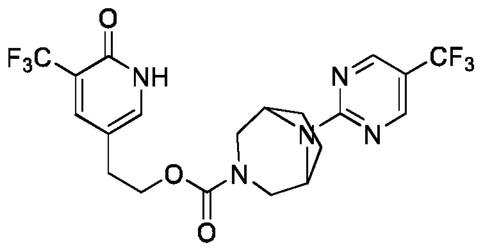
,

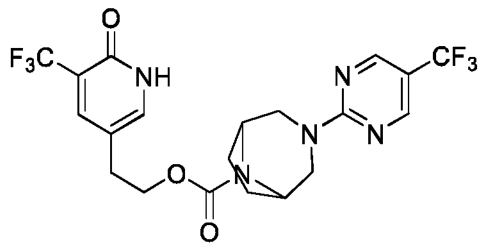
,

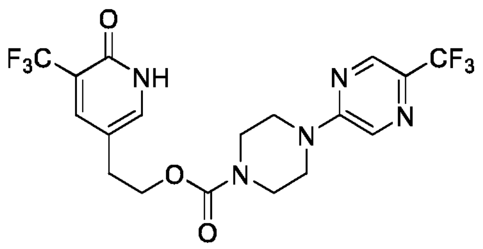
,

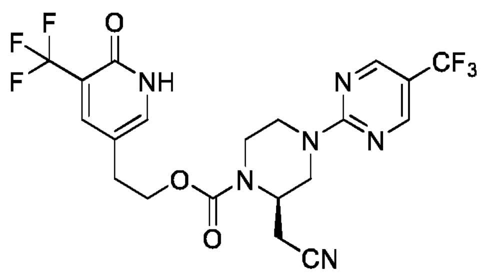
,

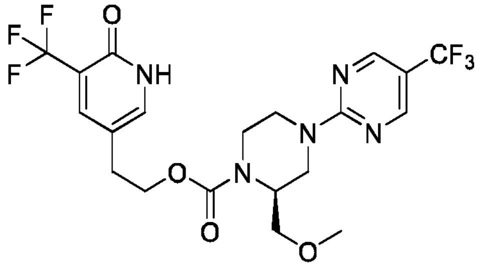 ,

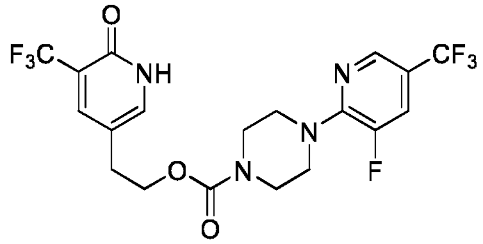 ,

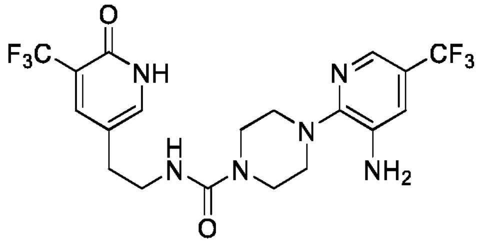 ,

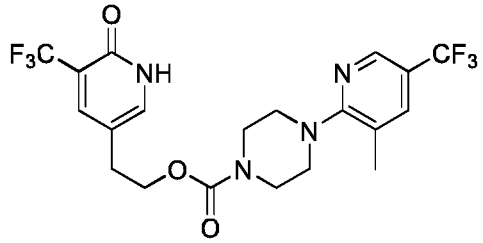 ,

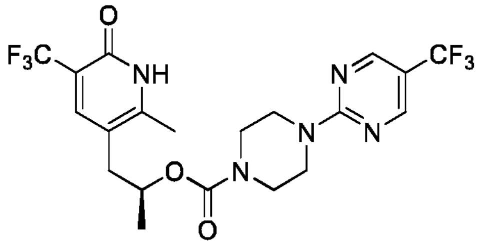 ,

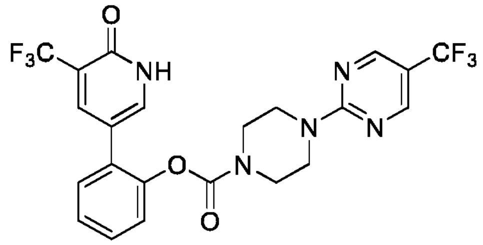 ,

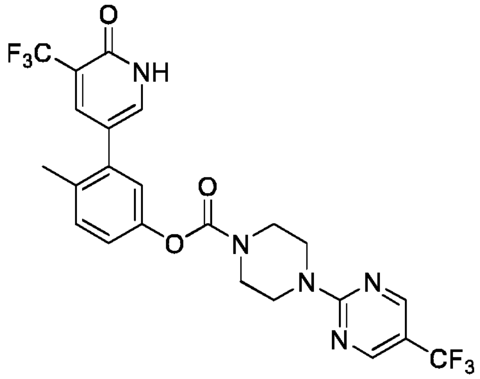 ,

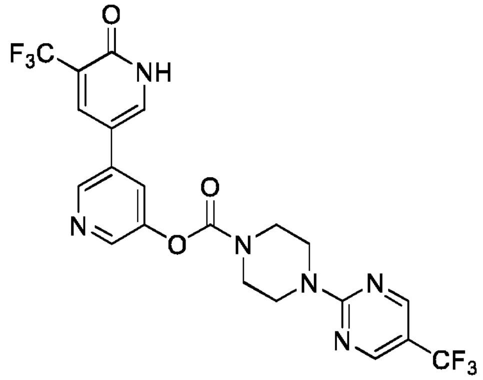 ,

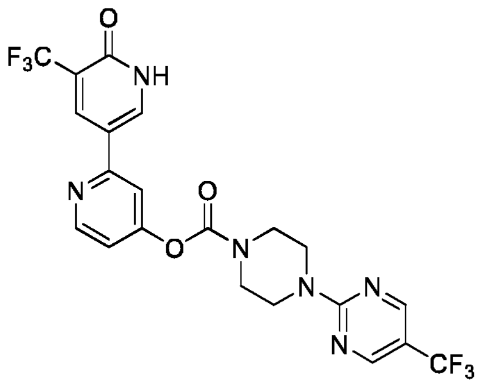 ,

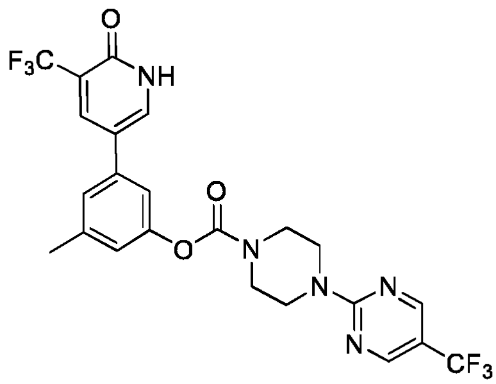 ,

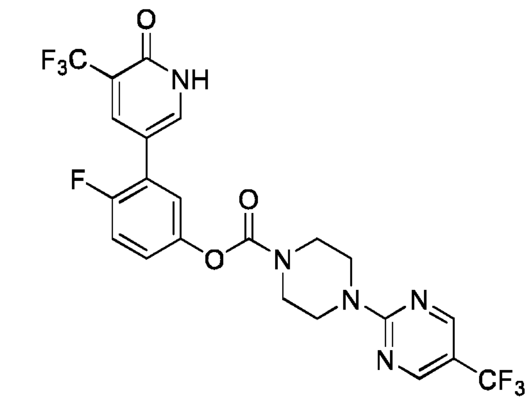
,
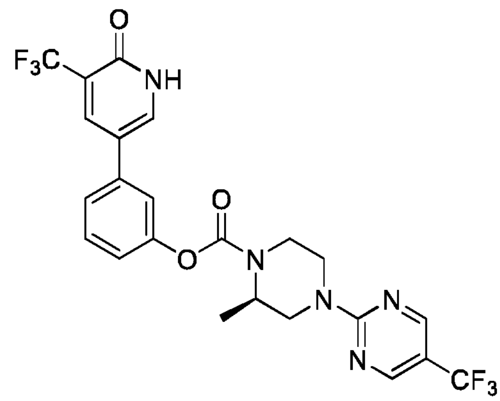
,
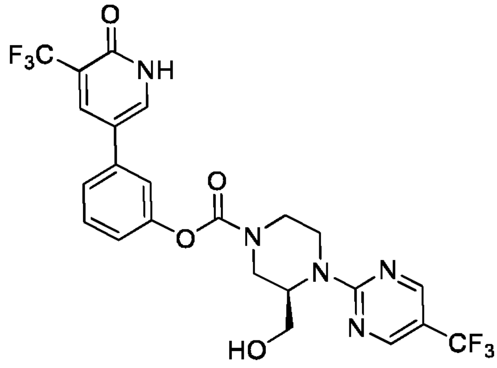
,
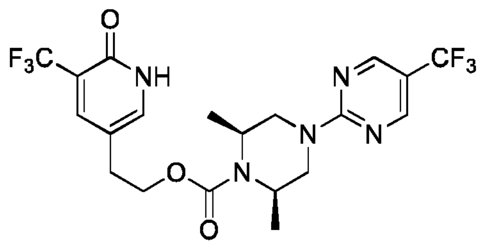
,
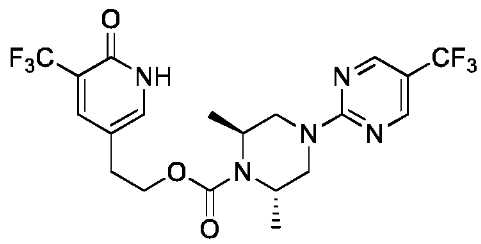
,
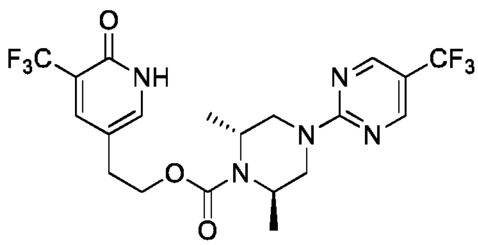
,
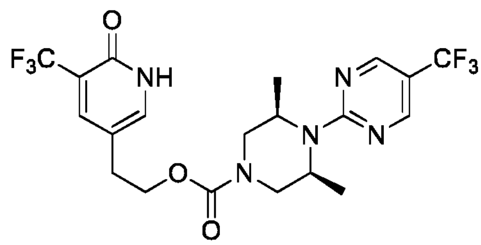
,
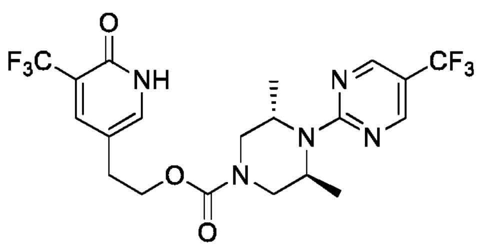
,
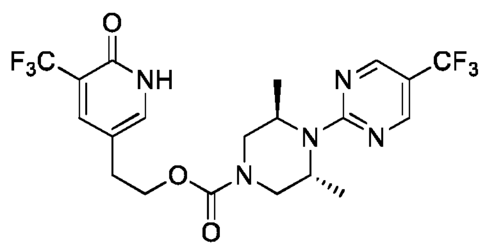
,

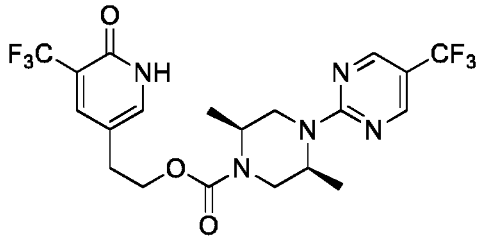
,
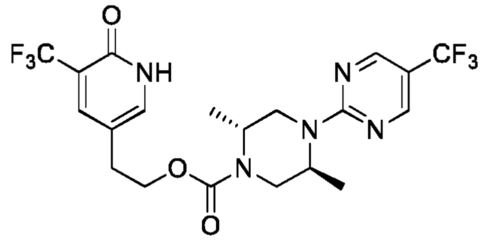
,
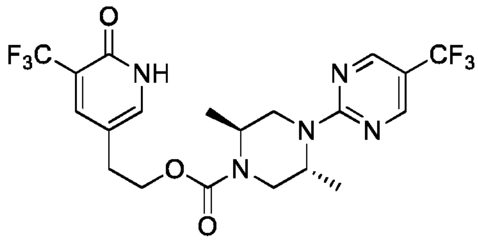
,
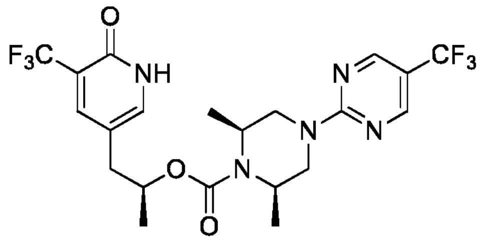
,
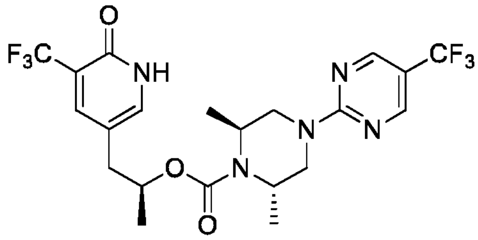
,
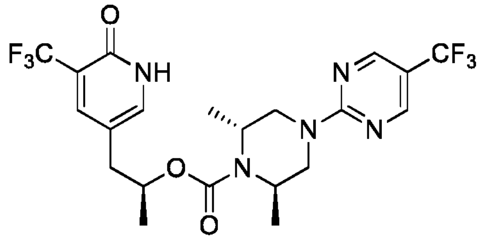
,
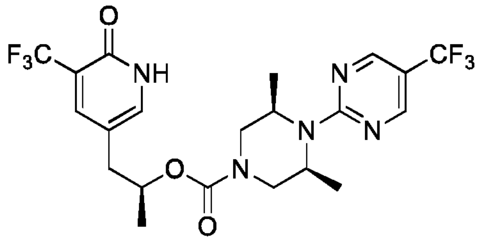
,
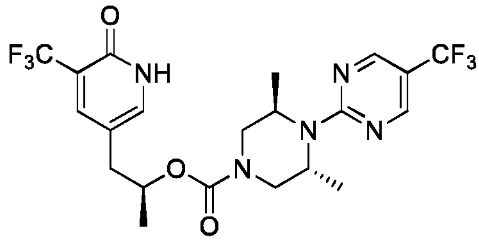
,
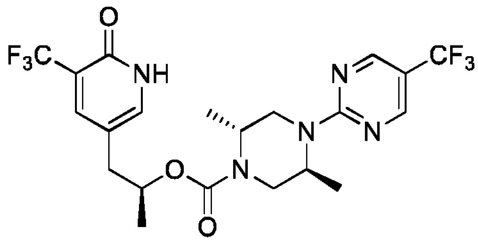
,
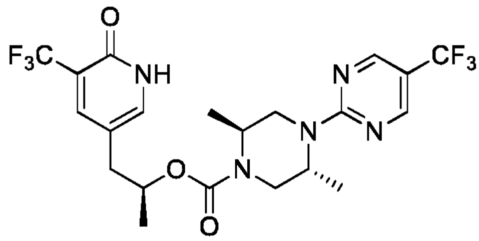
,
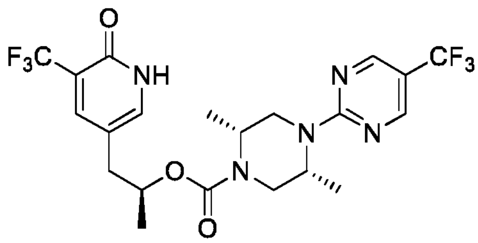
,
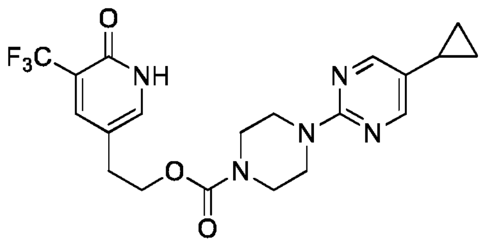
,

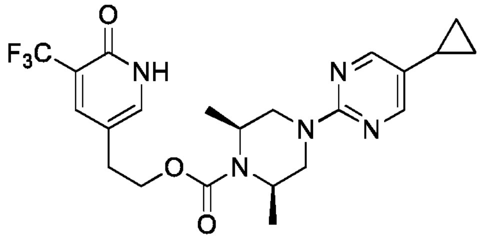,

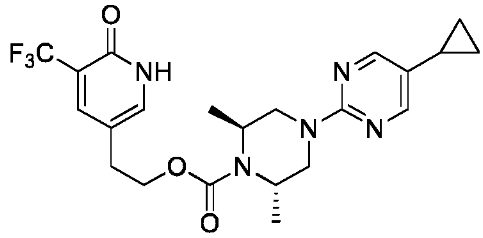,

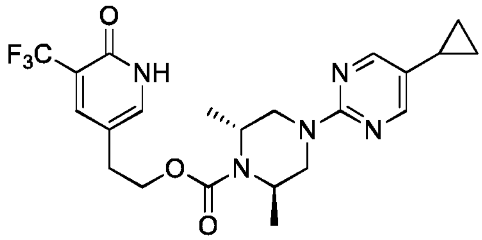,

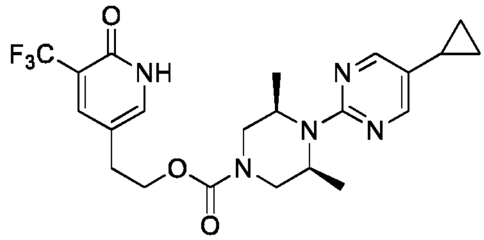,

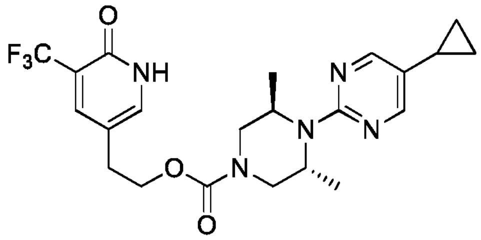,

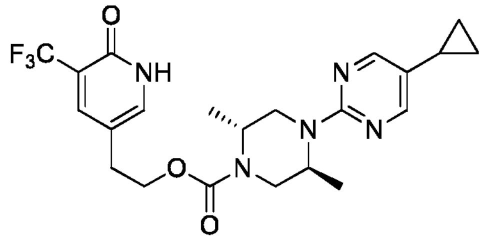,

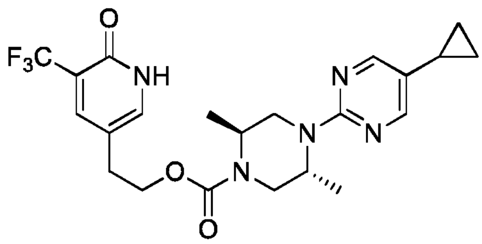,

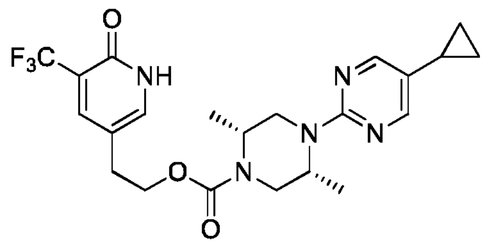,

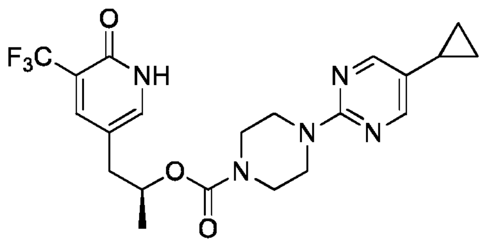,

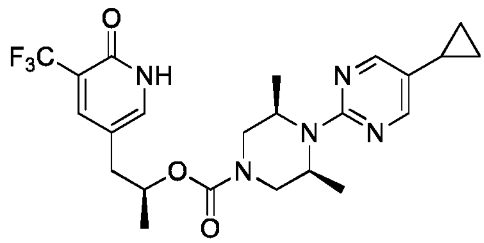,

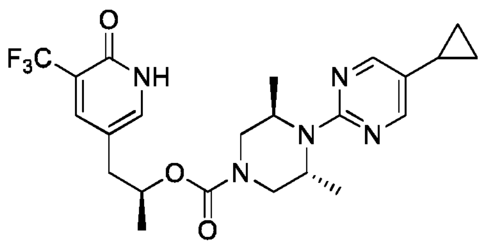,

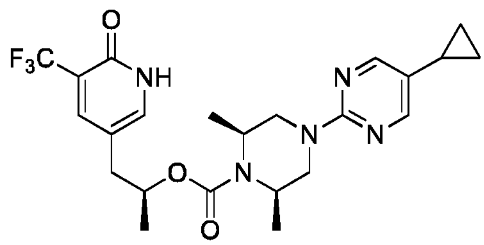,

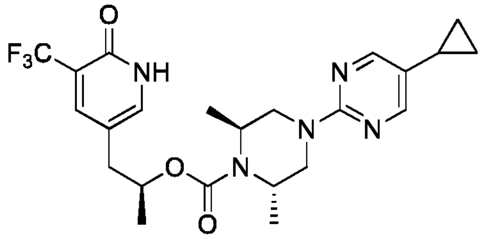
,
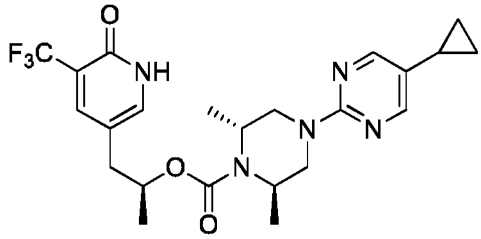
,
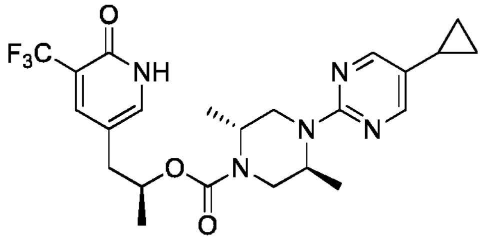
,
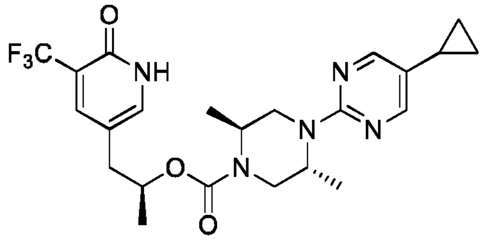
,
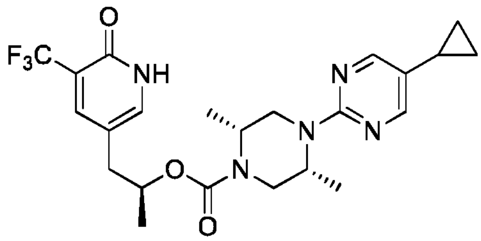
,
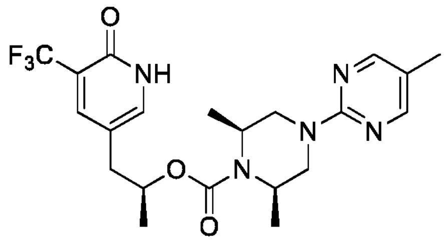
,
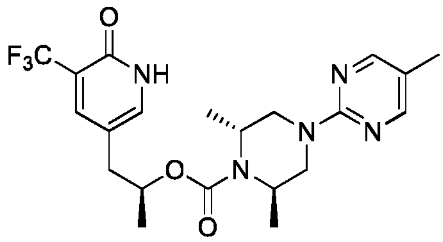
,
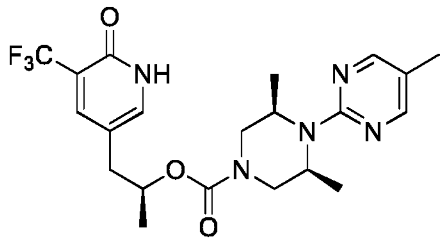
,
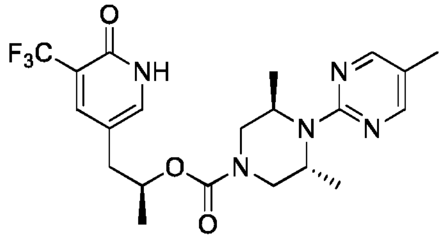
,
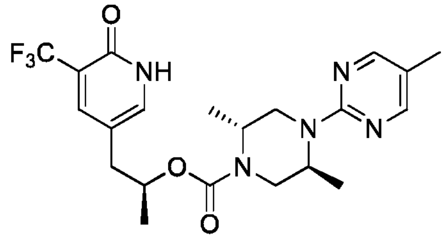
,
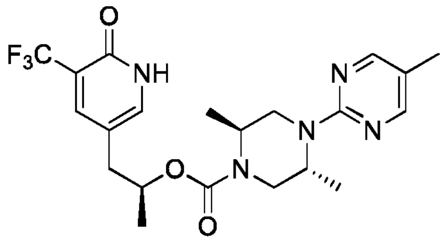
,
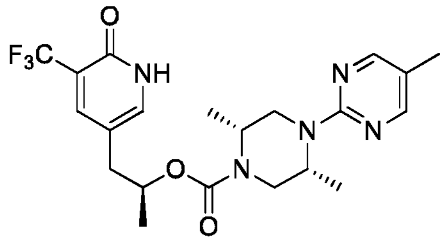
,

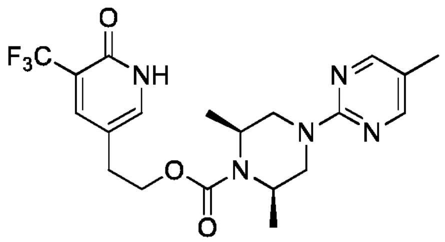

,

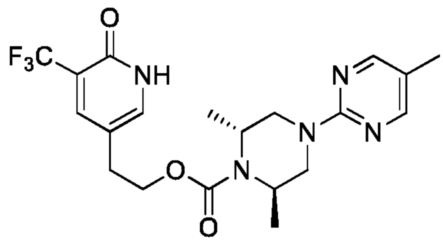

,

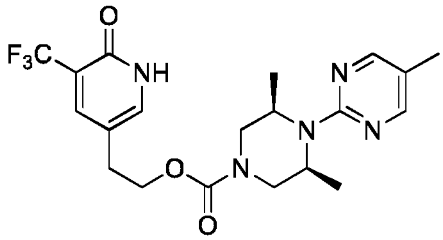

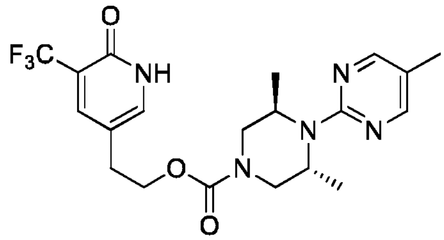

,

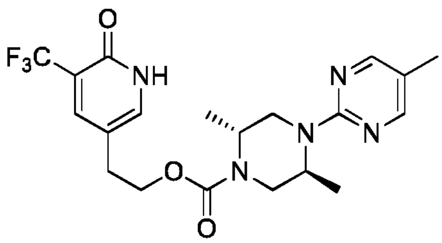

,

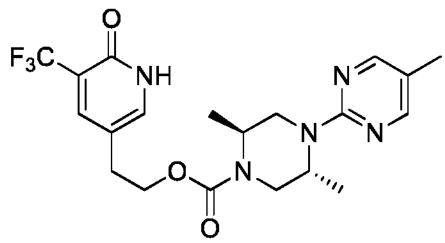

,

, and

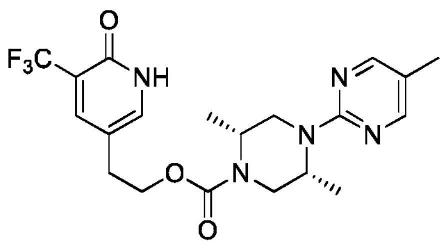

.

**33.** The compound or the pharmaceutically acceptable salt thereof according to any one of claims 1 to 32 for use in the treatment of cancer.

**34.** A pharmaceutical composition comprising the compound or the pharmaceutically acceptable salt thereof according to any one of claims 1 to 32 as an active ingredient and a pharmaceutically acceptable carrier.

**35.** A PARP7 inhibitor comprising the compound or the pharmaceutically acceptable salt thereof according to any one of claims 1 to 32 or the pharmaceutical composition according to claim 34.

**36.** A use of the compound or the pharmaceutically acceptable salt thereof according to any one of claims 1 to 32 or the pharmaceutical composition according to claim 34 in the manufacture of a medicament for the treatment of cancer.

**37.** A method for inhibiting the activity of PARP7, comprising contacting the compound or the pharmaceutically acceptable salt thereof according to any one of claims 1 to 32 or the pharmaceutical composition according to claim 34 with the PARP7.

**38.** A method for the treatment of cancer, comprising administering to a patient a therapeutically effective amount of the compound or the pharmaceutically acceptable salt thereof according to any one of claims 1 to 32.

**INTERNATIONAL SEARCH REPORT**

International application No.
**PCT/CN2022/108475**

**A. CLASSIFICATION OF SUBJECT MATTER**

C07D 403/10(2006.01)i; C07D 237/32(2006.01)i; A61K 31/501(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, CNTXT, DWPI, WPABS, CAPLUS(STN), REGISTRY(STN), MARPAT(STN), CNKI: structural formula search, 酚酞酮, 吡嗪酮, 聚ADP核糖聚合酶, phthalazinone, pyridazin, PARP, cancer

**C. DOCUMENTS CONSIDERED TO BE RELEVANT**

| Category* | Citation of document, with indication, where appropriate, of the relevant passages | Relevant to claim No. |
|---|---|---|
| X | CAS. "STN Registry" *RN 1908832-77-8 et al.,* 12 May 2016 (2016-05-12), pp. 1-5 | 1-13, 15-16, 21-23, 25-26, 29-31 |
| X | US 2011172415 A1 (SHIONOGI & CO.) 14 July 2011 (2011-07-14) description, table 4, and claims 1 and 12 | 1-11, 15, 17, 22-27, 30-31, 34 |
| X | CN 1980674 A (JANSSEN PHARMACEUTICA NV) 13 June 2007 (2007-06-13) description, tables F-1 | 1, 3-11, 15, 17, 21-23, 30-31, 33-36, 38 |
| X | CN 111787916 A (CENTAURUS THERAPEUTICS) 16 October 2020 (2020-10-16) description, pages 146-150 | 1-11, 13, 15-16, 22-23, 29-31, 34 |
| X | CN 101855221 A (ISTITUTO DI RICERCHE DI BIOLOGIAMOLEOLARE P. ANGELETTIS.P.A.) 06 October 2010 (2010-10-06) claims 1 and 10-17, and description paragraph [0641] | 1-11, 15-16, 18, 22-26, 28, 30-38 |
| X | CN 102898377 A (NANJING SHENGHE PHARMACEUTICAL CO., LTD.) 30 January 2013 (2013-01-30) claims 1 and 8-10, and description paragraph [0170] | 1-10, 14-16, 21-26, 30-31, 33-38 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 October 2022** | **25 October 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/108475**

**C. DOCUMENTS CONSIDERED TO BE RELEVANT**

| Category* | Citation of document, with indication, where appropriate, of the relevant passages | Relevant to claim No. |
|---|---|---|
| X | CN 103570725 A (SHANGHAI INSTITUTE OF MATERIA MEDICA, CHINESE ACADEMY OF SCIENCES) 12 February 2014 (2014-02-12) claims 1, 6 and 8-10 | 1-10, 14-16, 21-26, 30-31, 33-38 |
| X | CN 106749261 A (SHANGHAI INSTITUTE OF MATERIA MEDICA, CHINESE ACADEMY OF SCIENCES) 31 May 2017 (2017-05-31) claims 1, 5 and 7-10 | 1-9, 21-26, 30-31, 33, 36-38 |
| X | WO 2012019427 A1 (SHANGHAI HENGRUI MEDICINE CO., LTD. et al.) 16 February 2012 (2012-02-16) claims 1, 9 and 11-12 | 1-9, 21-23, 25-26, 30-31, 33-38 |
| A | GOZGIT, J. M. et al. "Cancer Cell" *PARP7 negatively regulates the type I interferon response in cancer cells and its inhibition triggers antitumor immunity,* Vol. 39, No. 9, 22 July 2021 (2021-07-22), pp. 1214-1226 | 1-38 |

Form PCT/ISA/210 (second sheet) (January 2015)

**INTERNATIONAL SEARCH REPORT**

International application No.
**PCT/CN2022/108475**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **37-38**
because they relate to parts of the international application that do not comply with the prescribed requirements to such an extent that no meaningful international search can be carried out, specifically:

[1] The subject matter of claims 37-38 relates to a method for treating a disease of a human body as defined in PCT Rule 39.1(IV). A search was conducted on the basis of a use of a compound in the preparation of a drug for treating a corresponding disease.

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/108475**

| Patent document cited in search report | Publication date (day/month/year) | Patent family member(s) | Publication date (day/month/year) |
|---|---|---|---|
| US 2011172415 A1 | 14 July 2011 | WO 2007099828 A1 | 07 September 2007 |
| | | EP 2520567 A2 | 07 November 2012 |
| | | EP 1988077 A1 | 05 November 2008 |
| | | US 2009062261 A1 | 05 March 2009 |
| | | JP WO2007099828 A1 | 16 July 2009 |
| | | TW 200800182 A | 01 January 2008 |
| CN 1980674 A | 13 June 2007 | WO 2006003147 A1 | 12 January 2006 |
| | | US 2011065684 A1 | 17 March 2011 |
| | | CA 2569824 A1 | 12 January 2006 |
| | | TW 200610531 A | 01 April 2006 |
| | | US 2015072972 A1 | 12 March 2015 |
| | | NZ 551799 A | 27 November 2009 |
| | | MY 150781 A | 28 February 2014 |
| | | EA 200700192 A1 | 29 June 2007 |
| | | AR 049951 A1 | 20 September 2006 |
| | | ZA 200610774 B | 25 June 2008 |
| | | SG 154433 A1 | 28 August 2009 |
| | | ES 2563954 T3 | 16 March 2016 |
| | | BR PI0512902 A | 15 April 2008 |
| | | MX PA06014542 A | 23 March 2007 |
| | | US 2008139568 A1 | 12 June 2008 |
| | | NO 20070557 L | 30 January 2007 |
| | | KR 20070029246 A | 13 March 2007 |
| | | UA 93351 C2 | 10 February 2011 |
| | | JP 2008504348 A | 14 February 2008 |
| | | EP 1771175 A1 | 11 April 2007 |
| | | IL 180410 D0 | 03 June 2007 |
| | | AU 2005259189 A1 | 12 January 2006 |
| CN 111787916 A | 16 October 2020 | KR 20200119807 A | 20 October 2020 |
| | | US 2020339535 A1 | 29 October 2020 |
| | | WO 2019140188 A1 | 18 July 2019 |
| | | JP 2021510818 A | 30 April 2021 |
| | | AU 2019207887 A1 | 23 July 2020 |
| | | CA 3087729 A1 | 18 July 2019 |
| | | EP 3737361 A1 | 18 November 2020 |
| CN 101855221 A | 06 October 2010 | US 2010261709 A1 | 14 October 2010 |
| | | MX 2010005070 A | 24 May 2010 |
| | | BR PI0820236 A2 | 16 June 2015 |
| | | NZ 585395 A | 30 March 2012 |
| | | RU 2010123874 A | 20 December 2011 |
| | | IL 205142 D0 | 30 November 2010 |
| | | KR 20100087220 A | 03 August 2010 |
| | | JP 2011503166 A | 27 January 2011 |
| | | CA 2704714 A1 | 22 May 2009 |
| | | ES 2524787 T3 | 12 December 2014 |
| | | WO 2009063244 A1 | 22 May 2009 |
| | | AU 2008322676 A1 | 22 May 2009 |
| | | ZA 201002466 B | 29 December 2010 |
| | | EP 2220073 A1 | 25 August 2010 |
| CN 102898377 A | 30 January 2013 | None | |

Form PCT/ISA/210 (patent family annex) (January 2015)

**INTERNATIONAL SEARCH REPORT**
**Information on patent family members**

International application No.
**PCT/CN2022/108475**

| Patent document cited in search report | | | Publication date (day/month/year) | Patent family member(s) | | | Publication date (day/month/year) |
|---|---|---|---|---|---|---|---|
| CN | 103570725 | A | 12 February 2014 | US | 2015166544 | A1 | 18 June 2015 |
| | | | | CA | 2880739 | A1 | 06 February 2014 |
| | | | | JP | 2015527336 | A | 17 September 2015 |
| | | | | AU | 2013299117 | A1 | 19 March 2015 |
| | | | | WO | 2014019468 | A1 | 06 February 2014 |
| | | | | EP | 2881395 | A1 | 10 June 2015 |
| CN | 106749261 | A | 31 May 2017 | US | 2018265516 | A1 | 20 September 2018 |
| | | | | WO | 2017088723 | A1 | 01 June 2017 |
| | | | | AU | 2016359511 | A1 | 21 June 2018 |
| | | | | CN | 108290897 | A | 17 July 2018 |
| | | | | EP | 3381919 | A1 | 03 October 2018 |
| | | | | JP | 2018535229 | A | 29 November 2018 |
| WO | 2012019427 | A1 | 16 February 2012 | PL | 2604610 | T3 | 30 November 2016 |
| | | | | EP | 2604610 | A1 | 19 June 2013 |
| | | | | UA | 111161 | C2 | 11 April 2016 |
| | | | | JP | 2013535491 | A | 12 September 2013 |
| | | | | CN | 102686591 | A | 19 September 2012 |
| | | | | US | 2013131068 | A1 | 23 May 2013 |
| | | | | CN | 102372716 | A | 14 March 2012 |
| | | | | AU | 2011288876 | A1 | 31 January 2013 |
| | | | | KR | 20130110149 | A | 08 October 2013 |
| | | | | PT | 2604610 | T | 13 July 2016 |
| | | | | BR | 112013002220 | A2 | 24 May 2016 |
| | | | | RU | 2013106754 | A | 20 September 2014 |
| | | | | US | 2016151367 | A1 | 02 June 2016 |
| | | | | HU | E029275 | T2 | 28 February 2017 |
| | | | | CA | 2806324 | A1 | 16 February 2012 |
| | | | | ES | 2582315 | T3 | 12 September 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2021108626719
- CN 2022102087593
- CN 2022108397127


### Non-patent literature cited in the description


- **P. CHANG.** Insights into the biogenesis, function, and regulation of ADP- ribosylation. *Nat Chem Biol,* 2018, vol. 14, 236-243
- **S.VYAS et al.** Family-wide analysis of poly(ADP-ribose) polymerase activity. *Nat Commun,* 2014, vol. 5, 4426
- **A. OHMOTO ; S. YACHIDA.** Current status of poly(ADP-ribose) polymerase inhibitors and future directions. *Onco Targets Ther,* 2017, vol. 10, 5195-5208
- **E. L. GOODE et al.** A genome-wide association study identifies susceptibility loci for ovarian cancer at 2q31 and 8q24. *Nat Genet,* 2010, vol. 42, 874-879
- **C. BIGETSBOLL et al.** TCDD-Inducible poly-ADP-ribose (TIPARP/PARP7) mono-ADP-ribosylates and co-activates liver X receptors. *Biochem J,* 2016, vol. 473, 899-910